(19) Europäisches Patentamt / European Patent Office / Office européen des brevets

(11) **EP 3 296 320 A1**

(12) **EUROPEAN PATENT APPLICATION**

(43) Date of publication:
**21.03.2018 Bulletin 2018/12**

(51) Int Cl.:
*C07K 16/28* [(2006.01)]   *C07K 16/30* [(2006.01)]
*G01N 33/53* [(2006.01)]   *G01N 33/574* [(2006.01)]

(21) Application number: **17194775.7**

(22) Date of filing: **08.03.2013**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **08.03.2012 US 201261685089 P**

(62) Document number(s) of the earlier application(s) in
accordance with Art. 76 EPC:
**13710278.6 / 2 822 970**

(71) Applicant: **Halozyme, Inc.
San Diego, CA 92121 (US)**

(72) Inventors:
• **WEI, Ge
San Diego, CA 92131 (US)**
• **FROST, Gregory
Del Mar, CA 92014 (US)**

• **HUANG, Lei
San Diego, CA 92130 (US)**
• **SHEPARD, H. Michael
San Diego, CA 92122 (US)**
• **VAUGHN, Daniel Edward
Encinitas, CA 92024 (US)**

(74) Representative: **Boult Wade Tennant
Verulam Gardens
70 Gray's Inn Road
London WC1X 8BT (GB)**

Remarks:
•The complete document including Reference Tables
and the Sequence Listing can be downloaded from
the EPO website
•This application was filed on 04-10-2017 as a
divisional application to the application mentioned
under INID code 62.
•Claims filed after the date of receipt of the divisional
application (Rule 68(4) EPC).

(54) **CONDITIONALLY ACTIVE ANTI-EPIDERMAL GROWTH FACTOR RECEPTOR ANTIBODIES
AND METHODS OF USE THEREOF**

(57)   Provided herein are modified anti-EGFR antibodies and nucleic acid molecules encoding modified anti-EGFR
antibodies. Also provided are methods of treatment and uses using modified anti-EGFR antibodies.

EP 3 296 320 A1

## Description

### RELATED APPLICATIONS

Benefit of priority is claimed to U.S. Provisional Application Serial No. 61/685,089, entitled "Conditionally Active Anti-Epidermal Growth Factor Receptor Antibodies and Methods of Use Thereof" filed on March 8, 2012.
This application is related to United States Patent Application No. 13/815,553, filed the same day herewith, entitled "Conditionally Active Anti-Epidermal Growth Factor Receptor Antibodies and Methods of Use Thereof," which claims priority to U.S. Provisional Application Serial No. 61/685,089.
This application also is related to U.S. Application Serial No. 13/200,666, filed on September 27, 2011, to Lalitha Kodandapani, Louis Howard Bookbinder, Gregory I. Frost, Philip Lee Sheridan, Harold Michael Shepard, Ge Wei and Lei Huang, entitled "METHODS FOR ASSESSING AND IDENTIFYING OR EVOLVING CONDITIONALLY ACTIVE THERAPEUTIC PROTEINS," which is a continuation-in-part of International Application No. PCT/US 11/50891, filed on September 8, 2011, to Lalitha Kodandapani, Louis Howard Bookbinder, Gregory I. Frost, Philip Lee Sheridan, Harold Michael Shepard, Ge Wei and Lei Huang, entitled "METHODS FOR ASSESSING AND IDENTIFYING OR EVOLVING CONDITIONALLY ACTIVE THERAPEUTIC PROTEINS," which claims priority to U.S. Provisional Application Serial No. 61/402,979, entitled "METHODS FOR ASSESSING AND IDENTIFYING OR EVOLVING CONDITIONALLY ACTIVE THERAPEUTIC PROTEINS AND CONDITIONALLY ACTIVE THERAPEUTIC PROTEINS," filed on September 8, 2010, to Lalitha Kodandapani, Philip Lee Sheridan, Harold Michael Shepard, Louis H. Bookbinder and Gregory I. Frost.
The subject matter of each of the above-noted applications is incorporated by reference in its entirety.

### Incorporation by reference of Sequence Listing provided electronically

An electronic version of the Sequence Listing is filed herewith, the contents of which are incorporated by reference in their entirety. The electronic file was prepared March 8, 2013, is 1237 kilobytes in size, and titled 3104seqPC1.txt.

### FIELD OF THE INVENTION

Provided herein are conditionally active anti-EGFR antibodies, including modified anti-EGFR antibodies, and nucleic acid molecules encoding conditionally active anti-EGFR antibodies, including modified anti-EGFR antibodies. Also provided are methods of treatment using the conditionally active anti-EGFR antibodies.

### BACKGROUND

Anti-EGFR antibodies are used in the clinical setting to treat and diagnose human diseases, for example cancer. For example, exemplary therapeutic antibodies include Cetuximab. Cetuximab is approved for the treatment of recurrent or metastatic head and neck cancer, colorectal cancer and other diseases and conditions. It can also be used in the treatment of other diseases or conditions involving overexpression of EGFR or aberrant signaling or activation of EGFR. Administered anti-EGFR antibodies can bind to EGFR in healthy cells and tissue. This limits the dosages that can be administered. Hence, cetuximab and other anti-EGFR antibodies exhibit limitations when administered to patients. Accordingly, it is among the objects herein to provide improved anti-EGFR antibodies.

### SUMMARY

Provided are conditionally active anti-epidermal growth factor receptor (EGFR) antibodies and antigen binding fragments thereof. The antibodies and fragments thereof are conditionally active such that they exhibit greater activity in a target tissue, such as a tumor microenvironment, which has an acidic pH, than in non-target tissues, such as non-tumor tissue environment, such as that, which occurs in the basal layer of the skin, which has neutral pH around 7-7.2. Generally anti-EGFR antibodies that are employed as anti-tumor therapeutics bind to EGFR receptors and inhibit EGFR-mediated activities that occur upon binding of a ligand therefor. As a result, they can inhibit or treat tumors. Because tissues, other than tumors, such as tissues in the skin express EGFRs, the anti-EGFR antibodies inhibit activities of these receptors, thereby causing undesirable side-effects. The antibodies provided herein are conditionally active in that they exhibit reduced activity at non-tumor microenvironments (e.g. having a neutral pH) compared to antibodies that are not conditionally active and/or compared to their activity in the tumor microenvironment. By virtue of the selectivity to a tumor microenvironment, they exhibit fewer or lesser undesirable side-effects and/or exhibit improved efficacy by virtue of the ability to dose higher.
Provided herein are an anti-EGFR antibody, or antigen-binding fragment thereof, that is conditionally active under conditions in a tumor microenvironment. wherein the anti-EGFR antibody, or antigen-binding fragment thereof, exhibits a ratio of binding activity to human epidermal growth factor receptor (EGFR) or a soluble fragment thereof under conditions in a tumor environment compared to under conditions in a non-tumor environment of at least 3.0. In such an example, the conditions in a tumor environment contain one or both of pH between or about between 5.6 to 6.8 or lactate concentration between or about between 5 mM to 20 mM, and protein concentration of 10 mg/mL to 50 mg/mL; and the conditions in a non-tumor environment contain one or both of pH between or about between 7.0 to 7.8 or lactate concentration between or about between 0.5 mM to 5 mM, and protein concentration of 10 mg/mL to 50 mg/mL. For example, the anti-EGFR antibody, or antigen-binding fragment thereof, exhibits the ratio of activity under conditions that exist in a tumor microenvironment that contain a pH of between or about between 5.6 to 6.8 compared to under conditions that exist in a non-tumor microenvironment that comprise a pH of between or about between 7.0 to 7.8. In another example, the anti-EGFR antibody, or antigen-binding fragment thereof, exhibits the ratio of activity under conditions that exist in a tumor microenvironment that contain a pH of between or about between 6.0 to 6.5 compared to under conditions that exist in a non-tumor microenvironment that comprise a pH of about 7.4. In some instances, the anti-EGFR antibody, or antigen-binding fragment thereof, exhibits the ratio of activity under conditions that exist in a tumor microenvironment that contain lactate concentration between or about between 5 mM to 20 mM compared to under conditions that exist in a non-tumor microenvironment that contain lactate concentration between or about between 0.5 mM to 5 mM. In particular examples herein, the anti-EGFR antibody or, or antigen-binding fragment thereof, of any of claims 1-4, exhibits the ratio of activity under conditions of a tumor microenvironment that contain pH of 6.0 to 6.5 and lactate concentration of 10 mM to 20 mM compared to under condition of a non-tumor microenvironment that contain pH of 7.0 to 7.4, inclusive, and lactate concentration of 0.5 mM to 2 mM.

In any of such examples, the ratio of binding activity is present or exists in the presence of a protein concentration between or about between 10 mg/mL to 50 mg/mL, wherein the protein concentration under conditions in a tumor microenvironment and under conditions in a non-tumor microenvironment is substantially the same or is the same. For example, the protein concentration is at least 12 mg/mL, 15 mg/mL, 20 mg/mL, 25 mg/mL, 30 mg/mL, 35 mg/mL, 40 mg/mL, 45 mg/mL or 50 mg/mL. The protein can be serum albumin, such as human serum albumin. In the protein is provided in serum, such as human serum. For example, the concentration of serum is 20% (vol/vol) to 90% (vol/vol), 20% (vol/vol) to 50% (vol/vol) or 20% (vol/vol) to 40% (vol/vol), for example it is less than 90% (vol/vol) and is about or is at least or is 20% (vol/vol), 25% (vol/vol), 30% (vol/vol), 35% (vol/vol), 40% (vol/vol), 45% (vol/vol) or 50% (vol/vol). In particular example, the ratio of activity is present under conditions containing a serum concentration, such as human serum concentration that is or is about 25% (vol/vol).

In any of the above examples of modified anti-EGFR antibody, or antigen-binding fragment thereof, provided herein, the ratio of binding activity is the ratio of activity under the conditions in a tumor microenvironment compared to under conditions in a non-tumor microenvironment as determined in any assay capable of measuring or assessing binding activity to human EGFR, or to a soluble fragment thereof. For example, binding activity is determined in vitro in a solid-phase binding assay. The solid-phase binding assay can be an immunoassay, such as an enzyme-linked immunosorbent assay (ELISA). In such examples, the binding activity is a spectrophotometric measurement of binding, and the ratio of binding activity is the ratio of the spectrophotometric measurement for binding under conditions that exist in a tumor microenvironment compared to under conditions that exist in a non-tumor microenvironment at the same concentration of antibody, such as a concentration of antibody that is between or about between 1 ng/mL to 100 ng/mL.

In other examples, binding activity is the dissociation constant ($K_D$) as determined using a biosensor, and the antibody, or antigen-binding fragment thereof, exhibits a ratio of at least 3 if there is at least 3-fold tighter affinity under conditions in the tumor-microenvironment compared to under conditions in a non-tumor microenvironment. In such examples, the anti-EGFR antibody, or antigen-binding fragment thereof, typically has a dissociation constant ($K_D$) that is less than $1 \times 10^{-8}$ M, $5 \times 10^{-9}$ M, $1 \times 10^{-9}$ M, $5 \times 10^{-10}$ M, $1 \times 10^{-10}$ M, $5 \times 10^{-11}$ M, $1 \times 10^{-11}$ M or less under conditions that exist in a tumor microenvironment. In further examples, binding activity is the off-rate as determined using a biosensor, and the antibody, or antigen-binding fragment thereof, exhibits a ratio of at least 3 if the off-rate is at least 3 times slower under conditions that exist in a tumor microenvironment compared to under conditions that exist under a non-tumor microenvironment. In any of such examples using a biosensor, the biosensor can be a Biacore sensor or Octet sensor or other similar biosensor known to the skilled artisan.

In a further example herein, binding activity is assessed *in vivo* in a subject in a tumor microenvironment expressing EGFR or in a non-tumor microenvironment expressing EGFR. In such an example, the non-tumor microenvironment is the basal layer of the skin expressing human EGFR. Such in vivo binding activity can be determined in a subject that is a non-human animal, where the tumor microenvironment is a human tumor xenograft expressing human EGFR and the non-tumor microenvironment is a human skin xenograft expressing human EGFR. For example, the human tumor xenograft is an A431 xenograft. In such examples, the anti-EGFR antibody, or antigen-binding fragment thereof, can be fluorescently labeled, and binding activity under both conditions is determined as the fluorescent signal intensity, which can be normalized to a control IgG.

In any of the examples of the anti-EGFR antibody, or antigen-binding fragment thereof, provided herein, the ratio of activity is at least 3.5, 4, 5, 6, 7, 8, 9, 10, 11, 12, 13, 14, 15, 16, 17, 18, 19, 20, 25, 30, 35, 40, 45, 50, 55, 60 or more.

In any of the examples of the anti-EGFR antibody, or antigen-binding fragment thereof, provided herein, the anti-EGFR antibody, or antigen-binding fragment thereof, contains a variable heavy chain that exhibits at least 56% sequence identity to its closest human $V_H$ gene segment germline sequence; and a light chain that exhibits at least 75% sequence identity to its closest human $V_L$ gene segment germline sequence.

For example, including among anti-EGFR antibodies, or antigen-binding fragments thereof, provided herein is an antibody that contains: a variable heavy (VH) chain having the sequence of amino acids set forth in SEQ ID NO:495, 1062, 1112, 1114-1118, 1124-1126, 1128-1130, 1134-1137, or 1146-1152, or a sequence of amino acids that exhibits at least 85% sequence identity to any of SEQ ID NOS: 495, 1062, 1112, 1114-1118, 1124-1126, 1128-1130, 1134-1137, or 1146-1152; and a variable light (VL) chain having the sequence of amino acids set forth in SEQ ID NO:4, 10, 1138-1145, 1153-1159 or 1186, or a sequence of amino acids that exhibits at least 85% sequence identity to SEQ ID NO:4, 10, 1138-1145, 1153-1159 or 1186.

For example, among non-limiting examples of anti-EGFR antibody, or antigen-binding fragment thereof, provided herein is an antibody that contains:

a) the variable heavy chain set forth in SEQ ID NO:495 or a sequence of amino acids that exhibits at least 85% sequence identity to SEQ ID NO:495, and the variable light chain set forth in SEQ ID NO:4 or 10 or a sequence of amino acids that exhibits at least 85% sequence identity to SEQ ID NO:4 or 10;

b) the variable heavy chain set forth in SEQ ID NO:1062 or a sequence of amino acids that exhibits at least 85% sequence identity to SEQ ID NO:1062, and the variable light chain set forth in SEQ ID NO:4 or 10 or a sequence of amino acids that exhibits at least 85% sequence identity to SEQ ID NO:4 or 10;

c) the variable heavy chain set forth in SEQ ID NO:1112 or a sequence of amino acids that exhibits at least 85% sequence identity to SEQ ID NO: 1112, and the variable light chain set forth in SEQ ID NO:4 or 10 or a sequence of amino acids that exhibits at least 85% sequence identity to SEQ ID NO:4 or 10;

d) the variable heavy chain set forth in SEQ ID NO:1114 or a sequence of amino acids that exhibits at least 85% sequence identity to SEQ ID NO: 1114, and the variable light chain set forth in SEQ ID NO:4 or 10 or a sequence of amino acids that exhibits at least 85% sequence identity to SEQ ID NO:4 or 10;

e) the variable heavy chain set forth in SEQ ID NO:1115 or a sequence of amino acids that exhibits at least 85% sequence identity to SEQ ID NO: 1115, and the variable light chain set forth in SEQ ID NO:4 or 10 or a sequence of amino acids that exhibits at least 85% sequence identity to SEQ ID NO:4 or 10;

f) the variable heavy chain set forth in SEQ ID NO:1116 or a sequence of amino acids that exhibits at least 85% sequence identity to SEQ ID NO: 1116, and the variable light chain set forth in SEQ ID NO:4 or 10 or a sequence of amino acids that exhibits at least 85% sequence identity to SEQ ID NO:4 or 10;

g) the variable heavy chain set forth in SEQ ID NO:1117 or a sequence of amino acids that exhibits at least 85% sequence identity to SEQ ID NO:1117, and the variable light chain set forth in SEQ ID NO:4 or 10 or a sequence of amino acids that exhibits at least 85% sequence identity to SEQ ID NO:4 or 10;

h) the variable heavy chain set forth in SEQ ID NO:1124 or a sequence of amino acids that exhibits at least 85% sequence identity to SEQ ID NO:1124, and the variable light chain set forth in SEQ ID NO:4 or 10 or a sequence of amino acids that exhibits at least 85% sequence identity to SEQ ID NO:4 or 10;

i) the variable heavy chain set forth in SEQ ID NO:1125 or a sequence of amino acids that exhibits at least 85% sequence identity to SEQ ID NO:1125, and the variable light chain set forth in SEQ ID NO:4 or 10 or a sequence of amino acids that exhibits at least 85% sequence identity to SEQ ID NO:4 or 10;

j) the variable heavy chain set forth in SEQ ID NO:1126 or a sequence of amino acids that exhibits at least 85% sequence identity to SEQ ID NO:1126, and the variable light chain set forth in SEQ ID NO:4 or 10 or a sequence of amino acids that exhibits at least 85% sequence identity to SEQ ID NO:4 or 10;

k) the variable heavy chain set forth in SEQ ID NO:1128 or a sequence of amino acids that exhibits at least 85% sequence identity to SEQ ID NO:1128, and the variable light chain set forth in SEQ ID NO:4 or 10 or a sequence of amino acids that exhibits at least 85% sequence identity to SEQ ID NO:4 or 10;

1) the variable heavy chain set forth in SEQ ID NO:1129 or a sequence of amino acids that exhibits at least 85% sequence identity to SEQ ID NO:1129, and the variable light chain set forth in SEQ ID NO:4 or 10 or a sequence of amino acids that exhibits at least 85% sequence identity to SEQ ID NO:4 or 10;

m) the variable heavy chain set forth in SEQ ID NO:1130 or a sequence of amino acids that exhibits at least 85% sequence identity to SEQ ID NO:1130, and the variable light chain set forth in SEQ ID NO:4 or 10 or a sequence of amino acids that exhibits at least 85% sequence identity to SEQ ID NO:4 or 10;

n) the variable heavy chain set forth in SEQ ID NO:1134 or a sequence of amino acids that exhibits at least 85% sequence identity to SEQ ID NO:1134, and the variable light chain set forth in SEQ ID NO:1138 or a sequence of amino acids that exhibits at least 85% sequence identity to SEQ ID NO:1138;

o) the variable heavy chain set forth in SEQ ID NO:1134 or a sequence of amino acids that exhibits at least 85% sequence identity to SEQ ID NO:1134, and the variable light chain set forth in SEQ ID NO:1139 or a sequence of amino acids that exhibits at least 85% sequence identity to SEQ ID NO:1139;

p) the variable heavy chain set forth in SEQ ID NO:1135 or a sequence of amino acids that exhibits at least 85% sequence identity to SEQ ID NO:1135, and the variable light chain set forth in SEQ ID NO:1138 or a sequence of amino acids that exhibits at least 85% sequence identity to SEQ ID NO:1138;

q) the variable heavy chain set forth in SEQ ID NO:1134 or a sequence of amino acids that exhibits at least 85% sequence identity to SEQ ID NO:1134, and the variable light chain set forth in SEQ ID NO:1140 or a sequence of amino acids that exhibits at least 85% sequence identity to SEQ ID NO:1140;

r) the variable heavy chain set forth in SEQ ID NO:1134 or a sequence of amino acids that exhibits at least 85% sequence identity to SEQ ID NO:1134, and the variable light chain set forth in SEQ ID NO:1141 or a sequence of amino acids that exhibits at least 85% sequence identity to SEQ ID NO:1141;

s) the variable heavy chain set forth in SEQ ID NO:1134 or a sequence of amino acids that exhibits at least 85% sequence identity to SEQ ID NO: 1134, and the variable light chain set forth in SEQ ID NO:1142 or a sequence of amino acids that exhibits at least 85% sequence identity to SEQ ID NO:1142;

t) the variable heavy chain set forth in SEQ ID NO:1135 or a sequence of amino acids that exhibits at least 85% sequence identity to SEQ ID NO:1135, and the variable light chain set forth in SEQ ID NO:1142 or a sequence of amino acids that exhibits at least 85% sequence identity to SEQ ID NO:1142;

u) the variable heavy chain set forth in SEQ ID NO:1134 or a sequence of amino acids that exhibits at least 85% sequence identity to SEQ ID NO:1134, and the variable light chain set forth in SEQ ID NO:1143 or a sequence of amino acids that exhibits at least 85% sequence identity to SEQ ID NO:1143;

v) the variable heavy chain set forth in SEQ ID NO:1136 or a sequence of amino acids that exhibits at least 85% sequence identity to SEQ ID NO:1136, and the variable light chain set forth in SEQ ID NO:1142 or a sequence of amino acids that exhibits at least 85% sequence identity to SEQ ID NO:1142;

w) the variable heavy chain set forth in SEQ ID NO:1137 or a sequence of amino acids that exhibits at least 85% sequence identity to SEQ ID NO:1137, and the variable light chain set forth in SEQ ID NO:1144 or a sequence of amino acids that exhibits at least 85% sequence identity to SEQ ID NO:1144;

x) the variable heavy chain set forth in SEQ ID NO:1136 or a sequence of amino acids that exhibits at least 85% sequence identity to SEQ ID NO:1136, and the variable light chain set forth in SEQ ID NO:1144 or a sequence of amino acids that exhibits at least 85% sequence identity to SEQ ID NO:1144;

y) the variable heavy chain set forth in SEQ ID NO:1137 or a sequence of amino acids that exhibits at least 85% sequence identity to SEQ ID NO:1137, and the variable light chain set forth in SEQ ID NO:1145 or a sequence of amino acids that exhibits at least 85% sequence identity to SEQ ID NO:1145;

z) the variable heavy chain set forth in SEQ ID NO:1136 or a sequence of amino acids that exhibits at least 85% sequence identity to SEQ ID NO:1136, and the variable light chain set forth in SEQ ID NO:1145 or a sequence of amino acids that exhibits at least 85% sequence identity to SEQ ID NO:1145;

aa) the variable heavy chain set forth in SEQ ID NO:1146 or a sequence of amino acids that exhibits at least 85% sequence identity to SEQ ID NO:1146, and the variable light chain set forth in SEQ ID NO:1153 or a sequence of amino acids that exhibits at least 85% sequence identity to SEQ ID NO:1153;

bb) the variable heavy chain set forth in SEQ ID NO:1147 or a sequence of amino acids that exhibits at least 85% sequence identity to SEQ ID NO:1147, and the variable light chain set forth in SEQ ID NO:1153 or a sequence of amino acids that exhibits at least 85% sequence identity to SEQ ID NO:1153;

cc) the variable heavy chain set forth in SEQ ID NO:1148 or a sequence of amino acids that exhibits at least 85% sequence identity to SEQ ID NO:1148, and the variable light chain set forth in SEQ ID NO:1154 or a sequence of amino acids that exhibits at least 85% sequence identity to SEQ ID NO:1154;

dd) the variable heavy chain set forth in SEQ ID NO:1149 or a sequence of amino acids that exhibits at least 85% sequence identity to SEQ ID NO:1149, and the variable light chain set forth in SEQ ID NO:1154 or a sequence of amino acids that exhibits at least 85% sequence identity to SEQ ID NO:1154;

ee) the variable heavy chain set forth in SEQ ID NO:1150 or a sequence of amino acids that exhibits at least 85% sequence identity to SEQ ID NO:1150, and the variable light chain set forth in SEQ ID NO:1155 or a sequence of amino acids that exhibits at least 85% sequence identity to SEQ ID NO:1155;

ff) the variable heavy chain set forth in SEQ ID NO:1151 or a sequence of amino acids that exhibits at least 85% sequence identity to SEQ ID NO:1151, and the variable light chain set forth in SEQ ID NO:1156 or a sequence of amino acids that exhibits at least 85% sequence identity to SEQ ID NO:1156;

gg) the variable heavy chain set forth in SEQ ID NO:1146 or 1148 or a sequence of amino acids that exhibits at least 85% sequence identity to SEQ ID NO:1146 or 1148, and the variable light chain set forth in SEQ ID NO:1156 or a sequence of amino acids that exhibits at least 85% sequence identity to SEQ ID NO:1156;

hh) the variable heavy chain set forth in SEQ ID NO:1149 or a sequence of amino acids that exhibits at least 85% sequence identity to SEQ ID NO:1149, and the variable light chain set forth in SEQ ID NO:1156 or a sequence of amino acids that exhibits at least 85% sequence identity to SEQ ID NO:1156;

ii) the variable heavy chain set forth in SEQ ID NO:1150 or a sequence of amino acids that exhibits at least 85% sequence identity to SEQ ID NO:1150, and the variable light chain set forth in SEQ ID NO:1157 or a sequence of amino acids that exhibits at least 85% sequence identity to SEQ ID NO:1157;

jj) the variable heavy chain set forth in SEQ ID NO:1152 or a sequence of amino acids that exhibits at least 85% sequence identity to SEQ ID NO:1152, and the variable light chain set forth in SEQ ID NO:1157 or a sequence of amino acids that exhibits at least 85% sequence identity to SEQ ID NO:1157;

kk) the variable heavy chain set forth in SEQ ID NO:1148 or a sequence of amino acids that exhibits at least 85% sequence identity to SEQ ID NO:1148, and the variable light chain set forth in SEQ ID NO:1157 or a sequence of amino acids that exhibits at least 85% sequence identity to SEQ ID NO:1157;

11) the variable heavy chain set forth in SEQ ID NO:1149 or a sequence of amino acids that exhibits at least 85% sequence identity to SEQ ID NO:1149, and the variable light chain set forth in SEQ ID NO:1157 or a sequence of amino acids that exhibits at least 85% sequence identity to SEQ ID NO:1157;

mm) the variable heavy chain set forth in SEQ ID NO:1150 or a sequence of amino acids that exhibits at least 85% sequence identity to SEQ ID NO:1150, and the variable light chain set forth in SEQ ID NO:1186 or a sequence of amino acids that exhibits at least 85% sequence identity to SEQ ID NO:1186;

nn) the variable heavy chain set forth in SEQ ID NO:1152 or a sequence of amino acids that exhibits at least 85% sequence identity to SEQ ID NO:1152, and the variable light chain set forth in SEQ ID NO:1186 or a sequence of amino acids that exhibits at least 85% sequence identity to SEQ ID NO:1186;

oo) the variable heavy chain set forth in SEQ ID NO:1148 or a sequence of amino acids that exhibits at least 85% sequence identity to SEQ ID NO:1148, and the variable light chain set forth in SEQ ID NO:1186 or a sequence of amino acids that exhibits at least 85% sequence identity to SEQ ID NO:1186;

pp) the variable heavy chain set forth in SEQ ID NO:1149 or a sequence of amino acids that exhibits at least 85% sequence identity to SEQ ID NO:1149, and the variable light chain set forth in SEQ ID NO:1186 or a sequence of amino acids that exhibits at least 85% sequence identity to SEQ ID NO:1186;

qq) the variable heavy chain set forth in SEQ ID NO:1150 or a sequence of amino acids that exhibits at least 85% sequence identity to SEQ ID NO:1150, and the variable light chain set forth in SEQ ID NO:1158 or a sequence of amino acids that exhibits at least 85% sequence identity to SEQ ID NO:1158;

rr) the variable heavy chain set forth in SEQ ID NO:1152 or a sequence of amino acids that exhibits at least 85% sequence identity to SEQ ID NO:1152, and the variable light chain set forth in SEQ ID NO:1159 or a sequence of amino acids that exhibits at least 85% sequence identity to SEQ ID NO:1159;

ss) the variable heavy chain set forth in SEQ ID NO:1146 or a sequence of amino acids that exhibits at least 85% sequence identity to SEQ ID NO:1146, and the variable light chain set forth in SEQ ID NO:1159 or a sequence of amino acids that exhibits at least 85% sequence identity to SEQ ID NO:1159;

tt) the variable heavy chain set forth in SEQ ID NO:1146 or a sequence of amino acids that exhibits at least 85% sequence identity to SEQ ID NO:1146, and the variable light chain set forth in SEQ ID NO:1157 or a sequence of amino acids that exhibits at least 85% sequence identity to SEQ ID NO:1157;

uu) the variable heavy chain set forth in SEQ ID NO:1146 or a sequence of amino acids that exhibits at least 85% sequence identity to SEQ ID NO:1146, and the variable light chain set forth in SEQ ID NO:1186 or a sequence of amino acids that exhibits at least 85% sequence identity to SEQ ID NO:1186; and

vv) the variable heavy chain set forth in SEQ ID NO:1118 or a sequence of amino acids that exhibits at least 85% sequence identity to SEQ ID NO:1118, and the variable light chain set forth in SEQ ID NO:4 or 10 or a sequence of amino acids that exhibits at least 85% sequence identity to SEQ ID NO:4 or 10.

33. The anti-EGFR antibody, or antigen-binding fragment thereof, of claim 31 or claim 32, wherein sequence identity is at least 86%, 87%, 88%, 89%, 90%, 91%, 92%, 93%, 94%, 95%, 96%, 97%, 98%, 98%, 99% or more.

Included among any of the anti-EGFR antibodies, or antigen-binding fragments thereof, provided herein, are antibodies or antigen-binding fragments thereof that are capable of being expressed in mammalian cells containing nucleic acid(s) encoding the antibody at a concentration of at least 1 mg/mL, for example at least 1.5 mg/mL, 2 mg/mL, 3 mg/mL, 5 mg/mL, 6 mg/mL, 7 mg/mL, 8 mg/mL, 9 mg/mL, 10 mg/mL or more.

Any of the anti-EGFR antibodies, or antigen-binding fragments, provided herein are antibodies that are modified anti-EGFR antibodies or antigen-binding fragments thereof. For example, among the conditionally active anti-EGFR antibodies provided herein, are anti-EGFR antibodies and antigen-binding fragments thereof that are variants of anti-EGFR antibodies that do not exhibit this conditional activity or that exhibit conditional activity to a lesser extent. Hence, provided are antibodies that are modified forms of the therapeutic antibody designated cetuximab and other variants of cetuximab, such as humanized versions thereof and other forms (see, *e.g.,* published International PCT application Nos. WO2011059762, WO2005056606A2, WO2006009694, WO2010080463, WO2012020059, WO2008152537, WO9640210 and U.S. Patent Nos. 7,060,808 7,723,484 and 7,930,107, which describe anti-EGFR antibodies). Thus, the unmodified antibody can be a cetuximab antibody, antigen-binding fragment thereof and variants thereof that do not include the amino acid replacement and specifically binds to EGFR (see, *e.g.,* those anti-EGFR antibodies described in any of published International PCT application Nos. WO2011059762, WO2005056606A2, WO2006009694, WO2010080463, WO2012020059, WO2008152537, WO9640210 and U.S. Patent Nos. 7,060,808, 7,723,484 and 7,930,107 and other family member applications/patents). The modified anti-EGFR antibody and fragments thereof are conditionally active in a tumor microenvironment.

The conditionally active antibodies, such as modified, anti-EGFR antibodies and antigen-binding fragments thereof include those with an amino acid replacement(s) in a variable heavy chain, variable light chain or both of the unmodified antibody or in such regions in the antigen-binding fragments thereof. In some examples, the unmodified anti-EGFR antibody is cetuximab, an antigen-binding fragment thereof or a variant thereof that does not include the amino acid replacement and specifically binds to EGFR. In particular examples, the modified anti-EGFR antibody and fragment thereof can exhibit a ratio of binding activity for EGFR at or about pH 6.0 to pH 6.5 compared to at or about pH 7.4 of at least 2.0, when measured under the same conditions except for the difference in pH. In some examples, the modified anti-EGFR antibody exhibits less than 40% of the binding activity for EGFR at pH 7.4 compared to the unmodified antibody at pH 7.4 when measured under the same conditions, with the proviso that the modified anti-EGFR antibody and fragment thereof does not include: a) a variable heavy chain that includes an amino acid replacement selected from among N31I, N31V, V50L, Y59E and T64N; or b) a variable light chain that includes an amino acid replacement L4C.

In any of the examples of the modified anti-EGFR antibodies and fragments thereof provided herein, the modified anti-EGFR antibody exhibits at least 20% of the binding activity for EGFR at or about pH 6.0 to pH 6.5 compared to the unmodified antibody at pH 6.0 to pH 6.5 when measured under the same conditions.

In any examples of the modified anti-EGFR antibodies and fragments thereof, the variable heavy chain, or a portion thereof, includes an amino acid replacement corresponding to an amino acid replacement selected from among HC-V24E, HC-V24I, HC-V24L, HC-S25C, HC-S25H, HC-S25R, HC-S25A, HC-S25D, HC-S25G, HC-S25M, HC-S25Q, HC-S25V, HC-S25L, HC-S28C, HC-L29H, HC-N31H, HC-G54D, HC-G54S, HC-F63R, HC-F63C, HC-F63M, HC-F63P, HC-F63S, HC-T64V, HC-L67G, HC-D72L, HC-D72P, HC-D72W, HC-N73Q, HC-K75H, HC-K75G, HC-K75P, HC-K75W, HC-S76I, HC-S76V, HC-Q77E, HC-T100P, HC-Y104D, HC-Y104S, HC-Y104V, HC-Q111I, HC-Q111V, with reference to amino acid positions set forth in SEQ ID NO:3. Corresponding amino acid positions can be identified by alignment of the VH chain of the antibody with the VH chain set forth in SEQ ID NO:3. The portion thereof can be sufficient to form an antigen binding site and include the amino acid replacement. In some examples, the modified variable light chain, or portion thereof, includes an amino acid replacement corresponding to an amino acid replacement selected from among LC-L4F, LC-L4V, LC-T5P and LC-R24G, with reference to amino acid positions set forth in SEQ ID NO:4. Corresponding amino acid positions can be identified by alignment of the VL chain of the antibody with the VL chain set forth in SEQ ID NO:4, and the portion thereof can be sufficient to form an antigen binding site and include the amino acid replacement.

Also included among modified anti-EGFR antibodies and antigen binding fragments thereof provided herein include those with an amino acid replacement(s) of one or more amino acid residues in the complementarity determining region (CDR) L2 of a variable light chain of the unmodified antibody. In the modified anti-EGFR antibodies and antigen-binding fragments thereof provided herein, the variable light chain, or portion thereof, can include an amino acid replacement corresponding to an amino acid replacement selected from among LC-A51T, LC-A51L, LC-S52A, LC-S52C, LC-S52D, LC-S52E, LC-S52G, LC-S52I, LC-S52M, LC-S52Q, LC-S52V, LC-S52W, LC-S52R, LC-S52K, LC-E53G, LC-S54M, LC-I55A, LC-I55F, LC-S56G, LC-S56L, LC-S56A, LC-S56C, LC-S56D, LC-S56E, LC-S56F, LC-S56N, LC-S56P, LC-S56Q, LC-S56V, LC-S56W, LC-S56H, LC-S56R and LC-S56K corresponding to amino acid residues set forth in SEQ ID NO:4. The portion thereof can be sufficient to form an antigen binding site and include the amino acid replacement. In some examples of any of the modified anti-EGFR antibodies and fragments thereof provided herein, the modified anti-EGFR antibody and fragment thereof is conditionally active in a tumor microenvironment.

Also included among the modified anti-EGFR antibodies and antigen-binding fragment thereof, are any that can include an amino acid replacement in a variable heavy (VH) chain, variable light (VL) chain or both of the unmodified antibody. In some examples of the modified anti-EGFR antibodies provided herein, the unmodified anti-EGFR antibody is cetuximab, an antigen-binding fragment or a variant thereof that does not include the amino acid replacement and specifically binds to EGFR. The amino acid replacement residue in the VH chain can occur at an amino acid position corresponding to amino acid residues selected from among, for example, 26, 36, 66, 69, 75, 93, 94, 109, 110, 111 and 112 with reference to amino acid positions set forth in SEQ ID NO:3, and corresponding amino acid positions are identified by alignment of the VH chain of the antibody with the VH chain set forth in SEQ ID NO:3. In some examples, the amino acid replacement in the VL chain occurs at an amino acid position corresponding to amino acid residues selected from among 29, 48, 51, 52, 53, 55, 56, 86 and 98, with reference to amino acid positions set forth in SEQ ID NO:4, and corresponding amino acid positions are identified by alignment of the VL chain of the antibody with the VL chain set forth in SEQ ID NO:4. In some examples of any of the modified anti-EGFR antibodies and fragments thereof provided herein, the modified anti-EGFR antibody and fragment thereof is conditionally active in a tumor microenvironment.

The modified anti-EGFR antibodies, or antigen-binding fragments thereof, provided herein include any in which the variable heavy chain, or portion thereof, includes an amino acid replacement corresponding to an amino acid replacement selected from among G26H, G026R, G026D, G026F, G026M, G026N, G026P, G026Q, G026S, G026Y, G026L, W036K, W036A, W036I, W036V, W036Y, R066L, R066A, R066C, R066E, R066F, R066N, R066P, R066Q, R066S, R066T, R066V, R066G, I069A, I069C, I069G, I069Y, K075H, K075R, K075L, K075A, K075C, K075E, K075F, K75G, K075M, K75P, K075Q, K075T, K075V, K075W, K075Y, Y093H, Y093V, Y093W, Y094R, Y094L, W109I, W109M, W109Y, G110R, G110A, G110M, G110P, G110T, Q111K, Q111H, Q111R, Q111L, Q111D, Q111E, Q111G, Q111I, Q111M, Q111P, Q111S, Q111T, Q111V, Q111W, Q111Y, G112A, G112N, G112P, G112S, G112T and HC-G112Y, with reference to amino acid residues set forth in SEQ ID NO:3, and the portion thereof is sufficient to form an antigen binding site and includes the amino acid replacement; and/or the variable light chain, or portion thereof, includes an amino replacement corresponding to an amino acid replacement selected from among I029A, I029E, I029F, I029S, I029T, I029R, I048M, I048S, I048L, I048K, A051T, A051L, S052A, S052C, S052D, S052E, S052G, S052I, S052M, S052Q, S052V, S052W, S052R, S052K, E53G, I055A, I055F, S056G, S056L, S056A, S056C, S056D, S056E, S056F, S056N, S056P, S056Q, S056V, S056W, S056H, S056R, S056K, Y086F, Y086M, Y086H, F098A, F098M, F098S, F098V and F098Y, with reference to residues set forth in SEQ ID NO:4, and the portion thereof is sufficient to form an antigen binding site and includes the amino acid replacement.

In any of the examples herein, the ratio of binding activity of the modified anti-EGFR antibodies and antigen-binding fragments thereof, at pH 6.0 or pH 6.5, compared to at or about pH 7.4 is at least 1.1, 1.2, 1.3, 1.4, 1.5, 1.6, 1.7, 1.8, 1.9, 2.0, 2.5, 3.0, 4.0, 4.5, 5.0, or greater. In any of the examples herein, the modified anti-EGFR antibodies, and antigen-binding fragments thereof, provided herein, exhibit greater binding activity for EGFR at or about pH 6.0 to pH 6.5 compared to at or about pH 7.4 of at least 2.0, when measured under the same conditions except for the difference in pH.

In any of the examples herein, the modified anti-EGFR antibodies, or antigen-binding fragments thereof, provided herein, can exhibit reduced binding activity at pH 7.4 for EGFR compared to the corresponding form of a cetuximab antibody that includes a variable heavy chain set forth in SEQ ID NO:3 and a variable light chain set forth in SEQ ID NO:4 or SEQ ID NO:10 at pH 7.4, and binding activity is measured under the same conditions. For example, the modified anti-EGFR antibodies, or antigen-binding fragments thereof, can exhibit less than 90%, 80%, 70%, 60%, 50%, 40%, 30%, 20% or less of the binding activity of the corresponding form of a cetuximab antibody.

In any of the examples herein, the modified anti-EGFR antibodies, or antigen-binding fragments thereof, provided herein, can exhibit increased binding activity at or about pH 6.0 to 6.5 for EGFR compared to the corresponding form of a cetuximab antibody that includes a variable heavy chain set forth in SEQ ID NO:3 and a variable light chain set forth in SEQ ID NO:4 or SEQ ID NO:10 at pH 7.4, and binding activity is measured under the same conditions. For example, the modified anti-EGFR antibodies, or antigen-binding fragments thereof, can exhibit greater than 110%, 120%, 130%, 140%, 150%, 160%, 170%, 180%, 190%, 200%, 250%, 300%, 350%, 400%, 500% or more of the binding activity of the corresponding form of a cetuximab antibody.

Also included among anti-EGFR antibodies provided herein are modified anti-EGFR antibodies, or antigen-binding fragments thereof, that include an amino acid replacement(s) in a variable heavy (VH) chain, variable light (VL) chain or both of the unmodified antibody. In some examples, the unmodified anti-EGFR antibody is cetuximab, an antigen-binding fragment thereof or variant thereof that does not include the amino acid replacement(s) and specifically binds to EGFR. The VH chain, or portion thereof, can include one or more amino acid replacement(s) corresponding to amino acid replacement(s) selected from among T023H, T023R, T023C, T023E, T023G, T023I, T023M, T023N, T023P, T023S, T023V, T023W, T023L, V024R, V024F, V024G, V024I, V024M, V024P, V024S, V024T, V024L, S025H, S025R, S025A, S025D, S025E, S025F, S025G, S025I, S025M, S025P, S025Q, S025T, S025V, S025L, G026H, G026R, G026D, G026F, G026M, G026N, G026P, G026Q, G026S, G026Y, G026L, F027H, F027R, F027A, F027D, F027E, F027M, F027P, F027Q, F027S, F027T, F027V, F027W, F027Y, F027L, S028K, S028H, S028R, S028A, S028D, S028I, S028M, S028P, S028Q, S028V, S028W, S028L, L029K, L029H, L029A, L029D, L029G, L029M, L029N, L029S, L029V, T030H, T030R, T030D, T030G, T030I, T030M, T030N, T030P, T030V, T030W, T030Y, N031K, N031H, N031E, N031G, N031L, Y032H, Y032C, Y032M, Y032N, Y032T, Y032V, Y032L, G033M, G033S, G033T, V034A, V034C, V034I, V034M, V034P, H035I, H035Q, W036K, W036A, W036I, W036V, W036Y, V050K, V050H, V050A, V050D, V050G, V050T, I051K, I051H, I051E, I051N, I051Y, I051L, W052I, W052N, S053H, S053R, S053A, S053C, S053G, S053I, S053M, S053P, S053L, S053V, S053Y, G054H, G054R, G054A, G054C, G054D, G054P, G054S, G055R, G055M, G055S, G055Y, N056K, N056P, N056V, T057H, T057R, T057L, T057C, T057F, T057M, T057N, T057Q, T057W, T057Y, D058L, D058G, D058M, D058Q, Y059R, Y059D, Y059I, Y059T, Y059V, N060K, N060C, N060F, N060G, N060P, N060Q, N060S, N060T, N060Y, T061N, T061Q, P062G, F063H, F063R, F063A, F063C, F063D, F063G, F063M, F063N, F063Q, F063S, T064R, T064L, T064C, T064F, T064G, T064Q, T064V, S065H, S065R, S065L, S065C, S065E, S065F, S065I, S065M, S065N, S065P, S065Q, S065T, S065W, S065Y, R066L, R066A, R066C, R066E, R066F, R066N, R066P, R066Q, R066S, R066T, R066V, R066G, L067A, L067C, L067D, L067E, L067I, L067M, L067Q, L067S, L067T, L067Y, S068K, S068H, S068R, S068L, S068C, S068D, S068E, S068F, S068G, S068I, S068N, S068Q, S068V, I069A, I069C, I069G, I069Y, N070H, N070R, N070L, N070D, N070E, N070F, N070G, N070I, N070P, N070Q, N070V, N070Y, K071H, K071R, K071L, K071C, K071F, K071G, K071Q, K071S, K071T, K071W, K071Y, D072K, D072H, D072R, D072L, D072A, D072G, D072I, D072M, D072N, D072Q, D072S, D072V, D072W, D072Y, N073H, N073R, N073L, N073A, N073C, N073G, N073I, N073M, N073P, N073Q, N073S, N073V, N073W, N073Y, S074K, S074H, S074R, S074L, S074C, S074D, S074E, S074G, S074I, S074M, S074P, S074T, S074V, S074Y, K075H, K075R, K075L, K075A, K075C, K075E, K075F, K075M, K075Q, K075T, K075V, K075W, K075Y, S076H, S076R, S076L, S076A, S076C, S076D, S076E, S076F, S076M, S076P, S076Q, S076T, S076Y, Q077H, Q077R, Q077L, Q077A, Q077E, Q077G, Q077I, Q077M, Q077N, Q077V, Q077W, Q077Y, Y093H, Y093V, Y093W, Y094R, Y094L, R097H, R097W, A098P, L099N, L099W, T100H, T100L, T100I, T100N, T100P, T100Q, T100S, T100V, T100Y, Y101H, Y101E, Y101F, Y101M, Y102R, Y102D, Y102I, Y102N, Y102W, D103R, D103L, D103A, D103I, D103Q, D103Y, D103P, Y104H, Y104L, Y104D, Y104F, Y104I, Y104M, Y104S, Y104V, E105H, E105T, F106L, F106V, F106W, A107K, A107H, A107R, A107L, A107E, A107G, A107N, A107S, A107T, A107Y, A107D, Y108K, Y108H, Y108R, Y108L, Y108I, Y108N, Y108S, Y108T, Y108V, Y108W, W109I, W109M, W109Y, G110R, G110A, G110M, G110P, G110T, Q111K, Q111H, Q111R, Q111L, Q111D, Q111E, Q111G, Q111M, Q111P, Q111S, Q111T, Q111W, Q111Y, G112A, G112N, G112P, G112S, G112T, G112Y, V24E, S28C, F63P, L67D, D72P, K75G, K75P, S76I, S76V, Q111I and Q111V, with reference to amino acid positions set forth in SEQ ID NO:3, and corresponding amino acid positions are identified by alignment of the VH chain of the antibody with the VH chain set forth in SEQ ID NO:3; and the portion thereof is sufficient to form an antigen binding site and includes the amino acid replacement. In some examples, the VL chain, or portion thereof, includes an amino acid replacement(s) corresponding to amino acid replacement(s) selected from among D001W, I002V, I002W, L003D, L003F, L003G, L003S, L003W, L003Y, L003R, L004E, L004F, L004I, L004P, L004S, L004T, L004V, L004W, L004K, L004H, L004R, T005A, T005D, T005E, T005F, T005G, T005N, T005S, T005W, T005L, T005K, T005H, T005R, R024A, R024C, R024F, R024L, R024M, R024S, R024W, R024Y, A025G, S026A, S026C, S026I, S026M, S026N, S026V, S026W, S026L, S026G, S026H, S026R, Q027A, Q027D, Q027I, Q027M, Q027N, Q027P, S028A, S028D, S028N, S028Q, S028L, S028K, S028H, I029A, I029E, I029F, I029S, I029T, I029R, G030E, G030I, G030P, G030V, G030L, T031A, T031F, T031G, T031M, T031S, T031W, T031L, T031K, T031H, N032G, I033F, I033G, I033M, I033T, I033V, I033H, I048M, I048S, I048L, I048K, K049A, K049E, K049G, K049N, K049Q, K049S, K049T, K049V, K049L, K049H, K049R, A051T, A051L, S052A, S052C, S052D, S052E, S052G, S052I, S052M, S052Q, S052V, S052W, S052R, S052K, E053G, S054M, I055A, I055F, S056G, S056L, S056A, S056C, S056D, S056E, S056F, S056N, S056P, S056Q, S056V, S056W, S056H, S056R, S056K, Y086F, Y086M, Y086H, Y087L, Y087C, Y087D, Y087F, Y087G, Y087I, Y087N, Y087P, Y087T, Y087V, Y087W, Y087K, Y087H, Y087R, Q089E, N091A, N091I, N091M, N091S, , N091T, N091V, N091H, N091R, N092D, N092S, N092T, N092V, N092W, N092R, N093T, T096M, T096V, T097V, F098A, F098M, F098S, F098V, F098Y, G099L, G099D, G099E, G099F, G099I, G099M, G099N, G099S, G099T, G099V, G099K, G099H, Q100C, Q100D, Q100E, Q100F, Q100I, Q100M, Q100N, Q100P, Q100T, Q100V, Q100W, Q100Y, Q100K, Q100H and Q100R, with reference to amino acid positions set forth in SEQ ID NO:4, and corresponding amino acid positions are identified by alignment of the VL chain of the antibody with the VL chain set forth in SEQ ID NO:4; and the portion thereof is sufficient to form an antigen binding site and includes the amino acid replacement.

In any of the examples of the modified anti-EGFR antibodies, or antigen binding fragments thereof, provided herein, the variable heavy chain, or portion thereof, can include an amino acid replacement(s) selected from among V024I, V024E, V024L, S025C, S025G, S025I, S025Q, S025T, S025L, S025V, F027R, T030F, Y032T, S053G, G054R, G054C, G054P, D058M, F063R, F063C, F063G, F063M, D072K, D072M, D072W, D072L, S074H, S074R, S074D, S074G, S074Y, K075H, K075W, Q077R, N091V, R097H, T100I, Y104D, Y104F, F027R, L029S, R097H and Q111P; and/or the variable light chain, or portion thereof can include an amino acid replacement L4V or I29S.

In particular examples herein, the modified anti-EGFR antibodies, or antigen-binding fragments thereof, provided herein the variable heavy chain, or portion thereof, can include an amino acid replacement(s) selected from among V24E, V24I, V24L, S25C, S25H, S25R, S25A, S25D, S25G, S25M, S25Q, S25V, S25L, S28C, L29H, N31H, G54D, G54S, F63R, F63C, F63M, F63P, F63S, T64V, L67D, D72L, D72P, D72W, N73Q, K75H, K75G, K75P, K75W, S76I, S76V, Q77E, T100P, Y104D, Y104S, Y104V, Q111I, Q111V, and can further include an amino acid replacement(s) V24E, S25C, S25V, F27R, T30F, S53G, D72L, R97H, Y104D and Q111P. The modified anti-EGFR antibody, or antigen-binding fragment thereof, can contain 2, 3, 4, 5, 6, 7, 8, 9, 10, 11, 12, 13, 14, 15, 16, 17, 18, 19, 20 or more amino acid replacement(s) in the variable heavy chain, in the variable light chain or both. For example, the modified anti-EGFR antibody, or antigen-binding fragment thereof, contains at least two amino acid replacement(s) in cetuximab, an antigen-binding fragment thereof or a variant thereof that does not comprise the amino acid replacement and specifically binds to EGFR, where the amino acid replacements in the VH chain corresponds to an amino acid replacement selected from among V24E, S25C, S25V, F27R, T30F, S53G, D72L, R97H, Y104D and Q111P, with reference to amino acid positions set forth in SEQ ID NO:3, wherein corresponding amino acid positions are identified by alignment of the VH chain of the antibody with the VH chain set forth in SEQ ID NO:3; and the amino acid replacement in the VL chain corresponds to amino acid replacement I29S, with reference to the amino acid position set forth in SEQ ID NO:4; wherein corresponding amino acid positions are identified by alignment of the VL chain of the antibody with the VL chain set forth in SEQ ID NO:4. For example, that anti-EGFR antibody, or antigen-binding fragment thereof, contains the amino acid replacement(s) HC-Y104D/ HC-Q111P; HC-S25C/ HC-Y104D; HC-Y104D/LC-I29S; HC-Y104D/HC-Q111P/LC-I29S; HC-S53G/HC-Y104D; HC-S53G/HC-Y104D/HC-Q111P; HC-S25V/HC-Y104D; HC-S25V/HC-Y104D/HC-Q111P; HC-S25V/HC-S53G/HC-Y104D; HC-S25V/HC-S53G/HC-Y104D/HC-Q111P; HC-T30F/HC-Y104D; HC-T30F/HC-Y104D/HC-Q111P; HC-T30F/HC-S53G/HC-Y104D; HC-T30F/HC-S53G/HC-Y104D/HC-Q111P; HC-D72L/HC-Y104D; HC-D72L/HC-Y104D/HC-Q111P; HC-S53G/ HC-D72L/HC-Y104D; HC-S53G/HC-D72L/HC-Y104D/HC-Q111P; HC-S25C/ HC-Q111P; HC-V24E/ HC-F27R/ HC-R97H/ HC-Q111P; HC-S25C/LC-I29S; or HC-Q111P/LC-I29S. In any of such examples, the modified anti-EGFR antibody or fragment thereof exhibits a ratio of binding activity for EGFR at or about pH 6.0 to pH 6.5 compared to at or about pH 7.4 of at least 1.1, 1.2, 1.3, 1.4, 1.5, 1.6, 1.7, 1.8, 1.9, 2.0, 2.5, 3.0, 4.0, 4.5, 5.0 or greater.

In particular examples, the modified anti-EGFR antibody or fragment thereof exhibits a ratio of binding activity for EGFR at or about pH 6.0 to pH 6.5 compared to at or about pH 7.4 of at least 2.0, and generally at least 3.0 or higher as described herein. In such examples, the anti-EGFR antibody, or antigen-binding fragment thereof, includes the amino acid replacement Y104D. For example, the amino acid replacements are HC-Y104D/ HC-Q111P; HC-S25C/ HC-Y104D; HC-S53G/HC-Y104D; HC-S53G/HC-Y104D/HC-Q111P; HC-S25V/HC-Y104D; HC-S25V/HC-Y104D/HC-Q111P; HC-S25V/HC-S53G/HC-Y104D; HC-S25V/HC-S53G/HC-Y104D/HC-Q111P; HC-T30F/HC-Y104D; HC-T30F/HC-Y104D/HC-Q111P; HC-T30F/HC-S53G/HC-Y104D; HC-T30F/HC-S53G/HC-Y104D/HC-Q111P; HC-D72L/HC-Y104D; HC-D72L/HC-Y104D/HC-Q111P; HC-S53G/ HC-D72L/HC-Y104D; or HC-S53G/HC-D72L/HC-Y104D/HC-Q111P.

In any of the examples of the modified anti-EGFR antibodies, or antigen-binding fragments thereof, provided herein, the unmodified cetuximab antibody, antigen-binding fragment thereof or variant thereof includes: a) a heavy chain having a sequence of amino acids set forth in SEQ ID NO: 1 or a sequence of amino acids that exhibits at least 75% sequence identity to the sequence of amino acids set forth in SEQ ID NO:1 and a light chain having a sequence of amino acids set forth SEQ ID NO:2 or a sequence of amino acids that exhibits at least 75% sequence identity to the sequence of amino acids set forth in SEQ ID NO:2; or b) a heavy chain having a having a sequence of amino acids set forth in SEQ ID NO: 8 or a sequence of amino acids that exhibits at least 75% sequence identity to the sequence of amino acids set forth in SEQ ID NO:8 and a light chain having a sequence of amino acids set forth SEQ ID NO:9 or a sequence of amino acids that exhibits at least 75% sequence identity to the sequence of amino acids set forth in SEQ ID NO:9.

In any of the examples provided herein, the modified anti-EGFR antibodies, or antigen-binding fragments thereof, provided herein include those in which the unmodified cetuximab is a variant that is humanized. For example, in any of the examples of the modified anti-EGFR antibodies, or antigen-binding fragments thereof, provided herein, the unmodified cetuximab includes a variable heavy chain set forth in SEQ ID NO:28 and a variable light chain set forth in SEQ ID NO:29.

In any of the examples of conditionally active anti-EGFR antibodies, or antigen-binding fragments, provided herein, the antibody is a full-length antibody or is an antigen-binding fragment. For example, the antigen-binding fragment is selected from among a Fab, Fab', F(ab')₂, single-chain Fv (scFv), Fv, dsFv, diabody, Fd and Fd' fragments.

In any of the examples of the modified anti-EGFR antibodies, or antigen-binding fragments thereof, provided herein, the unmodified cetuximab, antigen-binding fragment thereof or variant thereof is an antigen-binding fragment thereof and the antigen-binding fragment is selected from among a Fab, Fab', F(ab')₂, single-chain Fv (scFv), Fv, dsFv, diabody, Fd and Fd' fragments. For example, the unmodified cetuximab can be a Fab fragment that includes a heavy chain having a sequence of amino acids set forth in SEQ ID NO:5 or a sequence of amino acids that exhibits at least 75% sequence identity to SEQ ID NO:5 and a light chain having a sequence of amino acids set forth in SEQ ID NO:2 or a sequence of amino acids that exhibits at least 75% sequence identity to a sequence of amino acids set forth in SEQ ID NO:2.

Provided herein are modified anti-EGFR antibodies, or antigen-binding fragments thereof, that include: a) a variable heavy (VH) chain set forth in any of SEQ ID NOS: 31-32, 35-57, 59-85, 87-112, 114-125, 127-131, 133-134, 138-139, 141-149, 148-168, 170, 174, 176-177, 180, 182, 186-189, 191-198, 200, 202-205, 207-210, 212-216, 218, 220-224, 226, 228-236, 238-240, 243, 246, 250-251, 253, 255, 257-268, 270-277, 279-283, 285-292, 294-322, 324-336, 338-352, 355-359, 361-366, 368-394, 396-402, 404-448, 450-465, 467-477, 479, 481-483, 485-487, 489-505, 507-510, 512-523, 525-557, 1062, 1063, 1093, 1098-1107 and 1112-1113 or a sequence of amino acids that exhibits at least 75% sequence identity to any of SEQ ID NOS: 31-32, 35-57, 59-85, 87-112, 114-125, 127-131, 133-134, 138-139, 141-149, 148-168, 170, 174, 176-177, 180, 182, 186-189, 191-198, 200, 202-205, 207-210, 212-216, 218, 220-224, 226, 228-236, 238-240, 243, 246, 250-251, 253, 255, 257-268, 270-277, 279-283, 285-292, 294-322, 324-336, 338-352, 355-359, 361-366, 368-394, 396-402, 404-448, 450-465, 467-477, 479, 481-483, 485-487, 489-505, 507-510, 512-523, 525-557, 1062, 1063, 1093, 1098-1107 and 1112-1113 and includes the amino acid replacement; and/or b) a variable (VL) chain set forth in any of SEQ ID NOS: 558, 560-565, 568-570, 572-583, 585-603, 605, 608-609, 611-621, 624-627, 629-641, 643, 645, 647, 649-650, 652, 656-660, 662-678, 680-685, 687-733, 735-741, 743, 745-751, 753, 756-759, 764-768, 775, 778-809, 810, 812-817, 820-822, 824-835, 837-855, 857, 860-861, 863-873, 876-879, 881-893, 895, 897, 899, 901-902, 904, 908-912, 914-930, 932-937, 939-985, 987-993, 995, 997-1003, 1005, 1008-1011, 1016-1020, 1027, 1030-1061, or a sequence of amino acids that exhibits at least 75% sequence identity to any of SEQ ID NOS: 558, 560-565, 568-570, 572-583, 585-603, 605, 608-609, 611-621, 624-627, 629-641, 643, 645, 647, 649-650, 652, 656-660, 662-678, 680-685, 687-733, 735-741, 743, 745-751, 753, 756-759, 764-768, 775, 778-809, 810, 812-817, 820-822, 824-835, 837-855, 857, 860-861, 863-873, 876-879, 881-893, 895, 897, 899, 901-902, 904, 908-912, 914-930, 932-937, 939-985, 987-993, 995, 997-1003, 1005, 1008-1011, 1016-1020, 1027, 1030-1061 and includes the amino acid replacement.

In some examples, the modified anti-EGFR antibodies, or antigen-binding fragments thereof, include: a) a variable heavy (VH) chain set forth in any of SEQ ID NOS: 31-32, 35-57, 59-85, 87-112, 114-125, 127-131, 133-134, 138-139, 141-149, 148-168, 170, 174, 176-177, 180, 182, 186-189, 191-198, 200, 202-205, 207-210, 212-216, 218, 220-224, 226, 228-236, 238-240, 243, 246, 250-251, 253, 255, 257-268, 270-277, 279-283, 285-292, 294-322, 324-336, 338-352, 355-359, 361-366, 368-394, 396-402, 404-448, 450-465, 467-477, 479, 481-483, 485-487, 489-505, 507-510, 512-523, 525-557, 1062, 1063, 1093, 1098-1107 and 1112-1113 or a sequence of amino acids that exhibits at least 75% sequence identity to any of SEQ ID NOS: 31-32, 35-57, 59-85, 87-112, 114-125, 127-131, 133-134, 138-139, 141-149, 148-168, 170, 174, 176-177, 180, 182, 186-189, 191-198, 200, 202-205, 207-210, 212-216, 218, 220-224, 226, 228-236, 238-240, 243, 246, 250-251, 253, 255, 257-268, 270-277, 279-283, 285-292, 294-322, 324-336, 338-352, 355-359, 361-366, 368-394, 396-402, 404-448, 450-465, 467-477, 479, 481-483, 485-487, 489-505, 507-510, 512-523, 525-557, 1062, 1063, 1093, 1098-1107 and 1112-1113 and includes the amino acid replacement; and b) a variable light (VL) chain set forth in SEQ ID NO:4 or SEQ ID NO:10, or a sequence of amino acids that exhibits at least 75% sequence identity to SEQ ID NO:4 or SEQ ID NO:10.

In some examples, the modified anti-EGFR antibodies, or antigen-binding fragments thereof, include: a) a variable heavy (VH) chain set forth in SEQ ID NO:3 or a sequence of amino acids that exhibits at least 75% sequence identity to SEQ ID NO:3; and b) a variable light chain (VL) set forth in any of SEQ ID NOS: 558, 560-565, 568-570, 572-583, 585-603, 605, 608-609, 611-621, 624-627, 629-641, 643, 645, 647, 649-650, 652, 656-660, 662-678, 680-685, 687-733, 735-741, 743, 745-751, 753, 756-759, 764-768, 775, 778-809, 810, 812-817, 820-822, 824-835, 837-855, 857, 860-861, 863-873, 876-879, 881-893, 895, 897, 899, 901-902, 904, 908-912, 914-930, 932-937, 939-985, 987-993, 995, 997-1003, 1005, 1008-1011, 1016-1020, 1027, 1030-1061, or a sequence of amino acids that exhibits at least 75% sequence identity to any of SEQ ID NOS: 558, 560-565, 568-570, 572-583, 585-603, 605, 608-609, 611-621, 624-627, 629-641, 643, 645, 647, 649-650, 652, 656-660, 662-678, 680-685, 687-733, 735-741, 743, 745-751, 753, 756-759, 764-768, 775, 778-809, 810, 812-817, 820-822, 824-835, 837-855, 857, 860-861, 863-873, 876-879, 881-893, 895, 897, 899, 901-902, 904, 908-912, 914-930, 932-937, 939-985, 987-993, 995, 997-1003, 1005, 1008-1011, 1016-1020, 1027, 1030-1061 and includes the amino acid replacement.

In particular examples herein of a conditionally active anti-EGFR antibodies, including modified anti-EGFR antibodies, containing an amino acid replacement corresponding to Y104D in the heavy chain and exhibiting a ratio of binding activity of at least 2.0 as described herein, the modified anti-EGFR antibody, or antigen-binding fragment thereof, contains: a variable heavy (VH) chain having the sequence of amino acids set forth in SEQ ID NO:495, 1062, 1112, 1114, 1115, 1116, 1117, 1118, 1119, 1124, 1125, 1126, 1127, 1128, 1129, 1130 or 1131, or a sequence of amino acids that exhibits at least 85% sequence identity to any of SEQ ID NOS: 495, 1062, 1112, 1114, 1115, 1116, 1117, 1118, 1119, 1124, 1125, 1126, 1127, 1128, 1129, 1130 or 1131; and a variable light (VL) chain comprising the sequence of amino acids set forth in SEQ ID NO:4 or 10, or a sequence of amino acids that exhibits at least 85% sequence identity to SEQ ID NO:4 or 10. For example, the modified anti-EGFR antibody, or antigen-binding fragment thereof, contains a variable heavy (VH) chain containing the sequence of amino acids set forth in SEQ ID NO:1062 or 1125, or a sequence of amino acids that exhibits at least 85% sequence identity to any of SEQ ID NOS: 1062 or 1125; and a variable light (VL) chain comprising the sequence of amino acids set forth in SEQ ID NO:4 or 10, or a sequence of amino acids that exhibits at least 85% sequence identity to SEQ ID NO:4 or 10.

In any of the examples herein, the anti-EGFR, or antigen-binding fragment thereof, is humanized. Typically, in such examples, the anti-EGFR, or antigen-binding fragment thereof, retains the conditional activity and exhibits a ratio of activity in a tumor microenvironment compared to a non-tumor microenvironment of at least 2.0, and generally at least 3.0 or higher. In some cases of a humanized antibody provided herein, the variable heavy chain exhibits less than 85% sequence identity to the variable heavy chain set forth in SEQ ID NO:3 and greater than 65% sequence identity to the variable heavy chain set forth in SEQ ID NO:3; and the variable light chain exhibits less than 85% sequence identity to the variable light chain set forth in SEQ ID NO:4 and greater than 65% sequence identity to the variable light chain set forth in SEQ ID NO:4. Exemplary of such antibodies are any that contain the sequence of amino acids of:

a) the variable heavy chain set forth in SEQ ID NO:1134 or a sequence of amino acids that exhibits at least 85% sequence identity to SEQ ID NO:1134, and the variable light chain set forth in SEQ ID NO:1138 or a sequence of amino acids that exhibits at least 85% sequence identity to SEQ ID NO:1138;
b) the variable heavy chain set forth in SEQ ID NO:1134 or a sequence of amino acids that exhibits at least 85% sequence identity to SEQ ID NO:1134, and the variable light chain set forth in SEQ ID NO:1139 or a sequence of amino acids that exhibits at least 85% sequence identity to SEQ ID NO:1139;

c) the variable heavy chain set forth in SEQ ID NO:1135 or a sequence of amino acids that exhibits at least 85% sequence identity to SEQ ID NO:1135, and the variable light chain set forth in SEQ ID NO:1138 or a sequence of amino acids that exhibits at least 85% sequence identity to SEQ ID NO:1138;

d) the variable heavy chain set forth in SEQ ID NO:1134 or a sequence of amino acids that exhibits at least 85% sequence identity to SEQ ID NO:1134, and the variable light chain set forth in SEQ ID NO:1140 or a sequence of amino acids that exhibits at least 85% sequence identity to SEQ ID NO:1140;

e) the variable heavy chain set forth in SEQ ID NO:1134 or a sequence of amino acids that exhibits at least 85% sequence identity to SEQ ID NO:1134, and the variable light chain set forth in SEQ ID NO:1141 or a sequence of amino acids that exhibits at least 85% sequence identity to SEQ ID NO:1141;

f) the variable heavy chain set forth in SEQ ID NO:1134 or a sequence of amino acids that exhibits at least 85% sequence identity to SEQ ID NO:1134, and the variable light chain set forth in SEQ ID NO:1142 or a sequence of amino acids that exhibits at least 85% sequence identity to SEQ ID NO:1142;

g) the variable heavy chain set forth in SEQ ID NO:1135 or a sequence of amino acids that exhibits at least 85% sequence identity to SEQ ID NO:1135, and the variable light chain set forth in SEQ ID NO:1142 or a sequence of amino acids that exhibits at least 85% sequence identity to SEQ ID NO:1142;

h) the variable heavy chain set forth in SEQ ID NO:1134 or a sequence of amino acids that exhibits at least 85% sequence identity to SEQ ID NO:1134, and the variable light chain set forth in SEQ ID NO:1143 or a sequence of amino acids that exhibits at least 85% sequence identity to SEQ ID NO:1143;

i) the variable heavy chain set forth in SEQ ID NO:1136 or a sequence of amino acids that exhibits at least 85% sequence identity to SEQ ID NO:1136, and the variable light chain set forth in SEQ ID NO:1142 or a sequence of amino acids that exhibits at least 85% sequence identity to SEQ ID NO:1142;

j) the variable heavy chain set forth in SEQ ID NO:1137 or a sequence of amino acids that exhibits at least 85% sequence identity to SEQ ID NO:1137, and the variable light chain set forth in SEQ ID NO:1144 or a sequence of amino acids that exhibits at least 85% sequence identity to SEQ ID NO:1144;

k) the variable heavy chain set forth in SEQ ID NO:1136 or a sequence of amino acids that exhibits at least 85% sequence identity to SEQ ID NO:1136, and the variable light chain set forth in SEQ ID NO:1144 or a sequence of amino acids that exhibits at least 85% sequence identity to SEQ ID NO:1144;

1) the variable heavy chain set forth in SEQ ID NO:1137 or a sequence of amino acids that exhibits at least 85% sequence identity to SEQ ID NO:1137, and the variable light chain set forth in SEQ ID NO:1145 or a sequence of amino acids that exhibits at least 85% sequence identity to SEQ ID NO:1145;

m) the variable heavy chain set forth in SEQ ID NO:1136 or a sequence of amino acids that exhibits at least 85% sequence identity to SEQ ID NO:1136, and the variable light chain set forth in SEQ ID NO:1145 or a sequence of amino acids that exhibits at least 85% sequence identity to SEQ ID NO:1145;

n) the variable heavy chain set forth in SEQ ID NO:1146 or a sequence of amino acids that exhibits at least 85% sequence identity to SEQ ID NO:1146, and the variable light chain set forth in SEQ ID NO:1153 or a sequence of amino acids that exhibits at least 85% sequence identity to SEQ ID NO:1153;

o) the variable heavy chain set forth in SEQ ID NO:1147 or a sequence of amino acids that exhibits at least 85% sequence identity to SEQ ID NO:1147, and the variable light chain set forth in SEQ ID NO:1153 or a sequence of amino acids that exhibits at least 85% sequence identity to SEQ ID NO:1153;

p) the variable heavy chain set forth in SEQ ID NO:1148 or a sequence of amino acids that exhibits at least 85% sequence identity to SEQ ID NO:1148, and the variable light chain set forth in SEQ ID NO:1154 or a sequence of amino acids that exhibits at least 85% sequence identity to SEQ ID NO:1154;

q) the variable heavy chain set forth in SEQ ID NO:1149 or a sequence of amino acids that exhibits at least 85% sequence identity to SEQ ID NO:1149, and the variable light chain set forth in SEQ ID NO:1154 or a sequence of amino acids that exhibits at least 85% sequence identity to SEQ ID NO:1154;

r) the variable heavy chain set forth in SEQ ID NO:1150 or a sequence of amino acids that exhibits at least 85% sequence identity to SEQ ID NO:1150, and the variable light chain set forth in SEQ ID NO:1155 or a sequence of amino acids that exhibits at least 85% sequence identity to SEQ ID NO:1155;

s) the variable heavy chain set forth in SEQ ID NO:1151 or a sequence of amino acids that exhibits at least 85% sequence identity to SEQ ID NO:1151, and the variable light chain set forth in SEQ ID NO:1156 or a sequence of amino acids that exhibits at least 85% sequence identity to SEQ ID NO:1156;

t) the variable heavy chain set forth in SEQ ID NO:1146 or 1148 or a sequence of amino acids that exhibits at least 85% sequence identity to SEQ ID NO:1146 or 1148, and the variable light chain set forth in SEQ ID NO:1156 or a sequence of amino acids that exhibits at least 85% sequence identity to SEQ ID NO:1156;

u) the variable heavy chain set forth in SEQ ID NO:1149 or a sequence of amino acids that exhibits at least 85% sequence identity to SEQ ID NO:1149, and the variable light chain set forth in SEQ ID NO:1156 or a sequence of amino acids that exhibits at least 85% sequence identity to SEQ ID NO:1156;

v) the variable heavy chain set forth in SEQ ID NO:1150 or a sequence of amino acids that exhibits at least 85% sequence identity to SEQ ID NO:1150, and the variable light chain set forth in SEQ ID NO:1157 or a sequence of amino acids that exhibits at least 85% sequence identity to SEQ ID NO:1157;

w) the variable heavy chain set forth in SEQ ID NO:1152 or a sequence of amino acids that exhibits at least 85% sequence identity to SEQ ID NO:1152, and the variable light chain set forth in SEQ ID NO:1157 or a sequence of amino acids that exhibits at least 85% sequence identity to SEQ ID NO:1157;

x) the variable heavy chain set forth in SEQ ID NO:1148 or a sequence of amino acids that exhibits at least 85% sequence identity to SEQ ID NO:1148, and the variable light chain set forth in SEQ ID NO:1157 or a sequence of amino acids that exhibits at least 85% sequence identity to SEQ ID NO:1157;

y) the variable heavy chain set forth in SEQ ID NO:1149 or a sequence of amino acids that exhibits at least 85% sequence identity to SEQ ID NO:1149, and the variable light chain set forth in SEQ ID NO:1157 or a sequence of amino acids that exhibits at least 85% sequence identity to SEQ ID NO:1157;

z) the variable heavy chain set forth in SEQ ID NO:1150 or a sequence of amino acids that exhibits at least 85% sequence identity to SEQ ID NO:1150, and the variable light chain set forth in SEQ ID NO:1186 or a sequence of amino acids that exhibits at least 85% sequence identity to SEQ ID NO:1186;

aa) the variable heavy chain set forth in SEQ ID NO:1152 or a sequence of amino acids that exhibits at least 85% sequence identity to SEQ ID NO:1152, and the variable light chain set forth in SEQ ID NO:1186 or a sequence of amino acids that exhibits at least 85% sequence identity to SEQ ID NO:1186;

bb) the variable heavy chain set forth in SEQ ID NO:1148 or a sequence of amino acids that exhibits at least 85% sequence identity to SEQ ID NO:1148, and the variable light chain set forth in SEQ ID NO:1186 or a sequence of amino acids that exhibits at least 85% sequence identity to SEQ ID NO:1186;

cc) the variable heavy chain set forth in SEQ ID NO:1149 or a sequence of amino acids that exhibits at least 85% sequence identity to SEQ ID NO:1149, and the variable light chain set forth in SEQ ID NO:1186 or a sequence of amino acids that exhibits at least 85% sequence identity to SEQ ID NO:1186;

dd) the variable heavy chain set forth in SEQ ID NO:1150 or a sequence of amino acids that exhibits at least 85% sequence identity to SEQ ID NO:1150, and the variable light chain set forth in SEQ ID NO:1158 or a sequence of amino acids that exhibits at least 85% sequence identity to SEQ ID NO:1158;

ee) the variable heavy chain set forth in SEQ ID NO:1152 or a sequence of amino acids that exhibits at least 85% sequence identity to SEQ ID NO:1152, and the variable light chain set forth in SEQ ID NO:1159 or a sequence of amino acids that exhibits at least 85% sequence identity to SEQ ID NO:1159;

ff) the variable heavy chain set forth in SEQ ID NO:1146 or a sequence of amino acids that exhibits at least 85% sequence identity to SEQ ID NO:1146, and the variable light chain set forth in SEQ ID NO:1159 or a sequence of amino acids that exhibits at least 85% sequence identity to SEQ ID NO:1159;

gg) the variable heavy chain set forth in SEQ ID NO:1146 or a sequence of amino acids that exhibits at least 85% sequence identity to SEQ ID NO:1146, and the variable light chain set forth in SEQ ID NO:1157 or a sequence of amino acids that exhibits at least 85% sequence identity to SEQ ID NO:1157; and

hh) the variable heavy chain set forth in SEQ ID NO:1146 or a sequence of amino acids that exhibits at least 85% sequence identity to SEQ ID NO:1146, and the variable light chain set forth in SEQ ID NO:1186 or a sequence of amino acids that exhibits at least 85% sequence identity to SEQ ID NO:1186.

In any of the examples provided herein, the conditionally active anti-EGFR antibodies, including modified anti-EGFR antibodies, are full-length IgG antibodies. For example, the conditionally active anti-EGFR antibodies, including modified anti-EGFR antibody, can include a heavy chain constant region set forth in any of SEQ ID NOS:22-25, 1069 and 1070, or a variant thereof that exhibits at least 75% sequence identity thereto; and a light chain constant region set forth in any of SEQ ID NOS: 1072-1073, or a variant thereof that exhibits at least 75% sequence identity thereto.

In any of the examples of the conditionally active anti-EGFR antibodies provided herein, including modified anti-EGFR antibodies and antigen-binding fragments provided herein, the antigen-binding fragment can be selected from among a Fab, Fab', F(ab')₂, single-chain Fv (scFv), Fv, dsFv, diabody, Fd and Fd' fragments. In some examples, the conditionally active anti-EGFR antibody, such as a modified anti-EGFR antibody or antigen-binding fragment, is a Fab or scFv.

In any of the examples herein, a sequence of amino acids provided herein that exhibits sequence identity to a reference sequence or SEQ ID NO, such as, for example, a sequence of amino acids in a modified anti-EGFR antibody or an unmodified cetuximab, exhibits at least 80%, 81%, 82%, 83%, 84%, 85%, 86%, 87%, 88%, 89%, 90%, 91%, 92%, 93%, 94%, 95%, 96%, 97%, 98%, or 99% sequence identity thereto. Sequence identity can be determined using global alignment with or without gaps.

In any of the examples of conditionally active anti-EGFR antibodies provided herein, including a modified anti-EGFR antibody, or antigen binding fragment, the antibody or antigen-binding fragment also can include an amino acid replacement selected from among: a) an amino acid replacement(s) in the variable heavy chain corresponding to an amino acid replacement(s) selected from among replacement of Glutamine (Q) at position 1 with Glutamic acid (E), Q1C, V2C, Q3T, Q3C, L4C, K5Q, K5V, K5L, K5C, Q6E, Q6C, S7C, G8C, P9A, P9G, P9C, G10V, G10C, L11C, V12C, Q13K, Q13R, Q13C, P14C, S15G, S15T, S15C, Q16G, Q16R, Q16E, Q16C, S17T, S17C, L18C, S19K, S19R, S19T, S19C, I20L, I20C, T21S, T21C, T23A, T23K, T23C, V24A, V24C, S25C, F27G, S28N, S28T, L29I, T30S, T30K, N31V, N31D, N31I, N31T, N32S, Y32R, Y32W, G33A, G33D, G33E, G33Y, V34L, V34N, V34E, V34Q, V34S, V34W, H35S, V37I, S40A, S40P, P41T, G44A, L48V, L48I, G49S, G49A, V50L, V50Q, V50E, V50I, V50Y, V50N, I51G, I51M, I51S, I51Q, I51A, 151C, I51V, W52F, W52Y, W52G, W52T, S53Q, S53T, S53N, S53Y, G54A, G54V, G54L, G54I, G54S, G55D, G55A, G55E, G55H, G55F, N56A, N56G, N56S, N56T, T57A, T57D, T57G, T57S, T57E, T57P, D58Y, D58N, Y59A, Y59C, Y59E, Y59F, Y59G, Y59S, Y59W, T59H, Y59P, Y59Q, N60D, N60A, T61E, T61P, P62S, F63L, F63V, T64K, T64E, T64A, T64N, T64D, S65G, L67F, L67V, S68T, N70S, N70T, K71V, D72E, N73T, S74A, S76N, Q77T, Q77S, V78L, V78F, V78A, F79Y, F79S, F79V, F80L, F80M, K81Q, K81T, K81E, K81Q, M82L, N83T, N83S, S84N, L85M, L85V, Q86R, Q86D, Q86T, S87A, S87P, N88E, N88V, N88G, N88A, N88D, I92T, I92V, A96C, R97C, A98C, L99C, L99E, T100D, T100C, T100A, Y101C, Y101W, Y101A, Y102C, Y102F, Y102A, Y102W, D103E, D103P, D103C, Y104C, E105C, E105N, E105D, E105Y, F106C, F106D, F106Y, A107C, A107D, Y108C and Y108F, with reference to amino acid positions set forth in SEQ ID NO:1 or 3, and corresponding amino acid positions are identified by alignment of the VH chain of the antibody with the VH chain set forth in SEQ ID NO:3; and/or b) an amino acid replacement(s) in the variable light chain corresponding to an amino acid replacement selected from among replacement of Aspartate (D) at position 1 with Glutamate (E), D1C, I2T, I2C, L3V, L3T, L3C, L4C, T5C, Q6C, S7C, P8C, V9C, V9A, V9D, V9G, V9P, V9S, I10T, I10S, I10F, I10C, L11Q, L11C, S12A, S12C, V13L, V13M, V13S, V13A, V13C, S14T, S14C, P15V, P15L, P15C, G16K, G16C, E17D, E17K, E17C, R18V, R18K, R18C, V19A, V19T, V19C, S20T, S20C, S20A, F21I, F21L, F21C, S22T, S22C, R24P, A25V, A25S, A25I, A25P, A25T, A25Y, A25C, A25F, A25M, A25L, A25W, S26D, Q27W, Q27E, Q27F, Q27Y, Q27T, Q27H, S28R, S28F, G30Y, G30C, G30H, G30K, G30Q, G30R, G30W, G30F, G30T, G30M, G30S, G30A, T31E, T31V, T31D, T31R, N32H, I33L, H34C, Q38K, R39K, T40P, T40S, N41G, N41D, G42Q, G42K, G42E, S43A, S43P, R45K, K49Y, K49F, Y50G, S53V, S60D, S60A, G64S, G64A, D70E, D70V, F71Y, S74T, N76S, N76T, S77R, S77G, V78L, E79Q, S80P, S80A, E81A, I83F, I83S, I83V, I83A, D85V, D85T, D85I, D85M, Y87S, Q89C, Q89H, Q90C, N91C, N91Q, N91L, N92C, N92L, N92R N92K, N92M, N92Y, N92H, N92E, N92F, N93A, N93D, N93E, N93V, N93K, N93C, W94F, W94Y, P95C, T96C, T96L, T96E, T97C, T97A, T97D, T97E, T97P, T97K, T97N, T97Q, T97I, T97G, T97L, T97H, T97R, T97S, G99A, A100G, A100Q, K103T, L104V and L106I, with reference to amino acid positions set forth in SEQ ID NO:2 or 4, and corresponding amino acid positions are identified by alignment of the VL chain of the antibody with the VL chain set forth in SEQ ID NO:4; and/or c) amino acid replacements in the heavy chain constant regions selected from among replacement of Proline (P) at position 230 with Alanine (A), E233D, L234D, L234E, L234N, L234Q, L234T, L234H, L234Y, L234I, L234V, L234F, L235D, L235S, L235N, L235Q, L235T, L235H, L235Y, L235I, L235V, L235F, S239D, S239E, S239N, S239Q, S239F, S239T, S239H, S239Y, V240I, V240A, V240T, V240M, F241W, F241L, F241Y, F241E, F241R, F243W, F243L F243Y, F243R, F243Q, P244H, P245A, P247V, P247G, V262I, V262A, V262T, V262E, V263I, V263A, V263T, V263M, V264L, V264I, V264W, V264T, V264R, V264F, V264M, V264Y, V264E, D265G, D265N, D265Q, D265Y, D265F, D265V, D265I, D265L, D265H, D265T, V266I, V266A, V266T, V266M, S267Q, S267L, S267T, S267H, S267D, S267N, E269H, E269Y, E269F, E269R, E269T, E269L, E269N, D270Q, D270T, D270H, E272S, E272K, E272I, E272Y, V273I, K274T, K274E, K274R, K274L, K274Y, F275W, N276S, N276E, N276R, N276L, N276Y, Y278T, Y278E, Y278K, Y278W, E283R, Y296E, Y296Q, Y296D, Y296N, Y296S, Y296T, Y296L, Y296I, Y296H, N297S, N297D, N297E, S298H, T299I, T299L, T299A, T299S, T299V, T299H, T299F, T299E, V302I, W313F, E318R, K320T, K320D, K320I, K322T, K322H, V323I, S324T, S324D, S324R, S324I, S324V, S324L, S324Y, N325Q, N325L, N325I, N325D, N325E, N325A, N325T, N325V, N325H, K326L, K326I, K326T, A327N, A327L, A327D, A327T, L328M, L328D, L328E, L328N, L328Q, L328F, L328I, L328V, L328T, L328H, L328A, P329F, A330L, A330Y, A330V, A330I, A330F, A330R, A330H, A330S, A330W, A330M, P331V, P331H, I332D, I332E, I332N, I332Q, I332T, I332H, I332Y, I332A, E333T, E333H, E333I, E333Y, K334I, K334T, K334F, T335D, T335R, T335Y, D221K, D221Y, K222E, K222Y, T223E, T223K, H224E, H224Y, T225E, T225E, T225K, T225W, P227E, P227K, P227Y, P227G, P228E, P228K, P228Y, P228G, P230E, P230Y, P230G, A231E, A231K, A231Y, A231P, A231G, P232E, P232K, P232Y, P232G, E233N, E233Q, E233K, E233R, E233S, E233T, E233H, E233A, E233V, E233L, E233I, E233F, E233M, E233Y, E233W, E233G, L234K, L234R, L234S, L234A, L234M, L234W, L234P, L234G, L235E, L235K, L235R, L235A, L235M, L235W, L235P, L235G, G236D, G236E, G236N, G236Q, G236K, G236R, G236S, G236T, G236H, G236A, G236V, G236L, G236I, G236F, G236M, G236Y, G236W, G236P, G237D, G237E, G237N, G237Q, G237K, G237R, G237S, G237T, G237H, G237V, G237L, G237I, G237F, G237M, G237Y, G237W, G237P, P238D, P238E, P238N, P238Q, P238K, P238R, P238S, P238T, P238H, P238V, P238L, P238I, P238F, P238M, P238Y, P238W, P238G, S239Q, S239K, S239R, S239V, S239L, S239I, S239M, S239W, S239P, S239G, F241D, F241E, F241Y, F243E, K246D, K246E, K246H, K246Y, D249Q, D249H, D249Y, R255E, R255Y, E258S, E258H, E258Y, T260D, T260E, T260H, T260Y, V262E, V262F, V264D, V264E, V264N, V264Q, V264K, V264R, V264S, V264H, V264W, V264P, V264G, D265Q, D265K, D265R, D265S, D265T, D265H, D265V, D265L, D265I, D265F, D265M, D265Y, D265W, D265P, S267E, S267Q, S267K, S267R, S267V, S267L, S267I, S267F, S267M, S267Y, S267W, S267P, H268D, H268E, H268Q, H268K, H268R, H268T, H268V, H268L, H268I, H268F, H268M, H268W, H268P, H268G, E269K, E269S, E269V, E269I, E269M, E269W, E269P, E269G, D270R, D270S, D270L, D270I, D270F, D270M, D270Y, D270W, D270P, D270G, P271D, P271E, P271N, P271Q, P271K, P271R, P271S, P271T, P271H, P271A, P271V, P271L, P271I, P271F, P271M, P271Y, P271W, P271G, E272D, E272R, E272T, E272H, E272V, E272L, E272F, E272M, E272W, E272P, E272G, K274D, K274N, K274S, K274H, K274V, K274I, K274F, K274M, K274W, K274P, K274G, F275L, N276D, N276T, N276H, N276V, N276I, N276F, N276M, N276W, N276P, N276G, Y278D, Y278N, Y278Q, Y278R, Y278S, Y278H, Y278V, Y278L, Y278I, Y278M, Y278P, Y278G, D280K, D280L, D280W, D280P, D280G, G281D, G281K, G281Y, G281P, V282E, V282K, V282Y, V282P, V282G, E283K, E283H, E283L, E283Y, E283P, E283G, V284E, V284N, V284T, V284L, V284Y, H285D, H285E, H285Q, H285K, H285Y, H285W, N286E, N286Y, N286P, N286G, K288D, K288E, K288Y, K290D, K290N, K290H, K290L, K290W, P291D, P291E, P291Q, P291T, P291H, P291I, P291G, R292D, R292E, R292T, R292Y, E293N, E293R, E293S, E293T, E293H, E293V, E293L, E293I, E293F, E293M, E293Y, E293W, E293P, E293G, E294K, E294R, E294S, E294T, E294H, E294V, E294L, E294I, E294F, E294M, E294Y, E294W, E294P, E294G, Q295D, Q295E, Q295N, Q295R, Q295S, Q295T, Q295H, Q295V, Q295I, Q295F, Q295M, Q295Y, Q295W, Q295P, Q295G, Y296K, Y296R, Y296A, Y296V, Y296M, Y296G, N297Q, N297K, N297R, N297T, N297H, N297V, N297L, N297I, N297F, N297M, N297Y, N297W, N297P, N297G, S298D, S298E, S298Q, S298K, S298R, S298I, S298F, S298M, S298Y, S298W, T299D, T299E, T299N, T299Q, T299K, T299R, T299L, T299F, T299M, T299Y, T299W, T299P, T299G, Y300D, Y300E, Y300N, Y300Q, Y300K, Y300R, Y300S, Y300T, Y300H, Y300A, Y300V, Y300M, Y300W, Y300P, Y300G, R301D, R301E, R301H, R301Y, V303D, V303E, V303Y, S304D, S304N, S304T, S304H, S304L, V305E, V305T, V305Y, K317E, K317Q, E318Q, E318H, E318L, E318Y, K320N, K320S, K320H, K320V, K320L, K320F, K320Y, K320W, K320P, K320G, K322D, K322S, K322V, K322I, K322F, K322Y, K322W, K322P, K322G, S324H, S324F, S324M, S324W, S324P, S324G, N325K, N325R, N325S, N325F, N325M, N325Y, N325W, N325P, N325G, K326P, A327E, A327K, A327R, A327H, A327V, A327I, A327F, A327M, A327Y, A327W, A327P, L328D, L328Q, L328K, L328R, L328S, L328T, L328V, L328I, L328Y, L328W, L328P, L328G, P329D, P329E, P329N, P329Q, P329K, P329R, P329S, P329T, P329H, P329V, P329L, P329I, P329M, P329Y, P329W, P329G, A330E, A330N, A330T, A330P, A330G, P331D, P331Q, P331R, P331T, P331L, P331I, P331F, P331M, P331Y, P331W, I332K, I332R, I332S, I332V, I332F, I332M, I332W, I332P, I332G, E333L, E333F, E333M, E333P, K334P, T335N, T335S, T335H, T335V, T335L, T335I, T335F, T335M, T335W, T335P, T335G, I336E, I336K, I336Y, S337E, S337N, S337H, S298A, K326A, K326S, K326N, K326Q, K326D, K326E, K326W, K326Y, E333A, E333S, K334A, K334E, Y300I, Y300L, Q295K, E294N, S298N, S298V, S298D, D280H, K290S, D280Q, D280Y, K290G, K290T, K290Y, T250Q, T250E, M428L, M428F, S239D, S239E, S239N, S239Q, S239T, V240I, V240M, V264I, V264T, V264Y, E272Y, K274E, Y278T, N297D, T299A, T299V, T299I, T299H, K326T, L328A, L328H, A330Y, A330L, A330I, I332D, I332E, I332N, and I332Q, according to EU index numbering.

Any of the examples of an anti-EGFR antibody, or antigen-binding fragment thereof, provided herein can immunospecifically bind to EGFR.

Also provided herein are conjugates containing any of the anti-EGFR antibody, or antigen-binding fragment thereof, provided herein linked directly or indirectly to a targeted agent. The conjugate can contain the following components: (Ab), $(L)_q$, and (targeted agent)$_m$, wherein:

Ab is the anti-EGFR antibody or antigen-binding fragment thereof that binds to EGFR;
L is a linker for linking the Ab to the targeted agent;
m is at least 1, such as at least 1 to 8;
q is 0 or more, such as 0 to 8, as long as the resulting conjugate binds to the EGFR; and
the resulting conjugate binds to the EGFR.

In examples of any of the conjugates provided herein the targeted agent can be a protein, peptide, nucleic acid or small molecule. For example, the targeted agent is a therapeutic moiety. The therapeutic moiety can be a cytotoxic moiety, a radioisotope, a chemotherapeutic agent, a lytic peptide or a cytokine. Non-limiting examples of a therapeutic moiety in a conjugate herein can be a taxol; cytochalasin B; gramicidin D; ethidium bromide; emetine; mitomycin; etoposide; teniposide; vincristine; vinblastine; colchicine; doxorubicin; daunorubicin; dihydroxy anthracin dione; maytansine or an analog or derivative thereof; an auristatin or a functional peptide analog or derivative thereof; dolastatin 10 or 15 or an analogue thereof; irinotecan or an analogue thereof; mitoxantrone; mithramycin; actinomycin D; 1-dehydrotestosterone; a glucocorticoid; procaine; tetracaine; lidocaine; propranolol; puromycin; calicheamicin or an analog or derivative thereof; an antimetabolite; an alkylating agent; a platinum derivative; duocarmycin A, duocarmycin SA, rachelmycin (CC-1065), or an analog or derivative thereof; an antibiotic; pyrrolo[2,1-c][1, 4]-benzodiazepines (PDB); a toxin; ribonuclease (RNase); DNase I, Staphylococcal enterotoxin A; or pokeweed antiviral protein.

For example, the therapeutic moiety is a maytansine derivative that is a maytansinoid selected from among ansamitocin or mertansine (DM1). In another example, the therapeutic moiety is an auristatin or a functional peptide analog or derivative thereof that is monomethyl auristatin E (MMAE) or F (MMAF). In another example, the therapeutic moiety is an antimetabolite selected from among methotrexate, 6 mercaptopurine, 6 thioguanine, cytarabine, fludarabine, 5 fluorouracil, decarbazine, hydroxyurea, asparaginase, gemcitabine, and cladribine. In another example, the therapeutic moiety is an alkylating agent selected from among mechlorethamine, thiotepa, chlorambucil, melphalan, carmustine (BCNU), lomustine (CCNU), cyclophosphamide, busulfan, dibromomannitol, streptozotocin, dacarbazine (DTIC), procarbazine and mitomycin C. In another example, the therapeutic moiety is a platinum derivative that is cisplatin or carboplatin. In another example, the therapeutic moiety is an antibiotic selected from among dactinomycin, bleomycin, daunorubicin, doxorubicin, idarubicin, mithramycin, mitomycin, mitoxantrone, plicamycin and anthramycin (AMC). In another example, the therapeutic moiety is a toxin selected from among a diphtheria toxin and active fragments thereof and hybrid molecules, a ricin toxin, cholera toxin, a Shiga-like toxin, LT toxin, C3 toxin, Shiga toxin, pertussis toxin, tetanus toxin, soybean Bowman-Birk protease inhibitor, Pseudomonas exotoxin, alorin, saporin, modeccin, gelanin, abrin A chain, modeccin A chain, alpha-sarcin, Aleurites fordii proteins, dianthin proteins, Phytolacca americana proteins, momordica charantia inhibitor, curcin, crotin, gelonin, mitogillin, restrictocin, phenomycin, and enomycin toxins.

In any of the examples of conjugates provided herein, the antibody and targeted agent are linked directly. For example, the antibody and targeted agent are joined via a linker. The linker can be a peptide or a polypeptide or is a chemical linker. The linker can be a cleavable linker or a non-cleavable linker. The linker can be conjugated to one or more free thiols on the antibody or can be conjugated to one or more primary amines.

Provided herein are nucleic acid molecules that include a sequence of nucleotides encoding one or more heavy chain(s) of a conditionally active anti-EGFR antibody or antigen-binding fragment thereof, such as a modified anti-EGFR antibody or antigen-binding fragment thereof, provided herein. Also provided herein are nucleic acid molecules that include a sequence of nucleotides encoding one or more light chain(s) of a conditionally active anti-EGFR antibodies or antigen-binding fragment thereof, such as a modified anti-EGFR antibody or antigen-binding fragment thereof, provided herein. Also provided herein are vectors that include the nucleic acid molecules provided herein, and cells that include the vectors provided herein. Examples of cells provided herein include prokaryotic and eukaryotic cells.

Provided herein are combinations that include a conditionally active anti-EGFR antibody or antigen-binding fragment thereof, such as a modified anti-EGFR antibody or antigen-binding fragment provided herein, and a chemotherapeutic agent. A chemotherapeutic agent can be selected from among alkylating agents, nitrosoureas, topoisomerase inhibitors, and antibodies. In some examples, a chemotherapeutic agent is an additional anti-EGFR antibody or antigen-binding fragment thereof that differs from the first antibody. In some examples, the additional anti-EGFR antibody is selected from among cetuximab, panitumumab, nimotuzumab, and antigen-binding fragments thereof or variants thereof.

Provided herein are kits that include an antibody or antigen-binding fragment provided herein, or a combination provided herein, in one or more containers, and instructions for use.

Provided herein are pharmaceutical compositions that include any of the conditionally active anti-EGFR antibodies or antigen-binding fragments provided herein, such as any of the modified anti-EGFR antibody or antigen-binding fragment provided herein, and a pharmaceutically acceptable carrier or excipient. The pharmaceutical compositions also can include any of the combinations provided herein that include the antibody or antigen-binding fragment provided herein and an additional agent or agents. A pharmaceutical composition provided herein can be formulated as a gel, ointment, liquid, suspension, aerosol, tablet, pill or powder and/or can formulated for systemic, parenteral, topical, oral, mucosal, intranasal, subcutaneous, aerosolized, intravenous, bronchial, pulmonary, vaginal, vulvovaginal, esophageal, or oroesophageal administration. A pharmaceutical composition provided herein can be formulated for single dosage administration or for multiple dosage administration. In some examples, a pharmaceutical composition provided herein is a sustained release formulation.

Provided herein are methods for treating a condition responsive to treatment with an anti-EGFR antibody. In some examples, the methods are for treating a condition responsive to treatment with an anti-EGFR antibody in a subject and include administering to the subject a pharmaceutically effective amount of any of the pharmaceutical compositions provided herein.

Also provided herein are methods for treating a condition responsive to treatment with an anti-EGFR antibody. In some examples, the methods are for treating a condition responsive to treatment with an anti-EGFR antibody in a subject and include: a) identifying a subject with a condition responsive to treatment with an anti-EGFR antibody, and the subject exhibits side-effects associated with administration of an anti-EGFR antibody; and b) administering a conditionally active anti-EGFR antibody or antigen-binding fragment thereof, such as a modified anti-EGFR antibody or an antigen-binding fragment thereof, to the subject, and the modified anti-EGFR antibody, or antigen-binding fragment thereof. In such examples, the conditionally active anti-EGFR antibody or antigen binding fragment thereof is a modified antibody that includes an amino acid replacement(s) in a variable heavy chain, variable light chain or both of the unmodified anti-EGFR antibody, and the modified anti-EGFR antibody is conditionally active in the tumor microenvironment. In some examples, the unmodified anti-EGFR antibody is cetuximab, an antigen-binding fragment thereof or a variant thereof that does not include the amino acid replacement and specifically binds to EGFR.

In the methods herein, the conditionally active anti-EGFR antibody or antigen-binding fragment thereof, such as a modified anti-EGFR antibody or antigen-binding fragment thereof, can exhibit a higher ratio of binding activity for EGFR at or about pH 6.0 to pH 6.5 compared to at or about pH 7.4, when measured under the same conditions except for the difference in pH. In the methods herein, the conditionally active anti-EGFR antibody or antigen-binding fragment thereof, such as a modified anti-EGFR antibody or antigen-binding fragment thereof, can exhibit a ratio of binding activity for EGFR at or about pH 6.0 to pH 6.5 compared to at or about pH 7.4 of at least 1.1, 1.2, 1.3, 1.4, 1.5, 1.6, 1.7, 1.8, 1.9, 2.0, 2.5, 3.0, 3.5, 4.0, 5.0 or more, when measured under the same conditions except for the difference in pH.

In some examples of the methods herein, the conditionally active anti-EGFR antibody or antigen-binding fragment thereof is a modified anti-EGFR antibody or fragment thereof that has a higher activity at a pH selected from among pH 6.0 - pH 7.0 than at pH 7.4 than the unmodified antibody; or the modified anti-EGFR antibody and fragment thereof has a lower activity at a pH selected from among pH 6.0 - pH 7.0 than at pH 7.4, compared to the unmodified antibody.

In the methods provided herein, for therapeutic administration, the dose of the conditionally active anti-EGFR antibody or antigen-binding fragment thereof, such as a modified anti-EGFR antibody or antigen-binding fragment thereof, can be adjusted depending upon its relative activity to the reference or unmodified antibody in the tumor microenvironment. Hence the dosage can be lower, particularly if the reference conditionally active (e.g. modified antibody) is more active in the tumor microenvironment than the reference or unmodified antibody, higher or about the same or the same. In instances where the dosage is lower, the reduction in side-effects can result from the lower dosage. In certain aspects, conditionally active antibodies that exhibit increased selectivity to a tumor microenvironment can be administered at a higher dosage than existing similar therapeutics, resulting in increased efficacy. Dosage readily can be empirically determined by the skilled practitioner .

In any of the examples of the methods provided herein, a subject to whom the antibody or fragment thereof has been administered, is one that is identified to exhibit side-effects associated with binding of an anti-EGFR antibody to the EGFR receptor in basal keratinocytes. Side effects include, but are not limited to, for example, acneiform rash, papulopustular rash, hair growth abnormalities, dry and itchy skin and periungual inflammation with tenderness, telangiectasia, hyperpigmentation, pruritus without rash, erythema, oral aphthae, anaphylactic reactions, dyspnea, cough, wheezing, pneumonia, hypoxemia, respiratory insufficiency/failure, pulmonary embolus, pleural effusion and non-specific respiratory disorders, fever, chills, asthenia/malaise, mucosal surface problems, nausea, gastrointestinal problems, abdominal pain, headache and hypomagnesemia.

In any of the examples of practice of the methods provided herein, the conditionally active anti-EGFR antibody or antigen-binding fragment thereof is a modified anti-EGFR or antigen-binding fragment thereof. The VH chain, or a portion thereof, of the modified anti-EGFR antibody, or an antigen-binding fragment thereof, includes one or more amino acid replacement(s) corresponding to an amino acid replacement selected from among T023K, T023H, T023R, T023A, T023C, T023E, T023G, T023I, T023M, T023N, T023P, T023S, T023V, T023W, T023L, V024R, V024A, V024F, V024G, V024I, V024M, V024P, V024S, V024T, V024L, V024E, S025H, S025R, S025A, S025C, S025D, S025E, S025F, S025G, S025I, S025M, S025P, S025Q, S025T, S025V, S025L, G026H, G026R, G026D, G026F, G026M, G026N, G026P, G026Q, G026S, G026Y, G026L, F027H, F027R, F027A, F027D, F027E, F027G, F027M, F027P, F027Q, F027S, F027T, F027V, F027W, F027Y, F027L, S028K, S028H, S028R, S028A, S028D, S028I, S028M, S028P, S028Q, S028V, S028W, S028L, L029K, L029H, L029A, L029D, L029G, L029I, L029M, L029N, L029S, L029V, T030H, T030R, T030D, T030G, T030I, T030M, T030N, T030P, T030S, T030V, T030W, T030Y, N031K, N031H, N031D, N031E, N031G, N031I, N031T, N031V, N031L, Y032H, Y032R, Y032C, Y032M, Y032N, Y032T, Y032V, Y032L, G033E, G033M, G033S, G033T, G033Y, V034A, V034C, V034I, V034M, V034P, V034L, H035I, H035Q, W036K, W036A, W036I, W036V, W036Y, V050K, V050H, V050A, V050D, V050E, V050G, V050I, V050N, V050Q, V050T, V050L, I051K, I051H, I051A, I051C, 1051E, I051G, I051N, I051Q, I051S, I051V, I051Y, I051L, W052I, W052N, W052Y, S053H, S053R, S053A, S053C, S053G, S053I, S053M, S053P, S053Q, S053L, S053T, S053V, S053Y, G054H, G054R, G054A, G054C, G054D, G054P, G054S, G055H, G055R, G055M, G055S, G055Y, N056K, N056A, N056P, N056S, N056V, N056G, T057H, T057R, T057L, T057A, T057C, T057D, T057F, T057M, T057N, T057Q, T057W, T057Y, D058L, D058G, D058M, D058N, D058Q, Y059H, Y059R, Y059A, Y059C, Y059D, Y059E, Y059G, Y059I, Y059P, Y059Q, Y059S, Y059T, Y059V, Y059W, N060K, N060A, N060C, N060D, N060F, N060G, N060P, N060Q, N060S, N060T, N060Y, T061N, T061Q, P062G, F063H, F063R, F063L, F063A, F063C, F063D, F063G, F063M, F063N, F063Q, F063S, F063V, T064R, T064L, T064C, T064F, T064G, T064N, T064Q, T064V, S065H, S065R, S065L, S065C, S065E, S065F, S065G, S065I, S065M, S065N, S065P, S065Q, S065T, S065W, S065Y, R066L, R066A, R066C, R066E, R066F, R066N, R066P, R066Q, R066S, R066T, R066V, R066G, L067A, L067C, L067D, L067E, L067I, L067M, L067Q, L067S, L067T, L067V, L067Y, S068K, S068H, S068R, S068L, S068C, S068D, S068E, S068F, S068G, S068I, S068N, S068Q, S068T, S068V, I069A, I069C, I069G, I069Y, N070H, N070R, N070L, N070D, N070E, N070F, N070G, N070I, N070P, N070Q, N070S, N070T, N070V, N070Y, K071H, K071R, K071L, K071A, K071C, K071F, K071G, K071Q, K071S, K071T, K071V, K071W, K071Y, D072K, D072H, D072R, D072L, D072A, D072G, D072I, D072M, D072N, D072Q, D072S, D072V, D072W, D072Y, N073H, N073R, N073L, N073A, N073C, N073G, N073I, N073M, N073P, N073Q, N073S, N073T, N073V, N073W, N073Y, S074K, S074H, S074R, S074L, S074A, S074C, S074D, S074E, S074G, S074I, S074M, S074P, S074T, S074V, S074Y, K075H, K075R, K075L, K075A, K075C, K075E, K075F, K075M, K075Q, K075T, K075V, K075W, K075Y, S076H, S076R, S076L, S076A, S076C, S076D, S076E, S076F, S076M, S076P, S076Q, S076T, S076Y, Q077H, Q077R, Q077L, Q077A, Q077E, Q077G, Q077I, Q077M, Q077N, Q077S, Q077V, Q077W, Q077Y, Y093H, Y093V, Y093W, Y094R, Y094L, R097H, R097W, A098P, L099N, L099W, T100H, T100L, T100A, T100D, T100I, T100N, T100P, T100Q, T100S, T100V, T100Y, Y101H, Y101E, Y101F, Y101M, Y101W, Y102R, Y102C, Y102D, Y102I, Y102N, Y102W, D103R, D103L, D103A, D103C, D103I, D103P, D103Q, D103Y, Y104H, Y104L, Y104D, Y104F, Y104I, Y104M, Y104S, Y104V, E105H, E105T, F106L, F106V, F106W, F106Y, A107K, A107H, A107R, A107L, A107C, A107D, A107E, A107G, A107N, A107S, A107T, A107Y, Y108K, Y108H, Y108R, Y108L, Y108C, Y108F, Y108I, Y108N, Y108S, Y108T, Y108V, Y108W, W109I, W109M, W109Y, G110R, G110A, G110M, G110P, G110T, Q111K, Q111H, Q111R, Q111L, Q111D, Q111E, Q111G, Q111M, Q111P, Q111S, Q111T, Q111W, Q111Y, Q111V, G112A, G112N, G112P, G112S, G112T, G112Y, Y104D/Q111P and V24E/F27R/R97H/Q111P with reference to amino acid positions set forth in SEQ ID NO: 1 or 3, corresponding amino acid positions are identified by alignment of the VH chain of the antibody with the VH chain set forth in SEQ ID NO:3 and the portion thereof is sufficient to form an antigen binding site and includes the amino acid replacement; and/or the modified VL chain, or portion thereof, includes an amino acid replacement(s) corresponding to amino acid replacement(s) selected from among D001W, I002C, I002V, I002W, L003D, L003F, L003G, L003S, L003T, L003V, L003W, L003Y, L003R, L004C, L004E, L004F, L004I, L004P, L004S, L004T, L004V, L004W, L004K, L004H, L004R, T005A, T005C, T005D, T005E, T005F, T005G, T005N, T005S, T005W, T005L, T005K, T005H, T005R, T005P, R024A, R024C, R024F, R024L, R024M, R024S, R024W, R024Y, R024G, A025C, A025G, A025L, A025V, S026A, S026C, S026D, S026I, S026M, S026N, S026V, S026W, S026L, S026G, S026H, S026R, Q027A, Q027D, Q027E, Q027F, Q027I, Q027M, Q027N, Q027P, Q027T, S028A, S028D, S028N, S028Q, S028L, S028K, S028H, I029A, I029E, I029F, I029S, I029T, I029R, G030A, G030E, G030F, G030I, G030M, G030P, G030Q, G030S, G030V, G030Y, G030L, G030K, G030H, G030R, T031A, T031F, T031G, T031M, T031S, T031V, T031W, T031L, T031K, T031H, N032G, I033F, I033G, I033M, I033T, I033V, I033H, I048M, I048S, I048L, I048K, K049A, K049E, K049F, K049G, K049N, K049Q, K049S, K049T, K049V, K049Y, K049L, K049H, K049R, A051T, A051L, S052A, S052C, S052D, S052E, S052G, S052I, S052M, S052Q, S052V, S052W, S052R, S052K, E053G, S054M, I055A, I055F, S056G, S056L, S056A, S056C, S056D, S056E, S056F, S056N, S056P, S056Q, S056V, S056W, S056H, S056R, S056K, Y086F, Y086M, Y086H, Y087L, Y087C, Y087D, Y087F, Y087G, Y087I, Y087N, Y087P, Y087S, Y087T, Y087V, Y087W, Y087K, Y087H, Y087R, Q089E, N091L, N091A, N091C, N091I, N091M, N091S, N091T, N091V, N091H, N091R, N092C, N092D, N092L, N092M, N092S, N092T, N092V, N092W, N092Y, N092H, N092K, N092R, N093T, T096L, T096C, T096M, T096V, T097L, T097A, T097D, T097G, T097Q, T097S, T097V, T097K, T097R, F098A, F098M, F098S, F098V, F098Y, G099L, G099D, G099E, G099F, G099I, G099M, G099N, G099S, G099T, G099V, G099K, G099H, Q100C, Q100D, Q100E, Q100F, Q100I, Q100M, Q100N, Q100P, Q100T, Q100V, Q100W, Q100Y, Q100K, Q100H and Q100R with reference to amino acid positions set forth in SEQ ID NO:2 or 4, corresponding amino acid positions are identified by alignment of the VL chain of the antibody with the VL chain set forth in SEQ ID NO:4, and the portion thereof is sufficient to form an antigen binding site and includes the amino acid replacement.

In examples of the methods provided herein, the unmodified anti-EGFR antibody or variant thereof can include a heavy chain having a sequence of amino acids set forth in SEQ ID NO: 1 or a sequence of amino acids that exhibits at least 75% sequence identity to the sequence of amino acids set forth in SEQ ID NO:1 and a light chain having a sequence of amino acids set forth SEQ ID NO:2 or a sequence of amino acids that exhibits at least 75% sequence identity to the sequence of amino acids set forth in SEQ ID NO:2; or a heavy chain having a having a sequence of amino acids set forth in SEQ ID NO: 8 or a sequence of amino acids that exhibits at least 75% sequence identity to the sequence of amino acids set forth in SEQ ID NO:8 and a light chain having a sequence of amino acids set forth SEQ ID NO:9 or a sequence of amino acids that exhibits at least 75% sequence identity to the sequence of amino acids set forth in SEQ ID NO:9.

In some examples of the methods provided herein, the unmodified antibody, antigen-binding fragment thereof or variant thereof is humanized. In some examples of the methods provided herein, the unmodified antibody, antigen-binding fragment thereof or variant thereof includes a variable heavy chain set forth in SEQ ID NO:28 and a variable light chain set forth in SEQ ID NO:29. In some examples of the methods provided herein, the unmodified antibody, antigen-binding fragment thereof or variant thereof is an antigen-binding fragment thereof and the antigen-binding fragment is selected from among a Fab, Fab', F(ab')$_2$, single-chain Fv (scFv), Fv, dsFv, diabody, Fd and Fd' fragments.

In some examples of the methods provided herein, the unmodified anti-EGFR antibody, antigen-binding fragment thereof or variant thereof is a Fab fragment that includes a heavy chain having a sequence of amino acids set forth in SEQ ID NO:5 or a sequence of amino acids that exhibits at least 75% sequence identity to SEQ ID NO:5 and a light chain having a sequence of amino acids set forth in SEQ ID NO:2 or a sequence of amino acids that exhibits at least 75% sequence identity to a sequence of amino acids set forth in SEQ ID NO:2.

In any of the Examples of the methods provided herein, the conditionally active anti-EGFR antibody, such as a modified anti-EGFR antibody or an antigen-binding fragment thereof can include a variable heavy (VH) chain set forth in any of SEQ ID NOS: 30-557, 1063, 1064, 1062, 1093, 1098-1107, 1112-1131, 1134-1137 or 1146-1152 or a sequence of amino acids that exhibits at least 75% sequence identity to any of SEQ ID NOS: 30-557, 1063, 1064, 1062, 1093, 1098-1107, 1112-1131, 1134-1137 or 1146-1152; and/or a variable (VL) chain set forth in any of SEQ ID NOS: 810-1061, 1067-1068, 1138-1145 or 1153-1159 or a sequence of amino acids that exhibits at least 75% sequence identity to any of SEQ ID NOS: 810-1061, 1067-1068, 1138-1145 or 1153-1159.

In any of the examples of the methods provided herein, the condition responsive to treatment with an anti-EGFR antibody is a tumor, cancer or metastasis. Examples of conditions responsive to treatment with an anti-EGFR antibody are head and neck cancer, non-small cell lung cancer or colorectal cancer. In the methods provided herein, the subject to whom the antibody is administered includes mammals such as, for example, a human.

In examples of the methods provided herein, the pharmaceutical composition can be administered topically, parenterally, locally, systemically. In some examples, the pharmaceutical composition is administered intranasally, intramuscularly, intradermally, intraperitoneally, intravenously, subcutaneously, orally, or by pulmonary administration.

The methods provided herein can include combination therapies in which the other anti-tumor therapies, such as surgery, radiation, chemotherapy, viral therapy and other anti-tumor antibodies, is/are administered with, before, during after, and intermittently with antibody therapy. Chemotherapeutic agents that can be administered in combination therapy, include, but are not limited to, for example, irinotecan, simvastatin and 5-fluorouracil (5-FU). The methods provided herein can include administering one or more additional anti-EGFR antibodies and antigen-binding fragments thereof. Non-limiting examples of additional anti-EGFR antibodies include cetuximab, panitumumab, nimotuzumab, and antigen-binding fragments thereof.

In the methods provided herein, the pharmaceutical composition and the anticancer agent can be formulated as a single composition or as separate compositions. The pharmaceutical composition and the anticancer agent can be administered sequentially, simultaneously or intermittently.

In the methods provided herein, the antibody can be administered at a dosage of about or 0.1 mg/kg to about or 100 mg/kg, such as, for example, about or 0.5 mg/kg to about or 50 mg/kg, about or 5 mg/kg to about or 50 mg/kg, about or 1 mg/kg to about or 20 mg/kg, about or 1 mg/kg to about or 100 mg/kg, about or 10 mg/kg to about or 80 mg/kg, or about or 50 mg/kg to about or 100 mg/kg or more; or at a dosage of about or $0.01$ mg/m$^2$ to about or $800$ mg/m$^2$ or more, such as for example, about or $0.01$ mg/m$^2$, about or $0.1$ mg/m$^2$, about or $0.5$ mg/m$^2$, about or $1$ mg/m$^2$, about or $5$ mg/m$^2$, about or $10$ mg/m$^2$, about or $15$ mg/m$^2$, about or $20$ mg/m$^2$, about or $25$ mg/m$^2$, about or $30$ mg/m$^2$, about or $35$ mg/m$^2$, about or $40$ mg/m$^2$, about or $45$ mg/m$^2$, about or $50$ mg/m$^2$, about or $100$ mg/m$^2$, about or $150$ mg/m$^2$, about or $200$ mg/m$^2$, about or $250$ mg/m$^2$, about or $300$ mg/m$^2$, about or $400$ mg/ m$^2$, about or $500$ mg/ m$^2$, about or $600$ mg/ m$^2$ about or $700$ mg/ m$^2$ or about or $800$ mg/ m$^2$ or more.

In some aspects of the methods herein, the subject has a tumor that does not contain a marker that confers resistance to anti-EGFR therapy, such as where the marker is a mutation in *KRAS, NRAS* or *BRAF.* For example, the subject has a KRAS mutation-negative epidermal growth factor receptor (EGFR)-expressing colorectal cancer.

In other examples of the methods herein, the subject contains a tumor with a marker that confers resistance to anti-EGFR therapy, such as a marker that is a mutation in *KRAS, NRAS* or *BRAF* and the antibody or fragment thereof is effective against tumors with such markers.

The compositions provided herein can be for treating any condition responsive to treatment with an anti-EGFR antibody, such as, for example, a tumor, cancer and metastasis. In some examples, the condition responsive to treatment with an anti-EGFR antibody is head and neck cancer, non-small cell lung cancer or other lung cancer or colorectal cancer.

**The invention will now be understood with reference to the following clauses:**

1. An anti-EGFR antibody, or antigen-binding fragment thereof, wherein:

the anti-EGFR antibody, or antigen-binding fragment thereof, exhibits a ratio of binding activity to human epidermal growth factor receptor (EGFR) or a soluble fragment thereof under conditions in a tumor environment compared to under conditions in a non-tumor environment of at least 3.0;
conditions in a tumor environment comprise one or both of pH between or about between 5.6 to 6.8 or lactate concentration between or about between 5 mM to 20 mM, and protein concentration of 10 mg/mL to 50 mg/mL;
conditions in a non-tumor environment comprise one or both of pH between or about between 7.0 to 7.8 or lactate concentration between or about between 0.5 mM to 5 mM, and protein concentration of 10 mg/mL to 50 mg/mL , whereby the anti-EGFR antibody or fragment thereof is conditionally active under conditions in a tumor microenvironment.

2. The anti-EGFR antibody, or antigen-binding fragment thereof, of 1, wherein the anti-EGFR antibody, or antigen-binding fragment thereof, exhibits the ratio of activity under conditions that exist in a tumor microenvironment that comprise a pH of between or about between 5.6 to 6.8 compared to under conditions that exist in a non-tumor microenvironment that comprise a pH of between or about between 7.0 to 7.8.

3. The anti-EGFR antibody, or antigen-binding fragment thereof, of 1 or 2, wherein the anti-EGFR antibody, or antigen-binding fragment thereof, exhibits the ratio of activity under conditions that exist in a tumor microenvironment that comprise a pH of between or about between 6.0 to 6.5 compared to under conditions that exist in a non-tumor microenvironment that comprise a pH of about 7.4.

4. The anti-EGFR antibody, or antigen-binding fragment thereof, of any of 1-3, wherein the anti-EGFR antibody, or antigen-binding fragment thereof, exhibits the ratio of activity under conditions that exist in a tumor microenvironment that comprise lactate concentration between or about between 5 mM to 20 mM compared to under conditions that exist in a non-tumor microenvironment that comprise lactate concentration between or about between 0.5 mM to 5 mM.

5. The anti-EGFR antibody, or antigen-binding fragment thereof, of any of 1-4, wherein the anti-EGFR antibody or, or antigen-binding fragment thereof, of any of 1-4, exhibits the ratio of activity under conditions of a tumor microenvironment that comprise pH of 6.0 to 6.5 and lactate concentration of 10 mM to 20 mM compared to under condition of a non-tumor microenvironment that comprise pH of 7.0 to 7.4, inclusive, and lactate concentration of 0.5 mM to 2 mM.

6. The anti-EGFR antibody, or antigen-binding fragment thereof, of any of 1-5, wherein the protein concentration under conditions in a tumor microenvironment and under conditions in a non-tumor microenvironment is substantially the same or is the same.

7. The anti-EGFR antibody, or antigen-binding fragment thereof, of any of 1-6, wherein the protein concentration is at least 12 mg/mL, 15 mg/mL, 20 mg/mL, 25 mg/mL, 30 mg/mL, 35 mg/mL, 40 mg/mL, 45 mg/mL or 50 mg/mL.

8. The anti-EGFR antibody, or antigen-binding fragment thereof, of any of 1-7, wherein the protein is serum albumin.

9. The anti-EGFR antibody, or antigen-binding fragment thereof, of any of 1-8, wherein the protein is human serum albumin.

10. The anti-EGFR antibody, or antigen-binding fragment thereof, of any of 1-9, wherein the protein is provided in serum.

11. The anti-EGFR antibody, or antigen-binding fragment thereof, of 10, wherein the concentration of serum is 20% (vol/vol) to 90% (vol/vol), 20% (vol/vol) to 50% (vol/vol) or 20% (vol/vol) to 40% (vol/vol).

12. The anti-EGFR antibody, or antigen-binding fragment thereof, of 10 or 11, wherein the concentration of serum is less than 90% (vol/vol) and is about or is at least or is 20% (vol/vol), 25% (vol/vol), 30% (vol/vol), 35% (vol/vol), 40% (vol/vol), 45% (vol/vol) or 50% (vol/vol).

13. The anti-EGFR antibody, or antigen-binding fragment thereof, of any of 10-12, wherein the concentration of serum is or is about 25% (vol/vol).

14. The anti-EGFR antibody, or antigen-binding fragment thereof, of any of 10-13, wherein the serum is human serum.

15. The anti-EGFR antibody, or antigen-binding fragment thereof, of any of 1-14, wherein the binding activity is determined in vitro in a solid-phase binding assay.

16. The anti-EGFR antibody, or antigen-binding fragment thereof, of any of 1-15, wherein the solid-phase binding assay is an immunoassay.

17. The anti-EGFR antibody, or antigen-binding fragment thereof, of any of 1-16, wherein the immunoassay is an enzyme-linked immunosorbent assay (ELISA).

18. The anti-EGFR antibody, or antigen-binding fragment thereof, of any of 1-17, wherein:

the binding activity is a spectrophotometric measurement of binding; and
the ratio of binding activity is the ratio of the spectrophotometric measurement for binding under conditions that exist in a tumor microenvironment compared to under conditions that exist in a non-tumor microenvironment at the same concentration of antibody.

19. The anti-EGFR antibody, or antigen-binding fragment thereof, of 18, wherein the concentration of antibody is between or about between 1 ng/mL to 100 ng/mL.

20. The anti-EGFR antibody, or antigen-binding fragment thereof, of any of 1-14, wherein:

the binding activity is the dissociation constant ($K_D$) as determined using a biosensor; and
the antibody, or antigen-binding fragment thereof, exhibits a ratio of at least 3 if there is at least 3-fold tighter affinity under conditions in the tumor-microenvironment compared to under conditions in a non-tumor microenvironment.

21. The anti-EGFR antibody, or antigen-binding fragment thereof, of 20, wherein the dissociation constant ($K_D$) is less than $1 \times 10^{-8}$ M, $5 \times 10^{-9}$ M, $1 \times 10^{-9}$ M, $5 \times 10^{-10}$ M, $1 \times 10^{-10}$ M, $5 \times 10^{-11}$ M, $1 \times 10^{-11}$ M or less under conditions that exist in a tumor microenvironment.

22. The anti-EGFR antibody, or antigen-binding fragment thereof, of any of 1-14, wherein:

the binding activity is the off-rate as determined using a biosensor; and
the antibody, or antigen-binding fragment thereof, exhibits a ratio of at least 3 if the off-rate is at least 3 times slower under conditions that exist in a tumor microenvironment compared to under conditions that exist under a non-tumor microenvironment.

23. The anti-EGFR antibody, or antigen-binding fragment thereof, of any of 20-22, wherein the biosensor is a Biacore sensor or Octet sensor.

24. The anti-EGFR antibody, or antigen-binding fragment thereof, of any of 1-14, wherein:

binding activity is assessed *in vivo* in a subject in a tumor microenvironment expressing EGFR or in a non-tumor microenvironment expressing EGFR.

25. The anti-EGFR antibody, or antigen-binding fragment thereof, of 24, wherein the non-tumor microenvironment is the basal layer of the skin expressing human EGFR.

26. The anti-EGFR antibody, or antigen-binding fragment thereof, of 24 or 25, wherein:

the subject is a non-human animal;
the tumor microenvironment comprises a human tumor xenograft expressing human EGFR; and
the non-tumor microenvironment comprises a human skin xenograft expressing human EGFR.

27. The anti-EGFR antibody, or antigen-binding fragment thereof, of 26, wherein the human tumor xenograft is an A431 xenograft.

28. The anti-EGFR antibody, or antigen-binding fragment thereof, of any of 24-27, wherein:

the anti-EGFR antibody, or antigen-binding fragment thereof, is fluorescently labeled; and
binding activity is the fluorescent signal intensity.

29. The anti-EGFR antibody, or antigen-binding fragment thereof, of any of 1-28, wherein the ratio of activity is at least 3.5, 4, 5, 6, 7, 8, 9, 10, 11, 12, 13, 14, 15, 16, 17, 18, 19, 20, 25, 30, 35, 40, 45, 50, 55, 60 or more.

30. The anti-EGFR antibody, or antigen-binding fragment thereof, of any of 1-29, wherein:

the $V_H$ region of the variable heavy chain exhibits at least 56% sequence identity to its closest human $V_H$ gene segment germline sequence; and
the $V_L$ region of the light chain exhibits at least 75% sequence identity to its closest human $V_L$ gene segment germline sequence.

31. The anti-EGFR antibody, or antigen-binding fragment thereof, of any of 1-30, comprising:

a) a variable heavy (VH) chain comprising the sequence of amino acids set forth in SEQ ID NO:495, 1062, 1112, 1114-1117, 1124-1126, 1128-1130, 1134-1137, or 1146-1152, or a sequence of amino acids that exhibits at least 85% sequence identity to any of SEQ ID NOS: 495, 1062, 1112, 1114-1117, 1124-1126, 1128-1130, 1134-1137, or 1146-1152; and
b) a variable light (VL) chain comprising the sequence of amino acids set forth in SEQ ID NO:4, 10, 1138-1145, 1153-1159 or 1186, or a sequence of amino acids that exhibits at least 85% sequence identity to SEQ ID NO:4, 10, 1138-1145, 1153-1159 or 1186.

32. The anti-EGFR antibody, or antigen-binding fragment thereof, of any of 1-31, selected from among an antibody comprising:

a) the variable heavy chain set forth in SEQ ID NO:495 or a sequence of amino acids that exhibits at least 85% sequence identity to SEQ ID NO:495, and the variable light chain set forth in SEQ ID NO:4 or 10 or a sequence of amino acids that exhibits at least 85% sequence identity to SEQ ID NO:4 or 10;
b) the variable heavy chain set forth in SEQ ID NO:1062 or a sequence of amino acids that exhibits at least 85% sequence identity to SEQ ID NO:1062, and the variable light chain set forth in SEQ ID NO:4 or 10 or a sequence of amino acids that exhibits at least 85% sequence identity to SEQ ID NO:4 or 10;
c) the variable heavy chain set forth in SEQ ID NO:1112 or a sequence of amino acids that exhibits at least 85% sequence identity to SEQ ID NO: 1112, and the variable light chain set forth in SEQ ID NO:4 or 10 or a sequence of amino acids that exhibits at least 85% sequence identity to SEQ ID NO:4 or 10;

d) the variable heavy chain set forth in SEQ ID NO:1114 or a sequence of amino acids that exhibits at least 85% sequence identity to SEQ ID NO: 1114, and the variable light chain set forth in SEQ ID NO:4 or 10 or a sequence of amino acids that exhibits at least 85% sequence identity to SEQ ID NO:4 or 10;

e) the variable heavy chain set forth in SEQ ID NO:1115 or a sequence of amino acids that exhibits at least 85% sequence identity to SEQ ID NO: 1115, and the variable light chain set forth in SEQ ID NO:4 or 10 or a sequence of amino acids that exhibits at least 85% sequence identity to SEQ ID NO:4 or 10;

f) the variable heavy chain set forth in SEQ ID NO:1116 or a sequence of amino acids that exhibits at least 85% sequence identity to SEQ ID NO: 1116, and the variable light chain set forth in SEQ ID NO:4 or 10 or a sequence of amino acids that exhibits at least 85% sequence identity to SEQ ID NO:4 or 10;

g) the variable heavy chain set forth in SEQ ID NO:1117 or a sequence of amino acids that exhibits at least 85% sequence identity to SEQ ID NO: 1117, and the variable light chain set forth in SEQ ID NO:4 or 10 or a sequence of amino acids that exhibits at least 85% sequence identity to SEQ ID NO:4 or 10;

h) the variable heavy chain set forth in SEQ ID NO:1124 or a sequence of amino acids that exhibits at least 85% sequence identity to SEQ ID NO:1124, and the variable light chain set forth in SEQ ID NO:4 or 10 or a sequence of amino acids that exhibits at least 85% sequence identity to SEQ ID NO:4 or 10;

i) the variable heavy chain set forth in SEQ ID NO:1125 or a sequence of amino acids that exhibits at least 85% sequence identity to SEQ ID NO:1125, and the variable light chain set forth in SEQ ID NO:4 or 10 or a sequence of amino acids that exhibits at least 85% sequence identity to SEQ ID NO:4 or 10;

j) the variable heavy chain set forth in SEQ ID NO:1126 or a sequence of amino acids that exhibits at least 85% sequence identity to SEQ ID NO:1126, and the variable light chain set forth in SEQ ID NO:4 or 10 or a sequence of amino acids that exhibits at least 85% sequence identity to SEQ ID NO:4 or 10;

k) the variable heavy chain set forth in SEQ ID NO:1128 or a sequence of amino acids that exhibits at least 85% sequence identity to SEQ ID NO:1128, and the variable light chain set forth in SEQ ID NO:4 or 10 or a sequence of amino acids that exhibits at least 85% sequence identity to SEQ ID NO:4 or 10;

1) the variable heavy chain set forth in SEQ ID NO:1129 or a sequence of amino acids that exhibits at least 85% sequence identity to SEQ ID NO:1129, and the variable light chain set forth in SEQ ID NO:4 or 10 or a sequence of amino acids that exhibits at least 85% sequence identity to SEQ ID NO:4 or 10;

m) the variable heavy chain set forth in SEQ ID NO:1130 or a sequence of amino acids that exhibits at least 85% sequence identity to SEQ ID NO:1130, and the variable light chain set forth in SEQ ID NO:4 or 10 or a sequence of amino acids that exhibits at least 85% sequence identity to SEQ ID NO:4 or 10;

n) the variable heavy chain set forth in SEQ ID NO:1134 or a sequence of amino acids that exhibits at least 85% sequence identity to SEQ ID NO: 1134, and the variable light chain set forth in SEQ ID NO:1138 or a sequence of amino acids that exhibits at least 85% sequence identity to SEQ ID NO:1138;

o) the variable heavy chain set forth in SEQ ID NO:1134 or a sequence of amino acids that exhibits at least 85% sequence identity to SEQ ID NO: 1134, and the variable light chain set forth in SEQ ID NO:1139 or a sequence of amino acids that exhibits at least 85% sequence identity to SEQ ID NO:1139;

p) the variable heavy chain set forth in SEQ ID NO:1135 or a sequence of amino acids that exhibits at least 85% sequence identity to SEQ ID NO:1135, and the variable light chain set forth in SEQ ID NO:1138 or a sequence of amino acids that exhibits at least 85% sequence identity to SEQ ID NO:1138;

q) the variable heavy chain set forth in SEQ ID NO:1134 or a sequence of amino acids that exhibits at least 85% sequence identity to SEQ ID NO:1134, and the variable light chain set forth in SEQ ID NO:1140 or a sequence of amino acids that exhibits at least 85% sequence identity to SEQ ID NO:1140;

r) the variable heavy chain set forth in SEQ ID NO:1134 or a sequence of amino acids that exhibits at least 85% sequence identity to SEQ ID NO:1134, and the variable light chain set forth in SEQ ID NO:1141 or a sequence of amino acids that exhibits at least 85% sequence identity to SEQ ID NO:1141;

s) the variable heavy chain set forth in SEQ ID NO:1134 or a sequence of amino acids that exhibits at least 85% sequence identity to SEQ ID NO:1134, and the variable light chain set forth in SEQ ID NO:1142 or a sequence of amino acids that exhibits at least 85% sequence identity to SEQ ID NO:1142;

t) the variable heavy chain set forth in SEQ ID NO:1135 or a sequence of amino acids that exhibits at least 85% sequence identity to SEQ ID NO:1135, and the variable light chain set forth in SEQ ID NO:1142 or a sequence of amino acids that exhibits at least 85% sequence identity to SEQ ID NO:1142;

u) the variable heavy chain set forth in SEQ ID NO:1134 or a sequence of amino acids that exhibits at least 85% sequence identity to SEQ ID NO:1134, and the variable light chain set forth in SEQ ID NO:1143 or a sequence of amino acids that exhibits at least 85% sequence identity to SEQ ID NO:1143;

v) the variable heavy chain set forth in SEQ ID NO:1136 or a sequence of amino acids that exhibits at least 85% sequence identity to SEQ ID NO:1136, and the variable light chain set forth in SEQ ID NO:1142 or a sequence of amino acids that exhibits at least 85% sequence identity to SEQ ID NO:1142;

w) the variable heavy chain set forth in SEQ ID NO:1137 or a sequence of amino acids that exhibits at least 85% sequence identity to SEQ ID NO:1137, and the variable light chain set forth in SEQ ID NO:1144 or a sequence of amino acids that exhibits at least 85% sequence identity to SEQ ID NO:1144;

x) the variable heavy chain set forth in SEQ ID NO:1136 or a sequence of amino acids that exhibits at least 85% sequence identity to SEQ ID NO:1136, and the variable light chain set forth in SEQ ID NO:1144 or a sequence of amino acids that exhibits at least 85% sequence identity to SEQ ID NO:1144;

y) the variable heavy chain set forth in SEQ ID NO:1137 or a sequence of amino acids that exhibits at least 85% sequence identity to SEQ ID NO:1137, and the variable light chain set forth in SEQ ID NO:1145 or a sequence of amino acids that exhibits at least 85% sequence identity to SEQ ID NO:1145;

z) the variable heavy chain set forth in SEQ ID NO:1136 or a sequence of amino acids that exhibits at least 85% sequence identity to SEQ ID NO:1136, and the variable light chain set forth in SEQ ID NO:1145 or a sequence of amino acids that exhibits at least 85% sequence identity to SEQ ID NO:1145;

aa) the variable heavy chain set forth in SEQ ID NO:1146 or a sequence of amino acids that exhibits at least 85% sequence identity to SEQ ID NO:1146, and the variable light chain set forth in SEQ ID NO:1153 or a sequence of amino acids that exhibits at least 85% sequence identity to SEQ ID NO:1153;

bb) the variable heavy chain set forth in SEQ ID NO:1147 or a sequence of amino acids that exhibits at least 85% sequence identity to SEQ ID NO:1147, and the variable light chain set forth in SEQ ID NO:1153 or a sequence of amino acids that exhibits at least 85% sequence identity to SEQ ID NO:1153;

cc) the variable heavy chain set forth in SEQ ID NO:1148 or a sequence of amino acids that exhibits at least 85% sequence identity to SEQ ID NO:1148, and the variable light chain set forth in SEQ ID NO:1154 or a sequence of amino acids that exhibits at least 85% sequence identity to SEQ ID NO:1154;

dd) the variable heavy chain set forth in SEQ ID NO:1149 or a sequence of amino acids that exhibits at least 85% sequence identity to SEQ ID NO:1149, and the variable light chain set forth in SEQ ID NO:1154 or a sequence of amino acids that exhibits at least 85% sequence identity to SEQ ID NO:1154;

ee) the variable heavy chain set forth in SEQ ID NO:1150 or a sequence of amino acids that exhibits at least 85% sequence identity to SEQ ID NO:1150, and the variable light chain set forth in SEQ ID NO:1155 or a sequence of amino acids that exhibits at least 85% sequence identity to SEQ ID NO:1155;

ff) the variable heavy chain set forth in SEQ ID NO:1151 or a sequence of amino acids that exhibits at least 85% sequence identity to SEQ ID NO:1151, and the variable light chain set forth in SEQ ID NO:1156 or a sequence of amino acids that exhibits at least 85% sequence identity to SEQ ID NO:1156;

gg) the variable heavy chain set forth in SEQ ID NO:1146 or a sequence of amino acids that exhibits at least 85% sequence identity to SEQ ID NO:1146, and the variable light chain set forth in SEQ ID NO:1156 or a sequence of amino acids that exhibits at least 85% sequence identity to SEQ ID NO:1156;

hh) the variable heavy chain set forth in SEQ ID NO:1149 or a sequence of amino acids that exhibits at least 85% sequence identity to SEQ ID NO:1149, and the variable light chain set forth in SEQ ID NO:1156 or a sequence of amino acids that exhibits at least 85% sequence identity to SEQ ID NO:1156;

ii) the variable heavy chain set forth in SEQ ID NO:1150 or a sequence of amino acids that exhibits at least 85% sequence identity to SEQ ID NO:1150, and the variable light chain set forth in SEQ ID NO:1157 or a sequence of amino acids that exhibits at least 85% sequence identity to SEQ ID NO:1157;

jj) the variable heavy chain set forth in SEQ ID NO:1152 or a sequence of amino acids that exhibits at least 85% sequence identity to SEQ ID NO:1152, and the variable light chain set forth in SEQ ID NO:1157 or a sequence of amino acids that exhibits at least 85% sequence identity to SEQ ID NO:1157;

kk) the variable heavy chain set forth in SEQ ID NO:1148 or a sequence of amino acids that exhibits at least 85% sequence identity to SEQ ID NO:1148, and the variable light chain set forth in SEQ ID NO:1157 or a sequence of amino acids that exhibits at least 85% sequence identity to SEQ ID NO:1157;

11) the variable heavy chain set forth in SEQ ID NO:1149 or a sequence of amino acids that exhibits at least 85% sequence identity to SEQ ID NO:1149, and the variable light chain set forth in SEQ ID NO:1157 or a sequence of amino acids that exhibits at least 85% sequence identity to SEQ ID NO:1157;

mm) the variable heavy chain set forth in SEQ ID NO:1150 or a sequence of amino acids that exhibits at least 85% sequence identity to SEQ ID NO:1150, and the variable light chain set forth in SEQ ID NO:1186 or a sequence of amino acids that exhibits at least 85% sequence identity to SEQ ID NO:1186;

nn) the variable heavy chain set forth in SEQ ID NO:1152 or a sequence of amino acids that exhibits at least 85% sequence identity to SEQ ID NO:1152, and the variable light chain set forth in SEQ ID NO:1186 or a sequence of amino acids that exhibits at least 85% sequence identity to SEQ ID NO:1186;

oo) the variable heavy chain set forth in SEQ ID NO:1148 or a sequence of amino acids that exhibits at least 85% sequence identity to SEQ ID NO:1148, and the variable light chain set forth in SEQ ID NO:1186 or a sequence of amino acids that exhibits at least 85% sequence identity to SEQ ID NO:1186;

pp) the variable heavy chain set forth in SEQ ID NO:1149 or a sequence of amino acids that exhibits at least 85% sequence identity to SEQ ID NO:1149, and the variable light chain set forth in SEQ ID NO:1186 or a sequence of amino acids that exhibits at least 85% sequence identity to SEQ ID NO:1186;

qq) the variable heavy chain set forth in SEQ ID NO:1150 or a sequence of amino acids that exhibits at least 85% sequence identity to SEQ ID NO:1150, and the variable light chain set forth in SEQ ID NO:1158 or a sequence of amino acids that exhibits at least 85% sequence identity to SEQ ID NO:1158;

rr) the variable heavy chain set forth in SEQ ID NO:1152 or a sequence of amino acids that exhibits at least 85% sequence identity to SEQ ID NO:1152, and the variable light chain set forth in SEQ ID NO:1159 or a sequence of amino acids that exhibits at least 85% sequence identity to SEQ ID NO:1159;

ss) the variable heavy chain set forth in SEQ ID NO:1146 or a sequence of amino acids that exhibits at least 85% sequence identity to SEQ ID NO:1146, and the variable light chain set forth in SEQ ID NO:1159 or a sequence of amino acids that exhibits at least 85% sequence identity to SEQ ID NO:1159;

tt) the variable heavy chain set forth in SEQ ID NO:1146 or a sequence of amino acids that exhibits at least 85% sequence identity to SEQ ID NO:1146, and the variable light chain set forth in SEQ ID NO:1157 or a sequence of amino acids that exhibits at least 85% sequence identity to SEQ ID NO:1157;

uu) the variable heavy chain set forth in SEQ ID NO:1146 or a sequence of amino acids that exhibits at least 85% sequence identity to SEQ ID NO:1146, and the variable light chain set forth in SEQ ID NO:1186 or a sequence of amino acids that exhibits at least 85% sequence identity to SEQ ID NO:1186; and

vv) the variable heavy chain set forth in SEQ ID NO:1118 or a sequence of amino acids that exhibits at least 85% sequence identity to SEQ ID NO:1118, and the variable light chain set forth in SEQ ID NO:4 or 10 or a sequence of amino acids that exhibits at least 85% sequence identity to SEQ ID NO:4 or 10.

33. The anti-EGFR antibody, or antigen-binding fragment thereof, of 31 or 32, wherein sequence identity is at least 86%, 87%, 88%, 89%, 90%, 91%, 92%, 93%, 94%, 95%, 96%, 97%, 98%, 98%, 99% or more.

34. The anti-EGFR antibody, or antigen-binding fragment thereof, of any of 1-33, wherein the antibody or antigen-binding fragment thereof is expressed in mammalian cells at a concentration of at least 1 mg/mL.

35. The anti-EGFR antibody, or antigen-binding fragment thereof, of any of 1-34 that is a modified anti-EGFR antibody or antigen-binding fragment thereof comprising an amino acid replacement(s) in a variable heavy chain, variable light chain or both of the unmodified antibody, wherein:

the unmodified anti-EGFR antibody is cetuximab, an antigen-binding fragment thereof or a variant thereof that does not comprise the amino acid replacement and specifically binds to EGFR.

36. A modified anti-EGFR antibody or antigen-binding fragment thereof, comprising an amino acid replacement(s) in a variable heavy chain, variable light chain or both of the unmodified antibody, wherein:

the unmodified anti-EGFR antibody is cetuximab, an antigen-binding fragment thereof or a variant thereof that does not comprise the amino acid replacement and specifically binds to EGFR;

the modified anti-EGFR antibody or fragment thereof exhibits a ratio of binding activity for EGFR at or about pH 6.0 to pH 6.5 compared to at or about pH 7.4 of at least 2.0, when measured under the same conditions except for the difference in pH; and

the modified anti-EGFR antibody exhibits less than 40% of the binding activity for EGFR at pH 7.4 compared to the unmodified antibody at pH 7.4 when measured under the same conditions, with the proviso that the modified anti-EGFR antibody or fragment thereof does not comprise:

a) a variable heavy chain comprising an amino acid replacement selected from among N31I, N31V, V50L, Y59E and T64N; or

b) a variable light chain comprising an amino acid replacement L4C.

37. The modified anti-EGFR antibody or fragment thereof of 36, wherein the modified anti-EGFR antibody exhibits at least 20% of the binding activity for EGFR at or about pH 6.0 to pH 6.5 compared to the unmodified antibody at pH 6.0 to pH 6.5 when measured under the same conditions.

38. The modified anti-EGFR antibody or fragment thereof of any of 35-37, wherein:

a) the variable heavy chain, or portion thereof, contains an amino acid replacement corresponding to an amino acid replacement selected from among V24E, V24I, V24L, S25C, S25H, S25R, S25A, S25D, S25G, S25M, S25Q, S25V, S25L, S28C, L29H, N31H, G54D, G54S, F63R, F63C, F63M, F63P, F63S, T64V, L67G, D72L, D72P, D72W, N73Q, K75H, K75G, K75P, K75W, S76I, S76V, Q77E, T100P, Y104D, Y104S, Y104V, Q111I, Q111V, with reference to amino acid positions set forth in SEQ ID NO:3, wherein:

corresponding amino acid positions are identified by alignment of the VH chain of the antibody with the VH chain set forth in SEQ ID NO:3; and

the portion thereof is sufficient to form an antigen binding site and contains the amino acid replacement; and/or

b) the modified variable light chain, or portion thereof, contains an amino acid replacement corresponding to an amino acid replacement selected from among L4F, L4V, T5P, R24G, with reference to amino acid positions set forth in SEQ ID NO:4, wherein:

corresponding amino acid positions are identified by alignment of the VL chain of the antibody with the VL chain set forth in SEQ ID NO:4; and

the portion thereof is sufficient to form an antigen binding site and contains the amino acid replacement.

39. The modified anti-EGFR antibody of any of 35-38, wherein the variable heavy chain, or portion thereof, contains the amino acid replacement Y104D.

40. The modified anti-EGFR antibody of 38 or 39, wherein:

the variable heavy chain, or portion thereof, contains a further amino acid replacement selected from among V24E, S25C, S25V, F27R, T30F, S53G, D72L, R97H, Y104D and Q111P.

41. A modified anti-EGFR antibody or antigen-binding fragment thereof, comprising at least two amino acid replacement, wherein:

the amino acid replacements are in a variable heavy (VH) chain, variable light (VL) chain or both of the unmodified antibody:

the unmodified anti-EGFR antibody is cetuximab, an antigen-binding fragment thereof or a variant thereof that does not comprise the amino acid replacement and specifically binds to EGFR;

the amino acid replacements in the VH chain corresponds to an amino acid replacement selected from among V24E, S25C, S25V, F27R, T30F, S53G, D72L, R97H, Y104D and Q111P, with reference to amino acid positions set forth in SEQ ID NO:3, wherein corresponding amino acid positions are identified by alignment of the VH chain of the antibody with the VH chain set forth in SEQ ID NO:3; and

the amino acid replacement in the VL chain corresponds to amino acid replacement I29S, with reference to the amino acid position set forth in SEQ ID NO:4; wherein corresponding amino acid positions are identified by alignment of the VL chain of the antibody with the VL chain set forth in SEQ ID NO:4.

42. The modified anti-EGFR antibody, or antigen-binding fragment thereof, of any of 39-41, wherein the amino acid replacements are HC-Y104D/ HC-Q111P; HC-S25C/ HC-Y104D; HC-Y104D/LC-I29S; HC-Y104D/HC-Q111P/LC-I29S; HC-S53G/HC-Y104D; HC-S53G/HC-Y104D/HC-Q111P; HC-S25V/HC-Y104D; HC-S25V/HC-Y104D/HC-Q111P; HC-S25V/HC-S53G/HC-Y104D; HC-S25V/HC-S53G/HC-Y104D/HC-Q111P; HC-T30F/HC-Y104D; HC-T30F/HC-Y104D/HC-Q111P; HC-T30F/HC-S53G/HC-Y104D; HC-T30F/HC-S53G/HC-Y104D/HC-Q111P; HC-D72L/HC-Y104D; HC-D72L/HC-Y104D/HC-Q111P; HC-S53G/ HC-D72L/HC-Y104D; or HC-S53G/HC-D72L/HC-Y104D/HC-Q111P.

43. The modified anti-EGFR antibody, or antigen-binding fragment thereof, of 40 or 41, wherein the amino acid replacements are HC-S25C/ HC-Q11IP; HC-V24E/ HC-F27R/ HC-R97H/ HC-Q111P; HC-S25C/LC-I29S; and HC-Q111P/LC-I29S.

44. The modified anti-EGFR antibody, or antigen-binding fragment thereof, of any of 41-43, wherein the modified anti-EGFR antibody or fragment thereof exhibits a ratio of binding activity for EGFR at or about pH 6.0 to pH 6.5 compared to at or about pH 7.4 of at least 1.1, 1.2, 1.3, 1.4, 1.5, 1.6, 1.7, 1.8, 1.9, 2.0, 2.5, 3.0, 4.0, 4.5, 5.0 or greater.

45. The modified anti-EGFR antibody, or antigen-binding fragment thereof, of 44, wherein the modified anti-EGFR antibody or fragment thereof exhibits a ratio of binding activity for EGFR at or about pH 6.0 to pH 6.5 compared to at or about pH 7.4 of at least 2.0.

46. The modified anti-EGFR antibody, or antigen-binding fragment thereof, of 45, wherein the amino acid replacements are HC-Y104D/ HC-Q11 IP; HC-S25C/ HC-Y104D; HC-S53G/HC-Y104D; HC-S53G/HC-Y104D/HC-Q111P; HC-S25V/HC-Y104D; HC-S25V/HC-Y104D/HC-Q111P; HC-S25V/HC-S53G/HC-Y104D; HC-S25V/HC-S53G/HC-Y104D/HC-Q111P; HC-T30F/HC-Y104D; HC-T30F/HC-Y104D/HC-Q111P; HC-T30F/HC-S53G/HC-Y104D; HC-T30F/HC-S53G/HC-Y104D/HC-Q111P; HC-D72L/HC-Y104D; HC-D72L/HC-Y104D/HC-Q111P; HC-S53G/ HC-D72L/HC-Y104D; or HC-S53G/HC-D72L/HC-Y104D/HC-Q111P.

47. The modified anti-EGFR antibody, or antigen-binding fragment thereof, of any of 35-46, wherein the unmodified cetuximab antibody, antigen-binding fragment thereof or variant thereof comprises a variable heavy chain set forth in SEQ ID NO:3 and a variable light chain set forth in SEQ ID NO:4 or 10.

48. The modified anti-EGFR antibody, or antigen-binding fragment thereof, of any of 35-47, wherein the unmodified cetuximab antibody, antigen-binding fragment thereof or variant thereof comprises:

a heavy chain having a sequence of amino acids set forth in SEQ ID NO:1 or a sequence of amino acids that exhibits at least 85% sequence identity to the sequence of amino acids set forth in SEQ ID NO:1 and a light chain having a sequence of amino acids set forth SEQ ID NO:2 or a sequence of amino acids that exhibits at least 85% sequence identity to the sequence of amino acids set forth in SEQ ID NO:2; or

a heavy chain having a having a sequence of amino acids set forth in SEQ ID NO: 8 or a sequence of amino acids that exhibits at least 85% sequence identity to the sequence of amino acids set forth in SEQ ID NO:8 and a light chain having a sequence of amino acids set forth SEQ ID NO:9 or a sequence of amino acids that exhibits at least 85% sequence identity to the sequence of amino acids set forth in SEQ ID NO:9.

49. The modified anti-EGFR antibody, or antigen-binding fragment thereof, of any of 35-48, wherein the unmodified cetuximab is a variant that is humanized.

50. The modified anti-EGFR antibody, or antigen-binding fragment thereof, of 49, wherein the unmodified cetuximab comprises a variable heavy chain set forth in SEQ ID NO:28 and a variable light chain set forth in SEQ ID NO:29.

51. The modified anti-EGFR antibody, or antigen-binding fragment thereof, of any of 35-47, wherein the unmodified cetuximab, antigen-binding fragment thereof or variant thereof is an antigen-binding fragment thereof and the antigen-binding fragment is selected from among a Fab, Fab', F(ab')$_2$, single-chain Fv (scFv), Fv, dsFv, diabody, Fd and Fd' fragments.

52. The modified anti-EGFR antibody, or antigen-binding fragment thereof, of 51, wherein the unmodified cetuximab is a Fab fragment comprising a heavy chain having a sequence of amino acids set forth in SEQ ID NO:5 or a sequence of amino acids that exhibits at least 75% sequence identity to SEQ ID NO:5 and a light chain having a sequence of amino acids set forth in SEQ ID NO:2 or a sequence of amino acids that exhibits at least 75% sequence identity to a sequence of amino acids set forth in SEQ ID NO:2.

53. The modified anti-EGFR antibody, or antigen-binding fragment thereof, of any of 36-52, comprising:

a) a variable heavy (VH) chain comprising the sequence of amino acids set forth in SEQ ID NO:495, 1062, 1112, 1114, 1115, 1116, 1117, 1118, 1119, 1124, 1125, 1126, 1127, 1128, 1129, 1130 or 1131, or a sequence of amino acids that exhibits at least 85% sequence identity to any of SEQ ID NOS: 495, 1062, 1112, 1114, 1115, 1116, 1117, 1118, 1119, 1124, 1125, 1126, 1127, 1128, 1129, 1130 or 1131; and

b) a variable light (VL) chain comprising the sequence of amino acids set forth in SEQ ID NO:4 or 10, or a sequence of amino acids that exhibits at least 85% sequence identity to SEQ ID NO:4 or 10.

54. The modified anti-EGFR antibody, or antigen-binding fragment thereof, of any of 35-53, comprising:

a) a variable heavy (VH) chain comprising the sequence of amino acids set forth in SEQ ID NO:1062 or 1125, or a sequence of amino acids that exhibits at least 85% sequence identity to any of SEQ ID NOS: 1062 or 1125; and

b) a variable light (VL) chain comprising the sequence of amino acids set forth in SEQ ID NO:4 or 10, or a sequence of amino acids that exhibits at least 85% sequence identity to SEQ ID NO:4 or 10.

55. The anti-EGFR antibody, or antigen-binding fragment thereof, of any of 1-54 that is humanized.

56. The anti-EGFR antibody, or antigen-binding fragment thereof, of 55, wherein:

the variable heavy chain exhibits less than 85% sequence identity to the variable heavy chain set forth in SEQ ID NO:3 and greater than 65% sequence identity to the variable heavy chain set forth in SEQ ID NO:3; and

the variable light chain exhibits less than 85% sequence identity to the variable light chain set forth in SEQ ID NO:4 and greater than 65% sequence identity to the variable light chain set forth in SEQ ID NO:4.

57. The anti-EGFR antibody, or antigen-binding fragment thereof, of 55 or 56, comprising a sequence of amino acids selected from among:

a) the variable heavy chain set forth in SEQ ID NO:1134 or a sequence of amino acids that exhibits at least 85% sequence identity to SEQ ID NO:1134, and the variable light chain set forth in SEQ ID NO:1138 or a sequence of amino acids that exhibits at least 85% sequence identity to SEQ ID NO:1138;

b) the variable heavy chain set forth in SEQ ID NO:1134 or a sequence of amino acids that exhibits at least 85% sequence identity to SEQ ID NO:1134, and the variable light chain set forth in SEQ ID NO:1139 or a sequence of amino acids that exhibits at least 85% sequence identity to SEQ ID NO:1139;

c) the variable heavy chain set forth in SEQ ID NO:1135 or a sequence of amino acids that exhibits at least 85% sequence identity to SEQ ID NO:1135, and the variable light chain set forth in SEQ ID NO:1138 or a sequence of amino acids that exhibits at least 85% sequence identity to SEQ ID NO:1138;

d) the variable heavy chain set forth in SEQ ID NO:1134 or a sequence of amino acids that exhibits at least 85% sequence identity to SEQ ID NO:1134, and the variable light chain set forth in SEQ ID NO:1140 or a sequence of amino acids that exhibits at least 85% sequence identity to SEQ ID NO:1140;

e) the variable heavy chain set forth in SEQ ID NO:1134 or a sequence of amino acids that exhibits at least 85% sequence identity to SEQ ID NO:1134, and the variable light chain set forth in SEQ ID NO:1141 or a sequence of amino acids that exhibits at least 85% sequence identity to SEQ ID NO:1141;

f) the variable heavy chain set forth in SEQ ID NO:1134 or a sequence of amino acids that exhibits at least 85% sequence identity to SEQ ID NO:1134, and the variable light chain set forth in SEQ ID NO:1142 or a sequence of amino acids that exhibits at least 85% sequence identity to SEQ ID NO:1142;

g) the variable heavy chain set forth in SEQ ID NO:1135 or a sequence of amino acids that exhibits at least 85% sequence identity to SEQ ID NO:1135, and the variable light chain set forth in SEQ ID NO:1142 or a sequence of amino acids that exhibits at least 85% sequence identity to SEQ ID NO:1142;

h) the variable heavy chain set forth in SEQ ID NO:1134 or a sequence of amino acids that exhibits at least 85% sequence identity to SEQ ID NO:1134, and the variable light chain set forth in SEQ ID NO:1143 or a sequence of amino acids that exhibits at least 85% sequence identity to SEQ ID NO:1143;

i) the variable heavy chain set forth in SEQ ID NO:1136 or a sequence of amino acids that exhibits at least 85% sequence identity to SEQ ID NO:1136, and the variable light chain set forth in SEQ ID NO:1142 or a sequence of amino acids that exhibits at least 85% sequence identity to SEQ ID NO:1142;

j) the variable heavy chain set forth in SEQ ID NO:1137 or a sequence of amino acids that exhibits at least 85% sequence identity to SEQ ID NO:1137, and the variable light chain set forth in SEQ ID NO:1144 or a sequence of amino acids that exhibits at least 85% sequence identity to SEQ ID NO:1144;

k) the variable heavy chain set forth in SEQ ID NO:1136 or a sequence of amino acids that exhibits at least 85% sequence identity to SEQ ID NO:1136, and the variable light chain set forth in SEQ ID NO:1144 or a sequence of amino acids that exhibits at least 85% sequence identity to SEQ ID NO:1144;

1) the variable heavy chain set forth in SEQ ID NO:1137 or a sequence of amino acids that exhibits at least 85% sequence identity to SEQ ID NO:1137, and the variable light chain set forth in SEQ ID NO:1145 or a sequence of amino acids that exhibits at least 85% sequence identity to SEQ ID NO:1145;

m) the variable heavy chain set forth in SEQ ID NO:1136 or a sequence of amino acids that exhibits at least 85% sequence identity to SEQ ID NO:1136, and the variable light chain set forth in SEQ ID NO:1145 or a sequence of amino acids that exhibits at least 85% sequence identity to SEQ ID NO:1145;

n) the variable heavy chain set forth in SEQ ID NO:1146 or a sequence of amino acids that exhibits at least 85% sequence identity to SEQ ID NO:1146, and the variable light chain set forth in SEQ ID NO:1153 or a sequence of amino acids that exhibits at least 85% sequence identity to SEQ ID NO:1153;

o) the variable heavy chain set forth in SEQ ID NO:1147 or a sequence of amino acids that exhibits at least 85% sequence identity to SEQ ID NO:1147, and the variable light chain set forth in SEQ ID NO:1153 or a sequence of amino acids that exhibits at least 85% sequence identity to SEQ ID NO:1153;

p) the variable heavy chain set forth in SEQ ID NO:1148 or a sequence of amino acids that exhibits at least 85% sequence identity to SEQ ID NO:1148, and the variable light chain set forth in SEQ ID NO:1154 or a sequence of amino acids that exhibits at least 85% sequence identity to SEQ ID NO:1154;

q) the variable heavy chain set forth in SEQ ID NO:1149 or a sequence of amino acids that exhibits at least 85% sequence identity to SEQ ID NO:1149, and the variable light chain set forth in SEQ ID NO:1154 or a sequence of amino acids that exhibits at least 85% sequence identity to SEQ ID NO:1154;

r) the variable heavy chain set forth in SEQ ID NO:1150 or a sequence of amino acids that exhibits at least 85% sequence identity to SEQ ID NO:1150, and the variable light chain set forth in SEQ ID NO:1155 or a sequence of amino acids that exhibits at least 85% sequence identity to SEQ ID NO:1155;

s) the variable heavy chain set forth in SEQ ID NO:1151 or a sequence of amino acids that exhibits at least 85% sequence identity to SEQ ID NO:1151, and the variable light chain set forth in SEQ ID NO:1156 or a sequence of amino acids that exhibits at least 85% sequence identity to SEQ ID NO:1156;

t) the variable heavy chain set forth in SEQ ID NO:1146 or a sequence of amino acids that exhibits at least 85% sequence identity to SEQ ID NO:1146, and the variable light chain set forth in SEQ ID NO:1156 or a sequence of amino acids that exhibits at least 85% sequence identity to SEQ ID NO:1156;

u) the variable heavy chain set forth in SEQ ID NO:1149 or a sequence of amino acids that exhibits at least 85% sequence identity to SEQ ID NO:1149, and the variable light chain set forth in SEQ ID NO:1156 or a sequence of amino acids that exhibits at least 85% sequence identity to SEQ ID NO:1156;

v) the variable heavy chain set forth in SEQ ID NO:1150 or a sequence of amino acids that exhibits at least 85% sequence identity to SEQ ID NO:1150, and the variable light chain set forth in SEQ ID NO:1157 or a sequence of amino acids that exhibits at least 85% sequence identity to SEQ ID NO:1157;

w) the variable heavy chain set forth in SEQ ID NO:1152 or a sequence of amino acids that exhibits at least 85% sequence identity to SEQ ID NO:1152, and the variable light chain set forth in SEQ ID NO:1157 or a sequence of amino acids that exhibits at least 85% sequence identity to SEQ ID NO:1157;

x) the variable heavy chain set forth in SEQ ID NO:1148 or a sequence of amino acids that exhibits at least 85% sequence identity to SEQ ID NO:1148, and the variable light chain set forth in SEQ ID NO:1157 or a sequence of amino acids that exhibits at least 85% sequence identity to SEQ ID NO:1157;

y) the variable heavy chain set forth in SEQ ID NO:1149 or a sequence of amino acids that exhibits at least 85% sequence identity to SEQ ID NO:1149, and the variable light chain set forth in SEQ ID NO:1157 or a sequence of amino acids that exhibits at least 85% sequence identity to SEQ ID NO:1157;

z) the variable heavy chain set forth in SEQ ID NO:1150 or a sequence of amino acids that exhibits at least 85% sequence identity to SEQ ID NO:1150, and the variable light chain set forth in SEQ ID NO:1186 or a sequence of amino acids that exhibits at least 85% sequence identity to SEQ ID NO:1186;

aa) the variable heavy chain set forth in SEQ ID NO:1152 or a sequence of amino acids that exhibits at least 85% sequence identity to SEQ ID NO:1152, and the variable light chain set forth in SEQ ID NO:1186 or a sequence of amino acids that exhibits at least 85% sequence identity to SEQ ID NO:1186;

bb) the variable heavy chain set forth in SEQ ID NO:1148 or a sequence of amino acids that exhibits at least 85% sequence identity to SEQ ID NO:1148, and the variable light chain set forth in SEQ ID NO:1186 or a sequence of amino acids that exhibits at least 85% sequence identity to SEQ ID NO:1186;

cc) the variable heavy chain set forth in SEQ ID NO:1149 or a sequence of amino acids that exhibits at least 85% sequence identity to SEQ ID NO:1149, and the variable light chain set forth in SEQ ID NO:1186 or a sequence of amino acids that exhibits at least 85% sequence identity to SEQ ID NO:1186;

dd) the variable heavy chain set forth in SEQ ID NO:1150 or a sequence of amino acids that exhibits at least 85% sequence identity to SEQ ID NO:1150, and the variable light chain set forth in SEQ ID NO:1158 or a sequence of amino acids that exhibits at least 85% sequence identity to SEQ ID NO:1158;

ee) the variable heavy chain set forth in SEQ ID NO:1152 or a sequence of amino acids that exhibits at least 85% sequence identity to SEQ ID NO:1152, and the variable light chain set forth in SEQ ID NO:1159 or a sequence of amino acids that exhibits at least 85% sequence identity to SEQ ID NO:1159;

ff) the variable heavy chain set forth in SEQ ID NO:1146 or a sequence of amino acids that exhibits at least 85% sequence identity to SEQ ID NO:1146, and the variable light chain set forth in SEQ ID NO:1159 or a sequence of amino acids that exhibits at least 85% sequence identity to SEQ ID NO:1159;

gg) the variable heavy chain set forth in SEQ ID NO:1146 or a sequence of amino acids that exhibits at least 85% sequence identity to SEQ ID NO:1146, and the variable light chain set forth in SEQ ID NO:1157 or a sequence of amino acids that exhibits at least 85% sequence identity to SEQ ID NO: 1157; and

hh) the variable heavy chain set forth in SEQ ID NO:1146 or a sequence of amino acids that exhibits at least 85% sequence identity to SEQ ID NO:1146, and the variable light chain set forth in SEQ ID NO:1186 or a sequence of amino acids that exhibits at least 85% sequence identity to SEQ ID NO:1186.

58. The anti-EGFR antibody, or antigen-binding fragment thereof, or 53, 54 or 57, wherein the sequence identity is at least 86%, 87%, 88%, 89%, 90%, 91%, 92%, 93%, 94%, 95%, 96%, 97%, 98%, 99% or more thereto.

59. The anti-EGFR antibody or antigen binding fragment of any of 1-58 that is a full-length IgG antibody.

60. The anti-EGFR antibody or antigen binding fragment of 59, comprising:

a heavy chain constant region set forth in any of SEQ ID NOS:22-25, 1069 and 1070, or a variant thereof that exhibits at least 85% sequence identity thereto; and

a light chain constant region set forth in any of SEQ ID NOS: 1072-1073, or a variant thereof that exhibits at least 85% sequence identity thereto.

61. The anti-EGFR antibody or antigen binding fragment of any of 1-58 that is an antigen-binding fragment selected from among a Fab, Fab', F(ab')$_2$, single-chain Fv (scFv), Fv, dsFv, diabody, Fd and Fd' fragments.

62. The anti-EGFR antibody, or antigen-binding fragment, of 61 that is a Fab or scFv.

63. The anti-EGFR antibody, or antigen-binding fragment, of any of 1-62 that is isolated or purified.

64. A conjugate, comprising an anti-EGFR antibody, or antigen-binding fragment thereof, of any of 1-63 linked directly or indirectly to a targeted agent.

65. The conjugate of 64, comprising the following components:

(Ab), (L)$_q$, and (targeted agent)$_m$, wherein:

Ab is the anti-EGFR antibody or antigen-binding fragment thereof that binds to EGFR;
L is a linker for linking the Ab to the targeted agent;
m is at least 1;
q is 0 or more as long as the resulting conjugate binds to the EGFR;
the resulting conjugate binds to the EGFR.

66. The conjugate of 65, wherein m is 1 to 8 and q is 0 to 8.

67. The conjugate of any of 63-66, wherein the targeted agent is a protein, peptide, nucleic acid or small molecule.

68. The conjugate of any of 63-67, wherein the targeted agent is a therapeutic moiety.

69. The conjugate of 68, wherein the therapeutic moiety is a cytotoxic moiety, a radioisotope, a chemotherapeutic agent, a lytic peptide or a cytokine.

70. The conjugate of 69, wherein the therapeutic moiety is selected from among taxol; cytochalasin B; gramicidin D; ethidium bromide; emetine; mitomycin; etoposide; tenoposide; vincristine; vinblastine; colchicin; doxorubicin; daunorubicin; dihydroxy anthracin dione; maytansine or an analog or derivative thereof; an auristatin or a functional peptide analog or derivative thereof; dolastatin 10 or 15 or an analogue thereof; irinotecan or an analogue thereof; mitoxantrone; mithramycin; actinomycin D; 1-dehydrotestosterone; a glucocorticoid; procaine; tetracaine; lidocaine; propranolol; puromycin; calicheamicin or an analog or derivative thereof; an antimetabolite; an alkylating agent; a platinum derivative; duocarmycin A, duocarmycin SA, rachelmycin (CC-1065), or an analog or derivative thereof; an antibiotic; pyrrolo[2,1-c][1, 4]-benzodiazepines (PDB); a toxin; ribonuclease (RNase); DNase I, Staphylococcal enterotoxin A; and pokeweed antiviral protein.

71. The conjugate of 69 or 70, wherein the therapeutic moiety is a maytansine derivative that is a maytansinoids selected from among ansamitocin or mertansine (DM1).

72. The conjugate of 69 or 70, wherein the therapeutic moiety is an auristatin or a functional peptide analog or derivative thereof that is monomethyl auristatin E (MMAE) or F (MMAF).

73. The conjugate of 69 or 70, wherein the therapeutic moiety is an antimetabolite selected from among methotrexate, 6 mercaptopurine, 6 thioguanine, cytarabine, fludarabin, 5 fluorouracil, decarbazine, hydroxyurea, asparaginase, gemcitabine, and cladribine.

74. The conjugate of 69 or 70, wherein the therapeutic moiety is an alkylating agent selected from among mechlorethamine, thioepa, chlorambucil, melphalan, carmustine (BSNU), lomustine (CCNU), cyclophosphamide, busulfan, dibromomannitol, streptozotocin, dacarbazine (DTIC), procarbazine and mitomycin C.

75. The conjugate of 69 or 70, wherein the therapeutic moiety is a platinum derivative that is cisplatin or carboplatin.

76. The conjugate of 69 or 70, wherein the therapeutic moiety is an antibiotic selected from among dactinomycin, bleomycin, daunorubicin, doxorubicin, idarubicin, mithramycin, mitomycin, mitoxantrone, plicamycin and anthramycin (AMC).

77. The conjugate of 69 or 70, wherein the therapeutic moiety is a toxin selected from among a diphtheria toxin and active fragments thereof and hybrid molecules, a ricin toxin, cholera toxin, a Shiga-like toxin, LT toxin, C3 toxin, Shiga toxin, pertussis toxin, tetanus toxin, soybean Bowman-Birk protease inhibitor, Pseudomonas exotoxin, alorin, saporin, modeccin, gelanin, abrin A chain, modeccin A chain, alpha-sarcin, Aleurites fordii proteins, dianthin proteins, Phytolacca americana proteins, momordica charantia inhibitor, curcin, crotin, , gelonin, mitogellin, restrictocin, phenomycin, and enomycin toxins.

78. The conjugate of any of 64-77, wherein the antibody and targeted agent are linked directly.

79. The conjugate of any of 64-77, wherein the antibody and targeted agent are joined via a linker.

80. The conjugate of 79, wherein the linker comprises a peptide or a polypeptide or is a chemical linker.

81. The conjugate of 79 or 80, wherein the linker is a cleavable linker or a non-cleavable linker.

82. The conjugate of any of 79-81, wherein the linker is conjugated to one or more free thiols on the antibody.

83. The conjugate of any of 79-81, wherein the linker is conjugated to one o more primary amines.

84. A nucleic acid molecule(s), comprising a sequence of nucleotides encoding an anti-EGFR antibody, or antigen-binding fragment thereof, of any of 1-63.

85. A nucleic acid molecule, comprising a sequence of nucleotides encoding the heavy chain of any of the anti-EGFR antibody, or antigen-binding fragment thereof, of any of 1-63.

86. A nucleic acid molecule, comprising a sequence of nucleotides encoding the light chain of any of the anti-EGFR antibody, or antigen-binding fragment thereof, of any of 1-63.

87. A vector, comprising the nucleic acid molecule(s) of any of 84-86.

88. A cell, comprising a vector or vectors of 87.

89. The cell of 88, wherein the cell is a prokaryotic or eukaryotic cell.

90. A method of making an anti-EGFR antibody, or antigen-binding fragment thereof, comprising expressing the heavy chain or light chain encoded from a vector or vectors of 87 encoding the heavy chain and the light chain in a suitable host cells and recovering the antibody.

91. The method of 90, wherein:

the encoded antibody, or antigen-bind fragment thereof are any of 26 and 48-55; and
greater than 1 mg/mL antibody is produced by the host cell.

92. A combination, comprising:

an anti-EGFR antibody or antigen-binding fragment of any of 1-63 or a conjugate of any of 64-83; and
a chemotherapeutic agent or anti-cancer agent.

93. The combination of 92, wherein the chemotherapeutic agent is selected from among alkylating agents, nitrosoureas, topoisomerase inhibitors, and antibodies.

94. The combination of 92, wherein the chemotherapeutic agent is selected from among irinotecan, oxaliplatin, 5-fluorouracil (5-FU), Xeloda, Camptosar, Eloxatin, Adriamycin, paclitaxel, docetaxel, Cisplatin, gemcitabine and carboplatin.

95. The combination of any of 92-94, wherein the chemotherapeutic agent is an additional anti-EGFR antibody or antigen-binding fragment thereof that differs from the first antibody.

96. The combination of 95, wherein the additional anti-EGFR antibody is selected from among cetuximab, panitumumab, nimotuzumab, and antigen-binding fragments thereof or variants thereof.

97. A kit comprising the antibody or antigen-binding fragment of any of 1-63, conjugates of any of 64-83 or the combination of any of 92-96, in one or more containers, and instructions for use.

98. A pharmaceutical composition comprising:

an anti-EGFR antibody or antigen-binding fragment of any of 1-63, conjugate of any of 64-83 or the combination of any of 92-96; and
a pharmaceutically acceptable carrier or excipient.

99. The pharmaceutical composition of 98 that is formulated as a gel, ointment, liquid, suspension, aerosol, tablet, pill, powder or lyophile.

100. The pharmaceutical composition of 98 or 99 that is formulated for systemic, parenteral, topical, oral, mucosal, intranasal, subcutaneous, aerosolized, intravenous, bronchial, pulmonary, vaginal, vulvovaginal, esophageal, or oroesophageal administration.

101. The pharmaceutical composition of any of 98-100 that is formulated for single dosage administration.

102. The pharmaceutical composition of any of 98-100 that is formulated for multiple dosage administration.

103. The pharmaceutical composition of any of 98-102 that is a sustained release formulation.

104. A method of treating a condition responsive to treatment with an anti-EGFR antibody in a subject, comprising administering to the subject a pharmaceutically effective amount of the pharmaceutical composition of any of 98-103.

105. The method of 104, wherein the condition responsive to treatment with an anti-EGFR antibody is a tumor, cancer or metastasis.

106. The method of 105, wherein the tumor expressess EGFR.

107. The method of 105 or 106, wherein the tumor is a solid tumor.

108. The method of any of any of 104-107, wherein the condition responsive to treatment with an anti-EGFR antibody is head and neck cancer, non-small cell lung cancer or colorectal cancer.

109. The method of any of 104-108, wherein the subject has a tumor that does not comprise a marker that confers resistance to anti-EGFR therapy.

110. The method of 109, wherein the marker is a mutation in *KRAS, NRAS* or *BRAF*.

111. The method of any of 104-108, wherein the subject has a KRAS mutation-negative epidermal growth factor receptor (EGFR)-expressing colorectal cancer.

112. The method of any of 104-111, wherein the subject is a mammal.

113. The method of any of 104-112, wherein the subject is a human.

114. The method of any of 104-113, wherein the pharmaceutical composition is administered topically, parenterally, locally, or systemically.

115. The method of any of 104-114, wherein the pharmaceutical composition is administered intranasally, intramuscularly, intradermally, intraperitoneally, intravenously, subcutaneously, orally, or by pulmonary administration.

116. The method of any of 104-115, further comprising administration of one or more anticancer agents or treatments

117. The method of 116, wherein the anticancer agent or treatment is selected from among irinotecan, oxaliplatin, 5-fluorouracil (5-FU), Xeloda, Camptosar, Eloxatin, Adriamycin, paclitaxel, docetaxel, Cisplatin, gemcitabine, carboplatin and radiation.

118. The method of any of 104-117, further comprising administering one or more additional anti-EGFR antibodies or antigen-binding fragments thereof.

119. The method of 118, wherein the one or more additional anti-EGFR antibodies are selected from among cetuximab, panitumumab, nimotuzumab, and antigen-binding fragments thereof.

120. The method of any of 116-119, wherein the pharmaceutical composition and the anticancer agent are formulated as a single composition or as separate compositions.

121. The method of any of 116-120, wherein the pharmaceutical composition and the anticancer agent are administered sequentially, simultaneously or intermittently.

122. The method of any of 104-121, wherein the antibody, or antigen-binding fragment thereof, is administered at a dosage of about or 0.1 mg/kg to 100 mg/kg, 0.5 mg/kg to 50 mg/kg, 5 mg/kg to 50 mg/kg, 5 1 mg/kg to 20 mg/kg, 1 mg/kg to 100 mg/kg, 10 mg/kg to 80 mg/kg, or 50 mg/kg to 100 mg/kg.

123. The method of any of 104-122, wherein the antibody, or antigen-binding fragment thereof, is administered at a dosage of about or 0.01 mg/m$^2$ to about or 800 mg/m$^2$.

124. The method of any of 104-123, wherein the antibody, or antigen-binding fragment thereof, is administered at a dosage of at least or at least about or about or 0.01 mg/m$^2$, 0.1 mg/m$^2$, 0.5 mg/m$^2$, 1 mg/m$^2$, 5 mg/m$^2$, 10 mg/m$^2$, 15 mg/m$^2$, 20 mg/m$^2$, 25 mg/m$^2$, 30 mg/m$^2$, 35 mg/m$^2$, 40 mg/m$^2$, 45 mg/m$^2$, 50 mg/m$^2$, 100 mg/m$^2$, 150 mg/m$^2$, 200 mg/m$^2$, 250 mg/m$^2$, 300 mg/m$^2$, 400 mg/ m$^2$, 500 mg/ m$^2$, 600 mg/ m$^2$ or 700 mg/ m$^2$.

125. A pharmaceutical composition of any of 89-103 formulated as a medicament for treating a condition responsive to treatment with an anti-EGFR antibody in a subject.

126. Use of a pharmaceutical composition of any of 89-103 for treating a condition responsive to treatment with an anti-EGFR antibody in a subject.

127. The pharmaceutical composition of 125 or the use of 126, wherein the condition responsive to treatment with an anti-EGFR antibody is a tumor, cancer or metastasis.

128. The pharmaceutical composition or use of 127, wherein the tumor expressess EGFR.

129. The pharmaceutical composition or use of 127 or 128, wherein the tumor is a solid tumor.

130. The pharmaceutical composition or use of any of 126-129, wherein the condition responsive to treatment with an anti-EGFR antibody is head and neck cancer, non-small cell lung cancer or colorectal cancer.

**BRIEF DESCRIPTION OF THE FIGURES**

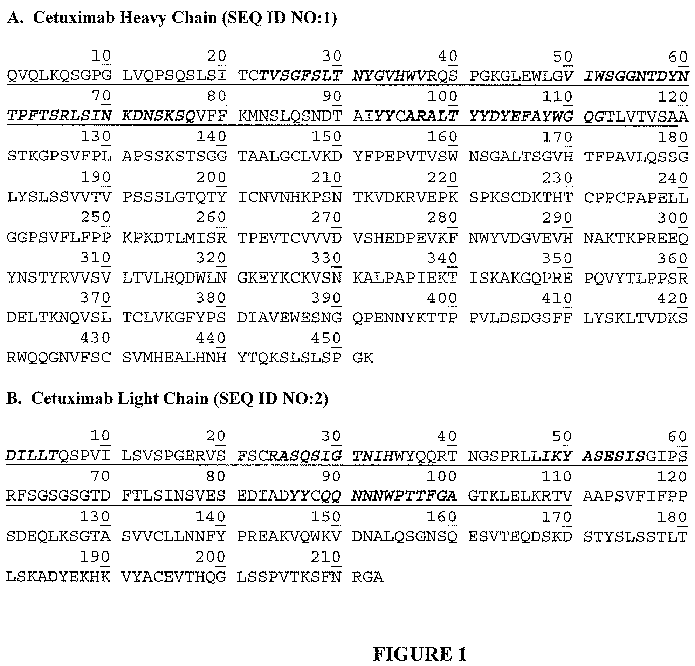

**FIGURE 1. Sequence of monoclonal antibody cetuximab (Erbitux®).** Figure 1 depicts the sequence of cetuximab (SEQ ID NO:1 and 2). **FIGURE 1A** depicts the sequence of the heavy chain. **FIGURE 1B** depicts the sequence of the light chain. The variable chains are underlined and the residues selected for modification are in boldface, italic type.

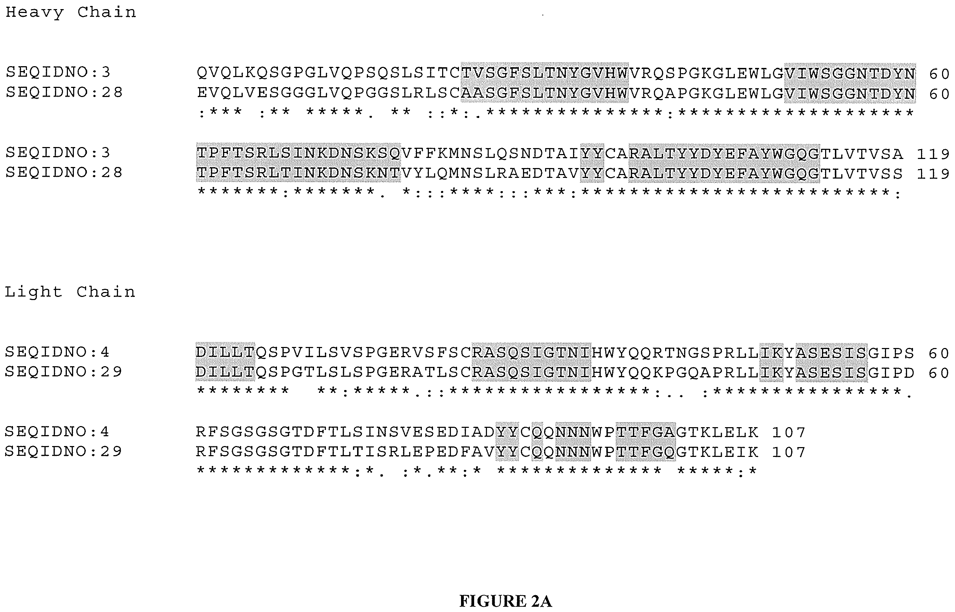

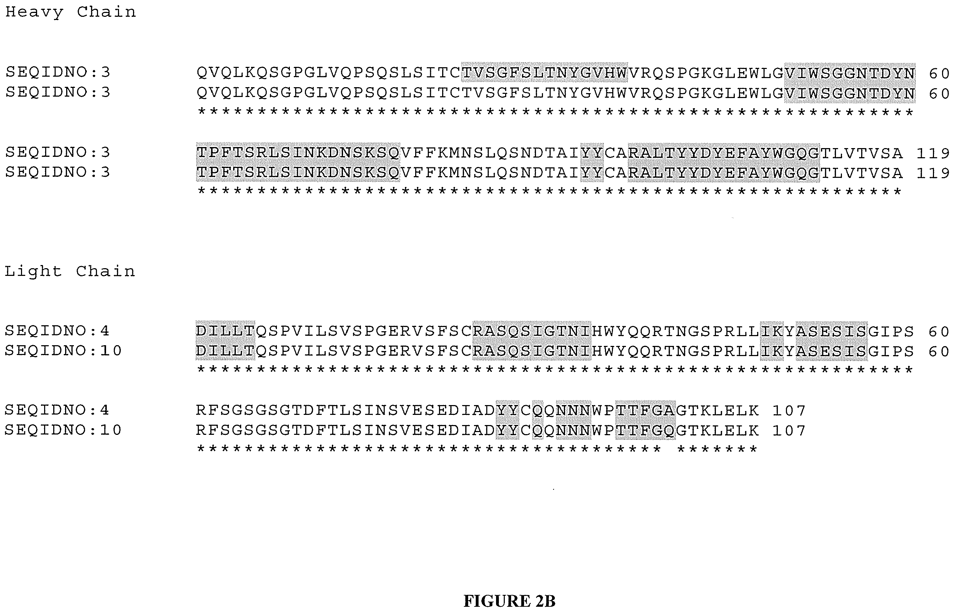

**FIGURE 2. Alignments of anti-EGFR antibodies.** Figure 2 depicts exemplary alignments of the cetuximab heavy and light chains with other anti-EGFR antibodies. A "*" means that the aligned residues are identical, a ":" means that aligned residues are not identical, but are similar and contain conservative amino acids residues at the aligned position, and a "." means that the aligned residues are similar and contain semi-conservative amino acid residues at the aligned position. Exemplary, non-limiting, corresponding positions for amino acid replacements are indicated by highlighting. For example, **Figure 2A** depicts the alignment of the cetuximab heavy chain variable region (V$_H$; SEQ ID NO:3 and light chain variable region (V$_L$; SEQ ID NO:4) with Hu225, V$_H$ set forth in SEQ ID NO:28 and V$_L$ set forth in SEQ ID NO:29. **Figure 2B** depicts the alignment of the cetuximab heavy chain variable region (V$_H$; SEQ ID NO:3 and light chain variable region (V$_L$; SEQ ID NO:4) with a reference anti-EGFR antibody, V$_H$ set forth in SEQ ID NO:3 and V$_L$ set forth in SEQ ID NO:10.

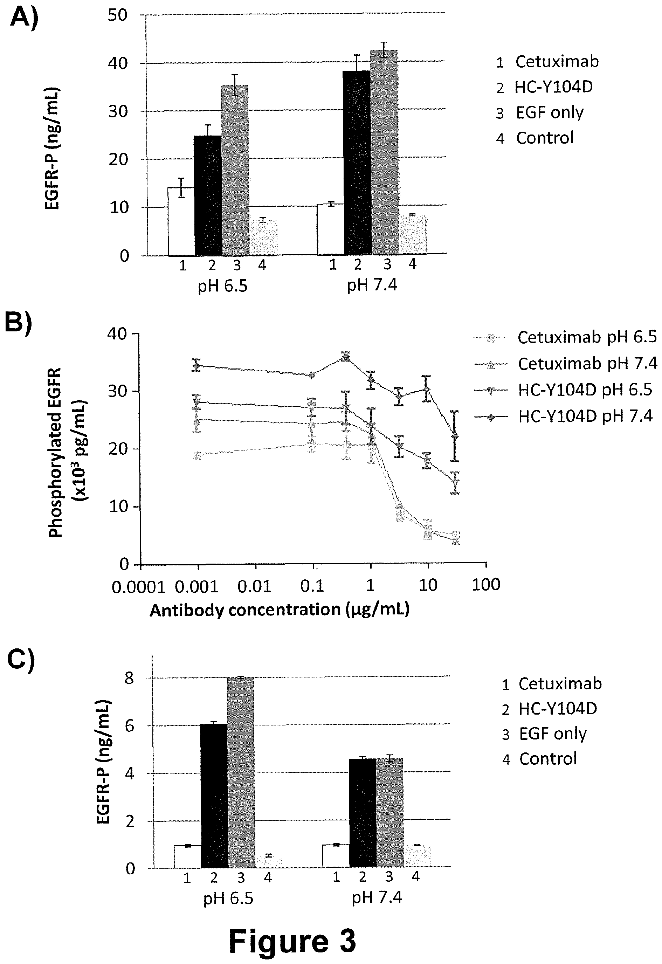

**FIGURE 3. Inhibition of EGF antigen induced phosphorylation of EGFR.** Figure 3 depicts inhibition of EGFR phosphorylation by Cetuximab and the HC-Y104D modified anti-EGFR antibody. **Figure 3A** depicts inhibition of EGF-induced phosphorylation of A431 cells. **Figure 3B** depicts the dose-dependent inhibitory effects with the concentration of phosphorylated EGFR plotted against the concentration of antibody (Cetuximab or HC-Y104D anti-EGFR antibody). **Figure 3C** depicts inhibition of EGF-induced phosphorylation of neonatal Keratinocytes.

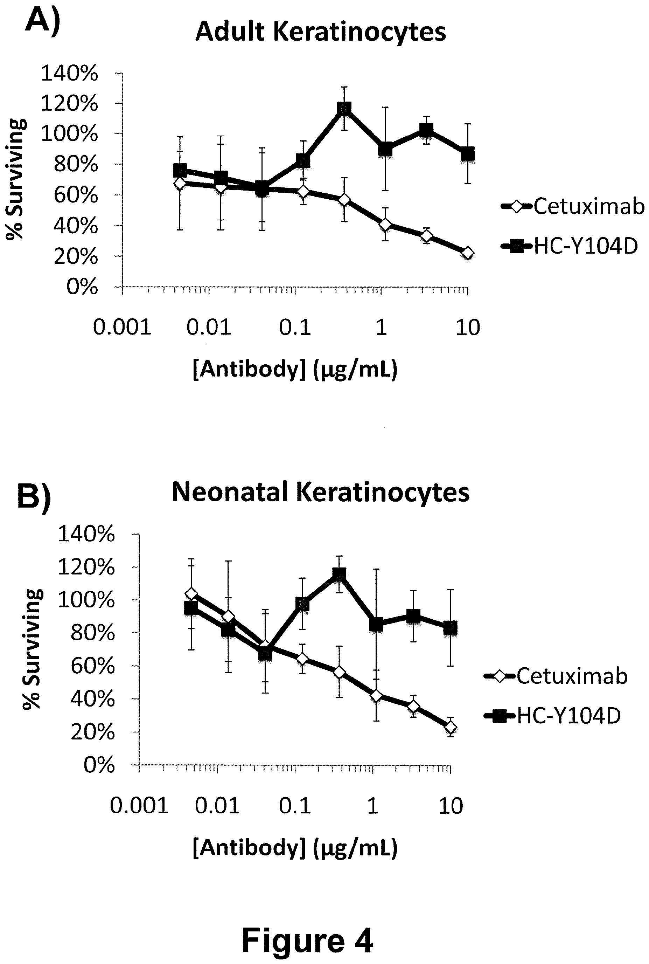

**FIGURE 4. Cell growth inhibition of Human adult keratinocytes or Human neonatal keratinocytes in the presence of Cetuximab or modified HC-Y104D anti-EGFR antibody.** Figure 4 depicts the growth of Human adult keratinocytes or Human neonatal keratinocytes with Cetuximab or HC-Y104D modified anti-EGFR antibody. **Figure**

**4A** depicts growth of Human adult keratinocytes with Cetuximab or HC-Y104D modified anti-EGFR antibody. **Figure 4B** depicts growth of Human neonatal Keratinocytes with Cetuximab or HC-Y104D modified anti-EGFR antibody.

**FIGURE 5. *In vivo* animal model of administered Cetuximab or modified HC-Y104D anti-EGFR antibody.** Figure 5 depicts inhibition of tumor growth in a mouse xenograft tumor model by Cetuximab and the HC-Y104D modified anti-EGFR antibody.

**FIGURE 6. Difference in tumor and skin binding between Cetuximab and modified HC-Y104D anti-EGFR antibody.** Figure 6 depicts the ratio of DL755-labeled Cetuximab and modified HC-Y104D antibody binding of xenograft tumors to human skin grafts over a 7-day time course, following administration of a single i.v. dose of antibody.

**DETAILED DESCRIPTION**

**Outline**

**A. DEFINITIONS**
**B. EGFR AND ANTI-EGFR ANTIBODIES**

    **1. EGFR**
    **2. Anti-EGFR Antibodies and Side Effects**
    **3. Cetuximab**

        **a. Structure**
        **b. Function**

**C. MODIFIED ANTI-EGFR ANTIBODIES AND CONDITIONALLY ACTIVE ANTI-EGFR ANTIBODIES**

    **1. Modified Anti-EGFR Antibodies**

        **a. Heavy Chain Modifications**
        **b. Light Chain Modifications**
        **c. Exemplary modified Anti-EGFR Antibodies and Fragments Thereof**

    **2. Humanized Anti-EGFR Antibodies**
    **3. Additional Modifications**
    **4. Conjugates**

        **a. Targeted Agents**

            **i. Maytansinoid Drug Moieties**
            **ii. Auristatins and Dolastatins Drug Moieties**
            **iii. Cell Toxin Moieties**
            **iv. Nucleic acids for targeted delivery**

        **b. Linkers**

            **i. Peptide Linkers**
            **ii. Chemical Linkers**

**D. METHODS FOR IDENTIFYING AND ASSESSING ANTI-EGFR ANTIBODY PROPERTIES AND ACTIVITIES**

    **1. Binding Assays**

        **a. Solid Support Binding Assays**

            **i. Surface plasmon resonance**
            **ii. Bio-layer interferometry**

iii. Immunoassays

a) ELISA
b) Immunoprecipitation
c) Western blot
d) Immunohistochemistry
e) Radioimmunoassay

b. Solution Binding Assays

i. Isothermal titration calorimetry (ITC)
ii. Spectroscopic assays

2. Cell Based Assays
3. Animal Models

a. Assessing Side Effects

4. Pharmacokinetics and Pharmacodynamics assays

E. METHODS OF IDENTIFYING GENERATING AND PRODUCING ANTI-EGFR ANTIBODIES

1. Identifying Conditionally Therapeutic Proteins
2. Generating and Producing Anti-EGFR Antibodies

a. Vectors
b. Cells and Expression Systems

i. Prokaryotic Expression
ii. Yeast
iii. Insects
iv. Mammalian Cells
v. Plants

3. Purification

F. PHARMACEUTICAL COMPOSITIONS, FORMULATIONS, KITS, ARTICLES OF MANUFACTURE AND COMBINATIONS

1. Pharmaceutical Compositions and Formulations
2. Articles of Manufacture/Kits
3. Combinations

G. THERAPEUTIC USES

1. Exemplary Diseases and Conditions

a. Cancer
b. Non-Cancer Hyperproliferative Diseases
c. Autoimmune Diseases or Disorders
d. Inflammatory Disorders
e. Infectious Diseases
f. Other Diseases and Conditions

2. Subjects for therapy

a. Selection of Subjects Overexpressing EGFR
b. Selection of Subjects Exhibiting EGFR-associated Polymorphism

c. Identifying Subjects Exhibiting Anti-EGFR-Associated Side Effects

    i. Skin toxicities
    ii. Hypomagnesemia

d. Other Methods of Selecting or Identifying Subjects For Treatment

3. Dosages
4. Routes of Administration
5. Combination Therapies

## H. EXAMPLES

## A. DEFINITIONS

Unless defined otherwise, all technical and scientific terms used herein have the same meaning as is commonly understood by one of skill in the art to which the invention(s) belong. All patents, patent applications, published applications and publications, Genbank sequences, databases, websites and other published materials referred to throughout the entire disclosure herein, unless noted otherwise, are incorporated by reference in their entirety. In the event that there are a plurality of definitions for terms herein, those in this section prevail. Where reference is made to a URL or other such identifier or address, it is understood that such identifiers can change and particular information on the internet can come and go, but equivalent information can be found by searching the internet. Reference thereto evidences the availability and public dissemination of such information.

As used herein, a conditionally active protein is more active in one environment, particularly one *in vivo* environment, compared to a second environment. For example, a conditionally active protein can be more active in a tumor environment than in a non-tumor environment, such as a non-tumor environment in the skin, GI tract or other non-tumor environment.

As used herein, a therapeutic agent that has "conditional activity in a tumor microenvironment," or is "conditionally active in a tumor microenvironment," or variations thereof, is a therapeutic agent, such as an anti-EGFR antibody (e.g. a modified anti-EGFR antibody) provided herein, that is more active as a therapeutic in a tumor microenvironment than in a non-tumor microenvironment (e.g. a healthy or non-diseased tissue or cell, such as the basal layer of the skin). Conditional activity in a tumor microenvironment can be assessed in vivo or in vitro. For example, conditional activity in a tumor microenvironment can be assessed in vitro in binding assays for binding to EGFR under conditions that that exist in a tumor microenvironment, such as under low pH (e.g. pH 6.0 to 6.5) or elevated lactate concentrations (e.g. 10 mM to 20 mM), compared to conditions that exist in a non-tumor environment, such as neutral pH (e.g. 7.0 to 7.4) or low lactate concentrations (e.g. 1 mM to 5 mM). Conditional activity exists if the ratio of activity (e.g. binding activity) is greater under conditions of the tumor environment (e.g. pH 6.0 to 6.5 and/or 10 mM to 20 mM lactate) than under conditions of a non-tumor environment (e.g. pH 7.0 to 7.4 and 1 mM to 5 mM lactate). For example, conditional activity in a tumor environment exists if the ratio of activity is at least 1.1, 1.2, 1.3, 1.4, 1.5, 1.6, 1.7, 1.8, 1.9, 2.0, 2.5, 3.0, 3.5, 4.0, 4.5, 5.0 or more. In some cases, the conditional activity in a tumor environment exists if the ratio of activity is greater than 5.0, such as at least 6.0, 7.0, 8.0, 9.0, 10.0, 11.0, 12.0, 13.0, 14.0, 15.0, 16.0, 17.0, 18.0, 19.0, 20.0, 25.0, 30.0, 35.0, 40.0, 45.0, 50.0 or more.

Included among an anti-EGFR antibody provided herein that is conditionally active in a tumor microenvironment are antibodies that contain one or more modification(s) (*e.g.* amino acid replacement(s), insertions or deletions) compared to the same antibody without the modifications, and by virtue of the modification(s) is more active in a tumor microenvironment than in a non-tumor microenvironment. For example, the antibodies that are modified to render them conditionally active generally contain one or more modifications in cetuximab or an antigen-binding fragment thereof or variants thereof. The variants include those with modifications other than the modifications provided herein, such as by humanization to decrease immunogenicity. Typically, the modified anti-EGFR antibodies provided herein are more active (*i.e.* exhibit greater or increased activity) in a tumor microenvironment than in a non-tumor microenvironment compared to the corresponding form of the unmodified cetuximab, antigen-binding fragment thereof or variant thereof. For example, conditional activity can result from decreased activity (*e.g.* binding activity to an EGFR) of the modified anti-EGFR antibody in a non-tumor environment compared to the unmodified antibody, while retaining or exhibiting similar activity or increased activity compared to the unmodified antibody in the tumor environment.

As used herein, "conditions that simulate" a diseased or non-diseased microenvironment, refer to *in vitro* or *in vivo* assay conditions that correspond to a condition or conditions that exist in the environment *in vivo.* For example, if a microenvironment is characterized by low pH, then conditions that simulate the microenvironment include buffer or assay conditions having a low pH.

As used herein, conditions that exist in a tumor microenvironment include conditions that exist therein compared to a non-tumor microenvironment (e.g. a healthy or non-diseased cell or tissue). Conditions that exist in a tumor microenvironment include increased vascularization, hypoxia, low pH, increased lactate concentration, increased pyruvate concentration, increased interstitial fluid pressure and altered metabolites or metabolism indicative of a tumor. For example, a condition that exists in a tumor microenvironment is low pH less than 7.4, typically between or about between 5.6 to 6.8, such as less than or about or pH 5.6, 5.7, 5.8, 5.9, 6.0, 6.1, 6.2, 6.3, 6.4, 6.5, 6.6, 6.7, or 6.8. In another example, a condition that exists in a tumor microenvironment is high lactate concentration at or about between 5 mM to 20 mM lactic acid, for example 10 mM to 20 mM lactic acid such as 15 mM to 18 mM, and in particular at least or at least about or 16 mM, 16.5 mM or 17 mM lactic acid.

As used herein, conditions that exist in a non-tumor microenvironment include a condition or conditions that are not present in a tumor microenvironment. For purposes herein, the conditions or condition is the corresponding property or characteristic that is present in a tumor microenvironment and non-tumor environment, such as pH, lactate concentration or pyruvate concentration, but that differs between the two microenvironments. A condition that exists in a non-tumor microenvironment (e.g. basal layer of the skin) is pH from about 7.0 to about 7.8, such as at least or about or pH 7.1, 7.2, 7.3, 7.4, 7.5, 7.6, 7.7 or 7.8 (see, e.g., US Patent No. 7781405), in some examples pH 7.4. For example, the pH is a neutral pH of between or about between 7.0 to 7.4. A condition that exists in a non-tumor microenvironment (e.g. basal layer of the skin) is lactate concentration that is 0.5 to 5 mM lactate, such as, for example 0.2 mM to 4 mM lactic acid, such as 0.5, 1, 2, 3, 4, or 5 mM lactic acid.

As used herein, "low pH" refers to a pH ranging from about 5.6 to about 6.8, such as less than or about or pH 5.6, 5.7, 5.8 , 5.9, 6.0, 6.1, 6.2, 6.3, 6.4, 6.5, 6.6, 6.7, or 6.8.

As used herein, recitation that proteins are "compared under the same conditions" means that different proteins are treated identically or substantially identically such that any one or more conditions that can influence the activity or properties of a protein or agent are not varied or not substantially varied between the test agents. For example, when the activity of a modified anti-EGFR antibody is compared to an unmodified anti-EGFR antibody any one or more conditions such as amount or concentration of the polypeptide; presence, including amount, of excipients, carriers or other components in a formulation other than the active agent (*e.g.* modified anti-EGFR antibody); temperature; pH, time of storage; storage vessel; properties of storage (*e.g.* agitation) and/or other conditions associated with exposure or use are identical or substantially identical between and among the compared polypeptides.

As used herein, an "adverse effect," or "side effect" or "adverse event," or "adverse side effect" refers to a harmful, deleterious and/or undesired effect associated with administering a therapeutic agent. For example, side effects associated with administration of an anti-EGFR antibody, such as cetuximab are known to one of skill in the art and described herein. Such side effects include, for example, dermatological or dermal toxicity such as rash. Side effects or adverse effects are graded on toxicity and various toxicity scales exist providing definitions for each grade. Exemplary of such scales are toxicity scales of the National Cancer Institute Common Toxicity Criteria version 2.0, the World Health Organization or Common Terminology Criteria for Adverse Events (CTCAE) scale. Generally, the scale is as follows: Grade 1 = mild side effects; Grade 2= moderate side effects; Grade 3= Severe side effects; Grade 4= Life Threatening or Disabling side-effects; Grade 5= Fatal. Assigning grades of severity is within the experience of a physician or other health care professional.

As used herein, epidermal growth factor receptor (EGFR; Uniprot Accession No. P00533 and set forth in SEQ ID NO:6) refers to a tyrosine kinase growth factor receptor that is a member of the ErbB family of receptor tyrosine kinases and that is bound and activated by ligands such as epidermal growth factor (EGF), as well as other endogenous EGF-like ligands including TGF-$\alpha$, amphiregulin, heparin-binding EGF (HB-EGF) and betacellulin. Upon activation, EGFR is involved in signaling cascades important for cell growth, proliferation, survival and motility. In addition to their presence on a tumor cells, epidermal growth factor receptors are ubiquitous, distributed randomly on the surface of normal cells, excluding hematopoietic cells and cells of epidermal origin. For example, EGFR is expressed on skin keratinocytes.

As used herein, anti-EGFR antibody refers to any antibody that specifically binds to EGFR and blocks the binding of ligands to EGFR, thereby resulting in competitive inhibition of EGFR and inhibition of EGFR activation. Hence, anti-EGFR antibodies are EGFR inhibitors. Reference to anti-EGFR antibodies herein include a full-length antibody and antigen-binding fragments thereof that specifically bind to EGFR.

As used herein, cetuximab (225, also known and marketed as Erbitux) refers to an anti-EGFR antibody that is a chimeric (mouse/human) monoclonal antibody that is an EGFR inhibitor. Cetuximab has the sequence of amino acids set forth in SEQ ID NO:1 (heavy chain) and SEQ ID NO:2 (light chain).

As used herein, an antigen-binding fragment of cetuximab refers to and antibody derived from cetuximab but that is less than the full length of cetuximab but contains at least a portion of the variable region of the antibody sufficient to form an antigen binding site (*e.g.* one or more CDRs) and thus retains the binding specificity and/or activity of cetuximab. Exemplary of antigen-binding fragments of cetuximab include antibodies that contain the sequence of amino acids set forth in SEQ ID NO:3 (variable heavy chain) and the sequence of amino acids set forth in SEQ ID NO:4 (variable light chain), or a portion of SEQ ID NO:3 and SEQ ID NO:4 sufficient to bind to antigen. For example, exemplary of an antigen-binding fragment of cetuximab is a Fab antibody that contains the sequence of amino acids set forth in SEQ ID NO:5 (VH-CH1) and SEQ ID NO:2 (light chain VH-CL).

As used herein, a variant of cetuximab refers to an antibody derived from cetuximab or an antigen-binding fragment thereof that exhibits one or more modifications in cetuximab other than the modifications provided herein, and that specifically binds EGFR. For example, variants of cetuximab include humanization variants to reduce toxicity. Exemplary variants of cetuximab include those that have a sequence of amino acids for a variable heavy chain that exhibit at least 75%, 80%, 85%, 90%, 91%, 92%, 93%, 94%, 95%, 96%, 97%, 98%, 99% or more sequence identity to the sequence of amino acids set forth in SEQ ID NO:3 and/or a sequence of amino acids for a variable light chain that exhibits at least 75%, 80%, 85%, 90%, 91%, 92%, 93%, 94%, 95%, 96%, 97%, 98%, 99% or more sequence identity to the sequence of amino acids set forth in SEQ ID NO:4, and that do not contain any of the modification(s) provided herein (prior to modification thereof) and that specifically bind to EGFR. For example, exemplary of cetuximab variants provided herein are antibodies having a variable heavy chain set forth in SEQ ID NO:1 and a variable light chain set forth in SEQ ID NO:10, or antibodies having a variable heavy chain set forth in SEQ ID NO:28 and a variable light chain set forth in SEQ ID NO:29 or antibodies having a heavy chain set forth in SEQ ID NO:8 and a light chain set forth in SEQ ID NO:9, and corresponding antibody forms thereof. It is understood that variants of cetuximab that do not initially contain modifications provided herein can be used as an unmodified antibody and can be further modified to contain modifications provided herein.

As used herein, "both" with reference to modifications in a variable heavy chain, variable light chain or both means that an antibody contains one or more modifications in the variable heavy chain and one or more modifications in the variable light chain of the antibody.

As used herein, an "unmodified antibody" refers to a starting polypeptide heavy and light chain or fragment thereof that is selected for modification as provided herein. The starting target polypeptide can be a wild-type or reference form of an antibody, which is a predominant reference polypeptide to which activity is assessed. For example, cetuximab is a predominant or reference polypeptide for modification herein. The unmodified or starting target antibody can be altered or mutated, such that it differs from a predominant or reference form of the antibody, but is nonetheless referred to herein as a starting unmodified target protein relative to the subsequently modified polypeptides produced herein (e.g. antigen-binding fragments or variants of cetuximab). Thus, existing proteins known in the art that have been modified to have a desired increase or decrease in a particular activity or property compared to an unmodified reference protein can be selected and used as the starting unmodified target protein. For example, a protein that has been modified from a predominant or reference form by one or more single amino acid changes and possesses either an increase or decrease in a desired property, such as reduced immunogenicity can be a target protein, referred to herein as unmodified, for further modification of either the same or a different property.

As used herein, "modified anti-EGFR antibody" or "variant anti-EGFR antibody" refers to an anti-EGFR antibody that contains at least one amino acid addition, deletion or replacement as described herein in its sequence of amino acids compared to a reference or unmodified anti-EGFR antibody. Exemplary reference or unmodified anti-EGFR antibodies are full length anti-EGFR antibody polypeptides set forth in SEQ ID NOS: 1 (Heavy Chain) and 2 (Light Chain) or SEQ ID NO: 8 (Heavy Chain) and SEQ ID NO:9 (Light Chain); or antigen-binding fragments thereof such as an anti-EGFR antibody polypeptide set forth in SEQ ID NO:3 (variable Heavy Chain) and SEQ ID NO:4 (variable light chain), SEQ ID NO:5 (VH-CH1) and SEQ ID NO:2 (VL), or SEQ ID NO:3 (variable heavy chain) and SEQ ID NO:10 (variable light chain) or antibody variants thereof that exhibit heavy or light chains or portions thereof that exhibit at least 68%, 69%, 70%, 75%, 80%, 85%, 86%, 87%, 88%, 89%, 90%, 91%, 92%, 93%, 94%, 95%, 96%, 97%, 98%, or 99% sequence identity thereto to any of the recited SEQ ID NOS, whereby the resulting antibody specifically binds EGFR. A modified anti-EGFR antibody can have up to 150 amino acid replacements, so long as the resulting modified anti-EGFR antibody exhibits binding to EGFR. Typically, a modified anti-EGFR antibody contains 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 amino acid replacements. It is understood that a modified anti-EGFR antibody also can include any one or more other modifications, in addition to at least one amino acid addition, deletion or replacement as described herein.

As used herein, a "modification" is in reference to modification of a sequence of amino acids of a polypeptide or a sequence of nucleotides in a nucleic acid molecule and includes deletions, insertions, and replacements of amino acids and nucleotides, respectively. Methods of modifying a polypeptide are routine to those of skill in the art, such as by using recombinant DNA methodologies.

As used herein, "deletion," when referring to a nucleic acid or polypeptide sequence, refers to the deletion of one or more nucleotides or amino acids compared to a sequence, such as a target polynucleotide or polypeptide or a native or wild-type sequence.

As used herein, "insertion" when referring to a nucleic acid or amino acid sequence, describes the inclusion of one or more additional nucleotides or amino acids, within a target, native, wild-type or other related sequence. Thus, a nucleic acid molecule that contains one or more insertions compared to a wild-type sequence, contains one or more additional nucleotides within the linear length of the sequence. As used herein, "additions," to nucleic acid and amino acid sequences describe addition of nucleotides or amino acids onto either termini compared to another sequence.

As used herein, "substitution" or "replacement" refers to the replacing of one or more nucleotides or amino acids in a native, target, wild-type or other nucleic acid or polypeptide sequence with an alternative nucleotide or amino acid, without changing the length (as described in numbers of residues) of the molecule. Thus, one or more substitutions in a molecule does not change the number of amino acid residues or nucleotides of the molecule. Amino acid replacements compared to a particular polypeptide can be expressed in terms of the number of the amino acid residue along the length of the polypeptide sequence. For example, a modified polypeptide having a modification in the amino acid at the 19th position of the amino acid sequence that is a substitution of Isoleucine (Ile; I) for cysteine (Cys; C) can be expressed as I19C, Ile19Cys, or simply C19, to indicate that the amino acid at the modified 19th position is a cysteine. In this example, the molecule having the substitution has a modification at Ile 19 of the unmodified polypeptide. For purposes herein, since modifications are in a heavy chain (HC) or light chain (LC) of an antibody, modifications also can be denoted by reference to HC- or LC- to indicate the chain of the polypeptide that is altered.

As used herein, "at a position corresponding to" or recitation that nucleotides or amino acid positions "correspond to" nucleotides or amino acid positions in a disclosed sequence, such as set forth in the Sequence listing, refers to nucleotides or amino acid positions identified upon alignment with the disclosed sequence to maximize identity using a standard alignment algorithm, such as the GAP algorithm. For purposes herein, residues for modification provided herein are with reference to amino acid positions set forth in the variable heavy chain set forth in SEQ ID NO:3 and the variable light chain set forth in SEQ ID NO:4. Hence, corresponding residues can be determined by alignment of a reference heavy chain sequence, or portion thereof, with the sequence set forth in SEQ ID NO:3 and/or by alignment of a reference light chain sequence, or portion thereof, with the sequence set forth in SEQ ID NO:4. By aligning the sequences, one skilled in the art can identify corresponding residues, for example, using conserved and identical amino acid residues as guides. In general, to identify corresponding positions, the sequences of amino acids are aligned so that the highest order match is obtained (see, *e.g.:* Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; Carrillo et al. (1988) SIAM J Applied Math 48:1073). Exemplary alignments are provided in Figure 2 and exemplary amino acid replacements based on corresponding aligned residues are set forth in Table 5 and Table 7.

As used herein, alignment of a sequence refers to the use of homology to align two or more sequences of nucleotides or amino acids. Typically, two or more sequences that are related by 50 % or more identity are aligned. An aligned set of sequences refers to 2 or more sequences that are aligned at corresponding positions and can include aligning sequences derived from RNAs, such as ESTs and other cDNAs, aligned with genomic DNA sequence. Related or variant polypeptides or nucleic acid molecules can be aligned by any method known to those of skill in the art. Such methods typically maximize matches, and include methods, such as using manual alignments and by using the numerous alignment programs available (e.g., BLASTP) and others known to those of skill in the art. By aligning the sequences of polypeptides or nucleic acids, one skilled in the art can identify analogous portions or positions, using conserved and identical amino acid residues as guides. Further, one skilled in the art also can employ conserved amino acid or nucleotide residues as guides to find corresponding amino acid or nucleotide residues between and among human and non-human sequences. Corresponding positions also can be based on structural alignments, for example by using computer simulated alignments of protein structure. In other instances, corresponding regions can be identified. One skilled in the art also can employ conserved amino acid residues as guides to find corresponding amino acid residues between and among human and non-human sequences.

As used herein, a "property" of a polypeptide, such as an antibody, refers to any property exhibited by a polypeptide, including, but not limited to, binding specificity, structural configuration or conformation, protein stability, resistance to proteolysis, conformational stability, thermal tolerance, and tolerance to pH conditions. Changes in properties can alter an "activity" of the polypeptide. For example, a change in the binding specificity of the antibody polypeptide can alter the ability to bind an antigen, and/or various binding activities, such as affinity or avidity, or *in vivo* activities of the polypeptide.

As used herein, an "activity" or a "functional activity" of a polypeptide, such as an antibody, refers to any activity exhibited by the polypeptide. Such activities can be empirically determined. Exemplary activities include, but are not limited to, ability to interact with a biomolecule, for example, through antigen-binding, DNA binding, ligand binding, or dimerization, enzymatic activity, for example, kinase activity or proteolytic activity. For an antibody (including antibody fragments), activities include, but are not limited to, the ability to specifically bind a particular antigen, affinity of antigen-binding (e.g. high or low affinity), avidity of antigen-binding (e.g. high or low avidity), on-rate, off-rate, effector functions, such as the ability to promote antigen neutralization or clearance, virus neutralization, and *in vivo* activities, such as the ability to prevent infection or invasion of a pathogen, or to promote clearance, or to penetrate a particular tissue or fluid or cell in the body. Activity can be assessed *in vitro* or *in vivo* using recognized assays, such as ELISA, flow cytometry, surface plasmon resonance or equivalent assays to measure on- or off-rate, immunohistochemistry and immunofluorescence histology and microscopy, cell-based assays, flow cytometry and binding assays (*e.g.*, panning assays). For example, for an antibody polypeptide, activities can be assessed by measuring binding affinities, avidities, and/or binding coefficients (*e.g.,* for on-/off-rates), and other activities *in vitro* or by measuring various effects *in vivo,* such as immune effects, *e.g.* antigen clearance, penetration or localization of the antibody into tissues, protection from disease, e.g. infection, serum or other fluid antibody titers, or other assays that are well known in the art. The results of such assays that indicate that a polypeptide exhibits an activity can be correlated to activity of the polypeptide *in vivo,* in which *in vivo* activity can be referred to as therapeutic activity, or biological activity. Activity of a modified polypeptide can be any level of percentage of activity of the unmodified polypeptide, including but not limited to, 1 % of the activity, 2 %, 3 %, 4 %, 5 %, 10 %, 20 %, 30 %, 40 %, 50 %, 60 %, 70 %, 80 %, 90 %, 91 %, 92 %, 93 %, 94 %, 95 %, 96 %, 97 %, 98 %, 99 %, 100 %, 200 %, 300 %, 400 %, 500 %, or more of activity compared to the unmodified polypeptide. Assays to determine functionality or activity of modified (e.g. variant) antibodies are well known in the art.

As used herein, "exhibits at least one activity" or "retains at least one activity" refers to the activity exhibited by a modified polypeptide, such as a variant antibody or other therapeutic polypeptide (e.g. a-modified anti-EGFR antibody or antigen-binding fragment thereof), compared to the target or unmodified polypeptide, that does not contain the modification. A modified, or variant, polypeptide that retains an activity of a target polypeptide can exhibit improved activity, decreased activity, or maintain the activity of the unmodified polypeptide. In some instances, a modified, or variant, polypeptide can retain an activity that is increased compared to a target or unmodified polypeptide. In some cases, a modified, or variant, polypeptide can retain an activity that is decreased compared to an unmodified or target polypeptide. Activity of a modified, or variant, polypeptide can be any level of percentage of activity of the unmodified or target polypeptide, including but not limited to, 1 % of the activity, 2 %, 3 %, 4 %, 5 %, 10 %, 20 %, 30 %, 40 %, 50 %, 60 %, 70 %, 80 %, 90 %, 91 %, 92 %, 93 %, 94 %, 95 %, 96 %, 97 %, 98 %, 99 %, 100 %, 200 %, 300 %, 400 %, 500 %, or more activity compared to the unmodified or target polypeptide. In other embodiments, the change in activity is at least about 2 times, 3 times, 4 times, 5 times, 6 times, 7 times, 8 times, 9 times, 10 times, 20 times, 30 times, 40 times, 50 times, 60 times, 70 times, 80 times, 90 times, 100 times, 200 times, 300 times, 400 times, 500 times, 600 times, 700 times, 800 times, 900 times, 1000 times, or more times greater than unmodified or target polypeptide. Assays for retention of an activity depend on the activity to be retained. Such assays can be performed *in vitro* or *in vivo.* Activity can be measured, for example, using assays known in the art and described in the Examples below for activities such as but not limited to ELISA and panning assays. Activities of a modified, or variant, polypeptide compared to an unmodified or target polypeptide also can be assessed in terms of an *in vivo* therapeutic or biological activity or result following administration of the polypeptide.

As used herein, "increased activity" with reference to a modified anti-EGFR antibody means that, when tested under the same conditions, the modified anti-EGFR antibody exhibits greater activity compared to an unmodified anti-EGFR antibody not containing the amino acid replacement(s). For example, a modified anti-EGFR antibody exhibits at least or about at least 110%, 120%, 130%, 140%, 150%, 160%, 170%, 180%, 190%, 200%, 250%, 300%, 400%, 500% , 600%, 700%, 800%, 900%, 1000% or more of the activity of the unmodified or reference anti-EGFR antibody.

As used herein, "bind," "bound" or grammatical variations thereof refers to the participation of a molecule in any attractive interaction with another molecule, resulting in a stable association in which the two molecules are in close proximity to one another. Binding includes, but is not limited to, non-covalent bonds, covalent bonds (such as reversible and irreversible covalent bonds), and includes interactions between molecules such as, but not limited to, proteins, nucleic acids, carbohydrates, lipids, and small molecules, such as chemical compounds including drugs. Exemplary of bonds are antibody-antigen interactions and receptor-ligand interactions. When an antibody "binds" a particular antigen, bind refers to the specific recognition of the antigen by the antibody, through cognate antibody-antigen interaction, at antibody combining sites. Binding also can include association of multiple chains of a polypeptide, such as antibody chains which interact through disulfide bonds.

As used herein, binding activity refer to characteristics of a molecule, e.g. a polypeptide, relating to whether or not, and how, it binds one or more binding partners. Binding activities include the ability to bind the binding partner(s), the affinity with which it binds to the binding partner (e.g. high affinity), the avidity with which it binds to the binding partner, the strength of the bond with the binding partner and/or specificity for binding with the binding partner.

As used herein, "affinity" or "binding affinity" describes the strength of the interaction between two or more molecules, such as binding partners, typically the strength of the noncovalent interactions between two binding partners. The affinity of an antibody or antigen-binding fragment thereof for an antigen epitope is the measure of the strength of the total noncovalent interactions between a single antibody combining site and the epitope. Low-affinity antibody-antigen interaction is weak, and the molecules tend to dissociate rapidly, while high affinity antibody-antigen-binding is strong and the molecules remain bound for a longer amount of time. Methods for calculating affinity are well known, such as methods for determining association/dissociation constants. For example, a high antibody affinity means that the antibody specifically binds to a target protein with an equilibrium association constant ($K_A$) of greater than or equal to about $10^6$ $M^{-1}$, greater than or equal to about $10^7$ $M^{-1}$, greater than or equal to about $10^8$ $M^{-1}$, or greater than or equal to about $10^9$ $M^{-1}$, $10^{10}$ $M^{-1}$, $10^{11}$ $M^{-1}$ or $10^{12}$ $M^{-1}$. Antibodies also can be characterized by an equilibrium dissociation constant ($K_D$) $10^{-4}$ M, $10^{-6}$ M to $10^{-7}$ M, or $10^{-8}$ M, $10^{-10}$ M, $10^{-11}$ M or $10^{-12}$ M or lower. Affinity can be estimated empirically or affinities can be determined comparatively, e.g. by comparing the affinity of one antibody and another antibody for a particular antigen. For example, such affinities can be readily determined using conventional techniques, such as by equilibrium dialysis; by using the BIAcore 2000 instrument, using general procedures outlined by the manufacturer; by radioimmunoassay using radiolabeled target antigen; or by another method known to the skilled artisan. The affinity data can be analyzed, for example, by the method of Scatchard et al., Ann N.Y. Acad. Sci., 51:660 (1949).

As used herein, antibody avidity refers to the strength of multiple interactions between a multivalent antibody and its cognate antigen, such as with antibodies containing multiple binding sites associated with an antigen with repeating epitopes or an epitope array. A high avidity antibody has a higher strength of such interactions compared with a low avidity antibody.

As used herein, "affinity constant" refers to an association constant (Ka) used to measure the affinity of an antibody for an antigen. The higher the affinity constant the greater the affinity of the antibody for the antigen. Affinity constants are expressed in units of reciprocal molarity (*i.e.* $M^{-1}$) and can be calculated from the rate constant for the association-dissociation reaction as measured by standard kinetic methodology for antibody reactions (*e.g.*, immunoassays, surface plasmon resonance, or other kinetic interaction assays known in the art). The binding affinity of an antibody also can be expressed as a dissociation constant, or Kd. The dissociation constant is the reciprocal of the association constant, Kd = 1/Ka. Hence, an affinity constant also can be represented by the Kd.

As used herein, the term "the same," when used in reference to antibody binding affinity, means that the association constant (Ka) or dissociation constant (Kd) is within about 1 to 100 fold or 1 to 10 fold of the reference antibody (1-100 fold greater affinity or 1-100 fold less affinity, or any numerical value or range or value within such ranges, than the reference antibody).

As used herein, "substantially the same" when used in reference to association constant (Ka) or dissociation constant (Kd), means that the association constant is within about 5 to 5000 fold greater or less than the association constant, Ka, of the reference antibody (5-5000 fold greater or 5-5000 fold less than the reference antibody).

As used herein, "specifically bind" or "immunospecifically bind" with respect to an antibody or antigen-binding fragment thereof are used interchangeably herein and refer to the ability of the antibody or antigen-binding fragment to form one or more noncovalent bonds with a cognate antigen, by noncovalent interactions between the antibody combining site(s) of the antibody and the antigen. Typically, an antibody that immunospecifically binds (or that specifically binds) to EGFR is one that binds to EGFR with an affinity constant Ka of about or $1\times10^7$ $M^{-1}$ or $1x\ 10^8$ $M^{-1}$ or greater (or a dissociation constant ($K_d$) of $1x\ 10^{-7}$ M or $1\times10^{-8}$ M or less). Affinity constants can be determined by standard kinetic methodology for antibody reactions, for example, immunoassays, surface plasmon resonance (SPR) (Rich and Myszka (2000) Curr. Opin. Biotechnol 11:54; Englebienne (1998) Analyst. 123:1599), isothermal titration calorimetry (ITC) or other kinetic interaction assays known in the art (see, *e.g.,* Paul, ed., Fundamental Immunology, 2nd ed., Raven Press, New York, pages 332-336 (1989); see also U.S. Pat. No. 7,229,619 for a description of exemplary SPR and ITC methods for calculating the binding affinity of antibodies). Instrumentation and methods for real time detection and monitoring of binding rates are known and are commercially available (*e.g.*, BiaCore 2000, Biacore AB, Upsala, Sweden and GE Healthcare Life Sciences; Malmqvist (2000) Biochem. Soc. Trans. 27:335). Antibodies or antigen-binding fragments that immunospecifically bind to a particular antigen (*e.g.* EGFR) can be identified, for example, by immunoassays, such as radioimmunoassays (RIA), enzyme-linked immunosorbent assays (ELISAs), surface plasmon resonance, or other techniques known to those of skill in the art.

As used herein, the term "surface plasmon resonance" refers to an optical phenomenon that allows for the analysis of real-time interactions by detection of alterations in protein concentrations within a biosensor matrix, for example, using the BiaCore system (GE Healthcare Life Sciences).

As used herein, "antibody" refers to immunoglobulins and immunoglobulin fragments, whether natural or partially or wholly synthetically, such as recombinantly, produced, including any fragment thereof containing at least a portion of the variable heavy chain and light region of the immunoglobulin molecule that is sufficient to form an antigen binding site and, when assembled, to specifically bind antigen. Hence, an antibody includes any protein having a binding domain that is homologous or substantially homologous to an immunoglobulin antigen-binding domain (antibody combining site). For example, an antibody refers to an antibody that contains two heavy chains (which can be denoted H and H') and two light chains (which can be denoted L and L'), where each heavy chain can be a full-length immunoglobulin heavy chain or a portion thereof sufficient to form an antigen binding site (e.g. heavy chains include, but are not limited to, VH chains, VH-CH1 chains and VH-CH1-CH2-CH3 chains), and each light chain can be a full-length light chain or a portion thereof sufficient to form an antigen binding site (e.g. light chains include, but are not limited to, VL chains and VL-CL chains). Each heavy chain (H and H') pairs with one light chain (L and L', respectively). Typically, antibodies minimally include all or at least a portion of the variable heavy (VH) chain and/or the variable light (VL) chain. The antibody also can include all or a portion of the constant region.

For purposes herein, the term antibody includes full-length antibodies and portions thereof including antibody fragments, such as anti-EGFR antibody fragments. Antibody fragments, include, but are not limited to, Fab fragments, Fab' fragments, F(ab')$_2$ fragments, Fv fragments, disulfide-linked Fvs (dsFv), Fd fragments, Fd' fragments, single-chain Fvs (scFv), single-chain Fabs (scFab), diabodies, anti-idiotypic (anti-Id) antibodies, or antigen-binding fragments of any of the above. Antibody also includes synthetic antibodies, recombinantly produced antibodies, multispecific antibodies (e.g., bispecific antibodies), human antibodies, non-human antibodies, humanized antibodies, chimeric antibodies, and intrabodies. Antibodies provided herein include members of any immunoglobulin type (e.g., IgG, IgM, IgD, IgE, IgA and IgY), any class (e.g. IgG1, IgG2, IgG3, IgG4, IgA1 and IgA2) or subclass (e.g., IgG2a and IgG2b).

As used herein, a form of an antibody refers to a particular structure of an antibody. Antibodies herein include full length antibodies and portions thereof, such as, for example, an Fab fragment or other antibody fragment. Thus, an Fab is a particular form of an antibody.

As used herein, reference to a "corresponding form" of an antibody means that when comparing a property or activity of two antibodies, the property is compared using the same form of the antibody. For example, if it's stated that an antibody has less activity compared to the activity of the corresponding form of a first antibody, that means that a particular form, such as an Fab of that antibody, has less activity compared to the Fab form of the first antibody.

As used herein, a full-length antibody is an antibody having two full-length heavy chains (e.g. VH-CH1-CH2-CH3 or VH-CH1-CH2-CH3-CH4) and two full-length light chains (VL-CL) and hinge regions, such as human antibodies produced by antibody secreting B cells and antibodies with the same domains that are produced synthetically.

As used herein, antibody fragment or antibody portion refers to any portion of a full-length antibody that is less than full length but contains at least a portion of the variable region of the antibody sufficient to form an antigen binding site (e.g. one or more CDRs) and thus retains the binding specificity and/or an activity of the full-length antibody; antibody fragments include antibody derivatives produced by enzymatic treatment of full-length antibodies, as well as synthetically, e.g. recombinantly produced derivatives. Examples of antibody fragments include, but are not limited to, Fab, Fab', F(ab)$_2$, single-chain Fvs (scFv), Fv, dsFv, diabody, Fd and Fd fragments (see, for example, Methods in Molecular Biology, Vol 207: Recombinant Antibodies for Cancer Therapy Methods and Protocols (2003); Chapter 1; p 3-25, Kipriyanov). The fragment can include multiple chains linked together, such as by disulfide bridges and/or by peptide linkers. An antibody fragment generally contains at least about 50 amino acids and typically at least 200 amino acids.

As used herein, an Fv antibody fragment is composed of one variable heavy domain ($V_H$) and one variable light ($V_L$) domain linked by noncovalent interactions.

As used herein, a dsFv refers to an Fv with an engineered intermolecular disulfide bond, which stabilizes the $V_H$-$V_L$ pair.

As used herein, an Fd fragment is a fragment of an antibody containing a variable domain ($V_H$) and one constant region domain ($C_H$1) of an antibody heavy chain.

As used herein, a Fab fragment is an antibody fragment that results from digestion of a full-length immunoglobulin with papain, or a fragment having the same structure that is produced synthetically, *e.g.* by recombinant methods. A Fab fragment contains a light chain (containing a $V_L$ and $C_L$) and another chain containing a variable domain of a heavy chain ($V_H$) and one constant region domain of the heavy chain ($C_H$1).

As used herein, a F(ab')$_2$ fragment is an antibody fragment that results from digestion of an immunoglobulin with pepsin at pH 4.0-4.5, or a fragment having the same structure that is produced synthetically, *e.g.* by recombinant methods. The F(ab')$_2$ fragment essentially contains two Fab fragments where each heavy chain portion contains an additional few amino acids, including cysteine residues that form disulfide linkages joining the two fragments.

As used herein, a Fab' fragment is a fragment containing one half (one heavy chain and one light chain) of the F(ab')$_2$ fragment.

As used herein, an Fd' fragment is a fragment of an antibody containing one heavy chain portion of a F(ab')$_2$ fragment.

As used herein, an Fv' fragment is a fragment containing only the $V_H$ and $V_L$ domains of an antibody molecule.

As used herein, hsFv refers to antibody fragments in which the constant domains normally present in a Fab fragment have been substituted with a heterodimeric coiled-coil domain (see, e.g., Arndt et al. (2001) J Mol Biol. 7:312:221-228).

As used herein, an scFv fragment refers to an antibody fragment that contains a variable light chain ($V_L$) and variable heavy chain ($V_H$), covalently connected by a polypeptide linker in any order. The linker is of a length such that the two variable domains are bridged without substantial interference. Exemplary linkers are (Gly-Ser)$_n$ residues with some Glu or Lys residues dispersed throughout to increase solubility.

As used herein, diabodies are dimeric scFv; diabodies typically have shorter peptide linkers than scFvs, and preferentially dimerize.

As used herein, a polypeptide "domain" is a part of a polypeptide (a sequence of three or more, generally 5, 10 or more amino acids) that is structurally and/or functionally distinguishable or definable. Exemplary of a polypeptide domain is a part of the polypeptide that can form an independently folded structure within a polypeptide made up of one or more structural motifs (*e.g.* combinations of alpha helices and/or beta strands connected by loop regions) and/or that is recognized by a particular functional activity, such as enzymatic activity, dimerization or antigen-binding. A polypeptide can have one or more, typically more than one, distinct domains. For example, the polypeptide can have one or more structural domains and one or more functional domains. A single polypeptide domain can be distinguished based on structure and function. A domain can encompass a contiguous linear sequence of amino acids. Alternatively, a domain can encompass a plurality of non-contiguous amino acid portions, which are non-contiguous along the linear sequence of amino acids of the polypeptide. Typically, a polypeptide contains a plurality of domains. For example, each heavy chain and each light chain of an antibody molecule contains a plurality of immunoglobulin (Ig) domains, each about 110 amino acids in length. Those of skill in the art are familiar with polypeptide domains and can identify them by virtue of structural and/or functional homology with other such domains. For exemplification herein, definitions are provided, but it is understood that it is well within the skill in the art to recognize particular domains by name. If needed, appropriate software can be employed to identify domains.

As used herein, a functional region of a polypeptide is a region of the polypeptide that contains at least one functional domain (which imparts a particular function, such as an ability to interact with a biomolecule, for example, through antigen-binding, DNA binding, ligand binding, or dimerization, or by enzymatic activity, for example, kinase activity or proteolytic activity); exemplary of functional regions of polypeptides are antibody domains, such as $V_H$, $V_L$, $C_H$, $C_L$, and portions thereof, such as CDRs, including CDR1, CDR2 and CDR3, or antigen-binding portions, such as antibody combining sites.

As used herein, a structural region of a polypeptide is a region of the polypeptide that contains at least one structural domain.

As used herein, an Ig domain is a domain, recognized as such by those in the art, that is distinguished by a structure, called the Immunoglobulin (Ig) fold, which contains two beta-pleated sheets, each containing anti-parallel beta strands of amino acids connected by loops. The two beta sheets in the Ig fold are sandwiched together by hydrophobic interactions and a conserved intra-chain disulfide bond. Individual immunoglobulin domains within an antibody chain further can be distinguished based on function. For example, a light chain contains one variable region domain (VL) and one constant region domain (CL), while a heavy chain contains one variable region domain (VH) and three or four constant region domains (CH). Each VL, CL, VH, and CH domain is an example of an immunoglobulin domain.

As used herein, a variable domain with reference to an antibody is a specific Ig domain of an antibody heavy or light chain that contains a sequence of amino acids that varies among different antibodies. Each light chain and each heavy chain has one variable region domain (VL and VH). The variable domains provide antigen specificity, and thus are responsible for antigen recognition. Each variable region contains CDRs that are part of the antigen binding site domain and framework regions (FRs).

As used herein, "hypervariable region," "HV," "complementarity-determining region," "CDR" and "antibody CDR" are used interchangeably to refer to one of a plurality of portions within each variable region that together form an antigen binding site of an antibody. Each variable region domain contains three CDRs, named CDR1, CDR2, and CDR3. The three CDRs are non-contiguous along the linear amino acid sequence, but are proximate in the folded polypeptide. The CDRs are located within the loops that join the parallel strands of the beta sheets of the variable domain.

As used herein, "antigen-binding domain," "antigen-binding site," "antigen combining site" and "antibody combining site" are used synonymously to refer to a domain within an antibody that recognizes and physically interacts with cognate antigen. A native conventional full-length antibody molecule has two conventional antigen-binding sites, each containing portions of a heavy chain variable region and portions of a light chain variable region. A conventional antigen-binding site contains the loops that connect the anti-parallel beta strands within the variable region domains. The antigen combining sites can contain other portions of the variable region domains. Each conventional antigen-binding site contains three hypervariable regions from the heavy chain and three hypervariable regions from the light chain. The hypervariable regions also are called complementarity-determining regions (CDRs).

As used herein, "portion thereof' with reference to an antibody heavy or light chain or variable heavy or light chain refers to a contiguous portion thereof that is sufficient to form an antigen binding site such that, when assembled into an antibody containing a heavy and light chain, it contains at least 1 or 2, typically 3, 4, 5 or all 6 CDRs of the variable heavy (VH) and variable light (VL) chains sufficient to retain at least a portion of the binding specificity of the corresponding full-length antibody containing all 6 CDRs. Generally, a sufficient antigen binding site requires CDR3 of the heavy chain (CDRH3). It typically further requires the CDR3 of the light chain (CDRL3). As described herein, one of skill in the art knows and can identify the CDRs based on Kabat or Chothia numbering (see e.g., Kabat, E.A. et al. (1991) Sequences of Proteins of Immunological Interest, Fifth Edition, U.S. Department of Health and Human Services, NIH Publication No. 91-3242, and Chothia, C. et al. (1987) J. Mol. Biol. 196:901-917).

As used herein, framework regions (FRs) are the domains within the antibody variable region domains that are located within the beta sheets; the FR regions are comparatively more conserved, in terms of their amino acid sequences, than the hypervariable regions.

As used herein, a constant region domain is a domain in an antibody heavy or light chain that contains a sequence of amino acids that is comparatively more conserved among antibodies than the variable region domain. Each light chain has a single light chain constant region (CL) domain and each heavy chain contains one or more heavy chain constant region (CH) domains, which include, CH1, CH2, CH3 and CH4. Full-length IgA, IgD and IgG isotypes contain CH1, CH2, CH3 and a hinge region, while IgE and IgM contain CH1, CH2, CH3 and CH4. CH1 and CL domains extend the Fab arm of the antibody molecule, thus contributing to the interaction with antigen and rotation of the antibody arms. Antibody constant regions can serve effector functions, such as, but not limited to, clearance of antigens, pathogens and toxins to which the antibody specifically binds, e.g. through interactions with various cells, biomolecules and tissues.

As used herein, "Kabat numbering" refers to the index numbering of the IgG1 Kabat antibody (see e.g., Kabat, E.A. et al. (1991) Sequences of Proteins of Immunological Interest, Fifth Edition, U.S. Department of Health and Human Services, NIH Publication No. 91-3242). For example, based on Kabat numbering, CDR-LI corresponds to residues L24-L34; CDR-L2 corresponds to residues L50-L56; CDR-L3 corresponds to residues L89-L97; CDR-H1 corresponds to residues H31 - H35, 35a or 35b depending on the length; CDR-H2 corresponds to residues H50-H65; and CDR-H3 corresponds to residues H95-H102. One of skill in the art can identify regions of the constant region using Kabat. Tables 1 and 2 set forth corresponding residues using kabat numbering and EU numbering schemes for the exemplary antibody cetuximab.

As used herein, "EU numbering" or "EU index" refer to the numbering scheme of the EU antibody described in Edelman et al., Proc Natl. Acad. Sci. USA 63 (1969) 78-85. "EU index as in Kabat" refers to EU index numbering of the human IgG1 Kabat antibody as set forth in Kabat, E.A. et al. (1991) Sequences of Proteins of Immunological Interest, Fifth Edition, U.S. Department of Health and Human Services, NIH Publication No. 91-3242. EU numbering or EU numbering as in Kabat are frequently used by those of skill in the art to number amino acid residues of the Fc regions of the light and heavy antibody chains. For example, One of skill in the art can identify regions of the constant region using EU numbering. For example, the CL domain corresponds to residues L108-L216 according to Kabat numbering or L108-L214 according to EU numbering. CH1 corresponds to residues 118-215 (EU numbering) or 114-223 (Kabat numbering); CH2 corresponds to residues 231-340 (EU numbering) or 244-360 (Kabat numbering); CH3 corresponds to residues 341-446 (EU numbering) or 361-478 (Kabat numbering) domain corresponds to ; CDR-L2 corresponds to residues L50-L56; CDR-L3 corresponds to residues L89-L97; CDR-H1 corresponds to residues H31 - H35, 35a or 35b depending on the length; CDR-H2 corresponds to residues H50-H65; and CDR-H3 corresponds to residues H95-H102. Tables 1 and 2 set forth corresponding residues using Kabat and EU numbering for the exemplary antibody cetuximab. The top row (**bold**) sets forth the amino acid residue number; the second row (**bold**) provides the 1-letter code for the amino acid residue at the position indicated by the number in the top row; the third row (*italic*) indicates the corresponding Kabat number according to Kabat numbering; and the fourth row (not-bold, not-italic) indicates the corresponding EU index number according to EU numbering.

**Table 1. Kabat and EU Numbering of Cetuximab Light Chain**

| **1** | **2** | **3** | **4** | **5** | **6** | **7** | **8** | **9** | **10** | **11** | **12** | **13** | **14** | **15** |
|---|---|---|---|---|---|---|---|---|---|---|---|---|---|---|
| **D** | **I** | **L** | **L** | **T** | **Q** | **S** | **P** | **V** | **I** | **L** | **S** | **V** | **S** | **P** |
| *1* | *2* | *3* | *4* | *5* | *6* | *7* | *8* | *9* | *10* | *11* | *12* | *13* | *14* | *15* |
| 1 | 2 | 3 | 4 | 5 | 6 | 7 | 8 | 9 | 10 | 11 | 12 | 13 | 14 | 15 |
| | | | | | | | | | | | | | | |
| **16** | **17** | **18** | **19** | **20** | **21** | **22** | **23** | **24** | **25** | **26** | **27** | **28** | **29** | **30** |
| **G** | **E** | **R** | **V** | **S** | **F** | **S** | **C** | **R** | **A** | **S** | **Q** | **S** | **I** | **G** |
| *16* | *17* | *18* | *19* | *20* | *21* | *22* | *23* | *24* | *25* | *26* | *27* | *28* | *29* | *30* |
| 16 | 17 | 18 | 19 | 20 | 21 | 22 | 23 | 24 | 25 | 26 | 27 | 28 | 29 | 30 |
| | | | | | | | | | | | | | | |
| **31** | **32** | **33** | **34** | **35** | **36** | **37** | **38** | **39** | **40** | **41** | **42** | **43** | **44** | **45** |
| **T** | **N** | **I** | **H** | **W** | **Y** | **Q** | **Q** | **R** | **T** | **N** | **G** | **S** | **P** | **R** |
| *31* | *32* | *33* | *34* | *35* | *36* | *37* | *38* | *39* | *40* | *41* | *42* | *43* | *44* | *45* |
| 31 | 32 | 33 | 34 | 35 | 36 | 37 | 38 | 39 | 40 | 41 | 42 | 43 | 44 | 45 |
| | | | | | | | | | | | | | | |

(continued)

| 46 | 47 | 48 | 49 | 50 | 51 | 52 | 53 | 54 | 55 | 56 | 57 | 58 | 59 | 60 |
|-----|-----|-----|-----|-----|-----|-----|-----|-----|-----|-----|-----|-----|-----|-----|
| L | L | I | K | Y | A | S | E | S | I | S | G | I | P | S |
| 46 | 47 | 48 | 49 | 50 | 51 | 52 | 53 | 54 | 55 | 56 | 57 | 58 | 59 | 60 |
| 46 | 47 | 48 | 49 | 50 | 51 | 52 | 53 | 54 | 55 | 56 | 57 | 58 | 59 | 60 |

| 61 | 62 | 63 | 64 | 65 | 66 | 67 | 68 | 69 | 70 | 71 | 72 | 73 | 74 | 75 |
|-----|-----|-----|-----|-----|-----|-----|-----|-----|-----|-----|-----|-----|-----|-----|
| R | F | S | G | S | G | S | G | T | D | F | T | L | S | I |
| 61 | 62 | 63 | 64 | 65 | 66 | 67 | 68 | 69 | 70 | 71 | 72 | 73 | 74 | 75 |
| 61 | 62 | 63 | 64 | 65 | 66 | 67 | 68 | 69 | 70 | 71 | 72 | 73 | 74 | 75 |

| 76 | 77 | 78 | 79 | 80 | 81 | 82 | 83 | 84 | 85 | 86 | 87 | 88 | 89 | 90 |
|-----|-----|-----|-----|-----|-----|-----|-----|-----|-----|-----|-----|-----|-----|-----|
| N | S | V | E | S | E | D | I | A | D | Y | Y | C | Q | Q |
| 76 | 77 | 78 | 79 | 80 | 81 | 82 | 83 | 84 | 85 | 86 | 87 | 88 | 89 | 90 |
| 76 | 77 | 78 | 79 | 80 | 81 | 82 | 83 | 84 | 85 | 86 | 87 | 88 | 89 | 90 |

| 91 | 92 | 93 | 94 | 95 | 96 | 97 | 98 | 99 | 100 | 101 | 102 | 103 | 104 | 105 |
|-----|-----|-----|-----|-----|-----|-----|-----|-----|-----|-----|-----|-----|-----|-----|
| N | N | N | W | P | T | T | F | G | A | G | T | K | L | E |
| 91 | 92 | 93 | 94 | 95 | 96 | 97 | 98 | 99 | 100 | 101 | 102 | 103 | 104 | 105 |
| 91 | 92 | 93 | 94 | 95 | 96 | 97 | 98 | 99 | 100 | 101 | 102 | 103 | 104 | 105 |

| 106 | 107 | 108 | 109 | 110 | 111 | 112 | 113 | 114 | 115 | 116 | 117 | 118 | 119 | 120 |
|-----|-----|-----|-----|-----|-----|-----|-----|-----|-----|-----|-----|-----|-----|-----|
| L | K | R | T | V | A | A | P | S | V | F | I | F | P | P |
| 106 | 107 | 108 | 109 | 110 | 111 | 112 | 113 | 114 | 115 | 116 | 117 | 118 | 119 | 120 |
| 106 | 107 | 108 | 109 | 110 | 111 | 112 | 113 | 114 | 115 | 116 | 117 | 118 | 119 | 120 |

| 121 | 122 | 123 | 124 | 125 | 126 | 127 | 128 | 129 | 130 | 131 | 132 | 133 | 134 | 135 |
|-----|-----|-----|-----|-----|-----|-----|-----|-----|-----|-----|-----|-----|-----|-----|
| S | D | E | Q | L | K | S | G | T | A | S | V | V | C | L |
| 121 | 122 | 123 | 124 | 125 | 126 | 127 | 128 | 129 | 130 | 131 | 132 | 133 | 134 | 135 |
| 121 | 122 | 123 | 124 | 125 | 126 | 127 | 128 | 129 | 130 | 131 | 132 | 133 | 134 | 135 |

| 136 | 137 | 138 | 139 | 140 | 141 | 142 | 143 | 144 | 145 | 146 | 147 | 148 | 149 | 150 |
|-----|-----|-----|-----|-----|-----|-----|-----|-----|-----|-----|-----|-----|-----|-----|
| L | N | N | F | Y | P | R | E | A | K | V | Q | W | K | V |
| 136 | 137 | 138 | 139 | 140 | 141 | 142 | 143 | 144 | 145 | 146 | 147 | 148 | 149 | 150 |
| 136 | 137 | 138 | 139 | 140 | 141 | 142 | 143 | 144 | 145 | 146 | 147 | 148 | 149 | 150 |

| 151 | 152 | 153 | 154 | 155 | 156 | 157 | 158 | 159 | 160 | 161 | 162 | 163 | 164 | 165 |
|-----|-----|-----|-----|-----|-----|-----|-----|-----|-----|-----|-----|-----|-----|-----|
| D | N | A | L | Q | S | G | N | S | Q | E | S | V | T | E |
| 151 | 152 | 153 | 154 | 155 | 156 | 157 | 158 | 159 | 160 | 161 | 162 | 163 | 164 | 165 |
| 151 | 152 | 153 | 154 | 155 | 156 | 157 | 158 | 159 | 160 | 161 | 162 | 163 | 164 | 165 |

(continued)

| 166 | 167 | 168 | 169 | 170 | 171 | 172 | 173 | 174 | 175 | 176 | 177 | 178 | 179 | 180 |
|-----|-----|-----|-----|-----|-----|-----|-----|-----|-----|-----|-----|-----|-----|-----|
| Q | D | S | K | D | S | T | Y | S | L | S | S | T | L | T |
| *166* | *167* | *168* | *169* | *170* | *171* | *172* | *173* | *174* | *175* | *176* | *177* | *178* | *179* | *180* |
| 166 | 167 | 168 | 169 | 170 | 171 | 172 | 173 | 174 | 175 | 176 | 177 | 178 | 179 | 180 |

| 181 | 182 | 183 | 184 | 185 | 186 | 187 | 188 | 189 | 190 | 191 | 192 | 193 | 194 | 195 |
|-----|-----|-----|-----|-----|-----|-----|-----|-----|-----|-----|-----|-----|-----|-----|
| L | S | K | A | D | Y | E | K | H | K | V | Y | A | C | E |
| *181* | *182* | *183* | *184* | *185* | *186* | *187* | *188* | *189* | *190* | *191* | *192* | *193* | *194* | *195* |
| 181 | 182 | 183 | 184 | 185 | 186 | 187 | 188 | 189 | 190 | 191 | 192 | 193 | 194 | 195 |

| 196 | 197 | 198 | 199 | 200 | 201 | 202 | 203 | 204 | 205 | 206 | 207 | 208 | 209 | 210 |
|-----|-----|-----|-----|-----|-----|-----|-----|-----|-----|-----|-----|-----|-----|-----|
| V | T | H | Q | G | L | S | S | P | V | T | K | S | F | N |
| *196* | *197* | *198* | *199* | *200* | *201* | *202* | *203* | *204* | *205* | *206* | *207* | *208* | *209* | *210* |
| 196 | 197 | 198 | 199 | 200 | 201 | 202 | 203 | 204 | 205 | 206 | 207 | 208 | 209 | 210 |

| 211 | 212 | 213 |
|-----|-----|-----|
| R | G | A |
| *211* | *212* | *213* |
| 211 | 212 | 213 |

**Table 2. Kabat and EU Numbering of Cetuximab Heavy Chain**

| 1 | 2 | 3 | 4 | 5 | 6 | 7 | 8 | 9 | 10 | 11 | 12 | 13 | 14 | 15 |
|---|---|---|---|---|---|---|---|---|----|----|----|----|----|----|
| Q | V | Q | L | K | Q | S | G | P | G | L | V | Q | P | S |
| *1* | *2* | *3* | *4* | *5* | *6* | *7* | *8* | *9* | *10* | *11* | *12* | *13* | *14* | *15* |
| 1 | 2 | 3 | 4 | 5 | 6 | 7 | 8 | 9 | 10 | 11 | 12 | 13 | 14 | 15 |

| 16 | 17 | 18 | 19 | 20 | 21 | 22 | 23 | 24 | 25 | 26 | 27 | 28 | 29 | 30 |
|----|----|----|----|----|----|----|----|----|----|----|----|----|----|----|
| Q | S | L | S | I | T | C | T | V | S | G | F | S | L | T |
| *16* | *17* | *18* | *19* | *20* | *21* | *22* | *23* | *24* | *25* | *26* | *27* | *28* | *29* | *30* |
| 16 | 17 | 18 | 19 | 20 | 21 | 22 | 23 | 24 | 25 | 26 | 27 | 28 | 29 | 30 |

| 31 | 32 | 33 | 34 | 35 | 36 | 37 | 38 | 39 | 40 | 41 | 42 | 43 | 44 | 45 |
|----|----|----|----|----|----|----|----|----|----|----|----|----|----|----|
| N | Y | G | V | H | W | V | R | Q | S | P | G | K | G | L |
| *31* | *32* | *33* | *34* | *35* | *36* | *37* | *38* | *39* | *40* | *41* | *42* | *43* | *44* | *45* |
| 31 | 32 | 33 | 34 | 35 | 36 | 37 | 38 | 39 | 40 | 41 | 42 | 43 | 44 | 45 |

| 46 | 47 | 48 | 49 | 50 | 51 | 52 | 53 | 54 | 55 | 56 | 57 | 58 | 59 | 60 |
|----|----|----|----|----|----|----|----|----|----|----|----|----|----|----|
| E | W | L | G | V | I | W | S | G | G | N | T | D | Y | N |
| *46* | *47* | *48* | *49* | *50* | *51* | *52* | *53* | *54* | *55* | *56* | *57* | *58* | *59* | *60* |
| 46 | 47 | 48 | 49 | 51 | 52 | 53 | 54 | 55 | 56 | 57 | 58 | 59 | 60 | 61 |

| 61 | 62 | 63 | 64 | 65 | 66 | 67 | 68 | 69 | 70 | 71 | 72 | 73 | 74 | 75 |
|----|----|----|----|----|----|----|----|----|----|----|----|----|----|----|
| T | P | F | T | S | R | L | S | I | N | K | D | N | S | K |
| *61* | *62* | *63* | *64* | *65* | *66* | *67* | *68* | *69* | *70* | *71* | *72* | *73* | *74* | *75* |
| 61 | 62 | 63 | 64 | 65 | 66 | 67 | 68 | 69 | 70 | 71 | 72 | 73 | 74 | 75 |

(continued)

| 62 | 63 | 64 | 65 | 66 | 67 | 68 | 69 | 70 | 71 | 72 | 73 | 74 | 75 | 76 |
|---|---|---|---|---|---|---|---|---|---|---|---|---|---|---|
| 76 | 77 | 78 | 79 | 80 | 81 | 82 | 83 | 84 | 85 | 86 | 87 | 88 | 89 | 90 |
| S | Q | V | F | F | K | M | N | S | L | Q | S | N | D | T |
| 76 | 77 | 78 | 79 | 80 | 81 | 82 | 82A | 82B | 82C | 83 | 84 | 85 | 86 | 87 |
| 77 | 78 | 79 | 80 | 81 | 82 | 83 | 84 | 85 | 86 | 87 | 88 | 89 | 90 | 91 |
| 91 | 92 | 93 | 94 | 95 | 96 | 97 | 98 | 99 | 100 | 101 | 102 | 103 | 104 | 105 |
| A | I | Y | Y | C | A | R | A | L | T | Y | Y | D | Y | E |
| 88 | 89 | 90 | 91 | 92 | 93 | 94 | 95 | 96 | 97 | 98 | 99 | 100 | 100A | 100B |
| 92 | 93 | 94 | 95 | 96 | 97 | 98 | 99 | 100 | 101 | 102 | 103 | 104 | 105 | 106 |
| 106 | 107 | 108 | 109 | 110 | 111 | 112 | 113 | 114 | 115 | 116 | 117 | 118 | 119 | 120 |
| F | A | Y | W | G | Q | G | T | L | V | T | V | S | A | A |
| 100C | 101 | 102 | 103 | 104 | 105 | 106 | 107 | 108 | 109 | 110 | 111 | 112 | 113 | 114 |
| - | 107 | 108 | 109 | 110 | - | 111 | - | 112 | 113 | 114 | 115 | 116 | 117 | 118 |
| 121 | 122 | 123 | 124 | 125 | 126 | 127 | 128 | 129 | 130 | 131 | 132 | 133 | 134 | 135 |
| S | T | K | G | P | S | V | F | P | L | A | P | S | S | K |
| 115 | 116 | 117 | 118 | 119 | 120 | 121 | 122 | 123 | 124 | 125 | 126 | 127 | 128 | 129 |
| 119 | 120 | 121 | 122 | 123 | 124 | 125 | 126 | 127 | 128 | 129 | 130 | 131 | 132 | 133 |
| 136 | 137 | 138 | 139 | 140 | 141 | 142 | 143 | 144 | 145 | 146 | 147 | 148 | 149 | 150 |
| S | T | S | G | G | T | A | A | L | G | C | L | V | K | D |
| 130 | 133 | 134 | 135 | 136 | 137 | 138 | 139 | 140 | 141 | 142 | 143 | 144 | 145 | 146 |
| 134 | 135 | 136 | 137 | 138 | 139 | 140 | 141 | 142 | 143 | 144 | 145 | 146 | 147 | 148 |
| 151 | 152 | 153 | 154 | 155 | 156 | 157 | 158 | 159 | 160 | 161 | 162 | 163 | 164 | 165 |
| Y | F | P | E | P | V | T | V | S | W | N | S | G | A | L |
| 147 | 148 | 149 | 150 | 151 | 152 | 153 | 154 | 156 | 157 | 162 | 163 | 164 | 165 | 166 |
| 149 | 150 | 151 | 152 | 153 | 154 | 155 | 156 | 157 | 158 | 159 | 160 | 161 | 162 | 163 |
| 166 | 167 | 168 | 169 | 170 | 171 | 172 | 173 | 174 | 175 | 176 | 177 | 178 | 179 | 180 |
| T | S | G | V | H | T | F | P | A | V | L | Q | S | S | G |
| 167 | 168 | 169 | 171 | 172 | 173 | 174 | 175 | 176 | 177 | 178 | 179 | 180 | 182 | 183 |
| 164 | 165 | 166 | 167 | 168 | 169 | 170 | 171 | 172 | 173 | 174 | 175 | 176 | 177 | 178 |
| 181 | 182 | 183 | 184 | 185 | 186 | 187 | 188 | 189 | 190 | 191 | 192 | 193 | 194 | 195 |
| L | Y | S | L | S | S | V | V | T | V | P | S | S | S | L |
| 184 | 185 | 186 | 187 | 188 | 189 | 190 | 191 | 192 | 193 | 194 | 195 | 196 | 197 | 198 |
| 179 | 180 | 181 | 182 | 183 | 184 | 185 | 186 | 187 | 188 | 189 | 190 | 191 | 192 | 193 |
| 196 | 197 | 198 | 199 | 200 | 201 | 202 | 203 | 204 | 205 | 206 | 207 | 208 | 209 | 210 |
| G | T | Q | T | Y | I | C | N | V | N | H | K | P | S | N |
| 199 | 200 | 203 | 205 | 206 | 207 | 208 | 209 | 210 | 211 | 212 | 213 | 214 | 215 | 216 |
| 194 | 195 | 196 | 197 | 198 | 199 | 200 | 201 | 202 | 203 | 204 | 205 | 206 | 207 | 208 |
| 211 | 212 | 213 | 214 | 215 | 216 | 217 | 218 | 219 | 220 | 221 | 222 | 223 | 224 | 225 |
| T | K | V | D | K | R | V | E | P | K | S | C | D | K | T |
| 217 | 218 | 219 | 220 | 221 | 222 | 223 | 226 | 227 | 228 | 232 | 233 | 234 | 235 | 236 |
| 209 | 210 | 211 | 212 | 213 | 214 | 215 | 216 | 217 | 218 | 219 | 220 | 221 | 222 | 223 |

(continued)

| 226 | 227 | 228 | 229 | 230 | 231 | 232 | 233 | 234 | 235 | 236 | 237 | 238 | 239 | 240 |
|---|---|---|---|---|---|---|---|---|---|---|---|---|---|---|
| H | T | C | P | P | C | P | A | P | E | L | L | G | G | P |
| *237* | *238* | *239* | *240* | *241* | *242* | *243* | 244 | 245 | 246 | 247 | 248 | 249 | 250 | 251 |
| 224 | 225 | 226 | 227 | 228 | 229 | 230 | 231 | 232 | 233 | 234 | 235 | 236 | 237 | 238 |

| 241 | 242 | 243 | 244 | 245 | 246 | 247 | 248 | 249 | 250 | 251 | 252 | 253 | 254 | 255 |
|---|---|---|---|---|---|---|---|---|---|---|---|---|---|---|
| S | V | F | L | F | P | P | K | P | K | D | T | L | M | I |
| *252* | *253* | *254* | *255* | *256* | *257* | *258* | *259* | *260* | *261* | *262* | *263* | *264* | *265* | *266* |
| 239 | 240 | 241 | 242 | 243 | 244 | 245 | 246 | 247 | 248 | 249 | 250 | 251 | 252 | 253 |

| 256 | 257 | 258 | 259 | 260 | 261 | 262 | 263 | 264 | 265 | 266 | 267 | 268 | 269 | 270 |
|---|---|---|---|---|---|---|---|---|---|---|---|---|---|---|
| S | R | T | P | E | V | T | C | V | V | V | D | V | S | H |
| *267* | *268* | *269* | *270* | *271* | *272* | *273* | *274* | *275* | *276* | *277* | *278* | *279* | *280* | *281* |
| 254 | 255 | 256 | 257 | 258 | 259 | 260 | 261 | 262 | 263 | 264 | 265 | 266 | 267 | 268 |

| 271 | 272 | 273 | 274 | 275 | 276 | 277 | 278 | 279 | 280 | 281 | 282 | 283 | 284 | 285 |
|---|---|---|---|---|---|---|---|---|---|---|---|---|---|---|
| E | D | P | E | V | K | F | N | W | Y | V | D | G | V | E |
| *282* | *283* | *284* | *285* | *286* | *287* | *288* | *289* | *290* | *291* | *292* | *295* | *296* | *299* | *300* |
| 269 | 270 | 271 | 272 | 273 | 274 | 275 | 276 | 277 | 278 | 279 | 280 | 281 | 282 | 283 |

| 286 | 287 | 288 | 289 | 290 | 291 | 292 | 293 | 294 | 295 | 296 | 297 | 298 | 299 | 300 |
|---|---|---|---|---|---|---|---|---|---|---|---|---|---|---|
| V | H | N | A | K | T | K | P | R | E | E | Q | Y | N | S |
| *301* | *302* | *303* | *304* | *305* | *306* | *307* | *308* | *309* | *310* | *311* | *312* | *313* | *314* | *317* |
| 284 | 285 | 286 | 287 | 288 | 289 | 290 | 291 | 292 | 293 | 294 | 295 | 296 | 297 | 298 |

| 301 | 302 | 303 | 304 | 305 | 306 | 307 | 308 | 309 | 310 | 311 | 312 | 313 | 314 | 315 |
|---|---|---|---|---|---|---|---|---|---|---|---|---|---|---|
| T | Y | R | V | V | S | V | L | T | V | L | H | Q | D | W |
| *318* | *319* | *320* | *321* | *322* | *323* | *324* | *325* | *326* | *327* | *328* | *329* | *330* | *331* | *332* |
| 299 | 300 | 301 | 302 | 303 | 304 | 305 | 306 | 307 | 308 | 309 | 310 | 311 | 312 | 313 |

| 316 | 317 | 318 | 319 | 320 | 321 | 322 | 323 | 324 | 325 | 326 | 327 | 328 | 329 | 330 |
|---|---|---|---|---|---|---|---|---|---|---|---|---|---|---|
| L | N | G | K | E | Y | K | C | K | V | S | N | K | A | L |
| *333* | *334* | *335* | *336* | *337* | *338* | *339* | *340* | *341* | *342* | *343* | *344* | *345* | *346* | *347* |
| 314 | 315 | 316 | 317 | 318 | 319 | 320 | 321 | 322 | 323 | 324 | 325 | 326 | 327 | 328 |

| 331 | 332 A | 333 | 334 I | 335 E | 336 K | 337 T | 338 I | 339 S | 340 K | 341 A | 342 K | 343 G | 344 Q | 345 P |
|---|---|---|---|---|---|---|---|---|---|---|---|---|---|---|
| *348* | *349* | *350* | *351* | *352* | *353* | *354* | *355* | *357* | *358* | *359* | *360* | *361* | *363* | *364* |
| 329 | 330 | 331 | 332 | 333 | 334 | 335 | 336 | 337 | 338 | 339 | 340 | 341 | 342 | 343 |

| 346 | 347 | 348 | 349 | 350 | 351 | 352 | 353 | 354 | 355 | 356 | 357 | 358 | 359 | 360 |
|---|---|---|---|---|---|---|---|---|---|---|---|---|---|---|
| R | E | P | Q | V | Y | T | L | P | P | S | R | D | E | L |
| *365* | *366* | *367* | *368* | *369* | *370* | *371* | *372* | *373* | *374* | *375* | *376* | *377* | *378* | *381* |
| 344 | 345 | 346 | 347 | 348 | 349 | 350 | 351 | 352 | 353 | 354 | 355 | 356 | 357 | 358 |

| 361 | 362 | 363 | 364 | 365 | 366 | 367 | 368 | 369 | 370 | 371 | 372 | 373 | 374 | 375 |
|---|---|---|---|---|---|---|---|---|---|---|---|---|---|---|
| T | K | N | Q | V | S | L | T | C | L | V | K | G | F | Y |
| *382* | *383* | *384* | *385* | *386* | *387* | *388* | *389* | *390* | *391* | *392* | *393* | *394* | *395* | *396* |
| 359 | 360 | 361 | 362 | 363 | 364 | 365 | 366 | 367 | 368 | 369 | 370 | 371 | 372 | 373 |

| 376 | 377 | 378 | 379 | 380 | 381 | 382 | 383 | 384 | 385 | 386 | 387 | 388 | 389 | 390 |
|---|---|---|---|---|---|---|---|---|---|---|---|---|---|---|
| P | S | D | I | A | V | E | W | E | S | N | G | Q | P | E |
| *397* | *398* | *399* | *400* | *401* | *402* | *405* | *406* | *407* | *408* | *410* | *411* | *414* | *415* | *416* |

(continued)

| 374 | 375 | 376 | 377 | 378 | 379 | 380 | 381 | 382 | 383 | 384 | 385 | 386 | 387 | 388 |
|-----|-----|-----|-----|-----|-----|-----|-----|-----|-----|-----|-----|-----|-----|-----|
| **391** | **392** | **393** | **394** | **395** | **396** | **397** | **398** | **399** | **400** | **401** | **402** | **403** | **404** | **405** |
| N | N | Y | K | T | T | P | P | V | L | D | S | D | G | S |
| 417 | 418 | 419 | 420 | 421 | 422 | 423 | 424 | 425 | 426 | 427 | 428 | 430 | 433 | 434 |
| 389 | 390 | 391 | 392 | 393 | 394 | 395 | 396 | 397 | 398 | 399 | 400 | 401 | 402 | 403 |
| **406** | **407** | **408** | **409** | **410** | **411** | **412** | **413** | **414** | **415** | **416** | **417** | **418** | **419** | **420** |
| F | F | L | Y | S | K | L | T | V | D | K | S | R | W | Q |
| 435 | 436 | 437 | 438 | 439 | 440 | 441 | 442 | 443 | 444 | 445 | 446 | 447 | 448 | 449 |
| 404 | 405 | 406 | 407 | 408 | 409 | 410 | 411 | 412 | 413 | 414 | 415 | 416 | 417 | 418 |
| **421** | **422** | **423** | **424** | **425** | **426** | **427** | **428** | **429** | **430** | **431** | **432** | **433** | **434** | **435** |
| Q | G | N | V | F | S | C | S | V | M | H | E | A | L | H |
| 450 | 451 | 452 | 453 | 454 | 455 | 456 | 457 | 458 | 459 | 460 | 461 | 462 | 463 | 464 |
| 419 | 420 | 421 | 422 | 423 | 424 | 425 | 426 | 427 | 428 | 429 | 430 | 431 | 432 | 433 |
| **436** | **437** | **438** | **439** | **440** | **441** | **442** | **443** | **444** | **445** | **446** | **447** | **448** | **449** | |
| N | H | Y | T | Q | K | S | L | S | L | S | P | G | K | |
| 465 | 466 | 467 | 468 | 469 | 470 | 471 | 472 | 473 | 474 | 475 | 476 | 477 | 478 | |
| 434 | 435 | 436 | 437 | 438 | 439 | 440 | 441 | 442 | 443 | 444 | 445 | 446 | - | |

As used herein, "antibody hinge region" or "hinge region" refers to a polypeptide region that exists naturally in the heavy chain of the gamma, delta and alpha antibody isotypes, between the $C_H1$ and $C_H2$ domains that has no homology with the other antibody domains. This region is rich in proline residues and gives the IgG, IgD and IgA antibodies flexibility, allowing the two "arms" (each containing one antibody combining site) of the Fab portion to be mobile, assuming various angles with respect to one another as they bind antigen. This flexibility allows the Fab arms to move in order to align the antibody combining sites to interact with epitopes on cell surfaces or other antigens. Two interchain disulfide bonds within the hinge region stabilize the interaction between the two heavy chains. In some embodiments provided herein, the synthetically produced antibody fragments contain one or more hinge regions, for example, to promote stability via interactions between two antibody chains. Hinge regions are exemplary of dimerization domains.

As used herein, the phrase "derived from" when referring to antibody fragments derived from another antibody, such as a monoclonal antibody, refers to the engineering of antibody fragments (*e.g.*, Fab, F(ab'), F(ab')$_2$, single-chain Fv (scFv), Fv, dsFv, diabody, Fd and Fd' fragments) that retain the binding specificity of the original antibody. Such fragments can be derived by a variety of methods known in the art, including, but not limited to, enzymatic cleavage, chemical crosslinking, recombinant means or combinations thereof. Generally, the derived antibody fragment shares the identical or substantially identical heavy chain variable region ($V_H$) and light chain variable region ($V_L$) of the parent antibody, such that the antibody fragment and the parent antibody bind the same epitope.

As used herein, a "parent antibody" or "source antibody" refers the to an antibody from which an antibody fragment (e.g., Fab, F(ab'), F(ab)$_2$, single-chain Fv (scFv), Fv, dsFv, diabody, Fd and Fd' fragments) is derived.

As used herein, the term "epitope" refers to any antigenic determinant on an antigen to which the paratope of an antibody binds. Epitopic determinants typically contain chemically active surface groupings of molecules such as amino acids or sugar side chains and typically have specific three dimensional structural characteristics, as well as specific charge characteristics.

As used herein, humanized antibodies refer to antibodies that are modified to include "human" sequences of amino acids so that administration to a human does not provoke an immune response. A humanized antibody typically contains complementarity determining regions (CDRs or hypervariable loops) derived from a non-human species immunoglobulin and the remainder of the antibody molecule derived mainly from a human immunoglobulin. Methods for preparation of such antibodies are known. For example, DNA encoding a monoclonal antibody can be altered by recombinant DNA techniques to encode an antibody in which the amino acid composition of the non-variable regions is based on human antibodies. Methods for identifying such regions are known, including computer programs, which are designed for identifying the variable and non-variable regions of immunoglobulins. Hence, in general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. With respect to the variable region, a humanized antibody typically is one that exhibits greater than 56% sequence identity, such as at least 57%, 58%, 59%, 60%, 65%, 70% or more sequence identity, to the closest $V_H$ region derived from a human $V_H$ gene segment, and at least 75% sequence identity, such as at least 76%, 77%, 78%, 79%, 80%, 85% or more sequence identity, to the closest $V_L$ region derived from a human $V_L$ gene segment. Hence, a humanized antibody exhibits at least 1%, 2%, 3%, 4%, 5%, 6%, 7%, 8%, 9%, 10%, 11%, 12%, 13%, 14%, 15% or more sequence identity to its closest human V region derived from V germline segment than the parent or reference or unmodified antibody prior to humanization.

As used herein, germline gene segments refer to immunoglobulin (Ig) variable (V), diversity (D) and junction (J) or constant (C) genes from the germline that encode immunoglobulin heavy or light (kappa and lambda) chains. There are multiple V, D, J and C gene segments in the germline, but gene rearrangement results in only one segment of each occurring in each functional rearranged gene. For example, a functionally rearranged heavy chain contains one V, one D and one J and a functionally rearranged light chain gene contains one V and one J. Hence, these gene segments are carried in the germ cells but cannot be transcribed and translated into heavy and light chains until they are arranged into functional genes. During B-cell differentiation in the bone marrow, these gene segments are randomly shuffled by a dynamic genetic system capable of generating more than $10^{10}$ specificities. For purposes herein, the gene segments are rearranged *in vitro* by combination or compilation of the individual germline segments.

Reference to a variable germline segment herein refers to V, D and J groups, subgroups, genes or alleles thereof. Gene segment sequences are accessible from known database (e.g., National Center for Biotechnology Information (NCBI), the international ImMunoGeneTics information system® (IMGT), the Kabat database and the Tomlinson's VBase database (Lefranc (2003) Nucleic Acids Res., 31:307-310; Martin et al., Bioinformatics Tools for Antibody Engineering in Handbook of Therapeutic Antibodies, Wiley-VCH (2007), pp. 104-107; see also published International PCT Application No. WO2010/054007).

As used herein, a "group" with reference to a germline segment refers to a core coding region from an immunoglobulin, i.e. a variable (V) gene, diversity (D) gene, joining (J) gene or constant (C) gene encoding a heavy or light chain. Exemplary of germline segment groups include $V_H$, $D_H$, $J_H$, $V_L$ ($Y_\kappa$ or $V_\lambda$) and $J_L$ ($J_\kappa$ or $J_\lambda$).

As used herein, a "subgroup" with reference to a germline segment refers to a set of sequences that are defined by nucleotide sequence similarity or identity. Generally, a subgroup is a set of genes that belong to the same group [V, D, J or C], in a given species, and that share at least 75% identity at the nucleotide level. Subgroups are classified based on IMGT nomenclature (imgt.cines.fr; see e.g., Lefranc et al. (2008) Briefings in Bioinformatics, 9:263-275). Generally, a subgroup represent a multigene family.

As used herein, an allele of a gene refer to germline sequences that have sequence polymorphism due to one or more nucleotide differences in the coding region compared to a reference gene sequence (e.g. substitutions, insertions or deletions). Thus, IG sequences that belong to the same subgroup can be highly similar in their coding sequence, but nonetheless exhibit high polymorphism. Subgroup alleles are classified based on IMGT nomenclature with an asterisk(*) followed by a two figure number.

As used herein, a "family" with reference to a germline segment refers to sets of germline segment sequences that are defined by amino acid sequence similarity or identity. Generally, a germline family includes all alleles of a gene.

As used herein, reference to a V gene segment "derived from a germline segment" refers to the corresponding nucleotides in a VH or VL nucleic acid sequence, that by recombination events, derived from a V germline gene ($V_H$ or $V_L$ germline segment).

As used herein, reference to a V region in an antibody heavy chain ($V_H$ region) or light chain ($V_L$ region), or portion or fragment thereof, refers to amino acids encoded by nucleotides that, by recombination events, derive from a corresponding V germline segment gene.

As used herein, a multimerization domain refers to a sequence of amino acids that promotes stable interaction of a polypeptide molecule with one or more additional polypeptide molecules, each containing a complementary multimerization domain, which can be the same or a different multimerization domain to form a stable multimer with the first domain. Generally, a polypeptide is joined directly or indirectly to the multimerization domain. Exemplary multimerization domains include the immunoglobulin sequences or portions thereof, leucine zippers, hydrophobic regions, hydrophilic regions, and compatible protein-protein interaction domains. The multimerization domain, for example, can be an immunoglobulin constant region or domain, such as, for example, the Fc domain or portions thereof from IgG, including IgG1, IgG2, IgG3 or IgG4 subtypes, IgA, IgE, IgD and IgM and modified forms thereof.

As used herein, dimerization domains are multimerization domains that facilitate interaction between two polypeptide sequences (such as, but not limited to, antibody chains). Dimerization domains include, but are not limited to, an amino acid sequence containing a cysteine residue that facilitates formation of a disulfide bond between two polypeptide sequences, such as all or part of a full-length antibody hinge region, or one or more dimerization sequences, which are sequences of amino acids known to promote interaction between polypeptides (e.g., leucine zippers, GCN4 zippers).

As used herein, "Fc" or "Fc region" or "Fc domain" refers to a polypeptide containing the constant region of an antibody heavy chain, excluding the first constant region immunoglobulin domain. Thus, Fc refers to the last two constant region immunoglobulin domains of IgA, IgD, and IgE, or the last three constant region immunoglobulin domains of IgE and IgM. Optionally, an Fc domain can include all or part of the flexible hinge N-terminal to these domains. For IgA and IgM, Fc can include the J chain. For an exemplary Fc domain of IgG, Fc contains immunoglobulin domains Cγ2 and Cγ3, and optionally, all or part of the hinge between Cγ1 and Cγ2. The boundaries of the Fc region can vary, but typically, include at least part of the hinge region. In addition, Fc also includes any allelic or species variant or any variant or modified form, such as any variant or modified form that alters the binding to an FcR or alters an Fc-mediated effector function.

As used herein, "Fc chimera" refers to a chimeric polypeptide in which one or more polypeptides is linked, directly or indirectly, to an Fc region or a derivative thereof. Typically, an Fc chimera combines the Fc region of an immunoglobulin with another polypeptide. Derivatives of or modified Fc polypeptides are known to those of skill in the art.

As used herein, a chimeric polypeptide refers to a polypeptide that contains portions from at least two different polypeptides or from two non-contiguous portions of a single polypeptide. Thus, a chimeric polypeptide generally includes a sequence of amino acid residues from all or part of one polypeptide and a sequence of amino acids from all or part of another different polypeptide. The two portions can be linked directly or indirectly and can be linked via peptide bonds, other covalent bonds or other non-covalent interactions of sufficient strength to maintain the integrity of a substantial portion of the chimeric polypeptide under equilibrium conditions and physiologic conditions, such as in isotonic pH 7 buffered saline.

As used herein, a fusion protein is a polypeptide engineered to contain sequences of amino acids corresponding to two distinct polypeptides, which are joined together, such as by expressing the fusion protein from a vector containing two nucleic acids, encoding the two polypeptides, in close proximity, *e.g.*, adjacent, to one another along the length of the vector. Accordingly, a fusion protein refers to a chimeric protein containing two, or portions from two, or more proteins or peptides that are linked directly or indirectly via peptide bonds. The two molecules can be adjacent in the construct or separated by a linker, or spacer polypeptide.

As used herein, "linker" or "spacer" peptide refers to short sequences of amino acids that join two polypeptide sequences (or nucleic acid encoding such an amino acid sequence). "Peptide linker" refers to the short sequence of amino acids joining the two polypeptide sequences. Exemplary of polypeptide linkers are linkers joining a peptide transduction domain to an antibody or linkers joining two antibody chains in a synthetic antibody fragment such as an scFv fragment. Linkers are well-known and any known linkers can be used in the provided methods. Exemplary of polypeptide linkers are (Gly-Ser)$_n$ amino acid sequences, with some Glu or Lys residues dispersed throughout to increase solubility. Other exemplary linkers are described herein; any of these and other known linkers can be used with the provided compositions and methods.

As used herein, a "tag" or an "epitope tag" refers to a sequence of amino acids, typically added to the N- or C-terminus of a polypeptide, such as an antibody provided herein. The inclusion of tags fused to a polypeptide can facilitate polypeptide purification and/or detection. Typically, a tag or tag polypeptide refers to a polypeptide that has enough residues to provide an epitope recognized by an antibody or can serve for detection or purification, yet is short enough such that it does not interfere with activity of the polypeptide to which it is linked. The tag polypeptide typically is sufficiently unique so an antibody that specifically binds thereto does not substantially cross-react with epitopes in the polypeptide to which it is linked. Suitable tag polypeptides generally have at least 5 or 6 amino acid residues and usually between about 8-50 amino acid residues, typically between 9-30 residues. The tags can be linked to one or more chimeric polypeptides in a multimer and permit detection of the multimer or its recovery from a sample or mixture. Such tags are well known and can be readily synthesized and designed. Exemplary tag polypeptides include those used for affinity purification and include, FLAG tags, His tags, the influenza hemagglutinin (HA) tag polypeptide and its antibody 12CA5, (Field et al. (1988) Mol. Cell. Biol. 8:2159-2165); the c-myc tag and the 8F9, 3C7, 6E10, G4, B7 and 9E10 antibodies thereto (see, *e.g.,* Evan et al. (1985) Molecular and Cellular Biology 5 :3610-3616); and the Herpes Simplex virus glycoprotein D (gD) tag and its antibody (Paborsky et al. (1990) Protein Engineering 3:547-553). An antibody used to detect an epitope-tagged antibody is typically referred to herein as a secondary antibody.

As used herein, a label or detectable moiety is a detectable marker (e.g., a fluorescent molecule, chemiluminescent molecule, a bioluminescent molecule, a contrast agent (*e.g.,* a metal), a radionuclide, a chromophore, a detectable peptide, or an enzyme that catalyzes the formation of a detectable product) that can be attached or linked directly or indirectly to a molecule (*e.g.,* an antibody or antigen-binding fragment thereof, such as an anti-EGFR antibody or antigen-binding fragment thereof provided herein) or associated therewith and can be detected *in vivo* and/or *in vitro.* The detection method can be any method known in the art, including known *in vivo* and/or *in vitro* methods of detection (*e.g.,* imaging by visual inspection, magnetic resonance (MR) spectroscopy, ultrasound signal, X-ray, gamma ray spectroscopy (e.g., positron emission tomography (PET) scanning, single-photon emission computed tomography (SPECT)), fluorescence spectroscopy or absorption). Indirect detection refers to measurement of a physical phenomenon, such as energy or particle emission or absorption, of an atom, molecule or composition that binds directly or indirectly to the detectable moiety (*e.g.*, detection of a labeled secondary antibody or antigen-binding fragment thereof that binds to a primary antibody (*e.g.*, an anti-EGFR antibody or antigen-binding fragment thereof provided herein).

As used herein, "nucleic acid" refers to at least two linked nucleotides or nucleotide derivatives, including a deoxyribonucleic acid (DNA) and a ribonucleic acid (RNA), joined together, typically by phosphodiester linkages. Also included in the term "nucleic acid" are analogs of nucleic acids such as peptide nucleic acid (PNA), phosphorothioate DNA, and other such analogs and derivatives or combinations thereof. Nucleic acids also include DNA and RNA derivatives containing, for example, a nucleotide analog or a "backbone" bond other than a phosphodiester bond, for example, a phosphotriester bond, a phosphoramidate bond, a phosphorothioate bond, a thioester bond, or a peptide bond (peptide nucleic acid). The term also includes, as equivalents, derivatives, variants and analogs of either RNA or DNA made from nucleotide analogs, single (sense or antisense) and double-stranded nucleic acids. Deoxyribonucleotides include deoxyadenosine, deoxycytidine, deoxyguanosine and deoxythymidine. For RNA, the uracil base is uridine.

As used herein, an isolated nucleic acid molecule is one which is separated from other nucleic acid molecules which are present in the natural source of the nucleic acid molecule. An "isolated" nucleic acid molecule, such as a cDNA molecule, can be substantially free of other cellular material, or culture medium when produced by recombinant techniques, or substantially free of chemical precursors or other chemicals when chemically synthesized. Exemplary isolated nucleic acid molecules provided herein include isolated nucleic acid molecules encoding an antibody or antigen-binding fragments provided.

As used herein, "operably linked" with reference to nucleic acid sequences, regions, elements or domains means that the nucleic acid regions are functionally related to each other. For example, nucleic acid encoding a leader peptide can be operably linked to nucleic acid encoding a polypeptide, whereby the nucleic acids can be transcribed and translated to express a functional fusion protein, wherein the leader peptide effects secretion of the fusion polypeptide. In some instances, the nucleic acid encoding a first polypeptide (e.g., a leader peptide) is operably linked to nucleic acid encoding a second polypeptide and the nucleic acids are transcribed as a single mRNA transcript, but translation of the mRNA transcript can result in one of two polypeptides being expressed. For example, an amber stop codon can be located between the nucleic acid encoding the first polypeptide and the nucleic acid encoding the second polypeptide, such that, when introduced into a partial amber suppressor cell, the resulting single mRNA transcript can be translated to produce either a fusion protein containing the first and second polypeptides, or can be translated to produce only the first polypeptide. In another example, a promoter can be operably linked to nucleic acid encoding a polypeptide, whereby the promoter regulates or mediates the transcription of the nucleic acid.

As used herein, "synthetic," with reference to, for example, a synthetic nucleic acid molecule or a synthetic gene or a synthetic peptide refers to a nucleic acid molecule or polypeptide molecule that is produced by recombinant methods and/or by chemical synthesis methods.

As used herein, the residues of naturally occurring $\alpha$-amino acids are the residues of those 20 $\alpha$-amino acids found in nature which are incorporated into protein by the specific recognition of the charged tRNA molecule with its cognate mRNA codon in humans.

As used herein, "polypeptide" refers to two or more amino acids covalently joined. The terms "polypeptide" and "protein" are used interchangeably herein.

As used herein, a "peptide" refers to a polypeptide that is from 2 to about or 40 amino acids in length.

As used herein, an "amino acid" is an organic compound containing an amino group and a carboxylic acid group. A polypeptide contains two or more amino acids. For purposes herein, amino acids contained in the antibodies provided include the twenty naturally-occurring amino acids (Table 3), non-natural amino acids, and amino acid analogs (e.g., amino acids wherein the $\alpha$-carbon has a side chain). As used herein, the amino acids, which occur in the various amino acid sequences of polypeptides appearing herein, are identified according to their well-known, three-letter or one-letter abbreviations (see Table 3). The nucleotides, which occur in the various nucleic acid molecules and fragments, are designated with the standard single-letter designations used routinely in the art.

As used herein, "amino acid residue" refers to an amino acid formed upon chemical digestion (hydrolysis) of a polypeptide at its peptide linkages. The amino acid residues described herein are generally in the "L" isomeric form. Residues in the "D" isomeric form can be substituted for any L-amino acid residue, as long as the desired functional property is retained by the polypeptide. NH$_2$ refers to the free amino group present at the amino terminus of a polypeptide. COOH refers to the free carboxy group present at the carboxyl terminus of a polypeptide. In keeping with standard polypeptide nomenclature described in J. Biol. Chem., 243:3557-59 (1968) and adopted at 37 C.F.R. §§ 1.821 - 1.822, abbreviations for amino acid residues are shown in Table 3:

**TABLE 3 - Table of Correspondence**

| SYMBOL | | |
|---|---|---|
| 1-Letter | 3-Letter | AMINO ACID |
| Y | Tyr | Tyrosine |
| G | Gly | Glycine |
| F | Phe | Phenylalanine |
| M | Met | Methionine |
| A | Ala | Alanine |
| S | Ser | Serine |
| I | Ile | Isoleucine |
| L | Leu | Leucine |
| T | Thr | Threonine |
| V | Val | Valine |
| P | Pro | Proline |
| K | Lys | Lysine |
| H | His | Histidine |
| Q | Gln | Glutamine |
| E | Glu | Glutamic acid |
| Z | Glx | Glutamic Acid and/or Glutamine |
| W | Trp | Tryptophan |
| R | Arg | Arginine |
| D | Asp | Aspartic acid |
| N | Asn | Asparagine |
| B | Asx | Aspartic Acid and/or Asparagine |
| C | Cys | Cysteine |
| X | Xaa | Unknown or other |

All sequences of amino acid residues represented herein by a formula have a left to right orientation in the conventional direction of amino-terminus to carboxyl-terminus. In addition, the phrase "amino acid residue" is defined to include the amino acids listed in the Table of Correspondence (Table 3), modified, non-natural and unusual amino acids. Furthermore, a dash at the beginning or end of an amino acid residue sequence indicates a peptide bond to a further sequence of one or more amino acid residues or to an amino-terminal group such as $NH_2$ or to a carboxyl-terminal group such as COOH.

In a peptide or protein, suitable conservative substitutions of amino acids are known to those of skill in this art and generally can be made without altering a biological activity of a resulting molecule. Those of skill in this art recognize that, in general, single amino acid substitutions in non-essential regions of a polypeptide do not substantially alter biological activity (see, e.g., Watson et al., Molecular Biology of the Gene, 4th Edition, 1987, The Benjamin/Cummings Pub. co., p.224).

Such substitutions can be made in accordance with the exemplary substitutions set forth in Table 4 as follows:

**TABLE 4**

| Original residue | Conservative substitution |
|---|---|
| Ala (A) | Gly; Ser |
| Arg (R) | Lys |

(continued)

| Original residue | Conservative substitution |
|---|---|
| Asn (N) | Gln; His |
| Cys (C) | Ser |
| Gln (Q) | Asn |
| Glu (E) | Asp |
| Gly (G) | Ala; Pro |
| His (H) | Asn; Gln |
| Ile (I) | Leu; Val |
| Leu (L) | Ile; Val |
| Lys (K) | Arg; Gln; Glu |
| Met (M) | Leu; Tyr; Ile |
| Phe (F) | Met; Leu; Tyr |
| Ser (S) | Thr |
| Thr (T) | Ser |
| Trp (W) | Tyr |
| Tyr (Y) | Trp; Phe |
| Val (V) | Ile; Leu |

Other substitutions also are permissible and can be determined empirically or in accord with other known conservative or non-conservative substitutions.

As used herein, "naturally occurring amino acids" refer to the 20 L-amino acids that occur in polypeptides.

As used herein, the term "non-natural amino acid" refers to an organic compound that has a structure similar to a natural amino acid but has been modified structurally to mimic the structure and reactivity of a natural amino acid. Non-naturally occurring amino acids thus include, for example, amino acids or analogs of amino acids other than the 20 naturally occurring amino acids and include, but are not limited to, the D-stereoisomers of amino acids. Exemplary non-natural amino acids are known to those of skill in the art, and include, but are not limited to, 2-Aminoadipic acid (Aad), 3-Aminoadipic acid (Baad), β-alanine/β -Amino-propionic acid (Bala), 2-Aminobutyric acid (Abu), 4-Aminobutyric acid/piperidinic acid (4Abu), 6-Aminocaproic acid (Acp), 2-Aminoheptanoic acid (Ahe), 2-Aminoisobutyric acid (Aib), 3-Aminoisobutyric acid (Baib), 2-Aminopimelic acid (Apm), 2,4-Diaminobutyric acid (Dbu), Desmosine (Des), 2,2'-Diaminopimelic acid (Dpm), 2,3-Diaminopropionic acid (Dpr), N-Ethylglycine (EtGly), N-Ethylasparagine (EtAsn), Hydroxylysine (Hyl), allo-Hydroxylysine (Ahyl), 3-Hydroxyproline (3Hyp), 4-Hydroxyproline (4Hyp), Isodesmosine (Ide), allo-Isoleucine (Aile), N-Methylglycine, sarcosine (MeGly), N-Methylisoleucine (MeIle), 6-N-Methyllysine (MeLys), N-Methylvaline (MeVal), Norvaline (Nva), Norleucine (Nle), and Ornithine (Orn).

As used herein, a DNA construct is a single or double stranded, linear or circular DNA molecule that contains segments of DNA combined and juxtaposed in a manner not found in nature. DNA constructs exist as a result of human manipulation, and include clones and other copies of manipulated molecules.

As used herein, a DNA segment is a portion of a larger DNA molecule having specified attributes. For example, a DNA segment encoding a specified polypeptide is a portion of a longer DNA molecule, such as a plasmid or plasmid fragment, which, when read from the 5' to 3' direction, encodes the sequence of amino acids of the specified polypeptide.

As used herein, the term polynucleotide means a single- or double-stranded polymer of deoxyribonucleotides or ribonucleotide bases read from the 5' to the 3' end. Polynucleotides include RNA and DNA, and can be isolated from natural sources, synthesized *in vitro,* or prepared from a combination of natural and synthetic molecules. The length of a polynucleotide molecule is given herein in terms of nucleotides (abbreviated "nt") or base pairs (abbreviated "bp"). The term nucleotides is used for single- and double-stranded molecules where the context permits. When the term is applied to double-stranded molecules it is used to denote overall length and will be understood to be equivalent to the term base pairs. It will be recognized by those skilled in the art that the two strands of a double-stranded polynucleotide can differ slightly in length and that the ends thereof can be staggered; thus all nucleotides within a double-stranded polynucleotide molecule cannot be paired. Such unpaired ends will, in general, not exceed 20 nucleotides in length.

As used herein, production by recombinant means by using recombinant DNA methods means the use of the well known methods of molecular biology for expressing proteins encoded by cloned DNA.

As used herein, "expression" refers to the process by which polypeptides are produced by transcription and translation of polynucleotides. The level of expression of a polypeptide can be assessed using any method known in art, including, for example, methods of determining the amount of the polypeptide produced from the host cell. Such methods can include, but are not limited to, quantitation of the polypeptide in the cell lysate by ELISA, Coomassie blue staining following gel electrophoresis, Lowry protein assay and Bradford protein assay.

As used herein, a "host cell" is a cell that is used in to receive, maintain, reproduce and amplify a vector. A host cell also can be used to express the polypeptide encoded by the vector. The nucleic acid contained in the vector is replicated when the host cell divides, thereby amplifying the nucleic acids.

As used herein, a "vector" is a replicable nucleic acid from which one or more heterologous proteins, can be expressed when the vector is transformed into an appropriate host cell. Reference to a vector includes those vectors into which a nucleic acid encoding a polypeptide or fragment thereof can be introduced, typically by restriction digest and ligation. Reference to a vector also includes those vectors that contain nucleic acid encoding a polypeptide, such as a modified anti-EGFR antibody. The vector is used to introduce the nucleic acid encoding the polypeptide into the host cell for amplification of the nucleic acid or for expression/display of the polypeptide encoded by the nucleic acid. The vectors typically remain episomal, but can be designed to effect integration of a gene or portion thereof into a chromosome of the genome. Also contemplated are vectors that are artificial chromosomes, such as yeast artificial chromosomes and mammalian artificial chromosomes. Selection and use of such vehicles are well known to those of skill in the art. A vector also includes "virus vectors" or "viral vectors." Viral vectors are engineered viruses that are operatively linked to exogenous genes to transfer (as vehicles or shuttles) the exogenous genes into cells.

As used herein, an "expression vector" includes vectors capable of expressing DNA that is operatively linked with regulatory sequences, such as promoter regions, that are capable of effecting expression of such DNA fragments. Such additional segments can include promoter and terminator sequences, and optionally can include one or more origins of replication, one or more selectable markers, an enhancer, a polyadenylation signal, and the like. Expression vectors are generally derived from plasmid or viral DNA, or can contain elements of both. Thus, an expression vector refers to a recombinant DNA or RNA construct, such as a plasmid, a phage, recombinant virus or other vector that, upon introduction into an appropriate host cell, results in expression of the cloned DNA. Appropriate expression vectors are well known to those of skill in the art and include those that are replicable in eukaryotic cells and/or prokaryotic cells and those that remain episomal or those which integrate into the host cell genome.

As used herein, "primary sequence" refers to the sequence of amino acid residues in a polypeptide or the sequence of nucleotides in a nucleic acid molecule.

As used herein, "sequence identity" refers to the number of identical or similar amino acids or nucleotide bases in a comparison between a test and a reference polypeptide or polynucleotide. Sequence identity can be determined by sequence alignment of nucleic acid or protein sequences to identify regions of similarity or identity. For purposes herein, sequence identity is generally determined by alignment to identify identical residues. The alignment can be local or global. Matches, mismatches and gaps can be identified between compared sequences. Gaps are null amino acids or nucleotides inserted between the residues of aligned sequences so that identical or similar characters are aligned. Generally, there can be internal and terminal gaps. When using gap penalties, sequence identity can be determined with no penalty for end gaps (*e.g.* terminal gaps are not penalized). Alternatively, sequence identity can be determined without taking into account gaps as the number of identical positions/length of the total aligned sequence x 100.

As used herein, a "global alignment" is an alignment that aligns two sequences from beginning to end, aligning each letter in each sequence only once. An alignment is produced, regardless of whether or not there is similarity or identity between the sequences. For example, 50% sequence identity based on "global alignment" means that in an alignment of the full sequence of two compared sequences each of 100 nucleotides in length, 50% of the residues are the same. It is understood that global alignment also can be used in determining sequence identity even when the length of the aligned sequences is not the same. The differences in the terminal ends of the sequences will be taken into account in determining sequence identity, unless the "no penalty for end gaps" is selected. Generally, a global alignment is used on sequences that share significant similarity over most of their length. Exemplary algorithms for performing global alignment include the Needleman-Wunsch algorithm (Needleman et al. J. Mol. Biol. 48: 443 (1970). Exemplary programs for performing global alignment are publicly available and include the Global Sequence Alignment Tool available at the National Center for Biotechnology Information (NCBI) website (ncbi.nlm.nih.gov/), and the program available at deepc2.psi.iastate.edu/aat/align/align.html.

As used herein, a "local alignment" is an alignment that aligns two sequence, but only aligns those portions of the sequences that share similarity or identity. Hence, a local alignment determines if sub-segments of one sequence are present in another sequence. If there is no similarity, no alignment will be returned. Local alignment algorithms include BLAST or Smith-Waterman algorithm (Adv. Appl. Math. 2: 482 (1981)). For example, 50% sequence identity based on "local alignment" means that in an alignment of the full sequence of two compared sequences of any length, a region of similarity or identity of 100 nucleotides in length has 50% of the residues that are the same in the region of similarity or identity.

For purposes herein, sequence identity can be determined by standard alignment algorithm programs used with default gap penalties established by each supplier. Default parameters for the GAP program can include: (1) a unary comparison matrix (containing a value of 1 for identities and 0 for non identities) and the weighted comparison matrix of Gribskov et al. Nucl. Acids Res. 14: 6745 (1986), as described by Schwartz and Dayhoff, eds., Atlas of Protein Sequence and Structure, National Biomedical Research Foundation, pp. 353-358 (1979); (2) a penalty of 3.0 for each gap and an additional 0.10 penalty for each symbol in each gap; and (3) no penalty for end gaps. Whether any two nucleic acid molecules have nucleotide sequences or any two polypeptides have amino acid sequences that are at least 80%, 85%, 90%, 95%, 96%, 97%, 98% or 99% "identical," or other similar variations reciting a percent identity, can be determined using known computer algorithms based on local or global alignment (*see e.g.,* wikipedia.org/wiki/Sequence_alignment_software, providing links to dozens of known and publicly available alignment databases and programs). Generally, for purposes herein sequence identity is determined using computer algorithms based on global alignment, such as the Needleman-Wunsch Global Sequence Alignment tool available from NCBI/BLAST (blast.ncbi.nlm.nih.gov/Blast.cgi?CMD=Web&Page_TYPE=BlastHome); LAlign (William Pearson implementing the Huang and Miller algorithm (Adv. Appl. Math. (1991) 12:337-357)); and program from Xiaoqui Huang available at deepc2.psi.iastate.edu/aat/align/align.html. Typically, the full-length sequence of each of the compared polypeptides or nucleotides is aligned across the full-length of each sequence in a global alignment. Local alignment also can be used when the sequences being compared are substantially the same length.

Therefore, as used herein, the term "identity" represents a comparison or alignment between a test and a reference polypeptide or polynucleotide. In one non-limiting example, "at least 90% identical to" refers to percent identities from 90 to 100% relative to the reference polypeptide or polynucleotide. Identity at a level of 90% or more is indicative of the fact that, assuming for exemplification purposes a test and reference polypeptide or polynucleotide length of 100 amino acids or nucleotides are compared, no more than 10% (*i.e.,* 10 out of 100) of amino acids or nucleotides in the test polypeptide or polynucleotide differ from those of the reference polypeptide. Similar comparisons can be made between a test and reference polynucleotides. Such differences can be represented as point mutations randomly distributed over the entire length of an amino acid sequence or they can be clustered in one or more locations of varying length up to the maximum allowable, *e.g.*, 10/100 amino acid difference (approximately 90% identity). Differences also can be due to deletions or truncations of amino acid residues. Differences are defined as nucleic acid or amino acid substitutions, insertions or deletions. Depending on the length of the compared sequences, at the level of homologies or identities above about 85-90%, the result can be independent of the program and gap parameters set; such high levels of identity can be assessed readily, often without relying on software.

As used herein, a disulfide bond (also called an S-S bond or a disulfide bridge) is a single covalent bond derived from the coupling of thiol groups. Disulfide bonds in proteins are formed between the thiol groups of cysteine residues, and stabilize interactions between polypeptide domains, such as antibody domains.

As used herein, "coupled" or "conjugated" means attached via a covalent or noncovalent interaction.

As used herein, the phrase "conjugated to an antibody" or "linked to an antibody" or grammatical variations thereof, when referring to the attachment of a moiety to an antibody or antigen-binding fragment thereof, such as a diagnostic or therapeutic moiety, means that the moiety is attached to the antibody or antigen-binding fragment thereof by any known means for linking peptides, such as, for example, by production of fusion protein by recombinant means or post-translationally by chemical means. Conjugation can employ any of a variety of linking agents to effect conjugation, including, but not limited to, peptide or compound linkers or chemical cross-linking agents.

As used herein, "Maytansinoid drug moiety" means the substructure of an antibody-drug conjugate that has the structure of a maytansine compound. Maytansine was first isolated from the east African shrub *Maytenus serrata* (U.S. Pat. No. 3,896,111). Subsequently, it was discovered that certain microbes also produce maytansinoids, such as maytansinol and C-3 maytansinol esters (U.S. Pat. No. 4,151,042). Synthetic maytansinol and maytansinol analogues have been reported. See U.S. Pat. Nos. 4,137,230; 4,248,870; 4,256,746; 4,260,608; 4,265,814; 4,294,757; 4,307,016; 4,308,268; 4,308,269; 4,309,428; 4,313,946; 4,315,929; 4,317,821; 4,322,348; 4,331,598; 4,361,650; 4,364,866; 4,424,219; 4,450,254; 4,362,663; and 4,371,533, and Kawai et al (1984) Chem. Pharm. Bull. 3441-3451).

A "free cysteine amino acid" refers to a cysteine amino acid residue that has a thiol functional group (-SH), and is not paired as an intramolecular or intermolecular disulfide bridge. It can be engineered into a parent antibody.

As used herein, "Linker", "Linker Unit", or "link" means a peptide or chemical moiety containing a chain of atoms that covalently attaches an antibody to a drug moiety or therapeutic moiety.

As used herein, "Antibody-dependent cell-mediated cytotoxicity" and "ADCC" refer to a cell-mediated reaction in which nonspecific cytotoxic cells that express Fc receptors (FcRs) (e.g., Natural Killer (NK) cells, neutrophils, and macrophages) recognize bound antibody on a target cell and subsequently cause lysis of the target cell. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, (1991) Annu. Rev.

Immunol, 9:457-92. To assess ADCC activity of a molecule of interest, an in vitro ADCC assay may be performed (U.S. Pat. No. 5,500,362; U.S. Pat. No. 5,821,337). Useful effector cells for such assays include peripheral blood mononuclear cells (PBMC) and Natural Killer (NK) cells. Alternatively, or additionally, ADCC activity of the molecule of interest may be assessed in vivo, e.g., in a animal model such as that disclosed in Clynes et al (1998) PNAS (USA), 95:652-656.

As used herein "therapeutic activity" refers to the *in vivo* activity of a therapeutic polypeptide. Generally, the therapeutic activity is the activity that is associated with treatment of a disease or condition. For example, the therapeutic activity of an anti-EGFR antibody includes inhibitory activities on EGFR phosphorylation, signaling and cell growth, and in particular inhibitory activities on tumor cell growth. Therapeutic activity of a modified polypeptide can be any level of percentage of therapeutic activity of the unmodified polypeptide, including but not limited to, 1 % of the activity, 2 %, 3 %, 4 %, 5 %, 10 %, 20 %, 30 %, 40 %, 50 %, 60 %, 70 %, 80 %, 90 %, 91 %, 92 %, 93 %, 94 %, 95 %, 96 %, 97 %, 98 %, 99 %, 100 %, 200 %, 300 %, 400 %, 500 %, or more of therapeutic activity compared to the unmodified polypeptide.

As used herein, the term "assessing" is intended to include quantitative and qualitative determination in the sense of obtaining an absolute value for the activity of a protein, such as a modified anti-EGFR antibody, or an antigen binding fragment thereof, present in the sample, and also of obtaining an index, ratio, percentage, visual, or other value indicative of the level of the activity. Assessment can be direct or indirect.

As used herein, "disease or disorder" refers to a pathological condition in an organism resulting from cause or condition including, but not limited to, infections, acquired conditions, genetic conditions, and characterized by identifiable symptoms.

As used herein, "EGFR-associated disease or condition" or "conditions responsive to treatment with an anti-EGFR antibody," refers to any disease or condition that is associated with or caused by aberrant EGFR signaling or overexpression of EGFR. Such diseases and conditions are known in the art, and exemplary of such are described herein. For example, EGFR-associated disease or conditions or conditions responsive to treatment with an anti-EGFR antibody include cancers, such as but not limited to, colorectal cancer, squamous cell cancer of the head and neck and non-small-cell lung cancer.

As used herein, "treating" a subject with a disease or condition means that the subject's symptoms are partially or totally alleviated, or remain static following treatment. Hence treatment encompasses prophylaxis, therapy and/or cure. Prophylaxis refers to prevention of a potential disease and/or a prevention of worsening of symptoms or progression of a disease. Treatment also encompasses any pharmaceutical use of any antibody or antigen-binding fragment thereof provided or compositions provided herein.

As used herein, "prevention" or prophylaxis, and grammatically equivalent forms thereof, refers to methods in which the risk of developing disease or condition is reduced.

As used herein, a "pharmaceutically effective agent" includes any therapeutic agent or bioactive agents, including, but not limited to, for example, anesthetics, vasoconstrictors, dispersing agents, conventional therapeutic drugs, including small molecule drugs and therapeutic proteins.

As used herein, a "therapeutic effect" means an effect resulting from treatment of a subject that alters, typically improves or ameliorates the symptoms of a disease or condition or that cures a disease or condition.

As used herein, a "therapeutically effective amount" or a "therapeutically effective dose" refers to the quantity of an agent, compound, material, or composition containing a compound that is at least sufficient to produce a therapeutic effect following administration to a subject. Hence, it is the quantity necessary for preventing, curing, ameliorating, arresting or partially arresting a symptom of a disease or disorder.

As used herein, "therapeutic efficacy" refers to the ability of an agent, compound, material, or composition containing a compound to produce a therapeutic effect in a subject to whom the an agent, compound, material, or composition containing a compound has been administered.

As used herein, a "prophylactically effective amount" or a "prophylactically effective dose" refers to the quantity of an agent, compound, material, or composition containing a compound that when administered to a subject, will have the intended prophylactic effect, *e.g.*, preventing or delaying the onset, or reoccurrence, of disease or symptoms, reducing the likelihood of the onset, or reoccurrence, of disease or symptoms, or reducing the incidence of viral infection. The full prophylactic effect does not necessarily occur by administration of one dose, and can occur only after administration of a series of doses. Thus, a prophylactically effective amount can be administered in one or more administrations.

As used herein, amelioration of the symptoms of a particular disease or disorder by a treatment, such as by administration of a pharmaceutical composition or other therapeutic, refers to any lessening, whether permanent or temporary, lasting or transient, of the symptoms that can be attributed to or associated with administration of the composition or therapeutic.

As used herein, "Prodrug" is a precursor or derivative form of a pharmaceutically active substance that is less cytotoxic to tumor cells compared to the parent drug and is capable of being enzymatically activated or converted into the more active parent form(see, e.g., Wilman, 1986, Biochemical Society Transactions, 615th Meeting Belfast, 14:375-382; and Stella et al., "Prodrugs: A Chemical Approach to Targeted Drug Delivery," Directed Drug Delivery, Borchardt et al., (ed.): 247-267, Humana Press, 1985.)

As used herein, an "anti-cancer agent" refers to any agent that is destructive or toxic to malignant cells and tissues. For example, anti-cancer agents include agents that kill cancer cells or otherwise inhibit or impair the growth of tumors or cancer cells. Exemplary anti-cancer agents are chemotherapeutic agents.

As used herein, an "anti-angiogenic agent" or "angiogenesis inhibitor" is a compound that blocks, or interferes with, the development of blood vessels.

As used herein, a "hyperproliferative disease" is a condition caused by excessive growth of non-cancer cells that express a member of the EGFR family of receptors.

As used herein, the term "subject" refers to an animal, including a mammal, such as a human being.

As used herein, a patient refers to a human subject.

As used herein, animal includes any animal, such as, but are not limited to primates including humans, gorillas and monkeys; rodents, such as mice and rats; fowl, such as chickens; ruminants, such as goats, cows, deer, sheep; pigs and other animals. Non-human animals exclude humans as the contemplated animal. The polypeptides provided herein are from any source, animal, plant, prokaryotic and fungal. Most polypeptides are of animal origin, including mammalian origin.

As used herein, a "composition" refers to any mixture. It can be a solution, suspension, liquid, powder, paste, aqueous, non-aqueous or any combination thereof.

As used herein, a "combination" refers to any association between or among two or more items. The combination can be two or more separate items, such as two compositions or two collections, can be a mixture thereof, such as a single mixture of the two or more items, or any variation thereof. The elements of a combination are generally functionally associated or related.

As used herein, combination therapy refers to administration of two or more different therapeutics, such as an anti-EGFR antibody (or antigen binding fragment thereof) and one or more therapeutics. The different therapeutic agents can be provided and administered separately, sequentially, intermittently, or can be provided in a single composition.

As used herein, a kit is a packaged combination that optionally includes other elements, such as additional reagents and instructions for use of the combination or elements thereof, for a purpose including, but not limited to, activation, administration, diagnosis, and assessment of a biological activity or property.

As used herein, a "unit dose form" refers to physically discrete units suitable for human and animal subjects and packaged individually as is known in the art.

As used herein, a "single dosage formulation" refers to a formulation for direct administration.

As used herein, a multi-dose formulation refers to a formulation that contains multiple doses of a therapeutic agent and that can be directly administered to provide several single doses of the therapeutic agent. The doses can be administered over the course of minutes, hours, weeks, days or months. Multidose formulations can allow dose adjustment, dose-pooling and/or dose-splitting. Because multi-dose formulations are used over time, they generally contain one or more preservatives to prevent microbial growth.

As used herein, an "article of manufacture" is a product that is made and sold. As used throughout this application, the term is intended to encompass any of the compositions provided herein contained in articles of packaging.

As used herein, a "fluid" refers to any composition that can flow. Fluids thus encompass compositions that are in the form of semi-solids, pastes, solutions, aqueous mixtures, gels, lotions, creams and other such compositions.

As used herein, an isolated or purified polypeptide or protein (*e.g.* an isolated antibody or antigen-binding fragment thereof) or biologically-active portion thereof (*e.g.* an isolated antigen-binding fragment) is substantially free of cellular material or other contaminating proteins from the cell or tissue from which the protein is derived, or substantially free from chemical precursors or other chemicals when chemically synthesized. Preparations can be determined to be substantially free if they appear free of readily detectable impurities as determined by standard methods of analysis, such as thin layer chromatography (TLC), gel electrophoresis and high performance liquid chromatography (HPLC), used by those of skill in the art to assess such purity, or sufficiently pure such that further purification does not detectably alter the physical and chemical properties, such as enzymatic and biological activities, of the substance. Methods for purification of the compounds to produce substantially chemically pure compounds are known to those of skill in the art. A substantially chemically pure compound, however, can be a mixture of stereoisomers. In such instances, further purification might increase the specific activity of the compound. As used herein, a "cellular extract" or "lysate" refers to a preparation or fraction which is made from a lysed or disrupted cell.

As used herein, a "control" refers to a sample that is substantially identical to the test sample, except that it is not treated with a test parameter, or, if it is a plasma sample, it can be from a normal volunteer not affected with the condition of interest. A control also can be an internal control.

As used herein, the singular forms "a," "an" and "the" include plural referents unless the context clearly dictates otherwise. Thus, for example, reference to a polypeptide, comprising "an immunoglobulin domain" includes polypeptides with one or a plurality of immunoglobulin domains.

As used herein, the term "or" is used to mean "and/or" unless explicitly indicated to refer to alternatives only or the alternatives are mutually exclusive.

As used herein, ranges and amounts can be expressed as "about" a particular value or range. About also includes the exact amount. Hence "about 5 amino acids" means "about 5 amino acids" and also "5 amino acids."

As used herein, "optional" or "optionally" means that the subsequently described event or circumstance does or does not occur and that the description includes instances where said event or circumstance occurs and instances where it does not. For example, an optionally variant portion means that the portion is variant or non-variant.

As used herein, the abbreviations for any protective groups, amino acids and other compounds, are, unless indicated otherwise, in accord with their common usage, recognized abbreviations, or the IUPAC-IUB Commission on Biochemical Nomenclature (see, Biochem. (1972) 11(9):1726-1732).

For clarity of disclosure, and not by way of limitation, the detailed description is divided into the subsections that follow.

## B. EGFR and ANTI-EGFR ANTIBODIES

Anti-EGFR antibodies are known and approved for various indications, including metastatic colorectal cancer (MCRC), squamous cell carcinoma of the head and neck (SCCHN) and non-small cell lung cancer (NSCLC). Anti-EGFR antibodies include, but are not limited to, Erbitux® (cetuximab, C225 or IMC-C225), 11F8 by Zhu (WO 2005/090407), EMD 72000 (matuzumab), Vectibix™ (panitumumab; ABX-EGF), TheraCIM (nimotuzumab), and Hu-Max-EGFR (zalutumumab). When administered to subjects, however, these therapeutic antibodies result in adverse side effects to the subjects (Eng C. (2009) Nat. Rev. Clin. Oncol., 6:207-218). This has limited their use. For example, anti-EGFR antibodies are associated with significant and characteristic adverse events including skin toxicities and digestive disturbances (including nausea, vomiting, diarrhea), that often lead to interruption of dosing and discontinuation of treatment. For example, EGFR, is highly expressed in pre-keratinocytes and basal cells of the skin. Blockade of EGFR signaling in the skin precursors by anti-EGFR antibodies leads to skin precursor growth inhibition, apoptosis and inflammation. This can result in skin toxicity, such as a rash and other skin lesions.

It is found herein that side effects can be reduced by providing antibodies that exhibit increased activity at targeted disease tissue, such as the tumor, but decreased activity at non-disease tissues or organs, in particular tissue sites (e.g. basal layer of skin or dermis) associated with adverse events. As a therapeutic, the activity of anti-EGFR antibodies is principally targeted to the tumor environment, which exhibits an acidic pH and elevated lactate levels, e.g., between 10-15 mM lactate.

In contrast, the dermis, which is where many side effects are localized, exhibits a neutral pH and normal lactate levels. The differences in conditions that characterize solid tumors, such as low pH and hypoxia, can be leveraged to provide antibodies that are more active in the diseased microenvironment of the tumor. Hence, provided herein are modified anti-EGFR antibodies that are conditionally active in the tumor microenvironment and exhibit altered activity or increased activity under conditions present in the tumor microenvironment compared to normal tissues. For example, the antibodies provided herein are more active at low pH and/or high lactate, than at neutral pH or low lactate. As a consequence of this altered activity, subjects treated with the antibodies have fewer and/or reduced side effects.

In particular, provided herein are anti-EGFR antibodies that exhibit reduced activity, for example binding activity, at neutral pH compared to activity at lower pH, for example, pH 5.8 to 6.8, such as the acidic pH environment of the tumor. In another example, the modified anti-EGFR antibodies exhibit increased activity, for example binding activity, at increased lactate concentrations, such as at concentrations between 10 and 15 mM lactate. In yet other examples, the anti-EGFR antibodies provided herein bind with increased activity, such as binding activity, at both reduced pH and elevated lactate levels. The anti-EGFR antibodies provided herein exhibit altered activity such that they confer reduced or fewer side effects when administered.

## 1. EGFR

Epidermal growth factor receptor (Uniprot Accession No. P00533; SEQ ID NO:6) is a 170 kDA Type I glycoprotein. EGFR is a member of the ErbB family of receptor tyrosine kinases, which includes HER2/c-neu (ErbB-2), Her3 (ErbB-3) and Her4 (ErbB-4). EGFR exists on cell surfaces and contains three domains, including an extracellular ligand-binding domain, an intracellular tyrosine kinase domain and a transmembrane lipophilic segment. In addition to their presence on a tumor cells, epidermal growth factor receptors are ubiquitous, distributed randomly on the surface of normal cells, excluding hematopoietic cells and cells of epidermal origin.

Epidermal growth factor receptor (EGFR; also known as receptor tyrosine-protein kinase erbB-1, ErbB-1, HER1) is a tyrosine kinase growth factor receptor involved in signaling cascades important for cell growth, proliferation, survival and motility. EGFR activity is stimulated or activated by binding of endogenous ligands such as epidermal growth factor (EGF), as well as other endogenous EGF-like ligands including TGF-$\alpha$, amphiregulin, heparin-binding EGF (HB-EGF) and betacellulin. Upon ligand binding, the ligand-EGFR complex undergoes dimerization and internalization into the cell. EGFR can homodimerize with other monomeric EGFR molecules, or alternatively, heterodimerize with another HER receptor, such as HER2, ErbB-3 or ErbB-4. EGFR dimerization turns on intrinsic intracellular protein-tyrosine kinase activity. Thus, dimerization activates the intracellular protein kinase via autophosphorylation of tyrosine residues in the cytoplasmic tail. These phosphotyrosine residues act as docking sites for downstream effectors such as adaptor molecules and enzymes leading to initiation of a variety of signal transduction pathways, including mitogen-activated protein kinase (MAPK), Akt/phosphatidylinositol-3-OH kinase (PI3K) and c-Jun N-terminal kinases (JNK), thereby regulating a variety of mitogenic mechanisms involved in DNA synthesis, cell proliferation, cell migration, cell survival and cell adhesion.

Aberrant signal transduction through activated growth factor receptors is common in many solid tumors (Yarden and Sliwkowski (2001) Nat Rev Mol Cell Biol 2:127-137). EGFRs have been observed in a variety of solid human tumors, including glioma and colon, head and neck, pancreatic, non-small cell lung, breast, renal, ovarian, and bladder carcinomas (Herbst and Hong (2002) Seminars in Oncology 29(5) Suppl. 14: 18-30). As such, EGFR is an attractive target for anti-cancer therapeutics. EGFR is important in regulating cell survival and apoptosis, angiogenesis, cell motility and metastasis (Herbst et al. (2001) Expert Opin. Biol. Ther. 1(4):719-732). Aberrant EGFR signaling and EGFR overexpression have been observed in various cancers and correlated with poor prognosis and elevated risk of invasive or metastatic disease (Herbst et al. (2001) Expert Opin. Biol. Ther. 1(4):719-732). EGFR activation is associated with significant upregulation of secretion of vascular endothelial growth factor, a stimulator of tumor angiogenesis (Petit at al. (1997) Am J Pathol 151:1523-1530).

**2. Anti-EGFR Antibodies and Side Effects**

Therapeutic agents that target and inhibit aberrant EGFR signaling include anti-EGFR antibodies. Anti-EGFR antibodies act by binding to epidermal growth factor receptor (EGFR). The anti-EGFR antibodies act by competing for and inhibiting the binding of ligands, such as EGF, to the extracellular ligand binding domain of EGF. The result of this is that cytoplasmic domain phosphorylation and the resulting signal transduction events are inhibited. Hence, anti-EGFR antibodies can be effective therapeutics by blocking EGFR-mediated cell signaling and cell growth.

Anti-EGFR antibodies, however, cannot distinguish between cancer cells and normal cells, and thus adverse side effects are common. For example, EGFR is widely distributed throughout epithelial tissues, resulting in skin toxicity shared by many EGFR inhibitors (Herbst and Hong (2002) Seminars in Oncology 29(5) Suppl. 14: 18-30). In human skin, EGFR is expressed in basal keratinocytes and can stimulate epidermal growth, inhibit differentiation, and accelerate wound healing (Lacouture and Melosky (2007) Skin Therapy Lett. 12, 1-5; Nanney et al. (1990) J. Invest. Dermatol 94(6):742-748; Lacouture, M.E. (2006) Nat Rev Cancer 6:803-812). Inhibition of EGFR function can impair growth and migration of keratinocytes, and result in inflammatory chemokine expression, resulting in rashes (Lacouture, M.E. (2006) Nat Rev Cancer 6:803-812). Increased apoptosis of keratinocytes upon treatment with EGFR inhibitors is correlated with onset of rash in subjects treated with the EGFR inhibitors (Lacouture, M.E. (2006) Nat Rev Cancer 6:803-812). Keratinocytes are located in the stratum basale, the deepest layer of the skin, which has a pH between 7.0 and 7.2. The blood vessels in the dermis provide nourishment and waste removal for the epidermis, thus making the epidermis, in particular the stratum basale, most susceptible to systemically circulated anti-EGFR therapies.

The most common side effects associated with anti-EGFR antibodies, such as cetuximab, are dermatologic reactions, which are seen in 45-100 % of patients (Le and Perez-Soler (2009) Target Oncol 4:107-119). Common dermatologic reactions include, acneiform rash, papulopustular rash, hair growth abnormalities, dry and itchy skin and periungual inflammation with tenderness (Eng (2009) Nat Rev Clin Oncol 6:207-218; Monti et al. (2007) Int J Biol Markers 22:S53-S61; Saif and Kim (2007) Expert Opin Drug Saf 6:175-182). Additional dermatologic reactions include telangiectasia, hyperpigmentation, pruritus without rash, erythema and oral aphthae (Eng (2009) Nat Rev Clin Oncol 6:207-218). Cetuximab elicits an immune response in about 5-15 % of patients, with some patients reporting severe anaphylactic reactions (Chung et al. (2008) N Engl J Med 358:1109-1117). These hypersensitivity reactions have been linked to galactose-alpha-1,3-galactose oligosaccharides on cetuximab that induce the production of IgG antibodies (Chung et al. (2008) N Engl J Med 358:1109-1117). Further side effects include pulmonary toxicities, including dyspnea, cough, wheezing, pneumonia, hypoxemia, respiratory insufficiency/failure, pulmonary embolus, pleural effusion and non-specific respiratory disorders (Hoag et al. (2009) J Experimental & Clinical Cancer Research 28:113). Other side effects include fever, chills, asthenia/malaise, mucosal surface problems, nausea, gastrointestinal problems, abdominal pain, headache and hypomagnesemia (Eng (2009) Nat Rev Clin Oncol 6:207-218; Fakih and Vincent, (2010) Curr. Oncol. 17(S1):S18-S30; Int. Pat. No. WO2011059762).

The conditionally active anti-EGFR antibodies provided herein exhibit selectivity for tumor cells compared to non-tumor cell targets, such as basal keratinocytes and other basal cell. Hence, the conditionally active anti-EGFR antibodies can result in reduced side effects when administered to patients compared to currently available anti-EGFR antibodies, including eliminating, minimizing or reducing systemic side effects, including dermal toxicities, while retaining their ability to block EGFR signaling. They also permit dosings to achieve increased efficacy compared to existing therapeutics.

### 3. Cetuximab

Included among the conditionally active anti-EGFR antibodies provided herein are modified anti-EGFR antibodies that are modified compared to the anti-EGFR antibody Cetuximab, antigen-binding fragments thereof or variants thereof (e.g. a humanized form of cetuximab, e.g. Hu225). Cetuximab (also known as C225 or IMC-C225) is a mouse/human chimeric, IgG1 monoclonal antibody that binds to human epidermal growth factor receptor. Cetuximab was derived from M225, which was identified using EGFR from human A431 epidermoid carcinoma cells as an immunogen (Gill et al. (1984) J Biol Chem 259:7755-7760; Sato et al., (1983) Mol Biol Med 1:511-529; Masui et al., (1984) Cancer Res 44:1002-1007; Kawamoto et al. (1983) Proc Natl Acad Sci USA 80:1337-1341). M225 inhibits binding of the epidermal growth factor to the EGF receptor and is an antagonist of *in vivo* EGF-stimulated tyrosine kinase activity. (Gill et al. (1984) J Biol Chem 259:7755-7760).

### a. Structure

Cetuximab is a full-length mouse/human chimeric IgG1 antibody. A full-length antibody contains four polypeptide chains, two identical heavy (H) chains (each usually containing about 440 amino acids) and two identical light (L) chains (each containing about 220 amino acids). The light chains exist in two distinct forms called kappa ($\kappa$) and lambda ($\lambda$). Each chain is organized into a series of domains organized as immunoglobulin (Ig) domains. An Ig domain is characterized by a structure called the Ig fold, which contains two beta-pleated sheets, each containing anti-parallel beta strands connected by loops. The two beta sheets in the Ig fold are sandwiched together by hydrophobic interactions and a conserved intra-chain disulfide bond. The plurality of Ig domains in the antibody chains are organized into variable (V) and constant (C) region domains. The variable domains confer antigen-specificity to the antibody through three portions called complementarity determining regions (CDRs) or hypervariable (HV) regions. The CDR regions are precisely defined and universally numbered in antibodies (see e.g., Kabat, E.A. et al. (1991) Sequences of Proteins of Immunological Interest, Fifth Edition, U.S. Department of Health and Human Services, NIH Publication No. 91-3242, and Chothia, C. et al. (1987) J. Mol. Biol. 196:901-917; AbM (Martin et al. (1989) Proc Natl Acad Sci USA 86:9268-9272; Martin et al. (1991) Methods Enzymol 203:121-153; Pederson et al. (1992) Immunomethods 1:126). Together, the three heavy chain CDRs and the three light chain CDRs make up an antigen-binding site (antibody combining site) of the antibody, which physically interacts with cognate antigen and provides the specificity of the antibody. The constant region promotes activation of complement and effector cells. Like CDR regions, constant regions are precisely defined and universally numbered in antibodies using EU index and Kabat numbering schemes (see e.g., Kabat, E.A. et al. (1991) Sequences of Proteins of Immunological Interest, Fifth Edition, U.S. Department of Health and Human Services, NIH Publication No. 91-3242). Light chains have two domains, corresponding to the C region ($C_L$) and the V region ($V_L$). Heavy chains have four domains, the V region (VH) and three or four domains in the C region ($C_H1$, $C_H2$, $C_H3$ and $C_H4$), and, in some cases, hinge region. Each heavy chain is linked to a light chain by a disulfide bond, and the two heavy chains are linked to each other by disulfide bonds. Linkage of the heavy chains is mediated by a flexible region of the heavy chain, known as the hinge region.

Cetuximab contains variable regions from mouse monoclonal antibody 225 (M225) and human constant regions, including a human IgG1 heavy chain constant region (SEQ ID NO:1069) and a human CK light chain constant region (SEQ ID NO:1071). The complete heavy chain of cetuximab has a sequence of amino acids set forth in SEQ ID NO:1, encoded by a sequence of nucleotides set forth in SEQ ID NO:1111, and the light chain has a sequence of amino acids set forth in SEQ ID NO:2, encoded by a sequence of nucleotides set forth in SEQ ID NO:1110. The heavy chain is composed of a mouse variable domain ($V_H$, amino acid residues 1-119 of SEQ ID NO:1, set forth in SEQ ID NO:3), and human constant domains $C_H1$-$C_H2$-hinge-$C_H3$, including $C_H1$ (amino acid residues 120-222 of SEQ ID NO:1), a hinge region (amino acid residues 223-238 of SEQ ID NO:1), $C_H2$ (amino acid residues 239-342 of SEQ ID NO:1) and $C_H3$ (amino acid residues 343-449 of SEQ ID NO:1). The light chain is composed of a mouse variable domain ($V_L$, amino acid residues 1-107 of SEQ ID NO:2, set forth in SEQ ID NO:4) and a human kappa light constant region (CK, amino acid residues 108-213 of SEQ ID NO:2).

The CDRs of cetuximab include, $V_H$ CDR 1 (amino acid residues 26-35, according to AbM definition (Martin et al. (1989) Proc Natl Acad Sci USA 86:9268-9272; Martin et al. (1991) Methods Enzymol 203:121-153; Pedersen et al. (1992) Immunomethods 1:126), or amino acid residues 31-35, according to Kabat definition, of SEQ ID NO:3, set forth in SEQ ID NOS:14 and 15, respectively); $V_H$ CDR 2 (amino acid residues 50-65 of SEQ ID NO:3, set forth in SEQ ID NO:16); $V_H$ CDR 3 (amino acid residues 98-108 of SEQ ID NO:3, set forth in SEQ ID NO:17); $V_L$ CDR 1 (amino acid residues 24-34 of SEQ ID NO:4, set forth in SEQ ID NO:18); $V_L$ CDR 2 (amino acid residues 50-56 of SEQ ID NO:4, set forth in SEQ ID NO:19); and $V_L$ CDR 3 (amino acid residues 89-97 of SEQ ID NO:4, set forth in SEQ ID NO:20).

According to the Kabat numbering (Kabat, E.A. et al. (1991) Sequences of Proteins of Immunological Interest, Fifth Edition, U.S. Department of Health and Human Services, NIH Publication No. 91-3242), the CDRs of cetuximab include $V_H$ CDR 1 (amino acid residues 26-35, according to AbM definition, or amino acid residues 31-35, according to Kabat definition); V$_H$ CDR 2 (amino acid residues 50-65); V$_H$ CDR 3 (amino acid residues 95-102); V$_L$ CDR 1 (amino acid residues 24-34); V$_L$ CDR 2 (amino acid residues 50-56); and V$_L$ CDR 3 (amino acid residues 89-97).

The crystal structure of cetuximab Fab bound to the extracellular domain of the EGFR (sEGFR) has previously been determined (Li et al., (2005) Cancer Cell 7:301-311). Cetuximab binds to domain III of the epidermal growth factor receptor (amino acids 310-514 of SEQ ID NO:6), with an epitope that partially overlaps with the natural ligand epidermal growth factor. Residues $^{L27}$Gln, $^{L50}$Tyr, $^{L94}$Trp, $^{H52}$Trp, $^{H58}$Asp, $^{H101}$Tyr, $^{H102}$Tyr, $^{H103}$Asp and $^{H104}$Tyr of cetuximab make contacts with domain III of sEGFR. The light chain of cetuximab binds to the C-terminal domain of EGFR, with V$_L$ CDR 1 residue $^{L27}$Gln of cetuximab binding to residue N473 of sEGFR. V$_H$ CDR 3 residue $^{H102}$Tyr protrudes into a hydrophobic pocket on the surface of a large β sheet of domain III, making hydrogen bonds to glutamine side chains of Q384 and Q408 of sEGFR. V$_H$ CDR 2 and V$_H$ CDR 3 lie over the hydrophobic pocket, anchored by side chain to side chain hydrogen bonds between $^{H52}$Trp and S418 of sEGFR and $^{H104}$Tyr and S468 of sEGFR, side chain to main chain interactions between $^{H54}$Gly and $^{H103}$Asp carbonyl oxygens and sEGFR S440 and R353, and indirect hydrogen bonds between $^{H56}$Asn and S418 and Q384 of sEGFR. In addition to blocking the binding of EGF to sEGFR, the variable heavy chain of cetuximab sterically blocks domain I thereby preventing domain II from adopting a conformation necessary for dimerization.

Other variants of cetuximab have been reported and are known. Hu225, a humanized version of cetuximab, that has a variable heavy chain that has a sequence of amino acids set forth in SEQ ID NO:28, and a variable light chain that has a sequence of amino acids set forth in SEQ ID NO:29. Compared to Cetuximab (225), Hu225 contains amino acid replacements at amino acid residues in the framework regions, including replacement (substitution) in the variable light chain (V$_L$) at positions corresponding to replacement of Valine (V) at position 9 with Glycine (G), I10T, V13L, V19A, S20T, F21L, R39K, T40P, N41G, G42Q, S43A, S60D, S74T, N76S, S77R, V78L, S80P, I83F, D85V, A100Q and L106I, in the sequence of amino acids set forth in SEQ ID NO:4 (Hu225 V$_L$ set forth in SEQ ID NO:29), and replacement (substitution) in the variable heavy chain (V$_H$) at positions corresponding to replacement of Glutamine (Q) at position 1 with Glutamic acid (E), K5V, Q6E, P9G, S16G, Q17G, S19R, I20L, T21S, T23A, V24A, S40A, S68T, S76N, Q77T, F79Y, F80L, K81Q, Q86R, S87A, N88E, I92V and A119S, in the sequence of amino acids set forth in SEQ ID NO:3 (Hu225 V$_H$ set forth in SEQ ID NO:28). Additional cetuximab variants include those having a heavy chain set forth in SEQ ID NO:8 and a light chain set forth in SEQ ID NO:9. Further a number of other variants have been described and are known in the art (see e.g. U.S. Pat. Nos. 7,657,380, 7,930,107, 7,060,808, 7,723,484, U.S. Pat. Publ. Nos. 2011014822, 2005142133, 2011117110, International Pat. Pub. Nos. WO2012003995, WO2010080463, WO2012020059, WO2008152537, and Lippow et al. (2007) Nat Biotechnol. 25(10):1171-1176). The modifications described herein can be in any cetuximab, antigen-binding fragment thereof or variant thereof, including any known in the art.

### b. Function

Cetuximab binds to the extracellular domain of EGFR on both normal and tumor cells preventing ligand binding and subsequent activation (Li et al., (2005) Cancer Cell 7:301-311; Blick et al., (2007) Drugs 67(17):2585-2607). Cetuximab competitively inhibits the binding of epidermal growth factor and transforming growth factor alpha (TGF-alpha) preventing cell growth and metastatic spread. That is, binding of cetuximab blocks phosphorylation and activation of tyrosine-receptor kinases, resulting in inhibition of cell growth, induction of apoptosis, decreased matrix metalloprotease secretion and reduced vascular endothelial growth factor production. Cetuximab can also induce an antitumor effect through inhibition of angiogenesis. Cetuximab inhibits expression of VEGF, IL-8 and bFGF in the highly metastatic human TCC 253JB-V cells in a dose dependent manner and decreases microvessel density (Perrotte et al. (1999), Clin. Cancer Res., 5:257-264). Cetuximab can down-regulate VEGF expression in tumor cells *in vitro* and *in vivo* (Petit et al. (1997), Am. J. Pathol., 151:1523-1530; Prewett et al. (1998), Clin. Cancer Res.4:2957-2966). Cetuximab is also involved in antibody-dependent cellular cytotoxicity (ADCC) and receptor internalization.

### C. MODIFIED ANTI-EGFR ANTIBODIES AND CONDITIONALLY ACTIVE ANTI-EGFR ANTIBODIES

Provided herein are conditionally active anti-EGFR antibodies or antigen-binding fragments, such as modified or variant anti-EGFR antibodies or antigen binding fragments thereof, that exhibit higher or greater activity in a tumor microenvironment than in a non-diseased or non-tumor microenvironment environment, such as the skin or basal layer of the skin. Such antibodies include any that exhibit greater binding activity for human epidermal growth factor receptor (EGFR), or a soluble fragment thereof, under conditions that exist in a tumor environment compared to under conditions that exist in a non-tumor microenvironment (e.g. basal layer of skin). By virtue of exhibiting greater binding activity in a tumor microenvironment, the anti-EGFR antibodies provided herein exhibit selective activity against tumors, and reduced binding activity to cells in non-tumor microenvironments. Such selectivity achieved by their conditional binding activity minimizes the undesired activity on non-tumor cells, such as basal keratinocytes of the skin. Thus, the anti-EGFR antibodies, or antigen binding fragments thereof, provided herein confer reduced or fewer side effects when administered to subjects.

An altered pH microenvironment is the most common microenvironment found in tumor microenvironments (see *e.g.* Fogh Andersen et al. (1995) Clin. Chem., 41:1522-1525; Bhujwalla et al. (2002) NMR Biomed., 15:114-119; Helmlinger et al. (1997) Nature Med., 3:177; Gerweck and Seetharaman (1996), Cancer Res. 56(6):1194-1198). For example, in many tumors the 'Warburg effect' creates a microenvironment with a pH ranging from 5.6 to 6.8. Also, elevated lactate levels have been found associated with a variety of tumors including, but not limited to, head and neck, metastatic colorectal cancer, cervical cancer and squamous cell carcinoma (see *e.g.,* Walenta et al. (1997) American Journal of Pathology 150(2): 409-415; Schwickert et al. (1995) Cancer Research 55: 4757-4759; Walenta et al. (2000) Cancer Research 60: 916-921; Guo et al. (2004) J Nucl Med 45: 1334-1339; Mathupala et al. (2007) J Bioenerg Biomembr 39: 73-77; Holroyde et al. (1979) Cancer Research 39: 4900-4904; Schurr and (2007) Neuroscience 147: 613-619; Quenneta et al. (2006) Radiotherapy and Oncology 81: 130-135). In many tumors, the 'Warburg effect' creates a microenvironment with lactate concentrations between 10 to 15 mM. In contrast to the tumor microenvironment, the dermis, where many side effects that result from administration of anti-EGFR antibodies are localized, exhibits a neutral pH and normal lactate levels.

The anti-EGFR antibodies provided herein, including modified anti-EGFR antibodies and antigen binding fragments of any of the anti-EGFR antibodies, bind to EGFR (particularly human EGFR) with a higher binding activity under conditions that exist in a tumor microenvironment that include one or both of pH between or about between pH 5.6 to 6.8 or lactate concentration of between or about between 5 mM to 20 mM compared to under conditions that exist in a non-tumor microenvironment that include one or both of pH between or about between pH 7.0 to 7.8 or lactate concentration between or about between 0.5 mM to 5 mM. The higher binding activity under conditions in a tumor microenvironment compared to under conditions in a non-tumor microenvironment can be a ratio of activity of at least or greater than 1.1, 1.2, 1.3, 1.4, 1.5, 1.6, 1.7, 1.8, 1.9, 2.0, 2.5, 3.0, 3.5, 4.0, 4.5, 5.0, 6.0, 7.0, 8.0, 9.0, 10.0, 11.0, 12.0, 13.0, 14.0, 15.0, 20.0, 25.0, 30.0, 35.0, 40.0, 45.0, 50.0 or more.

In general, the ratio of activity is exhibited in the presence of physiological levels of protein. In an *in vivo* or physiological environment, the interstitial protein concentration (such as albumin) is anywhere from 20-50% of plasma. Serum contains about 60-80 g/L protein, and various tissues have been demonstrated to contain 12 mg/mL to 40 mg/mL interstitial protein (see e.g. Aukland and Reed (1993) Physiological Reviews, 73:1-78). Hence, anti-EGFR antibodies that exhibit selective and conditional activity in vivo under these conditions, exhibit the ratio of activity in the presence of 10 mg/mL to 50 mg/mL protein, such as at least at least 12 mg/mL to 40 mg/mL protein (*e.g.* at least 12 mg/mL, 15 mg/mL, 20 mg/mL, 25 mg/mL, 30 mg/mL, 35 mg/mL or 40 mg/mL protein), which, for example, can be provided in serum, such as human serum, or as a serum albumin, such as human serum albumin, or other protein that does not interact with the antibody or receptor or otherwise directly alter antibody-receptor interactions. For example, the protein is provided in serum, and assays and methods to select or characterize anti-EGFR antibodies are performed in the presence of 20% to 50% serum (vol/vol), such as 20% to 50% human serum, such as at least 20%, 25%, 30%, 35%, 40%, 45% or 50% serum (vol/vol). Hence, in particular examples herein, the anti-EGFR antibodies provided herein, including modified anti-EGFR antibodies and antigen binding fragments of any of the anti-EGFR antibodies, bind to EGFR (particularly human EGFR) with a higher binding activity under conditions that exist in a tumor microenvironment that include one or both of pH between or about between pH 5.6 to 6.8 or lactate concentration of between or about between 5 mM to 20 mM and 10 mg/mL to 50 mg/mL protein (e.g. 20% to 50% human serum), compared to under conditions that exist in a non-tumor microenvironment that include one or both of pH between or about between pH 7.0 to 7.8 or lactate concentration between or about between 0.5 mM to 5 mM and 10 mg/mL to 50 mg/mL protein (e.g. 20% to 50% human serum). The higher binding activity under conditions in a tumor microenvironment compared to under conditions in a non-tumor microenvironment generally exists under conditions where the protein concentration under conditions in a tumor microenvironment and under conditions in a non-tumor microenvironment is substantially the same or is the same. In particular examples, the ratio of activity can be at least or greater than 1.1, 1.2, 1.3, 1.4, 1.5, 1.6, 1.7, 1.8, 1.9, 2.0, 2.5, 3.0, 3.5, 4.0, 4.5, 5.0, 6.0, 7.0, 8.0, 9.0, 10.0, 11.0, 12.0, 13.0, 14.0, 15.0, 20.0, 25.0, 30.0, 35.0, 40.0, 45.0, 50.0 or more.

In particular, the antibodies provided herein include those that bind to epidermal growth factor receptor (EGFR) with a higher binding activity at pH 6.0 to pH 6.5 than at a neutral pH (e.g. 7.4), and in the presence of 10 mg/mL to 50 mg/mL protein, such as at least 12 mg/mL to 40 mg/mL protein (*e.g.* at least 12 mg/mL, 15 mg/mL, 20 mg/mL, 25 mg/mL, 30 mg/mL, 35 mg/mL or 40 mg/mL protein). For example, the antibodies provided herein include those that bind to epidermal growth factor receptor (EGFR) with a higher binding activity at pH 6.0 to pH 6.5 than at a neutral pH (e.g. 7.4), and in the presence of 20% to 50% serum (vol/vol), such as 20% to 50% human serum, such as at least 20%, 25%, 30%, 35%, 40%, 45% or 50% serum (vol/vol). For example, the ratio of binding activity under conditions of pH 6.0 to pH 6.5 compared to under conditions at neutral pH (e.g. pH 7.4) is greater than 1.0, for example, at least or greater than 1.1, 1.2, 1.3, 1.4, 1.5, 1.6, 1.7, 1.8, 1.9, 2.0, 2.5, 3.0, 3.5, 4.0, 4.5, 5.0, 6.0, 7.0, 8.0, 9.0, 10.0, 11.0, 12.0, 13.0, 14.0, 15.0, 20.0, 25.0, 30.0, 35.0, 40.0, 45.0, 50.0 or more.

Included among the conditionally active antibodies provided herein, including modified anti-EGFR antibodies herein, are those that bind to epidermal growth factor receptor (EGFR) with a higher binding activity at an elevated lactate concentration of between 10 to 20 mM than a lactate concentration of 0.5 mM to 5 mM, and in the presence of 10 mg/mL to 50 mg/mL protein, such as at least 12 mg/mL to 40 mg/mL protein (*e.g.* at least 12 mg/mL, 15 mg/mL, 20 mg/mL, 25 mg/mL, 30 mg/mL, 35 mg/mL or 40 mg/mL protein). For example, the conditionally active antibodies provided herein, including modified anti-EGFR antibodies herein, are those that bind to epidermal growth factor receptor (EGFR) with a higher binding activity at an elevated lactate concentration of between 10 to 20 mM than a lactate concentration of 0.5 mM to 5 mM, and in the presence of 20% to 50% serum (vol/vol), such as 20% to 50% human serum, such as at least 20%, 25%, 30%, 35%, 40%, 45% or 50% serum (vol/vol). For example, the ratio of binding activity under conditions of 10 to 20 mM lactate, such as at or about 16 mM, compared to under conditions of 1 mM to 5 mM is greater than 1.0, for example, at least or greater than 1.1, 1.2, 1.3, 1.4, 1.5, 1.6, 1.7, 1.8, 1.9, 2.0, 2.5, 3.0, 3.5, 4.0, 4.5, 5.0, 6.0, 7.0, 8.0, 9.0, 10.0, 11.0, 12.0, 13.0, 14.0, 15.0, 20.0, 25.0, 30.0, 35.0, 40.0, 45.0, 50.0 or more or more.

In some examples, the anti-EGFR antibodies provided herein exhibit increased binding activity under conditions of pH 6.0 or pH 6.5 and lactate concentration of 10 mM to 20 mM than under conditions of neutral pH (about pH 7.4) and lactate concentration of 1 mM to 5 mM, and in the presence of 10 mg/mL to 50 mg/mL protein, such as at least 12 mg/mL to 40 mg/mL protein (*e.g.* at least 12 mg/mL, 15 mg/mL, 20 mg/mL, 25 mg/mL, 30 mg/mL, 35 mg/mL or 40 mg/mL protein). For example, the anti-EGFR antibodies provided herein exhibit increased binding activity under conditions of pH 6.0 or pH 6.5 and lactate concentration of 10 mM to 20 mM than under conditions of neutral pH (about pH 7.4) and lactate concentration of 1 mM to 5 mM, and in the presence of 20% to 50% serum (vol/vol), such as 20% to 50% human serum, such as at least 20%, 25%, 30%, 35%, 40%, 45% or 50% serum (vol/vol). For example, the ratio of binding activity under conditions of pH 6.0 or 6.5 and 10 to 20 mM lactate, such as or about 16 mM, compared to under conditions of neutral pH (e.g. 7.4) and 1 mM to 5 mM lactate is greater than 1.0, for example, at least or greater than 1.1, 1.2, 1.3, 1.4, 1.5, 1.6, 1.7, 1.8, 1.9, 2.0, 2.5, 3.0, 3.5, 4.0, 4.5, 5.0, 6.0, 7.0, 8.0, 9.0, 10.0, 11.0, 12.0, 13.0, 14.0, 15.0, 20.0, 25.0, 30.0, 35.0, 40.0, 45.0, 50.0 or more or more.

The ratio of binding activity under the above conditions in a tumor microenvironment compared to under conditions in a non-tumor microenvironment can be determined or assessed based on any methods known to a person of skill in the art to assess binding of an antibody, or antigen-binding fragment, to EGFR (e.g. human EGFR). Exemplary of such assays are described in Section D. In one example, the binding activity is determined *in vitro* in a solid-phase binding assay, such as in an immunoassay (e.g. an enzyme-linked immunosorbent assay; ELISA) under any of the above conditions in a tumor microenvironment and any of the above conditions in a non-tumor microenvironment. In such examples, the binding activity can be represented as a spectrophotometric measurement (*e.g.* optical density and an absorbance wavelength compatible with the particular detection methods employed), and the ratio of binding activity can be the ratio of the spectrophotometric measurement for binding under conditions that exist in a tumor microenvironment compared to under conditions that exist in a non-tumor microenvironment at the same concentration of antibody (e.g. an antibody concentration of 1 ng/mL to 100 ng/mL). This is exemplified in the Examples herein. An anti-EGFR antibody, or antigen-binding fragment thereof, is a conditionally active antibody if the ratio of activity as determined from spectrophotometric measurements or other similar quantitative measurements in a solid-phase immunoassay that is greater than 1.0, for example, at least or greater than 1.1, 1.2, 1.3, 1.4, 1.5, 1.6, 1.7, 1.8, 1.9, 2.0, 2.5, 3.0, 3.5, 4.0, 4.5, 5.0, 6.0, 7.0, 8.0, 9.0, 10.0, 11.0, 12.0, 13.0, 14.0, 15.0, 20.0, 25.0, 30.0, 35.0, 40.0, 45.0, 50.0 or more or more.

In another example, the binding activity is determined as a kinetic measure of binding (e.g. dissociation constant, $K_D$, association constant $K_A$, off-rate or other kinetic parameter of binding affinity) under any of the above conditions in a tumor microenvironment and any of the above conditions in a non-tumor microenvironment. Such measurements can be determined using any binding assay known to a skilled artisan. In particular examples, an affinity-based biosensor technology is utilized as a measure of binding affinity. Exemplary biosensor technologies include, for example, Biacore technologies, BioRad ProteOn, Reichert, GWC Technologies, IBIS SPIR Imaging, Nomadics SensiQ, Akubio RAPid, ForteBio Octet, IAsys, Nanofilm and others (see e.g. Rich et al. (2009) Analytical Biochemistry, 386:194-216). In such examples, the binding activity can be represented as a the dissociation constant ($K_D$), and the ratio of binding activity can be the ratio of tighter affinity binding under conditions that exist in a tumor microenvironment compared to under conditions that exist in a non-tumor microenvironment. For example, a ratio of binding activity of at least 2.0 means that there is at least 2-fold tighter affinity, a ratio of binding activity of at least 3.0 means that there is at least 3-fold tighter affinity, a ratio of binding activity of at least 4.0 means that there is at least 4-fold tighter affinity, a ratio of binding activity of at least 5.0 means that there is at least 5-fold tighter affinity, a ratio of binding activity of at least 10.0 means that there is at least 10-fold tighter affinity, where the ratio of each is under conditions in the tumor microenvironment compared to under conditions in a non-tumor microenvironment. The anti-EGFR antibodies, or antigen-binding fragments provided herein, typically have a dissociation constant ($K_D$) for binding EGFR (e.g. human EGFR) or a soluble fragment thereof that is less than $1 \times 10^{-8}$ M, $5 \times 10^{-9}$ M, $1 \times 10^{-9}$ M, $5 \times 10^{-10}$ M, $1 \times 10^{-10}$ M, $5 \times 10^{-11}$ M, $1 \times 10^{-11}$ M or less under conditions that exist in a tumor microenvironment. In another example, the binding activity can be represented as the off-rate, and the ratio of binding activity can be the ratio of the $k_{off}$ under conditions that exist in a tumor microenvironment compared to under conditions that exist in a non-tumor microenvironment. For example, a ratio of binding activity of at least 2.0 means that the antibody exhibits an off-rate that is at least 2 times slower, a ratio of binding activity of at least 3.0 means that the antibody exhibits an off-rate that is at least 3 times slower, a ratio of binding activity of at least 4.0 means that the antibody exhibits an off-rate that is at least 4 times slower, a ratio of binding activity of at least 5.0 means that the antibody exhibits an off-rate that is at least 5 times slower, a ratio of binding activity of at least 10.0 means that the antibody exhibits an off-rate that is at least 10 times slower, where the ratio of each is under conditions in the tumor microenvironment compared to under conditions in a non-tumor microenvironment. This is exemplified in the Examples herein. An anti-EGFR antibody, or antigen-binding fragment thereof, is a conditionally active antibody if the ratio of activity as determined using kinetic measurements of binding is greater than 1.0, for example, at least or greater than 1.1, 1.2, 1.3, 1.4, 1.5, 1.6, 1.7, 1.8, 1.9, 2.0, 2.5, 3.0, 3.5, 4.0, 4.5, 5.0, 6.0, 7.0, 8.0, 9.0, 10.0, 11.0, 12.0, 13.0, 14.0, 15.0, 20.0, 25.0, 30.0, 35.0, 40.0, 45.0, 50.0 or more or more.

In a further example, the binding activity is determined in an *in vivo* binding activity assay assessing binding in a tumor microenvironment and binding in a non-tumor microenvironment. Exemplary of a non-tumor microenvironment is binding of the antibody to the basal layer of the skin containing keratinocytes. The binding assays can be performed using animal models known to contain cells expressing EGFR in each environment. In particular, the animal models express human EGFR. For example, a murine animal model or other mammalian animal model can be used that is generated by xenograft procedures to engineer microenvironments to contain tumor or non-tumor cells expressing human EGFR. This is exemplified herein using tumor xenograft procedures (e.g. with A431 cells or other human tumor cells) and skin xenograft procedures. In such examples, the antibody, or antigen-binding fragment thereof, is detectably labeled, for example fluorescently labeled. In such examples, the binding activity can be represented as the detectable signal produced (*e.g.* intensity of the fluorescent signal), and the ratio of binding activity can be the ratio of the intensity of the detectable signal (e.g. fluorescent signal) for binding under conditions that exist in a tumor microenvironment compared to under conditions that exist in a non-tumor microenvironment. The staining intensity can be normalized by normalizing to staining of a control or reference antibody. This is exemplified in the Examples herein. An anti-EGFR antibody, or antigen-binding fragment thereof, is a conditionally active antibody if the ratio of activity as determined from in vivo binding in the two environments is greater than 1.0, for example, at least or greater than 1.1, 1.2, 1.3, 1.4, 1.5, 1.6, 1.7, 1.8, 1.9, 2.0, 2.5, 3.0, 3.5, 4.0, 4.5, 5.0, 6.0, 7.0, 8.0, 9.0, 10.0, 11.0, 12.0, 13.0, 14.0, 15.0, 20.0, 25.0, 30.0, 35.0, 40.0, 45.0, 50.0 or more or more.

By virtue of the conditional activity in a tumor microenvironment, such as increased binding activity under conditions present in a tumor microenvironment (*e.g.* low pH, such as pH 6.0 and elevated lactate, such as 10 to 20 mM), the antibodies provided herein exhibit increased inhibitory activity against EGFR in a tumor microenvironment compared to a non-diseased environment. Such inhibitory activities include, but are not limited to, inhibition of ligand-induced phosphorylation, dimerization and/or cell growth. As a result of such activities, antibodies provided herein exhibit tumor growth inhibition when administered *in vivo* to a subject having a tumor, such as a solid tumor. Tumor growth can be inhibited 30%, 40%, 50%, 60%, 70%, 80%, 90% or more compared to the growth of tumors in the absence of administered antibody. The functional activity of the anti-EGFR antibodies provided herein can be less than, similar to or greater than existing anti-EGFR therapies, such as therapies with cetuximab, when assessed in tumor models, so long as the activity in non-diseased tissues is reduced (e.g. incidence of skin rash). For example, the anti-EGFR antibodies provided herein exhibit efficacy *in vivo* in an *in vivo* animal tumor model, such as an A431 model as described herein, similar to cetuximab with a lower binding affinity (higher Kd) than cetuximab.

The conditionally active anti-EGFR antibodies provided herein, such as modified anti-EGFR antibodies provided herein, exhibit conditional and selective tumor-specific activity such that, upon administration to a subject, the subject exhibits reduced or fewer side effects, compared to the subject that is administered another existing anti-EGFR therapy, such as therapy with cetuximab (e.g. the corresponding form of a wildtype cetuximab having a heavy chain sequence of amino acids set forth in SEQ ID NO:1 and a light chain sequence of amino acids set forth in SEQ ID NO:2, or a heavy chain sequence of amino acids set forth in SEQ ID NO:8 and a light chain sequence of amino acids set forth in SEQ ID NO:9). For example, the provided anti-EGFR antibodies, or antigen binding fragments thereof, exhibit reduced dermal toxicity. Dermal toxicity, such as skin rash, can be assessed by standard assays known to one of skill in the art and described herein. For example, the anti-EGFR antibodies, or antigen binding fragments thereof, provided herein exhibit at least a 1.5-fold, 2-fold, 2.5-fold, 3-fold, 4-fold, 5-fold, or more decreased rash, such as assessed in a primate model.

It is within the level of a skilled artisan to identify or generate conditionally active anti-EGFR antibodies, or antigen-binding fragments thereof, that exhibit greater activity in a tumor microenvironment than in a non-tumor microenvironment as described herein. For example, anti-EGFR antibodies can be generated, including libraries of modified anti-EGFR antibodies, and can be screened using procedures and methods described herein in Section D. In the subsections below, exemplary anti-EGFR antibodies, including exemplary modified anti-EGFR antibodies derived from cetuximab or an antigen-binding fragment or variant thereof, that exhibit the altered properties and activities described above are set forth. It is understood that the resulting anti-EGFR antibody, or antigen-binding fragment thereof, minimally contains a variable heavy chain and a variable light chain, or a portion thereof that is sufficient to bind EGFR antigen (e.g. human EGFR), or a soluble fragment thereof, when assembled into an antibody.

## 1. MODIFIED ANTI-EGFR ANTIBODIES

Provided herein are modified or variant anti-EGFR antibodies, or antigen binding fragments thereof. Included among the modified anti-EGFR antibodies are antibodies that are conditionally active such that they exhibit higher or greater activity in a tumor microenvironment than in a non-diseased environment, such as the skin or basal layer of the skin. The antibodies provided herein are variants of the anti-EGFR antibody cetuximab or derivatives thereof. It is understood that the resulting anti-EGFR antibody, or antigen-binding fragment thereof, minimally contains a variable heavy chain and a variable light chain, or a portion thereof that is sufficient to bind EGFR antigen (e.g. human EGFR), or a soluble fragment thereof, when assembled into an antibody, whereby one or both of the variable heavy or light chain is modified. As described above, included among such modified anti-EGFR antibodies, or antigen-binding fragments thereof, are antibodies that bind to EGFR (particularly human EGFR) with a higher binding activity under conditions that exist in a tumor microenvironment that include one or both of pH between or about between pH 5.6 to 6.8 (e.g. pH 6.0 to 6.5) or lactate concentration of between or about between 5 mM to 20 mM (*e.g.* 10 mM to 20 mM, such as at least 16 mM) compared to under conditions that exist in a non-tumor microenvironment that include one or both of pH between or about between pH 7.0 to 7.8 (e.g. pH of 7.0 to 7.4) or lactate concentration between or about between 0.5 mM to 5 mM (e.g. 1 mM to 4 mM). The higher binding activity under conditions in a tumor microenvironment compared to under conditions in a non-tumor microenvironment can be a ratio of activity of at least or greater than 1.1, 1.2, 1.3, 1.4, 1.5, 1.6, 1.7, 1.8, 1.9, 2.0, 2.5, 3.0, 3.5, 4.0, 4.5, 5.0, 6.0, 7.0, 8.0, 9.0, 10.0, 11.0, 12.0, 13.0, 14.0, 15.0, 20.0, 25.0, 30.0, 35.0, 40.0, 45.0, 50.0 or more.

The modified anti-EGFR antibodies provided herein include those that exhibit increased or decreased or similar of the binding activity at pH 6.0 or pH 6.5 than the corresponding form of an unmodified cetuximab antibody, antigen-binding fragment thereof or variant thereof, such as a wildtype cetuximab having a heavy chain sequence of amino acids set forth in SEQ ID NO:1 and a light chain sequence of amino acids set forth in SEQ ID NO:2, or a heavy chain sequence of amino acids set forth in SEQ ID NO:8 and a light chain sequence of amino acids set forth in SEQ ID NO:9. In some examples, the antibodies exhibit at least 20%, 30%, 40%, 50%, 60%, 70%, 80%, 90%, 95% or more binding activity at pH 6.0 than the corresponding form of an unmodified cetuximab antibody, antigen-binding fragment thereof or variant thereof, such as a wildtype cetuximab having a heavy chain sequence of amino acids set forth in SEQ ID NO:1 and a light chain sequence of amino acids set forth in SEQ ID NO:2, or a heavy chain sequence of amino acids set forth in SEQ ID NO:8 and a light chain sequence of amino acids set forth in SEQ ID NO:9. Generally, the modified anti-EGFR antibodies provided herein exhibit 100 % to 500 %, such as at least 100 % or more (i.e. increased) of the binding activity, such as at or about or at least 100%, 105%, 110%, 115%, 120%, 125%, 130%, 135%, 140%, 145%, 150%, 155%, 160%, 165%, 170%, 175%, 180%, 185%, 190%, 195%, 200%, 250%, 300%, 350%, 400%, 450%, 500%, or more of the binding activity at pH 6.0 or pH 6.5 compared to the binding activity of the corresponding form of an unmodified cetuximab antibody, antigen-binding fragment thereof or variant thereof, such as the wildtype cetuximab having a heavy chain sequence of amino acids set forth in SEQ ID NO:1 and a light chain sequence of amino acids set forth in SEQ ID NO:2, or a heavy chain sequence of amino acids set forth in SEQ ID NO:8 and a light chain sequence of amino acids set forth in SEQ ID NO:9.

In some examples, the modified anti-EGFR antibodies provided herein exhibit 30 % to 95% of the EGFR binding activity at pH 7.4 of a corresponding form of an unmodified cetuximab, antigen-binding fragment thereof or variant thereof, such as a cetuximab having a heavy chain set forth in SEQ ID NO:1 and a light chain set forth in SEQ ID NO:2 or having a heavy chain set forth in SEQ ID NO:8 and a light chain set forth in SEQ ID NO:9. For example, anti-EGFR antibodies provided herein exhibit at least 30% of the binding activity, such as at or about or at least 30%, 35%, 40%, 45%, 50%, 55%, 60%, 65%, 70%, 75%, 80%, 85%, 90%, or 95% of the binding activity at neutral pH (e.g. pH 7.4) of the reference or unmodified cetuximab not containing the amino acid modification (e.g. replacement). In particular examples, the antibodies provided herein retain or exhibit similar or increased binding activity at pH 6.0 or pH 6.5 compared to binding activity of the unmodified cetuximab antibody or antigen-binding fragment or variant thereof under the same conditions, but exhibit decreased binding activity at neutral pH (e.g. pH 7.4), such as less than 20%, 30%, 40%, 50%, 60%, 70%, 80%, 90%, 95% binding activity at pH 7.4 than the corresponding form of an unmodified cetuximab antibody, antigen-binding fragment thereof or variant thereof, such as a wildtype cetuximab having a heavy chain sequence of amino acids set forth in SEQ ID NO:1 and a light chain sequence of amino acids set forth in SEQ ID NO:2, or a heavy chain sequence of amino acids set forth in SEQ ID NO:8 and a light chain sequence of amino acids set forth in SEQ ID NO:9. For example, modified anti-EGFR antibodies provided herein include those that exhibit 30 % to 95% of the EGFR binding activity at pH 7.4 and 100 % to 500 % of the EGFR binding activity at pH 6.0 of a reference anti-EGFR antibody that does not contain the modification, such as the corresponding form of cetuximab having a heavy chain set forth in SEQ ID NO:1 and a light chain set forth in SEQ ID NO:2 or having a heavy chain set forth in SEQ ID NO:8 and a light chain set forth in SEQ ID NO:9.

Included among the modified anti-EGFR antibodies provided herein are those that exhibit decreased, increased or similar EGFR binding activity at elevated lactate levels, *e.g.,* 10-20 mM lactate. Generally, the antibodies exhibit at least 20%, 30%, 40%, 50%, 60%, 70%, 80%, 90%, 95% or more binding activity under conditions of 10 to 20 mM lactate concentration than the corresponding form of an unmodified cetuximab antibody, antigen-binding fragment thereof or variant thereof, such as the wildtype cetuximab having a heavy chain sequence of amino acids set forth in SEQ ID NO:1 and a light chain sequence of amino acids set forth in SEQ ID NO:2, or a heavy chain sequence of amino acids set forth in SEQ ID NO: 8 and a light chain sequence of amino acids set forth in SEQ ID NO:9. In some cases, the antibodies exhibit increased binding activity under conditions of 10 to 20 mM lactate concentration, for example 100 % to 500 % of the activity, such as greater than 100 % of the binding activity, for example at least or about at least 110%, 120%, 130%, 140%, 150%, 160%, 170%, 180%, 190%, 200%, 300%, 400%, 500% or more of the binding activity than the corresponding form of the unmodified cetuximab antibody, antigen-binding fragment or variant thereof, such as a wildtype cetuximab having a heavy chain sequence of amino acids set forth in SEQ ID NO:1 and a light chain sequence of amino acids set forth in SEQ ID NO:2, or a heavy chain sequence of amino acids set forth in SEQ ID NO:8 and a light chain sequence of amino acids set forth in SEQ ID NO:9.

In some examples, the modified anti-EGFR antibodies provided herein exhibit 30 % to 95 % of the EGFR binding activity at normal lactate levels (*e.g.*, between 0 and 5 mM lactate) compared to the corresponding form of the unmodified cetuximab antibody, antigen-binding fragment or variant thereof, such as a wildtype cetuximab having a heavy chain sequence of amino acids set forth in SEQ ID NO:1 and a light chain sequence of amino acids set forth in SEQ ID NO:2, or a heavy chain sequence of amino acids set forth in SEQ ID NO:8 and a light chain sequence of amino acids set forth in SEQ ID NO:9. For example, the antibodies provided herein retain or exhibit similar binding activity under conditions of 10-20 mM lactate compared to binding activity of cetuximab under the same conditions, but exhibit decreased binding activity under conditions of 1 mM to 5 mM lactate, such as 20%, 30%, 40%, 50%, 60%, 70%, 80%, 90%, 95% or more binding activity under conditions of 1 mM to 5 mM lactate than the corresponding form of a wildtype cetuximab having a heavy chain sequence of amino acids set forth in SEQ ID NO:1 and a light chain sequence of amino acids set forth in SEQ ID NO:2, or a heavy chain sequence of amino acids set forth in SEQ ID NO:8 and a light chain sequence of amino acids set forth in SEQ ID NO:9. In yet other examples, the modified anti-EGFR antibodies provided herein exhibit 30 % to 95 % of the EGFR binding activity at normal lactate levels (*e.g.,* between 0 and 5 mM lactate, and 100 % to 500 % of the EGFR binding activity at elevated lactate levels (*e.g.,* 10-20 mM lactate) of a reference or unmodified anti-EGFR antibody that does not contain the modification, such as compared to the corresponding form of a wildtype cetuximab having a heavy chain sequence of amino acids set forth in SEQ ID NO:1 and a light chain sequence of amino acids set forth in SEQ ID NO:2, or a heavy chain sequence of amino acids set forth in SEQ ID NO:8 and a light chain sequence of amino acids set forth in SEQ ID NO:9.

In exemplary examples provided herein, modified anti-EGFR antibodies provided herein exhibit 30 % to 95 % of the EGFR binding activity at pH 7.4, 100 % to 500 % of the EGFR binding activity at pH 6.0, 30 % to 95 % of the EGFR binding activity at normal lactate levels (*e.g.*, between 0 and 5 mM lactate), and 100 % to 500 % of the EGFR binding activity at elevated lactate levels (*e.g.,* 10-20 mM lactate), compared to a reference anti-EGFR antibody that does not contain the modification(s), such as to the corresponding form of a wildtype cetuximab having a heavy chain sequence of amino acids set forth in SEQ ID NO:1 and a light chain sequence of amino acids set forth in SEQ ID NO:2, or a heavy chain sequence of amino acids set forth in SEQ ID NO:8 and a light chain sequence of amino acids set forth in SEQ ID NO:9. For example, the modified anti-EGFR antibodies provided herein exhibit increased binding to EGFR at an acidic pH (*e.g.,* pH 6.0), increased binding to EGFR at elevated lactate levels (*e.g.,* 16.6 mM lactate), decreased binding to EGFR at a neutral pH (*e.g.,* pH 7.4), and/or decreased binding to EGFR at normal lactate levels (*e.g.* 1 mM lactate).

In examples herein where binding activity is increased under conditions present in a tumor microenvironment, the provided antibodies can exhibit an increased binding affinity to EGFR at pH 6.0 or pH 6.5 and/or a decreased binding affinity at neutral pH (e.g. pH 7.4) compared to the corresponding form of an unmodified cetuximab or an antigen-binding fragment or variant thereof, such as a wildtype cetuximab having a heavy chain sequence of amino acids set forth in SEQ ID NO:1 and a light chain sequence of amino acids set forth in SEQ ID NO:2, or a heavy chain sequence of amino acids set forth in SEQ ID NO:8 and a light chain sequence of amino acids set forth in SEQ ID NO:9. In particular examples, the anti-EGFR antibodies provided herein exhibit at least a 1.5-fold, 2-fold, 2.5-fold, 3-fold, 4-fold, 5-fold or more decrease in binding affinity (*e.g.,* Kd) *in vitro* at pH 7.4 while retaining comparable binding to EGFR at pH 6.0.

Exemplary of anti-EGFR antibodies, or antigen binding fragments thereof, provided herein are those that contain modifications compared to a reference anti-EGFR antibody having a heavy chain set forth in any of SEQ ID NOS: 1, 3, 5, 8 or 28, and a light chain set forth in any of SEQ ID NOS:2, 4, 9, 10 or 29, or in a heavy chain that has a sequence of amino acids that is at least 65%, 70%, 75%, 80%, 85%, 86%, 87%, 88%, 89%, 90%, 91%, 92%, 93%, 94%, 95%, 96%, 97%, 98%, 99% identical to any of SEQ ID NOS:1, 3, 5, 8 or 28, and a light chain that has a sequence of amino acids that is at least 65%, 70%, 75%, 80%, 85%, 86%, 87%, 88%, 89%, 90%, 91%, 92%, 93%, 94%, 95%, 96%, 97%, 98%, 99% identical to any of SEQ ID NOS:2, 4, 9, 10 or 29. Included among the modified anti-EGFR antibodies provided herein are variants of the anti-EGFR antibody cetuximab that have altered properties as compared to cetuximab. In exemplary embodiments, the anti-EGFR antibodies, or antigen binding fragments thereof, are modified such that they are targeted to a tumor environment, for example, by binding EGFR under a condition or conditions that are associated with, or specific to, tumors.

The modifications described herein can be in any cetuximab anti-EGFR antibody or variant antibody thereof. For example, the modifications are made in cetuximab antibody containing: a heavy chain having a sequence of amino acids set forth in SEQ ID NO:1 and a light chain having a sequence set forth in SEQ ID NO:2, or a heavy chain having a sequence of amino set forth in SEQ ID NO:8 and a light chain having a sequence of amino acids set forth in SEQ ID NO:9, or in sequence variants of the heavy chain set forth in SEQ ID NO: 1 or 8 and/or the light chain set forth in SEQ ID NO:2 or 9 that exhibit at least 65%, 70%, 75%, 80%, 85%, 86%, 87%, 88%, 89%, 90%, 91%, 92%, 93%, 94%, 95%, 96%, 97%, 98%, 99% or more sequence identity to the heavy or light chain. In some examples, the modifications are made in a humanized cetuximab antibody containing a heavy chain having a sequence of amino acids set forth in SEQ ID NO:28 and a light chain having a sequence of amino acids set forth in SEQ ID NO:29; or in sequence variants that exhibit at least 65%, 70%, 75%, 80%, 85%, 86%, 87%, 88%, 89%, 90%, 91%, 92%, 93%, 94%, 95%, 96%, 97%, 98%, 99% or more sequence identity to the heavy chain set forth in SEQ ID NO:28 and/or the light chain set forth in SEQ ID NO:29.

Generally, the modifications are made in the variable region of such antibodies. For example, the modifications are made in the heavy and/or light chain variable regions of such a cetuximab antibody, for example, in a sequence containing a variable heavy chain sequence set forth in SEQ ID NO:3 and a variable light chain sequence set forth in SEQ ID NO:4; or having a variable heavy chain sequence set forth in SEQ ID NO:3 and a variable light chain sequence set forth in SEQ ID NO: 10. The resulting modified anti-EGFR antibodies can be full-length IgG1 antibodies, or can be fragments thereof, for example, a Fab, Fab', F(ab')$_2$, single-chain Fv (scFv), Fv, dsFv, diabody, Fd and Fd' fragments. Further, the resulting modified anti-EGFR antibodies can contain a domain other than IgG1.

The modifications can be a single amino acid modification, such as single amino acid replacements (substitutions), insertions or deletions, or multiple amino acid modifications, such as multiple amino acid replacements, insertions or deletions. Exemplary of modification are amino acid replacements, including single or multiple amino acid replacements. Modified anti-EGFR antibodies provided herein can contain at least or 1, 2, 3, 4, 5, 6, 7, 8, 9, 10, 11, 12, 13, 14, 15, 16, 17, 18, 19, 20, or more modified positions compared to the anti-EGFR antibody not containing the modification. In some examples, the modified anti-EGFR antibody provided contains only 1 or only 2 amino acid replacements compared to an unmodified cetuximab or antigen-binding fragment or variant thereof. The amino acid replacement can be a conservative substitution, such as set forth in Table 4, or a non-conservative substitution, such as any described herein. It is understood that an anti-EGFR antibody, or antigen-binding fragment thereof, containing an exemplary modification herein that confers conditional activity as described herein can be further modified by humanization as described below, as long as the resulting antibody retains conditional activity in a tumor microenvironment compared to a non-tumor microenvironment.

For purposes herein, reference to positions and amino acids for modification, including amino acid replacement or replacements, are with reference to the variable heavy chain set forth in SEQ ID NO:3 and the variable light chain set forth in SEQ ID NO:4. It is within the level of one of skill in the art to make any of the modifications provided herein in another anti-EGFR antibody by identifying the corresponding amino acid residue in another heavy chain, such as set forth in SEQ ID NOS:1, 5, 8 or 28, or another light chain, such as set forth in SEQ ID NOS:2, 9, 10 or 29, or variants thereof that exhibit at least 65%, 70%, 75%, 80%, 85%, 86%, 87%, 88%, 89%, 90%, 91%, 92%, 93%, 94%, 95%, 96%, 97%, 98%, 99% or more sequence identity to any of SEQ ID NOS: 1, 2, 5, 8-10 or 28-29. Corresponding positions in another anti-EGFR antibody can be identified by alignment of the anti-EGFR antibody heavy chain or light chain with the reference anti-EGFR heavy chain set forth in SEQ ID NO:3 or light chain set forth in SEQ ID NO:4. For example, Figure 2 depicts alignment of anti-EGFR antibodies with SEQ ID NO:3 and 4, and identification of exemplary corresponding positions. For purposes of modification (e.g. amino acid replacement), the corresponding amino acid residue can be any amino acid residue, and need not be identical to the residues set forth in SEQ ID NO:3 or 4. Typically, the corresponding amino acid residue identified by alignment with residues in SEQ ID NO:3 or 4 is an amino acid residue that is identical to SEQ ID NO:3 or 4, or is a conservative or semi-conservative amino acid residue thereto (*see e.g.* Figure 2). It is also understood that the exemplary replacements provided herein can be made at the corresponding residue in an anti-EGFR antibody heavy chain or light chain, so long as the replacement is different than exists in the unmodified form of the anti-EGFR antibody heavy chain or light chain. Based on this description and the description elsewhere herein, it is within the level of one of skill in the art to generate a modified anti-EGFR antibody containing any one or more of the described mutations, and test each for a property or activity as described herein.

Modifications in an anti-EGFR antibody also can be made to an anti-EGFR antibody that also contains other modifications, including modifications in the variable regions of the antibody and modifications in the constant regions of the antibody, for example, in the $C_H1$, hinge, $C_H2$, $C_H3$ or $C_L$ regions.

The modified anti-EGFR antibodies provided herein can be produced by standard recombinant DNA techniques known to one of skill in the art. Any method known in the art to effect mutation of any one or more amino acids in a target protein can be employed. Methods include standard site-directed or random mutagenesis of encoding nucleic acid molecules, or solid phase polypeptide synthesis methods. For example, nucleic acid molecules encoding a heavy chain or light chain of an anti-EGFR antibody can be subjected to mutagenesis, such as random mutagenesis of the encoding nucleic acid, error- prone PCR, site-directed mutagenesis, overlap PCR, gene shuffling, or other recombinant methods. The nucleic acid encoding the anti-EGFR antibodies can then be introduced into a host cell to be expressed heterologously. Hence, also provided herein are nucleic acid molecules encoding any of the modified anti-EGFR antibodies provided herein.

A non-limiting example of exemplary modifications in the variable heavy chain and/or a variable light chain, or a portion thereof, of a cetuximab antibody or antigen-binding fragment thereof with reference to the variable heavy chain set forth in SEQ ID NO:3 and the variable light chain set forth in SEQ ID NO:4 are provided below.

**a. Heavy Chain Modifications**

Provided herein are modified anti-EGFR antibodies containing a modification(s), such as an amino acid replacement, in a variable heavy chain of a cetuximab antibody, antigen-binding fragment thereof or variant thereof, corresponding to amino acid residue(s) in a cetuximab antibody containing a variable heavy chain set forth in SEQ ID NO:3. The resulting modification(s) can be in a heavy chain, or portion thereof, such as set forth in any of SEQ ID NOS:1, 3, 5, 8 or 28 or a variant thereof having at least 75%, 80%, 81%, 82%, 83%, 84%, 85%, 86%, 86%, 88%, 89%, 90%, 91%, 92%, 93%, 94%, 95%, 96%, 97%, 98%, 99% or more sequence identity thereto. The modification can be in a complementarity determining region (CDR) or in a framework region.

For example, provided herein are modified anti-EGFR antibodies containing a variable heavy chain, or portion thereof, having at least one amino acid replacement or substitution at any of positions corresponding to positions 23, 24, 25, 26, 27, 28, 29, 30, 31, 32, 33, 34, 35, 36, 50, 51, 52, 53, 54, 55, 56, 57, 58, 59, 60, 61, 62, 63, 64, 65, 66, 67, 68, 69, 70, 71, 72, 73, 74, 75, 76, 77, 93, 94, 97, 98, 99, 100, 101, 102, 103, 104, 105, 106, 107, 108, 109, 110, 111 or 112 with reference to the amino acid positions set forth in SEQ ID NO:3. For example, the amino acid positions can be replacements at positions corresponding to replacement of Threonine (T) at position 23 (T23), V24, S25, G26, F27, S28, L29, T30, N31, Y32, G33, V34, H35, W36, V50, I51, W52, S53, G54, G55, N56, T57, D58, Y59, N60, T61, P62, F63, T64, S65, R66, L67, S68, I69, N70, K71, D72, N73, S74, K75, S76, Q77, Y93, Y94, R97, A98, L99, T100, Y101, Y102, D103, Y104, E105, F106, A107, Y108, W109, G110, Q111 or G112 with reference to the amino acid positions set forth in SEQ ID NO:3.

With reference to Kabat numbering, such positions in the heavy chain that can be modified, for example by amino acid replacement or substitution, include, but are not limited to, any of the positions corresponding to positions 23, 24, 25, 26, 27, 28, 29, 30, 31, 32, 33, 34, 35, 36, 50, 51, 52, 53, 54, 55, 56, 57, 58, 59, 60, 61, 62, 63, 64, 65, 66, 67, 68, 69, 70, 71, 72, 73, 74, 75, 76, 77, 90, 91, 94, 95, 96, 97, 98, 99, 100, 100a, 100b, 100c, 101, 102, 103, 104, 105 or 106. In some examples, the amino acid residue that is modified (*e.g.* replaced) at the position corresponding to any of the above positions is a conservative residue or a semi-conservative amino acid residue to the amino acid set forth in SEQ ID NO:3 (see e.g. Figure 2).

In one example, provided herein are modified anti-EGFR antibodies containing a variable heavy chain having a modification(s) in a CDR or CDRs, such as, for example, CDRH1, CDRH2 and/or CDRH3. For example, provided herein are modified anti-EGFR antibodies containing a variable heavy chain having one or more amino acid replacements in a CDR1 at any of positions corresponding to positions 26, 27, 28, 29, 30, 31, 32, 33, 34 or 35 with reference to the amino acid positions set forth in SEQ ID NO:3; CDR2 at any of positions corresponding to positions 50, 51, 52, 53, 54, 55, 56, 57, 58, 59, 60, 61, 62, 63, 64 or 65 with reference to the amino acid positions set forth in SEQ ID NO:3; CDR3 at any of positions corresponding to positions 98, 99, 100, 101, 102, 103, 104, 105, 106, 107 or 108 with reference to the amino acid positions set forth in SEQ ID NO:3.

In other examples, provided herein are modified anti-EGFR antibodies containing a variable heavy chain containing a modification(s) in a framework region (FW) heavy chain FW1, FW2, FW3 or FW4. For example, provided herein are modified anti-EGFR antibodies containing a variable heavy chain having one or more amino acid replacements in a heavy chain FW1 at any of positions corresponding to positions 23, 24 or 25 with reference to the amino acid positions set forth in SEQ ID NO:3; FW2 at any of positions corresponding to positions 36 or 37 with reference to the amino acid positions set forth in SEQ ID NO:3; FW3 at any of positions corresponding to positions 66, 67, 68, 69, 70, 71, 72, 73, 74, 75, 76, 77, 93, 94 or 97 with reference to the amino acid positions set forth in SEQ ID NO:3; and FW4 at any of positions corresponding to positions 109, 110, 111 or 112 with reference to the amino acid positions set forth in SEQ ID NO:3.

Provided herein are modified anti-EGFR antibodies having at least one amino acid replacement in the variable heavy chain, or portion thereof, corresponding to replacements set forth in Table 5 with reference to positions set forth in SEQ ID NO:3

| Table 5. Exemplary heavy chain amino acid replacements | | | | | |
|---|---|---|---|---|---|
| T023K | T030H | G054D | S065P | N073R | T100S |
| T023H | T030R | G054P | S065Q | N073L | T100V |
| T023R | T030D | G054S | S065T | N073A | T100Y |
| T023A | T030G | G055H | S065W | N073C | Y101H |
| T023C | T030I | G055R | S065Y | N073G | Y101E |
| T023E | T030M | G055M | R066L | N073I | Y101F |
| T023G | T030N | G055S | R066A | N073M | Y101M |
| T023I | T030P | G055Y | R066C | N073P | Y101W |
| T023M | T030S | N056K | R066E | N073Q | Y102R |
| T023N | T030V | N056A | R066F | N073S | Y102C |
| T023P | T030W | N056P | R066N | N073T | Y102D |
| T023S | T030Y | N056S | R066P | N073V | Y102I |
| T023V | N031K | N056V | R066Q | N073W | Y102N |
| T023W | N031H | N056G | R066S | N073Y | Y102W |
| T023L | N031D | T057H | R066T | S074K | D103R |
| V024R | N031E | T057R | R066V | S074H | D103L |
| V024A | N031G | T057L | R066G | S074R | D103A |
| V024F | N031I | T057A | L067A | S074L | D103C |
| V024G | N031T | T057C | L067C | S074A | D103I |
| V024I | N031V | T057D | L067D | S074C | D103P |
| V024M | N031L | T057F | L067E | S074D | D103Q |
| V024P | Y032H | T057M | L067I | S074E | D103Y |
| V024S | Y032R | T057N | L067M | S074G | Y104H |
| V024T | Y032C | T057Q | L067Q | S074I | Y104L |
| V024L | Y032M | T057W | L067S | S074M | Y104D |
| V024E | Y032N | T057Y | L067T | S074P | Y104F |
| S025H | Y032T | D058L | L067V | S074T | Y104I |
| S025R | Y032V | D058G | L067Y | S074V | Y104M |
| S025A | Y032L | D058M | L067G | S074Y | Y104S |
| S025C | G033E | D058N | S068K | K075H | Y104V |
| S025D | G033M | D058Q | S068H | K075R | E105H |
| S025E | G033S | Y059H | S068R | K075L | E105T |
| S025F | G033T | Y059R | S068L | K075A | F106L |
| S025G | G033Y | Y059A | S068C | K075C | F106V |
| S025I | V034A | Y059C | S068D | K075E | F106W |
| S025M | V034C | Y059D | S068E | K075F | F106Y |
| S025P | V034I | Y059E | S068F | K075M | A107K |
| S025Q | V034M | Y059G | S068G | K075Q | A107H |

(continued)

| Table 5. Exemplary heavy chain amino acid replacements | | | | | |
|---|---|---|---|---|---|
| S025T | V034P | Y059I | S068I | K075T | A107R |
| S025V | V034L | Y059P | S068N | K075V | A107L |
| S025L | H035I | Y059Q | S068Q | K075W | A107C |
| G026H | H035Q | Y059S | S068T | K075Y | A107D |
| G026R | W036K | Y059T | S068V | K075G | A107E |
| G026D | W036A | Y059V | I069A | K075P | A107G |
| G026F | W036I | Y059W | I069C | S076H | A107N |
| G026M | W036V | N060K | I069G | S076R | A107S |
| G026N | W036Y | N060A | I069Y | S076L | A107T |
| G026P | V050K | N060C | N070H | S076A | A107Y |
| G026Q | V050H | N060D | N070R | S076C | Y108K |
| G026S | V050A | N060F | N070L | S076D | Y108H |
| G026Y | V050D | N060G | N070D | S076E | Y108R |
| G026L | V050E | N060P | N070E | S076F | Y108L |
| F027H | V050G | N060Q | N070F | S076M | Y108C |
| F027R | V050I | N060S | N070G | S076P | Y108F |
| F027A | V050N | N060T | N070I | S076Q | Y108I |
| F027D | V050Q | N060Y | N070P | S076T | Y108N |
| F027E | V050T | T061N | N070Q | S076Y | Y108S |
| F027G | V050L | T061Q | N070S | S076I | Y108T |
| F027M | I051K | P062G | N070T | S076V | Y108V |
| F027P | I051H | F063H | N070V | Q077H | Y108W |
| F027Q | I051A | F063R | N070Y | Q077R | W109I |
| F027S | I051C | F063L | K071H | Q077L | W109M |
| F027T | I051E | F063A | K071R | Q077A | W109Y |
| F027V | I051G | F063C | K071L | Q077E | G110R |
| F027W | I051N | F063D | K071A | Q077G | G110A |
| F027Y | I051Q | F063G | K071C | Q077I | G110M |
| F027L | I051 S | F063M | K071F | Q077M | G110P |
| S028K | I05IV | F063N | K071G | Q077N | G110T |
| S028H | I051Y | F063Q | K071Q | Q077S | Q111K |
| S028R | I051L | F063S | K071S | Q077V | Q111H |
| S028A | W052I | F063V | K071T | Q077W | Q111R |
| S028D | W052N | F063P | K071V | Q077Y | QIIIL |
| S028I | W052Y | T064R | K071W | Y093H | QUID |
| S028M | S053H | T064L | K071Y | Y093V | Q111E |
| S028P | S053R | T064C | D072K | Y093W | QIIIG |
| S028Q | S053A | T064F | D072H | Y094R | Q111M |

(continued)

| Table 5. Exemplary heavy chain amino acid replacements | | | | | |
|---|---|---|---|---|---|
| S028V | S053C | T064G | D072R | Y094L | QUIP |
| S028W | S053G | T064N | D072L | R097H | QIIIS |
| S028L | S053I | T064Q | D072A | R097W | QUIT |
| S028C | S053M | T064V | D072G | A098P | QIIIW |
| L029K | S053P | S065H | D072I | L099N | Q111Y |
| L029H | S053Q | S065R | D072M | L099W | QIIIV |
| L029A | S053L | S065L | D072N | T100H | Q111I |
| L029D | S053T | S065C | D072Q | T100L | G112A |
| L029G | S053V | S065E | D072S | T100A | G112N |
| L029I | S053Y | S065F | D072V | T100D | G112P |
| L029M | G054H | S065G | D072W | T100I | G112S |
| L029N | G054R | S065I | D072Y | T100N | G112T |
| L029S | G054A | S065M | D072P | T100P | G112Y |
| L029V | G054C | S065N | N073H | T100Q | |

Exemplary of modified anti-EGFR antibodies containing modifications in a variable heavy chain, or portion thereof, provided herein are those that exhibit conditional activity in a tumor environment as described herein above. For example, exemplary antibodies provided herein include those that bind to EGFR (particularly human EGFR) with a higher binding activity under conditions that exist in a tumor microenvironment that include one or both of pH between or about between pH 5.6 to 6.8 or lactate concentration of between or about between 5 mM to 20 mM (e.g. pH 6.0 and/or 16.6 mM lactate) and 10 mg/mL to 50 mg/mL protein (e.g. 20% to 50% human serum), compared to under conditions that exist in a non-tumor microenvironment that include one or both of pH between or about between pH 7.0 to 7.8 or lactate concentration between or about between 0.5 mM to 5 mM (e.g. pH 7.4 and/or 1 mM lactate) and 10 mg/mL to 50 mg/mL protein (e.g. 20% to 50% human serum) of greater than 1.0, such as greater than 1.2, 1.3, 1.4, 1.5, 1.6, 1.7, 1.8, 1.9, 2.0, 3.0, 4.0, 5.0 or greater as described herein above.

For example, exemplary modified anti-EGFR antibodies that are conditionally active as described herein contain a variable heavy chain having one or more amino acid replacements at a position or positions corresponding to 24, 25, 27, 28, 29, 30, 31, 32, 50, 53, 54, 58, 59, 63, 64, 67, 68, 72, 73, 74, 75, 76, 77, 97, 100, 101, 104, 107, 111 with reference to the heavy chain amino acid positions set forth in any of SEQ ID NO:3. For example, the amino acid positions can be replacements at positions corresponding to replacement of Valine (V) at position 24 (V24), S25, F27, S28, L29, T30, N31, Y32, V50, S53, G54, D58, Y59, F63, T64, L67, S68, D72, N73, S74, K75, S76, Q77, R97, T100, Y101, Y104, A107, Q111 with reference to the amino acid positions set forth in any of SEQ ID NO:3. For example, exemplary anti-EGFR antibodies provided herein contain one or more amino acid replacements corresponding to heavy chain replacement or replacements V24I, V24L, V24E, S25C, S25G, S25I, S25M, S25V, S25Q, S25T, S25L, S25H, S25R, S25A, S25D, F27R, S28C, L29H, T30F, N31H, N31I, N31T, N31V, Y32T, V50L, S53G, G54D, G54S, G54R, G54C, G54P, D58M, Y59E, F63R, F63C, F63G, F63M, F63V, F63P, F63S, T64N, T64V, L67G, S68F, S68Q, D72K, D72L, D72P, D72M, D72W, N73Q, S74H, S74R, S74D, S74G, S74Y, K75H, K75G, K75W, K75P, S76I, S76V, Q77R, Q77E, R97H, T100I, T100P, Y101W, Y104D, Y104F, Y104S, Y105V, A107N, Q111I, Q111P, Q111V.

In particular examples, exemplary modifications provided herein include modification of a heavy chain of an anti-EGFR antibody at positions corresponding to positions 24, 25, 27, 30, 53, 72, 97, 104 and 111 with reference to the amino acid positions set forth in SEQ ID NO:3. For example, the amino acid positions can be replacements at positions corresponding to replacement of Valine (V) at position 24 (V24), S25, F27, T30, S53, D72, R97, Y104 or Q111 with reference to the amino acid positions set forth in SEQ ID NO:3. Exemplary of amino acid replacements in the modified anti-EGFR antibodies provided herein, include but are not limited to, replacement of a heavy chain residue with: glutamic acid (E) at a position corresponding to 24; C at a position corresponding to 25; V at a position corresponding to position 25; R at a position corresponding to position 27; F at a position corresponding to position 30; G at a position corresponding to position 53; L at a position corresponding to position 72; H at a position corresponding to position 97; D at a position corresponding to 104 or P at a position corresponding to 111. For example, the anti-EGFR antibodies provided herein contain one or more amino acid replacements corresponding to heavy chain replacements of V24E, S25C, S25V, F27R, T30F, S53G, D72L, R97H, Y104D or Q111P with reference to the sequence of amino acids set forth in SEQ ID NO:3. The anti-EGFR antibody, or antigen-binding fragment thereof, can contain only a single amino acid replacement in the variable heavy chain. Typically, the anti-EGFR antibody, or antigen-binding fragment thereof, contains at least two or more of the above amino acid replacements in the variable heavy chain, such as at least 2, 3, 4, 5, 6, 7, 8 or 9 amino acid replacements from among V24E, S25C, S25V, F27R, T30F, S53G, D72L, R97H, Y104D or Q111P with reference to the sequence of amino acids set forth in SEQ ID NO:3. The anti-EGFR, or antigen-binding fragments thereof, can contain additional modifications in the heavy chain, for example as described below in subsection 3, or as a result of humanization of the antibody as described herein. In particular, provided herein is a modified anti-EGFR antibody, or antigen-binding fragment thereof that contains an amino acid replacement of heavy chain residue Y104, such as amino acid replacement Y104D, Y104F or Y104S.

Non-limiting amino acid replacements in a heavy chain are set forth in Table 6 with reference to numbering set forth in SEQ ID NO:3. An exemplary SEQ ID NO of a variable heavy chain containing the amino acid replacement is set forth. For any of the amino acid replacements in a variable heavy chain provided herein above, it is understood that the replacements can be made in the corresponding position in another anti-EGFR antibody by alignment therewith with the sequence set forth in SEQ ID NO:3 (*see e.g.* Figure 2), whereby the corresponding position is the aligned position. Hence, the antibody can contain a heavy chain constant region, or portion thereof. In particular examples, the amino acid replacement(s) can be at the corresponding position in a cetuximab heavy chain, or portion thereof, such as set forth in any of SEQ ID NOS:1, 3, 5, 8 or 28 or a variant thereof having at least 75%, 80%, 81%, 82%, 83%, 84%, 85%, 86%, 86%, 88%, 89%, 90%, 91%, 92%, 93%, 94%, 95%, 96%, 97%, 98%, 99% or more sequence identity thereto, so long as the resulting modified antibody containing the modified variable heavy chain, or portion thereof, exhibits a ratio of binding activity under conditions that exist in a tumor microenvironment that include one or both of pH between or about between pH 5.6 to 6.8 or lactate concentration of between or about between 5 mM to 20 mM (e.g. pH 6.0 and/or 16.6 mM lactate) and 10 mg/mL to 50 mg/mL protein (e.g. 20% to 50% human serum), compared to under conditions that exist in a non-tumor microenvironment that include one or both of pH between or about between pH 7.0 to 7.8 or lactate concentration between or about between 0.5 mM to 5 mM (e.g. pH 7.4 and/or 1 mM lactate) and 10 mg/mL to 50 mg/mL protein (e.g. 20% to 50% human serum) of greater than 1.0 as described herein above.

| Table 6: Exemplary Heavy Chain Amino Acid Replacements | |
|---|---|
| **Amino Acid Replacements** | **SEQ ID NO** |
| HC-Y104D/HC-QIIIP | 1062 |
| HC-S25C/HC-Y104D | 1112 |
| HC-S53G/HC-Y104D | 1114 |
| HC-S53G/HC-Y104D/HC-Q111P | 1115 |
| HC-S25V/HC-Y104D | 1116 |
| HC-S25V/HC-Y104D/HC-Q111P | 1117 |
| HC-S25V/HC-S53G/HC-Y104D | 1118 |
| HC-S25V/HC-S53G/HC-Y104D/HC-Q11 1P | 1119 |
| HC-T30F/HC-Y104D | 1124 |
| HC-T30F/HC-Y104D/HC-Q111P | 1125 |
| HC-T30F/HC-S53G/HC-Y104D | 1126 |
| HC-T30F/HC-S53G/HC-Y104D/HC-Q111P | 1127 |
| HC-D72L/HC-Y104D | 1128 |
| HC-D72L/HC-Y104D/HC-Q111P | 1129 |
| HC-S53G/HC-D72L/HC-Y104D | 1130 |
| HC-S53G/HC-D72L/HC-Y104D/HC-Q111P | 1131 |
| HC-S25C/ HC-Q111P | 1113 |
| HC-V24E/ HC-F27R/ HC-R97H/ HC-Q111P | 1093 |

**b. Light Chain Modifications**

Provided herein are modified anti-EGFR antibodies containing a modification(s), such as amino acid replacement, in a variable light chain of a cetuximab antibody, antigen-binding fragment thereof or variant thereof, corresponding to amino acid residue(s) in a cetuximab antibody containing a variable light chain set forth in SEQ ID NO:4. The resulting modification(s) can be in a light chain set forth in SEQ ID NO: 2, 4, 9, 10 or 29, or in a variant thereof, having at least 75%, 80%, 81%, 82%, 83%, 84%, 85%, 86%, 86%, 88%, 89%, 90%, 91%, 92%, 93%, 94%, 95%, 96%, 97%, 98%, 99% or more sequence identity thereto. The modifications can be in a complementarity determining region (CDR) or in a framework region.

For example, provided herein are modified anti-EGFR antibodies containing a at least one amino acid replacement or substitution in the variable light chain, or a portion thereof, at any of positions corresponding to 1, 2, 3, 4, 5, 24, 25, 26, 27, 28, 29, 30, 31, 32, 33, 48, 49, 51, 52, 53, 54, 55, 56, 86, 87, 89, 91, 92, 93, 96, 97, 98, 99 or 100 with reference to the amino acid positions set forth in SEQ ID NO:4. For example, the amino acid positions can be replacements at positions corresponding to replacement of Aspartic Acid (D) at position 1 (D1), 12, L3, L4, T5, R24, A25, S26, Q27, S28, I29, G30, T31, N32, I33, I48, K49, A51, S52, E53, S54, I55, S56, Y86, Y87, Q89, N91, N92, N93, T96, T97, F98, G99 or A100 with reference to the amino acid positions set forth in SEQ ID NO:4. With respect to Kabat numbering, exemplary positions in the light chain that can be modified, for example by amino acid replacement or substitution, include, but are not limited to, any of positions corresponding to positions 1, 2, 3, 4, 5, 24, 25, 26, 27, 28, 29, 30, 31, 32, 33, 48, 49, 51, 52, 53, 54, 55, 56, 86, 87, 89, 91, 92, 93, 96, 97, 98, 99 or 100. In some examples, the amino acid residue that is modified (e.g. replaced) at the position corresponding to any of the above positions is a conservative residue or a semi-conservative amino acid residue to the amino acid set forth in any of SEQ ID NOS:2, 4, 9, 10 or 29.

| Table 7. Exemplary light chain amino acid replacements | | | | | |
|---|---|---|---|---|---|
| D001W | R024M | G030A | K049V | Y087D | T097D |
| I002C | R024S | G030E | K049Y | Y087F | T097G |
| I002V | R024W | G030F | K049L | Y087G | T097Q |
| I002W | R024Y | G030I | K049H | Y087I | T097S |
| L003D | R024G | G030M | K049R | Y087N | T097V |
| L003F | A025C | G030P | A051T | Y087P | T097K |
| L003G | A025G | G030Q | A051L | Y087S | T097R |
| L003S | A025L | G030S | S052A | Y087T | F098A |
| L003T | A025V | G030V | S052C | Y087V | F098M |
| L003V | S026A | G030Y | S052D | Y087W | F098S |
| L003W | S026C | G030L | S052E | Y087K | F098V |
| L003Y | S026D | G030K | S052G | Y087H | F098Y |
| L003R | S026I | G030H | S052I | Y087R | G099L |
| L004C | S026M | G030R | S052M | Q089E | G099D |
| L004E | S026N | T031A | S052Q | N091L | G099E |
| L004F | S026V | T031F | S052V | N091A | G099F |
| L004I | S026W | T031G | S052W | N091C | G099I |
| L004P | S026L | T031M | S052R | N091I | G099M |
| L004S | S026G | T031S | S052K | N091M | G099N |
| L004T | S026H | T031V | E053G | N091S | G099S |
| L004V | S026R | T031W | S054M | N091T | G099T |
| L004W | Q027A | T031L | I055A | N091V | G099V |
| L004K | Q027D | T031K | I055F | N091H | G099K |
| L004H | Q027E | T031H | S056G | N091R | G099H |

(continued)

| Table 7. Exemplary light chain amino acid replacements | | | | | |
|---|---|---|---|---|---|
| L004R | Q027F | N032G | S056L | N092C | Q100C |
| T005A | Q027I | I033F | S056A | N092D | Q100D |
| T005C | Q027M | I033G | S056C | N092L | Q100E |
| T005D | Q027N | I033M | S056D | N092M | Q100F |
| T005E | Q027P | I033T | S056E | N092S | Q100I |
| T005F | Q027T | I033V | S056F | N092T | Q100M |
| T005G | S028A | I033H | S056N | N092V | Q100N |
| T005N | S028D | I048M | S056P | N092W | Q100P |
| T005S | S028N | 1048S | S056Q | N092Y | Q100T |
| T005W | S028Q | I048L | S056V | N092H | Q100V |
| T005L | S028L | I048K | S056W | N092K | Q100W |
| T005K | S028K | K049A | S056H | N092R | Q100Y |
| T005H | S028H | K049E | S056R | N093T | Q100K |
| T005R | I029A | K049F | S056K | T096L | Q100H |
| T005P | I029E | K049G | Y086F | T096C | Q100R |
| R024A | I029F | K049N | Y086M | T096M | |
| R024C | 1029S | K049Q | Y086H | T096V | |
| R024F | I029T | K049S | Y087L | T097L | |
| R024L | I029R | K049T | Y087C | T097A | |

In one example, provided herein are modified anti-EGFR antibodies containing a variable light chain having a modification in a CDR, such as, for example, CDRL1, CDRL2 or CDRL3. For example, provided herein are modified anti-EGFR antibodies containing one or more amino acid replacements in a light chain CDR1 at any of positions corresponding to positions 24, 25, 26, 27, 28, 29, 30, 31, 32 or 33 with reference to the amino acid positions set forth in SEQ ID NO:4; CDR2 at any of positions corresponding to positions 51, 52, 53, 54, 55 or 56 with reference to the amino acid positions set forth in SEQ ID NO:4; CDR3 at any of positions corresponding to positions 89, 91, 92, 93, 96 or 97 with reference to the amino acid positions set forth in SEQ ID NO:4.

In other examples, provided herein are modified anti-EGFR antibodies containing a variable light chain containing a modification in a framework region (FW), for example, light chain FW1, FW2, FW3 or FW4. For example, provided herein are modified anti-EGFR antibodies containing one or more amino acid replacements in a light chain FW1 at any of positions corresponding to positions 1, 2, 3, 4 or 5 with reference to the amino acid positions set forth in SEQ ID NO:4; FW2 at any of positions corresponding to positions 48 or 49 with reference to the amino acid positions set forth in SEQ ID NO:4; FW3 at any of positions corresponding to positions 86 or 87 with reference to the amino acid positions set forth in SEQ ID NO:4; and FW4 at any of positions corresponding to positions 98, 99 or 100 with reference to the amino acid positions set forth in SEQ ID NO:4.

Provided herein are modified anti-EGFR antibodies containing at least one amino acid replacement in a variable light chain, or portion thereof, corresponding to any set forth in Table 7 with reference to a position set forth in SEQ ID NO:4.

Exemplary of modified anti-EGFR antibodies containing modifications in a variable light chain, or portion thereof, provided herein are those that exhibit conditional activity in a tumor environment as described herein above. For example, exemplary antibodies provided herein include those that bind to EGFR (particularly human EGFR) with a higher binding activity under conditions that exist in a tumor microenvironment that include one or both of pH between or about between pH 5.6 to 6.8 or lactate concentration of between or about between 5 mM to 20 mM (e.g. pH 6.0 and/or 16.6 mM lactate) and 10 mg/mL to 50 mg/mL protein (e.g. 20% to 50% human serum), compared to under conditions that exist in a non-tumor microenvironment that include one or both of pH between or about between pH 7.0 to 7.8 or lactate concentration between or about between 0.5 mM to 5 mM (e.g. pH 7.4 and/or 1 mM lactate) and 10 mg/mL to 50 mg/mL protein (e.g. 20% to 50% human serum) of greater than 1.0, such as greater than 1.2, 1.3, 1.4, 1.5, 1.6, 1.7, 1.8, 1.9, 2.0, 3.0, 4.0, 5.0 or greater as described herein above.

For example, exemplary modified anti-EGFR antibodies that are conditionally active as described herein contain a variable light chain having one or more amino acid replacements at a position or positions corresponding to 4, 5, 24, 29, 56 or 91 with reference to the light chain amino acid positions set forth in any of SEQ ID NO:4. For example, the amino acid positions can be replacements at positions corresponding to replacement of Leucine (L) at position 4 (L4), T5, R24, I29, S56 or N91 with reference to the amino acid positions set forth in SEQ ID NO:4. For example, exemplary anti-EGFR antibodies provided herein contain one or more amino acid replacements corresponding to light chain replacement or replacements L4C, L4F, L4V, T5P, R24G, I29S, S56H or N91V. The anti-EGFR antibody, or antigen-binding fragment thereof, can contain only a single amino acid replacement in the variable light chain. Typically, the anti-EGFR antibody, or antigen-binding fragment thereof, contains at least two or more of the above amino acid replacements in the variable light chain, such as at least 2, 3, 4, 5 or 6 amino acid replacements from among L4C, L4F, L4V, T5P, R24G, I29S, S56H or N91V with reference to the sequence of amino acids set forth in SEQ ID NO:4. The anti-EGFR, or antigen-binding fragments thereof, can contain additional modification in the light chain, for example as described below in subsection 3, or as a result of humanization of the antibody as described herein.

In particular examples, exemplary modifications provided herein include modification of a light chain of an anti-EGFR antibody at position corresponding to positions 29 with reference to the amino acid positions set forth in SEQ ID NO:4. For example, the amino acid positions can be replacements at positions corresponding to replacement of Isoleucine (I) at position 29 (I29) with reference to the amino acid positions set forth in SEQ ID NO:4. Exemplary of amino acid replacements in the modified anti-EGFR antibodies provided herein, include but are not limited to, replacement of a light chain residue with: serine (S) at a position corresponding to 29. For example, the anti-EGFR antibodies provided herein contain an amino acid replacement corresponding to light chain replacement of I29S in a sequence of amino acids set forth in SEQ ID NO:4.

Any of the modification(s) in a heavy chain as described above and any of the modification(s) in a light chain as described herein can be combined in an anti-EGFR antibody, or antigen-binding fragment thereof. Non-limiting examples of such modification(s) include HC-Y104D/LC-I29S; HC-Y104D/HC-Q111P/LC-I29S; HC-S25C/LC-I29S; or HC-Q111P/LC-I29S.

For any of the amino acid replacements in a variable light chain provided herein above, it is understood that the replacements can be made in the corresponding position in another anti-EGFR antibody by alignment therewith with the sequence set forth in SEQ ID NO:4 (*see e.g.* Figure 2), whereby the corresponding position is the aligned position. In particular examples, the amino acid replacement(s) can be at the corresponding position in a cetuximab light chain set forth in any of SEQ ID NOS:2, 4, 9, 10 or 29, or a variant thereof having at least 75%, 80%, 81%, 82%, 83%, 84%, 85%, 86%, 86%, 88%, 89%, 90%, 91%, 92%, 93%, 94%, 95%, 96%, 97%, 98%, 99% or more sequence identity thereto, so long as the resulting modified antibody containing the modified variable light chain exhibits a ratio of binding activity under conditions that exist in a tumor microenvironment that include one or both of pH between or about between pH 5.6 to 6.8 or lactate concentration of between or about between 5 mM to 20 mM (e.g. pH 6.0 and/or 16.6 mM lactate) and 10 mg/mL to 50 mg/mL protein (e.g. 20% to 50% human serum), compared to under conditions that exist in a non-tumor microenvironment that include one or both of pH between or about between pH 7.0 to 7.8 or lactate concentration between or about between 0.5 mM to 5 mM (e.g. pH 7.4 and/or 1 mM lactate) and 10 mg/mL to 50 mg/mL protein (e.g. 20% to 50% human serum) of greater than 1.0 as described herein above.

**c. Exemplary modified Anti-EGFR Antibodies and Fragments Thereof**

Modified anti-EGFR antibodies provided herein, such as any described herein above, minimally contain a modified variable heavy chain and/or modified variable light chain, or portion thereof sufficient to bind antigen when assembled into an antibody, as described herein above. Provided herein are modified anti-EGFR antibodies containing a modified variable heavy chain set forth in any of SEQ ID NOS:30-557, 1062-1064, 1093, 1098-1107 or 1112-1131, or a sequence that exhibits at least 75%, 80%, 81%, 82%, 83%, 84%, 85%, 86%, 86%, 88%, 89%, 90%, 91%, 92%, 93%, 94%, 95%, 96%, 97%, 98%, 99% or more sequence identity to any of SEQ ID NOS: 30-557, 1062-1064, 1093, 1098-1107 or 1112-1131; and a variable light chain set forth in SEQ ID NO:4 or 10. In other examples, provided herein are modified anti-EGFR antibodies containing a variable heavy chain set forth in SEQ ID NO: 3; and a variable light chain set forth in any of SEQ ID NOS:558-1061 or 1065-1068, or a sequence that exhibits at least 75%, 80%, 81%, 82%, 83%, 84%, 85%, 86%, 86%, 88%, 89%, 90%, 91%, 92%, 93%, 94%, 95%, 96%, 97%, 98%, 99% or more sequence identity to any of SEQ ID NOS: 558-1061 or 1065-1068.

In some examples, provided herein are modified anti-EGFR antibodies containing modifications in both the variable heavy chain and variable light chain, whereby the anti-EGFR antibody contains a modified variable heavy chain set forth in any of SEQ ID NOS: 30-557, 1062-1064, 1093, 1098-1107 or 1112-1131, or a sequence that at least 75%, 80%, 81%, 82%, 83%, 84%, 85%, 86%, 86%, 88%, 89%, 90%, 91%, 92%, 93%, 94%, 95%, 96%, 97%, 98%, 99% or more sequence identity to any of SEQ ID NOS: 30-557, 1062-1064, 1093, 1098-1107 or 1112-1131; and a variable light chain set forth in any of SEQ ID NOS:558-1061 or 1065-1068, or a sequence that exhibits at least 75%, 80%, 81%, 82%, 83%, 84%, 85%, 86%, 86%, 88%, 89%, 90%, 91%, 92%, 93%, 94%, 95%, 96%, 97%, 98%, 99% or more sequence identity to any of SEQ ID NOS:558-1061 or 1065-1068. In particular examples, provided herein is a modified anti-EGFR containing a modified variable heavy chain set forth in SEQ ID NO:495, or a sequence that exhibits at least 75%, 80%, 81%, 82%, 83%, 84%, 85%, 86%, 86%, 88%, 89%, 90%, 91%, 92%, 93%, 94%, 95%, 96%, 97%, 98%, 99% to SEQ ID NO:495 and a modified variable light chain set forth in SEQ ID NO:639 or SEQ ID NO: 891 or a sequence that exhibits at least 75%, 80%, 81%, 82%, 83%, 84%, 85%, 86%, 86%, 88%, 89%, 90%, 91%, 92%, 93%, 94%, 95%, 96%, 97%, 98%, 99% or more sequence identity to SEQ ID NO: 639 or 891 (designated HC-Y104D/LC-I29S). In another example, provided herein is a modified anti-EGFR containing a modified variable heavy chain set forth in SEQ ID NO:1062, or a sequence that exhibits at least 75%, 80%, 81%, 82%, 83%, 84%, 85%, 86%, 86%, 88%, 89%, 90%, 91%, 92%, 93%, 94%, 95%, 96%, 97%, 98%, 99% to SEQ ID NO:1062 and a modified variable light chain set forth in SEQ ID NO:639 or SEQ ID NO: 891 or a sequence that exhibits at least 75%, 80%, 81%, 82%, 83%, 84%, 85%, 86%, 86%, 88%, 89%, 90%, 91%, 92%, 93%, 94%, 95%, 96%, 97%, 98%, 99% or more sequence identity to SEQ ID NO: 639 or 891 (designated HC-Y104D/HC-Q111P/LC-I29S). In a further example, provided herein is a modified anti-EGFR containing a modified variable heavy chain set forth in SEQ ID NO:58, or a sequence that exhibits at least 75%, 80%, 81%, 82%, 83%, 84%, 85%, 86%, 86%, 88%, 89%, 90%, 91%, 92%, 93%, 94%, 95%, 96%, 97%, 98%, 99% to SEQ ID NO:58 and a modified variable light chain set forth in SEQ ID NO:639 or SEQ ID NO: 891 or a sequence that exhibits at least 75%, 80%, 81%, 82%, 83%, 84%, 85%, 86%, 86%, 88%, 89%, 90%, 91%, 92%, 93%, 94%, 95%, 96%, 97%, 98%, 99% or more sequence identity to SEQ ID NO: 639 or 891 (designated HC-S25C/LC-I29S). In another example, provided herein is a modified anti-EGFR containing a modified variable heavy chain set forth in SEQ ID NO:547, or a sequence that exhibits at least 75%, 80%, 81%, 82%, 83%, 84%, 85%, 86%, 86%, 88%, 89%, 90%, 91%, 92%, 93%, 94%, 95%, 96%, 97%, 98%, 99% to SEQ ID NO:547 and a modified variable light chain set forth in SEQ ID NO:639 or SEQ ID NO: 891 or a sequence that exhibits at least 75%, 80%, 81%, 82%, 83%, 84%, 85%, 86%, 86%, 88%, 89%, 90%, 91%, 92%, 93%, 94%, 95%, 96%, 97%, 98%, 99% or more sequence identity to SEQ ID NO: 639 or 891 (designated HC-Q111P/LC-I29S).

In particular, provided herein is a modified anti-EGFR containing a variable heavy chain set forth in SEQ ID NOS:495, 1062, 1112, 1114-1119, 1124-1131 or a sequence that exhibits at least 75%, 80%, 81%, 82%, 83%, 84%, 85%, 86%, 86%, 88%, 89%, 90%, 91%, 92%, 93%, 94%, 95%, 96%, 97%, 98%, 99% or more sequence identity to any of SEQ ID NOS: 495, 1062, 1112, 1114-1119, 1124-1131; and a variable light chain set forth in SEQ ID NOS:4 or 10.

The antibodies provided herein can be full-length IgG1 antibodies, or other subtype from among IgG2, IgG3 or IgG4. For example, the anti-EGFR antibodies can be full-length IgG1 antibodies containing a kappa light chain constant region from cetuximab (set forth in SEQ ID NO:1071) or an IgG1 heavy chain constant region from cetuximab (set forth in SEQ ID NO: 1069). The heavy chain constant region also can be a human IgG1 heavy chain set forth in SEQ ID NO:22, from an Ig class, such as IgG2 (set forth in SEQ ID NO:23), IgG3 (set forth in SEQ ID NO:24) or IgG4 (set forth in SEQ ID NO:25), or can be a modified IgG1 heavy chain constant region set forth in SEQ ID NO:26, 27 or 1070. The light chain constant region also can be a human kappa light chain (set forth in SEQ ID NO: 1072) or a human lambda light chain (set forth in SEQ ID NO:1073).

For example, the heavy chain of modified anti-EGFR antibodies provided herein can contain a modified variable heavy chain described herein above and an IgG1 heavy chain set forth in SEQ ID NO:1069. In another example, the heavy chain of modified anti-EGFR antibodies provided herein can contain a modified variable heavy chain described herein above and an IgG1 heavy chain set forth in SEQ ID NO:22. In yet another example, the heavy chain of modified anti-EGFR antibodies provided herein can contain a modified variable heavy chain described herein above and an IgG1 heavy chain set forth in SEQ ID NO: 1070. In one example, the light chain of modified anti-EGFR antibodies provided herein can contain a modified variable light chain described herein above and a kappa light chain set forth in SEQ ID NO:1071. In another example, the light chain of modified anti-EGFR antibodies provided herein can contain a modified variable light chain described herein above and a kappa light chain set forth in SEQ ID NO:1072.

For example, provided herein are modified anti-EGFR antibodies containing a variable heavy chain set forth in any of SEQ ID NOS: 30-557, 1062-1064, 1093, 1098-1107 or 1112-1131, or a sequence that exhibits at least 75%, 80%, 81%, 82%, 83%, 84%, 85%, 86%, 86%, 88%, 89%, 90%, 91%, 92%, 93%, 94%, 95%, 96%, 97%, 98%, 99% or more sequence identity to any of SEQ ID NOS: 30-557, 1062-1064, 1093, 1098-1107 or 1112-1131, further containing a sequence of amino acids corresponding to an IgG1 constant region set forth in any of SEQ ID NOS:22, 1069 or 1070; and a light chain set forth in SEQ ID NO:2 or 9. In some examples, provided herein are modified anti-EGFR antibodies containing a variable heavy chain set forth in any of SEQ ID NOS: 1 or 8; and a light chain set forth in any of SEQ ID NOS:558-1061 or 1065-1068, or a sequence that exhibits at least 75%, 80%, 81%, 82%, 83%, 84%, 85%, 86%, 86%, 88%, 89%, 90%, 91%, 92%, 93%, 94%, 95%, 96%, 97%, 98%, 99% or more sequence identity to any of SEQ ID NOS: 558-1061 or 1065-1068, further containing a sequence of amino acids corresponding to a kappa light chain constant region set forth in SEQ ID NO:1071 or 1072.

Modified anti-EGFR antibodies provided herein also include antibody fragments, which are derivatives of full-length antibodies that contain less than the full sequence of the full-length antibodies but retain at least a portion of the specific binding abilities of the full-length antibody, for example the variable portions of the heavy and light chain. The antibody fragments also can include antigen-binding portions of an antibody that can be inserted into an antibody framework (e.g., chimeric antibodies) in order to retain the binding affinity of the parent antibody. Examples of antibody fragments include, but are not limited to, Fab, Fab', F(ab')$_2$, single-chain Fv (scFv), Fv, dsFv, diabody, Fd and Fd' fragments, and other fragments, including modified fragments (see, for example, Methods in Molecular Biology, Vol. 207: Recombinant Antibodies for Cancer Therapy Methods and Protocols (2003); Chapter 1; p 3-25, Kipriyanov). Antibody fragments can include multiple chains linked together, such as by disulfide bridges and can be produced recombinantly. Antibody fragments also can contain synthetic linkers, such as peptide linkers, to link two or more domains. Methods for generating antigen-binding fragments are well-known known in the art and can be used to modify any antibody provided herein. Fragments of antibody molecules can be generated, such as for example, by enzymatic cleavage. For example, upon protease cleavage by papain, a dimer of the heavy chain constant regions, the Fc domain, is cleaved from the two Fab regions (*i.e.* the portions containing the variable regions).

Single chain antibodies can be recombinantly engineered by joining a heavy chain variable region ($V_H$) and light chain variable region ($V_L$) of a specific antibody. The particular nucleic acid sequences for the variable regions can be cloned by standard molecular biology methods, such as, for example, by polymerase chain reaction (PCR) and other recombination nucleic acid technologies. Methods for producing scFvs are described, for example, by Whitlow and Filpula (1991) Methods, 2: 97-105; Bird et al. (1988) Science 242:423-426; Pack et al. (1993) Bio/Technology 11:1271-77; and U.S. Patent Nos. 4,946,778, 5,840,300, 5,667,988, 5,658,727, 5,258,498).

Fragments of modified anti-EGFR antibodies provided herein, such as any described herein above, minimally contain a modified variable heavy chain and/or modified variable light chain as described herein above. Also provided are antigen-binding fragments of any of the above antibodies containing a modified variable heavy chain set forth in any of SEQ ID NOS: 30-557, 1062-1064, 1093, 1098-1107 or 1112-1131, and/or a modified variable light chain set forth in any of SEQ ID NOS:558-1061 or 1065-1068, or variable chains having a sequence identity of at least 75%, 80%, 81%, 82%, 83%, 84%, 85%, 86%, 86%, 88%, 89%, 90%, 91%, 92%, 93%, 94%, 95%, 96%, 97%, 98%, 99% or more to any of SEQ ID NOS: 30-1068, 1093 or 1098-1131. For example, examples of antibody fragments include, but are not limited to, Fab, Fab', F(ab')$_2$, single-chain Fv (scFv), Fv, dsFv, diabody, Fd and Fd' fragments.

For example, such anti-EGFR antibodies can be Fab fragments further containing a heavy chain $C_H1$ constant region from cetuximab (set forth in SEQ ID NO:11) or a kappa light chain constant region from cetuximab (set forth in SEQ ID NO:1071). The heavy chain $C_H1$ constant region can also be a human IgG1 $C_H1$ constant region set forth in SEQ ID NO:1108. In one example, the heavy chain of modified anti-EGFR antibodies provided herein can contain a modified variable heavy chain described herein above, such as any set forth in SEQ ID NOS: 30-557, 1062-1064, 1093, 1098-1107 or 1112-1131, and a $C_H1$ heavy chain domain set forth in SEQ ID NO:11. In another example, the heavy chain of modified anti-EGFR antibodies provided herein can contain a modified variable heavy chain described herein above, such as any set forth in SEQ ID NOS: 30-557, 1062-1064, 1093, 1098-1107 or 1112-1131, and a $C_H1$ heavy chain domain set forth in SEQ ID NO:1108. In one example, the light chain of modified anti-EGFR antibodies provided herein can contain a modified variable light chain described herein above, such as any set forth in SEQ ID NOS:558-1061 or 1065-1068, and a kappa light chain set forth in SEQ ID NO:1071. In another example, the light chain of modified anti-EGFR antibodies provided herein can contain a modified variable light chain described herein above, such as any set forth in SEQ ID NOS:558-1061 or 1065-1068, and a kappa light chain set forth in SEQ ID NO:1072.

In particular examples, the modified anti-EGFR antibody is a single chain antibody. A single chain antibody can be generated from the antigen-binding domain of any of the anti-EGFR antibodies provided herein. Methods for generating single chain antibodies using recombinant techniques are known in the art, such as those described in, for example, Marasco et al. (1993) Proc. Natl. Acad. Sci. USA 90:7889-7893, Whitlow and Filpula (1991) Methods, 2: 97-105; Bird et al. (1988) Science 242:423-426; Pack et al. (1993) Bio/Technology 11:1271-77; and U.S. Patent Nos. 4,946,778, 5,840,300, 5,667,988, 5,658,727.

A single chain antibody can contain a light chain variable ($V_L$) domain or functional region thereof and a heavy chain variable ($V_H$) domain or functional region thereof of any anti-EGFR antibody or antigen-binding fragment thereof provided herein. In some examples, the $V_L$ domain or functional region thereof of the single chain antibody contains a complementarity determining region 1 (CDR1), a complementarity determining region 2 (CDR2) and/or a complementarity determining region 3 (CDR3) of an anti-EGFR antibody or antigen-binding fragment thereof provided herein. In some examples, the $V_H$ domain or functional region thereof of the single chain antibody contains a complementarity determining region 1 (CDR1), a complementarity determining region 2 (CDR2) and a complementarity determining region 3 (CDR3) of any anti-EGFR antibody or antigen-binding fragment thereof provided herein. In some examples, the single chain antibody further contains a peptide linker. In such examples, a peptide linker can be located between the light chain variable domain ($V_L$) and the heavy chain variable domain ($V_H$).

The single chain antibody can contain a peptide spacer, or linker, between the one or more domains of the antibody. For example, the light chain variable domain ($V_L$) of an antibody can be coupled to a heavy chain variable domain ($V_H$) via a flexible linker peptide. Various peptide linkers are well-known in the art and can be employed in the provided methods. A peptide linker can include a series of glycine residues (Gly) or Serine (Ser) residues. Exemplary of polypeptide linkers are peptides having the amino acid sequences $(Gly-Ser)_n$, $(Gly_mSer)_n$ or $(Ser_mGly)_n$, in which m is 1 to 6, generally 1 to 4, and typically 2 to 4, and n is 1 to 30, or 1 to 10, and typically 1 to 4, with some glutamic acid (Glu) or lysine (Lys) residues dispersed throughout to increase solubility (see, e.g., International PCT application No. WO 96/06641, which provides exemplary linkers for use in conjugates). Exemplary peptide linkers include, but are not limited to peptides having the sequence $(Gly_4Ser)_3$ (SEQ ID NO:21), GGSSRSSSSGGGGSGGGG (SEQ ID NO: 1074), GSGRSGGGGSGGGGS (SEQ ID NO: 1075), EGKSSGSGSESKST (SEQ ID NO: 1076), EGKSSGSGSESKSTQ (SEQ ID NO: 1077), EGKSSGSGSESKVD (SEQ ID NO: 1078), GSTSGSGKSSEGKG (SEQ ID NO: 1079), KESGSVSSEQLAQFRSLD (SEQ ID NO: 1080), and ESGSVSSEELAFRSLD (SEQ ID NO: 1081). Generally, the linker peptides are approximately 1-50 amino acids in length. The linkers used herein also can increase intracellular availability, serum stability, specificity and solubility or provide increased flexibility or relieve steric hindrance. Linking moieties are described, for example, in Huston et al. (1988) Proc Natl Acad Sci USA 85:5879-5883, Whitlow et al. (1993) Protein Engineering 6:989-995, and Newton et al., (1996) Biochemistry 35:545-553. Other suitable peptide linkers include any of those described in U.S. Patent No. 4,751,180 or 4,935,233, which are hereby incorporated by reference.

## 2. Humanized Anti-EGFR Antibodies

Provided herein are human or humanized anti-EGFR antibodies. For example, any known anti-EGFR antibody, or antigen-binding fragment thereof, such as any modified anti-EGFR containing a modified heavy chain and/or modified light chain as provided in subsection 1 above, can be humanized. Methods of humanization are well known to the skilled artisan. Antibody humanization can be used to evolve mouse or other non-human antibodies into human antibodies. The resulting antibody contains an increased in human sequence and decrease to no mouse or non-human antibody sequence, while maintaining similar binding affinity and specificity as the starting antibody.

Methods for engineering or humanizing non-human or human antibodies can be used and are well known in the art. Generally, a humanized or engineered antibody has one or more amino acid residues from a source which is non-human, e.g., but not limited to mouse, rat, rabbit, non-human primate or other mammal. The human amino acid residues are imported thereto, and hence are often referred to as "import" residues, which are typically taken from an "import" variable, constant or other domain of a known human sequence. Known human Ig sequences are disclosed, e.g., ncbi.nlm.nih.gov/entrez/query.fcgi; atcc.org/phage/hdb.html; sciquest.com/; www.abcam.com/; antibodyresource.com/onlinecomp.html; public.iastate.edu/.about.pedro/research_tools.html; mgen.uni-heidelberg.de/SD/IT/IT.html; whfreeman.com/immunology/CH05/kuby05.htm; library.thinkquest.org/12429/Immune/Antibody.html; hhmi.org/grants/lectures/1996/vlab/; path.cam.ac.uk/.about.mrc7/mikeimages.html; antibodyresource.com/; mcb.harvard.edu/BioLinks/Immunology.html. immunologylink.com/; pathbox.wustl.edu/.about.hcenter/index.html; biotech.ufl.edu/.about.hcl/; www.pebio.com/pa/340913/340913.html; nal.usda.gov/awic/pubs/antibody/; m.ehime-u.ac.jp/.about.yasuhito/Elisa.html; biodesign.com/table.asp; icnet.uk/axp/facs/davies/links.html; biotech.ufl.edu/.about.fccl/protocol.html; isac-net.org/sites_geo.html; aximt1.imt.uni-marburg.de/.about.rek/AEPStart.html; baserv.uci.kun.nl/.about.jraats/links1 .html; recab.uni-hd.de/immuno.bme.nwvu.edu/; mrc-cpe.cam.ac.uk/imt-doc/public/INTRO.html; ibt.unam.mx/vir/V_mice.html; imgt.cnusc.fr:8104/; biochem.ucl.ac.uk/.about.martin/abs/index.html; antibody.bath.ac.uk/; abgen.cvm.tamu.edu/lab/wwwabgen.html; unizh.ch/.about.honegger/AHOseminar/Slide01.html; www.cryst.bbk.ac.uk/.about.ubcg07s/; nimr.mrc.ac.uk/CC/ccaewg/ccaewg.htm; path.cam.ac.uk/.about.mrc7/humanisation/TAHHP.html; ibt.unam.mx/vir/structure/stat_aim.html; biosci.missouri.edu/smithgp/index.html; cryst.bioc.cam.ac.uk/.about.fmolina/Web-pages/Pept/spottech.html; jerini.de/fr_products.htm; patents.ibm.con/ibm.html. Kabat et al. Sequences of Proteins of Immunological Interest, U.S. Dept. Health (1983).

Such imported sequences can be used to reduce immunogenicity or reduce, enhance or modify binding, affinity, on-rate, off-rate, avidity, specificity, half-life, or any other suitable characteristic, as known in the art. Generally part or all of the non-human or human CDR sequences are maintained while the non-human sequences of the variable and constant regions are replaced with human or other amino acids. Antibodies can also optionally be humanized with retention of high affinity for the antigen and other favorable biological properties. To achieve this goal, humanized antibodies can be optionally prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the consensus and import sequences so that the desired antibody characteristic, such as increased affinity for the target antigen(s), is achieved. In general, the CDR residues are directly and most substantially involved in influencing antigen binding. Humanization or engineering of antibodies of the present invention can be performed using any known method, such as but not limited to those described in, Winter (Jones et al., Nature 321:522 (1986); Riechmann et al., Nature 332:323 (1988); Verhoeyen et al., Science 239:1534 (1988)), Sims et al., J. Immunol. 151: 2296 (1993); Chothia and Lesk, J. Mol. Biol. 196:901 (1987), Carter et al., Proc. Natl. Acad. Sci. U.S.A. 89:4285 (1992); Presta et al., J. Immunol. 151:2623 (1993), U.S. Pat. Nos. 5,723,323, 5,976,862, 5,824,514, 5,817,483, 5,814,476, 5,763,192, 5,723,323, 5,766,886, 5,714,352, 6,204,023, 6,180,370, 5,693,762, 5,530,101, 5,585,089, 5,225,539; 4,816,567, PCT/: US98/16280, US96/18978, US91/09630, US91/05939, US94/01234, GB89/01344, GB91/01134, GB92/01755; WO90/14443, WO90/14424, WO90/14430, EP 229246, each entirely incorporated herein by reference, including references cited therein.

Typically, the starting reference or parental antibody, generally one that is partially non-human, that is humanized herein is one that has a ratio of binding activity under conditions that exist in a tumor microenvironment that include one or both of pH between or about between pH 5.6 to 6.8 or lactate concentration of between or about between 5 mM to 20 mM (e.g. pH 6.0 and/or 16.6 mM lactate) and 10 mg/mL to 50 mg/mL protein (e.g. 20% to 50% human serum), compared to under conditions that exist in a non-tumor microenvironment that include one or both of pH between or about between pH 7.0 to 7.8 or lactate concentration between or about between 0.5 mM to 5 mM (e.g. pH 7.4 and/or 1 mM lactate) and 10 mg/mL to 50 mg/mL protein (e.g. 20% to 50% human serum) of greater than 1.0 as described herein above, such as generally at least greater than 2.0, 3.0, 4.0, 5.0, 6.0, 7.0, 7.0, 8.0, 9.0, 10.0, 15.0, 20.0, 25.0, 30.0, 35.0, 40.0, 50.0 or more. Exemplary of such antibodies are any that contain a variable heavy chain set forth in SEQ ID NOS:495, 1062, 1112, 1114-1119, 1124-1131 or a sequence that exhibits at least 75%, 80%, 81%, 82%, 83%, 84%, 85%, 86%, 86%, 88%, 89%, 90%, 91%, 92%, 93%, 94%, 95%, 96%, 97%, 98%, 99% or more sequence identity to any of SEQ ID NOS: 495, 1062, 1112, 1114-1119, 1124-1131; and a variable light chain set forth in SEQ ID NOS:4 or 10.

For example, antibody humanization can be performed by, for example, synthesizing a combinatorial library containing the six CDRs of a target antibody to be humanized (e.g. any of the antibodies set forth above) fused in frame to a pool of individual human frameworks. For example, the CDRs can be derived from any one or more of the CDRH1 (amino acid residues 26-35, according to AbM definition, or amino acid residues 31-35, according to Kabat definition), CDRH2 (amino acid residues 50-65) or CDRH3 (amino acid residues 95-102) set forth in any of SEQ ID NOS: 495, 1062, 1112, 1114-1119, 1124-1131 and/or can be derived from any one or more of the CDRL1 (amino acid residues 24-34), CDRL2 (amino acid residues 50-56) or CDRL3 (amino acid residues 89-97) set forth in any of SEQ ID NOS: 4 or 10. A human framework library that contains genes representative of all known heavy and light chain human germline genes can be utilized. The resulting combinatorial libraries can then be screened for binding to antigens of interest. This approach can allow for the selection of the most favorable combinations of fully human frameworks in terms of maintaining the binding activity to the parental antibody. Humanized antibodies can then be further optimized by a variety of techniques.

The number of amino acid substitutions or replacements a skilled artisan can make to effect humanization depends on many factors, including those described above. Generally speaking, the number of amino acid replacements (substitutions), insertions or deletions for an anti-EGFR antibody, fragment or variant will not be more than 40, 30, 20, 19, 18, 17, 16, 15, 14, 13, 12, 11, 10, 9, 8, 7, 6, 5, 4, 3, 2, 1, such as 1-30 or any range or value therein, as specified herein.

Amino acids in an anti-EGFR antibody that are essential for function can be identified by methods known in the art, such as site-directed mutagenesis or alanine-scanning mutagenesis (e.g., Ausubel, supra, Chapters 8, 15; Cunningham and Wells, Science 244:1081-1085 (1989)). The latter procedure introduces single alanine mutations at every residue in the molecule. The resulting mutant molecules are then tested for biological activity, such as, but not limited to binding to EGFR using any of the methods described herein. Sites that are critical for antibody binding can also be identified by structural analysis such as crystallization, nuclear magnetic resonance or photoaffinity labeling (Smith, et al., J. Mol. Biol. 224:899-904 (1992) and de Vos, et al., Science 255:306-312 (1992)).

A humanized clone provided herein includes any that exhibits at least 56% sequence identity, such as at least 57%, 58%, 59%, 60%, 61%, 62%, 63%, 64%, 65%, 66%, 67%, 68%, 69%, 70% or more sequence identity to its closest human $V_H$ gene segment germline sequence; and at least 75%, 76%, 77%, 78%, 79%, 80% or more sequence identity to its closest human $V_L$ gene segment germline sequence. The sequence of human germline segments are known and available to a skilled artisan. For example, gene segment sequences are accessible from known database (e.g., National Center for Biotechnology Information (NCBI), the international ImMunoGeneTics information system® (IMGT), the Kabat database and the Tomlinson's VBase database (Lefranc (2003) Nucleic Acids Res., 31:307-310; Martin et al., Bioinformatics Tools for Antibody Engineering in Handbook of Therapeutic Antibodies, Wiley-VCH (2007), pp. 104-107; see also published International PCT Application No. WO2010/054007). Further, databases are available that can be searched for closest germline sequences, such as IgBlast from the National Center for Biotechnology Information(NCBI; www.ncbi.nlm.nih.gov/igblast/), which is designed to analyze the V (variable) region of an Ig sequence. In performing such a search, the query sequence must contain some part of the V gene segment (e.g. residues 1-97 of the variable heavy chain; residues 1-95 of the variable light chain).

In one example, humanized clones provided herein are derived from an anti-EGFR antibody designated Y104D/Q111P (DP) having a variable heavy chain set forth in SEQ ID NO:1062 and a variable light chain set forth in SEQ ID NO:4 or 10. In another example, humanized clones provided herein are derived from an anti-EGFR antibody designated T30F/Y104D/Q111P (FDP) having a variable heavy chain set forth in SEQ ID NO: 1125 and a variable light chain set forth in SEQ ID NO: 4 or 10. Non-limiting examples of such humanized clones are set forth in Table 8. Tables 8-10 set forth the SEQ ID NO (SEQ) of the variable heavy and light chain of each clone. Tables 9 and 10 also summarize the sequence identity of the humanized clones to the variable sequence of the parental cetuximab and to its closest human V region germline sequences designated IGHV3-33(VH) and IGKV6-21 (VL) (*see e.g.* Nagdelaine-Beuzelin et al. (2007) Critical Reviews in Oncology/Hematology (2007) 64:210-225). The closest germline sequence of each clone as identified using IgBlast also is indicated in bold.

| Table 8: Anti-EGFR Antibody Clones | | | | |
|---|---|---|---|---|
| | variable heavy chain | | variable light chain | |
| Name | SEQ ID NO: (protein) | SEQ ID NO: (DNA) | SEQ ID NO: (protein) | SEQ ID NO: (DNA) |
| DP-h01 | 1134 | 1160 | 1138 | 1164 |
| DP-h02 | 1134 | 1160 | 1139 | 1165 |
| DP-h03 | 1135 | 1161 | 1138 | 1164 |
| DP-h04 | 1134 | 1160 | 1140 | 1166 |
| DP-h05 | 1134 | 1160 | 1141 | 1167 |
| DP-h06 | 1134 | 1160 | 1142 | 1168 |
| DP-h07 | 1135 | 1161 | 1142 | 1168 |
| DP-h08 | 1134 | 1160 | 1143 | 1169 |
| DP-h09 | 1136 | 1162 | 1142 | 1168 |
| DP-h10 | 1137 | 1163 | 1144 | 1170 |
| DP-h12 | 1136 | 1162 | 1144 | 1170 |
| DP-h13 | 1137 | 1163 | 1145 | 1171 |
| DP-h14 | 1136 | 1162 | 1145 | 1171 |
| | | | | |
| FDP-h01 | 1146 | 1172 | 1153 | 1179 |
| FDP-h02 | 1147 | 1173 | 1153 | 1179 |
| FDP-h03 | 1148 | 1174 | 1154 | 1180 |
| FDP-h04 | 1149 | 1175 | 1154 | 1180 |
| FDP-h05 | 1150 | 1176 | 1155 | 1181 |
| FDP-h06 | 1151 | 1177 | 1156 | 1182 |
| FDP-h07 | 1148 | 1174 | 1156 | 1182 |
| FDP-h07* | 1146 | 1172 | 1156 | 1182 |
| FDP-h08 | 1149 | 1175 | 1156 | 1182 |
| FDP-h09 | 1150 | 1176 | 1157 | 1183 |
| FDP-h10 | 1152 | 1178 | 1157 | 1183 |
| FDP-h11 | 1148 | 1174 | 1157 | 1183 |
| FDP-h12 | 1149 | 1175 | 1157 | 1183 |
| FDP-h 13 | 1150 | 1176 | 1186 | 1187 |
| FDP-h14 | 1152 | 1178 | 1186 | 1187 |
| FDP-h15 | 1148 | 1174 | 1186 | 1187 |
| FDP-h16 | 1149 | 1175 | 1186 | 1187 |

(continued)

| Table 8: Anti-EGFR Antibody Clones | | | | |
|---|---|---|---|---|
| | variable heavy chain | | variable light chain | |
| Name | SEQ ID NO: (protein) | SEQ ID NO: (DNA) | SEQ ID NO: (protein) | SEQ ID NO: (DNA) |
| FDP-h17 | 1150 | 1176 | 1158 | 1184 |
| FDP-h 18 | 1152 | 1178 | 1159 | 1185 |
| FDP-h19 | 1146 | 1172 | 1159 | 1185 |
| FDP-h20 | 1146 | 1172 | 1157 | 1183 |
| FDP-h21 | 1146 | 1172 | 1186 | 1187 |

| TABLE 9: Anti-EGFR Variable Heavy Chain Sequence Identity (V region gene Segment) | | cetuximab (SEQ ID NO:3) | | IGHV3-33*01 SEQ ID NO:119 1 | IGHV1-3*01 SEQ ID NO:119 2 | IGHV1-46*03 SEQ ID NO:119 3 | IGHV3-NL1*01 SEQ ID NO:119 4 | IGHV2-26*01 SEQ ID NO:119 5 | IGHV3-15*07 SEQ ID NO:119 6 |
|---|---|---|---|---|---|---|---|---|---|
| | SEQ ID NO | variable domain (1-119) | V segment (1-97) | V segment (1-98) | V segment (1-98) | V segment (1-98) | V segment (1-98) | V segment (1-100) | V segment (1-100) |
| cetuximab | 3 | | | **55** | 49 | 50 | 54 | 58 | 52 |
| DP-h01 | 1134 | 78 | 75 | **64** | | | | | |
| DP-h02 | 1134 | 78 | 75 | **64** | | | | | |
| DP-h03 | 1135 | 70 | 65 | | **67** | | | | |
| DP-h04 | 1134 | 78 | 75 | **64** | | | | | |
| DP-h05 | 1134 | 78 | 75 | **64** | | | | | |
| DP-h06 | 1134 | 78 | 75 | **64** | | | | | |
| DP-h07 | 1135 | 70 | 65 | | **67** | | | | |
| DP-h08 | 1134 | 78 | 75 | **64** | | | | | |
| DP-h09 | 1136 | 72 | 68 | | | **56** | | | |
| DP-h10 | 1137 | 71 | 67 | | | | **63** | | |
| DP-h12 | 1136 | 72 | 68 | | | **56** | | | |
| DP-h13 | 1137 | 71 | 67 | | | | **63** | | |
| DP-h14 | 1136 | 72 | 68 | | | **56** | | | |
| FDP-h01 | 1146 | 71 | 67 | | | | **65** | | |
| FDP-h02 | 1147 | 68 | 63 | | **65** | | | | |
| FDP-h03 | 1148 | 69 | 64 | | **65** | | | | |
| FDP-h04 | 1149 | 78 | 75 | | | | | **70** | |
| FDP-h05 | 1150 | 71 | 67 | | | | **65** | | |
| FDP-h06 | 1151 | 71 | 67 | | | | **63** | | |

(continued)

| TABLE 9: Anti-EGFR Variable Heavy Chain Sequence Identity (V region gene Segment) | | | | | | | | | |
|---|---|---|---|---|---|---|---|---|---|
| | | cetuximab (SEQ ID NO:3) | | IGHV3-33*01 SEQ ID NO:1191 | IGHV1-3*01 SEQ ID NO:1192 | IGHV1-46*03 SEQ ID NO:1193 | IGHV3-NL1*01 SEQ ID NO:1194 | IGHV2-26*01 SEQ ID NO:1195 | IGHV3-15*07 SEQ ID NO:1196 |
| | SEQ ID NO | variable domain (1-119) | V segment (1-97) | V segment (1-98) | V segment (1-98) | V segment (1-98) | V segment (1-98) | V segment (1-100) | V segment (1-100) |
| FDP-h07 | 1148 | 69 | 64 | | 65 | | | | |
| FDP-h07* | 1146 | 71 | 67 | | | | 65 | | |
| FDP-h08 | 1149 | 78 | 75 | | | | | 70 | |
| FDP-h09 | 1150 | 71 | 67 | | | | 65 | | |
| FDP-h10 | 1152 | 70 | 65 | | | | | | 65 |
| FDP-h11 | 1148 | 69 | 64 | | 65 | | | | |
| FDP-h12 | 1149 | 78 | 75 | | | | | 70 | |
| FDP-h13 | 1150 | 71 | 67 | | | | 65 | | |
| FDP-h14 | 1152 | 70 | 65 | | | | | | 65 |
| FDP-h15 | 1148 | 69 | 64 | | 65 | | | | |
| FDP-h16 | 1149 | 78 | 75 | | | | | 70 | |
| FDP-h17 | 1150 | 71 | 67 | | | | 65 | | |
| FDP-h18 | 1152 | 70 | 65 | | | | | | 65 |
| FDP-h19 | 1146 | 71 | 67 | | | | 65 | | |
| FDP-h20 | 1146 | 71 | 67 | | | | 65 | | |
| FDP-h21 | 1146 | 71 | 67 | | | | 65 | | |

| | SEQID NO: (Protein ) | variable domain (1-107) | cetuximab | | | | |
| | | | V segment (1-95) | IGKV1D-13*01 (SEQ ID NO: 1197) | IGKV1-39*01 (SEQ ID NO: 1198) | IGKV3-11*01 (SEQ ID NO: 1199) | IGKV3-15*01 (SEQ ID NO: 1200) |
|---|---|---|---|---|---|---|---|
| cetuximab | 4 | 100 | | 61 | 60 | 64 | 65 |
| DP-h01 | 1138 | 76 | 74 | **85** | | | |
| DP-h02 | 1139 | 75 | 73 | **83** | | | |
| DP-h03 | 1138 | 76 | 74 | **85** | | | |
| DP-h04 | 1140 | 75 | 73 | **87** | | | |
| DP-h05 | 1141 | 76 | 74 | | **78** | | |
| DP-h06 | 1142 | 75 | 73 | **77** | | | |
| DP-h07 | 1142 | 75 | 73 | 77 | | | |
| DP-h08 | 1143 | 76 | 74 | **80** | | | |
| DP-h09 | 1142 | 75 | 73 | 77 | | | |
| DP-h10 | 1144 | 77 | 75 | | | **82** | |
| DP-h12 | 1144 | 77 | 75 | | | **82** | |
| DP-h13 | 1145 | 76 | 74 | | | | **81** |
| DP-h14 | 1145 | 76 | 74 | | | | **81** |
| FDP-h01 | 1153 | 76 | 74 | | | | **81** |
| FDP-h02 | 1153 | 76 | 74 | | | | **81** |
| FDP-h03 | 1154 | 75 | 73 | **77** | | | |
| FDP-h04 | 1154 | 75 | 73 | **77** | | | |
| FDP-h05 | 1155 | 77 | 75 | | | **82** | |
| FDP-h06 | 1156 | 78 | 76 | | | **82** | |
| FDP-h07 | 1156 | 78 | 76 | | | **82** | |
| FDP-h07* | 1156 | 78 | 76 | | | **82** | |
| FDP-h08 | 1156 | 78 | 76 | | | **82** | |
| FDP-h09 | 1157 | 76 | 74 | **80** | | | |
| FDP-h10 | 1157 | 76 | 74 | **80** | | | |
| FDP-h11 | 1157 | 76 | 74 | **80** | | | |
| FDP-h12 | 1157 | 76 | 74 | **80** | | | |
| FDP-h13 | 1186 | 76 | 74 | **80** | | | |
| FDP-h14 | 1186 | 76 | 74 | **80** | | | |
| FDP-h15 | 1186 | 76 | 74 | **80** | | | |
| FDP-h16 | 1186 | 76 | 74 | **80** | | | |
| FDP-h17 | 1158 | 78 | 76 | | | | **86** |
| FDP-h18 | 1159 | 75 | 73 | **77** | | | |
| FDP-h19 | 1159 | 75 | 73 | **77** | | | |

**TABLE 10: Anti-EGFR Variable Light Chain Sequence Identity (V region gene segment)**

(continued)

| TABLE 10: Anti-EGFR Variable Light Chain Sequence Identity (V region gene segment) | | | | | | | |
|---|---|---|---|---|---|---|---|
| | | | cetuximab | | | | |
| | SEQID NO: (Protein ) | variable domain (1-107) | V segment (1-95) | IGKV1D-13*01 (SEQ ID NO: 1197) | IGKV1-39*01 (SEQ ID NO: 1198) | IGKV3-11*01 (SEQ ID NO: 1199) | IGKV3-15*01 (SEQ ID NO: 1200) |
| FDP-h20 | 1157 | 76 | 74 | **80** | | | |
| FDP-h21 | 1186 | 76 | 74 | **80** | | | |

Hence, provided herein are anti-EGFR antibodies containing a variable heavy and light chain having a sequence of amino acids set forth as: the variable heavy chain set forth in SEQ ID NO:1134 or a sequence of amino acids that exhibits at least 85% sequence identity to SEQ ID NO:1134, and the variable light chain set forth in SEQ ID NO:1138 or a sequence of amino acids that exhibits at least 85% sequence identity to SEQ ID NO:1138;
the variable heavy chain set forth in SEQ ID NO:1134 or a sequence of amino acids that exhibits at least 85% sequence identity to SEQ ID NO:1134, and the variable light chain set forth in SEQ ID NO:1139 or a sequence of amino acids that exhibits at least 85% sequence identity to SEQ ID NO:1139;
the variable heavy chain set forth in SEQ ID NO:1135 or a sequence of amino acids that exhibits at least 85% sequence identity to SEQ ID NO:1135, and the variable light chain set forth in SEQ ID NO:1138 or a sequence of amino acids that exhibits at least 85% sequence identity to SEQ ID NO:1138;
the variable heavy chain set forth in SEQ ID NO:1134 or a sequence of amino acids that exhibits at least 85% sequence identity to SEQ ID NO:1134, and the variable light chain set forth in SEQ ID NO:1140 or a sequence of amino acids that exhibits at least 85% sequence identity to SEQ ID NO:1140;
the variable heavy chain set forth in SEQ ID NO: 1134 or a sequence of amino acids that exhibits at least 85% sequence identity to SEQ ID NO:1134, and the variable light chain set forth in SEQ ID NO:1141 or a sequence of amino acids that exhibits at least 85% sequence identity to SEQ ID NO:1141;
the variable heavy chain set forth in SEQ ID NO:1134 or a sequence of amino acids that exhibits at least 85% sequence identity to SEQ ID NO:1134, and the variable light chain set forth in SEQ ID NO:1142 or a sequence of amino acids that exhibits at least 85% sequence identity to SEQ ID NO:1142;
the variable heavy chain set forth in SEQ ID NO:1135 or a sequence of amino acids that exhibits at least 85% sequence identity to SEQ ID NO:1135, and the variable light chain set forth in SEQ ID NO:1142 or a sequence of amino acids that exhibits at least 85% sequence identity to SEQ ID NO:1142;
the variable heavy chain set forth in SEQ ID NO:1134 or a sequence of amino acids that exhibits at least 85% sequence identity to SEQ ID NO:1134, and the variable light chain set forth in SEQ ID NO:1143 or a sequence of amino acids that exhibits at least 85% sequence identity to SEQ ID NO:1143;
the variable heavy chain set forth in SEQ ID NO:1136 or a sequence of amino acids that exhibits at least 85% sequence identity to SEQ ID NO:1136, and the variable light chain set forth in SEQ ID NO:1142 or a sequence of amino acids that exhibits at least 85% sequence identity to SEQ ID NO:1142;
the variable heavy chain set forth in SEQ ID NO:1137 or a sequence of amino acids that exhibits at least 85% sequence identity to SEQ ID NO:1137, and the variable light chain set forth in SEQ ID NO:1144 or a sequence of amino acids that exhibits at least 85% sequence identity to SEQ ID NO:1144;
the variable heavy chain set forth in SEQ ID NO:1136 or a sequence of amino acids that exhibits at least 85% sequence identity to SEQ ID NO:1136, and the variable light chain set forth in SEQ ID NO:1144 or a sequence of amino acids that exhibits at least 85% sequence identity to SEQ ID NO:1144;
the variable heavy chain set forth in SEQ ID NO:1137 or a sequence of amino acids that exhibits at least 85% sequence identity to SEQ ID NO:1137, and the variable light chain set forth in SEQ ID NO:1145 or a sequence of amino acids that exhibits at least 85% sequence identity to SEQ ID NO:1145;
the variable heavy chain set forth in SEQ ID NO:1136 or a sequence of amino acids that exhibits at least 85% sequence identity to SEQ ID NO:1136, and the variable light chain set forth in SEQ ID NO:1145 or a sequence of amino acids that exhibits at least 85% sequence identity to SEQ ID NO:1145;
the variable heavy chain set forth in SEQ ID NO:1146 or a sequence of amino acids that exhibits at least 85% sequence identity to SEQ ID NO:1146, and the variable light chain set forth in SEQ ID NO:1153 or a sequence of amino acids that exhibits at least 85% sequence identity to SEQ ID NO:1153;
the variable heavy chain set forth in SEQ ID NO:1147 or a sequence of amino acids that exhibits at least 85% sequence identity to SEQ ID NO:1147, and the variable light chain set forth in SEQ ID NO:1153 or a sequence of amino acids that exhibits at least 85% sequence identity to SEQ ID NO:1153;
the variable heavy chain set forth in SEQ ID NO:1148 or a sequence of amino acids that exhibits at least 85% sequence identity to SEQ ID NO:1148, and the variable light chain set forth in SEQ ID NO:1154 or a sequence of amino acids that exhibits at least 85% sequence identity to SEQ ID NO:1154;

the variable heavy chain set forth in SEQ ID NO:1149 or a sequence of amino acids that exhibits at least 85% sequence identity to SEQ ID NO:1149, and the variable light chain set forth in SEQ ID NO:1154 or a sequence of amino acids that exhibits at least 85% sequence identity to SEQ ID NO:1154;

the variable heavy chain set forth in SEQ ID NO:1150 or a sequence of amino acids that exhibits at least 85% sequence identity to SEQ ID NO:1150, and the variable light chain set forth in SEQ ID NO:1155 or a sequence of amino acids that exhibits at least 85% sequence identity to SEQ ID NO:1155;

the variable heavy chain set forth in SEQ ID NO:1151 or a sequence of amino acids that exhibits at least 85% sequence identity to SEQ ID NO:1151, and the variable light chain set forth in SEQ ID NO:1156 or a sequence of amino acids that exhibits at least 85% sequence identity to SEQ ID NO:1156;

the variable heavy chain set forth in SEQ ID NO:1148 or a sequence of amino acids that exhibits at least 85% sequence identity to SEQ ID NO:1148, and the variable light chain set forth in SEQ ID NO:1156 or a sequence of amino acids that exhibits at least 85% sequence identity to SEQ ID NO:1156;

the variable heavy chain set forth in SEQ ID NO:1146 or a sequence of amino acids that exhibits at least 85% sequence identity to SEQ ID NO:1146, and the variable light chain set forth in SEQ ID NO:1156 or a sequence of amino acids that exhibits at least 85% sequence identity to SEQ ID NO:1156;

the variable heavy chain set forth in SEQ ID NO:1149 or a sequence of amino acids that exhibits at least 85% sequence identity to SEQ ID NO:1149, and the variable light chain set forth in SEQ ID NO:1156 or a sequence of amino acids that exhibits at least 85% sequence identity to SEQ ID NO:1156;

the variable heavy chain set forth in SEQ ID NO:1150 or a sequence of amino acids that exhibits at least 85% sequence identity to SEQ ID NO:1150, and the variable light chain set forth in SEQ ID NO:1157 or a sequence of amino acids that exhibits at least 85% sequence identity to SEQ ID NO:1157;

the variable heavy chain set forth in SEQ ID NO:1152 or a sequence of amino acids that exhibits at least 85% sequence identity to SEQ ID NO:1152, and the variable light chain set forth in SEQ ID NO:1157 or a sequence of amino acids that exhibits at least 85% sequence identity to SEQ ID NO:1157;

the variable heavy chain set forth in SEQ ID NO:1148 or a sequence of amino acids that exhibits at least 85% sequence identity to SEQ ID NO:1148, and the variable light chain set forth in SEQ ID NO:1157 or a sequence of amino acids that exhibits at least 85% sequence identity to SEQ ID NO:1157;

the variable heavy chain set forth in SEQ ID NO:1149 or a sequence of amino acids that exhibits at least 85% sequence identity to SEQ ID NO:1149, and the variable light chain set forth in SEQ ID NO:1157 or a sequence of amino acids that exhibits at least 85% sequence identity to SEQ ID NO:1157;

the variable heavy chain set forth in SEQ ID NO:1150 or a sequence of amino acids that exhibits at least 85% sequence identity to SEQ ID NO:1150, and the variable light chain set forth in SEQ ID NO:1186 or a sequence of amino acids that exhibits at least 85% sequence identity to SEQ ID NO:1186;

the variable heavy chain set forth in SEQ ID NO:1152 or a sequence of amino acids that exhibits at least 85% sequence identity to SEQ ID NO:1152, and the variable light chain set forth in SEQ ID NO:1186 or a sequence of amino acids that exhibits at least 85% sequence identity to SEQ ID NO:1186;

the variable heavy chain set forth in SEQ ID NO: 1148 or a sequence of amino acids that exhibits at least 85% sequence identity to SEQ ID NO:1148, and the variable light chain set forth in SEQ ID NO:1186 or a sequence of amino acids that exhibits at least 85% sequence identity to SEQ ID NO:1186;

the variable heavy chain set forth in SEQ ID NO:1149 or a sequence of amino acids that exhibits at least 85% sequence identity to SEQ ID NO:1149, and the variable light chain set forth in SEQ ID NO:1186 or a sequence of amino acids that exhibits at least 85% sequence identity to SEQ ID NO:1186;

the variable heavy chain set forth in SEQ ID NO:1150 or a sequence of amino acids that exhibits at least 85% sequence identity to SEQ ID NO:1150, and the variable light chain set forth in SEQ ID NO:1158 or a sequence of amino acids that exhibits at least 85% sequence identity to SEQ ID NO:1158;

the variable heavy chain set forth in SEQ ID NO:1152 or a sequence of amino acids that exhibits at least 85% sequence identity to SEQ ID NO:1152, and the variable light chain set forth in SEQ ID NO:1159 or a sequence of amino acids that exhibits at least 85% sequence identity to SEQ ID NO:1159;

the variable heavy chain set forth in SEQ ID NO:1146 or a sequence of amino acids that exhibits at least 85% sequence identity to SEQ ID NO:1146, and the variable light chain set forth in SEQ ID NO:1159 or a sequence of amino acids that exhibits at least 85% sequence identity to SEQ ID NO:1159;

the variable heavy chain set forth in SEQ ID NO:1146 or a sequence of amino acids that exhibits at least 85% sequence identity to SEQ ID NO:1146, and the variable light chain set forth in SEQ ID NO:1157 or a sequence of amino acids that exhibits at least 85% sequence identity to SEQ ID NO:1157; and

the variable heavy chain set forth in SEQ ID NO:1146 or a sequence of amino acids that exhibits at least 85% sequence identity to SEQ ID NO:1146, and the variable light chain set forth in SEQ ID NO:1186 or a sequence of amino acids that exhibits at least 85% sequence identity to SEQ ID NO:1186.

Any of the above anti-EGFR antibodies can further contain a heavy chain constant region or light chain constant region, or a portion thereof. The constant region can be any immunoglobulin type (e.g., IgG, IgM, IgD, IgE, IgA and IgY), any class (e.g. IgG1, IgG2, IgG3, IgG4, IgA1 and IgA2) or subclass (e.g. IgG2a and IgG2b). In particular examples, the antibodies provided herein can be full-length antibodies further containing a constant region from an IgG1 antibody, or other subtype from among IgG2, IgG3 or IgG4. For example, the anti-EGFR antibodies can be full-length IgG1 antibodies be full-length IgG1 antibodies containing a kappa light chain constant region from cetuximab (set forth in SEQ ID NO:1071) or an IgG1 heavy chain constant region from cetuximab (set forth in SEQ ID NO:1069). The heavy chain constant region also can be a human IgG1 heavy chain set forth in SEQ ID NO:22, from an Ig classes, such as IgG2 (set forth in SEQ ID NO:23), IgG3 (set forth in SEQ ID NO:24) or IgG4 (set forth in SEQ ID NO:25), or can be a modified IgG1 heavy chain constant region set forth in SEQ ID NO:26, 27 or 1070. The light chain constant region also can be a human kappa light chain (set forth in SEQ ID NO:1072) or a human lambda light chain (set forth in SEQ ID NO:1073).

Modified anti-EGFR antibodies provided herein also include antibody fragments, which are derivatives of full-length antibody that contain less than the full sequence of the full-length antibodies but retain at least a portion of the specific binding abilities of the full-length antibody, for example the variable portions of the heavy and light chain. The antibody fragments also can include antigen-binding portions of an antibody that can be inserted into an antibody framework (e.g., chimeric antibodies) in order to retain the binding affinity of the parent antibody. Examples of antibody fragments include, but are not limited to, Fab, Fab', F(ab')$_2$, single-chain Fv (scFv), Fv, dsFv, diabody, Fd and Fd' fragments, and other fragments, including modified fragments (see, for example, Methods in Molecular Biology, Vol. 207: Recombinant Antibodies for Cancer Therapy Methods and Protocols (2003); Chapter 1; p 3-25, Kipriyanov). Antibody fragments can include multiple chains linked together, such as by disulfide bridges and can be produced recombinantly. Antibody fragments also can contain synthetic linkers, such as peptide linkers, to link two or more domains. Methods for generating antigen-binding fragments are well-known known in the art and can be used to modify any antibody provided herein. Fragments of antibody molecules can be generated, such as for example, by enzymatic cleavage. For example, upon protease cleavage by papain, a dimer of the heavy chain constant regions, the Fc domain, is cleaved from the two Fab regions (*i.e.* the portions containing the variable regions).

Single chain antibodies can be recombinantly engineered by joining a heavy chain variable region (V$_H$) and light chain variable region (V$_L$) of a specific antibody. The particular nucleic acid sequences for the variable regions can be cloned by standard molecular biology methods, such as, for example, by polymerase chain reaction (PCR) and other recombination nucleic acid technologies. Methods for producing scFvs are described, for example, by Whitlow and Filpula (1991) Methods, 2: 97-105; Bird et al. (1988) Science 242:423-426; Pack et al. (1993) Bio/Technology 11:1271-77; and U.S. Patent Nos. 4,946,778, 5,840,300, 5,667,988, 5,658,727, 5,258,498).

The above anti-EGFR antibodies, or antigen-binding fragments the anti-EGFR antibodies provided herein, including modified anti-EGFR antibodies and antigen binding fragments of any of the anti-EGFR antibodies, bind to EGFR (particularly human EGFR) with a higher binding activity under conditions that exist in a tumor microenvironment that include one or both of pH between or about between pH 5.6 to 6.8 or lactate concentration of between or about between 5 mM to 20 mM and 10 mg/mL to 50 mg/mL protein (e.g. 20% to 50% human serum), compared to under conditions that exist in a non-tumor microenvironment that include one or both of pH between or about between pH 7.0 to 7.8 or lactate concentration between or about between 0.5 mM to 5 mM and 10 mg/mL to 50 mg/mL protein (e.g. 20% to 50% human serum). The higher binding activity under conditions in a tumor microenvironment compared to under conditions in a non-tumor microenvironment generally exists under conditions where the protein concentration under conditions in a tumor microenvironment and under conditions in a non-tumor microenvironment is substantially the same or is the same. In particular examples, the ratio of activity can be at least or greater than 2.0, and generally greater than 3.0, 3.5, 4.0, 4.5, 5.0, 6.0, 7.0, 8.0, 9.0, 10.0, 11.0, 12.0, 13.0, 14.0, 15.0, 20.0, 25.0, 30.0, 35.0, 40.0, 45.0, 50.0 or more.

Any of the above anti-EGFR antibodies, or antigen-binding fragments thereof, also effect significant productivity when produced in mammalian cells, particularly compared to the non-humanized parental antibody. For example, mammalian host cells containing nucleic acid encoding any of the above anti-EGFR antibodies (e.g. containing a nucleic acid encoding a heavy and light chain as set forth in Table 8) can effect expression of the antibody at a concentration that is greater than or greater than about or that is at least 1 mg/mL, 1.5 mg/mL, 2.0 mg/mL, 2.5 mg/mL, 3.0 mg/mL, 3.5 mg/mL, 4.0 mg/mL, 4.5 mg/mL, 5.0 mg/mL, 5.5 mg/mL, 6.0 mg/mL, 6.5 mg/mL, 7.0 mg/mL, 8.0 mg/mL, 9.0 mg/mL, 10.0 mg/mL or more.

### 3. Additional Modifications

Any of the modified anti-EGFR antibodies provided herein can contain one or more additional modifications. The modifications (e.g. amino acid replacements) can be in the variable region or constant region of the heavy or light chain. Examples of additional modifications that can be included in the modified anti-EGFR antibodies provided herein include, but are not limited to, those described in U.S. Pat. Nos. 7,657,380, 7,930,107, 7,060,808, 7,723,484, U.S. Pat. Publ. Nos. 2011014822, 2005142133, 2011117110, International Pat. Pub. Nos. WO2012003995, WO2010080463, WO2012020059, WO2008152537, and Lippow et al. (2007) Nat Biotechnol. 25(10):1171-1176. Non-limiting examples of exemplary amino acid modifications described in the art that can be included in any anti-EGFR antibody, or antigen binding fragment thereof, provided herein include, variants containing an amino acid replacement (substitution) in the variable light chain (V$_L$) at positions corresponding to replacement of Aspartate (D) at position 1 with Glutamate (E), D1C, I2T, I2C, L3V, L3T, L3C, L4C, T5C, Q6C, S7C, P8C, V9C, V9A, V9D, V9G, V9P, V9S, I1OT, I10S, I10F, II0C, L11Q, L11C, S12A, S12C, V13L, V13M, V13S, V13A, V13C, S14T, S14C, P15V, P15L, P15C, G16K, G16C, E17D, E17K, E17C, R18V, R18K, R18C, V19A, V19T, V19C, S20T, S20C, S20A, F21I, F21L, F21C, S22T, S22C, R24P, A25V, A25S, A25I, A25P, A25T, A25Y, A25C, A25F, A25M, A25L, A25W, S26D, Q27W, Q27E, Q27F, Q27Y, Q27T, Q27H, S28R, S28F, G30Y, G30C, G30H, G30K, G30Q, G30R, G30W, G30F, G30T, G30M, G30S, G30A, T31E, T31V, T31D, T31R, N32H, I33L, H34C, Q38K, R39K, T40P, T40S, N41G, N41D, G42Q, G42K, G42E, S43A, S43P, R45K, K49Y, K49F, Y50G, S53V, S60D, S60A, G64S, G64A, D70E, D70V, F71Y, S74T, N76S, N76T, S77R, S77G, V78L, E79Q, S80P, S80A, E81A, I83F, I83S, I83V, I83A, D85V, D85T, D85I, D85M, Y87S, Q89C, Q89H, Q90C, N91C, N91Q, N91L, N92C, N92L, N92R N92K, N92M, N92Y, N92H, N92E, N92F, N93A, N93D, N93E, N93V, N93K, N93C, W94F, W94Y, P95C, T96C, T96L, T96E, T97C, T97A, T97D, T97E, T97P, T97K, T97N, T97Q, T97I, T97G, T97L, T97H, T97R, T97S, G99A, A100G, A100Q, K103T, L104V and L106I, in the sequence of amino acids set forth in SEQ ID NO:4;

variants containing an amino acid replacement (substitution) in the variable heavy chain (V$_H$) at positions corresponding to replacement of Glutamine (Q) at position 1 with Glutamic acid (E), Q1C, V2C, Q3T, Q3C, L4C, K5Q, K5V, K5L, K5C, Q6E, Q6C, S7C, G8C, P9A, P9G, P9C, G10V, G10C, L11C, V12C, Q13K, Q13R, Q13C, P14C, S15G, S15T, S15C, Q16G, Q16R, Q16E, Q16C, S17T, S17C, L18C, S19K, S19R, S19T, S19C, I20L, I20C, T21S, T21C, T23A, T23K, T23C, V24A, V24C, S25C, F27G, S28N, S28T, L29I, T30S, T30K, N31V, N31D, N31I, N31T, N32S, Y32R, Y32W, G33A, G33D, G33E, G33Y, V34L, V34N, V34E, V34Q, V34S, V34W, H35S, V37I, S40A, S40P, P41T, G44A, L48V, L48I, G49S, G49A, V50L, V50Q, V50E, V50I, V50Y, V50N, 151G, I51M, I51S, 151Q, 151A, 151C, 151V, W52F, W52Y, W52G, W52T, S53Q, S53T, S53N, S53Y, G54A, G54V, G54L, G54I, G54S, G55D, G55A, G55E, G55H, G55F, N56A, N56G, N56S, N56T, T57A, T57D, T57G, T57S, T57E, T57P, D58Y, D58N, Y59A, Y59C, Y59E, Y59F, Y59G, Y59S, Y59W, T59H, Y59P, Y59Q, N60D, N60A, T61E, T61P, P62S, F63L, F63V, T64K, T64E, T64A, T64N, T64D, S65G, L67F, L67V, S68T, N70S, N70T, K71V, D72E, N73T, S74A, S76N, Q77T, Q77S, V78L, V78F, V78A, F79Y, F79S, F79V, F80L, F80M, K81Q, K81T, K81E, K81Q, M82L, N83T, N83S, S84N, L85M, L85V, Q86R, Q86D, Q86T, S87A, S87P, N88E, N88V, N88G, N88A, N88D, I92T, I92V, A96C, R97C, A98C, L99C, L99E, T100D, T100C, T100A, Y101C, Y101W, Y101A, Y102C, Y102F, Y102A, Y102W, D103E, D103P, D103C, Y104C, E105C, E105N, E105D, E105Y, F106C, F106D, F106Y, A107C, A107D, Y108C and Y108F, in the sequence of amino acids set forth in SEQ ID NO:3; and

variants containing amino acid replacement (substitution) in the heavy chain constant regions, for example, in the hinge, C$_H$2 and C$_H$3 regions, including replacement of Proline (P) at position 230 with Alanine (A), E233D, L234D, L234E, L234N, L234Q, L234T, L234H, L234Y, L234I, L234V, L234F, L235D, L235S, L235N, L235Q, L235T, L235H, L235Y, L235I, L235V, L235F, S239D, S239E, S239N, S239Q, S239F, S239T, S239H, S239Y, V240I, V240A, V240T, V240M, F241W, F241L, F241Y, F241E, F241R, F243W, F243L F243Y, F243R, F243Q, P244H, P245A, P247V, P247G, V262I, V262A, V262T, V262E, V263I, V263A, V263T, V263M, V264L, V264I, V264W, V264T, V264R, V264F, V264M, V264Y, V264E, D265G, D265N, D265Q, D265Y, D265F, D265V, D265I, D265L, D265H, D265T, V266I, V266A, V266T, V266M, S267Q, S267L, S267T, S267H, S267D, S267N, E269H, E269Y, E269F, E269R, E269T, E269L, E269N, D270Q, D270T, D270H, E272S, E272K, E272I, E272Y, V273I, K274T, K274E, K274R, K274L, K274Y, F275W, N276S, N276E, N276R, N276L, N276Y, Y278T, Y278E, Y278K, Y278W, E283R, Y296E, Y296Q, Y296D, Y296N, Y296S, Y296T, Y296L, Y296I, Y296H, N297S, N297D, N297E, S298H, T299I, T299L, T299A, T299S, T299V, T299H, T299F, T299E, V302I, W313F, E318R, K320T, K320D, K320I, K322T, K322H, V323I, S324T, S324D, S324R, S324I, S324V, S324L, S324Y, N325Q, N325L, N325I, N325D, N325E, N325A, N325T, N325V, N325H, K326L, K326I, K326T, A327N, A327L, A327D, A327T, L328M, L328D, L328E, L328N, L328Q, L328F, L328I, L328V, L328T, L328H, L328A, P329F, A330L, A330Y, A330V, A330I, A330F, A330R, A330H, A330S, A330W, A330M, P331V, P331H, I332D, I332E, I332N, I332Q, I332T, I332H, I332Y, I332A, E333T, E333H, E333I, E333Y, K334I, K334T, K334F, T335D, T335R, T335Y, D221K, D221Y, K222E, K222Y, T223E, T223K, H224E, H224Y, T225E, T225E, T225K, T225W, P227E, P227K, P227Y, P227G, P228E, P228K, P228Y, P228G, P230E, P230Y, P230G, A231E, A231K, A231Y, A231P, A231G, P232E, P232K, P232Y, P232G, E233N, E233Q, E233K, E233R, E233S, E233T, E233H, E233A, E233V, E233L, E233I, E233F, E233M, E233Y, E233W, E233G, L234K, L234R, L234S, L234A, L234M, L234W, L234P, L234G, L235E, L235K, L235R, L235A, L235M, L235W, L235P, L235G, G236D, G236E, G236N, G236Q, G236K, G236R, G236S, G236T, G236H, G236A, G236V, G236L, G236I, G236F, G236M, G236Y, G236W, G236P, G237D, G237E, G237N, G237Q, G237K, G237R, G237S, G237T, G237H, G237V, G237L, G237I, G237F, G237M, G237Y, G237W, G237P, P238D, P238E, P238N, P238Q, P238K, P238R, P238S, P238T, P238H, P238V, P238L, P238I, P238F, P238M, P238Y, P238W, P238G, S239Q, S239K, S239R, S239V, S239L, S239I, S239M, S239W, S239P, S239G, F241D, F241E, F241Y, F243E, K246D, K246E, K246H, K246Y, D249Q, D249H, D249Y, R255E, R255Y, E258S, E258H, E258Y, T260D, T260E, T260H, T260Y, V262E, V262F, V264D, V264E, V264N, V264Q, V264K, V264R, V264S, V264H, V264W, V264P, V264G, D265Q, D265K, D265R, D265S, D265T, D265H, D265V, D265L, D265I, D265F, D265M, D265Y, D265W, D265P, S267E, S267Q, S267K, S267R, S267V, S267L, S267I, S267F, S267M, S267Y, S267W, S267P, H268D, H268E, H268Q, H268K, H268R, H268T, H268V, H268L, H268I, H268F, H268M, H268W, H268P, H268G, E269K, E269S, E269V, E269I, E269M, E269W, E269P, E269G, D270R, D270S, D270L, D270I, D270F, D270M, D270Y, D270W, D270P, D270G, P271D, P271E, P271N, P271Q, P271K, P271R, P271S, P271T, P271H, P271A, P271V, P271L, P271I, P271F, P271M, P271Y, P271W, P271G, E272D, E272R, E272T, E272H, E272V, E272L, E272F, E272M, E272W, E272P, E272G, K274D, K274N, K274S, K274H, K274V, K274I, K274F, K274M, K274W, K274P, K274G, F275L, N276D, N276T, N276H, N276V, N276I, N276F, N276M, N276W, N276P, N276G, Y278D, Y278N, Y278Q, Y278R, Y278S, Y278H, Y278V, Y278L, Y278I, Y278M, Y278P, Y278G, D280K, D280L, D280W, D280P, D280G, G281D, G281K, G281Y, G281P, V282E, V282K, V282Y, V282P, V282G, E283K, E283H, E283L, E283Y, E283P, E283G, V284E, V284N, V284T, V284L, V284Y, H285D, H285E, H285Q, H285K, H285Y, H285W, N286E, N286Y, N286P, N286G, K288D, K288E, K288Y, K290D, K290N, K290H, K290L, K290W, P291D, P291E, P291Q, P291T, P291H, P291I, P291G, R292D, R292E, R292T, R292Y, E293N, E293R, E293S, E293T, E293H, E293V, E293L, E293I, E293F, E293M, E293Y, E293W, E293P, E293G, E294K, E294R, E294S, E294T, E294H, E294V, E294L, E294I, E294F, E294M, E294Y, E294W, E294P, E294G, Q295D, Q295E, Q295N, Q295R, Q295S, Q295T, Q295H, Q295V, Q295I, Q295F, Q295M, Q295Y, Q295W, Q295P, Q295G, Y296K, Y296R, Y296A, Y296V, Y296M, Y296G, N297Q, N297K, N297R, N297T, N297H, N297V, N297L, N297I, N297F, N297M, N297Y, N297W, N297P, N297G, S298D, S298E, S298Q, S298K, S298R, S298I, S298F, S298M, S298Y, S298W, T299D, T299E, T299N, T299Q, T299K, T299R, T299L, T299F, T299M, T299Y, T299W, T299P, T299G, Y300D, Y300E, Y300N, Y300Q, Y300K, Y300R, Y300S, Y300T, Y300H, Y300A, Y300V, Y300M, Y300W, Y300P, Y300G, R301D, R301E, R301H, R301Y, V303D, V303E, V303Y, S304D, S304N, S304T, S304H, S304L, V305E, V305T, V305Y, K317E, K317Q, E318Q, E318H, E318L, E318Y, K320N, K320S, K320H, K320V, K320L, K320F, K320Y, K320W, K320P, K320G, K322D, K322S, K322V, K322I, K322F, K322Y, K322W, K322P, K322G, S324H, S324F, S324M, S324W, S324P, S324G, N325K, N325R, N325S, N325F, N325M, N325Y, N325W, N325P, N325G, K326P, A327E, A327K, A327R, A327H, A327V, A327I, A327F, A327M, A327Y, A327W, A327P, L328D, L328Q, L328K, L328R, L328S, L328T, L328V, L328I, L328Y, L328W, L328P, L328G, P329D, P329E, P329N, P329Q, P329K, P329R, P329S, P329T, P329H, P329V, P329L, P329I, P329M, P329Y, P329W, P329G, A330E, A330N, A330T, A330P, A330G, P331D, P331Q, P331R, P331T, P331L, P331I, P331F, P331M, P331Y, P331W, I332K, I332R, I332S, I332V, I332F, I332M, I332W, I332P, I332G, E333L, E333F, E333M, E333P, K334P, T335N, T335S, T335H, T335V, T335L, T335I, T335F, T335M, T335W, T335P, T335G, I336E, I336K, I336Y, S337E, S337N, S337H, S298A, K326A, K326S, K326N, K326Q, K326D, K325E, K326W, K326Y, E333A, E333S, K334A, K334E, Y300I, Y300L, Q295K, E294N, S298N, S298V, S298D, D280H, K290S, D280Q, D280Y, K290G, K290T, K290Y, T250Q, T250E, M428L, M428F, S239D, S239E, S239N, S239Q, S239T, V240I, V240M, V264I, V264T, V264Y, E272Y, K274E, Y278T, N297D, T299A, T299V, T299I, T299H, K326T, L328A, L328H, A330Y, A330L, A330I, I332D, I332E, I332N, and I332Q, according to EU index numbering.

## 4. Conjugates

Also provided herein are conjugates that contain a conditionally active anti-EGFR antibody provided herein linked directly or via a linker to one or more targeted agents. These conjugates contain the following components: antibody (Ab), (linker (L))$_q$, (targeted agent)$_m$ and are represented by the formula: Ab-(L)$_q$-(targeted agent)$_m$, where q is 0 or more and m is at least 1. Thus, the conjugates provided herein contain one or more targeted agents covalently linked to an antibody provided herein that is conditionally active or selective for a tumor microenvironment and binds to EGFR.

Hence, these conjugates, also called antibody-drug conjugates (ADC) or immunoconjugates, can be used for targeted delivery of cytotoxic or cytostatic agents, *i.e.,* drugs to kill or inhibit tumor cells in the treatment of cancer. Such conjugates exhibit selectivity to tumor cells that are desired to be eliminated over non-diseased cells, and thereby do not result in unacceptable levels of toxicity to normal cells. Therefore, the conjugates achieve maximal efficacy with minimal toxicity and reduced side effects. Hence, such compounds can be used in the methods described herein of diagnosis or treatment of cancer and other diseases or disorders.

As stated above, the number of targeted agents is designated by the variable m, where m is an integer of 1 or greater. The targeted agent is conjugated to an antibody provided herein by the number of linkers designated by the variable q, where q is 0 or any integer. The variables q and m are selected such that the resulting conjugate interacts with the EGFR of target cells, in particular, tumor cells in an acidic microenvironment, and the targeted agent is internalized by the target cell. Typically, m is between 1 and 8. q is 0 or more, depending upon the number of linked targeting and targeted agents and/or functions of the linker; q is generally 0 to 4. When more than one targeted agent is present in a conjugate the targeted agents may be the same or different.

The targeted agents can be covalently linked to the anti-EGFR antibody directly or by one or more linkers. Any suitable association among the elements of the conjugate is contemplated as long as the resulting conjugates interact with the EGFR of a target cell such that internalization of the associated targeted agent is effected. Thus, the conjugates provided herein can be produced as fusion proteins, can be chemically coupled, or can include a fusion protein portion and a chemically linked portion or any combination thereof.

The targeted agents also can be modified to render them more suitable for conjugation with the linker and/or the anti-EGFR antibody or to increase their intracellular activity. For example, in the case of polypeptide targeted agents, such modifications include, but are not limited to, the introduction of a Cys residue at or near the N-terminus or C-terminus, derivatization to introduce reactive groups, such as thiol groups, and addition of sorting signals, such as (Xaa-Asp-Glu-Leu)$_n$ (SEQ ID NO. 1190) where Xaa is Lys or Arg, preferably Lys, and n is 1 to 6, preferably 1-3, at, preferably, the carboxy-terminus of the targeted agent (see, *e.g.,* Seetharam et al. (1991) J. Biol. Chem. 266:17376-17381; and Buchner et al. (1992) Anal. Biochem. 205:263-270), that direct the targeted agent to the endoplasmic reticulum.

In other examples, the targeted agent can be modified to eliminate one or more cysteine residues, for example, to provide more predictable thiol conjugation at preferred locations. Care must be taken to avoid altering specificity of the resulting modified targeted agent, unless such alteration is desired. In all instances, particular modifications can be determined empirically.

The linker, L, attaches the antibody to the targeted agent through covalent bond(s). The linker can be a peptide or a non-peptide and can be selected to relieve or decrease steric hindrance caused by proximity of the targeted agent to the anti-EGFR antibody and/or to increase or alter other properties of the conjugate, such as the specificity, toxicity, solubility, serum stability and/or intracellular availability of the targeted moiety and/or to increase the flexibility of the linkage between the anti-EGFR antibody and the targeted agent.

When fusion proteins are contemplated, the linker is selected such that the resulting nucleic acid molecule encodes a fusion protein that binds to and is internalized by cells in a tumor microenvironment that express EGFR and all or a portion of the internalized protein preferably traffics to the cytoplasm. It also is contemplated that several linkers can be joined in order to employ the advantageous properties of each linker. In such instances, the linker portion of a conjugate may contain more than 50 amino acid residues. The number of residues is not important as long as the resulting fusion protein binds to EGFR of the target cell and internalizes the linked targeted agent via a pathway that traffics the targeted agent to the cytoplasm and/or nucleus.

The targeted agent can be a protein, peptide, nucleic acid, small molecule, therapeutic moiety, or other agent in which targeted delivery to a selected population of tumor cells is desired. Such targeted agents include, but are not limited to, cytotoxic agents, DNA and RNA nucleases, toxins, drugs or other agents. Therapeutic moieties include, but are not limited to, cytotoxic moieties, radioisotopes, chemotherapeutic agents, lytic peptides and cytokines. Exemplary therapeutic moieties include, but are not limited to, among taxol; cytochalasin B; gramicidin D; ethidium bromide; emetine; mitomycin; etoposide; teniposide; vincristine; vinblastine; colchicine; doxorubicin; daunorubicin; dihydroxy anthracin dione; maytansine or an analog or derivative thereof; an auristatin or a functional peptide analog or derivative thereof; dolastatin 10 or 15 or an analog thereof; irinotecan or an analog thereof; mitoxantrone; mithramycin; actinomycin D; 1-dehydrotestosterone; a glucocorticoid; procaine; tetracaine; lidocaine; propranolol; puromycin; calicheamicin or an analog or derivative thereof; an antimetabolite; an alkylating agent; a platinum derivative; duocarmycin A, duocarmycin SA, rachelmycin (CC-1065), or an analog or derivative thereof; an antibiotic; pyrrolo[2,1-c][1,4]-benzodiazepine (PDB); a toxin; ribonuclease (RNase); DNase I, Staphylococcal enterotoxin A; and pokeweed antiviral protein.

Drugs also can be used as a targeted agent in these methods. Such drugs include 5-fluorouracil, vinca alkaloids, and antibiotics such as dactinomycin, bleomycin, daunorubicin, doxorubicin, idarubicin, methotrexate, mithramycin, mitomycin, mitoxantrone, plicamycin and anthramycin (AMC), neocarzinostatin (Takahashi et al. (1988) Cancer 61:881-888) and vindesine (Rowland et al., (1986) Cancer Immunol Immunother 21(3):183-187).

Toxins used in antibody-toxin conjugates include bacterial toxins such as diphtheria toxin, and active fragments thereof and hybrid molecules, plant toxins, such as ricin toxin (U.S. Pat. No. 4,753,894; U.S. Pat. No. 5,629,197; U.S. Pat. No. 4,958,009; U.S. Pat. No. 4,956,453), small molecule toxins such as geldanamycin (Mandler et al. (2000) J. Nat. Cancer Inst. 92(19):1573-1581; Mandler et al. (2000) Bioorg. Med. Chem. Lett. 10:1025-1028; Mandler et al. (2002) Bioconjug. Chem. 13:786-791), maytansinoids, such as DM1, DM3 and DM4 (EP 1391213; Chari (2008) Acc Chem Res 41:98-107; Liu et al., (1996) Proc. Natl. Acad. Sci. USA 93:8618-8623), and calicheamicin (Damle (2004) Expert Opin Biol Ther 4:1445-1452; Lode et al. (1998) Cancer Res. 58:2928; Hinman et al. (1993) Cancer Res. 53:3336-3342). Finally, the auristatin peptides, auristatin E (AE), monomethylauristatin E (MMAE), and monomethylauristatin F (MMAF), synthetic analogs of dolastatin can be employed (Doronin et al. (2003) Nature Biotechnology 21(7):778-784). Other toxins include cholera toxin, a Shiga-like toxin, LT toxin, C3 toxin, Shiga toxin, pertussis toxin, tetanus toxin, soybean Bowman-Birk protease inhibitor, Pseudomonas exotoxin, alorin, saporin, modeccin, galanin, abrin A chain, modeccin A chain, alpha-sarcin, Aleurites fordii proteins, dianthin proteins, Phytolacca americana proteins, momordica charantia inhibitor, curcin, crotin, gelonin, mitogillin, restrictocin, phenomycin, and enomycin toxins. The toxins can effect their cytotoxic and cytostatic effects by mechanisms including tubulin binding, DNA binding, or topoisomerase inhibition.

**a. Targeted Agents**

The targeted agent can be a protein, peptide, nucleic acid, small molecule, therapeutic moiety, or other agent in which targeted delivery to a selected population of tumor cells is desired. Such targeted agents include, but are not limited to, cytotoxic agents, DNA and RNA nucleases, toxins, drugs or other agents.

**i. Maytansinoid Drug Moieties**

Maytansinoid drug moieties are described in U.S. Patent No. 8,142,784. Maytansine compounds inhibit cell proliferation by inhibiting the formation of microtubules during mitosis through inhibition of polymerization of the microtubule protein, tubulin (Remillard et al. (1975) Science 189:1002-1005; U.S. Pat. No. 5,208,020). Maytansine and maytansinoids are highly cytotoxic but their clinical use in cancer therapy has been greatly limited by their severe systemic side-effects primarily attributed to their poor selectivity for tumors. Clinical trials with maytansine had been discontinued due to serious adverse effects on the central nervous system and gastrointestinal system (Issel et al. (1978) Can. Treatment. Rev. 5:199-207).

Maytansinoid drug moieties are attractive drug moieties in antibody-drug conjugates because they are: (i) relatively accessible to prepare by fermentation or chemical modification, derivatization of fermentation products, (ii) amenable to derivatization with functional groups suitable for conjugation through the non-disulfide linkers to antibodies, (iii) stable in plasma, and (iv) effective against a variety of tumor cell lines.

Maytansine compounds suitable for use as maytansinoid drug moieties are well known in the art, and can be isolated from natural sources according to known methods, produced using genetic engineering techniques (see Yu et al. (2002) PNAS 99:7968-7973), or maytansinol and maytansinol analogues prepared synthetically according to known methods.

Exemplary maytansinoid drug moieties include those having a modified aromatic ring, such as: C-19-dechloro (U.S. Pat. No. 4,256,746) (prepared by lithium aluminum hydride reduction of ansamitocin P2); C-20-hydroxy (or C-20-demethyl)+/-C-19-dechloro (U.S. Pat. Nos. 4,361,650 and 4,307,016) (prepared by demethylation using *Streptomyces* or *Actinomyces* or dechlorination using LAH); and C-20-demethoxy, C-20-acyloxy (-OCOR), +/-dechloro (U.S. Pat. No. 4,294,757) (prepared by acylation using acyl chlorides); and those having modifications at other positions.

Exemplary maytansinoid drug moieties also include those having modifications such as: C-9-SH, prepared by the reaction of maytansinol with $H_2S$ or P2S5 (U.S. Pat. No. 4,424,219); C-14-alkoxymethyl(demethoxy/$CH_2OR$)(U.S. Pat. No. 4,331,598); C-14-hydroxymethyl or acyloxymethyl ($CH_2OH$ or $CH_2OAc$) prepared from *Nocardia* (U.S. Pat. No. 4,450,254); C-15-hydroxy/acyloxy, prepared by the conversion of maytansinol by *Streptomyces* (U.S. Pat. No. 4,364,866); C-15-methoxy, isolated from Trewia nudiflora (U.S. Pat. No. 4,313,946 and U.S. Pat. No. 4,315,929); C-18-N-demethyl, prepared by the demethylation of maytansinol by *Streptomyces* (U.S. Pat. No. 4,362,663 and U.S. Pat. No. 4,322,348); and 4,5-deoxy, prepared by the titanium trichloride/LAH reduction of maytansinol (U.S. Pat. No. 4,371,533).

Many positions on maytansine compounds are known to be useful as the linkage position, depending upon the type of link. For example, for forming an ester linkage, the C-3 position having a hydroxyl group, the C-14 position modified with hydroxymethyl, the C-15 position modified with a hydroxyl group and the C-20 position having a hydroxyl group are all suitable.

Maytansinoid drug moieties can be linked to an anti-EGFR antibody by direct conjugation or using any of the linkers provided herein. In particular examples, the cytotoxic or drug agent is mertansine, also known as DM1 ($N_2$'-deacetyl-$N_2$'-(3-mercapto-2-oxopropyl)-maytansine). Mertansine can be linked via 4-mercaptovaleric acid. An emtansine conjugate also can be formed with the antibodies herein using the linker 4-(3-mercapto-2,5-dioxo-1-pyrrolidinylmethyl)-cylohexanecarboxylic acid (MCC).

**ii. Auristatins and Dolastatins Drug Moieties**

Auristatins and dolastatins are described in published U.S. Application No. US2011/0217321. Dolastatins and auristatins have been shown to interfere with microtubule dynamics, GTP hydrolysis, and nuclear and cellular division (Woyke et al. (2001) Antimicrob. Agents and Chemother. 45(12):3580-3584) and have anticancer (U.S. Pat. No. 5,663,149) and antifungal activity (Pettit et al. (1998) Antimicrob. Agents Chemother. 42:2961-2965). Further, auristatins are highly potent, synthetic, stable, and amenable to chemical modification to allow for linker attachment (Senter (2009) Curr Opin Chem Biol 13:235-244).

Because auristatins are synthetic, integral structural modifications can be made to significantly alter the properties of the parent drug. For example, monomethylauristatin F (MMAF) terminates with the amino acid residue phenylalanine, which impairs cell membrane permeability (Doronina et al., (2006) Bioconjug Chem. 17:114-124). Thus, conjugation of MMAF to an ADC can facilitate selective drug uptake by antigen-positive cells (Doronina et al., (2006) Bioconjug Chem. 17:114-124; Doronina et al., (2003) Nat Biotechnol. 21:778-784).

The dolastatin or auristatin drug moiety can be attached to antibodies through the N (amino) terminus or the C (carboxyl) terminus of the peptidic drug moiety (WO 2002/088172). Exemplary auristatin embodiments include N-terminally and C-terminally linked monomethylauristatin drug moieties MMAE and MMAF (Senter et al. (2004) "Proceedings of the American Association for Cancer Research," Volume 45, Abstract Number 623, and presented Mar. 28, 2004; U.S. Publication No. 2011/0020343).

Dolastatin or auristatin can be linked to an anti-EGFR antibody by direct conjugation or using any of the linkers provided herein. In particular examples, dolastatin or auristatin can be linked to an anti-EGFR antibody with a peptide linker, such as valine-citrulline (Val-Cit).

**iii. Cell Toxin Moieties**

Cell toxins suitable for use in the methods and compositions include small molecules, such as DNA cleaving agents, and proteinaceous cell toxins, including, but are not limited to, bacterial, fungal, plant, insect, snake and spider toxins. Amino acid sequences of exemplary cell toxins contemplated for incorporation in the conjugates provided herein are set forth in Table 11.

**TABLE 11: Exemplary Amino Acid Sequences of Toxins**

| Toxin | Sequence | SEQ ID |
|-------|----------|--------|
| Bryodin | DVSFRLSGATTTSYGVFIKNLREALPYERKVYNIPLLRSSISGRYTL LHLTNYADETISVAVDVTNVYIMGYLAGDVSYFFNEASATEAAK FVFKDAKKKVTLPYSGNYERLQTAAGKIRENIPLGLPALDSAITTL YYYTASSAASALLVLIQSTAESARYKFIEQQIGKRVDKTFLPSLATI SLENNWSALSKQIQIASTNNGQFESPVVLIDGNNQRVSITNASARV VTSNIALLLNRNNIA | 1202 |
| Saporin-6 | VTSITLDLVNPTAGQYSSFVDKIRNNVKDPNLKYGGTDIAVIPPSK EKFLRINFQSSRGTVSLGLKRDNLYVVAYLAMDNTNVNRAYYFR SEITSAESTALFPEATTANQKALEYTEDYQSIEKNAQITQGDQSRK ELGLGIDLLSTSMEAVNKKARVVKDEARFLLIAIQMTAEAARFRY IQNLVIKNFPNKFNSENKVIQFEVNWKKISTAIYGDAKNGVFNKD YDFGFGKVRQVKDLQMGLLMYLGKPKSSNEANSTVRHYGPLKP TLLIT | 1203 |
| Anti-Viral Protein MAP | APTLETIASLDLNNPTTYLSFITNIRTKVADKTEQCTIQKISKTFTQR YSYIDLIVSSTQKITLAIDMADLYVLGYSDIANNKGRAFFFKDVTE AVANNFFPGATGTNRIKLTFTGSYGDLEKNGGLRKDNPLGIFRLE NSIVNIYGKAGDVKKQAKFFLLAIQMVSEAARFKYISDKIPSEKYE EVTVDEYMTALENNWAKLSTAVYNSKPSTTTATKCQLATSPVTIS PWIFKTVEEIKLVMGLLKSS | 1204 |
| Shiga Toxin A-Chain | KEFTLDFSTAKTYVDSLNVIRSAIGTPLQTISSGGTSLLMIDSGTGD NLFAVDVRGIDPEEGRFNNLRLIVERNNLYVTGFVNRTNNVFYRF ADFSHVTFPGTTAVTLSGDSSYTTLQRVAGISRTGMQINRHSLTTS YLDLMSHSGTSLTQSVARAMLRFVTVTAEALRFRQIQRGFRTTLD DLSGRSYVMTAEDVDLTLNWGRLSSVLPDYHGQDSVRVGRISFG SINAILGSVALILNCHHASRVARMASDEFPSMCPADGRVRGITH NKILWDSSTLGAILMRRTISS | 1205 |
| Shiga-Like Toxin Subunit A (Verotoxin 2) | MKCILFKWVLCLLLGFSSVSYSREFTIDFSTQQSYVSSLNSIRTEIST PLEHISQGTTSVSVINHTPPGSYFAVDIRGLDVYQARFDHLRLIIEQ NNLYVAGFVNTATNTFYRFSDFTHISVPGVTTVSMTTDSSYTTLQ RVAALERSGMQISRHSLVSSYLALMEFSGNTMTRDASRAVLRFVT VTAEALRFRQIQREFRQALSETAPVYTMTPGDVDLTLNWGRISNV LPEYRGEDGVRVGRISFNNISAILGTVAVILNCHHQGARSVRAVN EESQPECQITGDRPVIKINNTLWESNTAAAFLNRKSQFLYTTGK | 1206 |

(continued)

| Toxin | Sequence | SEQ ID |
|---|---|---|
| Trichosanthin | DVSFRLSGATSSSYGVFISNLRKALPNERKLYDIPLLRSSLPGSQRY ALIHLTNYADETISVAIDVTNVYIMGYRAGDTSYFFNEASATEAA KYVFKDAMRKVTLPYSGNYERLQTAAGKIRENIPLGLPALDSAIT TLFYYNANSAASALMVLIQSTSEAARYKFIEQQIGKRVDKTFLPSL AIISLENSWSALSKQIQIASTNNGQFESPVVLINAQNQRVTITNVDA GVVTSNIALLLNRNNMA | 1207 |

### (a) DNA cleaving agents

Examples of DNA cleaving agents suitable for inclusion as the cell toxin in the chimeric ligand-toxin used in practicing the methods include, but are not limited to, anthraquinone-oligopyrrol-carboxamide, benzimidazole, leinamycin; dynemicin A; enediyne; as well as biologically active analogs or derivatives thereof (i.e., those having a substantially equivalent biological activity). Known analogs and derivatives are disclosed, for examples in Islam et al., J. Med. Chem. 34 2954-61, 1991; Skibo et al., J. Med. Chem. 37:78-92, 1994; Behroozi et al., Biochemistry 35:1568-74, 1996; Helissey et al., Anticancer Drug Res. 11:527-51, 1996; Unno et al., Chem. Pharm. Bull. 45:125-33, 1997; Unno et al., Bioorg. Med. Chem., 5:903-19, 1997; Unno et al., Bioorg. Med. Chem., 5: 883-901, 1997; and Xu et al., Biochemistry 37:1890-7, 1998). Other examples include, but are not limited to, endiyne quinone imines (U. S. Patent No. 5,622, 958); 2,2r-bis (2-aminoethyl)-4-4'-bithiazole (Lee et al., Biochem. Mol. Biol. Int. 40:151-7, 1996); epilliticine-salen.copper conjugates (Routier et al., Bioconjug. Chem., 8: 789-92, 1997).

### (b) Antimetabolites

Examples of antimetabolites useful for inclusion as the cell toxin in the chimeric ligand-toxin include, but are not limited to, 5-fluorouracil, methotrexate, melphalan, daunomycin, doxorubicin, nitrogen mustard and mitomycin c.

### (c) Proteinaceous cell toxins

Examples of proteinaceous cell toxins useful for incorporation into the chimeric ligand-toxins used in the methods include, but are not limited to, type one and type two ribosome inactivating proteins (RIP). Useful type one plant RIPs include, but are not limited to, dianthin 30, dianthin 32, lychnin, saporins 1-9, pokeweed activated protein (PAP), PAP II, PAP-R, PAP-S, PAP-C, mapalmin, dodecandrin, bryodin-L, bryodin, Colicin 1 and 2, luffin-A, luffin-B, luffin-S, 19K-protein synthesis inhibitory protein (PSI), 15K-PSI, 9K-PSI, alpha-kirilowin, beta-kirilowin, gelonin, momordin, momordin-II, momordin-Ic, MAP-30, alpha-momorcharin, beta-momorcharin, trichosanthin, TAP-29, trichokirin; barley RIP; flax RIP, tritin, corn RIP, Asparin 1 and 2 (Stirpe et al., Bio/Technology 10:405-12, 1992). Useful type two RIPs include, but are not limited to, volkensin, ricin, nigrin-b, CIP-29, abrin, modeccin, ebulitin-$\alpha$, ebulitin-$\beta$, ebulitin-$\gamma$, vircumin, porrectin, as well as the biologically active enzymatic subunits thereof (Stirpe et al., Bio/Technology 10:405-12, 1992; Pastan et al., Annu. Rev. Biochem. 61:331-54; Brinkmann and Pastan, Biochim. et Biophys. Acta 1198:27-45, 1994; and Sandvig and Van Deurs, Physiol. Rev. 76:949-66, 1996).

### (d) Bacterial toxins

Examples of bacterial toxins useful as cell toxins include, but are not limited to, shiga toxin and shiga-like toxins (i.e,. toxins that have the same activity or structure), as well as the catalytic subunits and biologically functional fragments thereof. These bacterial toxins also are type two RIPs (Sandvig and Van Deurs, Physiol. Rev. 76:949-66, 1996; Armstrong, J. Infect. Dis., 171:1042-5, 1995; Kim et al., Microbiol. Immunol. 41:805-8, 1997, and Skinner et al., Microb. Pathog. 24:117-22, 1998). Additional examples of useful bacterial toxins include, but are not limited to, *Pseudomonas* exotoxin and *Diphtheria* toxin (Pastan et al., Annu. Rev. Biochem. 61:331-54; and Brinkmann and Pastan, Biochim. et Biophys. Acta 1198:27-45, 1994). Truncated forms and mutants of the toxin enzymatic subunits also can be used as a cell toxin moiety (Pastan et al., Annu. Rev. Biochem. 61:331-54; Brinkmann and Pastan, Biochim. et Biophys. Acta 1198:27-45, 1994; Mesri et al., J. Biol. Chem. 268:4853-62, 1993; Skinner et al., Microb. Pathog. 24:117-22, 1998; and U.S. Patent No. 5,082,927). Other targeted agents include, but are not limited to the more than 34 described Colicin family of RNase toxins which include colicins A, B, D, E1-9, cloacin DF13 and the fungal RNase, $\alpha$-sarcin (Ogawa et al. Science 283: 2097-100, 1999; Smarda et al., Folia Microbiol (Praha) 43:563-82, 1998; Wool et al., Trends Biochem. Sci., 17: 266-69, 1992).

### (e) Porphyrins and other light activated toxins

Porphyrins are well known light activatable toxins that can be readily crosslinked to proteins (see, *e.g.,* U.S. Patent No. 5,257,970; U.S. Patent No. 5,252,720; U.S. Patent No. 5,238,940; U.S. Patent No. 5,192,788; U.S. Patent No. 5,171,749; U.S. Patent No. 5,149,708; U.S. Patent No. 5,202,317; U.S. Patent No. 5,217,966; U.S. Patent No. 5,053,423; U.S. Patent No. 5,109,016; U.S. Patent No. 5,087,636; U.S. Patent No. 5,028,594; U.S. Patent No. 5,093,349; U.S. Patent No. 4,968,715; U.S. Patent No. 4,920,143 and International Application WO 93/02192).

### iv. Nucleic acids for targeted delivery

The conjugates provided herein also can be used to deliver nucleic acids to targeted cells. The nucleic acids include DNA intended to modify the genome of a cell and thereby effect genetic therapy, and DNA and RNA for use as antisense agents. The nucleic acids include antisense RNA, DNA, ribozymes and other oligonucleotides that are intended to be used as antisense agents. The nucleic acids can also include RNA trafficking signals, such as viral packaging sequences (see, *e.g.,* Sullenger et al. (1994) Science 262:1566-1569). The nucleic acids also include DNA molecules that encode intact genes or that encode proteins intended to be used in gene therapy.

DNA (or RNA) that may be delivered to a cell to effect genetic therapy includes DNA that encodes tumor-specific cytotoxic molecules, such as tumor necrosis factor, viral antigens and other proteins to render a cell susceptible to anticancer agents, and DNA encoding genes, such as the defective gene (CFTR) associated with cystic fibrosis (see, *e.g.*, International Application WO 93/03709, which is based on U.S. Application Serial No. 07/745,900; and Riordan et al. (1989) Science 245:1066-1073), to replace defective genes.

Nucleic acids and oligonucleotides for use as described herein can be synthesized by any method known to those of skill in this art (see, *e.g.,* WO 93/01286, which is based on U.S. Application Serial No. 07/723,454; U.S.. Patent No. 5,218,088; U.S. Patent No. 5,175,269; U.S. Patent No. 5,109,124). Identification of oligonucleotides and ribozymes for use as antisense agents is well within the skill in this art. Selection of DNA encoding genes for targeted delivery for genetic therapy also is well within the level of skill of those in this art. For example, the desirable properties, lengths and other characteristics of such oligonucleotides are well known. Antisense oligonucleotides are designed to resist degradation by endogenous nucleolytic enzymes and include, but are not limited to: phosphorothioate, methylphosphonate, sulfone, sulfate, ketyl, phosphorodithioate, phosphoramidate, phosphate esters, and other such linkages (see, *e.g.,* Agrawal et al. (1987) Tetrahedron Lett. 28:3539-3542; Miller et al. (1971) J. Am. Chem. Soc. 93:6657-6665; Stec et al. (1985) Tetrahedron Lett. 26:2191-2194; Moody et al. (1989) Nucl. Acids Res. 17:4769-4782; Letsinger et al. (1984) Tetrahedron 40:137-143; Eckstein (1985) Annu. Rev. Biochem. 54:367-402; Eckstein (1989) Trends Biochem. Sci. 14:97-100; Stein (1989) In: Oligodeoxynucleotides. Antisense Inhibitors of Gene Expression, Cohen, ed, Macmillan Press, London, pp. 97-117; Jager et al. (1988) Biochemistry 27:7237-7246).

### (a) Antisense nucleotides, including: antisense oligonucleotides; triplex molecules; dumbbell oligonucleotides; DNA; extracellular protein binding oligonucleotides; and small nucleotide molecules

Antisense nucleotides are oligonucleotides that specifically bind to mRNA that has complementary sequences, thereby preventing translation of the mRNA (see, *e.g.,* U.S. Patent No. 5,168,053 to Altman et al. U.S. Patent No. 5,190,931 to Inouye, U.S. Patent No. 5,135,917 to Burch; U.S. Patent No. 5,087,617 to Smith and Clusel et al. (1993) Nucl. Acids Res. 21:3405-3411, which describes dumbbell antisense oligonucleotides). Triplex molecules refer to single DNA strands that target duplex DNA and thereby prevent transcription (see, *e.g.,* U.S. Patent No. 5,176,996 to Hogan et al. which describes methods for making synthetic oligonucleotides that bind to target sites on duplex DNA).

### (b) Ribozymes

Ribozymes are RNA constructs that specifically cleave messenger RNA. There are at least five classes of ribozymes that are known that are involved in the cleavage and/or ligation of RNA chains. Ribozymes can be targeted to any RNA transcript and can catalytically cleave such transcript (see, *e.g.,* U.S. Patent No. 5,272,262; U.S. Patent No. 5,144,019; and U.S. Patent Nos. 5,168,053, 5,180,818, 5,116,742 and 5,093,246 to Cech et al. which described ribozymes and methods for production thereof). Any such ribosome may be linked to a conditionally active anti-EGFR antibody for delivery to EGFR bearing cells under acidic conditions.

The ribozymes may be delivered to the targeted cells as DNA encoding the ribozyme linked to a eukaryotic promoter, such as a eukaryotic viral promoter, generally a late promoter, such that upon introduction into the nucleus, the ribozyme will be directly transcribed. In such instances, the construct will also include a nuclear translocation sequence, generally as part of the targeting agent or as part of a linker in order to render it suitable for delivering linked nucleic acids to the nucleus.

**(c) Nucleic acids encoding therapeutic products for targeted delivery**

Among the DNA that encodes therapeutic products contemplated for use is DNA encoding correct copies anticancer agents, such as tumor necrosis factors, and cytotoxic agents, such as shiga A1 toxin or saporin to EGFR bearing tumor cells. The conjugate should include a nuclear translocation sequence (NTS). If the conjugate is designed such that the targeting agent and linked DNA is cleaved in the cytoplasm, then the NTS should be included in a portion of the linker that remains bound to the DNA, so that, upon internalization, the conjugate will be trafficked to the nucleus. The nuclear translocation sequence (NTS) may be a heterologous sequence or a may be derived from the selected chemokine receptor targeting agent. A typical consensus NTS sequence contains an amino-terminal proline or glycine followed by at least three basic residues in an array of seven to nine amino acids (see, *e.g.,* Dang et al. (1989) J. Biol. Chem. 264:18019-18023, Dang et al. (1988) Mol. Cell. Biol. 8:4048-4054).

**(d) Coupling of nucleic acids to proteins**

To effect chemical conjugation herein, the targeting agent is linked to the nucleic acid either directly or via one or more linkers. Methods for conjugating nucleic acids, at the 5' ends, 3' ends and elsewhere, to the amino and carboxyl termini and other sites in proteins are known to those of skill in the art (for a review see *e.g.,* Goodchild, (1993) In: Perspectives in Bioconjugate Chemistry, Mears, Ed., American Chemical Society, Washington, D.C. pp. 77-99). For example, proteins have been linked to nucleic acids using ultraviolet irradiation (Sperling et al. (1978) Nucleic Acids Res. 5:2755-2773; Fiser et al. (1975) FEBS Lett. 52:281-283), bifunctional chemicals (Bäumert et al. (1978) Eur. J. Biochem. 89:353-359; and Oste et al. (1979) Mol. Gen. Genet. 168:81-86), and photochemical cross-linking (Vanin et al. (1981) FEBS Lett. 124:89-92; Rinke et al. (1980) J.Mol.Biol. 137:301-304; Millon et al. (1980) Eur. J. Biochem. 110:485-492).
In particular, the reagents (N-acetyl-N'-(p-glyoxylylbenzolyl)cystamine and 2-iminothiolane have been used to couple DNA to proteins, such as $\alpha_2$macroglobulin ($\alpha_2$M) via mixed disulfide formation (see, Cheng et al. (1983) Nucleic Acids Res. 11:659-669). N-acetyl-N'-(p-glyoxylylbenzolyl)cystamine reacts specifically with non-paired guanine residues and, upon reduction, generates a free sulfhydryl group. 2-Iminothiolane reacts with proteins to generate sulfhydryl groups that are then conjugated to the derivatized DNA by an intermolecular disulfide interchange reaction. Any linkage may be used provided that, upon internalization of the conjugate the targeted nucleic acid is active. Thus, it is expected that cleavage of the linkage may be necessary, although it is contemplated that for some reagents, such as DNA encoding ribozymes linked to promoters or DNA encoding therapeutic agents for delivery to the nucleus, such cleavage may not be necessary.
Thiol linkages readily can be formed using heterobifunctional reagents. Amines have also been attached to the terminal 5' phosphate of unprotected oligonucleotides or nucleic acids in aqueous solutions by reacting the nucleic acid with a water-soluble carbodiimide, such as 1-ethyl-3'[3-dimethylaminopropyl]carbodiimide (EDC) or N-ethyl-N'(3-dimethylaminopropylcarbodiimidehydrochloride (EDCI), in imidazole buffer at pH 6 to produce the 5'phosphorimidazolide. Contacting the 5'phosphorimidazolide with amine-containing molecules and ethylenediamine, results in stable phosphoramidates (see, *e.g.,* Chu et al. (1983) Nucleic Acids Res. 11:6513-6529; and WO 88/05077 in which the U.S. is designated). In particular, a solution of DNA is saturated with EDC, at pH 6 and incubated with agitation at 4° C overnight. The resulting solution is then buffered to pH 8.5 by adding, for example about 3 volumes of 100 mM citrate buffer, and adding about 5 $\mu$g - about 20 $\mu$g of a chemokine receptor targeting agent, and agitating the resulting mixture at 4 °C for about 48 hours. The unreacted protein may be removed from the mixture by column chromatography using, for example, SEPHADEX G75 (Pharmacia) using 0.1 M ammonium carbonate solution, pH 7.0 as an eluting buffer. The isolated conjugate may be lyophilized and stored until used.
U.S. Patent No. 5,237,016 provides methods for preparing nucleotides that are bromacetylated at their 5' termini and reacting the resulting oligonucleotides with thiol groups. Oligonucleotides derivatized at their 5'-termini bromoacetyl groups can be prepared by reacting 5'-aminohexyl-phosphoramidate oligonucleotides with bromoacetic acid-N-hydroxysuccinimide ester as described in U.S. Patent No. 5,237,016. U.S. Patent No. 5,237,016 also describes methods for preparing thiol-derivatized nucleotides, which can then be reacted with thiol groups on the selected growth factor. Briefly, thiol-derivatized nucleotides are prepared using a 5'-phosphorylated nucleotide in two steps: (1) reaction of the phosphate group with imidazole in the presence of a diimide and displacement of the imidazole leaving group with cystamine in one reaction step; and reduction of the disulfide bond of the cystamine linker with dithiothreitol (see, also, Chu et al. (1988) Nucl. Acids Res. 16:3671-3691, which describes a similar procedure). The 5'-phosphorylated starting oligonucleotides can be prepared by methods known to those of skill in the art (see, *e.g.,* Maniatis et al. (1982) Molecular Cloning: A Laboratory Manual, Cold Spring Harbor Laboratory, New York, p. 122).

The antisense oligomer or nucleic acid, such as a methylphosphonate oligonucleotide (MP-oligomer), may be derivatized by reaction with SPDP or SMPB. The resulting MP-oligomer may be purified by HPLC and then coupled to the chemokine receptor targeting agent. The MP-oligomer (about 0.1 μM) is dissolved in about 40-50 μl of 1:1 acetonitrile/water to which phosphate buffer (pH 7.5, final concentration 0.1 M) and a 1 mg MP-oligomer in about 1 ml phosphate buffered saline is added. The reaction is allowed to proceed for about 5-10 hours at room temperature and is then quenched with about 15 μL 0.1 iodoacetamide. The conjugates can be purified on heparin sepharose Hi Trap columns (1 ml, Pharmacia) and eluted with a linear or step gradient. The conjugate should elute in 0.6 M NaCl.

## b. Linkers

The linker, L, attaches the antibody to a targeted agent through covalent bond(s). The linker is a bifunctional or multifunctional moiety which can be used to link one or more targeted agent(s) to the anti-EGFR antibody to form an antibody-drug conjugate (ADC). ADCs can be readily prepared using a linker having reactive functionality for binding to the targeted agent and to the anti-EGFR antibody. A cysteine thiol group, or an amine group, *e.g.*, N-terminus or lysine side chain, of the anti-EGFR antibody can form a bond with a functional group of a linker reagent, targeted agent or targeted agent-linker reagent.

Linkers are preferably stable in the extracellular environment so that the antibody-drug conjugate (ADC) is stable and remains intact, *i.e.,* the antibody remains linked to the targeted agent, before transport or delivery into the target cell. Thus, the linkers are stable outside the target cell and may be cleaved or enable dissociation of the antibody and targeted agent at some efficacious rate once inside the cell. Contemplated linkers will (i) not interfere with the specific binding properties of the antibody; (ii) permit intracellular delivery of the conjugate or targeted agent; (iii) remain stable and intact, *i.e.,* not cleaved, until the conjugate has been delivered or transported to its targeted site; and (iv) not interfere with the cytotoxic, cell-killing effect or a cytostatic effect of the targeted agent. Stability of the ADC may be measured by standard analytical techniques such as mass spectrometry and/or HPLC.

Linkers have two reactive functional groups to permit covalent attachment to both the antibody and the targeted agent, and thus exhibit bivalency in a reactive sense. Such chemical cross-linking reagents, which are useful for attaching two or more functional or biologically active moieties, such as peptides, nucleic acids, drugs, toxins, antibodies, haptens, and reporter groups, are known, and methods have been described for their use in generating conjugates (Hermanson, G. T. (1996) Bioconjugate Techniques; Academic Press: New York, p234-242).

In some examples, a linker has a reactive functional group which has a nucleophilic group that is reactive to an electrophilic group present on an antibody. Useful electrophilic groups on an antibody include, but are not limited to, aldehyde and ketone carbonyl groups. The heteroatom of a nucleophilic group of a linker can react with an electrophilic group on an antibody and form a covalent bond to an antibody unit. Useful nucleophilic groups on a linker include, but are not limited to, hydrazide, oxime, amino, hydrazine, thiosemicarbazone, hydrazine carboxylate, and arylhydrazide. The electrophilic group on an antibody provides a convenient site for attachment to a linker.

### i. Peptide Linkers

Linkers can be peptidic, comprising one or more amino acid units. Peptide linker reagents may be prepared by solid phase or liquid phase synthesis methods (E. Schroder and K. Lubke, The Peptides, volume 1, pp 76-136 (1965) Academic Press) that are well known in the field of peptide chemistry, including t-BOC chemistry (Geiser et al. "Automation of solid-phase peptide synthesis" in Macromolecular Sequencing and Synthesis, Alan R. Liss, Inc., 1988, pp. 199-218) and Fmoc/HBTU chemistry (Fields, G. and Noble, R. (1990) "Solid phase peptide synthesis utilizing 9-fluorenylmethoxycarbonyl amino acids", Int. J. Peptide Protein Res. 35:161-214), on an automated synthesizer such as the Rainin Symphony Peptide Synthesizer (Protein Technologies, Inc.), or Model 433 (Applied Biosystems). Peptide-based linkers offer advantages over linkers that are hydrolytically or reductively labile, since proteolysis is enzymatic, and the enzymes can be selected for preferential expression within tumor cells. The cathepsin B-cleavable peptide linker, valine-citrulline (Val-Cit), and modifications thereof such as maleimidocaproyl-valine-citrulline (mc-vc), phenylalanine-lysine, Ala-Leu-Ala-Ala (SEQ ID NO: 1201), other tri/tetrapeptides are exemplary peptide linkers that have been employed in ADCs (Dosio et al., (2010) Toxins 3:848-883; Doronina et al., (2006) Bioconjug Chem. 17:114-124; Doronina et al., (2003) Nat Biotechnol. 21:778-784; Sanderson et al., (2005) Clin Cancer Res 11:843-852; Ducry and Stump (2010) Bioconjug Chem. 21:5-13). Exemplary non-cleavable peptide linkers include N-methyl-valine-citrulline. Other peptide linkers are described in U.S. Publication No. 2011/0020343.

Preferred peptide linkers are those that can be incorporated in fusion proteins and expressed in a host cell, such as *E. coli.* Such linkers include: enzyme substrates, such as cathepsin B substrate, cathepsin D substrate, trypsin substrate, thrombin substrate, subtilisin substrate, Factor Xa substrate, and enterokinase substrate; linkers that increase solubility, flexibility, and/or intracellular cleavability include linkers, such as $(gly_m ser)_n$ and $(ser_m gly)_n$, where m is 1 to 6, preferably 1 to 4, more preferably 2 to 4, and n is 1 to 6, preferably 1 to 4, more preferably 2 to 4 (see, *e.g.*, International PCT application No. WO 96/06641, which provides exemplary linkers for use in conjugates). In some embodiments, several linkers may be included in order to take advantage of desired properties of each linker.

### ii. Chemical Linkers

ADCs also can be prepared using linkers that are non-cleavable moieties or chemical cross-linking reagents. Exemplary non-cleavable linkers include amide linkers and amide and ester linkages with succinate spacers (Dosio et al., (2010) Toxins 3:848-883). Exemplary chemical cross-linking linkers include, but are not limited to, SMCC (Succinimidyl-4-(N-maleimidomethyl)cyclohexane-1-carboxylate) and SIAB (Succinimidyl (4-iodoacetyl)aminobenzoate). SMCC is an amine-to-sulfhydryl crosslinker that contains NHS-ester and maleimide reactive groups at opposite ends of a medium-length cyclohexane-stabilized spacer arm. SIAB is a short, NHS-ester and iodoacetyl crosslinker for amine-to-sulfhydryl conjugation. Other exemplary cross-linking reagents include, but are not limited to, thioether linkers, chemically labile hydrazone linkers, 4-mercaptovaleric acid, BMPEO, BMPS, EMCS, GMBS, HBVS, LC-SMCC, MBS, MPBH, SBAP, SIA, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate), and bis-maleimide reagents, such as DTME, BMB, BMDB, BMH, BMOE, BM(PEO)$_3$, and BM(PEO)$_4$, which are commercially available (Pierce Biotechnology, Inc.) Bis-maleimide reagents allow the attachment of a free thiol group of a cysteine residue of an antibody to a thiol-containing targeted agent, or linker intermediate, in a sequential or concurrent fashion. Other thiol-reactive functional groups besides maleimide, include iodoacetamide, bromoacetamide, vinyl pyridine, disulfide, pyridyl disulfide, isocyanate, and isothiocyanate. Other exemplary linkers and methods of use are described in U.S. Publication No. 2005/0276812 and Ducry and Stump (2010) Bioconjug Chem. 21:5-13.

Linkers optionally can be substituted with groups which modulate solubility or reactivity. For example, a sulfonate substituent may increase water solubility of the reagent and facilitate the coupling reaction of the linker reagent with the antibody or the drug moiety, or facilitate the coupling reaction of the anti-EGFR Ab-L with the targeted agent, or targeted agent-L with the anti-EGFR Ab, depending on the synthetic route employed to prepare the ADC.

Other linker reagents can also be obtained via commercial sources, such as Molecular Biosciences Inc. (Boulder, Colo.), or synthesized in accordance with procedures described in Toki et al. (2002) J. Org. Chem. 67:1866-1872; U.S. Pat. No. 6,214,345; WO 02/088172; U.S. 2003130189; U.S. 2003096743; WO 03/026577; WO 03/043583; and WO 04/032828. For example, linker reagents such as DOTA-maleimide (4-maleimidobutyramidobenzyl-DOTA) can be prepared by the reaction of aminobenzyl-DOTA with 4-maleimidobutyric acid (Fluka) activated with isopropylchloroformate (Aldrich), following the procedure of Axworthy et al. (2000) Proc. Natl. Acad. Sci. USA 97(4):1802-1807). DOTA-maleimide reagents react with the free cysteine amino acids of the cysteine engineered antibodies and provide a metal complexing ligand on the antibody (Lewis et al. (1998) Bioconj. Chem. 9:72-86). Chelating linker labelling reagents such as DOTA-NHS (1,4,7,10-tetraazacyclododecane-1,4,7,10-tetraacetic acid mono (N-hydroxysuccinimide ester) are commercially available (Macrocyclics, Dallas, Tex.).

The Linker may be a dendritic type linker for covalent attachment of more than one drug moiety through a branching, multifunctional linker moiety to an antibody (Sun et al. (2002) Bioorganic & Medicinal Chemistry Letters 12:2213-2215; Sun et al. (2003) Bioorganic & Medicinal Chemistry 11:1761-1768; King et al. (2002) Tetrahedron Letters 43:1987-1990). Dendritic linkers can increase the molar ratio of targeted agent to antibody, i.e., loading, which can increase the potency of the ADC. Thus, where an antibody bears only one reactive cysteine thiol group, a multitude of drug moieties may be attached through a dendritic linker. Exemplary dendritic linker reagents are described in U.S. Patent Publication No. 2005/0276812.

### D. METHODS FOR IDENTIFYING AND ASSESSING ANTI-EGFR ANTIBODY PROPERTIES AND ACTIVITIES

Anti-EGFR antibodies provided herein are selected based on exhibiting selective, and hence conditional, activity in a tumor microenvironment compared to a non-tumor microenvironment. Such antibodies can be identified by screening methods or other methods that compare the activity of an antibody or a collection of antibodies under two different conditions that simulate or reflect conditions that exist in a tumor microenvironment or non-tumor microenvironment. Identified antibodies, or antigen-binding fragments thereof, can be further characterized in a variety of assays known to one of skill in the art to assess clinical properties such as, for example, therapeutic efficacy, affinity for EGFR, toxicity, side effects, pharmacokinetics and pharmacodynamics.

As described herein, the differences in conditions that characterize solid tumors, such as low pH and hypoxia, can be leveraged to provide antibodies that are more active in the diseased microenvironment of the tumor. In performing such assays or methods, it is also found that the concentration of other proteins is a condition that affects or influences selection and conditional activity, and hence it is a parameter used in the screening assays. For example, as shown in Example 3, compared to the absence of added protein, the presence of added protein increases the ratio of activity or conditional activity of selected antibodies and this difference is greater at physiological concentrations of protein (e.g.

25% human serum). In an *in vivo* or physiological environment, the interstitial protein concentration (such as albumin) is anywhere from 20-50% of plasma. Serum contains about 60-80 g/L protein, and various tissues have been demonstrated to contain 12 mg/mL to 40 mg/mL interstitial protein (*see e.g.* Aukland and Reed (1993) Physiological Reviews, 73:1-78). Hence, in order to simulate these environments, assays and methods to select or characterize anti-EGFR antibodies are performed in the presence of 10 mg/mL to 50 mg/mL protein, which, for example, can be provided in serum, such as human serum, or as a serum albumin, such as human serum albumin, or other protein that does not interact with the antibody or receptor or otherwise directly alter antibody-receptor interactions. In some examples, assays and methods to select or characterize anti-EGFR antibodies are performed in the presence of 12-40 mg/mL protein, such as at least 12 mg/mL, 15 mg/mL, 20 mg/mL, 25 mg/mL, 30 mg/mL, 35 mg/mL or 40 mg/mL protein. In other examples, the protein is provided in serum, and assays and methods to select or characterize anti-EGFR antibodies are performed in the presence of 20% to 50% serum (vol/vol), such as 20% to 50% human serum, such as at least 20%, 25%, 30%, 35%, 40%, 45% or 50% serum (vol/vol).

In particular, the conditional activity of an anti-EGFR can be determined by performing an assay in a dual format, whereby each assay is performed twice, under different conditions, such as different pH and/or lactate concentrations, and in the presence of physiological concentrations of total protein. Thus, methods of assessing or selecting anti-EGFR antibodies that are conditionally active in a tumor microenvironment include any assay or method that assesses an activity under a first set of conditions (e.g. conditions that exist in a tumor microenvironment) that includes 20-50% serum (vol/vol) or 10-50 mg/mL protein (e.g. serum albumin), and an acidic pH of about between 5.8 to 6.8 and/or elevated lactate levels of 10 mM to 20 mM. For example, the first set of conditions can include at least 25% serum (vol/vol) or 12-40 mg/mL protein (e.g. serum albumin), and an acidic pH of about between 6.0 to 6.5 and/or elevated lactate levels of 15 mM to 20 mM. In such methods, the anti-EGFR antibody also is assessed for activity under a second set of conditions that includes 20-50% serum (vol/vol) or 10-50 mg/mL protein (e.g. serum albumin), and near neutral pH or neutral pH of about between 7.0 to 7.4 and/or a lactate concentration of 0.5 to 5 mM. For example, the second set of conditions includes at least 25% serum (vol/vol) or 12-40 mg/mL protein (e.g. serum albumin), and pH about between 7.0 to 7.2 and/or lactate concentration of 1 mM to 4 mM. In such examples, the amount of added protein to simulate a physiologic environment (e.g. serum protein) is typically the same or substantially the same for both sets of conditions, but can vary by ±25% or less from one condition to the other.

Anti-EGFR antibodies, or antigen-binding fragments thereof, that exhibit greater activity under the first set of conditions compared to under the second set of conditions are selected as anti-EGFR antibodies that are conditionally active or selective for a tumor microenvironment. For example, anti-EGFR antibodies, or antigen-binding fragments thereof, are selected that exhibit a ratio of activity under the first set of conditions compared to the second set of conditions of at least 1.1, 1.2, 1.3, 1.4, 1.5, 1.6, 1.7, 1.8, 1.9, 2.0, 2.5, 3.0, 4.0, 5.0, 6.0, 7.0, 8.0, 9.0, 10.0, 15.0, 20.0, 25.0, 30.0, 35.0, 40.0, 45.0, 50.0 or more. Typically, for use as a selective therapeutic for a tumor microenvironment, the anti-EGFR antibody, or antigen-binding fragment thereof, is one that exhibits at least a ratio of activity under the first set of conditions (e.g. conditions that exist in a tumor microenvironment) compared to the second set of conditions (e.g. conditions that exist in a non-tumor microenvironment) of at least 3.0 or more.

Anti-EGFR antibodies specific for EGFR that can be screened and/or assessed for conditional activity in a tumor microenvironment as described herein include any antibody that is specific for EGFR. Such antibodies can be made using hybridoma methods, for example, by immunizing an appropriate host animal or immunizing lymphocytes in vitro followed by fusion with myeloma cells to produce hybridomas (e.g. Kohler et al. (1975) Nature, 256:495, Goding, Monoclonal Antibodies: Principles and Practice, pp. 59-103 (Academic Press, 1986)). For example, antibodies can be immunized with EGFR-expressing cells, EGFR-derived peptides or other antigen. The antigen can be provided with a carrier to enhances its immunogenicity, can be provided and administered as formulations with adjuvants and/or can be administered in multiple injections. Antibodies also can be made by recombinant DNA methods (e.g. U.S. Patent No. 4,816,567).

Anti-EGFR antibodies also include modified anti-EGFR antibodies. Modified anti-EGFR antibody can be derived from any known anti-EGFR antibody, or antigen-binding fragment thereof. For example, exemplary anti-EGFR antibodies include, for example, Erbitux® (cetuximab, C225 or IMC-C225), Hu225, 11F8 by Zhu (WO 2005/090407), EMD 72000 (matuzumab), Vectibix™ (panitumumab; ABX-EGF), TheraCIM (nimotuzumab), and Hu-Max-EGFR (zalutumumab). Libraries or collections of mutant or variant forms of such antibodies can be generated by methods known in the art to introduce amino acid replacements, additions or deletions in a reference unmodified antibody. It is within the level of one of skill in the art to generate modified or variant proteins for use in the methods herein. Methods of mutagenesis are well known in the art and include, for example, site-directed mutagenesis such as for example QuikChange (Stratagene) or saturation mutagenesis. Mutagenesis methods include, but are not limited to, site-mediated mutagenesis, PCR mutagenesis, cassette mutagenesis, site-directed mutagenesis, random point mutagenesis, mutagenesis using uracil containing templates, oligonucleotide-directed mutagenesis, phosphorothioate-modified DNA mutagenesis, mutagenesis using gapped duplex DNA, point mismatch repair, mutagenesis using repair-deficient host strains, restriction-selection and restriction-purification, deletion mutagenesis, mutagenesis by total gene synthesis, double-strand break repair, and many others known to persons of skill. In the methods herein, mutagenesis can be effected across the full length of a protein or within a region of a protein. The mutations can be made rationally or randomly. In some examples of generated collections or libraries, each amino acid that is replaced is independently replaced by 19 of the remaining amino acids or by less than 19 of the remaining amino acids, such as 10, 11, 12, 13, 14, 15, 16, 17 or 18 of the remaining amino acids at each position or a subset of positions.

A full-length or antigen-binding fragment of an antibody can be assessed or screened for conditional activity as described herein. Hence, the antibody can be any form of an antibody so long as it minimally contains a sufficient portion of the variable heavy chain and a sufficient portion of the variable light chain to immunospecifically bind EGFR. In some examples, a fragment or variant of an anti-EGFR antibody, such as a modified anti-EGFR antibody, can be used in the assays provided herein, such as, for example, any variant or fragment described herein or known in the art.

In addition, *in vitro* assays and *in vivo* animal models, such as those provided herein, can be employed for measuring the activity and/or side effects of the modified anti-EGFR antibodies. The assays provided herein include any assays that can test or assess an activity of an anti-EGFR antibody, such as a modified anti-EGFR antibody, in a detectable or otherwise measurable manner. The assays provided herein can be developed in a high throughput format in order to assess an activity of numerous anti-EGFR antibodies, for example protein variants, at one time in dual format.

Such assays can be performed in vitro or in vivo. The activity assessed can be any activity of an anti-EGFR antibody, such as binding to EGFR, cell growth inhibition (CGI) activity or tumor growth inhibition activity. For example, in vitro binding assays can be performed using solid-support binding assays or solution binding assays, where the binding is performed under the above conditions. In other examples, binding assays can be performed in vivo where binding is compared on cells present in a tumor versus cells present in a non-tumor. In particular, an in vivo binding assay can be performed by assessing binding or localization of administered antibody to tumor cells versus basal skin keratinocytes. This is exemplified herein using xenograft or skin graft models. Other models also can be employed.

Provided herein are exemplary assays. The assays are not meant to be limiting. Any assay known to one of skill in the art is contemplated for use in the methods provided herein to identify, select or characterize anti-EGFR antibodies, including assays that detect binding, functional assays, *in vivo* assays, animal models and clinical assays. Descriptions of exemplary assays are provided below.

### 1. Binding Assays

For example, the anti-EGFR antibodies, such as modified anti-EGFR antibodies, can be assayed for the ability to bind to EGFR. The anti-EGFR antibodies provided herein can be assessed for their ability to bind EGFR by any method known to one of skill in the art. Exemplary assays are described herein below. In some examples, a fragment or variant of EGFR can be used in the assays provided herein. For example, EGFR can be expressed as a soluble protein. For example, a soluble EGFR that can be used in the assays described herein is the soluble EGF receptor extracellular domain (sECD).

Binding assays can be performed in solution, suspension or on a solid support. For example, EGFR can be immobilized to a solid support (*e.g.* a carbon or plastic surface, a tissue culture dish or chip) and contacted with antibody. Unbound antibody or target protein can be washed away and bound complexes can then be detected. Binding assays can be performed under conditions to reduce nonspecific binding, such as by using a high ionic strength buffer (*e.g.* 0.3-0.4 M NaCl) with nonionic detergent (*e.g.* 0.1 % Triton X-100 or Tween 20) and/or blocking proteins (*e.g.* bovine serum albumin or gelatin). Negative controls also can be included in such assays as a measure of background binding. Binding affinities can be determined using Scatchard analysis (Munson et al., (1980) Anal. Biochem., 107:220), surface plasmon resonance, isothermal calorimetry, quantitative ELISA or other methods known to one of skill in the art (*e.g.,* Liliom et al. (1991) J. Immunol Methods. 143(1):119-25).

The assays described herein include dual assay comparative methods, whereby binding is determined under two different binding conditions. Non-limiting examples of different binding conditions include, for example, pH, such as low pH (e.g., pH 6.0 or pH 6.5) compared to neutral pH (e.g., pH 7.4), or lactate concentrations, such as high lactate concentrations (10-20 mM) compared to low lactate concentrations (0-5 mM). Protein concentrations that include 20-50% serum (vol/vol) or 10-50 mg/mL protein (e.g. serum albumin) also can be included. Any of the steps of the assays described herein can be performed under dual conditions to simulate two different binding conditions. For example, where the assay is an ELISA, any of the steps of an ELISA, such as coating, blocking, incubation with test molecule (e.g. therapeutic antibody or antigen binding fragments or variants thereof), or detection, can be performed under conditions described herein. In the assay, each modified anti-EGFR antibody can be screened individually and separately for binding to its cognate binding partner (e.g. EGFR) under both simulated conditions. The binding activity of the modified anti-EGFR antibody for the cognate binding partner (e.g. EGFR) can be assessed and compared. Examples of assays that measure binding include solution binding assays and solid support binding assays, such as surface plasmon resonance and immunoassays, such as ELISA.

In some examples, the anti-EGFR antibodies provided herein can be assayed for the ability to immunospecifically bind to EGFR at different pH conditions, such as low pH and neutral pH. In some examples, the assays can identify modified anti-EGFR antibodies that have higher activity, for example binding activity, in low pH than at neutral pH. In particular examples herein, binding activity of a modified anti-EGFR antibody or variants thereof to EGFR or a soluble EGFR can be assessed under conditions of low pH (< 7.4) and elevated lactic acid concentrations, and under conditions of physiologic pH of about 7.3 to 7.4 and low lactate concentrations. In addition, human serum (e.g., 5% or 25% human serum) can be included in the binding assay to further mimic the natural environments.

Such assays can be performed, for example, in solution (e.g., Houghten (1992) Bio/Techniques 13:412-421), on beads (Lam (1991) Nature 354:82-84), on chips (Fodor (1993) Nature 364:555-556), on bacteria (U.S. Pat. No. 5,223,409), on spores (U.S. Pat. Nos. 5,571,698; 5,403,484; and 5,223,409), on plasmids (Cull et al. (1992) Proc. Natl. Acad. Sci. USA 89:1865-1869) or on phage (Scott and Smith (1990) Science 249:386-390; Devlin (1990) Science 249:404-406; Cwirla et al. (1990) Proc. Natl. Acad. Sci. USA 87:6378-6382; and Felici (1991) J. Mol. Biol. 222:301-310).

The anti-EGFR antibodies, such as modified anti-EGFR antibodies, can be labeled so that the binding activity can be assessed and determined. For example, to detect binding, the anti-EGFR antibodies, such as modified anti-EGFR antibodies, can be labeled with a detectable moiety or tag in order to facilitate detection. The skilled artisan can select an appropriate detectable moiety or tag for assay conditions. For example, some secondary reagents, such as anti-Ig antibodies cannot be used to detect binding of a modified protein that is an antibody in a solution that contains human serum. In addition, an anti-IgG antibody cannot be used to detect binding of a biomolecule that is an antibody.

Any detectable moiety or other moiety known to one of skill in the art that is capable of being detected or identified can be used. The moiety or tag can be linked to the test molecule, such as a therapeutic protein or antibody, directly or indirectly, for example using a linker. Linkage can be at the N- or C-terminus of the therapeutic antibody. Exemplary tags and moieties that can be used in the method herein, include but are not limited to, any set forth in Table 12.

| Table 12. Exemplary tags and moieties | | | | |
|---|---|---|---|---|
| **Name** | **Sequence** | **# of Residues** | **Size (Da)** | **SEQ ID NO** |
| c-Myc | EQKLISEEDL | 10 | 1200 | 1082 |
| FLAG | DYKDDDDK | 8 | 1012 | 13 |
| His | HHHHHH | 6 | | 12 |
| HA | YPYDVPDYA | 9 | 1102 | 1083 |
| VSV-G | YTDIEMNRLGK | 11 | 1339 | 1084 |
| HSV | QPELAPEDPED | 11 | 1239 | 1085 |
| V5 | GKPIPNPLLGLDST | 14 | 1421 | 1086 |
| Poly Arg | RRRRR | 5-6 | 800 | 1087 |
| Strep-tag-II | WSHPQFEK | 8 | 1200 | 1088 |
| S | KETAAAKFERQHMDS | 15 | 1750 | 1089 |
| 3x FLAG | DYKDHDGDYKDHDIDYKDDDDK | 22 | 2730 | 1090 |
| HAT | KDHLIHNVHKEFHAHAHNK | 19 | 2310 | 1091 |
| SBP | MDEKTTGWRGGHVVEGLAGELEQLRARLEHHP QGQREP | 38 | 4306 | 1092 |

Any linker known to one of skill in the art that is capable of linking the detectable moiety to the therapeutic antibodies described herein can be used. Exemplary linkers include the glycine rich flexible linkers (-G$_4$S-)$_n$, where n is a positive integer, such as 1 (SEQ ID NO:1094), 2 (SEQ ID NO:1095), 3 (SEQ ID NO: 21), 4 (SEQ ID NO: 1096), 5 (SEQ ID NO: 1097), or more.

Binding assays can be performed in solution, by affixing the modified anti-EGFR antibody to a solid support, or by affixing EGFR to a solid support. A description of exemplary assays that can be used to measure binding between the modified anti-EGFR antibodies and EGFR are provided in the subsections that follow.

**a. Solid Support Binding Assays**

The assays to assess activity of the anti-EGFR antibodies, such as modified anti-EGFR antibodies, provided herein include binding assays in which binding of the anti-EGFR antibody to EGFR is measured under conditions in which one or both is attached to a solid support. For example, the anti-EGFR antibody, such as modified anti-EGFR antibody, in solution can interact with EGFR immobilized on a solid support, or EGFR in solution can interact with a modified anti-EGFR antibody immobilized on a solid support. Solid support binding assays can be advantageous compared to solution binding assays because immobilization on the solid phase can facilitate separation of bound anti-EGFR antibody from unbound anti-EGFR antibody. Any solid support binding assay known to the skilled artisan is contemplated for use in the methods provided herein, including surface plasmon resonance, bio-layer interferometry and ELISA.

### i. Surface Plasmon Resonance

Surface Plasmon resonance (SPR) can detect binding of unlabeled molecules in highly sensitive assays by measuring refractive index changes that occur upon molecular binding of analyte molecules in a sample to immobilized molecules (Piliarik et al., (2009) Methods Mol Biol. 503:65-88). SPR occurs when surface plasmon waves, which are collective oscillations of electrons in a metal, are excited at a metal/dielectric interface. SPR reduces reflected light intensity at a specific combination of angle and wavelength. Molecular binding can change the refractive index and thickness of an ultra-thin organic (dielectric) layer on the metal film, which changes the SPR resonance conditions.

In some examples, SPR kinetic analysis can be used to determine the binding on and off rates of a modified anti-EGFR antibody to EGFR (see, *e.g.,* BiaCore 2000, Biacore AB, Upsala, Sweden and GE Healthcare Life Sciences; Malmqvist et al. (1993) Curr Opin Immunol. 5(2):282-6; Garcia-Ojeda et al. (2004) Infect Immun. 72(6):3451-60). SPR kinetic analysis comprises analyzing the binding and dissociation of an antigen from chips with immobilized antibodies on their surface. Using SPR to measure binding of anti-EGFR antibodies to the soluble extracellular domain of EGFR is within the ability of the skilled artisan (e.g., Saxena et al. (2011), J. Clin. Oncol. 29(suppl):e13030).

For example, a solution with one or more anti-EGFR antibodies, such as one or more modified anti-EGFR antibodies, can be passed over an immobilized EGFR, or a solution with EGFR can be passed over an immobilized anti-EGFR antibody or antibodies. Association rates can be measured by measuring SPR signal as a function of time. After association, a buffer solution can be passed over the solid support, and dissociation rates can be measured as a function of time. From the association and dissociation rates, an equilibrium binding constant can be calculated. (Jecklin et al. (2009), J. Mol. Recognit. 22(4):319-29; Nguyen et al, (2007) Methods. 42(2):150-61; Tanious et al. (2008), Methods Cell Biol. 84:53-77). Measuring activity of an anti-EGFR antibody by detecting binding to EGFR using SPR is within the ability of the skilled artisan (*see, e.g.,* Alvarenga et al. (2012) Anal. Biochem 421(1):138-151).

### ii. Bio-layer interferometry

The activity of the modified anti-EGFR antibodies provided herein can be assessed by measuring binding of the antibodies to EGFR by bio-layer interferometry. Bio-layer interferometry is a label-free method for detecting biomolecular interactions by measuring the interference pattern of visible light reflected from two surfaces: an immobilized biomolecule layer on a biosensor tip, and an internal reference layer. Binding of a molecule in solution to the immobilized biomolecule increases the thickness of the biomolecule layer, which results in a wavelength shift. After binding, the immobilized biomolecule can be contacted with a buffer solution, and dissociation of the molecule can be measured. Binding to the immobilized biomolecule can be measured in real time, and association rate constant, dissociation rate constants, binding affinity and binding specificity can be determined. For example, streptavidin can be attached to a biolayer, and biotinylated sEGFR can be bound to a streptavidin biolayer. An anti-EGFR antibody, such as a modified anti-EGFR antibody, in a suitable buffer can be added to the biolayer and contacted with the sEGFR. The concentration of the anti-EGFR antibody can be selected empirically or based on factors known to the skilled artisan, such as the approximate expected dissociation constant, solubility of the antibody, temperature, and buffer conditions. Binding between sEGFR and the anti-EGFR antibody can be quantitated by measuring changes in the interference pattern generated from light reflected from the optical layer and the biolayer. Binding kinetics can be measured to calculate the association rate constant. To measure the dissociation rate constant, the sensor can be incubated in a suitable buffer, and dissociation of the anti-EGFR antibody and EGFR can be measured. Binding affinity of the anti-EGFR antibody can be calculated as the ratio of the kinetic dissociation rate constant and the kinetic association rate constant. Examples of bio-layer interferometry assays to measure the dissociation constant between modified anti-EGFR antibodies and EGFR are described in Example 5.

### iii. Immunoassays

Exemplary immunoassays which can be used to analyze binding of the anti-EGFR antibodies, such as modified anti-EGFR antibodies, provided herein include, but are not limited to, competitive and non-competitive assay systems using techniques such as, but not limited to, western blots, radioimmunoassays, ELISA (enzyme linked immunosorbent assay), Meso Scale Discovery (MSD, Gaithersburg, Maryland), "sandwich" immunoassays, immunoprecipitation assays, ELISPOT, precipitin reactions, gel diffusion precipitin reactions, immunodiffusion assays, agglutination assays, complement-fixation assays, immunoradiometric assays, fluorescent immunoassays, protein A immunoassays, immunohistochemistry, or immuno-electron microscopy. Such assays are routine and well known in the art (see, *e.g.,* Ausubel et al., Eds, 1994, Current Protocols in Molecular Biology, Vol. 1, John Wiley & Sons, Inc., New York, which is incorporated by reference herein in its entirety). Other assay formats include liposome immunoassays (LIA), which use liposomes designed to bind specific molecules (e.g., antibodies) and release encapsulated reagents or markers. The released chemicals are then detected according to standard techniques (see Monroe et al., (1986) Amer. Clin. Prod. Rev. 5:34-41). Exemplary immunoassays not intended by way of limitation are described briefly below.

**a) ELISA**

Binding between an anti-EGFR antibody, such as a modified anti-EGFR antibody, and EGFR can be detected by Enzyme-linked immunosorbent Assay (ELISA). ELISA is an immunological assay that can be used to detect protein/ligand interactions, such as antibody/antigen interacts. Typically, in an ELISA, the antibody/antigen interactions are detected by measuring a signal from an enzyme marker linked directly or indirectly to the antibody/antigen complex.

For example, an ELISA can include steps of: 1) coating a solid phase with EGFR or a variant thereof; 2) incubating the solid phase with a blocking reagent to block non-specific binding sites on the solid phase; 3) incubating the solid phase with a modified anti-EGFR antibody; 4) incubating with a secondary detection agent, such as a labeled secondary antibody capable of detecting the modified anti-EGFR antibody, but not human serum components contained in the assay buffers, that can bind to the modified anti-EGFR antibody; and 5) detecting the secondary detection agent. Furthermore, one or more washing steps (e.g., 1, 2, 3, 4 or more washing steps) can be included between any steps of the method.

In the dual format or duplicate assay, EGFR can be immobilized under standard conditions that are the same. Typically, the buffer that is used to facilitate adsorption or immobilization under both conditions is a neutral or physiologic buffer. Exemplary of physiologic buffers include, but are not limited to, phosphate buffered saline (PBS), Hank's balanced salt solution (HBSS), Ringers or Krebs. The pH and buffering capacity is a function of the assay conditions and can be empirically determined or chosen by one of skill in the art. Exemplary of a physiologic buffer is Krebs-Ringer Bicarbonate (KRB) buffer (Sigma Aldrich, Catalog No. K4002). Further, adsorption or immobilization of the immobilized agent, typically the cognate binding partner, on a solid support is effected in a buffer that does not contain human serum, since human serum is used in the contacting step or screen to simulate natural environment conditions.

Varying concentrations of EGFR, in KRB buffer or other similar physiologic buffer can be adsorbed onto a solid support. For example, from at or about between 1 and 50 nM, for example, 3 and 30 nM, such as 5-20 nM, for example, at or about 3, 6, 9, 12, 13, 14, 15, 16, 17, 18, 19, 20, 25, 30, 35, 40 or 50 nM can be adsorbed. The amount of EGFR to be adsorbed is a function of the binding agent and can be empirically determined, such as by using a control known to bind the target antigen. Adsorption can proceed for any desired length of time and temperature to allow the cognate binding protein to bind to binding sites on the solid support. For example, adsorption is generally performed at 4 °C-37° C, such as 4° C, room temperature (i.e., 22° C) or 37° C. The time for adsorption is generally 30 minutes to 48 hours or more, and can vary as a function of the temperature.

Following affixation of EGFR to a support, the subsequent steps of the method can be performed as two separate assays. For example, supports are treated separately for performance of the binding assay under two varied assay conditions, such as at low pH and at neutral pH. The conditions also can include 20-50% serum (vol/vol) or 10-50 mg/mL protein (e.g. serum albumin). In some examples, it is understood that in performing the separate assays, the only conditions that are varied relate to the buffer conditions. Time and temperature incubation conditions are generally the same between the parallel assays.

In some examples, prior to adding an anti-EGFR antibody, non-specific protein binding sites on the surface of the solid phase support are typically blocked. Hence, the step of contacting the anti-EGFR antibodies with EGFR typically can be performed after a blocking step. Blocking of the solid support can reduce nonspecific binding to the solid support, reduce background signal, reduce nonspecific binding to adsorbed proteins, and stabilize the adsorbed protein. The selection of conditions for blocking is within the ability of one of skill in the art. Any blocking conditions described in the art can be used in the methods provided herein.

Typically, the incubation reaction can proceed for any desired length of time and temperature to allow the anti-EGFR antibody to bind to EGFR. For example, binding is generally performed at 4°C-37°C, such as 4°C, room temperature or 37°C. The time for binding is generally 30 minutes to 48 hours or more, and can be a function of the temperature. For example, contacting can be performed with 1mM lactic acid, pH 7.4, and 25% human serum. Separately, the contacting step can be performed with 16.5 mM lactic acid, pH 6.0, 25% human serum. In each contacting reaction, contacting can be for 1 hour at room temperature (*i.e.*, 22°C). The solid support can be washed in the same buffer used for binding to remove any unbound target antigen. In some examples, the ELISA assay can be performed in the presence of varying concentrations of modified anti-EGFR antibody. Generally, varying concentrations are tested in serial dilutions. Whole supernatant, diluted supernatant or purified protein can be tested.

The anti-EGFR antibodies, such as a modified anti-EGFR antibodies, that bind to EGFR can be selected or identified using any assay or method known to one of skill in the art. Typically, the reaction can proceed for any desired length of time and temperature to allow detection of the binding molecule or protein. For example, detection is generally performed at 4°C-37°C, such as 4°C, room temperature or 37°C. The time for binding is generally 30 minutes to 48 hours or more, and is a function of the temperature. Typically, binding of the binding molecule or protein is at room temperature at or about between 30 minutes to 4 hours, such as 1 hour to 2 hours, for example about 1 hour. The solid support can be washed in the same buffer used for binding to remove any unbound target antigen.

Once binding activity is determined under each assay condition, the binding activity under the first condition (e.g. low pH and/or elevated lactic acid concentration) and the second condition (e.g. normal pH and/or low lactic acid concentration) are compared. For example, the optical density in each well (or an average of two or more wells) can be compared (see, e.g., Tables 15 and 16). In some examples, the optical density in each well (or an average of two or more wells) is divided by the concentration of the modified anti-EGFR antibody to calculate a specific activity. In some examples, the specific activity is normalized to give a normalized specific activity (NSA) for each modified anti-EGFR antibody by dividing the specific activity of the modified anti-EGFR antibody by the specific activity of a reference antibody, such as an anti-EGFR parental antibody, including, for example, Cetuximab (see, e.g., Table 16).

Anti-EGFR antibodies, such as modified anti-EGFR antibodies, can be identified that have greater activity at low pH than at neutral pH. In some examples, modified anti-EGFR antibodies that have increased binding activity at low pH than at neutral pH can be identified. For example anti-EGFR antibodies with a NSA at low pH greater than the NSA at neutral pH can be identified. In some examples, anti-EGFR antibodies have a ratio of the (NSA at low pH) / (NSA at neutral pH) greater than 1, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.5, 4.0, 4.5, 5.0, 5.5, 6.0, 7.0, 8.0 or more. In some examples, anti-EGFR antibodies are identified with an (NSA at low pH) above a threshold value, such as 0.1, 0.2, 0.3, 0.4, 0.5, 0.6, 0.7, 0.8, 0.9, 1.0 or more. In some examples, anti-EGFR antibodies are identified with an (NSA at neutral pH) below a cutoff value, such as 0.1, 0.2, 0.3, 0.4, 0.5, 0.6, 0.7, 0.8, 0.9, 1.0 or more. Anti-EGFR antibodies, such as modified anti-EGFR antibodies, that are more active at low pH than at neutral pH can include anti-EGFR antibodies that meet one or more of these criteria. In some examples, the low pH is pH 6.0 or pH 6.5. In some examples, the neutral pH is pH 7.4.

The ELISA methods described herein are exemplified in Example 1. A further description of the steps of the ELISA method and components of the method are provided below.

Solid supports that can be used in the binding assays provided herein include any carrier that is capable of being affixed with a molecule, for example a test molecule or a cognate binding partner of a protein such as a ligand, receptor or antigen. Typically, to facilitate high throughput screening of variant test molecules (e.g. a library or collection of antibody variants such as anti-EGFR antibody variants), a cognate binding partner is affixed to the solid support. Examples of carriers for use as solid supports in the methods provided herein include, but are not limited to, glass, polystyrene, polypropylene, polyethylene, dextran, nylon, amyloses, natural and modified celluloses, polyacrylamides, agaroses and magnetic solid supports, such as solid supports that include magnetite. The solid support can be one or more beads or particles, microspheres, a surface of a tube or plate, a filter membrane, and other solid supports known in the art. Exemplary solid support systems include, but are not limited to, a flat surface constructed, for example, of glass, silicon, metal, nylon, cellulose, plastic or a composite, including multiwell plates or membranes; or can be in the form of a bead such as a silica gel, a controlled pore glass, a magnetic (Dynabead) or cellulose bead. Further, such methods can be adapted for use in suspension or in the form of a column.

It is within the level of one of skill in the art to select a suitable solid support depending on the particular assay conditions. For example, nickel coated microplates can be less suitable for binding of His-tagged proteins, since buffer pH can affect antigen coating to Ni-coated but not high-bind plates. It is within the level of one of skill in the art to determine whether a solid support is suitable for use with varying pH conditions.

Test molecules or cognate binding partners can be immobilized to the solid support by any method known to one of skill in the art. Covalent or non-covalent methods for attachment can be used. Typically, the test molecule or cognate binding partner (such as a ligand or antigen) is immobilized by adsorption from an aqueous medium. In some examples, adsorption can be carried out under conditions that simulate a diseased microenvironment (such as a tumor or cancer microenvironment), under conditions that simulate a normal microenvironment, or under standard conditions known to one of skill in the art. For example, adsorption can be carried out using a buffer with a pH range of at or about between 6.0 to 7.4, in some examples at or about pH 7.4. In particular, to effect adsorption, a high binding microplate can be used as a solid support. High binding plates are known to those of skill in the art and readily available from various manufacturers (see e.g., Nunc Maxisorp flat-bottom plates available from eBioscience, San Diego, CA, Cat. No. 44-2404-21; Costar 96-well EIA/RIA Stripwell plate, Costar 2592).

Other modes of affixation, such as covalent coupling or other well known methods of affixation of the target protein to the solid matrix can also be used. Covalent methods of attachment of therapeutic proteins and/or cognate binging partners include chemical crosslinking methods. Reactive reagents can create covalent bonds between the support and functional groups on the protein or cognate binding partner. Examples of functional groups that can be chemically reacted are amino, thiol, and carboxyl groups. N-ethylmaleimide, iodoacetamide, N-hydrosuccinimide, and glutaraldehyde are examples of reagents that react with functional groups. In other examples, test molecules and/or cognate binding partners can be indirectly attached to a solid support by methods such as, but not limited to, immunoaffinity or ligand-receptor interactions (*e.g.* biotin-streptavidin or glutathione S-transferase-glutathione). For example, test molecules can be coated to an ELISA plate, or other similar addressable array.

Blocking solutions include those containing human, bovine, horse or other serum albumin. Typically, the blocking solution contains human serum. Blocking of a solid support, such as a plate, can be performed using a binding assay buffer to which one or more blocking agents are added. Exemplary blocking agents include 1-5% Bovine Serum Albumin, 1-5% non-fat dry milk and 25% human serum. Detergents, such as Tween-20, and preservatives, such as thimerosal, can be added to the blocking solution. Binding assay buffers include i.e. the tumor microenvironment buffer or the normal physiologic buffer. The aqueous protein solution-solid support mixture is typically maintained for a time period of 30 minutes, 1 hour, or longer, and can vary as a function of the temperature. The blocking reaction can be performed at any temperature, and generally can be performed 4° C-37° C, such as 4° C, room temperature (i.e., 22° C) or 37°C. In some examples, the reaction is allowed to proceed for at least one hour at a temperature of about 4°C-37°C. For example, blocking can be achieved at room temperature for one hour. After incubation and blocking, the resulting solid phase can be thereafter rinsed free of unbound protein prior to contact with the test molecule (e.g. therapeutic protein or antibody or variants thereof).

Examples of enzyme labels include horseradish peroxidase, alkaline phosphatase, and beta-D-galactosidase. Examples of enzyme substrates that can be added to develop the signal include PNPP (p-Nitrophenyl Phosphate, Disodium Salt), ABTS (2,2'-Azinobis [3-ethylbenzothiazoline-6-sulfonic acid]-diammonium salt), OPD (o-phenylenediamine dihydrochloride), and TMB (3,3',5,5'-tetramethylbenzidine) (SOMA Labs, Romeo, Mich.), including Sureblue TMB Microwell Peroxidase Substrate 1-component (KPL, #52-00-03). The reaction can be stopped by adding a stopping reagent (e.g. TMB stop solution). The absorbance at a suitable wavelength (i.e. 450 nm) can be determined.

For fluorescence, a large number of fluorometers are available. For chemiluminescers, such as horseradish peroxidase (HRP), luminometers or films are available. With enzymes, a fluorescent, chemiluminescent, or colored product can be determined or measured fluorometrically, luminometrically, spectrophotometrically or visually. For example, an anti-tag reagent can be conjugated to horseradish peroxidase (HRP) or other detectable agent.

Detection can be facilitated by the presence of a fluorescent, radioactive or other detectable moiety. Typically, because the anti-EGFR antibodies are tagged, detection is effected using an anti-tag reagent. The choice of anti-tag reagent is a function of the tag that is employed with the binding molecule or protein. In addition, an anti-tag reagent is chosen that is compatible with the environment conditions (e.g. pH) used in the assay. It is within the level of one of skill in the art to identify or select such reagents, and test their compatibility with the assay conditions. For example, the Examples exemplify such procedures.

Anti-tag reagents are readily available such as from commercial sources or other sources. Exemplary anti-tag reagents that can be used for detection in the methods herein include, but are not limited to an anti-FLAG antibody or anti-Myc antibody (available from vendors such as Abcam, Cambridge, MA; GeneTex, Irvine, CA).

Typically, in the methods herein, the method of detection of the bound complex is one that is capable of being quantitated such that the level of activity can be assessed. For example, a label can produce a signal, such as a colorimetric signal, a chemiluminescent signal, a chemifluorescent signal or a radioactive signal. Depending upon the nature of the label, various techniques can be employed for detecting or detecting and quantitating the label. For example, methods of quantitation include, but are not limited to, spectrophotometric, fluorescent and radioactive methods.

**b) Immunoprecipitation**

Activity of the anti-EGFR antibodies, such as modified anti-EGFR antibodies, provided herein can be assessed by detecting binding to EGFR by immunoprecipitation. Immunoprecipitation protocols generally comprise lysing a population of cells in a lysis buffer such as 1% NP-40 Alternative, 20 mM Tris (pH 8.0), 137 mM NaCl, 10% glycerol, 2 mM EDTA, 1 mM activated sodium orthovanadate, 10 μg/mL Aprotinin, 10 μg/mL Leupeptin; or RIPA buffer (1 % NP-40 or Triton X-100, 1 % sodium deoxycholate, 0.1 % SDS, 0.15 M NaCl, 0.01 M sodium phosphate at pH 7.2, 1 % Trasylol). The lysis buffer can be supplemented with protein phosphatase and/or protease inhibitors (*e.g.*, EDTA, PMSF, aprotinin, sodium vanadate). Additional steps can include adding the modified anti-EGFR antibody to the cell lysate, and incubating for a period of time (*e.g.,* 1 to 4 hours) at 40 °C, adding protein A and/or protein G sepharose beads to the cell lysate, incubating for about an hour or more at 40 °C, washing the beads in lysis buffer and resuspending the beads in SDS/sample buffer. The ability of the modified anti-EGFR antibody to immunoprecipitate EGFR can be assessed by, *e.g.*, western blot analysis. One of skill in the art is knowledgeable as to the parameters that can be modified to increase the binding of an antibody to an antigen and decrease the background (*e.g.*, pre-clearing the cell lysate with sepharose beads). For further discussion regarding immunoprecipitation protocols see, *e.g.,* Ausubel et al., Eds, 1994, Current Protocols in Molecular Biology, Vol. 1, John Wiley & Sons, Inc., New York at 10.16.1.

**c) Western blot**

Activity of the anti-EGFR antibodies, such as modified anti-EGFR antibodies, provided herein can be assessed by detecting binding to EGFR by Western blot. Western blot analysis generally includes preparing extract samples (*e.g.* from a tissue that expresses EGFR, or a tissue from a subject or patient with a disease or disorder that can be treated by administering an anti-EGFR antibody, such as a disease or disorder described herein). Additional steps include electrophoresis of the samples in a polyacrylamide gel (*e.g.,* 8 %-20 % SDS-PAGE depending on the molecular weight of the antigen) or via 2-D gel electrophoresis (see, *e.g.,* WO 04/043276), transferring the sample from the polyacrylamide gel to a membrane such as nitrocellulose, PVDF or nylon, blocking the membrane in blocking solution (*e.g.,* PBS with 3 % BSA or non-fat milk), washing the membrane in washing buffer (*e.g.,* PBS-Tween 20), probing the membrane with primary antibody (*i.e.* the antibody of interest) diluted in blocking buffer, washing the membrane in washing buffer, probing the membrane with a secondary antibody (which recognizes the primary antibody, *e.g.,* an antihuman antibody) conjugated to an enzymatic substrate (*e.g.,* horseradish peroxidase or alkaline phosphatase) or radioactive molecule (*e.g.,* $^{32}$P or $^{125}$I) diluted in blocking buffer, washing the membrane in wash buffer, and detecting the presence of the antigen. One of skill in the art is knowledgeable as to the parameters that can be modified to increase the signal detected and to reduce the background noise. For further discussion regarding western blot protocols see, *e.g.,* Ausubel et al., Eds, 1994, Current Protocols in Molecular Biology, Vol. 1, John Wiley & Sons, Inc., New York at 10.8.1.

**d) Immunohistochemistry**

Activity of the anti-EGFR antibodies, such as modified anti-EGFR antibodies, provided herein can be assessed by detecting binding to EGFR by immunohistochemistry. Immunohistochemistry generally comprises preparing a tissue sample (*e.g.* from a tissue that expresses EGFR, or a tissue from a subject or patient with a disease or disorder that can be treated by administering an anti-EGFR antibody, such as a disease or disorder described herein), fixing the tissue to preserve protein molecules in their native conformation, bathing the sample in a permeabilization reagent (*e.g.* Tween, Nonidet P40) to penetrate the tissue, blocking the sample with blocking solution (*e.g.,* PBS with 3 % BSA or non-fat milk), washing the sample in washing buffer (*e.g.,* PBS-Tween 20), probing the sample with an anti-EGFR antibody (such as a modified anti-EGFR antibody described herein) diluted in blocking buffer, washing the sample in washing buffer, probing the sample with a secondary antibody (which recognizes the anti-EGFR antibody) conjugated to a fluorescent dye (e.g. fluorescein isothiocyanate, Alexa fluor, rhodamine) diluted in blocking buffer, washing the sample in wash buffer, and detecting the presence of the antigen via fluorescent microscopy. One of skill in the art is knowledgeable as to the parameters that can be modified to increase the signal detected and to reduce the background noise.

**e) Radioimmunoassay**

Activity of the anti-EGFR antibodies, such as modified anti-EGFR antibodies, provided herein can be assessed by detecting binding to EGFR by radioimmunoassay. The binding affinity of a modified anti-EGFR antibody to an antigen, such as EGFR, and the off-rate of the antibody-antigen interaction can be determined, for example, by competitive binding assays. One example of a competitive binding assay is a radioimmunoassay involving the incubation of labeled antigen (*e.g.,* $^{3}$H or $^{125}$I) with the antibody of interest in the presence of increasing amounts of unlabeled antigen, and the detection of the antibody bound to the labeled antigen. The affinity of an anti-EGFR antibody provided herein for EGFR and the binding off-rates can be determined from the data by Scatchard plot analysis. Competition with a second antibody can also be determined using radioimmunoassays. In this case, an EGFR antigen, such as the EGFR soluble fragment, is incubated with an anti-EGFR antibody provided herein conjugated to a labeled compound (*e.g.,* $^{3}$H or $^{125}$I) in the presence of increasing amounts of an unlabeled second antibody.

**b. Solution Binding Assays**

Solution binding assays can be used to measure the activity of the anti-EGFR antibodies, such as modified anti-EGFR antibodies, provided herein. In some examples, solution binding assays are used to measure the binding of the anti-EGFR antibodies to EGFR, or a fragment or variant thereof, such as the soluble EGFR fragment. The skilled artisan can select a solution binding assay to measure binding of the modified anti-EGFR antibodies provided herein. Below is a brief description of exemplary solution binding assays that can be used. However, these are not meant to be limiting, and any solution binding assay known to the skilled artisan is contemplated for use in the methods provided herein, including equilibrium dialysis, competitive binding assays (e.g., Myers et al., (1975) Proc. Natl. Acad. Sci. USA), radiolabeled binding assays (e.g., Feau et al., (2009) J. Biomol. Screen. 14(1):43-48), calorimetry (including isothermal titration calorimetry (ITC) and differential scanning calorimetry (e.g., Perozzo et al., (2004) J. Recept Signal. Transduct Res. 24(1-2):1-52; Holdgate (2001) Biotechniques 31(1):164-166, 168, 170), Celej et al. (2006) Anal. Biochem. 350(2):277-284)), and spectroscopic fluorescence assays, including fluorescence resonance energy transfer assays. The conditions for the method herein where binding activity is determined in solution can be determined by one of skill in the art based on the description herein. For example, the conditions can be adapted from conditions discussed above for binding assays performed on a solid support.

**i. Isothermal titration calorimetry (ITC)**

Activity of the anti-EGFR antibodies, such as modified anti-EGFR antibodies, can be assessed by detecting binding to EGFR by Isothermal titration calorimetry (ITC). In ITC, one binding partner is titrated into a solution containing the other binding partner, thereby generating or absorbing heat, which is quantified by the calorimeter. ITC can be used to detect heat effects from reactants in quantities of nanomoles or less. For example, isothermal titration calorimetry assays can be performed to measure all thermodynamic parameters, including free energy of binding ($\Delta G$), enthalpy ($\Delta H$), and entropy ($\Delta S$) of binding, and the heat capacity change ($\Delta Cp$), involved in binding of a therapeutic protein to a cognate binding partner. Analysis of these features can help elucidate the activity and thermodynamic parameters of binding between a modified anti-EGFR antibody and EGFR (Perozzo et al., (2004) J. Recept. Signal. Transduct. Res. 24(1-2):1-52). Measuring activity of an anti-EGFR antibody by detecting binding to EGFR using ITC is within the ability of the skilled artisan (see, e.g., Alvarenga et al. (2012) Anal. Biochem 421(1): 138-151).

**ii. Spectroscopic assays**

Spectroscopic assays can be used to measure activity of an anti-EGFR antibody, such as a modified anti-EGFR antibody, provided herein. Binding of a anti-EGFR antibody and EGFR can be detected by any spectroscopic assay known to one of skill in the art, including UV-vis spectroscopic techniques, fluorescence assays such as fluorescence resonance energy transfer assays and fluorescence quenching assays. (Wu et al. (2007), J. Pharm. Biomed. Anal.44(3):796-801) For example, changes in fluorescence or UV/vis absorption as a result of a anti-EGFR antibody binding to EGFR, such as quenching of inherent fluorescence, can be detected. In some examples, the anti-EGFR antibody and/or EGFR can be labeled with a fluorescent label or a UV/vis label. Labeling anti-EGFR antibodies is within the ability of the skilled artisan (see, e.g., Gleysteen et al. (2008) Head & Neck 30(6):782-789; Rosenthal et al. (2007) Mol. Cancer Ther. 6:1230-1238). After measuring a spectroscopic signal, the observed binding constant can be calculated (e.g., Zhang et al. (2009) Spectrochim Acta A Biomol. Spectrosc. 72(3):621-626).

**2. Cell Based Assays**

Assays to measure activity of the anti-EGFR antibody, such as a modified anti-EGFR antibodies, provided herein include cell based assays. Cell lines that can be used include any cell lines described in the art or cell lines that can be obtained from repositories such as the American Type Culture Collection (ATCC). The skilled artisan can select cell lines with desired properties. Generally, assays are performed using cell lines known to express EGFR. Such cells are known to one of skill in the art. For example, one can consult the ATCC Catalog (atcc.org) to identify cell lines. Also, if a particular cell type is desired, the means for obtaining such cells, and/or their instantly available source is known to those in the art. An analysis of the scientific literature can readily reveal appropriate choice of cells expressing EGFR. Exemplary cell lines that express EGFR that can be used in cell based assays to screen the anti-EGFR antibodies provided herein include DiFi human colorectal carcinoma cells, A431 cells (ATCC CRL-1555), Caco-2 colorectal adenocarcinoma cells (ATCC HTB-37), HRT-18 colorectal adenocarcinoma cells (ATCC CCL-244), HT-29 colorectal adenocarcinoma cells (ATCC HTB-38), human neonatal keratinocytes and MCF10A epithelial cells (ATCC CRL-10317) (see, e.g., Olive et al. (1993) In Vitro Cell Dev Biol. 29A(3 Pt 1):239-248; Wu et al. (1995) J. Clin. Invest. 95(4): 1897-1905). Exemplary cells that can be used in the cell based assays described herein include any cells described herein or known in the art, including, for example, tumor or cancer cells described herein.

In some examples, assays to measure the activity of an anti-EGFR antibody, such as a modified anti-EGFR antibodies, provided herein, such as the assays described herein, are performed using cell lines from a tissue associated with a side effect of anti-EGFR antibodies, such as any side effect described herein or known in the art. For example, assays can be performed using skin cell lines. EGFR is expressed in several cell types, including keratinocytes, such as basal keratinocytes and the outer root sheath of hair follicles; and cells of eccrine and sebaceous glands (Albanell et al. (2002) J. Clin. Oncol. 20(1): 110-124; Lacouture, and Melosky (2007) Skin Therapy Lett. 12, 1-5; Nanney et al. (1990) J. Invest. Dermatol 94(6):742-748). In some examples, cell-based assays to measure activity of the anti-EGFR antibodies provided herein are performed using keratinocytes, such as, for example, human neonatal keratinocytes;

cells from the outer root sheath of hair follicles; and cells of eccrine and sebaceous glands. Other cells that can be used in cell-based assays to measure activity of the anti-EGFR antibodies provided herein include, for example, melanocytes, such as, for example, newborn melanocytes; Langerhans cells; fibroblasts; Merkel's cells; nerve cells; glandular cells; sebaceous gland cells (sebocytes); and fibroblasts, such as, for example dermal fibroblasts and wound fibroblasts. Methods of culturing such cells are within the ability of the skilled artisan (see, e.g., Limat and Hunziker (1996) Methods Mol Med. 2:21-31; Abdel-Naser et al. (2005) Egypt. Dermatol. Online J. 1(2):1).

Cell lines expressing EGFR can be generated by transient or stable transfection. In addition, any primary cell or cell line can be assessed for expression of EGFR, such as by using fluorescently labeled anti-EGFR antibodies and fluorescence activated cell sorting (FACS). Exemplary cell lines include A549 (lung), HeLa , Jurkat, BJAB, Colo205, H1299, MCF7, MDA-MB-231, PC3, HUMEC, HUVEC, and PrEC.

In some examples, an anti-EGFR antibody, such as a modified anti-EGFR antibody, purified or unpurified, is added exogenously to cells. In some examples, one or more nucleic acid(s) encoding a modified anti-EGFR antibody, can be introduced into a vector suitable for expression in cells, such as a cell described herein. Cells can be transfected with the vector, and the anti-EGFR antibody therapeutic protein(s) are expressed by the cells. The anti-EGFR antibody can be expressed as secreted, soluble molecules or intracellular antibodies. Methods of transfection are known to those of skill in the art (see e.g., Kaufman R.J. (1990) Methods in Enzymology 185:537-566; Kaufman et al. (1990) Methods in Enzymology 185:537-566; Hahn and Scanlan (2010) Top. Curr. Chem. 296:1-13), and include, for example, chemical methods such as polycationic cyclodextrin vectors (e.g., Cryan et al, (2004) Eur J Pharm Sci. 21(5):625-33) and liposome complexes, including cationic liposomes (*e.g.,* Gao and Huang (1995) Gene Ther. 2(10):710-722). Exemplary cationic liposomes which can be used include those described in U.S. Pat No. 7,989,606, including 3-beta-[N-(N',N'-dimethyl-aminoethane)-1-carbamoyl]-cholesterol (DC-Chol), 1,2-bis(oleoyloxy-3-trimethylammonio-propane) (DOTAP) (see, for example, International Pat. Publ No. WO 98/07408), lysinylphosphatidylethanol amine (L-PE), lipopolyamines such as lipospermine, N-(2-hydroxyethyl)-N,N-d-dimethyl-2,3-bis(dodecyloxy) 1 -propanaminium bromide, dimethyl dioctadecyl ammonium bromide (DDAB), dioleoylphosphatidyl ethanolamine (DOPE), dioleoylphosphatidyl choline (DOPC), N(1,2,3-dioleyloxy) propyl-N,N,N-triethylammonium (DOTMA), DOSPA, DMRIE, GL-67, GL-89, Lipofectin, and Lipofectamine (Thiery, et al. Gene Ther. (1997); Felgner, et al., Annals N.Y. Acad. Sci. (1995); Eastman, et al., Hum. Gene Ther. (1997)). Methods of transfection also include nonchemical methods, such as electroporation (Chu et al. (1987), Nucl. Acid. Res. 15(3) 1311-1326), sonoporation (*e.g.,* Kumon, et al (2009), Ultrasound Med Biol. 35(3):494-506), gene gun (*e.g.,* O'Brien and Lummis (2004) Methods 33(2):121-125) and viral transduction (e.g., Flotte and Carter (1995) Gene Ther. 2(6):357-362).

Activity of the anti-EGFR antibodies, such as modified anti-EGFR antibodies, can be assessed, for example, using any assay that can detect the binding to the surface of the cells. Activity also can be assessed by assessing a functional activity of the anti-EGFR antibodies. In some examples, the assays are based on the biology of the ability of the anti-EGFR antibody to bind to EGFR and mediate some biochemical event, for example effector functions like cellular lysis, phagocytosis, ligand/receptor binding inhibition, inhibition of growth and/or proliferation and apoptosis.

Such assays often involve monitoring the response of cells to a modified anti-EGFR antibody, for example cell survival, cell death, cellular phagocytosis, cell lysis, change in cellular morphology, or transcriptional activation such as cellular expression of a natural gene or reporter gene. For example, cell proliferation assays, cell death assays, flow cytometry, cell separation techniques, fluorescence activated cell sorting (FACS), phase microscopy, fluorescence microscopy, receptor binding assays, cell signaling assays, immunocytochemistry, reporter gene assays, cellular morphology (*e.g.*, cell volume, nuclear volume, cell perimeter, and nuclear perimeter), ligand binding, substrate binding, nuclease activity, apoptosis, chemotaxis or cell migrations, cell surface marker expression, cellular proliferation, GFP positivity and dye dilution assays (e.g., cell tracker assays with dyes that bind to cell membranes), DNA synthesis assays (e.g., 3H-thymidine and fluorescent DNA-binding dyes such as BrdU or Hoechst dye with FACS analysis) and nuclear foci assays, are all suitable assays to measure the activity of the modified anti-EGFR antibodies provided herein. Other functional activities that can be measured include, but are not limited to, ligand binding, substrate binding, endonuclease and/or exonuclease activity, transcriptional changes to both known and uncharacterized genetic markers (e.g., northern blots), changes in cell metabolism, changes related to cellular proliferation, cell surface marker expression, DNA synthesis, marker and dye dilution assays (e.g., GFP and cell tracker assays), contact inhibition, tumor growth in nude mice, and others.

For example, anti-EGFR antibodies, such as modified anti-EGFR antibodies, provided herein can be assessed for their modulation of one or more phenotypes of a cell known to express EGFR. Phenotypic assays, kits and reagents for their use are well known to those skilled in the art and are herein used to measure the activity of modified anti-EGFR antibodies. Representative phenotypic assays, which can be purchased from any one of several commercial vendors, include those for determining cell viability, cytotoxicity, proliferation or cell survival (Molecular Probes, Eugene, Oregon; PerkinElmer, Boston, Mass.), protein-based assays including enzymatic assays (Panvera, LLC, Madison, Wis.; BD Biosciences, Franklin Lakes, N.J.; Oncogene Research Products, San Diego, Calif.), cell regulation, signal transduction, inflammation, oxidative processes and apoptosis (Assay Designs Inc., Ann Arbor, Mich.), triglyceride accumulation (Sigma-Aldrich, St. Louis, Mo.), angiogenesis assays, tube formation assays, cytokine and hormone assays and metabolic assays (Chemicon International Inc., Temecula, Calif.; Amersham Biosciences, Piscataway, N.J.).

Cells determined to be appropriate for a particular phenotypic assay (i.e., any cell described herein or known in the art to express EGFR) can be treated with a anti-EGFR antibody as well as control antibody. In some examples, EGF, or a fragment thereof, is included so that activation of the receptor is effected. At the end of the treatment period, treated and untreated cells can be analyzed by one or more methods described herein or known in the art. In some examples, activity of the anti-EGFR antibodies provided herein can be assessed by measuring changes in cell morphology, measuring EGFR phosphorylation or cell proliferation.

The assays can be performed to assess the effects of an anti-EGFR antibody, such as a modified anti-EGFR antibody, on EGFR and/or on cells that express EGFR. In some examples, the activity of EGFR can be stimulated in the presence of EGF or another stimulating agent in the presence or absence of the anti-EGFR antibody provided herein to determine if the antibody modulates (e.g. inhibits) the actions of EGF or another stimulating agent. For example, the anti-EGFR antibody can act by blocking the ability of EGF to interact with EGFR. Thus, the methods of screening herein can permit identification of antagonist anti-EGFR antibodies.

For example, EGFR phosphorylation assays can be used to measure the ability of the anti-EGFR antibodies provided herein to inhibit phosphorylation of EGFR. Binding of EGF to the extracellular domain of EGFR induces receptor dimerization, and tyrosine phosphorylation, and can result in uncontrolled proliferation (Seshacharyulu et al. (2012) Expert. Opin. Ther. Targets. 16(1):15-31). Anti-EGFR antibodies, such as the modified anti-EGFR antibodies provided herein, can inhibit EGF binding to EGFR and decrease EGFR phosphorylation (see, e.g., U.S. Patent No. 8,071,093). Thus, activity of a anti-EGFR antibody provided herein can be assessed by detecting phosphorylated EGFR. In some examples, phosphorylated EGFR can be detected in cell lysates by an ELISA assay using methods known in the art or described herein (*see, e.g.,* Example 6 and Figure 3). The dose-dependence of the modified anti-EGFR antibodies on the inhibitory effect can be determined by plotting the concentration of phosphorylated EGFR against the concentration of modified anti-EGFR antibody. Tyrosine phosphorylated forms of EGFR can be detected using EGFR Phospho ELISA kits available from, e.g., Sigma-Aldrich (St. Louis, Mo.), RAYBIO (Norcross, Ga) or Thermo Scientific (Rockford, IL).

Growth assays can be used to measure the activity of the modified anti-EGFR antibodies. The assays can measure growth inhibition of cells that express EGFR by an anti-EGFR antibody, such as a modified anti-EGFR antibody. Cells can be incubated for a sufficient time for cells to grow (such as, for example, 12 hours, or 1, 2, 3, 4, 5, 6, 7 days or longer). Cell growth can be measured by any method known in the art, including H-thymidine incorporation assay, 5-bromo-2-deoxyuridine (BrdU) ELISA, tetrazolium microplate assay and acid phosphatase assay (*e.g.,* Maghni et al. (1999) J. Immunol. Method. 223(2):185-194). Cell growth can also be measured using kits available from Invitrogen (Cyquant NF cell proliferation assay kit), Cambrex (ViaLight HS (high sensitivity) BioAssay), Promega (CellTiter-Glo Luminescent Cell Viability Assay, Guava Technologies (CellGrowth assay), Stratagene (Quantos cell proliferation assay) (e.g., Assays for Cell Proliferation Studies, Genetic Eng. Biotechnol. News. 26(6)). In some examples, the cell growth can be normalized to growth of cells without antibody. In exemplary growth assays, cells can be added to a well of a 96-well plate in normal growth medium that includes the anti-EGFR antibody to be assayed. An exemplary cell growth assay is described in Example 7.

### 3. Animal Models

*In vivo* studies using animal models also can be performed to assess the therapeutic activity of anti-EGFR antibodies, such as modified anti-EGFR antibodies, provided herein. An anti-EGFR antibody can be administered to animal models of the diseases and conditions for which therapy using an anti-EGFR antibody, such as a modified anti-EGFR antibody provided herein, is considered. Such animal models are known in the art, and include, but are not limited to, xenogenic cancer models wherein human cancer explants or passaged xenograft tissues are introduced into immune compromised animals, such as nude or SCID mice, (*see e.g.,* Klein. et al. (1997) Nature Medicine 3:402-408). Efficacy can be predicted using assays that measure inhibition of tumor formation, tumor regression or metastasis. Animal models can also be used to assess side effects of the anti-EGFR antibodies provided herein.

Various tumor cell lines or tumor animal models are known to one of skill in the art and are described herein. For example, the anti-EGFR antibody can be administered to a tumor-bearing animal, and body weights and tumor volumes monitored. In a further example, to assess adverse side effects, the anti-EGFR antibody can administered to normal animals, and body weights monitored. Activity of the anti-EGFR antibodies can be assessed by monitoring parameters indicative of treatment of a disease or condition that can be treated by administration of anti-EGFR antibodies. For example, a parameter indicative of anti-tumorigenicity is shrinkage of tumor size and/or delay in tumor progression. Hence, for example, anti-EGFR antibodies can be assessed to identify those that decrease tumor growth or size. Tumor size can be assessed *in vivo* in tumor-bearing human or animal models treated with a anti-EGFR antibody. Tumor shrinkage or tumor size can be assessed by various assays known in art, such as, by weight, volume or physical measurement.

Tumor-bearing animal models can be generated. *In vivo* tumors can be generated by any known method, including xenograft tumors generated by inoculating or implanting tumor cells (e.g. by subcutaneous injection) into an immunodeficient rodent, syngenic tumors models generated by inoculating (e.g. by subcutaneous injection) a mouse or rat tumor cell line into the corresponding immunocompetent mouse or rat strain, metastatic tumors generated by metastasis of a primary tumor implanted in the animal model, allograft tumors generated by the implantation of tumor cells into same species as the origin of the tumor cells, and spontaneous tumors generated by genetic manipulation of the animal. The tumor models can be generated orthotopically by injection of the tumor cells into the tissue or organ of their origin, for example, implantation of breast tumor cells into a mouse mammary fat pad. In some examples, xenograft models or syngenic models are used. For example, tumors can be established by subcutaneous injection at the right armpit with a tumor cell suspension (*e.g.* $1 \times 10^6$ to $5 \times 10^6$ cells/animal) into immunocompetent hosts (syngeneic) or immunodeficient hosts (*e.g.* nude or SCID mice; xenograft). The animal models include models in any organism described herein or known in the art, such as, for example, a mammal, including monkeys and mice.

The tumor can be syngeneic, allogeneic, or xenogeneic. The tumor can express endogenous or exogenous EGFR. Exogenous EGFR expression can be achieved using methods of recombinant expression known in the art or described herein via transfection or transduction of the cells with the appropriate nucleic acid. Exemplary cell lines include EGFR transfected NIH3T3, MCF7 (human mammary), human epidermoid squamous carcinoma A431, oral squamous cell carcinoma (OSCC) cell line BcaCD885, COLO 356/FG pancreatic cell lines and LS174T colorectal tumors (see e.g., Santon et al., (1986) Cancer Res. 46:4701-05 and Ozawa et al., (1987) Int. J. Cancer 40:706-10; U.S. Pat. Pub. No. 20110111059; Reusch et al. (2006) Clin. Cancer Res. 12(1):183-190; and Yang et al. (2011) Int. J. Nanomedicine 6:1739-1745).

Anti-EGFR antibodies, such as modified anti-EGFR antibodies, provided herein can be tested in a variety of orthotopic tumor models. These animal models are used by the skilled artisan to study pathophysiology and therapy of aggressive cancers such as, for example, pancreatic, prostate and breast cancer. Immune deprived mice including, but not limited to athymic nude or SCID mice can be used in scoring of local and systemic tumor spread from the site of intraorgan (e.g. pancreas, prostate or mammary gland) injection of human tumor cells or fragments of donor patients.

In some examples, the testing of anti-EGFR targeting proteins can include study of efficacy in primates (e.g. cynomolgus monkey model) to facilitate the evaluation of depletion of specific target cells harboring EGFR antigen. Additional primate models include but are not limited to that of the rhesus monkey.

For example, the recipient of the tumor can be any suitable murine strain. The recipient can be immunocompetent or immunocompromised in one or more immune-related functions, including but not limited to nu/nu, SCID, and beige mice. Examples of animals in which tumor cells can be transplanted include BALB/c mice, C57BL/6 mice, severe combined immunodeficient/Beige mice (SCID-Beige) *(see, e.g.,* U.S. Pat. Pub. No. 20110111059; Reusch et al. (2006) Clin. Cancer Res. 12(1):183-190; Yang et al. (2011) Int. J. Nanomedicine 6:1739-1745). Other examples include nude mice, SCID mice, xenograft mice, and transgenic mice (including knockins and knockouts). For example, a anti-EGFR antibody provided herein can be tested in a mouse cancer model, for example a xenograft mouse. In this method, a tumor or tumor cell line is grafted onto or injected into a mouse, and subsequently the mouse is treated with an anti-EGFR antibody to determine the ability of the anti-EGFR antibody to reduce or inhibit cancer growth and metastasis. Also contemplated is the use of a SCID murine model in which immune-deficient mice are injected with human peripheral blood lymphocytes (PBLs).

Exemplary human tumor xenograft models in mice, such as nude or SCID mice, include, but are not limited to, human lung carcinoma (A549 cells, ATCC No. CCL-185); human breast tumor (GI-101A cells, Rathinavelu et al., (1999) Cancer Biochem. Biophys., 17:133-146); human ovarian carcinoma (OVCAR-3 cells, ATCC No. HTB-161); human pancreatic carcinoma (PANC-Icells, ATCC No. CRL-1469 and MIA PaCa-2 cells, ATCC No. CRL-1420); DU145 cells (human prostate cancer cells, ATCC No. HTB-81); human prostate cancer (PC-3 cells, ATCC# CRL-1435); colon carcinoma (HT-29 cells); human melanoma (888-MEL cells, 1858-MEL cells or 1936-MEL cells; see e.g. Wang et al., (2006) J. Invest. Dermatol. 126:1372-1377); and human fibrosarcoma (HT-1080 cells, ATCC No. CCL-121,) and human mesothelioma (MSTO-211H cells). Exemplary rat tumor xenograft models in mice include, but are not limited to, glioma tumor (C6 cells; ATCC No. CCL-107). Exemplary mouse tumor homograft models include, but are not limited to, mouse melanoma (B16-F10 cells; ATCC No. CRL-6475). Exemplary cat tumor xenograft models in mice include, but are not limited to, feline fibrosarcoma (FC77.T cells; ATCC No. CRL-6105). Exemplary dog tumor xenograft models in mice include, but are not limited to, canine osteosarcoma (D17 cells; ATCC No. CCL-183). Nonlimiting examples of human xenograft models and syngeneic tumor models are set forth in the Tables 13 and 14 below.

| Table 13: Human Tumor Xenograft Models | | | |
|---|---|---|---|
| **Tumor Type** | **Cell Line Name** | **Tumor Type** | **Cell Line** |
| Adenoid cystic carcinoma | ACC-2 | Leukemia | HL-60 |

(continued)

| Table 13: Human Tumor Xenograft Models | | | |
|---|---|---|---|
| **Tumor Type** | **Cell Line Name** | **Tumor Type** | **Cell Line** |
| Bladder carcinoma | EJ | Liver carcinoma | Bel-7402 |
| Bladder carcinoma | T24 | Liver carcinoma | HepG-2 |
| Breast carcinoma | BCaP-37 | Liver carcinoma | QGY-7701 |
| Breast carcinoma | MX-1 | Liver carcinoma | SMMC7721 |
| Cervical carcinoma | SiHa | Lung carcinoma | A549 |
| Cervical carcinoma | Hela | Lung carcinoma | NCI-H460 |
| Colon carcinoma | Ls-174-T | Melanoma | A375 |
| Colon carcinoma | CL187 | Melanoma | M14 |
| Colon carcinoma | HCT-116 | Melanoma | MV3 |
| Colon carcinoma | SW116 | Ovary carcinoma | A2780 |
| Gastric carcinoma | MGC-803 | Pancreatic carcinoma | BXPC-3 |
| Gastric carcinoma | SGC-7901 | Prostate carcinoma | PC-3M |
| Gastric carcinoma | BGC-823 | Tongue carcinoma | Tca-8113 |
| Kidney carcinoma | Ketr-3 | | |

| Table 14: Syngeneic Mouse Tumor Model | | |
|---|---|---|
| **Tumor Type** | **Cell Line Name** | **Strain of Mice** |
| Cervical carcinoma | U14 | ICR |
| Liver carcinoma | H22 | ICR |
| Lung carcinoma | Lewis | C57BL6 |
| Melanoma | B16F1, B16F10, B16BL6 | C57BL6 |
| Sarcoma | S180 | ICR |

The route of administration for the anti-EGFR antibodies, such as modified anti-EGFR antibodies, can be any route of administration described herein or known in the art, such as intraperitoneal, intratumoral or intravenous. The anti-EGFR antibodies can be administered at varying dosages described herein or known in the art. For example, the modified anti-EGFR antibodies can be administered to tumor-bearing animals at or between, for example, about 0.1 mg/kg, 0.15 mg/kg, 0.2 mg/kg, 0.25 mg/kg, 0.30 mg/kg, 0.35 mg/kg, 0.40 mg/kg, 0.45 mg/kg, 0.5 mg/kg, 0.55 mg.kg, 0.6 mg/kg, 0.7 mg/kg, 0.8 mg/kg, 0.9 mg/kg, 1.0 mg/kg, 1.1 mg/kg, 1.2 mg/kg, 1.3 mg/kg, 1.4 mg/kg, 1.5 mg/kg, 1.6 mg/kg, 1.7 mg/kg, 1.8 mg/kg, 1.9 mg/kg, 2 mg/kg, 2.5 mg/kg, 3 mg/kg, 3.5 mg/kg, 4 mg/kg, 4.5 mg/kg, 5 mg/kg, 5.5 mg/kg, 6 mg/kg, 6.5 mg/kg, 7 mg/kg, 7.5 mg/kg, 8 mg/kg, 8.5 mg/kg, 9 mg/kg, 9.5 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, 20 mg/kg, 21 mg/kg, 22 mg/kg, 23 mg/kg, 24 mg/kg, 25 mg/kg, 30 mg/kg, 40 mg/kg, 50 mg/kg, 60 mg/kg, 70 mg/kg, 80 mg/kg, 90 mg/kg, 100 mg/kg or more. In some examples, exemplary dosages include, but are not limited to, about or 0.01 $mg/m^2$ to about or 800 $mg/m^2$, such as for example, about or 0.01 $mg/m^2$, about or 0.1 $mg/m^2$, about or 0.5 $mg/m^2$, about or 1 $mg/m^2$, about or 5 $mg/m^2$, about or 10 $mg/m^2$, about or 15 $mg/m^2$, about or 20 $mg/m^2$, about or 25 $mg/m^2$, about or 30 $mg/m^2$, about or 35 $mg/m^2$, about or 40 $mg/m^2$, about or 45 $mg/m^2$, about or 50 $mg/m^2$, about or 100 $mg/m^2$, about or 150 $mg/m^2$, about or 200 $mg/m^2$, about or 250 $mg/m^2$, about or 300 $mg/m^2$, about or 400 $mg/m^2$, about or 500 $mg/m^2$, about or 600 $mg/m^2$ and about or 700 $mg/m^2$. It is understood that one of skill in the art can recognize and convert dosages between units of mg/kg and $mg/m^2$ (see, e.g., Michael J. Derelanko, TOXICOLOGIST'S POCKET HANDBOOK, CRC Press, p.16 (2000)).

Tumor size and volume can be monitored based on techniques known to one of skill in the art. For example, tumor size and volume can be monitored by radiography, ultrasound imaging, necropsy, by use of calipers, by microCT

or by $^{18}$F-FDG-PET. Tumor size also can be assessed visually. In particular examples, tumor size (diameter) is measured directly using calipers. In other examples, tumor volume can be measured using an average of measurements of tumor diameter (D) obtained by caliper or ultrasound assessments. The volume can be determined from the formula $V = D^3 \times \pi / 6$ (for diameter measured using calipers); the formula $V = [length \times (width)^2]/2$ where length is the longest diameter and width is the shortest diameter perpendicular to length; or $V = D^2 \times d \times \pi / 6$ (for diameter measured using ultrasound where d is the depth or thickness). For example, caliper measurements can be made of the tumor length (1) and width (w) and tumor volume calculated as length x width$^2$ x 0.52. In another example, microCT scans can be used to measure tumor volume (*see e.g.* Huang et al. (2009) PNAS, 106:3426-3430). In such an example, mice can be injected with Optiray Pharmacy ioversol injection 74% contrast medium (*e.g.* 741 mg of ioversol/mL), mice anesthetized, and CT scanning done using a MicroCat 1A scanner or other similar scanner (*e.g.* IMTek) (40 kV, 600 $\mu$A, 196 rotation steps, total angle or rotation = 196). The images can be reconstructed using software (*e.g.* RVA3 software program; ImTek). Tumor volumes can be determined by using available software (*e.g.* Amira 3.1 software; Mercury Computer Systems). In some examples, the tumor is injected subcutaneously at day 0, and the volume of the primary tumor can be measured at designated time points.

Once the implanted tumors reach a predetermined size or volume, the modified anti-EGFR antibody can be administered. Progressing tumors can be visualized and tumor size and tumor volume can be measured using any technique known to one of skill in the art. For example, tumor volume or tumor size can be measured using any of the techniques described herein. Tumor volume and size can be assessed or measured at periodic intervals over a period of time following administration of the modified anti-EGFR antibodies provided herein, such as, for example, every hour, every 6 hours, every 12 hours, every 24 hours, every 36 hours, every 2 days, every 3 days, every 4 days, every 5 days, every 6 days, every 7-days, every week, every 3 weeks, every month or more post-infection. A graph of the median change in tumor volume over time can be made. This is exemplified in Example 8. The total area under the curve (AUC) can be calculated. A therapeutic index also can be calculated using the formula $AUC_{untreated\ animals} - AUC_{treated\ animals}/AUC_{untreated} \times 100$.

Generally, tumor-bearing animals generated in the same manner, at the same time and with the same type of tumor cells are used as controls. Such control tumor-bearing animals include those that remain untreated (not administered modified anti-EGFR antibody). Additional controls animals include those administered an anti-EGFR antibody known in the art. Exemplary of such anti-EGFR antibodies is Cetuximab. In examples where tumor-bearing animals are administered a known anti-EGFR antibody as a control, the amount of control antibody administered can be the same as the amount of the modified anti-EGFR antibody.

Assessment of the activity of an anti-EGFR antibody, such as a modified anti-EGFR antibody, can include identifying antibodies that mediate a decrease in tumor size (*e.g.* diameter), volume or weight compared to control treated or untreated tumor-bearing animals. It is understood that a decrease in tumor size, volume or weight compared to control treated or untreated tumor-bearing animals means that the anti-EGFR antibody itself is mediating tumor regression or shrinkage or that the anti-EGFR antibody is mediating delayed tumor progression compared to control treated or untreated tumor-bearing animals. Tumor shrinkage or delay in tumor progression are parameters indicative of anti-tumorigenicity.

For example, a anti-EGFR antibody can be selected as mediating a decrease in tumor size or volume based on visual assessment of tumor size in the animal compared to control treated or untreated tumor-bearing animals. In other examples, a anti-EGFR antibody is selected as mediating a decrease in tumor size or volume if the tumor size is decreased in diameter as assessed by any measurement known in the art (*e.g.* use of calipers) compared to an untreated tumor-bearing animal or compared to a tumor-bearing animal treated with a reference anti-EGFR antibody. It is understood that comparison of tumor size or volume can be made at any predetermined time post-infection, and can be empirically determined by one of skill in the art. In some examples, a comparison can be made at the day in which the untreated control is sacrificed. In other examples, analysis of the total AUC can be made, and AUC values compared as an indicator of the size and volume of the tumor over the time period.

Effects of an anti-EGFR antibody, such as a modified anti-EGFR antibody, on tumor size or volume can be presented as a ratio of tumor size or volume at a designated time post-administration of the control treated animal compared to the anti-EGFR antibody-treated animal (tumor size or volume of control-treated animals / tumor size or volume of modified anti-EGFR antibody -treated animals). Assessment can include identifying a anti-EGFR antibody that results in animals exhibiting a ratio of tumor shrinkage that is greater than 1.0, for example, that is greater than 1.1, 1.2, 1.3, 1.4, 1.5, 1.6, 1.7, 1.8, 1.9, 2, 3, 4, 5, 6, 7, 8, 9, 10, 20, 30, 40, 50 or more. In particular examples, the results are presented as a ratio of the total AUC area during the course of treatment (AUC of tumor size or volume of control-treated animals/AUC tumor size or volume of modified anti-EGFR antibody-treated animals). A anti-EGFR antibody can be selected that results in a ratio of tumor shrinkage in a subject as measured by AUC that is greater than 1.1, 1.2, 1.3, 1.4, 1.5, 1.6, 1.7, 1.8, 1.9, 2, 3, 4, 5, 6, 7, 8, 9, 10, 20, 30, 40, 50 or more. It is understood that a ratio of 1.2 or 5 means that the modified anti-EGFR antibody effects a decreased tumor size or volume and results in 120% or 500% anti-tumorigenicity activity compared to the reference or control.

In particular examples, the therapeutic index is determined as a measure of effects of an anti-EGFR antibody,

such as a modified anti-EGFR antibody, on tumor size or volume. A anti-EGFR antibody can have a therapeutic index that is at least or about at least or 120%, 130%, 140%, 150%, 160%, 170%, 180%, 190%, 200%, 250%, 300%, 400%, 500%, 600%, 700%, 800% or more compared to the therapeutic index of a control anti-EGFR antibody.

In additional examples, tumors can be harvested from the animals and weighed. Administration of anti-EGFR antibodies can result in a decrease in tumor weight compared to tumor harvested from control tumor-bearing animals. The weight also can be compared to tumors harvested from control treated animals at the same time post-administration. The change in weight can be presented as a ratio of the tumor weight (tumor weight control treated animals/tumor weights of anti-EGFR-treated animals). A anti-EGFR antibody can result is subjects exhibiting a ratio of tumor weight that is greater than 1.0, for example, that is greater than 1.1, 1.2, 1.3, 1.4, 1.5, 1.6, 1.7, 1.8, 1.9, 2, 3, 4, 5, 6, 7, 8, 9, 10, 20, 30, 40, 50 or more. It is understood that a ratio of tumor weight that is 1.2 or 5 means that the anti-EGFR antibody effects a decreased tumor weight and results in 120% or 500% anti-tumorigenicity activity compared to the reference or control.

In particular examples, the effect of the anti-EGFR antibody on other organs or tissues in the animal can be assessed. For example, other organs can be harvested from the animals, weighed and/or examined.

**a. Assessing Side Effects**

Studies to assess safety and tolerability also are known in the art and can be used herein. Following administration of a anti-EGFR antibody, such as a modified anti-EGFR antibody, the development of any adverse reactions, such as any adverse reaction described herein or known in the art, can be monitored. Animal studies can be performed to assess adverse side effects, such as side effects that cannot be evaluated in a standard pharmacology profile or occur only after repeated administration of the modified anti-EGFR antibody. In some examples, such assays can be performed in two species--e.g., a rodent and a non-rodent--to ensure that any unexpected adverse effects are not overlooked. In general, these models can measure a variety of toxicities including genotoxicity, chronic toxicity, immunogenicity, reproductive/developmental toxicity, carcinogenicity.

Other parameters that can be measured to assess side effects include standard measurement of food consumption, bodyweight, antibody formation, clinical chemistry, and macro-and microscopic examination of standard organs/tissues (e.g. cardiotoxicity). Additional parameters of measurement include injection site trauma and the measurement of any neutralizing antibodies. The anti-EGFR antibodies can be evaluated for cross-reactivity with normal tissues, immunogenicity/antibody production and conjugate or linker toxicity. Such studies can be individualized to address specific concerns and follow the guidance set by ICH S6 (see, e.g., "Preclinical Safety Evaluation Of Biotechnology-Derived Pharmaceuticals," International Conference on Harmonisation Of Technical Requirements For Registration of Pharmaceuticals For Human Use, July 1997 (addendum June 2011)). As such, the general principles include that the products are sufficiently well characterized and for which impurities/contaminants have been removed, that the test material is comparable throughout development, and GLP compliance.

The anti-EGFR antibodies, such as modified anti-EGFR antibodies, provided herein can be assessed to identify those that result in subjects exhibiting reduced and/or fewer side effects, such as adverse side effects. For example, the anti-EGFR antibodies can be tested for parameters indicative of their side effects. The reduced side effects of a modified anti-EGFR antibody can include any side effect of anti-EGFR antibodies described herein or known in the art. Side effects can be assessed in healthy animal models or in animal models of a disease or condition, such as the animal models described herein.

In some examples the subjects are evaluated for properties indicative of a side effect of an anti-EGFR antibody, such as side effects described herein or known in the art, including skin toxicities and hypomagnesemia. For example, side effects of Cetuximab include any described herein and/or known to one of skill in the art, including symptomatic hypomagnesemia, paronychia, fever, dermatologic toxicity, papulopustular rash of the face and upper trunk, hair growth abnormalities, loss of scalp hair, increased growth of facial hair and eyelashes, dry and itchy skin, and periungual inflammation with tenderness (Eng (2009) Nat. Rev. 6:207-218; Schrag et al. J. Natl. Cancer Inst. 97(16):1221-1224; Lacouture, and Melosky (2007) Skin Therapy Lett. 12:1-5). In some examples, the side effects of Cetuximab include dermatological toxicities, including papulopustular eruption, dry skin, pruritus, ocular and nail changes, acneiform skin reaction, acneiform rash, acneiform follicular rash, acne-like rash, maculopapular skin rash, monomorphic pustular lesions, papulopustular reaction. (Lacouture, and Melosky (2007) Skin Therapy Lett. 12, 1-5) Other dermatological toxicities that can be associated with administration of an anti-EGFR antibody, such as Cetuximab include pruritus, erythema and paronychial inflammation. (Lacouture, and Melosky (2007) Skin Therapy Lett. 12, 1-5).

It is within the ability of the skilled artisan to identify and classify such side effects. In some examples, the side effects of the anti-EGFR antibodies provided herein are assessed by evaluating skin toxicities in animals. For example, as described elsewhere herein, hypomagnesemia can be diagnosed and/or assessed by measurement of serum magnesium levels. Papulopustular rash and acneiform rash can be characterized in animal models, such as mouse models and cynomolgus monkey models, by observing eruptions consisting of papules (a small, raised pimple) and pustules (a small pus filled blister). Dry skin, can be characterized by flaky and dull skin, fine pores, and papery thin skin texture. Skin hyperpigmentation can be characterized by darkening of the skin due to excessive melanin deposition. Pruritus can be evaluated by observing animal scratching. Paronychia can be evaluated by examination. For example, the presence of skin toxicities can be evaluated in mouse models in which human skin is grafted onto mice (see, e.g., Nanney et al. (1996) J. Invest. Dermatol. 106(6):1169-1174). It addition, dermatologic side effects can be assessed in other animal models. For example, in cynomolgus monkeys, inflammation at the injection site and desquamation of the external integument after cetuximab administration can be assessed. Similar effects can be observed in the epithelial mucosa of the nasal passage, esophagus, and tongue, and degenerative changes in the renal tubular epithelium. Other epithelial toxicities that can be assessed include conjunctivitis, reddened and swollen eyes, and signs of intestinal disturbance (*see, e.g.,* Lutterbuese et al. (2010) Proc. Natl. Acad. Sci. 107(28):12605-12610; European Medicines Agency (2009) Summary of product characteristics (Erbitux)).

Other adverse reactions that can be assessed in animal models include skin rash, injection site reactions, such as edema or swelling, headache, fever, fatigue, chills, flushing, dizziness, urticaria, wheezing or chest tightness, nausea, vomiting, rigors, back pain, chest pain, muscle cramps, seizures or convulsions, changes in blood pressure and anaphylactic or severe hypersensitivity responses. In some examples, properties indicative of a side effect of a modified anti-EGFR antibody include one or more properties such as survival of the subject, decrease in body weight, existence of side effects such as fever, rash or other allergy, fatigue or abdominal pain, induction of an immune response in the subject, tissue distribution of the antibody. Typically, a range of doses and different dosing frequencies can be administered in the safety and tolerability studies to assess the effect of increasing or decreasing concentrations of anti-EGFR antibody in the dose.

The type and severity of adverse reactions that develop in a patient or subject after administration of a anti-EGFR antibody provided herein can be assessed and compared to the adverse reactions that develop in a patient or subject after administration of another anti-EGFR antibody, such as any anti-EGFR antibody known in the art or described herein. The differences between adverse reactions that develop after administration of a anti-EGFR antibody provided herein and another anti-EGFR antibody can be assessed.

Hence, any of the parameters described herein can be assessed as indicative of toxicity/safety of a anti-EGFR antibody. Anti-EGFR antibodies can be selected that result in subjects exhibiting minimal toxicity. In an animal model to assess side effects, the dosages and methods of administration of a anti-EGFR antibody provided herein can include any dosages and methods of administration described herein. Control subjects can include those that are not administered an anti-EGFR antibody, or that are administered a reference anti-EGFR antibody, such as Cetuximab or a variant thereof.

## 5. Pharmacokinetics and Pharmacodynamics assays

Pharmacokinetics (PK) and pharmacodynamics (PD) assays of the anti-EGFR antibodies, such as modified anti-EGFR antibodies, provided herein can be performed using methods described herein or known in the art (*see, e.g.,* Klutchko, et al., (1998) J. Med. Chem. 41:3276-3292). Examples of parameters of measurement generally include the maximum (peak) plasma concentration ($C_{max}$), the peak time (i.e. when maximum plasma concentration occurs; $T_{max}$), the minimum plasma concentration (i.e. the minimum plasma concentration between doses; $C_{min}$), the elimination half-life ($T_{1/2}$) and area under the curve (i.e. the area under the curve generated by plotting time versus plasma concentration; AUC), following administration. The absolute bioavailability of administered modified anti-EGFR antibody can be determined by comparing the area under the curve following subcutaneous delivery ($AUC_{sc}$) with the AUC following intravenous delivery ($AUC_{iv}$). Absolute bioavailability (F), can be calculated using the formula: $F = ([AUC]_{sc} \times dose_{sc}) / ([AUC]_{iv} \times dose_{iv})$. The concentration of anti-EGFR antibody in the plasma following administration can be measured using any method known in the art suitable for assessing concentrations of antibody in samples of blood. Exemplary methods include, but are not limited to, ELISA and nephelometry. Additional measured parameters can include compartmental analysis of concentration-time data obtained following i.v. administration and bioavailability. Biodistribution, dosimetry (for radiolabeled antibodies or Fc fusions), and PK studies can also be done in animal models, including animal models described herein or known in the art, including rodent models. Such studies can evaluate tolerance at some or all doses administered, toxicity to local tissues, preferential localization to rodent xenograft animal models and depletion of target cells (e.g. CD20 positive cells). Pharmacodynamic studies can include, but are not limited to, targeting specific tumor cells or blocking signaling mechanisms, measuring depletion of EGFR expressing cells or signals.

PK and PD assays can be performed in any animal model described herein or known in the art, including healthy animal models, diseased animal models and humans. Screening the modified anti-EGFR antibodies for PD and/or PK properties can be useful for defining the optimal balance of PD, PK, and therapeutic efficacy conferred by the modified anti-EGFR antibodies. For example, it is known in the art that the array of Fc receptors is differentially expressed on various immune cell types, as well as in different tissues. Differential tissue distribution of Fc receptors can affect the pharmacodynamic (PD) and pharmacokinetic (PK) properties of the modified anti-EGFR antibodies provided herein.

A range of doses and different dosing frequency of dosing can be administered in the pharmacokinetic studies to assess the effect of increasing or decreasing concentrations of the modified anti-EGFR antibody in the dose. Pharmacokinetic properties, such as bioavailability, of the administered modified anti-EGFR antibody, can be assessed with or without co-administration of a therapeutic agent or regimen described herein. For example, dogs, such as beagles, can be administered a modified anti-EGFR antibody alone or with one or more therapeutic agents or regimens described herein. The modified anti-EGFR antibody can be administered before, during or after administration of a therapeutic agent or regimen. Blood samples can then be taken at various time points and the amount of modified anti-EGFR antibody in the plasma determined, such as by nephelometry. The AUC can then be measured and the bioavailability of administered modified anti-EGFR antibody with or without co-administration of the additional therapeutic agent(s) or regimen(s) can be determined. Such studies can be performed to assess the effect of co-administration on pharmacokinetic properties, such as bioavailability, of administered anti-EGFR antibody.

Single or repeated administration(s) of the modified anti-EGFR antibodies can occur over a dose range of about 6000-fold (about 0.05-300 mg/kg) to evaluate the half-life using plasma concentration and clearance as well as volume of distribution at a steady state and level of systemic absorbance can be measured.

## E. METHODS OF IDENTIFYING GENERATING AND PRODUCING ANTI-EGFR ANTIBODIES

### 1. Identifying Conditionally Therapeutic Proteins

Conditionally active therapeutic proteins, for example antibodies, such as modified anti-EGFR antibodies provided herein, that are more active in a diseased microenvironment than in a non-diseased microenvironment (such as a healthy or normal environment) can be identified by any assay that permits quantitation of and assessment of activity under conditions present in the different environments. Such assays are described in Section D above. The activity can be compared and those therapeutic proteins that are more active in the diseased microenvironment (or under conditions present in the diseased environment) than in a normal environment (or under conditions present in the non-diseased or normal environment) can be identified. The result is that therapeutic proteins are identified whose activity is conditionally targeted to the diseased microenvironment, such that unwanted systemic effects, such as side effects or unwanted activity, is reduced or minimized.

As described in detail in Section D, such assays can be performed *in vivo* or *in vitro*. For example, assays to identify conditionally active molecules can be performed *in vitro* by manipulation of one or more conditions of buffers to contain or mimic one or more conditions present in a diseased microenvironment that are different than those present in a non-diseased or healthy or normal environment. For the tumor environment, these conditions include low or acidic pH, such as pH 5.8 to 6.8 (e.g. pH 6.0 to 6.5) and/or elevated lactate concentration (e.g. 10 mM to 16 mM). In contrast, conditions in the non-tumor microenvironment include neutral pH (e.g. pH 7.0 to 7.4) and/or lactate concentration of 1 mM to 5 mM. Exemplary of such assays of identifying conditionally active therapeutic proteins are described in U.S. Application Serial No. 13/200,666 and International Application No. PCT/US11/50891. Also conditions are modeled to simulate or mimic physiologic conditions to include 20-50% serum (vol/vol) or 10-50 mg/mL protein (e.g. serum albumin). Such methods can be used to identify conditionally active anti-cancer agents, including conditionally active antibodies, for example anti-EGFR antibodies, and other agents so that such agents are more active in the tumor. Similar methods also can be performed to identify any conditionally active therapeutic protein, such as anti-inflammatory agents, for example, infliximab (Remicade), etanercept (Enbrel), and other similar agents, in order to reduce systemic immunosuppressive activities.

### 2. Generating and Producing Anti-EGFR Antibodies

Anti-EGFR antibodies, such as the modified anti-EGFR antibodies provided herein, can be expressed using standard cell culture and other expression systems known in the art. Prior to use in the methods provided herein, the proteins can be purified. Alternatively, whole supernatant or diluted supernatant can be screened in the dual assay herein.

The anti-EGFR antibodies, such as modified anti-EGFR antibodies, provided herein can be produced by recombinant DNA methods that are within the purview of those skilled in the art. DNA encoding an anti-EGFR antibody can be synthetically produced or can be readily isolated and sequenced using conventional procedures (e.g. by using oligonucleotide probes that are capable of binding specifically to genes encoding the heavy and light chains of the antibody). For example, any cell source known to produce or express an anti-EGFR antibody can serve as a preferred source of such DNA. In another example, once the sequence of the DNA encoding the anti-EGFR is determined, nucleic acid sequences can be constructed using gene synthesis techniques.

Further, mutagenesis techniques also can be employed to generate modified forms of an anti-EGFR antibody. The DNA also can be modified. For example, gene synthesis or routine molecular biology techniques can be used to effect insertion, deletion, addition or replacement of nucleotides. For example, additional nucleotide sequences can be joined to a nucleic acid sequence. In one example linker sequences can be added, such as sequences containing restriction endonuclease sites for the purpose of cloning the antibody gene into a vector, for example, a protein expression vector. Furthermore, additional nucleotide sequences specifying functional DNA elements can be operatively linked to a nucleic acid molecule. Examples of such sequences include, but are not limited to, promoter sequences designed to facilitate intracellular protein expression, and leader peptide sequences designed to facilitate protein secretion.

Anti-EGFR antibodies, such as the modified anti-EGFR antibodies provided herein, can be expressed as full-length proteins or less than full length proteins. For example, antibody fragments can be expressed. Nucleic acid molecules and proteins provided herein can be made by any method known to one of skill in the art. Such procedures are routine and are well known to the skill artisan. They include routine molecular biology techniques including gene synthesis, PCR, ligation, cloning, transfection and purification techniques. A description of such procedures is provided below.

Once isolated, the DNA can be placed into expression vectors, which are then transfected into host cells. Choice of vector can depend on the desired application. For example, after insertion of the nucleic acid, the vectors typically are used to transform host cells, for example, to amplify the protein genes for replication and/or expression thereof. In such examples, a vector suitable for high level expression is used.

For expression of antibodies, generally, nucleic acid encoding the heavy chain of an antibody is cloned into a vector and the nucleic acid encoding the light chain of an antibody is cloned into a vector. The genes can be cloned into a single vector for dual expression thereof, or into separate vectors. If desired, the vectors also can contain further sequences encoding additional constant region(s) or hinge regions to generate other antibody forms. The vectors can be transfected and expressed in host cells. Expression can be in any cell expression system known to one of skill in the art. For example, host cells include cells that do not otherwise produce immunoglobulin protein, to obtain the synthesis of antibodies in the recombinant host cells. For example, host cells include, but are not limited to simian COS cells, Chinese hamster ovary (CHO) cells, 293FS cells, HEK293-6E cells, NSO cells or other myeloma cells. Other expression vectors and host cells are described herein.

In one example, nucleic acid encoding the heavy chain of an antibody, is ligated into a first expression vector and nucleic acid encoding the light chain of an antibody is ligated into a second expression vector. The expression vectors can be the same or different, although generally they are sufficiently compatible to allow comparable expression of proteins (heavy and light chain) therefrom. The first and second expression vectors are generally co-transfected into host cells, typically at a 1:1 ratio. Exemplary of vectors include, but are not limited to, pγ1HC and pκLC (Tiller et al. (2008) J Immunol. Methods, 329:112-24). Other expression vectors include the light chain expression vector pAG4622 and the heavy chain expression vector pAH4604 (Coloma et al. (1992) J Immunol. Methods, 152:89-104). The pAG4622 vector contains the genomic sequence encoding the C-region domain of the human κ L chain and the gpt selectable marker. The pAH4604 vectors contains the hisD selectable marker and sequences encoding the human H chain γ1 C-region domain. In another example, the heavy and light chain can be cloned into a single vector that has expression cassettes for both the heavy and light chain.

Hence, anti-EGFR antibodies, such as modified anti-EGFR antibodies, provided herein can be generated or expressed as full-length antibodies or as antibodies that are less than full length, including, but not limited to antigen-binding fragments thereof, such as, for example, Fab, Fab', Fab hinge, F(ab')$_2$, single-chain Fv (scFv), scFv tandem, Fv, dsFv, scFv hinge, scFv hinge($\Delta$E) diabody, Fd and Fd' fragments. Various techniques have been developed for the production of antibody fragments. Traditionally, these fragments were derived via proteolytic digestion of intact antibodies (see e.g. Morimoto et al. (1992) Journal of Biochemical and Biophysical Methods, 24:107-117; Brennance et al. (1985) Science, 229:81). Fragments also can be produced directly by recombinant host cells. For example, Fab, Fv and scFv antibody fragments can all be expressed in and secreted from host cells, such as *E. coli*, thus allowing the facile production of large amounts of these fragments. Also, Fab'-SH fragments can be chemically coupled to form F(ab')$_2$ fragments (Carter et al. (1992) Bio/Technology, 10:163-167). According to another approach, F(ab')$_2$ fragments can be isolated directly from recombinant host cell culture. In some examples, the modified anti-EGFR antibody is a single chain Fv fragment (scFv) (see e.g. WO93/16185; US Patent No. 5,571,894 and U.S. Patent No. 5,587,458). Fv and scFv are the only species with intact combining sites that are devoid of constant regions; thus, they are suitable for reduced nonspecific binding during *in vivo* use. scFv fusion proteins can be constructed to yield fusion of an effector protein at either the amino or the carboxy terminus of an scFv. The antibody fragment can also be a linear antibody (see e.g. U.S. Patent No. 5,641,870). Such linear antibody fragments can be monospecific or bispecific. Other techniques for the production of antibody fragments are known to one of skill in the art.

For example, upon expression, antibody heavy and light chains pair by disulfide bond to form a full-length antibody or fragments thereof. For example, for expression of a full-length Ig, sequences encoding the $V_HC_H1$-hinge-$C_H2$-$C_H3$ can be cloned into a first expression vector and sequences encoding the $V_L$-$C_L$ domains can be cloned into a second expression vector. Upon co-expression with the second expression vector encoding the $V_L$-$C_L$ domains, a full-length antibody is expressed. In another example, to generate a Fab, sequences encoding the $V_H$-$C_H1$ can be cloned into a first expression vector and sequences encoding the $V_L$-$C_L$ domains can be cloned into a second expression vector. The heavy chain pairs with a light chain and a Fab monomer is generated. Sequences of $C_H1$, hinge, $C_H2$ and/or $C_H3$ of various IgG sub-types are known to one of skill in the art (see e.g. U.S. Published Application No. 20080248028).

Similarly, sequences of $C_L$, lambda or kappa, also are known (see e.g. U.S. Published Application No. 20080248028). Exemplary of such sequences are provided herein.

Exemplary sequences that can be inserted into vectors for expression of whole antibodies and antibody fragments include sequences of antibody fragments described herein (see, e.g., and SEQ ID NOS:30-1068, 1093, 1098-1107, 1112-1131 and 1134-1159). For example, the variable heavy chain and variable light chain sequences of Cetuximab (SEQ ID NOS: 3 and 4, respectively) or the variable heavy chain and variable light chain sequences of any antibody as described herein (e.g., SEQ ID NOS: 30-1068, 1093, 1098-1107, 1112-1131 and 1134-1159, respectively) can be inserted into a suitable expression vector described herein or known to one of skill in the art. All or a portion of the constant region of the heavy chain or light chain also can be inserted or contained in the vector for expression of IgG antibodies or fragments thereof. In addition, $V_H$-$C_H$1 and $V_L$-$C_L$ sequences can be inserted into a suitable expression vector for expression of Fab molecules. Variable heavy chain and variable light chain domains of an antibody (i.e., SEQ ID NOS: 30-1068, 1093, 1098-1107, 1112-1131 and 1134-1159, respectively) can be expressed in a suitable expression vector, such as a vector encoding for a linker between the variable heavy chain and variable light chain to produce single chain antibodies. Exemplary linkers include the glycine rich flexible linkers (-$G_4$S-)$_n$, where n is a positive integer, such as 1 (SEQ ID NO:1094), 2 (SEQ ID NO:1095), 3 (SEQ ID NO: 21), 4 (SEQ ID NO: 1096), 5 (SEQ ID NO: 1097), or more.

**a. Vectors**

Choice of vector can depend on the desired application. Many expression vectors are available and known to those of skill in the art for the expression of anti-EGFR antibodies or portions thereof, such as antigen binding fragments. The choice of an expression vector is influenced by the choice of host expression system. Such selection is well within the level of skill of the skilled artisan. In general, expression vectors can include transcriptional promoters and optionally enhancers, translational signals, and transcriptional and translational termination signals. Expression vectors that are used for stable transformation typically have a selectable marker which allows selection and maintenance of the transformed cells. In some cases, an origin of replication can be used to amplify the copy number of the vectors in the cells. Vectors also generally can contain additional nucleotide sequences operably linked to the ligated nucleic acid molecule (e.g. His tag, Flag tag). For applications with antibodies, vectors generally include sequences encoding the constant region. Thus, antibodies or portions thereof also can be expressed as protein fusions. For example, a fusion can be generated to add additional functionality to a polypeptide. Examples of fusion proteins include, but are not limited to, fusions of a signal sequence, an epitope tag such as for localization, e.g. a $His_6$ tag or a myc tag, or a tag for purification, for example, a GST fusion, and a sequence for directing protein secretion and/or membrane association.

For example, expression of the anti-EGFR antibodies, such as modified anti-EGFR antibodies, can be controlled by any promoter/enhancer known in the art. Suitable bacterial promoters are well known in the art and described herein below. Other suitable promoters for mammalian cells, yeast cells and insect cells are well known in the art and some are exemplified below. Selection of the promoter used to direct expression of a heterologous nucleic acid depends on the particular application. Promoters which can be used include but are not limited to eukaryotic expression vectors containing the SV40 early promoter (Bernoist and Chambon, Nature 290:304-310 (1981)), the promoter contained in the 3' long terminal repeat of Rous sarcoma virus (Yamamoto et al. Cell 22:787-797 (1980)), the herpes thymidine kinase promoter (Wagner et al., Proc. Natl. Acad. Sci. USA 78:1441-1445 (1981)), the regulatory sequences of the metallothionein gene (Brinster et al., Nature 296:39-42 (1982)); prokaryotic expression vectors such as the β-lactamase promoter (Jay et al., (1981) Proc. Natl. Acad. Sci. USA 78:5543) or the *tac* promoter (DeBoer et al., Proc. Natl. Acad. Sci. USA 80:21-25 (1983)); see also "Useful Proteins from Recombinant Bacteria": in Scientific American 242:79-94 (1980)); plant expression vectors containing the nopaline synthetase promoter (Herrera-Estrella et al., Nature 303:209-213 (1984)) or the cauliflower mosaic virus 35S RNA promoter (Gardner et al., Nucleic Acids Res. 9:2871 (1981)), and the promoter of the photosynthetic enzyme ribulose bisphosphate carboxylase (Herrera-Estrella et al., Nature 310:115-120 (1984)); promoter elements from yeast and other fungi such as the Gal4 promoter, the alcohol dehydrogenase promoter, the phosphoglycerol kinase promoter, the alkaline phosphatase promoter, and the following animal transcriptional control regions that exhibit tissue specificity and have been used in transgenic animals: elastase I gene control region which is active in pancreatic acinar cells (Swift et al., Cell 38:639-646 (1984); Ornitz et al., Cold Spring Harbor Symp. Quant. Biol. 50:399-409 (1986); MacDonald, Hepatology 7:425-515 (1987)); insulin gene control region which is active in pancreatic beta cells (Hanahan et al., Nature 315:115-122 (1985)), immunoglobulin gene control region which is active in lymphoid cells (Grosschedl et al., Cell 38:647-658 (1984); Adams et al., Nature 318:533-538 (1985); Alexander et al., Mol. Cell Biol. 7:1436-1444 (1987)), mouse mammary tumor virus control region which is active in testicular, breast, lymphoid and mast cells (Leder et al., Cell 45:485-495 (1986)), albumin gene control region which is active in liver (Pinkert et al., Genes and Devel. 1:268-276 (1987)), alpha-fetoprotein gene control region which is active in liver (Krumlauf et al., Mol. Cell. Biol. 5:1639-1648 (1985); Hammer et al., Science 235:53-58 1987)), alpha-1 antitrypsin gene control region which is active in liver (Kelsey et al., Genes and Devel. 1:161-171 (1987)), beta globin gene control region which is active in myeloid cells (Magram et al., Nature 315:338-340 (1985); Kollias et al., Cell 46:89-94 (1986)), myelin basic protein gene control region which is active in oligodendrocyte cells of the brain (Readhead et al., Cell 48:703-712 (1987)), myosin light chain-2 gene control region which is active in skeletal muscle (Shani, Nature 314:283-286 (1985)), and gonadotrophic releasing hormone gene control region which is active in gonadotrophs of the hypothalamus (Mason et al., Science 234:1372-1378 (1986)).

In addition to the promoter, the expression vector typically contains a transcription unit or expression cassette that contains all the additional elements required for the expression of the antibody, or portion thereof, in host cells. A typical expression cassette contains a promoter operably linked to the nucleic acid sequence encoding the protein and signals required for efficient polyadenylation of the transcript, ribosome binding sites and translation termination. Additional elements of the cassette can include enhancers. In addition, the cassette typically contains a transcription termination region downstream of the structural gene to provide for efficient termination. The termination region can be obtained from the same gene as the promoter sequence or can be obtained from different genes.

Some expression systems have markers that provide gene amplification such as thymidine kinase and dihydrofolate reductase. Alternatively, high yield expression systems not involving gene amplification are also suitable, such as using a baculovirus vector in insect cells, with a nucleic acid sequence encoding a protein under the direction of the polyhedron promoter or other strong baculovirus promoter.

For purposes herein with respect to expression of anti-EGFR antibodies, such as modified anti-EGFR antibodies, vectors can contain a sequence of nucleotides that encodes a constant region of an antibody operably linked to the nucleic acid sequence encoding the variable region of the antibody. The vector can include the sequence for one or all of a $C_H1$, $C_H2$, hinge, $C_H3$ or $C_H4$ and/or $C_L$. Generally, such as for expression of Fabs, the vector contains the sequence for a $C_H1$ or $C_L$ (kappa or lambda light chains). The sequences of constant regions or hinge regions are known to one of skill in the art (see e.g. U.S. Published Application No. 20080248028). Exemplary of such sequences are provided herein.

Exemplary expression vectors include any mammalian expression vector such as, for example, pCMV. For bacterial expression, such vectors include pBR322, pUC, pSKF, pET23D, and fusion vectors such as MBP, GST and LacZ. Other eukaryotic vectors, for example any containing regulatory elements from eukaryotic viruses can be used as eukaryotic expression vectors. These include, for example, SV40 vectors, papilloma virus vectors, and vectors derived from Epstein-Bar virus. Exemplary eukaryotic vectors include pMSG, pAV009/A+, pMT010/A+, pMAMneo-5, baculovirus pDSCE, and any other vector allowing expression of proteins under the direction of the CMV promoter, SV40 early promoter, SV40 late promoter, metallothionein promoter, murine mammary tumor virus promoter, Rous sarcoma virus promoter, polyhedron promoter, or other promoters shown effective for expression in eukaryotes.

Any methods known to those of skill in the art for the insertion of DNA fragments into a vector can be used to construct expression vectors containing a nucleic acid encoding a protein or an antibody chain. These methods can include *in vitro* recombinant DNA and synthetic techniques and *in vivo* recombinants (genetic recombination). The insertion into a cloning vector can, for example, be accomplished by ligating the DNA fragment into a cloning vector which has complementary cohesive termini. If the complementary restriction sites used to fragment the DNA are not present in the cloning vector, the ends of the DNA molecules can be enzymatically modified. Alternatively, any site desired can be produced by ligating nucleotide sequences (linkers) onto the DNA termini; these ligated linkers can contain specific chemically synthesized nucleic acids encoding restriction endonuclease recognition sequences.

**b. Cells and Expression Systems**

Generally, any cell type that can be engineered to express heterologous DNA and has a secretory pathway is suitable for expression of the modified anti-EGFR antibodies provided herein. Expression hosts include prokaryotic and eukaryotic organisms such as bacterial cells (e.g. *E. coli*), yeast cells, fungal cells, Archaea, plant cells, insect cells and animal cells including human cells. Expression hosts can differ in their protein production levels as well as the types of post-translational modifications that are present on the expressed proteins. Further, the choice of expression host is often related to the choice of vector and transcription and translation elements used. For example, the choice of expression host is often, but not always, dependent on the choice of precursor sequence utilized. For example, many heterologous signal sequences can only be expressed in a host cell of the same species (i.e., an insect cell signal sequence is optimally expressed in an insect cell). In contrast, other signal sequences can be used in heterologous hosts such as, for example, the human serum albumin (hHSA) signal sequence which works well in yeast, insect, or mammalian host cells and the tissue plasminogen activator pre/pro sequence which has been demonstrated to be functional in insect and mammalian cells (Tan et al., (2002) Protein Eng. 15:337). The choice of expression host can be made based on these and other factors, such as regulatory and safety considerations, production costs and the need and methods for purification. Thus, the vector system must be compatible with the host cell used.

Expression in eukaryotic hosts can include expression in yeasts such as *Saccharomyces cerevisiae* and *Pichia pastoris,* insect cells such as *Drosophila* cells and *lepidopteran* cells, plants and plant cells such as tobacco, corn, rice, algae, and lemna. Eukaryotic cells for expression also include mammalian cells lines such as Chinese hamster ovary (CHO) cells or baby hamster kidney (BHK) cells. Eukaryotic expression hosts also include production in transgenic animals, for example, including production in serum, milk and eggs.

Recombinant molecules can be introduced into host cells via, for example, transformation, transfection, infection, electroporation and sonoporation, so that many copies of the gene sequence are generated. Generally, standard transfection methods are used to produce bacterial, mammalian, yeast, or insect cell lines that express large quantity of antibody chains, which is then purified using standard techniques (see e.g., Colley et al. (1989) J. Biol. Chem., 264:17619-17622; Guide to Protein Purification, in Methods in Enzymology, vol. 182 (Deutscher, ed.), 1990). Transformation of eukaryotic and prokaryotic cells are performed according to standard techniques (see, e.g., Morrison (1977) J. Bact. 132:349-351; Clark-Curtiss and Curtiss (1983) Methods in Enzymology, 101, 347-362). For example, any of the well-known procedures for introducing foreign nucleotide sequences into host cells can be used. These include the use of calcium phosphate transfection, polybrene, protoplast fusion, electroporation, biolistics, liposomes, microinjection, plasma vectors, viral vectors and any other the other well known methods for introducing cloned genomic DNA, cDNA, synthetic DNA or other foreign genetic material into a host cell. Generally, for purposes of expressing an antibody, host cells are transfected with a first vector encoding at least a $V_H$ chain and a second vector encoding at least a $V_L$ chain. Thus, it is only necessary that the particular genetic engineering procedure used be capable of successfully introducing at least both genes into the host cell capable of expressing antibody polypeptide, or modified form thereof.

Anti-EGFR antibodies, such as modified anti-EGFR antibodies, provided herein can be produced by any methods known in the art for protein production including *in vitro* and *in vivo* methods such as, for example, the introduction of nucleic acid molecules encoding antibodies into a host cell or host animal and expression from nucleic acid molecules encoding recombined antibodies *in vitro*. Prokaryotes, especially *E. coli,* provide a system for producing large amounts of reassembled antibodies or portions thereof, and are particularly desired in applications of expression and purification of proteins. Transformation of *E. coli* is a simple and rapid technique well known to those of skill in the art. *E. coli* host strains for high throughput expression include, but are not limited to, BL21 (EMD Biosciences) and LMG194 (ATCC). Exemplary of such an *E. coli* host strain is BL21. Vectors for high throughput expression include, but are not limited to, pBR322 and pUC vectors.

### i. Prokaryotic Expression

Prokaryotes, especially *E. coli,* provide a system for producing large amounts of modified anti-EGFR antibodies, or portions thereof. Transformation of *E. coli* is a simple and rapid technique well known to those of skill in the art. Expression vectors for *E. coli* can contain inducible promoters that are useful for inducing high levels of protein expression and for expressing antibodies that exhibit some toxicity to the host cells. Examples of inducible promoters include the lac promoter, the trp promoter, the hybrid tac promoter, the T7 and SP6 RNA promoters and the temperature regulated $\lambda P_L$ promoter.

Antibodies or portions thereof can be expressed in the cytoplasmic environment of *E. coli.* The cytoplasm is a reducing environment and for some antibodies, this can result in the formation of insoluble inclusion bodies. Reducing agents such as dithiothreitol and β-mercaptoethanol and denaturants (e.g., such as guanidine-HCl and urea) can be used to resolubilize the antibodies. An exemplary alternative approach is the expression of recombined antibodies or fragments thereof in the periplasmic space of bacteria which provides an oxidizing environment and chaperonin-like and disulfide isomerases leading to the production of soluble protein. Typically, a leader sequence is fused to the protein to be expressed which directs the protein to the periplasm. The leader is then removed by signal peptidases inside the periplasm. Exemplary pathways to translocate expressed proteins into the periplasm are the Sec pathway, the SRP pathway and the TAT pathway. Examples of periplasmic-targeting leader sequences include the pelB leader from the pectate lyase gene, the StII leader sequence, and the DsbA leader sequence. In some cases, periplasmic expression allows leakage of the expressed protein into the culture medium. The secretion of antibodies allows quick and simple purification from the culture supernatant. Antibodies that are not secreted can be obtained from the periplasm by osmotic lysis. Similar to cytoplasmic expression, in some cases proteins can become insoluble and denaturants and reducing agents can be used to facilitate solubilization and refolding. Temperature of induction and growth also can influence expression levels and solubility. Typically, temperatures between 25 °C and 37 °C are used. Mutations also can be used to increase solubility of expressed proteins. Typically, bacteria produce aglycosylated proteins. Thus, glycosylation can be added *in vitro* after purification from host cells.

### ii. Yeast

Yeasts such as *Saccharomyces cerevisiae, Schizosaccharomyces pombe, Yarrowia lipolytica, Kluyveromyces lactis,* and *Pichia pastoris* are useful expression hosts for recombined antibodies or portions thereof. Yeast can be transformed with episomal replicating vectors or by stable chromosomal integration by homologous recombination. Typically, inducible promoters are used to regulate gene expression. Examples of such promoters include AOX1, GAL1, GAL7, and GAL5 and metallothionein promoters such as CUP1. Expression vectors often include a selectable marker such as LEU2, TRP1, HIS3, and URA3 for selection and maintenance of the transformed DNA. Proteins expressed in yeast are often soluble. Co-expression with chaperonins such as Bip and protein disulfide isomerase can improve expression levels and solubility. Additionally, proteins expressed in yeast can be directed for secretion using secretion signal peptide fusions such as the yeast mating type alpha-factor secretion signal from *Saccharomyces cerevisae* and fusions with yeast cell surface proteins such as the Aga2p mating adhesion receptor or the *Arxula adeninivorans* glucoamylase. A protease cleavage site such as for the Kex-2 protease, can be engineered to remove the fused sequences from the expressed polypeptides as they exit the secretion pathway. Yeast also is capable of glycosylation at Asn-X-Ser/Thr motifs.

### iii. Insects

Insect cells, particularly using baculovirus expression, are useful for expressing modified anti-EGFR antibodies or portions thereof. Insect cells express high levels of protein and are capable of most of the post-translational modifications used by higher eukaryotes. Baculovirus have a restrictive host range which can improve the safety and reduce regulatory concerns of eukaryotic expression. Typical expression vectors use a promoter for high level expression such as the polyhedrin promoter and p10 promoter of baculovirus. Commonly used baculovirus systems include the baculoviruses such as *Autographa californica* nuclear polyhedrosis virus (AcNPV), and the *Bombyx mori* nuclear polyhedrosis virus (BmNPV) and an insect cell line such as Sf9 derived from *Spodoptera frugiperda* and TN derived from *Trichoplusia ni*. For high-level expression, the nucleotide sequence of the molecule to be expressed can be fused immediately downstream of the polyhedrin initiation codon of the virus. To generate baculovirus recombinants capable of expressing human antibodies, a dual-expression transfer, such as pAcUW51 (PharMingen) is utilized. Mammalian secretion signals are accurately processed in insect cells and can be used to secrete the expressed protein into the culture medium.

An alternative expression system in insect cell for expression of the modified anti-EGFR antibodies provided herein is the use of stably transformed cells. Cell lines such as Sf9 derived cells from *Spodoptera frugiperda* and TN derived cells from *Trichoplusia ni* can be used for expression. The baculovirus immediate early gene promoter IE1 can be used to induce consistent levels of expression. Typical expression vectors include the pIE1-3 and pI31-4 transfer vectors (Novagen). Expression vectors are typically maintained by the use of selectable markers such as neomycin and hygromycin.

### iv. Mammalian Cells

Mammalian expression systems can be used to express anti-EGFR antibodies, such as modified anti-EGFR antibodies, including antigen-binding fragments thereof. Expression constructs can be transferred to mammalian cells by viral infection such as adenovirus or by direct DNA transfer such as liposomes, calcium phosphate, DEAE-dextran and by physical means such as electroporation and microinjection. Expression vectors for mammalian cells typically include an mRNA cap site, a TATA box, a translational initiation sequence (Kozak consensus sequence) and polyadenylation elements. Such vectors often include transcriptional promoter-enhancers for high-level expression, for example the SV40 promoter-enhancer, the human cytomegalovirus (CMV) promoter and the long terminal repeat of Rous sarcoma virus (RSV). These promoter-enhancers are active in many cell types. Tissue and cell-type promoters and enhancer regions also can be used for expression. Exemplary promoter/enhancer regions include, but are not limited to, those from genes such as elastase I, insulin, immunoglobulin, mouse mammary tumor virus, albumin, alpha fetoprotein, alpha 1 antitrypsin, beta globin, myelin basic protein, myosin light chain 2, and gonadotropic releasing hormone gene control. Selectable markers can be used to select for and maintain cells with the expression construct. Examples of selectable marker genes include, but are not limited to, hygromycin B phosphotransferase, adenosine deaminase, xanthine-guanine phosphoribosyl transferase, aminoglycoside phosphotransferase, dihydrofolate reductase and thymidine kinase. Modified anti-EGFR antibodies can be produced, for example, using a NEO$^R$/G418 system, a dihydrofolate reductase (DHFR) system or a glutamine synthetase (GS) system. The GS system uses joint expression vectors, such as pEE12/pEE6, to express both heavy chain and light chain. Fusion with cell surface signaling molecules such as TCR-$\zeta$ and Fc$_\varepsilon$RI-$\gamma$ can direct expression of the proteins in an active state on the cell surface.

Many cell lines are available for mammalian expression including mouse, rat human, monkey, chicken and hamster cells. Exemplary cell lines include any known in the art or described herein, such as, for example, CHO, Balb/3T3, HeLa, MT2, mouse NS0 (nonsecreting) and other myeloma cell lines, hybridoma and heterohybridoma cell lines, lymphocytes, fibroblasts, Sp2/0, COS, NIH3T3, HEK293, 293S, 2B8, and HKB cells. Cell lines adapted to serum-free media which facilitates purification of secreted proteins from the cell culture media are also available. One such example is the serum free EBNA-1 cell line (Pham et al., (2003) Biotechnol. Bioeng. 84:332-42.)

**v. Plants**

Transgenic plant cells and plants can be used to express anti-EGFR antibodies, such as modified anti-EGFR antibodies, or a portion thereof described herein. Expression constructs are typically transferred to plants using direct DNA transfer such as microprojectile bombardment and PEG-mediated transfer into protoplasts, and with agrobacterium-mediated transformation. Expression vectors can include promoter and enhancer sequences, transcriptional termination elements and translational control elements. Expression vectors and transformation techniques are usually divided between dicot hosts, such as *Arabidopsis* and tobacco, and monocot hosts, such as corn and rice. Examples of plant promoters used for expression include the cauliflower mosaic virus CaMV 35S promoter, the nopaline synthase promoter, the ribose bisphosphate carboxylase promoter and the maize ubiquitin-1 (*ubi*-1) promoter promoters. Selectable markers such as hygromycin, phosphomannose isomerase and neomycin phosphotransferase are often used to facilitate selection and maintenance of transformed cells. Transformed plant cells can be maintained in culture as cells, aggregates (callus tissue) or regenerated into whole plants. Transgenic plant cells also can include algae engineered to produce proteases or modified proteases (see for example, Mayfield et al. (2003) PNAS 100:438-442). Because plants have different glycosylation patterns than mammalian cells, this can influence the choice of protein produced in these hosts.

**3. Purification**

Anti-EGFR antibodies, such as modified anti-EGFR antibodies and antigen binding portions thereof, can be purified by any procedure known to one of skill in the art or described herein. Proteins can be purified to substantial purity using standard protein purification techniques known in the art including but not limited to, SDS-PAGE, size fraction and size exclusion chromatography, ammonium sulfate precipitation, chelate chromatography, ionic exchange chromatography or column chromatography. For example, antibodies can be purified by column chromatography. Exemplary of a method to purify the anti-EGFR antibodies provided herein is by using column chromatography, wherein a solid support column material is linked to Protein G, a cell surface-associated protein from *Streptococcus,* that binds immunoglobulins with high affinity. In some examples, the anti-EGFR antibodies can be purified by column chromatography, wherein a solid support column material is linked to Protein A, a cell surface-associated protein from *Staphylococcus* that binds immunoglobulins, such as IgG antibodies, with high affinity (see, e.g., Liu et al. (2010) MAbs 2(5):480-499). Other immunoglobulin-binding bacterial proteins that can be used to purify the anti-EGFR antibodies provided herein include Protein A/G, a recombinant fusion protein that combines the IgG binding domains of Protein A and Protein G; and Protein L, a surface protein from *Peptostreptococcus* (Bjorck (1988) J. Immunol., 140(4):1194-1197; Kastern, et al. (1992) J. Biol. Chem. 267(18):12820-12825; Eliasson et al. (1988) J. Biol. Chem. 263:4323-4327).

The anti-EGFR antibodies can be purified to 60%, 70%, 80% purity and typically at least 90%, 91%, 92%, 93%, 94%, 95%, 96%, 97%, 98% or 99% purity. Purity can be assessed by standard methods such as by SDS-PAGE and coomassie staining.

Methods for purification of anti-EGFR antibodies, including antibodies or portions thereof from host cells depend on the chosen host cells and expression systems. For secreted molecules, proteins are generally purified from the culture media after removing the cells. For intracellular expression, cells can be lysed and the proteins purified from the extract. When transgenic organisms such as transgenic plants and animals are used for expression, tissues or organs can be used as starting material to make a lysed cell extract. Additionally, transgenic animal production can include the production of polypeptides in milk or eggs, which can be collected, and if necessary further the proteins can be extracted and further purified using standard methods in the art.

When proteins are expressed by transformed bacteria in large amounts, typically after promoter induction, although expression can be constitutive, the polypeptides can form insoluble aggregates. There are several protocols that are suitable for purification of polypeptide inclusion bodies known to one of skill in the art. Numerous variations will be apparent to those of skill in the art.

For example, in one method, the cell suspension is generally centrifuged and the pellet containing the inclusion bodies resuspended in buffer which does not dissolve but washes the inclusion bodies, e.g., 20 mM Tris-HCl (pH 7.2), 1 mM EDTA, 150 mM NaCl and 2% Triton-X 100, a non-ionic detergent. It can be necessary to repeat the wash step to remove as much cellular debris as possible. The remaining pellet of inclusion bodies can be resuspended in an appropriate buffer (e.g., 20 mM sodium phosphate, pH 6.8, 150 mM NaCl). Other appropriate buffers are apparent to those of skill in the art.

Alternatively, antibodies can be purified from bacteria periplasm. Where the antibody is exported into the periplasm of the bacteria, the periplasmic fraction of the bacteria can be isolated by cold osmotic shock in addition to other methods known to those of skill in the art. For example, in one method, to isolate recombinant polypeptides from the periplasm, the bacterial cells are centrifuged to form a pellet. The pellet can be resuspended in a suitable buffer containing 20% sucrose. To lyse the cells, the bacteria can be centrifuged and the pellet resuspended in ice-cold 5 mM $MgSO_4$ and kept in an ice bath for approximately 10 minutes. The cell suspension is centrifuged and the supernatant decanted and saved. Recombinant anti-EGFR antibodies present in the supernatant can be separated from the host proteins by standard separation techniques well known to those of skill in the art, such as the separation techniques described herein. These methods include, but are not limited to, the following steps: solubility fractionation, size differential filtration, and column chromatography.

## F. PHARMACEUTICAL COMPOSITIONS, FORMULATIONS, KITS, ARTICLES OF MANUFACTURE AND COMBINATIONS

### 1. Pharmaceutical Compositions and Formulations

Pharmaceutical compositions of any of anti-EGFR antibodies, such as modified anti-EGFR antibodies, or antigen-binding fragments thereof, are provided herein for administration. Pharmaceutically acceptable compositions are prepared in view of approvals for a regulatory agency or other agency prepared in accordance with generally recognized pharmacopeia for use in animals and in humans. Typically, the compounds are formulated into pharmaceutical compositions using techniques and procedures well known in the art (*see e.g.,* Ansel Introduction to Pharmaceutical Dosage Forms, Fourth Edition, 1985, 126).

The pharmaceutical composition can be used for therapeutic, prophylactic, and/or diagnostic applications. The anti-EGFR antibodies provided herein can be formulated with a pharmaceutical acceptable carrier or diluent. Generally, such pharmaceutical compositions utilize components which will not significantly impair the biological properties of the antibody, such as the binding of to its specific epitope (e.g. binding to EGFR). Each component is pharmaceutically and physiologically acceptable in the sense of being compatible with the other ingredients and not injurious to the patient. The formulations can conveniently be presented in unit dosage form and can be prepared by methods well known in the art of pharmacy, including but not limited to, tablets, pills, powders, liquid solutions or suspensions (e.g., including injectable, ingestible and topical formulations (e.g., eye drops, gels, pastes, creams, or ointments), aerosols (e.g., nasal sprays), liposomes, suppositories, pessaries, injectable and infusible solution and sustained release forms. See, *e.g.,* Gilman, et al. (eds. 1990) Goodman and Gilman's: The Pharmacological Bases of Therapeutics, 8th Ed., Pergamon Press; and Remington's Pharmaceutical Sciences, 17th ed. (1990), Mack Publishing Co., Easton, Pa.; Avis, et al. (eds. 1993) Pharmaceutical Dosage Forms: Parenteral Medications Dekker, NY; Lieberman, et al. (eds. 1990) Pharmaceutical Dosage Forms: Tablets Dekker, NY; and Lieberman, et al. (eds. 1990) Pharmaceutical Dosage Forms: Disperse Systems Dekker, NY. When administered systematically, the therapeutic composition is sterile, pyrogen-free, generally free of particulate matter, and in a parenterally acceptable solution having due regard for pH, isotonicity, and stability. These conditions are known to those skilled in the art. Methods for preparing parenterally administrable compositions are well known or will be apparent to those skilled in the art and are described in more detail in, *e.g.,* "Remington: The Science and Practice of Pharmacy (Formerly Remington's Pharmaceutical Sciences)", 19th ed., Mack Publishing Company, Easton, Pa. (1995).

Pharmaceutical compositions provided herein can be in various forms, e.g., in solid, semi-solid, liquid, powder, aqueous, or lyophilized form. Examples of suitable pharmaceutical carriers are known in the art and include but are not limited to water, buffering agents, saline solutions, phosphate buffered saline solutions, various types of wetting agents, sterile solutions, alcohols, gum arabic, vegetable oils, benzyl alcohols, gelatin, glycerin, carbohydrates such as lactose, sucrose, amylose or starch, magnesium stearate, talc, silicic acid, viscous paraffin, perfume oil, fatty acid monoglycerides and diglycerides, pentaerythritol fatty acid esters, hydroxy methylcellulose, powders, among others. Pharmaceutical compositions provided herein can contain other additives including, for example, antioxidants, preservatives, antimicrobial agents, analgesic agents, binders, disintegrants, coloring, diluents, excipients, extenders, glidants, solubilizers, stabilizers, tonicity agents, vehicles, viscosity agents, flavoring agents, emulsions, such as oil/water emulsions, emulsifying and suspending agents, such as acacia, agar, alginic acid, sodium alginate, bentonite, carbomer, carrageenan, carboxymethylcellulose, cellulose, cholesterol, gelatin, hydroxyethyl cellulose, hydroxypropyl cellulose, hydroxypropyl methylcellulose, methylcellulose, octoxynol 9, oleyl alcohol, povidone, propylene glycol monostearate, sodium lauryl sulfate, sorbitan esters, stearyl alcohol, tragacanth, xanthan gum, and derivatives thereof, solvents, and miscellaneous ingredients such as crystalline cellulose, microcrystalline cellulose, citric acid, dextrin, dextrose, liquid glucose, lactic acid, lactose, magnesium chloride, potassium metaphosphate, starch, among others (see, generally, Alfonso R. Gennaro (2000) Remington: The Science and Practice of Pharmacy, 20th Edition. Baltimore, MD: Lippincott Williams & Wilkins). Such carriers and/or additives can be formulated by conventional methods and can be administered to the subject at a suitable dose. Stabilizing agents such as lipids, nuclease inhibitors, polymers, and chelating agents can preserve the compositions from degradation within the body.

The route of antibody administration is in accord with known methods, e.g., injection or infusion by intravenous, intraperitoneal, intracerebral, intramuscular, subcutaneous, intraocular, intraarterial, intrathecal, inhalation or intralesional routes, topical or by sustained release systems as noted below. The antibody is typically administered continuously by infusion or by bolus injection. One can administer the antibodies in a local or systemic manner.

The anti-EGFR antibodies, such as modified antibodies, provided herein can be prepared in a mixture with a pharmaceutically acceptable carrier. Techniques for formulation and administration of the compounds are known to one of skill in the art (see e.g. "Remington's Pharmaceutical Sciences," Mack Publishing Co., Easton, Pa.). This therapeutic composition can be administered intravenously or through the nose or lung, preferably as a liquid or powder aerosol (lyophilized). The composition also can be administered parenterally or subcutaneously as desired. When administered systematically, the therapeutic composition should be sterile, pyrogen-free and in a parenterally acceptable solution having due regard for pH, isotonicity, and stability. These conditions are known to those skilled in the art.

Pharmaceutical compositions suitable for use include compositions wherein one or more anti-EGFR antibodies are contained in an amount effective to achieve their intended purpose. Determination of a therapeutically effective amount is well within the capability of those skilled in the art. Therapeutically effective dosages can be determined by using *in vitro* and *in vivo* methods as described herein. Accordingly, an anti-EGFR antibody provided herein, when in a pharmaceutical preparation, can be present in unit dose forms for administration.

Therapeutic formulations can be administered in many conventional dosage formulations. Briefly, dosage formulations of the antibodies provided herein are prepared for storage or administration by mixing the compound having the desired degree of purity with physiologically acceptable carriers, excipients, or stabilizers. Such materials are nontoxic to the recipients at the dosages and concentrations employed, and can include buffers such as TRIS HCl, phosphate, citrate, acetate and other organic acid salts; antioxidants such as ascorbic acid; low molecular weight (less than about ten residues) peptides such as polyarginine, proteins, such as serum albumin, gelatin, or immunoglobulins; hydrophilic polymers such as polyvinylpyrrolidinone; amino acids such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium and/or nonionic surfactants such as TWEEN, PLURONICS or polyethyleneglycol.

When used for *in vivo* administration, the modified anti-EGFR antibody formulation should be sterile and can be formulated according to conventional pharmaceutical practice. This is readily accomplished by filtration through sterile filtration membranes, prior to or following lyophilization and reconstitution. The antibody ordinarily will be stored in lyophilized form or in solution. Other vehicles such as naturally occurring vegetable oil like sesame, peanut, or cottonseed oil or a synthetic fatty vehicle like ethyl oleate or the like may be desired. Buffers, preservatives, antioxidants and the like can be incorporated according to accepted pharmaceutical practice.

The anti-EGFR antibodies, such as modified anti-EGFR antibodies, can be provided at a concentration of at or about 0.1 to 10 mg/mL, such as, for example at or about 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10 mg/mL or more. The volume of the solution can be at or about 1 to 100 mL, such as, for example, at or about 0.5, 1, 2, 3, 4, 5, 10, 15, 20, 25, 30, 35, 40, 45, 50, 55, 60, 65, 70, 75, 80, 85, 90, 95, 100 mL or more. In some examples, the anti-EGFR antibodies are supplied in phosphate buffered saline. For example, the anti-EGFR antibodies can be supplied as a 50-mL, single-use vial containing 100 mg of anti-EGFR antibody at a concentration of 2 mg/mL in phosphate buffered saline.

An anti-EGFR antibody provided herein can be lyophilized for storage and reconstituted in a suitable carrier prior to use. This technique has been shown to be effective with conventional immunoglobulins and protein preparations and art-known lyophilization and reconstitution techniques can be employed.

An anti-EGFR antibody provided herein can be provided as a controlled release or sustained release composition. Polymeric materials are known in the art for the formulation of pills and capsules which can achieve controlled or sustained release of the antibodies provided herein (see, *e.g.,* Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, New York (1984); Langer and Peppas (1983) J. Macromol. Sci. 23:61; see also Levy et al. (1985) Science 228:190; During et al. (1989) Ann. Neurol. 25:351; Howard et al. (1989) J. Neurosurg. 71:105; U.S. Pat. Nos. 5,679,377, 5,916,597, 5,912,015, 5,989,463, 5,128,326; and PCT Publication Nos. WO 99/15154 and WO 99/20253). Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N-vinyl pyrrolidone), poly(vinyl alcohol), polyacrylamide, poly(ethylene glycol), polylactides (PLA), poly(lactide-co-glycolides) (PLGA), and polyorthoesters. Generally, the polymer used in a sustained release formulation is inert, free of leachable impurities, stable on storage, sterile, and biodegradable. Any technique known in the art for the production of sustained release formulation can be used to produce a sustained release formulation containing one more anti-EGFR antibodies provided herein.

In some examples, the pharmaceutical composition contains an anti-EGFR antibody provided herein and one or more additional antibodies. In some examples, the one or more additional antibodies includes, but is not limited to, anti-EGFR antibodies described herein or known in the art, such as, for example, ABX-EGF or cetuximab.

### 2. Articles of Manufacture/Kits

Pharmaceutical compositions of anti-EGFR antibodies or nucleic acids encoding anti- EGFR antibodies, or a derivative or a biologically active portion thereof can be packaged as articles of manufacture containing packaging material, a pharmaceutical composition which is effective for treating a disease or conditions that can be treated by administration of an anti-EGFR antibody, such as the diseases and conditions described herein or known in the art, and a label that indicates that the antibody or nucleic acid molecule is to be used for treating the infection, disease or disorder. The pharmaceutical compositions can be packaged in unit dosage forms containing an amount of the pharmaceutical composition for a single dose or multiple doses. The packaged compositions can contain a lyophilized powder of the pharmaceutical compositions containing the modified anti-EGFR antibodies provided, which can be reconstituted (*e.g.* with water or saline) prior to administration.

The articles of manufacture provided herein contain packaging materials. Packaging materials for use in packaging pharmaceutical products are well known to those of skill in the art. See, for example, U.S. Patent Nos. 5,323,907, 5,052,558 and 5,033,252, each of which is incorporated herein in its entirety. Examples of pharmaceutical packaging materials include, but are not limited to, blister packs, bottles, tubes, inhalers (e.g., pressurized metered dose inhalers (MDI), dry powder inhalers (DPI), nebulizers (e.g., jet or ultrasonic nebulizers) and other single breath liquid systems), pumps, bags, vials, containers, syringes, bottles, and any packaging material suitable for a selected formulation and intended mode of administration and treatment.

The anti-EGFR antibodies, such as modified anti-EGFR antibodies, nucleic acid molecules encoding the antibodies thereof, pharmaceutical compositions or combinations provided herein also can be provided as kits. Kits can optionally include one or more components such as instructions for use, devices and additional reagents (*e.g.,* sterilized water or saline solutions for dilution of the compositions and/or reconstitution of lyophilized protein), and components, such as tubes, containers and syringes for practice of the methods. Exemplary kits can include the anti-EGFR antibodies provided herein, and can optionally include instructions for use, a device for administering the anti-EGFR antibodies to a subject, a device for detecting the anti-EGFR antibodies in a subject, a device for detecting the anti-EGFR antibodies in samples obtained from a subject, and a device for administering an additional therapeutic agent to a subject.

The kit can, optionally, include instructions. Instructions typically include a tangible expression describing the modified anti-EGFR antibodies and, optionally, other components included in the kit, and methods for administration, including methods for determining the proper state of the subject, the proper dosage amount, dosing regimens, and the proper administration method for administering the anti-EGFR antibodies. Instructions also can include guidance for monitoring the subject over the duration of the treatment time.

Kits also can include a pharmaceutical composition described herein and an item for diagnosis. For example, such kits can include an item for measuring the concentration, amount or activity of the selected anti-EGFR antibody in a subject.

In some examples, the anti-EGFR antibody is provided in a diagnostic kit for the detection of EGFR in an isolated biological sample (e.g., tumor cells, such as circulating tumor cells obtained from a subject or tumor cells excised from a subject). In some examples, the diagnostic kit contains a panel of one or more anti-EGFR antibodies and/or one or more control antibodies (i.e. non-EGFR binding antibodies or EGFR antibodies known in the art, such as cetuximab), where one or more antibodies in the panel is a modified anti-EGFR antibody provided herein.

Kits provided herein also can include a device for administering the anti-EGFR antibodies to a subject. Any of a variety of devices known in the art for administering medications to a subject can be included in the kits provided herein. Exemplary devices include, but are not limited to, a hypodermic needle, an intravenous needle, and a catheter. Typically the device for administering the modified anti-EGFR antibodies of the kit will be compatible with the desired method of administration of the modified anti-EGFR antibodies.

### 3. Combinations

Provided are combinations of the anti-EGFR antibodies, such as modified anti-EGFR antibodies, provided herein and a second agent, such as a second anti-EGFR antibody or other therapeutic or diagnostic agent. A combination can include any anti-EGFR antibody or reagent for effecting therapy thereof in accord with the methods provided herein. For example, a combination can include any anti-EGFR antibody and a chemotherapeutic agent. Combinations also can include an anti-EGFR antibody provided herein with one or more additional therapeutic antibodies. For example, the additional therapeutic agent is an anti-cancer agent, such as a chemotherapeutic agent, for example, as described in Section G. Combinations of the modified anti-EGFR antibodies thereof provided also can contain pharmaceutical compositions containing the anti-EGFR antibodies or host cells containing nucleic acids encoding the anti-EGFR antibodies as described herein. The combinations provided herein can be formulated as a single composition or in separate compositions.

## G. THERAPEUTIC USES

The anti-EGFR antibodies, or fragments thereof, provided herein can be used for any purpose known to the skilled artisan for use of an anti-EGFR antibody. For example, the anti-EGFR antibodies described herein can be used for one or more of therapeutic, diagnostic, industrial and/or research purpose(s). In particular, the methods provided herein include methods for the therapeutic uses of the anti-EGFR antibodies, such as modified anti-EGFR antibodies, provided herein. In some examples, the anti-EGFR antibodies described herein can be used to kill target cells that include EGFR, such as, for example cancer cells. In some examples, the anti-EGFR antibodies can block, antagonize, or agonize EGFR. By virtue of such activity, the anti-EGFR antibodies provided herein, or fragments thereof, can be administered to a patient or subject for treatment of any condition responsive to treatment with an anti-EGFR antibody, including, but not limited to, a tumor, cancer or metastasis. The therapeutic uses include administration of a therapeutically effective amount of a anti-EGFR antibody, alone or in combination with other treatments or agents.

The anti-EGFR antibodies, such as modified anti-EGFR antibodies, and fragments thereof, provided herein can be used as therapeutics for the treatment of any disease or condition in which existing anti-EGFR antibodies are used, such as Cetuximab. The anti-EGFR antibodies, when administered, result in subjects exhibiting reduced or lessened side effects compared to side effects that can be observed after administration of other anti-EGFR antibodies. As discussed elsewhere herein, existing anti-EGFR antibodies, such as Cetuximab, when administered, can result in subjects exhibiting local and systemic side effects, and in particular dermal side effects. These side effects limit the therapeutic use. In many cases, these side effects are associated with binding to EGFR at a neutral physiologic pH environment, such as in the skin dermis. The methods provided herein include administering a anti-EGFR provided herein, which is more active at low pH, such as a pH ranging from about 5.6 to about 6.8, such as less than or about or pH 5.6, 5.7, 5.8 , 5.9, 6.0, 6.1, 6.2, 6.3, 6.4, 6.5, 6.6, 6.7, or 6.8, than at a neutral pH, such as a pH ranging from about 6.8 to about 7.6, such as less than or about or pH 6.8, 6.9, 7.0, 7.1, 7.2, 7.3, 7.4, 7.5 or 7.6. Optionally, the conditions at low pH can include increased lactic acid concentrations, such as from about 10mM to about 30mM, such as 10mM, 11mM, 12mM, 13mM, 14mM, 15mM, 16mM, 17mM, 18mM, 19mM, 20mM, 25mM, 30mM or more. For example, the anti-EGFR antibodies provided herein can have greater activity in a tumor environment (which can have a low pH and/or increased lactic acid concentrations) than in a neutral physiologic environment that is associated with one or more side effects of an anti-EGFR antibody, such as the skin basal layer. This can be advantageous by targeting therapy only to diseased tissues, such as tumor tissues, in order to reduce or prevent side effects, including local and systemic side effects.

Anti-EGFR antibodies that are associated with reduced side effects, such as the modified anti-EGFR antibodies provided herein, can be used at higher dosing regimens, and can have improved efficacy and safety. Side effects that can be reduced compared to those observed by existing anti-EGFR antibody therapeutics, such as Cetuximab, include any undesirable nontherapeutic effect described herein or known in the art, such as nausea, emesis, chest tightness, headache, and related cardiovascular effects such as blood pressure instability and arterial constriction, dermal toxicity, bone marrow suppression, cardiotoxicity, hair loss, renal dysfunctions, stomatitis, anemia, seizures, immune reactions such as acute anaphylaxis, serum sickness, generation of antibodies, infections, cancer, autoimmune disease and cardiotoxicity.

In some examples, compared to side effects caused by administration of existing anti-EGFR antibody therapeutics, such as Cetuximab, administration of a anti-EGFR antibody provided herein decreases the severity of one or more side effects by at least or about 99 %, at least or about 95 %, at least or about 90 %, at least or about 85 %, at least or about 80 %, at least or about 75 %, at least or about 70 %, at least or about 65 %, at least or about 60 %, at least or about 55 %, at least or about 50 %, at least or about 45 %, at least or about 40 %, at least or about 35 %, at least or about 30 %, at least or about 25 %, at least or about 20 %, at least or about 15 %, or at least or about 10 % relative to the severity of the one or more side effects of an unmodified EGFR antibody.

The methods can include selection of a patient or subject for treatment, e.g. prior to treatment of the subject, for example to determine whether the patient or subject has an EGFR dependent disease or condition. In some examples, the methods provided herein include a step of identifying a patient or subject that has experienced or is experiencing an adverse side effect resulting from administration of an anti-EGFR antibody, such as Cetuximab. One skilled in the art would easily be able to diagnose such conditions, disorders and side effects using tests and assays known to one of skill in the art and/or described herein.

Treatment of diseases and conditions with anti-EGFR antibodies, such as modified anti-EGFR antibodies, can be effected by any suitable route of administration using suitable formulations as described herein including, but not limited to, infusion, subcutaneous injection, intramuscular, intradermal, oral, and topical and transdermal administration.

It is understood that while the anti-EGFR antibodies, such as modified anti-EGFR antibodies, and antibody fragments, provided herein, when administered, can result in subjects exhibiting lessened or reduced side effects compared to other anti-EGFR antibodies, such as Cetuximab, that some side effects can occur upon administration. It is understood that number and degree of tolerable side effects depends upon the condition for which the compounds are administered. For example, certain toxic and undesirable side effects are tolerated when treating life-threatening illnesses that would not be tolerated when treating disorders of lesser consequence. Amounts effective for therapeutic use can depend on the severity of the disease and the weight and general state of the subject as well as the route of administration. Local administration of the therapeutic agent will typically require a smaller dosage than any mode of systemic administration, although the local concentration of the therapeutic agent can, in some cases, be higher following local administration than can be achieved with safety upon systemic administration.

This section provides exemplary uses of, and administration methods for, the anti-EGFR antibodies, such as modified anti-EGFR antibodies, provided herein. These described uses are exemplary and do not limit the applications of the methods described herein. Such methods include, but are not limited to, methods of treatment of any condition or disease that can be treated by administration of an anti-EGFR antibody. It is within the skill of a treating physician to identify such diseases or conditions.

**1. Exemplary Diseases and Conditions**

The anti-EGFR antibodies, such as modified anti-EGFR antibodies, described herein can be used for any therapeutic purpose that antibodies, such as anti-EGFR antibodies can be used for (see, e.g., Reeves et al. (2011) Otolaryngol Head Neck Surg. 144(5):676-84; Adams et al. (2008) Expert Rev Anticancer Ther. 8(8):1237-45; Belda-Iniesta et al. (2006) Cancer Biol Ther. 5(8):912-4; Liu et al. (2010) Cancer Chemother Pharmacol. 65(5):849-61). In some examples, the anti-EGFR antibodies are administered to a patient to treat a disease or disorder that can be treated with an anti-EGFR antibody. In some examples, treatment of the disease includes administration of an anti-EGFR antibody described herein after clinical manifestation of the disease to combat the symptoms of the disease. In some examples, administration of an anti-EGFR antibody described herein is administered to eradicate the disease. Examples of diseases or disorders that can be treated with the modified anti-EGFR antibodies described herein include autoimmune and inflammatory diseases, infectious diseases, and cancer.

**a. Cancer**

EGFR is associated with cancer development and progression in a variety of human malignancies, such as lung cancer, head and neck cancer, colon cancer, breast cancer, ovarian cancer and glioma. EGFR-related molecular factors, such as copy number and gene mutations, have been identified as prognostic and predictive factors for cancer (see, e.g., Bronte et al. (2011) Front Biosci. 3:879-887; Harding and Burtness (2005) Drugs Today 41(2):107-127). For example, high EGFR expression is associated with poor prognosis in patients with head and neck squamous cell carcinoma (HNSCC) (Szabo et al. (2011) Oral Oncol. 47(6):487-496).

Anti-EGFR antibodies, such as the modified anti-EGFR antibodies described herein, can bind to and prevent stimulation of the EGF receptor. For example, binding of a modified anti-EGFR antibody to the receptor can inhibit the binding of epidermal growth factor (EGF) and/or result in internalization of the antibody-receptor complex (Harding and Burtness, *Drugs Today (Barc)*). Thus, anti-EGFR antibodies, such as the modified anti-EGFR antibodies provided herein, can, for example, prevent receptor phosphorylation and activation of the receptor-associated kinase activity, ultimately shutting off receptor-mediated cell signaling.

Due to the increased activity of anti-EGFR antibodies provided herein at low pH and/or elevated lactate concentrations, the anti-EGFR antibodies can be preferentially active at tumor microenvironments compared to non-target cells or tissues. Modified anti-EGFR antibodies, and fragments thereof, described herein, can be used to treat tumors, including solid tumors, that express EGFR. EGFR expressing tumors can be sensitive to EGF present in their local microenvironment, and can further be stimulated by tumor produced EGF or Transforming Growth Factor-alpha (TGF-$\alpha$). The diseases and conditions that can be treated or prevented by the present methods include, for example, those in which tumor growth is stimulated through an EGFR paracrine and/or autocrine loop. The methods described herein can therefore be useful for treating a tumor that is not vascularized, or is not yet substantially vascularized. In addition, anti-EGFR antibodies, such as the modified anti-EGFR antibodies described herein, can inhibit tumor associated angiogenesis. EGFR stimulation of vascular endothelium is associated with vascularization of tumors. Typically, vascular endothelium is stimulated in a paracrine fashion by EGF and/or TGF-$\alpha$ from other sources (e.g. tumor cells). Accordingly, anti-EGFR antibodies, such as the modified anti-EGFR antibodies described herein can be useful for treating subjects with vascularized tumors or neoplasms.

An altered pH microenvironment is the most common microenvironment found in disease states such as tumor microenvironments, and it is the most uniform within the disease microenvironment compared to other properties such as hypoxia (see *e.g.* Fogh Andersen et al. (1995) Clin. Chem., 41:1522-1525; Bhujwalla et al. (2002) NMR Biomed., 15:114-119; Helmlinger et al. (1997) Nature Med., 3:177; Gerweck and Seetharaman (1996), Cancer Res. 56(6):1194-1198). For example, in many tumors the 'Warburg effect' creates a microenvironment with a pH ranging from about 5.6 to about 6.8, such as less than or about or pH 5.6, 5.7, 5.8 , 5.9, 6.0, 6.1, 6.2, 6.3, 6.4, 6.5, 6.6, 6.7, or 6.8. Thus, anti-EGFR antibodies that are more active at acidic pH than at neutral pH, such as the modified anti-EGFR antibodies described herein, can be used to treat EGFR expressing tumors, while minimizing activity in non-target disease cells or tissues.

In addition, in many tumors, the 'Warburg effect' creates a microenvironment with lactate concentrations between 10 to 15 mM. Elevated lactate levels have been found associated with a variety of tumors including, but not limited to, head and neck, metastatic colorectal cancer, cervical cancer and squamous cell carcinoma (see *e.g.,* Correlation of High Lactate Levels in Head and Neck Tumors with Incidence of Metastasis. Stefan Walenta, Ahmad Salameh, Heidi Lyng, Jan F. Evensen, Margarethe Mitze, Einar K. Rofstad, and Wolfgang Mueller-Klieser. (1997) American Journal of Pathology 150(2): 409-415; Correlation of High Lactate Levels in Human Cervical Cancer with Incidence of Metastasis. Georg Schwickert, Stefan Walenta, Kolbein Suiulfor. Einar K. Rofstad, and Wolfgang Mueller-Klieser. (1995) Cancer Research 55: 4757-4759; High Lactate Levels Predict Likelihood of Metastases, Tumor Recurrence, and Restricted Patient Survival in Human Cervical Cancers. Stefan Walenta, Michael Wetterling, Michael Lehrke, Georg Schwickert, Kolbein Sundfør, Einar K. Rofstad, and Wolfgang Mueller-Klieser. (2000) Cancer Research 60: 916-921; *In Vitro* Proton Magnetic Resonance Spectroscopic Lactate and Choline Measurements, 18F-FDG Uptake, and Prognosis in Patients with Lung Adenocarcinoma. JianFei Guo, Kotaro Higashi, Hajime Yokota, Yosinobu Nagao, Yoshimichi Ueda, Yuko Kodama, Manabu Oguchi, Suzuka Taki, Hisao Tonami, and Itaru Yamamoto. (2004) J Nucl Med 45: 1334-1339; Lactate and malignant tumors: A therapeutic target at the end stage of glycolysis. Saroj P. Mathupala, Chaim B. Colen, Prahlad Parajuli, Andrew E. Sloan (2007) J Bioenerg Biomembr 39: 73-77; Lactate Metabolism in Patients with Metastatic Colorectal Cancer. Christopher P. Holroyde, Rita S. Axelrod, Charles L. Skutches, Agnes C. Haff, Pavle Paul, and George A. Reichard. (1979) Cancer Research 39: 4900-4904; Lactate, not pyruvate, is neuronal aerobic glycolysis end product: an in vitro electrophysiological study. A Schurr and R.S. Payne. (2007) Neuroscience 147: 613-619; Tumor lactate content predicts for response to fractionated irradiation of human squamous cell carcinomas in nude mice. Verena Quennet, Ala Yarominab, Daniel Zipsb, Andrea Rosnerb, Stefan Walentaa, Michael Baumannb, Wolfgang Mueller-Kliesera. (2006) Radiotherapy and Oncology 81: 130-135). Thus, anti-EGFR antibodies that are more active at increased lactate concentrations than at normal physiologic lactate concentrations, such as the modified anti-EGFR antibodies described herein, can be used to treat EGFR expressing tumors, while minimizing activity at non-target disease cells or tissues.

Tumors that can be treated include primary tumors and metastatic tumors, as well as refractory tumors. Refractory tumors include tumors that fail to respond or are resistant to treatment with chemotherapeutic agents alone, antibodies alone, radiation alone or combinations thereof. Refractory tumors also encompass tumors that appear to be inhibited by treatment with such agents, but recur up to five years, sometimes up to ten years or longer after treatment is discontinued. The tumors can express EGFR at normal levels or they can overexpress EGFR at levels, for example, that are at least 10, 100, or 1000 times normal levels.

Examples of tumors that express EGFR and can be treated by the modified anti-EGFR antibodies, and fragments thereof, provided herein include carcinomas, gliomas, sarcomas (including liposarcoma), adenocarcinomas, adenosarcomas, and adenomas. Such tumors can occur in virtually all parts of the body, including, for example, breast, heart, lung, small intestine, colon, spleen, kidney, bladder, head and neck, ovary, prostate, brain, pancreas, skin, bone, bone marrow, blood, thymus, uterus, testicles, cervix or liver.

Exemplary of tumors that can be treated by anti-EGFR antibodies, such as the modified anti-EGFR antibodies, and fragments thereof, provided herein, are those that overexpress EGFR. Some tumors observed to overexpress EGFR that can be treated include, but are not limited to, colorectal and head and neck tumors, especially squamous cell carcinoma of the head and neck, brain tumors such as glioblastomas, and tumors of the lung, breast, pancreas, esophagus, bladder, kidney, ovary, cervix, and prostate.

Other examples of tumors that can be treated by the anti-EGFR antibodies, and antibody fragments thereof, provided herein include Kaposi's sarcoma, CNS neoplasms, neuroblastomas, capillary hemangioblastomas, meningiomas and cerebral metastases, melanoma, gastrointestinal and renal carcinomas and sarcomas, rhabdomyosarcoma, glioblastoma (such as glioblastoma multiforme) and leiomyosarcoma. Examples of cancer that can express EGFR include but are not limited to lymphoma, blastoma, neuroendocrine tumors, mesothelioma, schwannoma, meningioma, melanoma, and leukemia or lymphoid malignancies. Examples of such cancers include hematologic malignancies, such as Hodgkin's lymphoma; non-Hodgkin's lymphomas (Burkitt's lymphoma, small lymphocytic lymphoma/chronic lymphocytic leukemia, mycosis fungoides, mantle cell lymphoma, follicular lymphoma, diffuse large B-cell lymphoma, marginal zone lymphoma, hairy cell leukemia and lymphoplasmacytic leukemia), tumors of lymphocyte precursor cells, including B-cell acute lymphoblastic leukemia/lymphoma, and T-cell acute lymphoblastic leukemia/lymphoma, thymoma, tumors of the mature T and NK cells, including peripheral T-cell leukemias, adult T-cell leukemia/T-cell lymphomas and large granular lymphocytic leukemia, Langerhans cell histocytosis, myeloid neoplasias such as acute myelogenous leukemias, including AML with maturation, AML without differentiation, acute promyelocytic leukemia, acute myelomonocytic leukemia, and acute monocytic leukemias, myelodysplastic syndromes, and chronic myeloproliferative disorders, including chronic myelogenous leukemia; tumors of the central nervous system such as glioma, glioblastoma, neuroblastoma, astrocytoma, medulloblastoma, ependymoma, and retinoblastoma; solid tumors of the head and neck (e.g.,

nasopharyngeal cancer, salivary gland carcinoma, and esophageal cancer), lung (e.g., small-cell lung cancer, non-small cell lung cancer, adenocarcinoma of the lung and squamous carcinoma of the lung), digestive system (e.g., gastric or stomach cancer including gastrointestinal cancer, cancer of the bile duct or biliary tract, colon cancer, rectal cancer, colorectal cancer, and anal carcinoma), reproductive system (e.g., testicular, penile, or prostate cancer, uterine, vaginal, vulval, cervical, ovarian, and endometrial cancer), skin (e.g., melanoma, basal cell carcinoma, squamous cell cancer, actinic keratosis), liver (e.g., liver cancer, hepatic carcinoma, hepatocellular cancer, and hepatoma), bone (e.g., osteoclastoma, and osteolytic bone cancers) additional tissues and organs (e.g., pancreatic cancer, bladder cancer, kidney or renal cancer, thyroid cancer, breast cancer, cancer of the peritoneum, and Kaposi's sarcoma), and tumors of the vascular system (e.g., angiosarcoma and hemangiopericytoma).

**b. Non-Cancer Hyperproliferative Diseases**

Anti-EGFR antibodies, such as modified anti-EGFR antibodies, and antibody fragments thereof, provided herein can be used to treat a non-cancer hyperproliferative disease in a subject. EGFR is a critical pathway element in signalling from G-protein-coupled receptors (GPCRs), cytokines, receptor tyrosine kinases and integrins to a variety of cellular responses such as mitogen activated protein kinase activation, gene transcription and proliferation. Ligand binding to EGFR can induce autophosphorylation of cytoplasmic tyrosine residues, which can initiate cellular pathways leading to cellular proliferation. Overexpression and/or overstimulation can result in hyperproliferation. For example, the EGFR vIII mutation causes the EGFR receptor to have a constitutively active kinase function and stimulate cellular proliferation. It is known in the art that anti-EGFR antibodies can treat non-cancer hyperproliferative disorders. For example, Ménétrier's disease, a rare premalignant, non-cancerous, hyperproliferative disorder of the stomach, can be treated with cetuximab (Fiske et al. (2009) Sci Trasl. Med. 1(8): 8ra18; Myers et al.(2012) Mol. Cell. Proteomics 11:10.1074/mcp.M111.015222, 1-15).

Examples of hyperproliferative diseases that can be treated by the anti-EGFR antibodies provided herein include any hyperproliferative diseases that can be treated by administration of an anti-EGFR antibody and include, for example, psoriasis, actinic keratoses, and seborrheic keratoses, warts, keloid scars, and eczema. Also included are hyperproliferative diseases caused by virus infections, such as papilloma virus infection. Different types of psoriasis can display characteristics such as pus-like blisters (pustular psoriasis), severe sloughing of the skin I (erythrodermic psoriasis), drop-like dots (guttae psoriasis) and smooth inflamed lesions (inverse psoriasis). It is understood that treatment of psoriasis includes treatment of all types of psoriasis (e. g., psoriasis vulgaris, psoriasis pustulosa, psoriasis erythrodermica, psoriasis arthropathica, parapsoriasis, palmoplantar pustulosis).

**c. Autoimmune Diseases or Disorders**

Anti-EGFR antibodies, such as modified anti-EGFR antibodies, and antibody fragments thereof, provided herein can be used to treat autoimmune diseases or disorders. Examples of autoimmune diseases or disorders that can be treated with the anti-EGFR antibodies described herein include, but are not limited to, allogenic islet graft rejection, alopecia areata, ankylosing spondylitis, antiphospholipid syndrome, autoimmune Addison's disease, antineutrophil cytoplasmic autoantibodies (AN CA), autoimmune diseases of the adrenal gland, autoimmune hemolytic anemia, autoimmune hepatitis, autoimmune myocarditis, autoimmune neutropenia, autoimmune oophoritis and orchitis, autoimmune thrombocytopenia, autoimmune urticaria, Behcet's disease, bullous pemphigoid, cardiomyopathy, Castleman's syndrome, celiac spruce-dermatitis, chronic fatigue immune dysfunction syndrome, chronic inflammatory demyelinating polyneuropathy, Churg-Strauss syndrome, cicatricial pemphigoid, CREST syndrome, cold agglutinin disease, Crohn's disease, dermatomyositis, discoid lupus, essential mixed cryoglobulinemia, factor VIII deficiency, fibromyalgia-fibromyositis, glomerulonephritis, Grave's disease, Guillain-Barre, Goodpasture's syndrome, graft-versus-host disease (GVHD), Hashimoto's thyroiditis, hemophilia A, idiopathic pulmonary fibrosis, idiopathic thrombocytopenia purpura (ITP), IgA neuropathy, IgM polyneuropathies, immune mediated thrombocytopenia, juvenile arthritis, Kawasaki's disease, lichen planus, lupus erythematosus, Meniere's disease, mixed connective tissue disease, multiple sclerosis, type 1 diabetes mellitus, myasthenia gravis, pemphigus vulgaris, pernicious anemia, polyarteritis nodosa, polychondritis, polyglandular syndromes, polymyalgia rheumatica, polymyositis and dermatomyositis, primary agammaglobulinemia, primary biliary cirrhosis, psoriasis, psoriatic arthritis, Reynaud's phenomenon, Reiter's syndrome, rheumatoid arthritis, sarcoidosis, scleroderma, Sjogren's syndrome, solid organ transplant rejection, stiff-man syndrome, systemic lupus erythematosus, Takayasu arteritis, temporal arteritis/giant cell arteritis, thrombotic thrombocytopenia purpura, ulcerative colitis, uveitis, vasculitides such as dermatitis herpetiformis vasculitis, vitiligo, and Wegner's granulomatosis.

**d. Inflammatory Disorders**

Anti-EGFR antibodies, such as modified anti-EGFR antibodies, and antibody fragments thereof, provided herein can be used to treat inflammatory diseases or disorders. Inflammatory disorders that can be treated by the modified anti-EGFR antibodies provided herein include but are not limited to acute respiratory distress syndrome (ARDS), acute septic arthritis, allergic encephalomyelitis, allergic rhinitis, allergic vasculitis, allergy, asthma, atherosclerosis, chronic inflammation due to chronic bacterial or viral infections, chronic obstructive pulmonary disease (COPD), coronary artery disease, encephalitis, inflammatory bowel disease, inflammatory osteolysis, inflammation associated with acute and delayed hypersensitivity reactions, inflammation associated with tumors, peripheral nerve injury or demyelinating diseases, inflammation associated with tissue trauma such as burns and ischemia, inflammation due to meningitis, multiple organ injury syndrome, pulmonary fibrosis, sepsis and septic shock, Stevens-Johnson syndrome, undifferentiated arthropathy, and undifferentiated spondyloarthropathy.

### e. Infectious Diseases

Anti-EGFR antibodies, such as modified anti-EGFR antibodies, and antibody fragments thereof, provided herein can be used to treat autoimmune diseases or disorders. Infectious diseases that can be treated by the anti-EGFR antibodies described herein include but are not limited to diseases caused by pathogens such as viruses, bacteria, fungi, protozoa, and parasites. Infectious diseases can be caused by viruses including adenovirus, cytomegalovirus, dengue, Epstein-Barr, hanta, hepatitis A, hepatitis B, hepatitis C, herpes simplex type I, herpes simplex type II, human immunodeficiency virus, (HIV), human papilloma virus (HPV), influenza, measles, mumps, papova virus, polio, respiratory syncytial virus, rinderpest, rhinovirus, rotavirus, rubella, SARS virus, smallpox and viral meningitis. Infectious diseases can also be caused by bacteria including *Bacillus anthracis, Borrelia burgdorferi, Campylobacter jejuni, Chlamydia trachomatis, Clostridium botulinum, Clostridium tetani, Diphtheria, E. coli, Legionella, Helicobacter pylori, Mycobacterium rickettsia, Mycoplasma Neisseria, Pertussis, Pseudomonas aeruginosa, S. pneumonia, Streptococcus, Staphylococcus, Vibrio cholerae* and *Yersinia pestis. Infectious diseases can also be caused by fungi such as Aspergillus fumigatus, Blastomyces dermatitidis, Candida albicans, Coccidioides immitis, Cryptococcus neoformans, Histoplasma capsulatum* and *Penicillium marneffei.* Infectious diseases can also be caused by protozoa and parasites such as chlamydia, kokzidiose, leishmania, malaria, rickettsia, and trypanosoma

### f. Other Diseases and Conditions

Anti-EGFR antibodies, such as modified anti-EGFR antibodies, and antibody fragments thereof, provided herein can be used to treat other diseases and conditions associated with expression of EGFR and/or for which exiting anti-EGFR antibodies, such as Cetuximab, are known to treat. Other diseases and conditions that can be treated by the anti-EGFR antibodies described herein include but are not limited to heart conditions such as congestive heart failure (CHF), myocarditis and other conditions of the myocardium; skin conditions such as rosacea, acne, and eczema; bone and tooth conditions such as bone loss, osteoporosis, Paget's disease, Langerhans' cell histiocytosis, periodontal disease, disuse osteopenia, osteomalacia, monostotic fibrous dysplasia, polyostotic fibrous dysplasia, bone metastasis, bone pain management, humoral malignant hypercalcemia, periodontal reconstruction, spinal cord injury, and bone fractures; metabolic conditions such as Gaucher's disease; endocrine conditions such as Cushing's syndrome; and neurological conditions.

### 2. Subjects for therapy

A subject or candidate for therapy with an anti-EGFR antibody, such as a modified anti-EGFR antibody provided herein, includes, but is not limited to, a subject, such as a human patient, that has a disease or condition that can be treated by administration of an anti-EGFR antibody, such as diseases or conditions described herein or known in the art.

### a. Selection of Subjects Overexpressing EGFR

In some examples, subjects or candidates for therapy are tested for evidence of positive EGFR expression using methods known in the art, such as for example Western blotting (WB) on membranes and total homogenates, and immunohistochemistry (IHC) on tissue microarrays. In addition, phosphorylated EGFR (pEGFR) can be measured by Western blotting on membranes (see, e.g., Thariat et al. (2012) Clin. Cancer Res. 18:1313). EGFR assessment can be evaluated using, for example, the EGFR PHARMDX scoring guidelines (Dako, Glostrup, Denmark). EGFR expression can be evaluated on sections that include the deepest region of tumor invasion, which can contain the greatest density of EGFR-positive cells. Such methods are within the ability of the skilled artisan (see, e.g., Ervin-Haynes et al. (2006) J. Clin. Oncol. ASCO Annual Meeting Proceedings Part I. Vol. 24, No. 18S (June 20 Supplement) 13000; Goldstein and Armin (2001) Cancer 92(5):1331-1346; Bibeau et al. (2006) Virchows Arch. 449(3):281-287)

**b. Selection of Subjects Exhibiting EGFR-associated Polymorphism**

In some examples, subjects or candidates for therapy are screened for one or more polymorphisms in order to predict the efficacy of the anti-EGFR antibodies provided herein. A number of the receptors that can interact with anti-EGFR antibodies, such as the modified EGFR antibodies provided herein, are polymorphic in the human population. For a given patient or population of patients, the efficacy of the modified anti-EGFR antibodies provided herein can be affected by the presence or absence of specific polymorphisms in proteins. For example, FcγRIIIa is polymorphic at position 158, which is commonly either V (high affinity) or F (low affinity). Patients with the V/V homozygous genotype mount a stronger natural killer (NK) response and are observed to have a better clinical response to treatment with the anti-CD20 antibody Rituxan® (rituximab), (Dall'Ozzo et. al. (2004) Cancer Res. 64:4664-4669). Additional polymorphisms include but are not limited to FcγRIIa R131 or H131, and such polymorphisms are known to either increase or decrease Fc binding and subsequent biological activity, depending on the polymorphism.

In some examples, subjects or candidates for therapy are screened for one or more polymorphisms in order to predict the efficacy of the anti-EGFR antibodies provided herein. Such methods are within the ability of the skilled artisan. This information can be used, for example, to select patients to include or exclude from clinical trials or, post-approval, to provide guidance to physicians and patients regarding appropriate dosages and treatment options. For example, in patients that are homozygous or heterozygous for FcγRIIIa 158F antibody drugs, such as the anti-CD20 mAb Rituximab, can have decreased efficacy (Carton 2002 Blood 99: 754-758; Weng 2003 J. Clin. Oncol. 21:3940-3947); such patients can show a much better clinical response to the modified anti-EGFR antibodies provided herein.

**c. Identifying Subjects Exhibiting Anti-EGFR-Associated Side Effects**

In some examples, a subject or candidate for therapy with an anti-EGFR antibody provided herein, such as a modified anti-EGFR antibody provided herein, includes, but is not limited to, a subject, such as a human patient, that has experienced one more side effects resulting from administration of an anti-EGFR antibody, such as any anti-EGFR antibody known in the art. Administration of a anti-EGFR provided herein to the subject in place of the anti-EGFR antibody therapy that caused the side effect(s) can result in comparable or improved therapeutic efficacy, while resulting in reduced or lessened side effect(s). Thus, in such methods, a subject that has been administered an anti-EGFR antibody therapeutic other than the anti-EGFR antibodies provided herein, and that exhibits one or more symptoms or side effects associated with administration of the therapeutic, is identified. The identified patient is then administered an anti-EGFR antibody provided herein and therapy is continued. The dosage regime, including dosage amount and frequency of administration, of the anti-EGFR provided herein can be the same or different than the previous anti-EGFR antibody therapy. In some cases, the dosage amount can be increased or decreased. It is within the skill of the practicing physician to determine the dosage regime based on factors such as the particular subject being treated, the nature of the disease or condition, the nature of the existing symptoms or side effects and the particular modified anti-EGFR antibody provided herein that is to be administered.

As discussed elsewhere herein, EGFR is expressed in many normal human tissues (Lacouture, and Melosky (2007) Skin Therapy Lett. 12, 1-5). Therefore, administration of many therapeutic anti-EGFR antibodies, such as Cetuximab, can result in undesirable reactions. Such side effects are well-known to one of skill in the art and can be assessed or identified. Methods to identify side effects caused by an anti-EGFR antibody therapeutic include any methods described herein, such as patient interview, patient examination and blood tests. Side effects that can be assessed include any side effects that are known to one of skill in the art to be associated with administration of an anti-EGFR antibody, including any side effects described herein, such as, for example, a side effect associated with administration of Cetuximab.

For example, side effects of Cetuximab include any described herein and/or known to one of skill in the art, including symptomatic hypomagnesemia, paronychia, fever, dermatologic toxicity, papulopustular rash of the face and upper trunk, hair growth abnormalities, loss of scalp hair, increased growth of facial hair and eyelashes, dry and itchy skin, and periungual inflammation with tenderness (Eng (2009) Nat. Rev. 6:207-218; Schrag et al. (2005) J. Natl. Cancer Inst. 97(16):1221-1224; Lacouture and Melosky (2007) Skin Therapy Lett. 12:1-5). In some examples, the side effects of Cetuximab include dermatological toxicities, including papulopustular eruption, dry skin, pruritus, ocular and nail changes, acneiform skin reaction, acneiform rash, acneiform follicular rash, acne-like rash, maculopapular skin rash, monomorphic pustular lesions, papulopustular reaction. (Lacouture and Melosky (2007) Skin Therapy Lett. 12:1-5) The side effects can be triggered by external events and/or can develop over time. For example, skin rashes can be triggered by sun exposure and can develop in stages, such as sensory disturbance, erythema, and edema (for example, week 1); papulopustular eruption (for example, week 2); and crusting (for example, week 4). If the rash is treated successfully, erythema and dry skin can be seen in areas previously affected by the papulopustular eruption (for example, weeks 4-6). Other dermatological toxicities that can be associated with administration of an anti-EGFR antibody, such as Cetuximab include pruritus, erythema and paronychial inflammation. (Lacouture, and Melosky (2007) Skin Therapy Lett.

12, 1-5). For example, Cetuximab elicits an immune response in about 5% of patients. Such an immune response can result in an immune complex-mediated clearance of the antibodies or fragments from the circulation, and make repeated administration unsuitable for therapy, thereby reducing the therapeutic benefit to the patient and limiting the re-administration of the antibody.

In some examples, the severity of side effects can be evaluated according to the National Cancer Institute Common Terminology Criteria for Adverse Events (CTCAE) v4.0, which sets forth criteria for grading the severity for side effects. The CTCAE includes Grades 1 through 5 that set forth unique clinical descriptions of severity for each adverse effect. Under the general guidelines of the CTCAE, Grade 1 adverse events are mild, asymptomatic or mild symptoms, clinical or diagnostic observations only; and intervention is not indicated. Grade 2 adverse events are moderate, minimal, local or noninvasive intervention indicated, limiting age-appropriate instrumental Activities of Daily Living (ADL). Grade 3 adverse events are severe or medically significant but not immediately life-threatening, with hospitalization or prolongation of hospitalization indicated, disabling and limiting self care ADL. Grade 4 adverse events are life-threatening consequences, and urgent intervention is indicated. Grade 5 adverse events are classified as death related to the adverse event(s). Thus, for example, administering a anti-EGFR antibody provided herein in a subject identified as having a particular grade of side effects can result in a reduction of side effects is characterized by a reduction in the grade of the side effect as classified under the CTCAE v4.0. In some examples, reduction of side effects is characterized by a reduction in the severity of the symptoms associated with the side effect, including any symptoms described herein or known to one of skill in the art.

Other methods to identify patients that exhibit a side effect of an anti-EGFR antibody are known to one of skill in the art, and include quality-of-life questionnaires (*e.g.,* Jonker et al. (2007) N. Engl. J. Med. 357:2040-2048). Examples of side effects of anti-EGFR antibodies, and methods known to the skilled artisan to identify the severity of side effects, are described below. These side effects are exemplary and not meant to be limiting. It is understood that any side effects known in the art or described herein that are associated with administration of an anti-EGFR antibody, such as Cetuximab, can be identified in a subject, whereby the subject can then be treated with an anti-EGFR antibody, such as a modified anti-EGFR antibody, provided herein so that such side effects are not further exacerbated and/or are reduced.

**i. Skin toxicities**

In human skin, EGFR is expressed in basal keratinocytes and can stimulate epidermal growth, inhibit differentiation, and accelerate wound healing (Lacouture, and Melosky (2007) Skin Therapy Lett. 12:1-5; Nanney et al. (1996) J. Invest. Dermatol 94(6):742-748). Inhibition of EGFR function can impair growth and migration of keratinocytes, and result in inflammatory chemokine expression. These effects can lead to inflammatory cell recruitment and subsequent cutaneous injury, which can result in side effects, such as side effects described herein. The pH of the skin basal layer environment is neutral (*e.g.,* at or about pH 7.0 - 7.2). Therefore, anti-EGFR antibodies, such as modified anti-EGFR antibodies, that have increased activity at low pH than at neutral pH, such as the anti-EGFR antibodies provided herein, can have decreased skin toxicity and decreased side effects. Examples of side effects resulting from EGFR inhibition in the skin, and methods of identification and classification thereof, are described below.

Papulopustular rash and acneiform rash, which are characterized by an eruption consisting of papules (a small, raised pimple) and pustules (a small pus filled blister), typically appearing in face, scalp, and upper chest and back. Unlike acne, papulopustular rash does not present with whiteheads or blackheads, and can be symptomatic, with itchy or tender lesions. (CTCAE v. 4.03, U.S. Department of Health and Human Services, published June 14, 2010). Papulopustular rash and acneiform rash can be identified and classified by examination of the patient and/or by clinical interview. Grade 1 papulopustular rash or acneiform rash is classified as papules and/or pustules covering <10% Body Surface Area (BSA), which can be associated with symptoms of pruritus or tenderness. Grade 2 papulopustular rash or acneiform rash is classified as papules and/or pustules covering 10-30% BSA, which can be associated with symptoms of pruritus or tenderness; associated with psychosocial impact; limiting instrumental activities of daily living (ADL). Grade 3 papulopustular rash or acneiform rash is classified as papules and/or pustules covering >30% BSA, which can be associated with symptoms of pruritus or tenderness; limiting self-care ADL; and can be associated with local superinfection with oral antibiotics indicated. Grade 4 papulopustular rash or acneiform rash is classified as papules and/or pustules covering any percent BSA, which can be associated with symptoms of pruritus or tenderness and are associated with extensive superinfection with IV antibiotics indicated; and life-threatening consequences. Grade 5 papulopustular rash or acneiform rash is classified as resulting in death. (CTCAE v. 4.03, U.S. Department of Health and Human Services, published June 14, 2010; Schrag J. Natl. Cancer. Inst. 97(16):1221-1224).

An example of a side effect of an anti-EGFR antibody, such as Cetuximab, is dry skin, which is a disorder characterized by flaky and dull skin; fine pores, and papery thin skin texture. Dry skin can be identified and classified by examination of the patient and/or by clinical interview. Grade 1 dry skin is classified as covering < 10% BSA and no associated erythema or pruritus. Grade 2 dry skin is classified as covering 10%- 30% BSA, associated with erythema or pruritus and limiting instrumental ADL. Grade 3 dry skin is classified as covering >30% BSA, associated with pruritus and limiting self care ADL. (CTCAE v. 4.03, U.S. Department of Health and Human Services, published June 14, 2010; Schrag J. Natl. Cancer. Inst. 97(16):1221-1224)

Skin hyperpigmentation is a side effect characterized by darkening of the skin due to excessive melanin deposition. Skin hyperpigmentation can be identified and classified by examination of the patient and/or by clinical interview. Grade 1 skin hyperpigmentation is classified as hyperpigmentation covering < 10% BSA, no psychosocial impact. Grade 2 skin hyperpigmentation is classified as hyperpigmentation covering > 10% BSA, and associated with psychosocial impact. (CTCAE v. 4.03, U.S. Department of Health and Human Services, published June 14, 2010; Schrag J. Natl. Cancer. Inst. 97(16): 1221-1224)

Pruritus is a side effect characterized by an intense itching sensation. Pruritus can be evaluated by patient examination and/or clinical interview. Grade 1 pruritus is classified as mild or localized itching, and topical intervention is indicated. Symptoms of grade 2 pruritus include intense or widespread itching, intermittent itching, skin changes from scratching (e.g., edema, papulation, excoriations, lichenification, oozing/crusts), limiting instrumental ADL, and oral intervention can be indicated. Symptoms of grade 3 pruritus include intense, widespread and/or constant itching, limiting self care ADL or sleep, and oral corticosteroid or immunosuppressive therapy can be indicated. (CTCAE v. 4.03, U.S. Department of Health and Human Services, published June 14, 2010; Schrag J. Natl. Cancer. Inst. 97(16):1221-1224)

Paronychia is a side effect characterized by an infectious process involving the soft tissues around the nail. Paronychia can be evaluated by patient examination and/or clinical interview. Grade 1 paronychia is classified as including symptoms of nail fold edema or erythema and disruption of the cuticle. Symptoms of grade 2 paronychia can include localized intervention indicated, oral intervention indicated (e.g., antibiotic, antifungal, antiviral), nail fold edema or erythema with pain, discharge or nail plate separation and limiting instrumental ADL. Symptoms of grade 3 paronychia can include limiting self care ADL, with surgical intervention or IV antibiotics indicated.

### ii. Hypomagnesemia

EGFR is highly expressed in the kidney, particularly in the ascending limb of the loop of Henle where 70% of filtered magnesium is reabsorbed. Therefore, antibodies that interact with EGFR can interfere with magnesium transport. Hypomagnesemia, a low concentration of magnesium in the blood, can be a side effect of administration of an anti-EGFR antibody. In one study, five percent of patients receiving cetuximab therapy exhibited grade 3 or 4 hypomagnesemia.

The loop of Henle has a neutral pH (e.g., pH 6.9 - 7.4) (Dieleman et al. (2001) J. Acquir Immune Defic Syndr. 28(1):9-13; Dantzler et al. (2000) Pflugers Arch. 440(1):140-148.). Therefore, modified anti-EGFR antibodies that have increased activity at low pH than at neutral pH, such as the modified anti-EGFR antibodies provided herein, can have decreased hypomagnesemia.

Hypomagnesemia can be diagnosed and/or assessed by measurement of serum magnesium levels. For example, the CTCAE classifies Grade 1 hypomagnesemia as a serum magnesium concentration of < Lower Limit of Normal (LLN) - 1.2 mg/dL; Grade 2 hypomagnesemia as 1.2-0.9 mg/dL serum magnesium; Grade 3 hypomagnesemia as <0.9-0.7 mg/dL serum magnesium, Grade 4 hypomagnesemia as <0.7 mg/dL serum magnesium and can be accompanied by life-threatening consequences and Grade 5 hypomagnesemia results in death. In addition, symptoms of hypomagnesemia are known to the skilled artisan and include fatigue, paresthesias and hypocalcemia. (CTCAE v. 4.03, U.S. Department of Health and Human Services, published June 14, 2010; Schrag J. Natl. Cancer. Inst. 97(16):1221-1224).

### d. Other Methods of Selecting or Identifying Subjects For Treatment

Other methods of screening candidates for therapy known in the art are contemplated. For example, Kirsten rat sarcoma viral oncogene homolog (KRAS) mutation status has recently been shown to be predictive of response to cetuximab therapy in colorectal cancer (Van Cutsem et al. (2008) J. Clin. Oncol 26 (May 20 suppl): Abstract 2). KRAS is a GTPase with a role in a number of signal transduction pathways. Mutations in the gene which encodes KRAS, present in over 25% of colorectal cancers, is predictive of the success of EGFR- inhibiting drugs. Expression of the mutated KRAS gene results in a diminished response to EGFR-inhibitor therapy. KRAS mutations can be detected by commercially available laboratory diagnostics.

### 3. Dosages

In the methods provided herein, a therapeutically effective amount of an anti-EGFR antibody or antibody fragment can be administered. Such dosage can be empirically determined by one of skill in the art, such as the treating physician. In some examples, the administered dosages are based on reference to dosage amounts of known anti-EGFR antibodies, such as Cetuximab, for a particular disease or condition. The therapeutically effective concentration of a anti-EGFR antibody, such as a modified anti-EGFR antibody, provided herein can be determined empirically by testing the anti-EGFR antibodies in known in vitro and in vivo systems such as by using the assays provided herein or known in the art.

An effective amount of anti-EGFR antibody to be administered therapeutically will depend, for example, upon the therapeutic objectives, the route of administration, and the condition of the patient. In addition, the attending physician can take into consideration various factors known to modify the action of drugs, including severity and type of disease, patient's health, body weight, sex, diet, time and route of administration, other medications and other relevant clinical factors. In addition, the therapist can consider the incidence and severity of side effects, such as side effects described herein or known in the art. Accordingly, the therapist can titer the dosage of the antibody or antigen-binding fragment thereof and modify the route of administration as required to obtain the optimal therapeutic effect and minimize undesirable side effects. The clinician can administer the antibody until a dosage is reached that achieves the desired effect. The progress of this therapy can be monitored by conventional assays described herein or known in the art. The dose of the modified anti-EGFR antibody can be varied to identify the optimal or minimal dose required to achieve activity while reducing or eliminating side effects.

Generally, the dosage ranges for the administration of the anti-EGFR antibody, such as modified anti-EGFR antibodies, provided herein are those large enough to produce the desired therapeutic effect in which the symptom(s) of the condition responsive to treatment with an anti-EGFR antibody are ameliorated. Generally, the dosage will vary with the age, condition, sex and the extent of the disease in the patient and can be determined by one of skill in the art. In some examples, the dosage is not so large as to cause adverse side effects. The dosage can be adjusted by the individual physician in the event of the appearance of any adverse side effect. Exemplary dosages include, but are not limited to, about or 0.1 mg/kg to 100 mg/kg, such as at least about or about 0.1 mg/kg, about or 0.15 mg/kg, about or 0.2 mg/kg, about or 0.25 mg/kg, about or 0.30 mg/kg, about or 0.35 mg/kg, about or 0.40 mg/kg, about or 0.45 mg/kg, about or 0.5 mg/kg, about or 0.55 mg.kg, about or 0.6 mg/kg, about or 0.7 mg/kg, about or 0.8 mg/kg, about or 0.9 mg/kg, about or 1.0 mg/kg, about or 1.1 mg/kg, about or 1.2 mg/kg, about or 1.3 mg/kg, about or 1.4 mg/kg, about or 1.5 mg/kg, about or 1.6 mg/kg, about or 1.7 mg/kg, about or 1.8 mg/kg, about or 1.9 mg/kg, about or 2 mg/kg, about or 2.5 mg/kg, about or 3 mg/kg, about or 3.5 mg/kg, about or 4 mg/kg, about or 4.5 mg/kg, about or 5 mg/kg, about or 5.5 mg/kg, about or 6 mg/kg, about or 6.5 mg/kg, about or 7 mg/kg, about or 7.5 mg/kg, about or 8 mg/kg, about or 8.5 mg/kg, about or 9 mg/kg, about or 9.5 mg/kg, about or 10 mg/kg, about or 11 mg/kg, about or 12 mg/kg, about or 13 mg/kg, about or 14 mg/kg, about or 15 mg/kg, about or 16 mg/kg, about or 17 mg/kg, about or 18 mg/kg, about or 19 mg/kg, about or 20 mg/kg, about or 21 mg/kg, about or 22 mg/kg, about or 23 mg/kg, about or 24 mg/kg, about or 25 mg/kg, about or 30 mg/kg, about or 40 mg/kg, about or 50 mg/kg, about or 60 mg/kg, about or 70 mg/kg, about or 80 mg/kg, about or 90 mg/kg, about or 100 mg/kg or more. In some examples, exemplary dosages include, but are not limited to, about or 0.01 mg/m$^2$ to about or 800 mg/m$^2$, such as for example, at least about or about or 0.01 mg/m$^2$, about or 0.1 mg/m$^2$, about or 0.5 mg/m$^2$, about or 1 mg/m$^2$, about or 5 mg/m$^2$, about or 10 mg/m$^2$, about or 15 mg/m$^2$, about or 20 mg/m$^2$, about or 25 mg/m$^2$, about or 30 mg/m$^2$, about or 35 mg/m$^2$, about or 40 mg/m$^2$, about or 45 mg/m$^2$, about or 50 mg/m$^2$, about or 100 mg/m$^2$, about or 150 mg/m$^2$, about or 200 mg/m$^2$, about or 250 mg/m$^2$, about or 300 mg/m$^2$, about or 400 mg/m$^2$, about or 500 mg/m$^2$, about or 600 mg/m$^2$ and about or 700 mg/m$^2$. It is understood that one of skill in the art can recognize and convert dosages between units of mg/kg and mg/m$^2$ (see, e.g., Michael J. Derelanko, TOXICOLOGIST'S POCKET HANDBOOK, CRC Press, p.16 (2000)).

For treatment of a disease or condition, the dosage of the anti-EGFR antibodies can vary depending on the type and severity of the disease. The anti-EGFR antibodies can be administered in a single dose, in multiple separate administrations, or by continuous infusion. For repeated administrations over several days or longer, depending on the condition, the treatment can be repeated until a desired suppression of disease symptoms occurs or the desired improvement in the patient's condition is achieved. Repeated administrations can include increased or decreased amounts of the anti- EGFR antibody depending on the progress of the treatment. For example, an initial loading dose can be larger than a maintenance dose. In some examples, the initial loading dose is 400 mg/ m$^2$, and the maintenance dose is 250 mg/m$^2$.

Other dosage regimens also are contemplated. For example, the dosage regime can be varied. Anti-EGFR antibodies, such as modified anti-EGFR antibodies, that are associated with reduced side effects can be used at higher dosing regimens. In addition, anti-EGFR antibodies that have increased activity in diseased tissues can be used at lower dosing regimens. Methods of determining activity of anti-EGFR antibodies, including the modified anti-EGFR antibodies described herein, are known to one of skill in the art and exemplary methods are described herein. In addition, the dosage regime can be empirically determined. The optimal quantity and spacing of individual dosages of an anti-EGFR antibody of the disclosure will be determined by the nature and extent of the condition being treated, the form, the route and site of administration, and the age and condition of the particular subject being treated, and a physician can determine appropriate dosages to be used. This dosage can be repeated as often as appropriate. If side effects develop, the amount and/or frequency of the dosage can be altered or reduced, in accordance with normal clinical practice. Such studies are within the level of one of skill in the art.

In some examples, the anti-EGFR antibodies are administered one time, two times, three times, four times, five times, six times, seven times, eight times, nine times, ten times or more per day or over several days. In some examples, the anti-EGFR antibodies are administered in a sequence of two or more administrations, where the administrations are separated by a selected time period. In some examples, the selected time period is at least or about 1 day, 2 days, 3 days, 4 days, 5 days, 6 days, 1 week, 2 weeks, 3 weeks, 1 month, 2 months, or 3 months.

Side effects of a particular dosage or dosage regimen also can be assessed, for example, by any methods described herein or known in the art, following administration of one or more doses of the anti-EGFR antibody thereof. Dosage amounts and/or frequency of administration can be modified depending on the type and severity of the side effect(s).

As will be understood by one of skill in the art, the optimal treatment regimen will vary and it is within the scope of the treatment methods to evaluate the status of the disease under treatment and the general health of the patient prior to, and following one or more cycles of therapy in order to determine the optimal therapeutic dosage and frequency of administration. It is to be further understood that for any particular subject, specific dosage regimens can be adjusted over time according to the individual need and the professional judgment of the person administering or supervising the administration of the pharmaceutical formulations, and that the dosages set forth herein are exemplary only and are not intended to limit the scope thereof. The amount of an anti-EGFR antibody to be administered for the treatment of a disease or condition, such as a disease or condition described herein, can be determined by standard clinical techniques described herein or known in the art. In addition, *in vitro* assays and animal models can be employed to help identify optimal dosage ranges. Such assays can provide dosages ranges that can be extrapolated to administration to subjects, such as humans. Methods of identifying optimal dosage ranges based on animal models are well known by those of skill in the art, and examples are described herein.

### 4. Routes of Administration

The anti-EGFR antibodies, such as modified anti-EGFR antibodies, provided herein can be administered to a subject by any method known in the art for the administration of polypeptides, including for example systemic or local administration. The anti-EGFR antibodies can be administered by routes, such as parenteral (*e.g.,* intradermal, intramuscular, intraperitoneal, intravenous, subcutaneous, or intracavity), topical, epidural, or mucosal (*e.g.* intranasal, oral, vaginally, vulvovaginal, esophageal, oroesophageal, bronchial, or pulmonary). The anti-EGFR antibodies can be administered externally to a subject, at the site of the disease for exertion of local or transdermal action. Compositions containing anti-EGFR antibodies or antigen-binding fragments can be administered by any convenient route, for example by infusion or bolus injection, by absorption through epithelial or mucocutaneous linings (*e.g.,* oral mucosa, vaginal, rectal and intestinal mucosa). Compositions containing anti- EGFR antibodies or antigen-binding fragments can be administered together with other biologically active agents. In particular examples, the anti- EGFR antibodies are administered by infusion delivery, such as by infusion pump or syringe pump, and can be administered in combination with another therapeutic agent or as a monotherapy

The method and/or route of administration can be altered to alleviate adverse side effects associated with administration of a anti-EGFR antibody provided herein. For example, if a patient experiences a mild or moderate (i.e., Grade 1 or 2) infusion reaction, the infusion rate can be reduced (e.g. reduced by 10%, 20%, 30%, 40%, 50%, 60%, 70%, 80%, 90% or more). If the patient experiences severe (i.e., Grade 3 or 4) infusion reactions, the infusion can be temporarily or permanently discontinued.

In some examples, if the subject experiences an adverse side effect, such as severe skin toxicity, for example severe acneform rash, treatment adjustments can be made. For example, after the occurrence of an adverse side effect, administration can be delayed, such as for 1 to 2 weeks or until the adverse side effect improves. In some examples, after additional occurrences of an adverse side effect, the dosage can be reduced. For example, if the dose is 250 mg/m$^2$, after the second occurrence of an adverse side effect, administration of the anti-EGFR antibody can be delayed for 1 to 2 weeks. If the side effect improves, administration of the anti-EGFR antibody can continue with the dose reduced to 250 mg/m$^2$. After the third occurrence of the side effect, administration of the anti-EGFR antibody can be delayed for 1 to 2 weeks. If the side effect improves, administration of the anti-EGFR antibody can continue with the dose reduced to 150 mg/m$^2$. After several occurrences of an adverse side effect, administration of the anti-EGFR antibody can be discontinued. In patients with mild or moderate skin toxicity, the skilled artisan can continue administration without dose modification. Such determinations are within the ability of the skilled artisan.

Appropriate methods for delivery, can be selected by one of skill in the art based on the properties of the dosage amount of the anti-EGFR antibody or the pharmaceutical composition containing the antibody or antigen-binding fragment thereof. Such properties include, but are not limited to, solubility, hygroscopicity, crystallization properties, melting point, density, viscosity, flow, stability and degradation profile.

**5. Combination Therapies**

In the methods provided herein, the anti-EGFR antibodies, such as modified anti-EGFR antibodies, provided herein can be administered before, after, or concomitantly with one or more other therapeutic regimens or agents. The skilled medical practitioner can determine empirically, or by considering the pharmacokinetics and modes of action of the agents, the appropriate dose or doses of each therapeutic regimen or agent, as well as the appropriate timings and methods of administration. The additional therapeutic regimes or agents can improve the efficacy or safety of the anti-EGFR antibody. In some examples, the additional therapeutic regimes or agents can treat the same disease or a comorbidity rather than to alter the action of the anti-EGFR antibody. In some examples, the additional therapeutic regimes or agents can ameliorate, reduce or eliminate one or more side effects known in the art or described herein that are associated with administration of an anti-EGFR antibody.

For example, an anti-EGFR antibody described herein can be administered with chemotherapy, radiation therapy, or both chemotherapy and radiation therapy. The modified anti-EGFR antibodies can be administered in combination with one or more other prophylactic or therapeutic agents, including but not limited to antibodies, cytotoxic agents, chemotherapeutic agents, cytokines, growth inhibitory agents, anti-hormonal agents, kinase inhibitors, anti-angiogenic agents, cardioprotectants, immunostimulatory agents, immunosuppressive agents, agents that promote proliferation of hematological cells, angiogenesis inhibitors, protein tyrosine kinase (PTK) inhibitors, additional anti-EGFR antibodies, FcγRIIb or other Fc receptor inhibitors, or other therapeutic agents.

The one or more additional agents can be administered simultaneously, sequentially or intermittently with the anti-EGFR antibody thereof. The agents can be co-administered with the anti-EGFR antibody thereof, for example, as part of the same pharmaceutical composition or same method of delivery. In some examples, the agents can be co-administered with the anti-EGFR antibody at the same time as the modified anti-EGFR antibody thereof, but by a different means of delivery. The agents also can be administered at a different time than administration of the anti-EGFR antibody thereof, but close enough in time to the administration of the anti-EGFR antibody to have a combined prophylactic or therapeutic effect. In some examples, the one or more additional agents are administered subsequent to or prior to the administration of the anti-EGFR antibody separated by a selected time period. In some examples, the time period is 1 day, 2 days, 3 days, 4 days, 5 days, 6 days, 1 week, 2 weeks, 3 weeks, 1 month, 2 months, or 3 months. In some examples, the one or more additional agents are administered multiple times and/or the anti-EGFR antibody provided herein is administered multiple times.

In some examples, an anti-EGFR antibody, such as a modified anti-EGFR antibody, provided herein is administered with one or more antibodies or antibody fragments. The anti-EGFR antibody can be administered with one or more other antibodies that have efficacy in treating the same disease or an additional comorbidity. For example, the one or more antibodies administered with the anti-EGFR antibody can be selected from among anti-cancer antibodies, antibodies to treat autoimmune or inflammatory disease, antibodies to treat transplant rejection, antibodies to treat graft-versus-host-disease (GVHD) and antibodies to treat infectious diseases. In some examples, two or more of the anti-EGFR antibodies provided herein are administered in combination.

Examples of anti-cancer antibodies that can be co-administered with a anti-EGFR antibody provided herein include, but are not limited to, anti 17-IA cell surface antigen antibodies such as Panorex® (edrecolomab); anti-4-1BB antibodies; anti-4Dc antibodies; anti-A33 antibodies such as A33 and CDP-833; anti-α1 integrin antibodies such as natalizumab; anti-α4β7 integrin antibodies such as LDP-02; anti-αVβ1 integrin antibodies such as F-200, M-200, and SJ-749; anti-αVβ3 integrin antibodies such as abciximab, CNTO-95, Mab-17E6, and Vitaxin®; anti-complement factor 5 (C5) antibodies such as 5G1.1; anti-CA125 antibodies such as OvaRex® (oregovomab); anti-CD3 antibodies such as Nuvion® (visilizumab) and Rexomab; anti-CD4 antibodies such as IDEC-151, MDX-CD4, OKT4A; anti-CD6 antibodies such as Oncolysin B and Oncolysin CD6; anti-CD7 antibodies such as HB2; anti-CD19 antibodies such as B43, MT-103, and Oncolysin B; anti-CD20 antibodies such as 2H7, 2H7.v16, 2H7.v114, 2H7.v115, Bexxar®(tositumomab), Rituxan® (rituximab), and Zevalin® (Ibritumomab tiuxetan); anti-CD22 antibodies such as Lymphocide® (epratuzumab); anti-CD23 antibodies such as IDEC-152; anti-CD25 antibodies such as basiliximab and Zenapax® (daclizumab); anti-CD30 antibodies such as AC10, MDX-060, and SGN-30; anti-CD33 antibodies such as Mylotarg® (gemtuzumab ozogamicin), Oncolysin M, and Smart MI 95; anti-CD38 antibodies; anti-CD40 antibodies such as SGN-40 and toralizumab; anti-CD40L antibodies such as 5c8, Antova®, and IDEC-131; anti-CD44 antibodies such as bivatuzumab; anti-CD46 antibodies; anti-CD52 antibodies such as Campath® (alemtuzumab); anti-CD55 antibodies such as SC-1; anti-CD56 antibodies such as huN901-DM1; anti-CD64 antibodies such as MDX-33; anti-CD66e antibodies such as XR-303; anti-CD74 antibodies such as IMMU-1 10; anti-CD80 antibodies such as galiximab and IDEC-1 14; anti-CD89 antibodies such as MDX-214; anti-CD123 antibodies; anti-CD138 antibodies such as B-B4-DM1; anti-CD146 antibodies such as AA-98; anti-CD148 antibodies; anti-CEA antibodies such as cT84.66, labetuzumab, and Pentacea®; anti-CTLA-4 antibodies such as MDX-101; anti-CXCR4 antibodies; anti-EGFR antibodies such as ABX-EGF, Erbitux® (cetuximab), IMC-C225, and Merck Mab 425; anti-EpCAM antibodies such as Crucell's anti-EpCAM, ING-1, and IS-IL-2; anti-ephrin B2/EphB4 antibodies; anti-Her2 antibodies such as Herceptin®), MDX-210; anti-FAP (fibroblast activation protein) antibodies such as sibrotuzumab; anti-ferritin antibodies such as NXT-211; anti-FGF-1 antibodies; anti-FGF-3 antibodies; anti-FGF-8 antibodies; anti-FGFR antibodies, anti-fibrin antibodies; anti-G250 antibodies such as WX-G250 and Rencarex®; anti-GD2 ganglioside antibodies such as EMD-273063 and TriGem; anti-GD3 ganglioside antibodies such as BEC2, KW-2871, and mitumomab; anti-gpIIb/IIIa antibodies such as ReoPro; anti-heparinase antibodies; anti-Her2/ErbB2 antibodies such as Herceptin® (trastuzumab), MDX-210, and pertuzumab; anti-HLA antibodies such as Oncolym®, Smart 1D10; anti-HM1.24 antibodies; anti-ICAM antibodies such as ICM3; anti-IgA receptor antibodies; anti-IGF-1 antibodies such as CP-751871 and EM-164; anti-IGF-1R antibodies such as IMC-A12; anti-IL-6 antibodies such as CNTO-328 and elsilimomab; anti-IL-15 antibodies such as HuMax®-IL15; anti-KDR antibodies; anti-laminin 5 antibodies; anti-Lewis Y antigen antibodies such as Hu3S193 and IGN-311; anti-MCAM antibodies; anti-Muc1 antibodies such as BravaRex and TriAb; anti-NCAM antibodies such as ERIC-1 and ICRT; anti-PEM antigen antibodies such as Theragyn and Therex; anti-PSA antibodies; anti-PSCA antibodies such as IG8; anti-Ptk antibodies; anti-PTN antibodies; anti-RANKL antibodies such as AMG-162; anti-RLIP76 antibodies; anti-SK-1 antigen antibodies such as Monopharm C; anti-STEAP antibodies; anti-TAG72 antibodies such as CC49-SCA and MDX-220; anti-TGF-β antibodies such as CAT-152; anti-TNF-a antibodies such as CDP571, CDP870, D2E7, Humira® (adalimumab), and Remicade® (infliximab); anti-TRAIL-R1 and TRAIL-R2 antibodies; anti-VE-cadherin-2 antibodies; and anti-VLA-4 antibodies such as Antegren®. Furthermore, anti-idiotype antibodies including but not limited to the GD3 epitope antibody BEC2 and the gp72 epitope antibody 105AD7, can be used. In addition, bispecific antibodies including but not limited to the anti-CD3/CD20 antibody Bi20 can be used.

Examples of antibodies that can treat autoimmune or inflammatory disease, transplant rejection, GVHD, that can be co-administered with a modified anti-EGFR antibody provided herein include, but are not limited to, anti-α4β7 integrin antibodies such as LDP-02, anti-beta2 integrin antibodies such as LDP-01, anti-complement (C5) antibodies such as 5G1.1, anti-CD2 antibodies such as BTI-322, MEDI-507, anti-CD3 antibodies such as OKT3, SMART anti-CD3, anti-CD4 antibodies such as IDEC-151, MDX-CD4, OKT4A, anti-CDlla antibodies, anti-CD14 antibodies such as IC14, anti-CD18 antibodies, anti-CD23 antibodies such as IDEC 152, anti-CD25 antibodies such as Zenapax, anti-CD40L antibodies such as 5c8, Antova, IDEC-131, anti-CD64 antibodies such as MDX-33, anti-CD80 antibodies such as IDEC-114, anti-CD 147 antibodies such as ABX-CBL, anti-E-selectin antibodies such as CDP850, anti-gpIIb/IIIa antibodies such as ReoPro®/Abcixima, anti-ICAM-3 antibodies such as ICM3, anti-ICE antibodies such as VX-740, anti-FcγR1 antibodies such as MDX-33, anti-IgE antibodies such as rhuMab-E25, anti-IL-4 antibodies such as SB-240683, anti-IL-5 antibodies such as SB-240563, SCH55700, anti-IL-8 antibodies such as ABX-IL8, anti-interferon gamma antibodies, and anti-TNFa antibodies such as CDP571, CDP870, D2E7, Infliximab, MAK-195F, anti-VLA-4 antibodies such as Antegren. Examples of other Fc-containing molecules that can be co-administered to treat autoimmune or inflammatory disease, transplant rejection and GVHD include, but are not limited to, the p75 TNF receptor/Fc fusion Enbrel® (etanercept) and Regeneron's IL-1 trap.

Examples of antibodies that can be co-administered to treat infectious diseases include, but are not limited to, anti-anthrax antibodies such as ABthrax, anti-CMV antibodies such as CytoGam and sevirumab, anti-cryptosporidium antibodies such as CryptoGAM, Sporidin-G, anti-helicobacter antibodies such as Pyloran, anti-hepatitis B antibodies such as HepeX-B, Nabi-HB, anti-HIV antibodies such as HRG-214, anti-RSV antibodies such as felvizumab, HNK-20, palivizumab, RespiGam, and anti-staphylococcus antibodies such as Aurexis, Aurograb, BSYX-A110, and SE-Mab.

In some examples, an anti-EGFR antibody, such as a modified anti-EGFR antibody, described herein is administered with one or more molecules that compete for binding to one or more Fc receptors. For example, co-administering inhibitors of the inhibitory receptor FcγRIIb can result in increased effector function. Similarly, co-administering inhibitors of the activating receptors such as FcγRIIIa can minimize unwanted effector function. Fc receptor inhibitors include, but are not limited to, Fc molecules that are engineered to act as competitive inhibitors for binding to FcγRIIb, FcγRIIIa, or other Fc receptors, as well as other immunoglobulins and specifically the treatment called IVIg (intravenous immunoglobulin). In one embodiment, the inhibitor is administered and allowed to act before the anti-EGFR antibody is administered. An alternative way of achieving the effect of sequential dosing would be to provide an immediate release dosage form of the Fc receptor inhibitor and then a sustained release formulation of the anti-EGFR antibody. The immediate release and controlled release formulations could be administered separately or be combined into one unit dosage form.

In some examples, an anti-EGFR antibody, such as a modified anti-EGFR antibody, described herein is administered with one or more chemotherapeutic agents. Examples of chemotherapeutic agents include but are not limited to alkylating agents such as thiotepa and cyclophosphamide (CYTOXAN®); alkyl sulfonates such as busulfan, improsulfan and piposulfan; androgens such as calusterone, dromostanolone propionate, epitiostanol, mepitiostane, testolactone; anti-adrenals such as aminoglutethimide, mitotane, trilostane; anti-androgens such as flutamide, nilutamide, bicalutamide, leuprolide, and goserelin; antibiotics such as aclacinomycins, actinomycin, anthramycin, azaserine, bleomycins, cactinomycin, calicheamicin, carubicin, carminomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti estrogens including for example tamoxifen, raloxifene, aromatase inhibiting 4(5)-imidazoles, 4-hydroxytamoxifen, trioxifene, keoxifene, LY 117018, onapristone, and toremifene (Fareston); anti-metabolites such as methotrexate and 5-fluorouracil (5-FU); folic acid analogues such as denopterin, methotrexate, pteropterin, trimetrexate; aziridines such as benzodepa, carboquone, meturedepa, and uredepa; ethylenimines and methylmelamines including altretamine, triethylenemelamine, triethylenephosphoramide, triethylenethiophosphoramide and trimethylol melamine; folic acid replenisher such as folinic acid; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, ranimustine; platinum analogs such as cisplatin and carboplatin; vinblastine; platinum; proteins such as arginine deiminase and asparaginase; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; taxanes, e.g. paclitaxel (TAXOL®, Bristol-Myers Squibb Oncology, Princeton, N.J.) and docetaxel (TAXOTERE®), Rhone-Poulenc Rorer, Antony, France); topoisomerase inhibitor RFS 2000; thymidylate synthase inhibitor (such as Tomudex); additional chemotherapeutics including aceglatone; aldophosphamide glycoside; aminolevulinic acid; amsacrine; bestrabucil; bisantrene; edatrexate; defosfamide; demecolcine; diaziquone; difluoromethylornithine (DMFO); eflornithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK®; razoxane; sizofiran; spirogermanium; tenuazonic acid; triaziquone; 2,2', 2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; chlorambucil; gemcitabine; 6-thioguanine; mercaptopurine; methotrexate; etoposide (VP-16); ifosfamide; mitomycin C; mitoxantrone; vincristine; vinorelbine; Navelbine; Novantrone; teniposide; daunomycin; aminopterin; Xeloda; ibandronate; CPT-11; retinoic acid; esperamycins; capecitabine; and topoisomerase inhibitors such as irinotecan. Pharmaceutically acceptable salts, acids or derivatives of any of the above can also be used. In some examples, a modified anti-EGFR antibody provided herein is administered with irinotecan (see, e.g., Pfeiffer et al. (2007) Acta. Oncol. 46(5):697-701).

A chemotherapeutic agent can be administered as a prodrug. Examples of prodrugs that can be administered with an anti-EGFR antibody described herein include, but are not limited to, phosphate-containing prodrugs, thiophosphate-containing prodrugs, sulfate-containing prodrugs, peptide-containing prodrugs, D-amino acid-modified prodrugs, glycosylated prodrugs, beta-lactam-containing prodrugs, optionally substituted phenoxy acetamide-containing prodrugs or optionally substituted phenylacetamide-containing prodrugs, 5-fluorocytosine and other 5-fluorouridine prodrugs which can be converted into the more active cytotoxic free drug.

In some examples, an anti-EGFR antibody, such as a modified anti-EGFR antibody, provided herein is administered with one or more anti-angiogenic agents. For example, the anti-angiogenic factor can be a small molecule or a protein (e.g., an antibody, Fc fusion, or cytokine) that binds to a growth factor or growth factor receptor involved in promoting angiogenesis. Examples of anti-angiogenic agents include but are not limited to antibodies that bind to Vascular Endothelial Growth Factor (VEGF) or that bind to VEGF-R, RNA-based therapeutics that reduce levels of VEGF or VEGF-R expression, VEGF-toxin fusions, Regeneron's VEGF-trap, angiostatin (plasminogen fragment), antithrombin III, angiozyme, ABT-627, Bay 12-9566, BeneFin, bevacizumab, bisphosphonates, BMS-275291, cartilage-derived inhibitor (CDI), CAI, CD59 complement fragment, CEP-7055, Col 3, Combretastatin A-4, endostatin (collagen XVIII fragment), farnesyl transferase inhibitors, fibronectin fragment, gro-beta, halofuginone, heparinases, heparin hexasaccharide fragment, HMV833, human chorionic gonadotropin (hCG), IM-862, interferon alpha, interferon beta, interferon gamma, interferon inducible protein 10 (IP-10), interleukin-12, kringle 5 (plasminogen fragment), marimastat, metalloproteinase inhibitors (e.g. TIMPs), 2-methoxyestradiol, MMI 270 (CGS 27023A), plasminogen activator inhibitor (PAI), platelet factor-4 (PF4), prinomastat, prolactin 16 kDa fragment, proliferin-related protein (PRP), PTK 787/ZK 222594, retinoids, solimastat, squalamine, SS3304, SU5416, SU6668, SU11248, tetrahydrocortisol-S, tetrathiomolybdate, thalidomide, thrombospondin-1 (TSP-1), TNP470, transforming growth factor beta (TGF-$\beta$), vasculostatin, vasostatin (calreticulin fragment), ZS6126, and ZD6474.

In some examples, an anti-EGFR antibody, such as a modified anti-EGFR antibody, provided herein is administered with one or more tyrosine kinase inhibitors. Examples of tyrosine kinase inhibitors include but are not limited to quinazolines, such as PD 153035, 4-(3-chloroanilino) quinazoline; pyridopyrimidines; pyrimidopyrimidines; pyrrolopyrimidines, such as CGP 59326, CGP 60261 and CGP 62706; pyrazolopyrimidines, 4-(phenylamino)-7H-pyrrolo(2,3-d) pyrimidines; curcumin (diferuloylmethane, 4,5-bis (4-fluoroanilino) phthalimide); tyrphostins containing nitrothiophene moieties; PD-0183805 (Warner-Lambert); antisense molecules (e.g. those that bind to ErbB-encoding nucleic acid); quinoxalines (U.S. Pat. No. 5,804,396); tyrphostins (U.S. Pat. No. 5,804,396); ZD6474 (Astra Zeneca); PTK-787 (Novartis/Schering A G); pan-ErbB inhibitors such as C1-1033 (Pfizer); Affinitac (ISIS 3521; Isis/Lilly); Imatinib mesylate (STI571, Gleevec®; Novartis); PKI 166 (Novartis); GW2016 (Glaxo SmithKline); C1-1033 (Pfizer) ; EKB-569 (Wyeth); Semaxinib (Sugen); ZD6474 (AstraZeneca); PTK-787 (Novartis/Schering A G); INC-1 C11 (Imclone); or as described in any of the following patent publications: U.S. Pat. No. 5,804,396; PCT WO 99/09016 (American Cyanimid); PCT WO 98/43960 (American Cyanamid); PCT WO 97/38983 (Warner-Lambert); PCT WO 99/06378 (Warner-Lambert); PCT WO 99/06396 (Warner-Lambert); PCT WO 96/30347 (Pfizer, Inc); PCT WO 96/33978 (AstraZeneca); PCT WO 96/33979 (AstraZeneca); PCT WO 96/33980 (AstraZeneca), gefitinib (Iressa®, ZD1839, AstraZeneca), and OSI-774 (Tarceva®, OSI Pharmaceuticals/Genentech).

In some examples, an anti-EGFR antibody, such as a modified anti-EGFR antibody, described herein is administered with one or more immunomodulatory agents. Such agents can increase or decrease production of one or more cytokines, up-or down-regulate self-antigen presentation, mask MHC antigens, or promote the proliferation, differentiation, migration, or activation state of one or more types of immune cells. Examples of immunomodulatory agents include but are not limited to non-steroidal anti-inflammatory drugs (NSAIDs) such as aspirin, ibuprofen, celecoxib, diclofenac, etodolac, fenoprofen, indomethacin, ketorolac, oxaprozin, nabumetone, sulindac, tolmetin, rofecoxib, naproxen, ketoprofen, and nabumetone; steroids (e.g. glucocorticoids, dexamethasone, cortisone, hydroxycortisone, methylprednisolone, prednisone, prednisolone, triamcinolone, azulfidine eicosanoids such as prostaglandins, thromboxanes, and leukotrienes; as well as topical steroids such as anthralin, calcipotriene, clobetasol, and tazarotene); cytokines such as TGFb, IFNa, IFNb, IFNg, IL-2, IL4, IL-10; cytokine, chemokine, or receptor antagonists including antibodies, soluble receptors, and receptor-Fc fusions against BAFF, B7, CCR2, CCR5, CD2, CD3, CD4, CD6, CD7, CD8, CD11, CD14, CD15, CD17, CD18, CD20, CD23, CD28, CD40, CD40L, CD44, CD45, CD52, CD64, CD80, CD86, CD147, CD152, complement factors (C5, D) CTLA4, eotaxin, Fas, ICAM, ICOS, IFN$\alpha$, IFN$\beta$, IFN$\gamma$, IFNAR, IgE, IL-1, IL-2, IL-2R, IL-4, IL-5R, IL-6, IL-8, IL-9 IL-12, IL-13, IL-13R1, IL-15, IL-18R, IL-23, integrins, LFA-1, LFA-3, MHC, selectins, TGF$\beta$, TNF$\alpha$, TNF$\beta$, TNF-R1, T-cell receptor, including Enbrel® (etanercept), Humira® (adalimumab), and Remicade® (infliximab); heterologous anti-lymphocyte globulin; other immunomodulatory molecules such as 2-amino-6-aryl-5 substituted pyrimidines, anti-idiotypic antibodies for MHC binding peptides and MHC fragments, azathioprine, brequinar, Bromocryptine, cyclophosphamide, cyclosporine A, D-penicillamine, deoxyspergualin, FK506, glutaraldehyde, gold, hydroxychloroquine, leflunomide, malononitriloamides (e.g. leflunomide), methotrexate, minocycline, mizoribine, mycophenolate mofetil, rapamycin, and sulfasalazine.

In some examples, an anti-EGFR antibody, such as a modified anti-EGFR antibody, described herein is administered with one or more cytokines. Examples of cytokines include but are not limited to lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-alpha and-beta; mullerian-inhibiting substance; mouse gonadotropin-associated peptide; inhibin; activin; vascular endothelial growth factor; integrin; thrombopoietin (TPO); nerve growth factors such as NGF-beta; platelet-growth factor; transforming growth factors (TGFs) such as TGF-alpha and TGF-beta; insulin-like growth factor-I and-II; erythropoietin (EPO); osteoinductive factors; interferons such as interferon-alpha, beta, and-gamma; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1alpha, IL-2, IL-3, IL-4, IL-5, IL-6, IL-7, IL-8, IL-9, IL-10, IL-11, IL-12; IL-15, a tumor necrosis factor such as TNF-alpha or TNF-beta; and other polypeptide factors including LIF and kit ligand (KL).

In some examples, an anti-EGFR antibody, such as a modified anti-EGFR antibody, described herein is administered with one or more cytokines or other agents that stimulate cells of the immune system and enhance desired effector function. For example, agents that stimulate NK cells, including but not limited to IL-2 can be administered with an anti-EGFR antibody described herein. In another embodiment, agents that stimulate macrophages, including but not limited to C5a, formyl peptides such as N-formyl-methionyl-leucyl-phenylalanine (Beigier-Bompadre et. al. (2003) Scand. J. Immunol. 57: 221-8), can be administered with an anti-EGFR antibody described herein. Also, agents that stimulate neutrophils, including but not limited to G-CSF and GM-CSF, can be administered with an anti-EGFR antibody described herein. Furthermore, agents that promote migration of such immunostimulatory cytokines can be administered with an anti-EGFR antibody described herein. Also additional agents including but not limited to interferon gamma, IL-3 and IL-7 can promote one or more effector functions. In some examples, an anti-EGFR antibody described herein is administered with one or more cytokines or other agents that inhibit effector cell function.

In some examples, an anti-EGFR antibody described herein is administered with one or more antibiotics, including but not limited to: aminoglycoside antibiotics (e.g. apramycin, arbekacin, bambermycins, butirosin, dibekacin, gentamicin, kanamycin, neomycin, netilmicin, paromomycin, ribostamycin, sisomicin, spectinomycin), aminocyclitols (e.g. spectinomycin), amphenicol antibiotics (e.g. azidamfenicol, chloramphenicol, florfenicol, and thiamphenicol), ansamycin antibiotics (e.g. rifamide and rifampin), carbapenems (e.g. imipenem, meropenem, panipenem); cephalosporins (e.g. cefaclor, cefadroxil, cefamandole, cefatrizine, cefazedone, cefozopran, cefpimizole, cefpiramide, cefpirome, cefprozil, cefuroxime, cefixime, cephalexin, cephradine), cephamycins (cefbuperazone, cefoxitin, cefminox, cefmetazole, and cefotetan); lincosamides (e.g. clindamycin, lincomycin); macrolide (e.g. azithromycin, brefeldin A, clarithromycin, erythromycin, roxithromycin, tobramycin), monobactams (e.g. aztreonam, carumonam, and tigemonam); mupirocin; Oxacephems (e.g. flomoxef, latamoxef, and moxalactam); penicillins (e.g. amdinocillin, amdinocillin pivoxil, amoxicillin, bacampicillin, benzylpenicillinic acid, benzylpenicillin sodium, epicillin, fenbenicillin, floxacillin, penamecillin, penethamate hydriodide, penicillin o-benethamine, penicillin O, penicillin V, penicillin V benzoate, penicillin V hydrabamine, penimepicycline, and phenethicillin potassium); polypeptides (e.g. bacitracin, colistin, polymixin B, teicoplanin, vancomycin); quinolones (amifloxacin, cinoxacin, ciprofloxacin, enoxacin, enrofloxacin, fleroxacin, flumequine, gatifloxacin, gemifloxacin, grepafloxacin, lomefloxacin, moxifloxacin, nalidixic acid, norfloxacin, ofloxacin, oxolinic acid, pefloxacin, pipemidic acid, rosoxacin, rufloxacin, sparfloxacin, temafloxacin, tosufloxacin, and trovafloxacin); rifampin; streptogramins (e.g. quinupristin, dalfopristin); sulfonamides (sulfanilamide, sulfamethoxazole); tetracyclines (chlortetracycline, demeclocycline hydrochloride, demethylchlortetracycline, doxycycline, Duramycin, minocycline, neomycin, oxytetracycline, streptomycin, tetracycline, and vancomycin).

In some examples, an anti-EGFR antibody, such as a modified anti-EGFR antibody, provided herein is administered with one or more anti-fungal agents, including but not limited to amphotericin B, ciclopirox, clotrimazole, econazole, fluconazole, flucytosine, itraconazole, ketoconazole, miconazole, nystatin, terbinafine, terconazole, and tioconazole. In some examples, an anti-EGFR antibody described herein is administered with one or more antiviral agents, including but not limited to protease inhibitors, reverse transcriptase inhibitors, and others, including type I interferons, viral fusion inhibitors, neuraminidase inhibitors, acyclovir, adefovir, amantadine, amprenavir, clevudine, enfuvirtide, entecavir, foscarnet, ganciclovir, idoxuridine, indinavir, lopinavir, pleconaril, ribavirin, rimantadine, ritonavir, saquinavir, trifluridine, vidarabine, and zidovudine.

An anti-EGFR antibody, such as a modified anti-EGFR antibody, provided herein can be combined with other therapeutic regimens. For example, in one embodiment, the patient to be treated with a modified anti-EGFR antibody provided herein can receive radiation therapy. Radiation therapy can be administered according to protocols commonly employed in the art and known to the skilled artisan. Such therapy includes but is not limited to cesium, iridium, iodine, or cobalt radiation. The radiation therapy can be whole body irradiation, or can be directed locally to a specific site or tissue in or on the body, such as the lung, bladder, or prostate. Typically, radiation therapy is administered in pulses over a period of time from about 1 to 2 weeks. The radiation therapy can, however, be administered over longer periods of time. For instance, radiation therapy can be administered to patients having head and neck cancer for about 6 to about 7 weeks. Optionally, the radiation therapy can be administered as a single dose or as multiple, sequential doses. The skilled medical practitioner can determine empirically the appropriate dose or doses of radiation therapy useful herein. In some examples, the anti-EGFR antibodies and optionally one or more other anti-cancer therapies are employed to treat cancer cells ex vivo. It is contemplated that such ex vivo treatment can be useful in bone marrow transplantation and particularly, autologous bone marrow transplantation. For instance, treatment of cells or tissue(s) containing cancer cells with a anti-EGFR antibody and one or more anti-cancer therapies, such as described herein, can be employed to deplete or substantially deplete the cancer cells prior to transplantation in a recipient patient.

Radiation therapy can also comprise treatment with an isotopically labeled molecule, such as an antibody. Examples of radioimmunotherapeutics include but Zevalin® (Y-90 labeled anti-CD20), LymphoCide® (Y-90 labeled anti-CD22) and Bexxar® (1-131 labeled anti-CD20).

In addition, it is contemplated that the anti-EGFR antibodies, such as modified anti-EGFR antibodies, provided herein can be administered to a patient or subject in combination with still other therapeutic techniques such as surgery or phototherapy.

## H. EXAMPLES

The following examples are included for illustrative purposes only and are not intended to limit the scope of the invention.

### Example 1

### Generation of anti-EGFR antibody Mutants and Screening for pH-Dependent Activity

### 1. Primary Screen

### a. Generation of Library

A reference Cetuximab anti-EGFR antibody was generated containing a light chain (SEQ ID NO:1110 and encoding SEQ ID NO:9) and a heavy chain (SEQ ID NO:1111 and encoding the complete heavy chain sequence set forth in SEQ ID NO:8), whereby a FLAG tag (SEQ ID NO:13) was linked at the C-terminal end of the constant domain and was cloned into an expression vector to encode an IgG antibody (full length DNA sequence set forth in SEQ ID NO:1109). A library of single point mutants of the Cetuximab anti-EGFR antibody was constructed and generated by site-directed mutagenesis. The library contained variants of Cetuximab anti-EGFR antibody, whereby each member contained a single amino acid mutation compared to the reference antibody at one of one hundred amino acid positions within the variable regions of either the heavy chain (SEQ ID NO:8 with the variable heavy chain set forth in SEQ ID NO:3) or light chain (SEQ ID NO:19 with the variable light chain set forth in SEQ ID NO:10) of Cetuximab. The positions that were varied were in the variable region of the light and heavy chains of the Cetuximab anti-EGFR antibody, with the majority of positions in the CDRs of the light or heavy chain (see Figure 1). At least 15 amino acid mutations were made at each position, whereby the amino acid histidine was included among the 15 mutations at each position. The total number of single variant members of the library that were generated was 1501. Each member of the library was sequenced. Glycerol stocks of members of the library were prepared and stored at -80 °C.

**b. Screening of Library Members**

For screening, an expression vector encoding a member of the library was separately expressed in CHO cells as IgG antibodies and supernatants collected. Plasmid DNA was transfected into monolayer CHO-S cells (Invitrogen, Cat. No. 11619-012) using Lipofectamine 2000 (Invitrogen, Cat. No. 11668-027) following the manufacturer's protocol. Briefly, CHO-S cells were seeded the night before transfection and grown in DMEM with 10% Fetal Bovine Serum (FBS). The next day, after the cells were 80% confluent, the medium of the CHO-S cells was replaced with Opti-MEM (Invitrogen). A mixture of plasmid DNA and Lipofectamine (0.2 $\mu$g DNA and 0.5 $\mu$L Lipofetamine) was added to the CHO-S cells and incubated overnight. The next day, the cells were supplemented with CD-CHO serum free media (Invitrogen, Cat. No. 10743-029). Supernatant from transfected cells was collected after transfection (generally 72 hours after transfection).

The supernatants were assayed for binding to soluble extracellular domain of EGF receptor (EGFR sECD) using a parallel, high-throughput pH sensitive ELISA under two conditions as described below. The EGFR sECD was conjugated to a His tag (sEGFR-H6) and was obtained commercially (Sino Biologics, Cat #10001-H08H).

Briefly, the sEGFR-H6 was immobilized on 96 well Hi-bind plates (Costar #2592) by coating the plate overnight at 4 °C or for 2 hours at room temperature (RT) with 100 $\mu$L sEGFR-H6 antigen at 12 nM (1.32 $\mu$g/mL) in Buffer A (Krebs-Ringer Buffer (KRB, Sigma Aldrich, # K4002), pH 7.4, no human serum). The plates were then washed 3x with 250 $\mu$L/well of Buffer A. Then, the plates were divided into two groups and the first group (pH 7.4 group) was subsequently blocked for 1 hour at RT with 250 $\mu$L of pH 7.4 Buffer B (1 mM lactic acid/ 25 % human serum) and the second group (pH 6.0 group) was subsequently blocked for 1 hour at RT with 250 $\mu$L of pH 6.0 Buffer C (16.6 mM lactic acid / 25 % human serum), while covered.

One hundred microliters (100 $\mu$L) of FLAG-tagged anti-EGFR antibody variant supernatants of each variant member of the Cetuximab anti-EGFR antibody library described above were added at two dilutions (Dilution 1 and Dilution 2) each to a separate well of two 96-well plates (one of each group, pH 6.0 group and pH 7.4 group) containing the bound sEGFR-H6 antigen. For the dilutions, the clarified supernatant samples described above were diluted in either pH 7.4 Buffer B (KRB, pH 7.4, 1 mM lactic acid / 25 % human serum; group 1) or pH 6.0 Buffer C (KRB, pH 6.0, 16.6 mM lactic acid / 25 % human serum; group 2) at 1:20 (Dilution 1) or 1:100 (Dilution 2) in the same buffer. The reference Cetuximab anti-EGFR antibody conjugated to a FLAG tag (anti-EGFR-FLAG antibody) was used as a standard and serial dilutions (3x, starting concentration 30 ng/mL, followed by 1:3 dilutions) were prepared in either pH 7.4 Buffer B (KRB, pH 7.4, 1 mM lactic acid / 25 % human serum) or pH 6.0 Buffer C (KRB, pH 6.0, 16.6 mM lactic acid / 25 % human serum), and 100 $\mu$L was added per well. After dilution, concentrations of Cetuximab anti-EGFR-FLAG antibody were 200 pM (30 ng/mL), 66.67 pM (10 ng/mL), 22.22 pM (3.33 ng/mL), 7.41 pM (1.11 ng/mL), 2.47 pM (0.37 ng/mL), 0.82 pM (0.123 ng/mL) and 0.

Plates containing the Cetuximab anti-EGFR-FLAG antibody standards and variant anti-EGFR-FLAG samples were covered and incubated at RT for 1hr. The plate was then washed 3x with 250 $\mu$L/well of either pH 7.4 Buffer B or pH 6.0 Buffer C. 100 $\mu$L/well goat anti-FLAG-HRP detection antibody (Abcam, #ab 1238) at 500 ng/mL in either pH 7.4 Buffer B or pH 6.0 Buffer C was added to each well and the plate was covered and incubated for 1 hr at RT. The plate was then washed 3x with 250 $\mu$L/well of either pH 7.4 Buffer B or pH 6.0 Buffer C. Finally, 100 $\mu$L Sureblue TMB Microwell Peroxidase Substrate 1-component (KPL, #52-00-03) solution was added to each well and the plate was allowed to develop for 15-20 minutes at RT (away from light). The reaction was stopped by adding 100 $\mu$L TMB stop solution (KPL, #50-85-06) to each well and the plate was read within 30 min at $OD_{450}$ nM using a Microplate Spectrophotometer (Molecular Devices, Spectra Max M2).

**c. Antibody Binding Results**

The ELISA was performed in duplicate, and the average OD values of the duplicate reactions were calculated. Based on the OD value, variant anti-EGFR antibodies that exhibited higher binding activity to sEGFR-H6 at pH 6.0 (and 16.6 mM lactic acid) compared to at pH7.4 (and 1 mM lactic acid) were identified and are set forth in Table 15. The Table sets forth the average OD at pH 6.0 ($OD_{6.0}$), average OD at pH 7.4 ($OD_{7.4}$), and the ratio of the average OD values at pH 6.0 and 7.4 ($OD_{6.0}/OD_{7.4}$) for the variant antibodies at Dilution 1 and Dilution 2. Table 15 also sets forth the SEQ ID NOS of the variable region of the Heavy Chain (HC) and Light Chain (LC) of the variant anti-EGFR antibodies.

| Table 15. Variant anti-EGFR antibodies | | | | | | | | |
|---|---|---|---|---|---|---|---|---|
| | | Average OD (pH 6.0) ($OD_{6.0}$) | | Average OD (pH 7.4) ($OD_{7.4}$) | | $OD_{6.0}$ / $OD_{7.4}$ | | SEQ ID NO | |
| Chain | Mutation | Dilution 1 | Dilution 2 | Dilution 1 | Dilution 2 | Dilution 1 | Dilution 2 | HC | LC |
| HC | T23K | 2.6495 | 1.048 | 2.125 | 0.619 | 1.25 | 1.695 | 30 | 9 |
| HC | T23H | 2.744 | 1.5525 | 2.3405 | 0.833 | 1.173 | 1.851 | 31 | 9 |
| HC | T23R | 2.5055 | 1.2625 | 2.061 | 0.6245 | 1.216 | 2.03 | 32 | 9 |
| HC | T23A | 2.8735 | 1.142 | 2.5135 | 0.5 | 1.15 | 2.283 | 33 | 9 |
| HC | T23C | 2.654 | 1.3115 | 2.2505 | 0.687 | 1.179 | 1.909 | 34 | 9 |
| HC | T23E | 2.8785 | 1.3525 | 2.678 | 0.667 | 1.075 | 2.028 | 35 | 9 |
| HC | T23G | 1.679 | 0.3445 | 0.9585 | 0.1655 | 1.753 | 2.08 | 36 | 9 |
| HC | T23I | 2.709 | 1.4085 | 2.309 | 0.81 | 1.175 | 1.736 | 37 | 9 |
| HC | T23M | 2.3595 | 0.8185 | 1.772 | 0.504 | 1.332 | 1.636 | 38 | 9 |
| HC | T23N | 2.627 | 1.0915 | 1.823 | 0.6175 | 1.45 | 1.778 | 39 | 9 |
| HC | T23P | 0.252 | 0.1 | 0.1395 | 0.0965 | 1.812 | 1.035 | 40 | 9 |
| HC | T23S | 1.644 | 1.2745 | 1.9785 | 0.692 | 0.832 | 1.841 | 41 | 9 |
| HC | T23V | 0.258 | 0.1445 | 0.1775 | 0.106 | 1.454 | 1.365 | 42 | 9 |
| HC | T23W | 2.346 | 0.8765 | 1.8475 | 0.3025 | 1.274 | 2.896 | 43 | 9 |
| HC | T23L | 2.602 | 0.576 | 1.7855 | 0.2815 | 1.575 | 2.048 | 44 | 9 |
| HC | V24R | 0.091 | 0.085 | 0.079 | 0.071 | 1.158 | 1.194 | 45 | 9 |
| HC | V24A | 3.065 | 1.568 | 2.184 | 0.523 | 1.403 | 3.003 | 46 | 9 |
| HC | V24E | 0.780 | 0.232 | 0.300 | 0.114 | 2.596 | 2.044 | 1063 | 9 |
| HC | V24F | 2.386 | 0.645 | 1.156 | 0.336 | 2.057 | 2.937 | 47 | 9 |
| HC | V24G | 3.144 | 1.932 | 2.687 | 0.716 | 1.170 | 2.701 | 48 | 9 |
| HC | V24I | 1.669 | 0.485 | 0.590 | 0.176 | 2.837 | 2.761 | 49 | 9 |
| HC | V24M | 2.765 | 0.957 | 1.311 | 0.350 | 2.110 | 2.738 | 50 | 9 |
| HC | V24P | 1.512 | 0.388 | 0.511 | 0.165 | 2.961 | 2.355 | 51 | 9 |
| HC | V24S | 3.093 | 1.588 | 2.109 | 0.533 | 1.467 | 2.979 | 52 | 9 |
| HC | V24T | 2.605 | 0.821 | 1.091 | 0.276 | 2.389 | 2.983 | 53 | 9 |
| HC | V24L | 1.678 | 0.538 | 0.431 | 0.146 | 3.889 | 3.695 | 54 | 9 |
| HC | S25H | 3.006 | 1.752 | 1.255 | 0.311 | 2.456 | 5.667 | 55 | 9 |
| HC | S25R | 3.104 | 1.367 | 1.807 | 0.388 | 1.721 | 3.484 | 56 | 9 |
| HC | S25A | 3.206 | 2.225 | 2.164 | 0.563 | 1.481 | 3.957 | 57 | 9 |
| HC | S25C | 2.947 | 1.369 | 1.858 | 0.431 | 1.586 | 3.184 | 58 | 9 |
| HC | S25D | 3.076 | 1.717 | 2.194 | 0.578 | 1.487 | 3.073 | 59 | 9 |
| HC | S25E | 3.099 | 1.210 | 2.658 | 0.663 | 1.166 | 1.827 | 60 | 9 |
| HC | S25F | 3.135 | 1.758 | 2.822 | 0.787 | 1.111 | 2.234 | 61 | 9 |
| HC | S25G | 2.937 | 1.218 | 1.142 | 0.317 | 2.579 | 3.845 | 62 | 9 |
| HC | S25I | 3.042 | 2.171 | 1.994 | 0.494 | 1.525 | 4.394 | 63 | 9 |

(continued)

| Chain | Mutation | Average OD (pH 6.0) (OD$_{6.0}$) | | Average OD (pH 7.4 (OD$_{7.4}$) | | OD$_{6.0}$ / OD$_{7.4}$ | | SEQ ID NO | |
|---|---|---|---|---|---|---|---|---|---|
| | | Dilution 1 | Dilution 2 | Dilution 1 | Dilution 2 | Dilution 1 | Dilution 2 | HC | LC |
| HC | S25M | 3.158 | 2.444 | 2.774 | 0.759 | 1.138 | 3.230 | 64 | 9 |
| HC | S25P | 0.899 | 0.240 | 0.250 | 0.107 | 3.629 | 2.240 | 65 | 9 |
| HC | S25Q | 1.999 | 0.527 | 0.495 | 0.146 | 4.034 | 3.628 | 66 | 9 |
| HC | S25T | 2.795 | 0.510 | 1.483 | 0.162 | 1.886 | 1.567 | 67 | 9 |
| HC | S25V | 3.245 | 2.478 | 2.331 | 0.804 | 1.393 | 3.082 | 68 | 9 |
| HC | S25L | 3.155 | 1.773 | 1.631 | 0.441 | 1.935 | 4.040 | 69 | 9 |
| HC | G26H | 1.7955 | 0.545 | 1.1055 | 0.303 | 1.625 | 0.902 | 70 | 9 |
| HC | G26R | 1.9395 | 0.6055 | 1.444 | 0.338 | 1.342 | 1.793 | 71 | 9 |
| HC | G26D | 2.2105 | 0.7555 | 1.4155 | 0.4275 | 1.56 | 1.77 | 72 | 9 |
| HC | G26F | 0.588 | 0.2175 | 0.323 | 0.1345 | 1.822 | 1.628 | 73 | 9 |
| HC | G26M | 1.32 | 0.4535 | 0.841 | 0.2495 | 1.571 | 1.817 | 74 | 9 |
| HC | G26N | 2.9605 | 1.9525 | 2.99 | 1.2305 | 0.99 | 1.587 | 75 | 9 |
| HC | G26P | 1.001 | 0.4445 | 1.0425 | 0.309 | 0.977 | 1.441 | 76 | 9 |
| HC | G26Q | 2.45 | 0.8875 | 1.9265 | 0.5285 | 1.272 | 1.687 | 77 | 9 |
| HC | G26S | 2.226 | 0.7665 | 1.883 | 0.463 | 1.185 | 1.673 | 78 | 9 |
| HC | G26Y | 1.4695 | 0.447 | 0.8715 | 0.252 | 1.686 | 1.772 | 79 | 9 |
| HC | G26L | 1.015 | 0.312 | 0.64 | 0.2245 | 1.586 | 1.395 | 80 | 9 |
| HC | F27H | 1.488 | 0.342 | 0.817 | 0.243 | 1.823 | 1.418 | 81 | 9 |
| HC | F27R | 1.367 | 0.861 | 0.774 | 0.239 | 1.767 | 3.628 | 82 | 9 |
| HC | F27A | 2.936 | 2.213 | 2.241 | 0.769 | 1.310 | 2.880 | 83 | 9 |
| HC | F27D | 3.061 | 1.792 | 2.674 | 1.026 | 1.147 | 1.754 | 84 | 9 |
| HC | F27E | 2.792 | 1.306 | 2.418 | 0.910 | 1.155 | 1.435 | 85 | 9 |
| HC | F27G | 2.644 | 2.445 | 1.733 | 0.536 | 1.536 | 4.766 | 86 | 9 |
| HC | F27M | 2.935 | 1.233 | 1.980 | 0.405 | 1.483 | 3.047 | 87 | 9 |
| HC | F27P | 2.711 | 0.953 | 1.603 | 0.501 | 1.720 | 1.990 | 88 | 9 |
| HC | F27Q | 2.207 | 1.265 | 1.554 | 0.439 | 1.420 | 2.880 | 89 | 9 |
| HC | F27S | 1.898 | 0.508 | 0.918 | 0.253 | 2.067 | 2.014 | 90 | 9 |
| HC | F27T | 2.836 | 1.241 | 1.875 | 0.531 | 1.513 | 2.341 | 91 | 9 |
| HC | F27V | 1.419 | 0.712 | 0.614 | 0.190 | 2.311 | 3.752 | 92 | 9 |
| HC | F27W | 1.270 | 0.319 | 0.577 | 0.176 | 2.204 | 1.816 | 93 | 9 |
| HC | F27Y | 2.187 | 0.711 | 1.017 | 0.245 | 2.217 | 2.908 | 94 | 9 |
| HC | F27L | 2.492 | 0.784 | 1.562 | 0.478 | 1.595 | 1.639 | 95 | 9 |
| HC | S28K | 3.1285 | 2.125 | 2.927 | 1.176 | 1.069 | 1.804 | 96 | 9 |
| HC | S28H | 2.1735 | 0.7705 | 1.4715 | 0.4045 | 1.481 | 1.918 | 97 | 9 |
| HC | S28R | 2.9975 | 1.3625 | 2.5995 | 0.8495 | 1.153 | 1.604 | 98 | 9 |

(continued)

| Table 15. Variant anti-EGFR antibodies | | | | | | | | | |
|---|---|---|---|---|---|---|---|---|---|
| | | Average OD (pH 6.0) $(OD_{6.0})$ | | Average OD (pH 7.4 $(OD_{7.4})$ | | $OD_{6.0}$ / $OD_{7.4}$ | | SEQ ID NO | |
| Chain | Mutation | Dilution 1 | Dilution 2 | Dilution 1 | Dilution 2 | Dilution 1 | Dilution 2 | HC | LC |
| HC | S28A | 2.148 | 0.8335 | 1.468 | 0.3875 | 1.464 | 2.158 | 99 | 9 |
| HC | S28D | 1.97 | 0.7175 | 1.1875 | 0.3805 | 1.663 | 1.89 | 100 | 9 |
| HC | S28I | 2.8715 | 1.3185 | 2.2545 | 0.6505 | 1.273 | 2.022 | 101 | 9 |
| HC | S28M | 2.635 | 0.984 | 1.911 | 0.574 | 1.38 | 1.718 | 102 | 9 |
| HC | S28P | 2.6535 | 1.132 | 1.94 | 0.606 | 1.371 | 1.868 | 103 | 9 |
| HC | S28Q | 2.98 | 1.4105 | 2.4315 | 0.775 | 1.229 | 1.823 | 104 | 9 |
| HC | S28V | 3.1155 | 1.6905 | 2.79 | 1.0175 | 1.12 | 1.675 | 105 | 9 |
| HC | S28W | 3.1335 | 1.685 | 2.628 | 0.909 | 1.193 | 1.855 | 106 | 9 |
| HC | S28L | 2.4775 | 1.9575 | 1.863 | 0.563 | 1.331 | 3.481 | 107 | 9 |
| HC | L29K | 1.476 | 0.837 | 0.747 | 0.371 | 1.976 | 2.418 | 108 | 9 |
| HC | L29H | 1.329 | 0.717 | 0.661 | 0.264 | 2.020 | 2.714 | 109 | 9 |
| HC | L29A | 1.626 | 0.643 | 1.109 | 0.344 | 1.473 | 2.080 | 110 | 9 |
| HC | L29D | 0.504 | 0.232 | 0.329 | 0.164 | 1.531 | 1.409 | 111 | 9 |
| HC | L29G | 0.728 | 0.198 | 0.464 | 0.163 | 1.567 | 1.224 | 112 | 9 |
| HC | L29I | 2.250 | 1.661 | 2.020 | 0.893 | 1.121 | 1.864 | 113 | 9 |
| HC | L29M | 2.220 | 1.031 | 1.836 | 0.637 | 1.214 | 1.619 | 114 | 9 |
| HC | L29N | 0.352 | 0.326 | 0.253 | 0.149 | 1.390 | 1.254 | 115 | 9 |
| HC | L29S | 0.916 | 0.414 | 0.470 | 0.206 | 1.952 | 2.038 | 116 | 9 |
| HC | L29V | 0.975 | 0.516 | 0.543 | 0.287 | 1.796 | 1.800 | 117 | 9 |
| HC | T30H | 1.483 | 0.576 | 1.123 | 0.290 | 1.326 | 1.999 | 118 | 9 |
| HC | T30R | 1.646 | 0.808 | 1.487 | 0.412 | 1.110 | 1.961 | 119 | 9 |
| HC | T30D | 1.445 | 0.582 | 1.043 | 0.295 | 1.387 | 1.974 | 120 | 9 |
| HC | T30G | 1.130 | 0.455 | 0.925 | 0.257 | 1.222 | 1.776 | 121 | 9 |
| HC | T30I | 1.407 | 0.801 | 1.108 | 0.308 | 1.280 | 1.433 | 122 | 9 |
| HC | T30M | 1.241 | 0.454 | 1.054 | 0.221 | 1.191 | 2.061 | 123 | 9 |
| HC | T30N | 1.471 | 0.530 | 1.126 | 0.270 | 1.306 | 1.956 | 124 | 9 |
| HC | T30P | 1.341 | 0.405 | 0.936 | 0.263 | 1.432 | 1.544 | 125 | 9 |
| HC | T30S | 1.225 | 0.510 | 1.080 | 0.287 | 1.134 | 1.785 | 126 | 9 |
| HC | T30V | 1.210 | 0.521 | 1.130 | 0.246 | 1.074 | 2.113 | 127 | 9 |
| HC | T30W | 1.393 | 0.528 | 0.960 | 0.242 | 1.451 | 2.183 | 128 | 9 |
| HC | T30Y | 1.121 | 0.534 | 0.941 | 0.369 | 1.193 | 1.432 | 129 | 9 |
| HC | N31K | 3.216 | 2.270 | 2.256 | 0.713 | 1.433 | 3.242 | 130 | 9 |
| HC | N31H | 3.153 | 2.116 | 1.952 | 0.544 | 1.656 | 3.922 | 131 | 9 |
| HC | N31D | 2.946 | 1.227 | 1.746 | 0.424 | 1.687 | 2.891 | 132 | 9 |
| HC | N31E | 3.210 | 2.909 | 2.668 | 1.594 | 1.233 | 1.914 | 133 | 9 |

(continued)

| Table 15. Variant anti-EGFR antibodies | | | | | | | | | |
|---|---|---|---|---|---|---|---|---|---|
| | | Average OD (pH 6.0) $(OD_{6.0})$ | | Average OD (pH 7.4 $(OD_{7.4})$ | | $OD_{6.0}$ / $OD_{7.4}$ | | SEQ ID NO | |
| Chain | Mutation | Dilution 1 | Dilution 2 | Dilution 1 | Dilution 2 | Dilution 1 | Dilution 2 | HC | LC |
| HC | N31G | 3.218 | 1.917 | 2.566 | 0.760 | 1.254 | 2.529 | 134 | 9 |
| HC | N31I | 2.651 | 0.860 | 0.921 | 0.241 | 2.881 | 3.567 | 135 | 9 |
| HC | N31T | 3.102 | 0.773 | 2.226 | 0.567 | 1.394 | 1.364 | 136 | 9 |
| HC | N31V | 2.724 | 1.003 | 1.105 | 0.137 | 2.466 | 3.747 | 137 | 9 |
| HC | N31L | 2.920 | 0.983 | 1.990 | 0.575 | 1.467 | 1.713 | 138 | 9 |
| HC | Y32H | 1.011 | 0.488 | 0.684 | 0.248 | 1.483 | 1.963 | 139 | 9 |
| HC | Y32R | 1.253 | 0.454 | 1.049 | 0.280 | 1.194 | 1.616 | 140 | 9 |
| HC | Y32C | 0.667 | 0.256 | 0.405 | 0.182 | 1.645 | 1.408 | 141 | 9 |
| HC | Y32M | 1.035 | 0.368 | 0.756 | 0.237 | 1.366 | 1.556 | 142 | 9 |
| HC | Y32N | 0.837 | 0.447 | 0.524 | 0.121 | 1.604 | 1.707 | 143 | 9 |
| HC | Y32T | 0.705 | 0.296 | 0.435 | 0.176 | 1.624 | 1.685 | 144 | 9 |
| HC | Y32V | 0.767 | 0.216 | 0.518 | 0.223 | 1.484 | 0.967 | 145 | 9 |
| HC | Y32L | 0.793 | 0.299 | 0.550 | 0.169 | 1.443 | 1.787 | 146 | 9 |
| HC | G33E | 3.048 | 1.162 | 2.323 | 0.474 | 1.349 | 2.617 | 147 | 9 |
| HC | G33M | 2.472 | 0.669 | 1.904 | 0.537 | 1.305 | 1.246 | 148 | 9 |
| HC | G33S | 3.245 | 2.463 | 3.160 | 1.936 | 1.027 | 1.303 | 149 | 9 |
| HC | G33T | 2.346 | 0.748 | 1.959 | 0.714 | 1.226 | 1.038 | 150 | 9 |
| HC | G33Y | 0.121 | 0.106 | 0.123 | 0.097 | 0.982 | 1.095 | 151 | 9 |
| HC | V34A | 0.566 | 0.197 | 0.280 | 0.102 | 2.024 | 1.928 | 152 | 9 |
| HC | V34C | 0.756 | 0.432 | 0.798 | 0.164 | 0.950 | 2.625 | 153 | 9 |
| HC | V34I | 1.803 | 0.772 | 1.352 | 0.391 | 1.334 | 1.971 | 154 | 9 |
| HC | V34M | 1.219 | 0.681 | 0.925 | 0.331 | 1.320 | 2.069 | 155 | 9 |
| HC | V34P | 0.064 | 0.058 | 0.060 | 0.026 | 1.074 | 1.116 | 156 | 9 |
| HC | V34L | 1.105 | 0.429 | 0.772 | 0.206 | 1.434 | 2.118 | 157 | 9 |
| HC | H35I | 0.069 | 0.457 | 0.055 | 0.056 | 1.260 | 1.024 | 158 | 9 |
| HC | H35Q | 0.895 | 0.219 | 0.450 | 0.155 | 1.996 | 1.409 | 159 | 9 |
| HC | W36K | 0.062 | 0.056 | 0.056 | 0.028 | 1.111 | 1.002 | 160 | 9 |
| HC | W36A | 0.532 | 0.150 | 0.274 | 0.104 | 1.944 | 1.453 | 161 | 9 |
| HC | W36I | 1.421 | 0.791 | 1.241 | 0.495 | 1.148 | 1.600 | 162 | 9 |
| HC | W36V | 1.501 | 0.790 | 1.364 | 0.480 | 1.099 | 1.647 | 163 | 9 |
| HC | W36Y | 1.189 | 0.456 | 0.887 | 0.277 | 1.340 | 1.648 | 164 | 9 |
| HC | V50K | 0.105 | 0.118 | 0.101 | 0.101 | 1.040 | 1.170 | 165 | 9 |
| HC | V50H | 2.570 | 0.974 | 2.352 | 0.727 | 1.095 | 1.340 | 166 | 9 |
| HC | V50A | 3.196 | 1.613 | 2.597 | 1.019 | 1.233 | 1.582 | 167 | 9 |
| HC | V50D | 0.626 | 0.212 | 0.406 | 0.149 | 1.543 | 1.434 | 168 | 9 |

(continued)

| Table 15. Variant anti-EGFR antibodies | | | | | | | | | |
|---|---|---|---|---|---|---|---|---|---|
| | | Average OD (pH 6.0) $(OD_{6.0})$ | | Average OD (pH 7.4 $(OD_{7.4})$ | | $OD_{6.0}$ / $OD_{7.4}$ | | SEQ ID NO | |
| Chain | Mutation | Dilution 1 | Dilution 2 | Dilution 1 | Dilution 2 | Dilution 1 | Dilution 2 | HC | LC |
| HC | V50E | 0.400 | 0.146 | 0.339 | 0.134 | 1.181 | 1.086 | 169 | 9 |
| HC | V50G | 2.847 | 1.118 | 2.232 | 0.841 | 1.277 | 1.333 | 170 | 9 |
| HC | V50I | 1.551 | 0.414 | 0.555 | 0.182 | 2.795 | 2.298 | 171 | 9 |
| HC | V50N | 1.816 | 0.522 | 0.804 | 0.239 | 2.268 | 2.188 | 172 | 9 |
| HC | V50Q | 2.843 | 1.043 | 1.913 | 0.503 | 1.487 | 2.079 | 173 | 9 |
| HC | V50T | 3.264 | 2.695 | 3.246 | 2.339 | 1.005 | 1.153 | 174 | 9 |
| HC | V50L | 0.695 | 0.232 | 0.298 | 0.064 | 2.387 | 1.833 | 175 | 9 |
| HC | I51K | 1.861 | 0.635 | 1.068 | 0.288 | 1.764 | 2.207 | 176 | 9 |
| HC | I51H | 2.446 | 1.912 | 1.183 | 0.304 | 2.070 | 2.334 | 177 | 9 |
| HC | I51A | 3.027 | 1.178 | 1.436 | 0.346 | 2.378 | 3.590 | 178 | 9 |
| HC | I51C | 2.501 | 0.848 | 1.306 | 0.307 | 1.916 | 2.774 | 179 | 9 |
| HC | I51E | 0.879 | 0.283 | 0.491 | 0.184 | 1.791 | 1.537 | 180 | 9 |
| HC | I51G | 1.017 | 0.313 | 0.347 | 0.143 | 2.925 | 2.186 | 181 | 9 |
| HC | I51N | 2.508 | 0.797 | 1.240 | 0.302 | 2.026 | 2.641 | 182 | 9 |
| HC | I51Q | 3.286 | 1.967 | 2.878 | 0.877 | 1.142 | 2.255 | 183 | 9 |
| HC | I51S | 3.087 | 1.406 | 2.276 | 0.582 | 1.357 | 2.418 | 184 | 9 |
| HC | I51V | 3.312 | 2.820 | 3.310 | 1.602 | 1.001 | 1.761 | 185 | 9 |
| HC | I51Y | 0.997 | 0.301 | 0.626 | 0.203 | 1.592 | 1.477 | 186 | 9 |
| HC | I51L | 3.286 | 2.289 | 3.038 | 0.951 | 1.082 | 2.408 | 187 | 9 |
| HC | W52I | 0.855 | 0.249 | 0.392 | 0.148 | 2.183 | 1.690 | 188 | 9 |
| HC | W52N | 2.980 | 1.888 | 2.290 | 0.917 | 1.307 | 2.061 | 189 | 9 |
| HC | W52Y | 2.989 | 2.413 | 2.187 | 0.883 | 1.369 | 2.092 | 190 | 9 |
| HC | S53H | 3.290 | 2.779 | 3.202 | 1.848 | 1.027 | 1.504 | 191 | 9 |
| HC | S53R | 1.585 | 0.458 | 1.356 | 0.346 | 1.372 | 1.658 | 192 | 9 |
| HC | S53A | 3.441 | 3.325 | 3.360 | 2.616 | 1.024 | 1.299 | 193 | 9 |
| HC | S53C | 3.202 | 1.915 | 3.321 | 1.734 | 0.964 | 1.069 | 194 | 9 |
| HC | S53G | 3.389 | 3.289 | 3.381 | 2.854 | 1.002 | 1.153 | 195 | 9 |
| HC | S53I | 3.311 | 2.974 | 3.261 | 2.174 | 1.016 | 1.370 | 196 | 9 |
| HC | S53M | 3.210 | 1.689 | 3.018 | 1.025 | 1.068 | 1.659 | 197 | 9 |
| HC | S53P | 3.229 | 2.414 | 3.160 | 1.676 | 1.022 | 1.444 | 198 | 9 |
| HC | S53Q | 2.856 | 1.126 | 1.921 | 0.400 | 1.624 | 3.485 | 199 | 9 |
| HC | S53L | 3.298 | 2.391 | 3.295 | 1.757 | 1.001 | 1.472 | 200 | 9 |
| HC | S53T | 3.272 | 2.617 | 3.473 | 1.037 | 0.948 | 2.643 | 201 | 9 |
| HC | S53V | 3.315 | 2.305 | 3.321 | 1.652 | 0.998 | 1.406 | 202 | 9 |
| HC | S53Y | 3.377 | 2.797 | 3.235 | 1.999 | 1.044 | 1.398 | 203 | 9 |

(continued)

| Table 15. Variant anti-EGFR antibodies | | | | | | | | | |
|---|---|---|---|---|---|---|---|---|---|
| | | Average OD (pH 6.0) ($OD_{6.0}$) | | Average OD (pH 7.4 ($OD_{7.4}$) | | $OD_{6.0}$ / $OD_{7.4}$ | | SEQ ID NO | |
| Chain | Mutation | Dilution 1 | Dilution 2 | Dilution 1 | Dilution 2 | Dilution 1 | Dilution 2 | HC | LC |
| HC | G54H | 2.800 | 1.241 | 2.238 | 0.855 | 1.251 | 1.454 | 204 | 9 |
| HC | G54R | 2.341 | 0.748 | 1.702 | 0.518 | 1.376 | 1.446 | 205 | 9 |
| HC | G54A | 3.253 | 1.980 | 2.792 | 1.083 | 1.172 | 2.214 | 206 | 9 |
| HC | G54C | 1.636 | 0.346 | 1.055 | 0.238 | 1.551 | 1.452 | 207 | 9 |
| HC | G54D | 2.758 | 1.191 | 1.987 | 0.553 | 1.390 | 2.156 | 208 | 9 |
| HC | G54P | 2.336 | 0.773 | 1.320 | 0.370 | 1.772 | 2.089 | 209 | 9 |
| HC | G54S | 0.769 | 0.217 | 0.389 | 0.136 | 2.004 | 1.609 | 210 | 9 |
| HC | G55H | 3.289 | 1.916 | 2.919 | 0.957 | 1.132 | 2.085 | 211 | 9 |
| HC | G55R | 3.195 | 2.738 | 3.099 | 1.332 | 1.031 | 1.355 | 212 | 9 |
| HC | G55M | 3.076 | 1.452 | 2.727 | 0.766 | 1.131 | 1.889 | 213 | 9 |
| HC | G55S | 3.007 | 1.282 | 2.530 | 0.579 | 1.189 | 2.225 | 214 | 9 |
| HC | G55Y | 1.350 | 0.339 | 0.707 | 0.204 | 1.923 | 1.666 | 215 | 9 |
| HC | N56K | 2.941 | 1.283 | 2.775 | 1.030 | 1.059 | 1.246 | 216 | 9 |
| HC | N56A | 3.111 | 1.131 | 1.799 | 0.374 | 1.730 | 3.022 | 217 | 9 |
| HC | N56P | 1.322 | 1.332 | 0.880 | 0.235 | 1.525 | 1.408 | 218 | 9 |
| HC | N56S | 3.288 | 1.415 | 2.511 | 0.693 | 1.311 | 2.044 | 219 | 9 |
| HC | N56V | 3.021 | 1.201 | 2.660 | 0.867 | 1.136 | 1.385 | 220 | 9 |
| HC | N56G | 2.992 | 0.991 | 1.578 | 0.390 | 1.897 | 2.545 | 221 | 9 |
| HC | T57H | 3.064 | 1.040 | 1.792 | 0.457 | 1.711 | 2.276 | 222 | 9 |
| HC | T57R | 3.367 | 2.070 | 3.090 | 1.247 | 1.090 | 1.661 | 223 | 9 |
| HC | T57L | 3.316 | 1.923 | 2.903 | 1.052 | 1.143 | 1.827 | 224 | 9 |
| HC | T57A | 3.376 | 2.238 | 2.975 | 1.110 | 1.135 | 2.020 | 225 | 9 |
| HC | T57C | 3.287 | 1.693 | 2.703 | 0.814 | 1.216 | 2.088 | 226 | 9 |
| HC | T57D | 1.860 | 0.440 | 0.804 | 0.203 | 2.318 | 2.167 | 227 | 9 |
| HC | T57F | 3.414 | 2.680 | 3.125 | 1.839 | 1.093 | 1.458 | 228 | 9 |
| HC | T57M | 3.349 | 1.930 | 2.975 | 0.531 | 1.127 | 1.840 | 229 | 9 |
| HC | T57N | 3.125 | 1.170 | 2.145 | 0.537 | 1.459 | 2.182 | 230 | 9 |
| HC | T57Q | 3.359 | 1.699 | 2.774 | 0.792 | 1.211 | 2.147 | 231 | 9 |
| HC | T57W | 3.311 | 1.776 | 2.772 | 0.725 | 1.195 | 2.452 | 232 | 9 |
| HC | T57Y | 3.456 | 2.210 | 3.124 | 1.459 | 1.106 | 1.515 | 233 | 9 |
| HC | D58L | 1.607 | 0.742 | 2.044 | 0.579 | 0.786 | 1.314 | 234 | 9 |
| HC | D58G | 3.291 | 1.793 | 2.723 | 0.965 | 1.209 | 1.862 | 235 | 9 |
| HC | D58M | 2.134 | 0.790 | 1.507 | 0.545 | 1.451 | 1.449 | 236 | 9 |
| HC | D58N | 3.266 | 2.134 | 2.887 | 1.412 | 1.132 | 1.325 | 237 | 9 |
| HC | D58Q | 1.683 | 0.481 | 0.844 | 0.256 | 2.005 | 1.878 | 238 | 9 |

(continued)

| Table 15. Variant anti-EGFR antibodies | | | | | | | | | |
|---|---|---|---|---|---|---|---|---|---|
| | | Average OD (pH 6.0) ($OD_{6.0}$) | | Average OD (pH 7.4 ($OD_{7.4}$) | | $OD_{6.0}$ / $OD_{7.4}$ | | SEQ ID NO | |
| Chain | Mutation | Dilution 1 | Dilution 2 | Dilution 1 | Dilution 2 | Dilution 1 | Dilution 2 | HC | LC |
| HC | Y59H | 1.692 | 0.571 | 1.066 | 0.251 | 1.610 | 2.246 | 239 | 9 |
| HC | Y59R | 2.971 | 1.756 | 2.709 | 0.914 | 1.097 | 2.003 | 240 | 9 |
| HC | Y59A | 1.621 | 0.399 | 0.699 | 0.186 | 2.832 | 2.149 | 241 | 9 |
| HC | Y59C | 2.628 | 0.883 | 1.790 | 0.421 | 1.579 | 2.078 | 242 | 9 |
| HC | Y59D | 1.032 | 0.272 | 0.353 | 0.145 | 2.967 | 1.863 | 243 | 9 |
| HC | Y59E | 2.457 | 0.801 | 1.227 | 0.164 | 2.016 | 2.581 | 244 | 9 |
| HC | Y59G | 2.663 | 1.600 | 2.376 | 0.842 | 1.116 | 1.900 | 245 | 9 |
| HC | Y59I | 2.962 | 1.866 | 2.199 | 0.996 | 1.483 | 1.922 | 246 | 9 |
| HC | Y59P | 0.575 | 0.187 | 0.183 | 0.132 | 3.219 | 1.417 | 247 | 9 |
| HC | Y59Q | 2.915 | 1.383 | 2.283 | 0.557 | 1.277 | 2.480 | 248 | 9 |
| HC | Y59S | 2.891 | 1.523 | 2.571 | 0.732 | 1.128 | 2.070 | 249 | 9 |
| HC | Y59T | 3.059 | 1.678 | 2.585 | 0.702 | 1.184 | 2.510 | 250 | 9 |
| HC | Y59V | 2.561 | 0.945 | 1.685 | 0.417 | 1.743 | 2.247 | 251 | 9 |
| HC | Y59W | 2.886 | 1.247 | 2.089 | 0.496 | 1.382 | 2.708 | 252 | 9 |
| HC | N60K | 3.012 | 1.697 | 2.313 | 0.893 | 1.306 | 1.902 | 253 | 9 |
| HC | N60A | 3.104 | 1.847 | 2.729 | 0.958 | 1.140 | 1.935 | 254 | 9 |
| HC | N60C | 2.070 | 0.596 | 1.170 | 0.299 | 1.824 | 1.999 | 255 | 9 |
| HC | N60D | 0.196 | 0.800 | 0.113 | 0.089 | 1.736 | 1.142 | 256 | 9 |
| HC | N60F | 2.386 | 0.935 | 1.355 | 0.398 | 2.039 | 2.370 | 257 | 9 |
| HC | N60G | 2.647 | 0.944 | 1.537 | 0.407 | 1.831 | 2.323 | 258 | 9 |
| HC | N60P | 1.097 | 0.342 | 0.419 | 0.171 | 2.634 | 2.003 | 259 | 9 |
| HC | N60Q | 1.676 | 0.484 | 0.889 | 0.262 | 1.946 | 1.854 | 260 | 9 |
| HC | N60S | 2.148 | 0.696 | 1.104 | 0.299 | 1.953 | 2.362 | 261 | 9 |
| HC | N60T | 2.755 | 1.083 | 1.910 | 0.520 | 1.490 | 2.093 | 262 | 9 |
| HC | N60Y | 2.844 | 1.291 | 2.407 | 0.676 | 1.197 | 1.921 | 263 | 9 |
| HC | T61N | 3.043 | 1.882 | 2.603 | 0.936 | 1.176 | 2.012 | 264 | 9 |
| HC | T61Q | 2.187 | 0.731 | 1.372 | 0.188 | 1.591 | 1.974 | 265 | 9 |
| HC | P62G | 2.593 | 1.009 | 1.765 | 0.508 | 1.469 | 1.985 | 266 | 9 |
| HC | F63H | 3.170 | 2.002 | 2.715 | 0.773 | 1.168 | 2.592 | 267 | 9 |
| HC | F63R | 2.377 | 0.681 | 0.957 | 0.259 | 2.485 | 2.636 | 268 | 9 |
| HC | F63L | 3.150 | 1.606 | 2.218 | 0.627 | 1.421 | 2.560 | 269 | 9 |
| HC | F63A | 2.387 | 0.746 | 1.016 | 0.263 | 2.349 | 2.841 | 270 | 9 |
| HC | F63C | 0.911 | 0.242 | 0.272 | 0.112 | 3.440 | 2.160 | 271 | 9 |
| HC | F63D | 2.984 | 1.277 | 1.839 | 0.456 | 1.629 | 2.806 | 272 | 9 |
| HC | F63G | 2.914 | 1.094 | 1.516 | 0.401 | 1.951 | 2.767 | 273 | 9 |

(continued)

| Table 15. Variant anti-EGFR antibodies | | | | | | | | | |
|---|---|---|---|---|---|---|---|---|---|
| | | Average OD (pH 6.0) ($OD_{6.0}$) | | Average OD (pH 7.4 ($OD_{7.4}$) | | $OD_{6.0}$ / $OD_{7.4}$ | | SEQ ID NO | |
| Chain | Mutation | Dilution 1 | Dilution 2 | Dilution 1 | Dilution 2 | Dilution 1 | Dilution 2 | HC | LC |
| HC | F63M | 3.073 | 1.526 | 2.122 | 0.449 | 1.448 | 3.401 | 274 | 9 |
| HC | F63N | 2.284 | 0.672 | 1.240 | 0.156 | 1.843 | 2.201 | 275 | 9 |
| HC | F63Q | 2.906 | 1.180 | 1.622 | 0.373 | 1.794 | 3.164 | 276 | 9 |
| HC | F63S | 2.894 | 1.014 | 1.511 | 0.162 | 1.917 | 6.301 | 277 | 9 |
| HC | F63V | 3.032 | 1.585 | 2.090 | 0.477 | 1.451 | 3.338 | 278 | 9 |
| HC | T64R | 3.052 | 1.908 | 2.925 | 0.933 | 1.044 | 2.051 | 279 | 9 |
| HC | T64L | 3.052 | 2.189 | 2.814 | 1.108 | 1.093 | 1.976 | 280 | 9 |
| HC | T64C | 2.770 | 1.082 | 2.220 | 0.589 | 1.250 | 1.839 | 281 | 9 |
| HC | T64F | 0.165 | 0.087 | 0.084 | 0.089 | 1.974 | 0.985 | 282 | 9 |
| HC | T64G | 3.088 | 1.925 | 3.011 | 0.955 | 1.026 | 2.018 | 283 | 9 |
| HC | T64N | 0.232 | 0.132 | 0.092 | 0.087 | 2.550 | 1.516 | 284 | 9 |
| HC | T64Q | 1.555 | 0.542 | 0.952 | 0.253 | 1.641 | 2.150 | 285 | 9 |
| HC | T64V | 2.784 | 1.255 | 2.046 | 0.261 | 1.362 | 2.224 | 286 | 9 |
| HC | S65H | 3.222 | 2.639 | 3.201 | 1.556 | 1.007 | 1.704 | 287 | 9 |
| HC | S65R | 3.199 | 2.297 | 3.080 | 1.033 | 1.041 | 2.226 | 288 | 9 |
| HC | S65L | 3.302 | 2.824 | 3.272 | 1.846 | 1.009 | 1.530 | 289 | 9 |
| HC | S65C | 3.233 | 2.804 | 2.969 | 1.317 | 1.090 | 1.761 | 290 | 9 |
| HC | S65E | 3.256 | 2.320 | 3.089 | 1.304 | 1.054 | 1.779 | 291 | 9 |
| HC | S65F | 3.231 | 2.362 | 3.025 | 1.420 | 1.068 | 1.664 | 292 | 9 |
| HC | S65G | 3.337 | 2.992 | 3.335 | 2.388 | 1.000 | 1.253 | 293 | 9 |
| HC | S65I | 3.220 | 2.108 | 2.996 | 1.180 | 1.075 | 1.788 | 294 | 9 |
| HC | S65M | 3.102 | 1.898 | 2.758 | 0.940 | 1.125 | 2.018 | 295 | 9 |
| HC | S65N | 3.224 | 2.277 | 2.919 | 1.060 | 1.106 | 2.151 | 296 | 9 |
| HC | S65P | 2.795 | 1.197 | 1.892 | 0.466 | 1.479 | 2.568 | 297 | 9 |
| HC | S65Q | 3.193 | 2.250 | 2.951 | 1.100 | 1.082 | 2.055 | 298 | 9 |
| HC | S65T | 3.191 | 1.802 | 2.779 | 0.915 | 1.149 | 1.972 | 299 | 9 |
| HC | S65W | 3.227 | 2.510 | 3.114 | 1.514 | 1.037 | 1.662 | 300 | 9 |
| HC | S65Y | 3.322 | 2.816 | 3.201 | 1.928 | 1.038 | 1.462 | 301 | 9 |
| HC | R66L | 3.149 | 1.674 | 2.785 | 0.636 | 1.131 | 2.636 | 302 | 9 |
| HC | R66A | 2.441 | 1.026 | 2.008 | 0.491 | 1.217 | 2.091 | 303 | 9 |
| HC | R66C | 2.036 | 0.645 | 1.022 | 0.281 | 1.992 | 2.298 | 304 | 9 |
| HC | R66E | 1.775 | 0.595 | 1.089 | 0.316 | 1.627 | 1.889 | 305 | 9 |
| HC | R66F | 2.462 | 0.416 | 1.195 | 0.259 | 2.070 | 1.603 | 306 | 9 |
| HC | R66N | 3.065 | 1.089 | 2.343 | 0.658 | 1.308 | 1.655 | 307 | 9 |
| HC | R66P | 0.469 | 0.169 | 0.306 | 0.123 | 1.537 | 1.378 | 308 | 9 |

(continued)

| Table 15. Variant anti-EGFR antibodies | | | | | | | | | |
|---|---|---|---|---|---|---|---|---|---|
| | | Average OD (pH 6.0) ($OD_{6.0}$) | | Average OD (pH 7.4 ($OD_{7.4}$) | | $OD_{6.0}$ / $OD_{7.4}$ | | SEQ ID NO | |
| Chain | Mutation | Dilution 1 | Dilution 2 | Dilution 1 | Dilution 2 | Dilution 1 | Dilution 2 | HC | LC |
| HC | R66Q | 3.010 | 1.421 | 2.386 | 0.712 | 1.261 | 1.999 | 309 | 9 |
| HC | R66S | 2.805 | 0.994 | 1.945 | 0.414 | 1.444 | 2.404 | 310 | 9 |
| HC | R66T | 0.612 | 0.200 | 0.326 | 0.123 | 1.879 | 1.628 | 311 | 9 |
| HC | R66V | 3.198 | 1.703 | 3.077 | 0.525 | 1.039 | 1.530 | 312 | 9 |
| HC | R66G | 2.234 | 0.565 | 0.977 | 0.247 | 2.291 | 2.292 | 313 | 9 |
| HC | L67A | 2.784 | 1.152 | 1.921 | 0.487 | 1.449 | 2.377 | 314 | 9 |
| HC | L67C | 3.189 | 1.868 | 2.640 | 0.675 | 1.208 | 2.768 | 315 | 9 |
| HC | L67D | 0.113 | 0.086 | 0.085 | 0.079 | 1.343 | 1.078 | 316 | 9 |
| HC | L67E | 2.953 | 1.155 | 2.003 | 0.552 | 1.475 | 2.151 | 317 | 9 |
| HC | L67I | 2.974 | 1.183 | 1.920 | 0.461 | 1.548 | 2.579 | 318 | 9 |
| HC | L67M | 2.889 | 1.300 | 2.100 | 0.558 | 1.376 | 2.345 | 319 | 9 |
| HC | L67Q | 2.297 | 0.634 | 1.116 | 0.297 | 2.057 | 2.151 | 320 | 9 |
| HC | L67S | 3.114 | 1.560 | 2.496 | 0.646 | 1.248 | 2.418 | 321 | 9 |
| HC | L67T | 2.929 | 1.127 | 1.712 | 0.393 | 1.713 | 2.871 | 322 | 9 |
| HC | L67V | 2.755 | 0.875 | 1.330 | 0.346 | 2.072 | 2.529 | 323 | 9 |
| HC | L67Y | 3.171 | 1.933 | 2.840 | 0.454 | 1.117 | 2.152 | 324 | 9 |
| HC | S68K | 3.274 | 2.096 | 2.959 | 1.092 | 1.109 | 1.920 | 325 | 9 |
| HC | S68H | 3.269 | 2.602 | 3.284 | 1.358 | 0.995 | 1.918 | 326 | 9 |
| HC | S68R | 3.146 | 2.252 | 2.931 | 1.108 | 1.074 | 2.033 | 327 | 9 |
| HC | S68L | 3.054 | 1.591 | 2.441 | 0.645 | 1.251 | 2.471 | 328 | 9 |
| HC | S68C | 3.161 | 2.327 | 3.050 | 1.209 | 1.037 | 1.924 | 329 | 9 |
| HC | S68D | 3.228 | 1.835 | 2.822 | 0.413 | 1.144 | 2.303 | 330 | 9 |
| HC | S68E | 3.123 | 2.025 | 2.841 | 0.965 | 1.100 | 2.104 | 331 | 9 |
| HC | S68F | 0.256 | 0.128 | 0.137 | 0.093 | 1.863 | 1.379 | 332 | 9 |
| HC | S68G | 2.935 | 1.566 | 2.300 | 0.778 | 1.278 | 2.013 | 333 | 9 |
| HC | S68I | 3.209 | 1.895 | 2.834 | 0.788 | 1.132 | 2.404 | 334 | 9 |
| HC | S68N | 3.114 | 1.621 | 2.721 | 0.762 | 1.145 | 2.132 | 335 | 9 |
| HC | S68Q | 3.222 | 2.075 | 3.033 | 1.071 | 1.064 | 1.938 | 336 | 9 |
| HC | S68T | 3.310 | 2.716 | 3.261 | 1.779 | 1.015 | 1.532 | 337 | 9 |
| HC | S68V | 3.099 | 1.701 | 2.661 | 0.761 | 1.165 | 2.237 | 338 | 9 |
| HC | I69A | 0.429 | 0.133 | 0.242 | 0.086 | 1.773 | 1.542 | 339 | 9 |
| HC | I69C | 1.045 | 0.317 | 0.810 | 0.186 | 1.291 | 1.705 | 340 | 9 |
| HC | I69G | 0.112 | 0.133 | 0.085 | 0.062 | 1.312 | 1.147 | 341 | 9 |
| HC | I69Y | 0.523 | 0.157 | 0.340 | 0.132 | 1.538 | 1.194 | 342 | 9 |
| HC | N70H | 3.459 | 1.652 | 2.155 | 0.741 | 1.736 | 2.229 | 343 | 9 |

(continued)

| Table 15. Variant anti-EGFR antibodies | | | | | | | | |
|---|---|---|---|---|---|---|---|---|
| | | Average OD (pH 6.0) $(OD_{6.0})$ | | Average OD (pH 7.4 $(OD_{7.4})$ | | $OD_{6.0}$ / $OD_{7.4}$ | | SEQ ID NO | |
| Chain | Mutation | Dilution 1 | Dilution 2 | Dilution 1 | Dilution 2 | Dilution 1 | Dilution 2 | HC | LC |
| HC | N70R | 1.720 | 0.369 | 0.689 | 0.206 | 2.997 | 1.792 | 344 | 9 |
| HC | N70L | 3.184 | 1.401 | 2.232 | 0.608 | 1.429 | 2.305 | 345 | 9 |
| HC | N70D | 1.788 | 0.523 | 0.817 | 0.257 | 2.242 | 2.036 | 346 | 9 |
| HC | N70E | 3.223 | 1.695 | 2.394 | 0.721 | 1.373 | 2.350 | 347 | 9 |
| HC | N70F | 3.263 | 2.109 | 2.985 | 1.368 | 1.095 | 1.557 | 348 | 9 |
| HC | N70G | 2.992 | 1.363 | 2.359 | 0.675 | 1.268 | 2.021 | 349 | 9 |
| HC | N70I | 3.240 | 1.310 | 1.934 | 0.575 | 1.862 | 2.278 | 350 | 9 |
| HC | N70P | 0.192 | 0.445 | 0.375 | 0.235 | 0.502 | 2.019 | 351 | 9 |
| HC | N70Q | 3.194 | 1.500 | 2.347 | 0.854 | 1.364 | 1.765 | 352 | 9 |
| HC | N70S | 3.247 | 2.088 | 2.937 | 0.496 | 1.105 | 2.094 | 353 | 9 |
| HC | N70T | 3.207 | 1.679 | 2.488 | 0.747 | 1.289 | 2.248 | 354 | 9 |
| HC | N70V | 0.241 | 2.063 | 2.833 | 1.232 | 0.085 | 1.677 | 355 | 9 |
| HC | N70Y | 3.152 | 1.553 | 2.029 | 0.788 | 1.888 | 1.980 | 356 | 9 |
| HC | K71H | 3.096 | 1.235 | 2.366 | 0.657 | 1.309 | 1.883 | 357 | 9 |
| HC | K71R | 2.741 | 0.871 | 1.745 | 0.462 | 1.571 | 1.888 | 358 | 9 |
| HC | K71L | 3.205 | 1.828 | 2.883 | 1.290 | 1.112 | 1.422 | 359 | 9 |
| HC | K71A | 1.772 | 0.457 | 1.075 | 0.320 | 1.649 | 1.430 | 360 | 9 |
| HC | K71C | 3.353 | 1.977 | 2.687 | 1.093 | 1.248 | 1.891 | 361 | 9 |
| HC | K71F | 3.342 | 1.506 | 3.119 | 1.260 | 1.072 | 1.195 | 362 | 9 |
| HC | K71G | 2.921 | 0.979 | 2.094 | 0.536 | 1.402 | 1.827 | 363 | 9 |
| HC | K71Q | 3.049 | 1.267 | 2.617 | 1.082 | 1.165 | 1.179 | 364 | 9 |
| HC | K71S | 3.114 | 1.168 | 2.534 | 0.688 | 1.237 | 1.716 | 365 | 9 |
| HC | K71T | 2.533 | 0.830 | 1.688 | 0.299 | 1.500 | 1.544 | 366 | 9 |
| HC | K71V | 3.160 | 1.663 | 2.787 | 0.929 | 1.134 | 1.790 | 367 | 9 |
| HC | K71W | 3.294 | 1.708 | 3.017 | 1.261 | 1.092 | 1.356 | 368 | 9 |
| HC | K71Y | 3.334 | 2.035 | 2.898 | 1.410 | 1.150 | 1.443 | 369 | 9 |
| HC | D72K | 3.108 | 1.388 | 2.427 | 1.747 | 1.281 | 0.795 | 370 | 9 |
| HC | D72H | 3.203 | 1.653 | 2.744 | 0.711 | 1.179 | 2.325 | 371 | 9 |
| HC | D72R | 3.355 | 2.011 | 3.182 | 0.938 | 1.055 | 2.144 | 372 | 9 |
| HC | D72L | 3.252 | 2.402 | 1.511 | 0.561 | 2.153 | 4.308 | 373 | 9 |
| HC | D72A | 2.976 | 1.272 | 3.026 | 1.109 | 0.982 | 1.415 | 374 | 9 |
| HC | D72G | 2.694 | 0.972 | 1.583 | 0.429 | 1.711 | 2.272 | 375 | 9 |
| HC | D72I | 3.200 | 1.798 | 2.711 | 0.827 | 1.182 | 2.179 | 376 | 9 |
| HC | D72M | 3.144 | 1.529 | 2.747 | 0.621 | 1.149 | 2.470 | 377 | 9 |
| HC | D72N | 3.303 | 1.878 | 2.982 | 0.927 | 1.112 | 2.026 | 378 | 9 |

(continued)

| Table 15. Variant anti-EGFR antibodies | | | | | | | | | |
|---|---|---|---|---|---|---|---|---|---|
| | | Average OD (pH 6.0) ($OD_{6.0}$) | | Average OD (pH 7.4 ($OD_{7.4}$) | | $OD_{6.0}$ / $OD_{7.4}$ | | SEQ ID NO | |
| Chain | Mutation | Dilution 1 | Dilution 2 | Dilution 1 | Dilution 2 | Dilution 1 | Dilution 2 | HC | LC |
| HC | D72Q | 3.157 | 2.535 | 2.782 | 0.790 | 1.137 | 2.402 | 379 | 9 |
| HC | D72S | 3.166 | 1.894 | 3.042 | 0.931 | 1.041 | 2.037 | 380 | 9 |
| HC | D72V | 3.241 | 2.071 | 3.115 | 1.044 | 1.041 | 1.987 | 381 | 9 |
| HC | D72W | 3.182 | 1.722 | 1.248 | 0.368 | 2.551 | 4.678 | 382 | 9 |
| HC | D72Y | 3.172 | 1.646 | 2.513 | 0.711 | 1.269 | 2.319 | 383 | 9 |
| HC | N73H | 3.095 | 1.105 | 2.128 | 0.423 | 1.455 | 2.618 | 384 | 9 |
| HC | N73R | 2.908 | 1.026 | 1.738 | 0.387 | 1.672 | 2.650 | 385 | 9 |
| HC | N73L | 3.179 | 1.682 | 2.800 | 0.883 | 1.137 | 1.917 | 386 | 9 |
| HC | N73A | 2.307 | 0.773 | 1.016 | 0.300 | 2.229 | 2.589 | 387 | 9 |
| HC | N73C | 3.111 | 1.210 | 2.023 | 0.483 | 1.558 | 2.506 | 388 | 9 |
| HC | N73G | 2.985 | 1.059 | 1.910 | 0.512 | 1.584 | 2.072 | 389 | 9 |
| HC | N73I | 3.336 | 2.124 | 3.024 | 1.005 | 1.107 | 2.116 | 390 | 9 |
| HC | N73M | 3.226 | 1.307 | 1.902 | 0.511 | 1.782 | 2.558 | 391 | 9 |
| HC | N73P | 2.396 | 0.732 | 1.262 | 0.359 | 1.913 | 2.036 | 392 | 9 |
| HC | N73Q | 3.055 | 1.153 | 2.047 | 0.221 | 1.494 | 2.850 | 393 | 9 |
| HC | N73S | 2.962 | 1.097 | 1.959 | 0.485 | 1.541 | 2.265 | 394 | 9 |
| HC | N73T | 2.752 | 1.024 | 1.951 | 0.544 | 1.404 | 1.896 | 395 | 9 |
| HC | N73V | 2.522 | 0.733 | 1.382 | 0.358 | 1.827 | 2.046 | 396 | 9 |
| HC | N73W | 2.294 | 0.718 | 1.278 | 0.342 | 1.783 | 2.100 | 397 | 9 |
| HC | N73Y | 3.150 | 1.234 | 2.165 | 0.464 | 1.455 | 2.656 | 398 | 9 |
| HC | S74K | 2.981 | 1.013 | 1.883 | 0.413 | 1.601 | 2.457 | 399 | 9 |
| HC | S74H | 3.070 | 1.253 | 1.963 | 0.476 | 1.579 | 2.634 | 400 | 9 |
| HC | S74R | 3.062 | 1.331 | 2.222 | 0.511 | 1.387 | 2.604 | 401 | 9 |
| HC | S74L | 3.292 | 2.221 | 3.205 | 1.053 | 1.027 | 2.110 | 402 | 9 |
| HC | S74A | 2.809 | 0.996 | 1.874 | 0.436 | 1.501 | 2.288 | 403 | 9 |
| HC | S74C | 2.721 | 0.882 | 1.705 | 0.347 | 1.619 | 2.544 | 404 | 9 |
| HC | S74D | 2.946 | 1.353 | 1.967 | 0.467 | 1.500 | 2.897 | 405 | 9 |
| HC | S74E | 3.001 | 1.279 | 2.213 | 0.444 | 1.358 | 2.892 | 406 | 9 |
| HC | S74G | 2.857 | 2.244 | 1.714 | 0.429 | 1.762 | 2.895 | 407 | 9 |
| HC | S74I | 2.986 | 1.082 | 2.151 | 0.495 | 1.388 | 2.194 | 408 | 9 |
| HC | S74M | 3.068 | 1.146 | 2.144 | 0.455 | 1.458 | 2.517 | 409 | 9 |
| HC | S74P | 3.196 | 1.545 | 2.503 | 0.615 | 1.280 | 2.511 | 410 | 9 |
| HC | S74T | 3.201 | 1.466 | 2.578 | 0.612 | 1.246 | 2.395 | 411 | 9 |
| HC | S74V | 3.242 | 1.928 | 3.245 | 0.910 | 0.999 | 2.118 | 412 | 9 |
| HC | S74Y | 2.854 | 0.982 | 1.605 | 0.337 | 1.866 | 2.919 | 413 | 9 |

(continued)

| Table 15. Variant anti-EGFR antibodies | | | | | | | | | |
|---|---|---|---|---|---|---|---|---|---|
| | | Average OD (pH 6.0) $(OD_{6.0})$ | | Average OD (pH 7.4 $(OD_{7.4})$ | | $OD_{6.0}$ / $OD_{7.4}$ | | SEQ ID NO | |
| Chain | Mutation | Dilution 1 | Dilution 2 | Dilution 1 | Dilution 2 | Dilution 1 | Dilution 2 | HC | LC |
| HC | K75H | 3.278 | 1.961 | 2.863 | 0.371 | 1.146 | 2.638 | 414 | 9 |
| HC | K75R | 3.111 | 1.259 | 2.012 | 0.479 | 1.559 | 2.639 | 415 | 9 |
| HC | K75L | 3.216 | 1.226 | 2.331 | 0.710 | 1.390 | 1.725 | 416 | 9 |
| HC | K75A | 2.879 | 1.070 | 1.846 | 0.428 | 1.570 | 2.504 | 417 | 9 |
| HC | K75C | 3.008 | 1.064 | 1.550 | 0.359 | 1.948 | 2.967 | 418 | 9 |
| HC | K75E | 3.070 | 1.191 | 2.020 | 0.523 | 1.560 | 2.279 | 419 | 9 |
| HC | K75F | 3.068 | 1.189 | 1.735 | 0.388 | 1.770 | 3.064 | 420 | 9 |
| HC | K75M | 2.776 | 0.884 | 1.342 | 0.362 | 2.076 | 2.450 | 421 | 9 |
| HC | K75Q | 3.200 | 1.533 | 2.319 | 0.526 | 1.384 | 2.914 | 422 | 9 |
| HC | K75T | 2.633 | 0.807 | 1.408 | 0.349 | 1.870 | 2.311 | 423 | 9 |
| HC | K75V | 2.908 | 0.939 | 1.435 | 0.325 | 2.032 | 2.962 | 424 | 9 |
| HC | K75W | 2.656 | 0.797 | 1.098 | 0.280 | 2.422 | 2.850 | 425 | 9 |
| HC | K75Y | 2.993 | 1.195 | 1.770 | 0.397 | 1.693 | 3.015 | 426 | 9 |
| HC | S76H | 2.719 | 0.806 | 1.324 | 0.300 | 2.054 | 2.694 | 427 | 9 |
| HC | S76R | 2.877 | 1.042 | 1.473 | 0.328 | 1.953 | 3.171 | 428 | 9 |
| HC | S76L | 2.187 | 0.500 | 0.830 | 0.215 | 2.636 | 2.323 | 429 | 9 |
| HC | S76A | 2.598 | 0.982 | 1.652 | 0.580 | 1.608 | 1.693 | 430 | 9 |
| HC | S76C | 2.490 | 0.855 | 1.304 | 0.339 | 1.910 | 2.537 | 431 | 9 |
| HC | S76D | 2.429 | 1.711 | 1.130 | 0.257 | 2.196 | 2.827 | 432 | 9 |
| HC | S76E | 3.053 | 1.236 | 1.893 | 0.457 | 1.615 | 2.706 | 433 | 9 |
| HC | S76F | 3.013 | 1.143 | 1.958 | 0.443 | 1.540 | 2.582 | 434 | 9 |
| HC | S76M | 2.936 | 1.267 | 1.924 | 0.458 | 1.527 | 2.767 | 435 | 9 |
| HC | S76P | 2.566 | 0.824 | 1.186 | 0.291 | 2.172 | 2.835 | 436 | 9 |
| HC | S76Q | 2.670 | 0.843 | 1.578 | 0.420 | 1.697 | 2.009 | 437 | 9 |
| HC | S76T | 2.515 | 0.805 | 1.182 | 0.268 | 2.133 | 3.024 | 438 | 9 |
| HC | S76Y | 2.788 | 0.921 | 1.393 | 0.344 | 2.004 | 2.685 | 439 | 9 |
| HC | Q77H | 3.135 | 1.285 | 2.396 | 0.640 | 1.310 | 2.008 | 440 | 9 |
| HC | Q77R | 2.600 | 1.185 | 1.976 | 0.618 | 1.344 | 1.957 | 441 | 9 |
| HC | Q77L | 2.256 | 0.589 | 0.937 | 0.234 | 2.408 | 2.520 | 442 | 9 |
| HC | Q77A | 3.109 | 1.370 | 2.320 | 0.532 | 1.343 | 2.577 | 443 | 9 |
| HC | Q77E | 3.162 | 1.660 | 2.729 | 0.331 | 1.159 | 2.647 | 444 | 9 |
| HC | Q77G | 2.148 | 0.548 | 0.843 | 0.216 | 2.551 | 2.545 | 445 | 9 |
| HC | Q77I | 2.653 | 0.784 | 1.189 | 0.292 | 2.232 | 2.690 | 446 | 9 |
| HC | Q77M | 2.489 | 0.861 | 1.213 | 0.289 | 2.108 | 2.989 | 447 | 9 |
| HC | Q77N | 3.002 | 1.184 | 1.800 | 0.471 | 1.668 | 2.516 | 448 | 9 |

(continued)

| Table 15. Variant anti-EGFR antibodies | | | | | | | | |
|---|---|---|---|---|---|---|---|---|
| | | Average OD (pH 6.0) $(OD_{6.0})$ | | Average OD (pH 7.4 $(OD_{7.4})$ | | $OD_{6.0} / OD_{7.4}$ | | SEQ ID NO | |
| Chain | Mutation | Dilution 1 | Dilution 2 | Dilution 1 | Dilution 2 | Dilution 1 | Dilution 2 | HC | LC |
| HC | Q77S | 2.791 | 1.085 | 1.936 | 0.496 | 1.441 | 2.193 | 449 | 9 |
| HC | Q77V | 3.246 | 1.643 | 2.722 | 0.633 | 1.193 | 2.597 | 450 | 9 |
| HC | Q77W | 1.891 | 0.537 | 0.880 | 0.243 | 2.149 | 2.209 | 451 | 9 |
| HC | Q77Y | 2.328 | 0.650 | 1.248 | 0.285 | 1.880 | 2.291 | 452 | 9 |
| HC | Y93H | 0.386 | 0.134 | 0.204 | 0.088 | 1.883 | 1.512 | 453 | 9 |
| HC | Y93V | 0.570 | 0.193 | 0.327 | 0.117 | 1.739 | 1.652 | 454 | 9 |
| HC | Y93W | 0.167 | 0.081 | 0.095 | 0.072 | 1.743 | 1.126 | 455 | 9 |
| HC | Y94R | 0.611 | 0.510 | 0.600 | 0.264 | 1.034 | 1.935 | 456 | 9 |
| HC | Y94L | 0.484 | 0.210 | 0.256 | 0.121 | 1.888 | 1.738 | 457 | 9 |
| HC | R97H | 1.065 | 0.411 | 0.502 | 0.219 | 2.148 | 1.884 | 458 | 9 |
| HC | R97W | 0.065 | 0.062 | 0.075 | 0.032 | 0.859 | 0.930 | 459 | 9 |
| HC | A98P | 1.057 | 0.812 | 0.619 | 0.386 | 1.709 | 1.755 | 460 | 9 |
| HC | L99N | 1.202 | 0.662 | 0.655 | 0.401 | 1.836 | 1.652 | 461 | 9 |
| HC | L99W | 1.312 | 1.114 | 0.926 | 0.350 | 1.417 | 1.659 | 462 | 9 |
| HC | T100H | 3.152 | 2.147 | 3.128 | 1.981 | 1.008 | 1.084 | 463 | 9 |
| HC | T100L | 3.133 | 1.851 | 2.685 | 1.361 | 1.167 | 1.364 | 464 | 9 |
| HC | T100A | 3.201 | 2.377 | 2.996 | 1.752 | 1.068 | 1.356 | 465 | 9 |
| HC | T100D | 2.957 | 0.907 | 2.741 | 0.868 | 1.079 | 1.046 | 466 | 9 |
| HC | T100I | 2.910 | 1.690 | 2.199 | 1.376 | 1.448 | 1.229 | 467 | 9 |
| HC | T100N | 3.070 | 1.883 | 2.895 | 1.350 | 1.060 | 1.398 | 468 | 9 |
| HC | T100P | 0.819 | 0.253 | 0.262 | 0.119 | 3.141 | 2.119 | 469 | 9 |
| HC | T100Q | 3.167 | 1.966 | 3.093 | 1.685 | 1.025 | 1.168 | 470 | 9 |
| HC | T100S | 3.166 | 1.748 | 2.953 | 0.816 | 1.072 | 2.142 | 471 | 9 |
| HC | T100V | 3.237 | 1.957 | 2.775 | 1.307 | 1.173 | 1.499 | 472 | 9 |
| HC | T100Y | 2.924 | 1.238 | 2.473 | 0.937 | 1.182 | 1.321 | 473 | 9 |
| HC | Y101H | 3.319 | 2.884 | 3.256 | 2.203 | 1.019 | 1.309 | 474 | 9 |
| HC | Y101E | 0.081 | 0.075 | 0.090 | 0.038 | 0.894 | 0.995 | 475 | 9 |
| HC | Y101F | 2.795 | 0.990 | 1.719 | 0.450 | 1.632 | 2.202 | 476 | 9 |
| HC | Y101M | 3.072 | 1.802 | 2.893 | 1.574 | 1.063 | 1.145 | 477 | 9 |
| HC | Y101W | 3.237 | 1.648 | 3.078 | 0.756 | 1.052 | 2.178 | 478 | 9 |
| HC | Y102R | 0.091 | 0.086 | 0.074 | 0.077 | 1.221 | 1.109 | 479 | 9 |
| HC | Y102C | 0.099 | 0.085 | 0.088 | 0.087 | 1.128 | 1.042 | 480 | 9 |
| HC | Y102D | 0.093 | 0.084 | 0.086 | 0.080 | 1.084 | 1.059 | 481 | 9 |
| HC | Y102I | 0.094 | 0.082 | 0.073 | 0.075 | 1.290 | 1.099 | 482 | 9 |
| HC | Y102N | 0.096 | 0.082 | 0.077 | 0.075 | 1.250 | 1.088 | 483 | 9 |

(continued)

| Table 15. Variant anti-EGFR antibodies | | | | | | | | | |
|---|---|---|---|---|---|---|---|---|---|
| | | Average OD (pH 6.0) ($OD_{6.0}$) | | Average OD (pH 7.4 ($OD_{7.4}$) | | $OD_{6.0}$ / $OD_{7.4}$ | | SEQ ID NO | |
| Chain | Mutation | Dilution 1 | Dilution 2 | Dilution 1 | Dilution 2 | Dilution 1 | Dilution 2 | HC | LC |
| HC | Y102W | 3.058 | 1.411 | 2.711 | 0.941 | 1.129 | 1.500 | 484 | 9 |
| HC | D103R | 0.134 | 0.093 | 0.115 | 0.098 | 1.168 | 0.942 | 485 | 9 |
| HC | D103L | 0.082 | 0.095 | 0.085 | 0.034 | 0.963 | 1.307 | 486 | 9 |
| HC | D103A | 3.114 | 0.281 | 2.833 | 1.442 | 1.099 | 0.195 | 487 | 9 |
| HC | D103C | 0.076 | 0.078 | 0.075 | 0.072 | 1.021 | 1.087 | 488 | 9 |
| HC | D103I | 0.109 | 0.091 | 0.087 | 0.091 | 1.254 | 1.006 | 489 | 9 |
| HC | D103P | 0.075 | 0.079 | 0.081 | 0.068 | 0.928 | 1.146 | 490 | 9 |
| HC | D103Q | 2.998 | 1.947 | 2.901 | 1.601 | 1.033 | 1.219 | 491 | 9 |
| HC | D103Y | 0.077 | 0.081 | 0.076 | 0.072 | 1.013 | 1.129 | 492 | 9 |
| HC | Y104H | 1.429 | 0.974 | 0.777 | 0.531 | 1.860 | 1.840 | 493 | 9 |
| HC | Y104L | 1.717 | 0.894 | 0.988 | 0.419 | 1.747 | 2.133 | 494 | 9 |
| HC | Y104D | 0.493 | 0.334 | 0.199 | 0.123 | 2.471 | 2.701 | 495 | 9 |
| HC | Y104F | 1.890 | 1.364 | 0.982 | 0.539 | 1.927 | 2.530 | 496 | 9 |
| HC | Y104I | 1.268 | 0.552 | 0.690 | 0.323 | 1.838 | 1.709 | 497 | 9 |
| HC | Y104M | 0.956 | 0.789 | 0.528 | 0.398 | 1.803 | 1.971 | 498 | 9 |
| HC | Y104S | 0.441 | 0.333 | 0.165 | 0.110 | 2.678 | 3.052 | 499 | 9 |
| HC | Y104V | 0.839 | 0.697 | 0.479 | 0.323 | 1.753 | 2.161 | 500 | 9 |
| HC | E105H | 0.061 | 0.059 | 0.060 | 0.030 | 1.021 | 0.997 | 501 | 9 |
| HC | E105T | 1.103 | 0.655 | 0.751 | 0.385 | 1.469 | 1.701 | 502 | 9 |
| HC | F106L | 1.149 | 0.640 | 0.712 | 0.357 | 1.618 | 1.816 | 503 | 9 |
| HC | F106V | 0.308 | 0.111 | 0.185 | 0.095 | 1.667 | 1.174 | 504 | 9 |
| HC | F106W | 1.076 | 0.399 | 0.748 | 0.229 | 1.420 | 1.749 | 505 | 9 |
| HC | F106Y | 1.705 | 0.929 | 1.699 | 0.530 | 1.008 | 1.753 | 506 | 9 |
| HC | A107K | 1.095 | 0.652 | 1.061 | 0.377 | 1.033 | 1.732 | 507 | 9 |
| HC | A107H | 1.208 | 0.830 | 1.208 | 0.468 | 1.014 | 1.776 | 508 | 9 |
| HC | A107R | 1.354 | 0.832 | 1.162 | 0.485 | 1.165 | 1.717 | 509 | 9 |
| HC | A107L | 1.244 | 0.841 | 0.799 | 0.227 | 1.560 | 1.874 | 510 | 9 |
| HC | A107C | 1.069 | 0.566 | 0.842 | 0.322 | 1.277 | 1.762 | 511 | 9 |
| HC | A107D | 0.952 | 0.485 | 0.587 | 0.271 | 1.624 | 1.787 | 512 | 9 |
| HC | A107E | 1.049 | 0.755 | 0.787 | 0.378 | 1.332 | 1.997 | 513 | 9 |
| HC | A107G | 1.161 | 0.776 | 0.923 | 0.424 | 1.258 | 1.830 | 514 | 9 |
| HC | A107N | 0.990 | 0.567 | 1.035 | 0.316 | 0.995 | 1.799 | 515 | 9 |
| HC | A107S | 1.071 | 0.680 | 1.153 | 0.388 | 0.954 | 1.755 | 516 | 9 |
| HC | A107T | 1.141 | 0.615 | 0.851 | 0.358 | 1.343 | 1.723 | 517 | 9 |
| HC | A107Y | 1.368 | 0.802 | 1.121 | 0.422 | 1.230 | 1.898 | 518 | 9 |

(continued)

| Table 15. Variant anti-EGFR antibodies | | | | | | | | | |
|---|---|---|---|---|---|---|---|---|---|
| | | Average OD (pH 6.0) ($OD_{6.0}$) | | Average OD (pH 7.4 ($OD_{7.4}$) | | $OD_{6.0}$ / $OD_{7.4}$ | | SEQ ID NO | |
| Chain | Mutation | Dilution 1 | Dilution 2 | Dilution 1 | Dilution 2 | Dilution 1 | Dilution 2 | HC | LC |
| HC | Y108K | 0.930 | 0.266 | 0.448 | 0.150 | 2.076 | 1.776 | 519 | 9 |
| HC | Y108H | 2.023 | 1.102 | 1.597 | 0.598 | 1.266 | 1.838 | 520 | 9 |
| HC | Y108R | 0.516 | 0.173 | 0.275 | 0.106 | 1.883 | 1.631 | 521 | 9 |
| HC | Y108L | 1.518 | 0.635 | 1.024 | 0.297 | 1.482 | 2.139 | 522 | 9 |
| HC | Y108C | 0.802 | 0.311 | 0.481 | 0.170 | 1.666 | 1.829 | 523 | 9 |
| HC | Y108F | 1.934 | 1.187 | 1.760 | 0.635 | 1.100 | 1.872 | 524 | 9 |
| HC | Y108I | 1.534 | 0.703 | 1.061 | 0.367 | 1.446 | 1.927 | 525 | 9 |
| HC | Y108N | 1.536 | 0.719 | 0.918 | 0.368 | 1.674 | 1.958 | 526 | 9 |
| HC | Y108S | 1.438 | 0.676 | 0.905 | 0.307 | 1.589 | 2.209 | 527 | 9 |
| HC | Y108T | 1.482 | 0.672 | 0.905 | 0.298 | 1.644 | 2.254 | 528 | 9 |
| HC | Y108V | 0.434 | 0.157 | 0.229 | 0.098 | 1.900 | 1.607 | 529 | 9 |
| HC | Y108W | 1.845 | 0.938 | 1.154 | 0.430 | 1.604 | 2.185 | 530 | 9 |
| HC | W109I | 0.919 | 0.266 | 0.470 | 0.151 | 1.957 | 1.755 | 531 | 9 |
| HC | W109M | 1.162 | 0.442 | 0.865 | 0.232 | 1.346 | 1.903 | 532 | 9 |
| HC | W109Y | 0.994 | 0.323 | 0.593 | 0.177 | 1.676 | 1.832 | 533 | 9 |
| HC | G110R | 0.069 | 0.062 | 0.077 | 0.037 | 0.972 | 0.850 | 534 | 9 |
| HC | G110A | 1.937 | 0.839 | 1.589 | 0.541 | 1.229 | 1.552 | 535 | 9 |
| HC | G110M | 0.100 | 0.068 | 0.053 | 0.064 | 1.875 | 1.058 | 536 | 9 |
| HC | G110P | 0.234 | 0.099 | 0.142 | 0.078 | 1.652 | 1.279 | 537 | 9 |
| HC | G110T | 1.117 | 0.371 | 0.774 | 0.234 | 1.442 | 1.594 | 538 | 9 |
| HC | Q111K | 3.167 | 1.888 | 2.878 | 1.122 | 1.101 | 1.693 | 539 | 9 |
| HC | Q111H | 2.442 | 0.722 | 1.412 | 0.363 | 1.729 | 1.992 | 540 | 9 |
| HC | Q111R | 2.940 | 1.110 | 2.019 | 0.507 | 1.456 | 2.192 | 541 | 9 |
| HC | Q111L | 2.960 | 1.155 | 2.111 | 0.542 | 1.403 | 2.132 | 542 | 9 |
| HC | Q111D | 2.881 | 1.072 | 2.046 | 0.503 | 1.417 | 2.132 | 543 | 9 |
| HC | Q111E | 3.087 | 1.497 | 2.422 | 0.649 | 1.275 | 2.311 | 544 | 9 |
| HC | Q111G | 2.853 | 1.136 | 2.115 | 0.568 | 1.351 | 1.998 | 545 | 9 |
| HC | Q111M | 1.621 | 0.420 | 0.776 | 0.093 | 2.094 | 2.197 | 546 | 9 |
| HC | Q111P | 2.558 | 0.817 | 1.423 | 0.369 | 1.797 | 2.211 | 547 | 9 |
| HC | Q111S | 2.912 | 1.292 | 2.334 | 0.588 | 1.250 | 2.204 | 548 | 9 |
| HC | Q111T | 3.156 | 2.059 | 2.713 | 1.020 | 1.163 | 2.018 | 549 | 9 |
| HC | Q111V | 0.928 | 0.287 | 0.389 | 0.143 | 2.426 | 2.021 | 1064 | 9 |
| HC | Q111W | 2.633 | 0.820 | 1.533 | 0.366 | 1.721 | 2.241 | 550 | 9 |
| HC | Q111Y | 2.705 | 1.111 | 1.891 | 0.506 | 1.431 | 2.192 | 551 | 9 |
| HC | G112A | 1.008 | 0.276 | 0.609 | 0.168 | 1.657 | 1.645 | 552 | 9 |

(continued)

| Table 15. Variant anti-EGFR antibodies | | | | | | | | |
|---|---|---|---|---|---|---|---|---|
| | | Average OD (pH 6.0) ($OD_{6.0}$) | | Average OD (pH 7.4 ($OD_{7.4}$) | | $OD_{6.0}$ / $OD_{7.4}$ | | SEQ ID NO | |
| Chain | Mutation | Dilution 1 | Dilution 2 | Dilution 1 | Dilution 2 | Dilution 1 | Dilution 2 | HC | LC |
| HC | G112N | 0.152 | 0.218 | 0.120 | 0.074 | 1.269 | 1.075 | 553 | 9 |
| HC | G112P | 1.396 | 0.443 | 1.154 | 0.293 | 1.210 | 1.515 | 554 | 9 |
| HC | G112S | 0.774 | 0.208 | 0.537 | 0.142 | 1.442 | 1.462 | 555 | 9 |
| HC | G112T | 0.195 | 0.085 | 0.129 | 0.072 | 1.509 | 1.169 | 556 | 9 |
| HC | G112Y | 0.176 | 0.080 | 0.114 | 0.068 | 1.565 | 1.172 | 557 | 9 |
| LC | D1W | 2.925 | 1.768 | 2.617 | 0.583 | 1.124 | 1.594 | 8 | 810 |
| LC | I2C | 2.076 | 1.460 | 1.622 | 0.332 | 1.284 | 1.475 | 8 | 811 |
| LC | I2V | 2.520 | 1.080 | 1.908 | 0.530 | 1.326 | 2.054 | 8 | 812 |
| LC | I2W | 1.308 | 0.324 | 0.909 | 0.092 | 1.448 | 3.539 | 8 | 813 |
| LC | L3D | 0.977 | 0.280 | 0.481 | 0.149 | 2.031 | 1.898 | 8 | 814 |
| LC | L3F | 1.085 | 0.313 | 0.495 | 0.178 | 2.194 | 1.784 | 8 | 815 |
| LC | L3G | 3.056 | 2.119 | 3.021 | 0.406 | 1.015 | 2.677 | 8 | 816 |
| LC | L3S | 1.494 | 0.390 | 0.760 | 0.219 | 1.967 | 1.780 | 8 | 817 |
| LC | L3T | 2.433 | 0.850 | 1.908 | 0.396 | 1.276 | 2.157 | 8 | 818 |
| LC | L3V | 2.544 | 1.051 | 2.034 | 0.294 | 1.258 | 3.578 | 8 | 819 |
| LC | L3W | 2.342 | 0.652 | 1.239 | 0.313 | 1.891 | 2.088 | 8 | 820 |
| LC | L3Y | 2.522 | 0.894 | 1.958 | 0.476 | 1.310 | 1.881 | 8 | 821 |
| LC | L3R | 3.123 | 1.858 | 3.257 | 0.799 | 0.959 | 2.324 | 8 | 822 |
| LC | L4C | 1.277 | 0.354 | 0.511 | 0.172 | 2.500 | 2.065 | 8 | 823 |
| LC | L4E | 2.282 | 0.635 | 0.992 | 0.268 | 2.301 | 2.374 | 8 | 824 |
| LC | L4F | 0.666 | 0.196 | 0.257 | 0.105 | 2.595 | 1.876 | 8 | 825 |
| LC | L4I | 2.044 | 0.594 | 0.954 | 0.244 | 2.141 | 2.445 | 8 | 826 |
| LC | L4P | 1.034 | 0.288 | 0.434 | 0.143 | 2.387 | 2.025 | 8 | 827 |
| LC | L4S | 0.714 | 0.207 | 0.286 | 0.108 | 2.496 | 1.928 | 8 | 828 |
| LC | L4T | 1.397 | 0.383 | 0.540 | 0.163 | 2.594 | 2.343 | 8 | 829 |
| LC | L4V | 1.497 | 0.413 | 0.573 | 0.085 | 2.614 | 2.559 | 8 | 830 |
| LC | L4W | 0.867 | 0.225 | 0.331 | 0.115 | 2.626 | 1.962 | 8 | 831 |
| LC | L4K | 0.917 | 0.249 | 0.363 | 0.122 | 2.555 | 2.042 | 8 | 832 |
| LC | L4H | 1.168 | 0.298 | 0.395 | 0.139 | 2.948 | 2.152 | 8 | 833 |
| LC | L4R | 2.025 | 0.583 | 0.817 | 0.229 | 2.507 | 2.543 | 8 | 834 |
| LC | T5A | 2.306 | 1.577 | 1.307 | 0.268 | 1.768 | 2.845 | 8 | 835 |
| LC | T5C | 1.929 | 0.521 | 0.747 | 0.192 | 2.622 | 2.719 | 8 | 836 |
| LC | T5D | 2.275 | 0.814 | 1.408 | 0.289 | 1.615 | 2.818 | 8 | 837 |
| LC | T5E | 2.809 | 1.421 | 2.377 | 0.555 | 1.182 | 2.564 | 8 | 838 |
| LC | T5F | 2.403 | 0.890 | 1.568 | 0.351 | 1.532 | 2.537 | 8 | 839 |

(continued)

| Table 15. Variant anti-EGFR antibodies | | | | | | | | | |
|---|---|---|---|---|---|---|---|---|---|
| | | Average OD (pH 6.0) ($OD_{6.0}$) | | Average OD (pH 7.4 ($OD_{7.4}$) | | $OD_{6.0}$ / $OD_{7.4}$ | | SEQ ID NO | |
| Chain | Mutation | Dilution 1 | Dilution 2 | Dilution 1 | Dilution 2 | Dilution 1 | Dilution 2 | HC | LC |
| LC | T5G | 2.079 | 0.697 | 1.277 | 0.267 | 1.629 | 2.608 | 8 | 840 |
| LC | T5N | 2.438 | 0.947 | 1.721 | 0.363 | 1.417 | 2.609 | 8 | 841 |
| LC | T5P | 1.226 | 0.364 | 0.584 | 0.171 | 2.098 | 2.127 | 8 | 1067 |
| LC | T5S | 2.515 | 0.908 | 1.421 | 0.320 | 1.772 | 2.843 | 8 | 842 |
| LC | T5W | 2.195 | 0.701 | 1.131 | 0.246 | 1.943 | 2.854 | 8 | 843 |
| LC | T5L | 2.512 | 1.262 | 2.186 | 0.525 | 1.149 | 2.405 | 8 | 844 |
| LC | T5K | 2.558 | 0.944 | 1.638 | 0.370 | 1.562 | 2.556 | 8 | 845 |
| LC | T5H | 2.800 | 1.163 | 1.669 | 0.355 | 1.678 | 3.277 | 8 | 846 |
| LC | T5R | 2.633 | 1.328 | 1.846 | 0.423 | 1.428 | 3.143 | 8 | 847 |
| LC | R24A | 2.819 | 1.801 | 2.525 | 0.751 | 1.119 | 2.406 | 8 | 848 |
| LC | R24C | 2.004 | 0.612 | 1.021 | 0.249 | 1.965 | 2.460 | 8 | 849 |
| LC | R24F | 2.121 | 0.749 | 1.259 | 0.288 | 1.688 | 2.605 | 8 | 850 |
| LC | R24G | 1.023 | 0.297 | 0.396 | 0.133 | 2.599 | 2.237 | 8 | 1068 |
| LC | R24L | 2.886 | 1.764 | 2.615 | 0.748 | 1.104 | 2.372 | 8 | 851 |
| LC | R24M | 2.880 | 2.141 | 2.619 | 0.562 | 1.100 | 1.749 | 8 | 852 |
| LC | R24S | 2.443 | 0.980 | 1.621 | 0.365 | 1.508 | 2.689 | 8 | 853 |
| LC | R24W | 2.019 | 0.655 | 1.111 | 0.261 | 1.816 | 2.512 | 8 | 854 |
| LC | R24Y | 2.557 | 1.315 | 2.221 | 0.545 | 1.152 | 2.413 | 8 | 855 |
| LC | A25C | 2.233 | 0.712 | 1.754 | 0.334 | 1.275 | 2.130 | 8 | 856 |
| LC | A25G | 2.406 | 1.123 | 2.373 | 0.568 | 1.014 | 1.986 | 8 | 857 |
| LC | A25L | 1.794 | 0.494 | 1.182 | 0.240 | 1.534 | 2.063 | 8 | 858 |
| LC | A25V | 2.351 | 1.883 | 1.718 | 0.463 | 1.370 | 1.926 | 8 | 859 |
| LC | S26A | 2.032 | 0.623 | 1.194 | 0.320 | 1.703 | 1.949 | 8 | 860 |
| LC | S26C | 1.490 | 0.370 | 0.672 | 0.204 | 2.232 | 1.814 | 8 | 861 |
| LC | S26D | 1.076 | 1.362 | 0.504 | 0.199 | 2.140 | 1.689 | 8 | 862 |
| LC | S26I | 1.847 | 0.549 | 1.137 | 0.297 | 1.642 | 1.850 | 8 | 863 |
| LC | S26M | 1.882 | 0.511 | 0.944 | 0.271 | 1.999 | 1.920 | 8 | 864 |
| LC | S26N | 2.649 | 1.069 | 2.006 | 0.472 | 1.325 | 2.269 | 8 | 865 |
| LC | S26V | 1.023 | 0.318 | 0.487 | 0.181 | 2.104 | 1.778 | 8 | 866 |
| LC | S26W | 1.416 | 0.394 | 0.640 | 0.211 | 2.215 | 1.871 | 8 | 867 |
| LC | S26L | 2.514 | 0.892 | 1.679 | 0.460 | 1.498 | 1.938 | 8 | 868 |
| LC | S26G | 2.563 | 1.076 | 1.773 | 0.470 | 1.448 | 2.293 | 8 | 869 |
| LC | S26H | 2.686 | 1.429 | 2.620 | 0.764 | 1.029 | 1.871 | 8 | 870 |
| LC | S26R | 0.578 | 0.206 | 0.310 | 0.166 | 1.869 | 1.245 | 8 | 871 |
| LC | Q27A | 2.910 | 1.942 | 2.602 | 0.970 | 1.118 | 2.002 | 8 | 872 |

(continued)

| Table 15. Variant anti-EGFR antibodies | | | | | | | | | |
|---|---|---|---|---|---|---|---|---|---|
| | | Average OD (pH 6.0) ($OD_{6.0}$) | | Average OD (pH 7.4 ($OD_{7.4}$) | | $OD_{6.0}$ / $OD_{7.4}$ | | SEQ ID NO | |
| Chain | Mutation | Dilution 1 | Dilution 2 | Dilution 1 | Dilution 2 | Dilution 1 | Dilution 2 | HC | LC |
| LC | Q27D | 2.850 | 1.856 | 2.682 | 0.962 | 1.064 | 1.940 | 8 | 873 |
| LC | Q27E | 2.980 | 1.656 | 2.752 | 0.774 | 1.084 | 2.141 | 8 | 874 |
| LC | Q27F | 3.022 | 1.396 | 2.597 | 0.684 | 1.164 | 2.044 | 8 | 875 |
| LC | Q27I | 3.166 | 2.049 | 2.605 | 1.092 | 1.216 | 1.881 | 8 | 876 |
| LC | Q27M | 3.076 | 1.975 | 2.485 | 0.917 | 1.243 | 2.153 | 8 | 877 |
| LC | Q27N | 2.816 | 1.768 | 2.563 | 0.976 | 1.099 | 1.811 | 8 | 878 |
| LC | Q27P | 1.967 | 1.368 | 1.815 | 0.645 | 1.128 | 2.123 | 8 | 879 |
| LC | Q27T | 3.165 | 2.567 | 2.919 | 0.861 | 1.085 | 1.506 | 8 | 880 |
| LC | S28A | 2.339 | 0.741 | 1.315 | 0.353 | 1.779 | 2.100 | 8 | 881 |
| LC | S28D | 2.972 | 1.878 | 2.403 | 0.971 | 1.268 | 1.964 | 8 | 882 |
| LC | S28N | 3.165 | 2.818 | 3.278 | 1.196 | 0.966 | 1.114 | 8 | 883 |
| LC | S28Q | 2.869 | 1.140 | 2.247 | 0.527 | 1.277 | 2.168 | 8 | 884 |
| LC | S28L | 1.871 | 0.518 | 1.004 | 0.256 | 1.859 | 2.022 | 8 | 885 |
| LC | S28K | 2.492 | 0.759 | 1.663 | 0.411 | 1.499 | 1.871 | 8 | 886 |
| LC | S28H | 2.843 | 1.108 | 2.146 | 0.484 | 1.325 | 2.293 | 8 | 887 |
| LC | I29A | 2.899 | 1.699 | 2.373 | 0.947 | 1.222 | 1.796 | 8 | 888 |
| LC | I29E | 2.217 | 0.833 | 1.193 | 0.437 | 1.862 | 1.908 | 8 | 889 |
| LC | I29F | 2.761 | 1.091 | 1.913 | 0.613 | 1.444 | 1.781 | 8 | 890 |
| LC | I29S | 2.910 | 1.745 | 1.779 | 0.627 | 1.742 | 3.037 | 8 | 891 |
| LC | I29T | 2.967 | 1.544 | 2.317 | 0.796 | 1.282 | 1.944 | 8 | 892 |
| LC | I29R | 0.124 | 1.528 | 0.294 | 0.140 | 0.422 | 0.673 | 8 | 893 |
| LC | G30A | 2.660 | 1.192 | 2.154 | 0.626 | 1.236 | 1.905 | 8 | 894 |
| LC | G30E | 3.158 | 1.981 | 2.865 | 1.070 | 1.109 | 1.852 | 8 | 895 |
| LC | G30F | 2.951 | 1.136 | 2.046 | 0.474 | 1.442 | 2.408 | 8 | 896 |
| LC | G30I | 2.653 | 1.210 | 2.221 | 0.642 | 1.195 | 1.885 | 8 | 897 |
| LC | G30M | 3.077 | 1.589 | 2.595 | 0.864 | 1.189 | 1.841 | 8 | 898 |
| LC | G30P | 2.643 | 1.034 | 1.826 | 0.541 | 1.447 | 1.911 | 8 | 899 |
| LC | G30Q | 2.855 | 1.151 | 2.261 | 0.608 | 1.263 | 1.895 | 8 | 900 |
| LC | G30S | 2.918 | 1.562 | 2.272 | 0.708 | 1.284 | 2.213 | 8 | 901 |
| LC | G30V | 2.539 | 0.882 | 1.477 | 0.406 | 1.720 | 2.172 | 8 | 902 |
| LC | G30Y | 2.270 | 0.630 | 1.137 | 0.244 | 1.998 | 2.586 | 8 | 903 |
| LC | G30L | 3.075 | 1.525 | 2.530 | 0.351 | 1.216 | 2.155 | 8 | 904 |
| LC | G30K | 2.747 | 0.945 | 1.681 | 0.385 | 1.634 | 2.456 | 8 | 905 |
| LC | G30H | 2.864 | 1.080 | 2.184 | 0.577 | 1.317 | 1.874 | 8 | 906 |
| LC | G30R | 2.634 | 1.078 | 2.126 | 0.529 | 1.239 | 2.041 | 8 | 907 |

| Table 15. Variant anti-EGFR antibodies | | | | | | | | |
|---|---|---|---|---|---|---|---|---|
| Chain | Mutation | Average OD (pH 6.0) ($OD_{6.0}$) | | Average OD (pH 7.4 ($OD_{7.4}$) | | $OD_{6.0}$ / $OD_{7.4}$ | | SEQ ID NO | |
| | | Dilution 1 | Dilution 2 | Dilution 1 | Dilution 2 | Dilution 1 | Dilution 2 | HC | LC |
| LC | T31A | 3.109 | 1.829 | 2.594 | 0.794 | 1.202 | 2.305 | 8 | 908 |
| LC | T31F | 2.585 | 1.954 | 1.545 | 0.378 | 1.673 | 2.444 | 8 | 909 |
| LC | T31G | 3.135 | 1.900 | 2.537 | 0.908 | 1.236 | 2.093 | 8 | 910 |
| LC | T31M | 3.168 | 2.090 | 2.724 | 0.921 | 1.163 | 2.270 | 8 | 911 |
| LC | T31S | 3.017 | 1.525 | 2.487 | 0.796 | 1.213 | 1.916 | 8 | 912 |
| LC | T31V | 3.059 | 1.618 | 2.684 | 0.843 | 1.140 | 1.923 | 8 | 913 |
| LC | T31W | 2.825 | 1.133 | 1.755 | 0.480 | 1.639 | 2.359 | 8 | 914 |
| LC | T31L | 2.910 | 1.274 | 2.135 | 0.647 | 1.365 | 1.969 | 8 | 915 |
| LC | T31K | 3.195 | 2.263 | 2.923 | 1.161 | 1.093 | 1.949 | 8 | 916 |
| LC | T31H | 3.172 | 2.169 | 3.026 | 1.098 | 1.049 | 1.976 | 8 | 917 |
| LC | N32G | 2.507 | 2.003 | 2.318 | 0.992 | 1.081 | 1.057 | 8 | 918 |
| LC | I33F | 2.150 | 0.712 | 1.647 | 0.362 | 1.306 | 1.971 | 8 | 919 |
| LC | I33G | 0.497 | 0.726 | 0.321 | 0.122 | 1.552 | 1.396 | 8 | 920 |
| LC | I33M | 2.452 | 0.922 | 1.788 | 0.471 | 1.391 | 1.957 | 8 | 921 |
| LC | I33T | 2.308 | 0.841 | 1.714 | 0.447 | 1.351 | 1.880 | 8 | 922 |
| LC | I33V | 2.684 | 1.395 | 2.296 | 0.674 | 1.171 | 2.089 | 8 | 923 |
| LC | I33H | 0.520 | 0.162 | 0.305 | 0.101 | 1.707 | 1.603 | 8 | 924 |
| LC | I48M | 3.195 | 2.000 | 2.971 | 0.998 | 1.076 | 2.004 | 8 | 925 |
| LC | I48S | 2.486 | 1.520 | 2.469 | 0.741 | 1.007 | 1.037 | 8 | 926 |
| LC | I48L | 3.126 | 1.720 | 2.560 | 0.804 | 1.221 | 2.142 | 8 | 927 |
| LC | I48K | 3.092 | 1.618 | 2.624 | 0.785 | 1.180 | 2.062 | 8 | 928 |
| LC | K49A | 3.111 | 2.465 | 3.143 | 1.634 | 0.990 | 1.508 | 8 | 929 |
| LC | K49E | 2.831 | 1.362 | 2.504 | 0.999 | 1.136 | 1.374 | 8 | 930 |
| LC | K49F | 2.953 | 1.733 | 2.622 | 0.910 | 1.126 | 1.904 | 8 | 931 |
| LC | K49G | 3.059 | 2.388 | 3.056 | 1.493 | 1.001 | 1.602 | 8 | 932 |
| LC | K49N | 2.967 | 2.078 | 2.833 | 1.037 | 1.048 | 2.009 | 8 | 933 |
| LC | K49Q | 3.070 | 2.336 | 2.908 | 1.708 | 1.058 | 1.376 | 8 | 934 |
| LC | K49S | 3.179 | 2.802 | 3.199 | 1.890 | 0.994 | 1.485 | 8 | 935 |
| LC | K49T | 3.161 | 2.528 | 3.076 | 1.343 | 1.028 | 1.884 | 8 | 936 |
| LC | K49V | 3.087 | 1.831 | 2.694 | 0.947 | 1.145 | 1.934 | 8 | 937 |
| LC | K49Y | 2.948 | 1.490 | 2.252 | 0.699 | 1.309 | 2.130 | 8 | 938 |
| LC | K49L | 2.767 | 2.365 | 2.614 | 1.220 | 1.060 | 1.459 | 8 | 939 |
| LC | K49H | 3.068 | 1.734 | 2.749 | 0.736 | 1.116 | 2.356 | 8 | 940 |
| LC | K49R | 3.091 | 2.911 | 3.020 | 2.277 | 1.023 | 1.278 | 8 | 941 |
| LC | A51T | 2.711 | 1.309 | 1.762 | 0.477 | 1.541 | 2.749 | 8 | 942 |

(continued)

| Table 15. Variant anti-EGFR antibodies | | | | | | | | |
|---|---|---|---|---|---|---|---|---|
| | | Average OD (pH 6.0) ($OD_{6.0}$) | | Average OD (pH 7.4 ($OD_{7.4}$) | | $OD_{6.0}$ / $OD_{7.4}$ | | SEQ ID NO | |
| Chain | Mutation | Dilution 1 | Dilution 2 | Dilution 1 | Dilution 2 | Dilution 1 | Dilution 2 | HC | LC |
| LC | A51L | 2.611 | 1.889 | 2.090 | 0.781 | 1.250 | 1.595 | 8 | 943 |
| LC | S52A | 3.173 | 2.440 | 2.815 | 0.622 | 1.130 | 2.031 | 8 | 944 |
| LC | S52C | 2.145 | 0.676 | 1.079 | 0.250 | 1.995 | 2.709 | 8 | 945 |
| LC | S52D | 3.127 | 2.159 | 2.649 | 0.884 | 1.180 | 2.446 | 8 | 946 |
| LC | S52E | 2.874 | 1.773 | 2.495 | 0.691 | 1.152 | 2.569 | 8 | 947 |
| LC | S52G | 2.398 | 0.920 | 1.424 | 0.356 | 1.692 | 2.585 | 8 | 948 |
| LC | S52I | 2.301 | 0.928 | 1.450 | 0.358 | 1.599 | 2.598 | 8 | 949 |
| LC | S52M | 2.462 | 0.880 | 1.489 | 0.312 | 1.668 | 2.833 | 8 | 950 |
| LC | S52Q | 2.678 | 1.044 | 1.825 | 0.409 | 1.471 | 2.562 | 8 | 951 |
| LC | S52V | 2.799 | 1.545 | 2.259 | 0.639 | 1.240 | 2.420 | 8 | 952 |
| LC | S52W | 2.632 | 1.007 | 1.620 | 0.386 | 1.632 | 2.623 | 8 | 953 |
| LC | S52R | 3.133 | 1.934 | 2.294 | 0.746 | 1.367 | 2.604 | 8 | 954 |
| LC | S52K | 3.028 | 1.494 | 1.964 | 0.510 | 1.542 | 2.954 | 8 | 955 |
| LC | E53G | 0.182 | 0.173 | 0.122 | 0.076 | 1.605 | 1.489 | 8 | 956 |
| LC | S54M | 2.365 | 2.496 | 2.846 | 1.362 | 0.831 | 1.427 | 8 | 957 |
| LC | I55A | 2.591 | 1.923 | 2.689 | 0.728 | 0.964 | 1.318 | 8 | 958 |
| LC | I55F | 2.450 | 1.521 | 2.503 | 0.781 | 0.980 | 1.950 | 8 | 959 |
| LC | S56G | 3.158 | 2.562 | 2.991 | 1.497 | 1.056 | 1.719 | 8 | 960 |
| LC | S56L | 3.088 | 2.195 | 2.849 | 1.078 | 1.084 | 2.042 | 8 | 961 |
| LC | S56A | 3.072 | 2.332 | 3.031 | 1.328 | 1.015 | 1.759 | 8 | 962 |
| LC | S56C | 2.974 | 1.448 | 2.383 | 0.328 | 1.250 | 2.158 | 8 | 963 |
| LC | S56D | 3.060 | 1.994 | 2.841 | 1.165 | 1.077 | 1.711 | 8 | 964 |
| LC | S56E | 3.130 | 2.431 | 2.972 | 1.482 | 1.053 | 1.642 | 8 | 965 |
| LC | S56F | 3.095 | 2.008 | 2.961 | 1.102 | 1.046 | 1.824 | 8 | 966 |
| LC | S56N | 3.043 | 2.136 | 3.044 | 1.188 | 1.000 | 1.804 | 8 | 967 |
| LC | S56P | 3.120 | 2.744 | 3.119 | 2.194 | 1.000 | 1.251 | 8 | 968 |
| LC | S56Q | 3.136 | 2.242 | 2.999 | 1.207 | 1.046 | 1.858 | 8 | 969 |
| LC | S56V | 3.034 | 2.233 | 2.949 | 1.338 | 1.029 | 1.671 | 8 | 970 |
| LC | S56W | 3.044 | 1.944 | 2.720 | 0.978 | 1.119 | 1.988 | 8 | 971 |
| LC | S56H | 0.132 | 0.088 | 0.100 | 0.094 | 1.309 | 0.932 | 8 | 972 |
| LC | S56R | 3.035 | 1.896 | 2.681 | 0.952 | 1.132 | 1.992 | 8 | 973 |
| LC | S56K | 3.126 | 2.375 | 2.994 | 1.459 | 1.044 | 1.629 | 8 | 974 |
| LC | Y86F | 0.314 | 0.106 | 0.176 | 0.083 | 1.789 | 1.291 | 8 | 975 |
| LC | Y86M | 0.265 | 0.095 | 0.152 | 0.077 | 1.751 | 1.235 | 8 | 976 |
| LC | Y86H | 0.454 | 0.140 | 0.259 | 0.046 | 1.754 | 1.475 | 8 | 977 |

(continued)

| Table 15. Variant anti-EGFR antibodies | | | | | | | | | |
|---|---|---|---|---|---|---|---|---|---|
| | | Average OD (pH 6.0) ($OD_{6.0}$) | | Average OD (pH 7.4 ($OD_{7.4}$) | | $OD_{6.0}$ / $OD_{7.4}$ | | SEQ ID NO | |
| Chain | Mutation | Dilution 1 | Dilution 2 | Dilution 1 | Dilution 2 | Dilution 1 | Dilution 2 | HC | LC |
| LC | Y87L | 1.364 | 0.391 | 0.662 | 0.189 | 2.060 | 2.070 | 8 | 978 |
| LC | Y87C | 2.233 | 0.766 | 1.316 | 0.354 | 1.697 | 2.169 | 8 | 979 |
| LC | Y87D | 0.692 | 0.193 | 0.295 | 0.114 | 2.345 | 1.700 | 8 | 980 |
| LC | Y87F | 2.372 | 1.681 | 2.434 | 1.000 | 0.981 | 1.709 | 8 | 981 |
| LC | Y87G | 0.941 | 0.252 | 0.344 | 0.118 | 2.738 | 2.145 | 8 | 982 |
| LC | Y87I | 2.941 | 1.874 | 2.773 | 0.977 | 1.061 | 1.917 | 8 | 983 |
| LC | Y87N | 1.369 | 0.921 | 0.571 | 0.166 | 2.397 | 2.199 | 8 | 984 |
| LC | Y87P | 0.697 | 0.195 | 0.358 | 0.144 | 1.947 | 1.353 | 8 | 985 |
| LC | Y87S | 2.337 | 0.880 | 1.488 | 0.387 | 1.571 | 2.273 | 8 | 986 |
| LC | Y87T | 2.232 | 0.926 | 1.739 | 0.533 | 1.283 | 1.746 | 8 | 987 |
| LC | Y87V | 2.621 | 1.571 | 2.360 | 0.730 | 1.110 | 2.155 | 8 | 988 |
| LC | Y87W | 2.260 | 1.231 | 2.159 | 0.739 | 1.046 | 1.667 | 8 | 989 |
| LC | Y87K | 1.493 | 0.385 | 0.700 | 0.207 | 2.137 | 1.860 | 8 | 990 |
| LC | Y87H | 0.295 | 0.118 | 0.194 | 0.104 | 1.522 | 1.132 | 8 | 991 |
| LC | Y87R | 1.711 | 0.562 | 0.997 | 0.296 | 1.716 | 1.949 | 8 | 992 |
| LC | Q89E | 2.195 | 0.799 | 1.637 | 0.441 | 1.342 | 1.815 | 8 | 993 |
| LC | N91L | 0.334 | 0.124 | 0.162 | 0.087 | 2.064 | 1.421 | 8 | 994 |
| LC | N91A | 2.624 | 2.060 | 2.319 | 1.753 | 1.131 | 1.182 | 8 | 995 |
| LC | N91C | 2.633 | 1.226 | 2.163 | 0.790 | 1.219 | 1.553 | 8 | 996 |
| LC | N91I | 2.911 | 1.849 | 2.630 | 0.571 | 1.108 | 1.607 | 8 | 997 |
| LC | N91M | 2.428 | 1.480 | 2.182 | 1.132 | 1.114 | 1.308 | 8 | 998 |
| LC | N91S | 2.994 | 2.783 | 2.760 | 2.387 | 1.085 | 1.166 | 8 | 999 |
| LC | N91T | 2.831 | 1.991 | 2.546 | 1.387 | 1.113 | 1.435 | 8 | 1000 |
| LC | N91V | 2.740 | 1.978 | 2.498 | 1.686 | 1.098 | 1.173 | 8 | 1001 |
| LC | N91H | 2.919 | 1.694 | 2.691 | 0.876 | 1.085 | 1.940 | 8 | 1002 |
| LC | N91R | 0.097 | 0.080 | 0.083 | 0.075 | 1.159 | 1.056 | 8 | 1003 |
| LC | N92C | 2.942 | 1.540 | 2.633 | 1.142 | 1.118 | 1.349 | 8 | 1004 |
| LC | N92D | 3.181 | 2.318 | 2.980 | 1.686 | 1.067 | 1.375 | 8 | 1005 |
| LC | N92L | 2.733 | 1.469 | 2.812 | 0.741 | 0.972 | 1.983 | 8 | 1006 |
| LC | N92M | 2.853 | 1.874 | 2.849 | 0.876 | 1.002 | 2.139 | 8 | 1007 |
| LC | N92S | 2.560 | 1.897 | 2.199 | 1.094 | 1.165 | 1.733 | 8 | 1008 |
| LC | N92T | 2.583 | 2.056 | 2.586 | 1.336 | 0.997 | 1.551 | 8 | 1009 |
| LC | N92V | 3.125 | 2.740 | 3.058 | 1.851 | 1.022 | 1.484 | 8 | 1010 |
| LC | N92W | 2.147 | 1.772 | 1.969 | 1.546 | 1.092 | 1.146 | 8 | 1011 |
| LC | N92Y | 2.125 | 0.636 | 1.703 | 0.477 | 1.248 | 1.332 | 8 | 1012 |

(continued)

| Table 15. Variant anti-EGFR antibodies | | | | | | | | | |
|---|---|---|---|---|---|---|---|---|---|
| | | Average OD (pH 6.0) $(OD_{6.0})$ | | Average OD (pH 7.4 $(OD_{7.4})$ | | $OD_{6.0}$ / $OD_{7.4}$ | | SEQ ID NO | |
| Chain | Mutation | Dilution 1 | Dilution 2 | Dilution 1 | Dilution 2 | Dilution 1 | Dilution 2 | HC | LC |
| LC | N92H | 3.094 | 2.440 | 2.804 | 1.941 | 1.103 | 1.257 | 8 | 1013 |
| LC | N92K | 2.429 | 0.625 | 2.546 | 0.519 | 0.955 | 2.118 | 8 | 1014 |
| LC | N92R | 3.085 | 1.643 | 2.966 | 0.691 | 1.040 | 2.377 | 8 | 1015 |
| LC | N93T | 0.197 | 0.094 | 0.192 | 0.048 | 1.029 | 1.021 | 8 | 1016 |
| LC | T96L | 3.174 | 1.793 | 2.779 | 0.701 | 1.176 | 2.936 | 8 | 1017 |
| LC | T96C | 2.941 | 1.378 | 2.394 | 0.699 | 1.230 | 1.972 | 8 | 1018 |
| LC | T96M | 2.899 | 1.972 | 2.673 | 0.834 | 1.084 | 1.673 | 8 | 1019 |
| LC | T96V | 3.101 | 1.774 | 3.006 | 0.936 | 1.032 | 1.898 | 8 | 1020 |
| LC | T97L | 2.411 | 0.799 | 1.773 | 0.399 | 1.366 | 2.004 | 8 | 1021 |
| LC | T97A | 2.794 | 1.273 | 3.309 | 0.332 | 0.845 | 1.954 | 8 | 1022 |
| LC | T97D | 1.749 | 0.461 | 1.007 | 0.237 | 1.737 | 1.951 | 8 | 1023 |
| LC | T97G | 1.691 | 0.521 | 1.423 | 0.276 | 1.190 | 1.888 | 8 | 1024 |
| LC | T97Q | 2.618 | 1.004 | 2.602 | 0.537 | 1.005 | 1.869 | 8 | 1025 |
| LC | T97S | 2.108 | 0.545 | 1.884 | 0.260 | 1.119 | 2.095 | 8 | 1026 |
| LC | T97V | 2.316 | 0.998 | 2.021 | 0.517 | 1.151 | 1.935 | 8 | 1027 |
| LC | T97K | 2.211 | 0.892 | 2.542 | 0.491 | 0.870 | 1.816 | 8 | 1028 |
| LC | T97R | 0.542 | 0.180 | 0.282 | 0.127 | 1.922 | 1.420 | 8 | 1029 |
| LC | F98A | 0.999 | 0.296 | 0.635 | 0.096 | 1.573 | 1.537 | 8 | 1030 |
| LC | F98M | 2.228 | 0.686 | 1.414 | 0.431 | 1.582 | 1.600 | 8 | 1031 |
| LC | F98S | 1.532 | 0.467 | 1.079 | 0.299 | 1.422 | 1.560 | 8 | 1032 |
| LC | F98V | 1.895 | 0.533 | 1.161 | 0.315 | 1.645 | 1.699 | 8 | 1033 |
| LC | F98Y | 2.871 | 1.365 | 2.439 | 0.785 | 1.177 | 1.738 | 8 | 1034 |
| LC | G99L | 0.578 | 0.164 | 0.310 | 0.096 | 1.864 | 1.713 | 8 | 1035 |
| LC | G99D | 0.521 | 0.132 | 0.308 | 0.088 | 1.692 | 1.498 | 8 | 1036 |
| LC | G99E | 0.496 | 0.166 | 0.300 | 0.126 | 1.655 | 1.324 | 8 | 1037 |
| LC | G99F | 0.583 | 0.183 | 0.255 | 0.094 | 2.288 | 1.940 | 8 | 1038 |
| LC | G99I | 0.480 | 0.141 | 0.293 | 0.100 | 1.645 | 1.420 | 8 | 1039 |
| LC | G99M | 0.599 | 0.182 | 0.291 | 0.111 | 2.057 | 1.640 | 8 | 1040 |
| LC | G99N | 0.611 | 0.154 | 0.373 | 0.124 | 1.639 | 1.235 | 8 | 1041 |
| LC | G99S | 1.517 | 0.525 | 1.365 | 0.236 | 1.112 | 2.226 | 8 | 1042 |
| LC | G99T | 1.203 | 0.307 | 0.812 | 0.173 | 1.488 | 1.783 | 8 | 1043 |
| LC | G99V | 0.701 | 0.186 | 0.431 | 0.105 | 1.631 | 1.768 | 8 | 1044 |
| LC | G99K | 0.360 | 0.120 | 0.203 | 0.042 | 1.793 | 1.481 | 8 | 1045 |
| LC | G99H | 0.496 | 0.126 | 0.346 | 0.069 | 1.440 | 1.831 | 8 | 1046 |
| LC | Q100C | 2.836 | 1.308 | 2.238 | 0.619 | 1.278 | 2.113 | 8 | 1047 |

(continued)

| Table 15. Variant anti-EGFR antibodies | | | | | | | | | |
|---|---|---|---|---|---|---|---|---|---|
| | | Average OD (pH 6.0) $(OD_{6.0})$ | | Average OD (pH 7.4 $(OD_{7.4})$ | | $OD_{6.0}$ / $OD_{7.4}$ | | SEQ ID NO | |
| Chain | Mutation | Dilution 1 | Dilution 2 | Dilution 1 | Dilution 2 | Dilution 1 | Dilution 2 | HC | LC |
| LC | Q100D | 3.035 | 2.136 | 3.057 | 1.429 | 0.993 | 1.495 | 8 | 1048 |
| LC | Q100E | 2.932 | 1.985 | 2.880 | 1.120 | 1.018 | 1.773 | 8 | 1049 |
| LC | Q100F | 3.039 | 2.002 | 2.863 | 1.155 | 1.061 | 1.736 | 8 | 1050 |
| LC | Q100I | 2.917 | 1.641 | 2.727 | 0.974 | 1.070 | 1.685 | 8 | 1051 |
| LC | Q100M | 3.079 | 1.799 | 2.753 | 1.005 | 1.119 | 1.802 | 8 | 1052 |
| LC | Q100N | 3.113 | 2.782 | 3.138 | 2.163 | 0.992 | 1.287 | 8 | 1053 |
| LC | Q100P | 3.072 | 2.357 | 3.146 | 1.497 | 0.977 | 1.575 | 8 | 1054 |
| LC | Q100T | 3.064 | 2.278 | 2.950 | 0.612 | 1.039 | 1.778 | 8 | 1055 |
| LC | Q100V | 3.095 | 2.148 | 2.942 | 1.292 | 1.052 | 1.671 | 8 | 1056 |
| LC | Q100W | 2.873 | 1.702 | 2.757 | 0.853 | 1.043 | 2.000 | 8 | 1057 |
| LC | Q100Y | 3.170 | 2.395 | 3.173 | 1.671 | 0.999 | 1.439 | 8 | 1058 |
| LC | Q100K | 3.076 | 2.031 | 2.852 | 1.110 | 1.078 | 1.834 | 8 | 1059 |
| LC | Q100H | 3.096 | 2.050 | 2.942 | 1.261 | 1.053 | 1.628 | 8 | 1060 |
| LC | Q100R | 2.930 | 1.902 | 2.831 | 1.030 | 1.035 | 1.846 | 8 | 1061 |
| [a] HC = Heavy Chain; LC = Light Chain [b] V = variable | | | | | | | | | |

### d. Determining Antibody Concentration

The antibody concentration was determined by anti-EGFR antibody quantitation ELISA. Briefly, plates were coated with 100 μL sEGFR-H6 (Sino Biologicals Inc, Cat# 10001-H08H) antigen at 12nM (1.32ug/mL) in PBS; washed three times with 250 μl / well of PBS; and blocked for 1 hour at room temperature with 250 μl of PBS with 5 mg/mL BSA. Serial dilutions of anti-EGFR-FLAG antibody standards (protein A column purified) were prepared in PBS with 5 mg/mL BSA. The starting antibody concentration was 100 ng/mL followed by 1:3 dilutions as specified. Test sample dilutions were prepared (1:3 dilutions), and 100 μl / well of standard and test sample were added to wells and incubated at room temperature for 1hr. Plates were washed 3x with 250 μl/well of PBS with 5 mg/mL BSA. 100 μL/well rabbit anti-human IgG-Fc-HRP was added at 1:5000 (final concentration 0.2 μL/mL) dilution in PBS/ 5mg/mL BSA. The plate was incubated for 1hr at RT; washed 3x with 250 μl / well of PBS/ 5mg/mL BSA. TMB Substrate was added and plates were read as described above.

### e. Calculating Specific Activity

The specific activity (SA) was calculated by dividing the average OD value by the antibody concentration. The specific activity was then normalized to give a normalized specific activity (NSA) for each variant by dividing the specific activity of the variant anti-EGFR antibody by the specific activity of the reference FLAG-tagged anti-EGFR parental antibody. Table 16 sets forth the normalized specific activity of each identified variant set forth above at dilution 1 and dilution 2. The variant anti-EGFR antibodies with an NSA > 0.4 at pH 6.0 and an NSA < 0.4 at pH 7.4 were identified and selected for further analysis. The mutations of these identified antibodies are antibodies containing light chain (LC) mutations: L004C, L004V, S056H or N091V; and antibodies containing heavy chain (HC) mutations: V024I, V024L, S025C, S025G, S025I, S025Q, S025T, S025L, N031I, N031T, N031V, Y032T, V050L, G054R, G054C, G054P, D058M, Y059E, F063R, F063C, F063G, F063M, F063V, T064N, T064V, S068F, S068Q, D072K, D072L, D072M, D072W, N073Q, S074H, S074R, S074D, S074G, S074Y, K075H, K075W, Q077R, Q077E, T100I, T100P, Y101W, Y104D, Y104F, Y104S or A107N.

| | | Normalized Specific Activity (NSA) (pH 6.0) | | Normalized Specific Activity (NSA) (pH 7.4) | |
|---|---|---|---|---|---|
| **Table 16. Normalized Specific Activity at pH 6.0 and pH 7.4 of Variant Anti-EGFR Antibodies.** | | | | | |
| **Chain[a]** | **Mutation** | **Dilution 1** | **Dilution 2** | **Dilution 1** | **Dilution 2** |
| HC | T23K | 1.020 | 2.018 | 0.986 | 1.435 |
| HC | T23H | 1.889 | 5.343 | 1.940 | 3.453 |
| HC | T23R | 1.057 | 2.664 | 1.048 | 1.587 |
| HC | T23A | 1.238 | 2.459 | 1.304 | 1.297 |
| HC | T23C | 1.073 | 2.651 | 1.096 | 1.673 |
| HC | T23E | 1.255 | 2.947 | 1.406 | 1.751 |
| HC | T23G | 1.057 | 1.084 | 0.727 | 0.627 |
| HC | T23I | 1.097 | 2.851 | 1.126 | 1.975 |
| HC | T23M | 0.546 | 0.946 | 0.494 | 0.702 |
| HC | T23N | 1.062 | 2.207 | 0.888 | 1.504 |
| HC | T23P | 0.118 | 0.235 | 0.079 | 0.273 |
| HC | T23S | 0.750 | 2.908 | 1.087 | 1.902 |
| HC | T23V | 0.117 | 0.329 | 0.097 | 0.290 |
| HC | T23W | 3.143 | 5.871 | 2.981 | 2.441 |
| HC | T23L | 3.495 | 3.868 | 2.889 | 2.277 |
| HC | V24R | 0.103 | 0.096 | 0.092 | 0.413 |
| HC | V24A | 0.600 | 0.307 | 0.439 | 0.525 |
| HC | V24F | 0.861 | 0.233 | 0.428 | 0.622 |
| HC | V24G | 0.525 | 0.323 | 0.461 | 0.614 |
| HC | V24I | 1.049 | 0.305 | 0.381 | 0.568 |
| HC | V24M | 1.007 | 0.348 | 0.490 | 0.653 |
| HC | V24P | 2.209 | 0.566 | 0.766 | 1.234 |
| HC | V24S | 0.657 | 0.337 | 0.460 | 0.581 |
| HC | V24T | 1.001 | 0.315 | 0.430 | 0.543 |
| HC | V24L | 0.551 | 0.176 | 0.145 | 0.245 |
| HC | S25H | 0.389 | 0.227 | 0.167 | 0.207 |
| HC | S25R | 0.397 | 0.175 | 0.237 | 0.255 |
| HC | S25A | 0.344 | 0.239 | 0.238 | 0.310 |
| HC | S25C | 0.564 | 0.262 | 0.365 | 0.423 |
| HC | S25D | 0.364 | 0.203 | 0.267 | 0.351 |
| HC | S25E | 0.365 | 0.142 | 0.321 | 0.400 |
| HC | S25F | 0.350 | 0.196 | 0.323 | 0.450 |
| HC | S25G | 0.573 | 0.237 | 0.228 | 0.317 |
| HC | S25I | 0.556 | 0.397 | 0.374 | 0.464 |
| HC | S25M | 0.320 | 0.248 | 0.288 | 0.394 |

(continued)

| Table 16. Normalized Specific Activity at pH 6.0 and pH 7.4 of Variant Anti-EGFR Antibodies. | | | | | |
|---|---|---|---|---|---|
| Chain[a] | Mutation | Normalized Specific Activity (NSA) (pH 6.0) | | Normalized Specific Activity (NSA) (pH 7.4) | |
| | | Dilution 1 | Dilution 2 | Dilution 1 | Dilution 2 |
| HC | S25P | 2.362 | 0.630 | 0.673 | 1.444 |
| HC | S25Q | 1.319 | 0.347 | 0.335 | 0.493 |
| HC | S25T | 0.648 | 0.118 | 0.353 | 0.192 |
| HC | S25V | 0.300 | 0.229 | 0.221 | 0.381 |
| HC | S25L | 0.504 | 0.283 | 0.267 | 0.361 |
| HC | G26H | 2.071 | 3.143 | 1.536 | 2.105 |
| HC | G26R | 1.821 | 2.842 | 1.633 | 1.911 |
| HC | G26D | 1.456 | 2.488 | 1.123 | 1.696 |
| HC | G26F | 0.672 | 1.242 | 0.444 | 0.925 |
| HC | G26M | 1.674 | 2.875 | 1.284 | 1.905 |
| HC | G26N | 0.705 | 2.325 | 0.858 | 1.765 |
| HC | G26P | 1.501 | 3.333 | 1.883 | 2.791 |
| HC | G26Q | 1.275 | 2.310 | 1.208 | 1.657 |
| HC | G26S | 1.298 | 2.234 | 1.322 | 1.625 |
| HC | G26Y | 1.638 | 2.491 | 1.170 | 1.692 |
| HC | G26L | 4.237 | 6.512 | 3.218 | 5.644 |
| HC | F27H | 1.267 | 0.291 | 0.713 | 1.059 |
| HC | F27R | 1.285 | 0.810 | 0.747 | 1.151 |
| HC | F27A | 0.643 | 0.485 | 0.504 | 0.864 |
| HC | F27D | 0.572 | 0.335 | 0.513 | 0.984 |
| HC | F27E | 0.500 | 0.234 | 0.444 | 0.836 |
| HC | F27G | 0.777 | 0.718 | 0.522 | 0.808 |
| HC | F27M | 0.723 | 0.304 | 0.501 | 0.512 |
| HC | F27P | 0.834 | 0.293 | 0.506 | 0.790 |
| HC | F27Q | 0.872 | 0.500 | 0.630 | 0.890 |
| HC | F27S | 1.248 | 0.334 | 0.620 | 0.852 |
| HC | F27T | 0.710 | 0.311 | 0.482 | 0.681 |
| HC | F27V | 1.758 | 0.882 | 0.781 | 1.205 |
| HC | F27W | 1.371 | 0.344 | 0.639 | 0.972 |
| HC | F27Y | 0.857 | 0.278 | 0.409 | 0.493 |
| HC | F27L | 0.813 | 0.256 | 0.523 | 0.800 |
| HC | S28K | 0.671 | 2.281 | 0.757 | 1.520 |
| HC | S28H | 1.188 | 2.106 | 0.969 | 1.332 |
| HC | S28R | 0.978 | 2.223 | 1.022 | 1.669 |
| HC | S28A | 1.412 | 2.739 | 1.162 | 1.534 |

(continued)

| Table 16. Normalized Specific Activity at pH 6.0 and pH 7.4 of Variant Anti-EGFR Antibodies. | | | | | |
|---|---|---|---|---|---|
| Chain[a] | Mutation | Normalized Specific Activity (NSA) (pH 6.0) | | Normalized Specific Activity (NSA) (pH 7.4) | |
| | | Dilution 1 | Dilution 2 | Dilution 1 | Dilution 2 |
| HC | S28D | 0.602 | 1.096 | 0.437 | 0.700 |
| HC | S28I | 1.363 | 3.128 | 1.289 | 1.859 |
| HC | S28M | 2.402 | 4.485 | 2.098 | 3.151 |
| HC | S28P | 1.701 | 3.628 | 1.498 | 2.339 |
| HC | S28Q | 1.048 | 2.480 | 1.030 | 1.642 |
| HC | S28V | 0.972 | 2.638 | 1.049 | 1.913 |
| HC | S28W | 0.814 | 2.190 | 0.823 | 1.423 |
| HC | S28L | 2.175 | 8.593 | 1.970 | 2.977 |
| HC | L29K | 0.392 | 1.111 | 0.282 | 0.698 |
| HC | L29H | 0.338 | 0.911 | 0.238 | 0.475 |
| HC | L29A | 1.181 | 2.332 | 1.142 | 1.769 |
| HC | L29D | 0.388 | 0.893 | 0.359 | 0.897 |
| HC | L29G | 2.475 | 3.360 | 2.240 | 3.922 |
| HC | L29I | 0.406 | 1.498 | 0.517 | 1.142 |
| HC | L29M | 1.067 | 2.477 | 1.252 | 2.170 |
| HC | L29N | 0.845 | 3.909 | 0.860 | 2.538 |
| HC | L29S | 0.684 | 1.545 | 0.497 | 1.088 |
| HC | L29V | 0.352 | 0.930 | 0.278 | 0.735 |
| HC | T30H | 1.092 | 2.120 | 1.109 | 1.430 |
| HC | T30R | 0.592 | 1.454 | 0.718 | 0.994 |
| HC | T30D | 1.159 | 2.336 | 1.122 | 1.588 |
| HC | T30G | 0.749 | 1.508 | 0.822 | 1.140 |
| HC | T30I | 1.025 | 2.917 | 1.082 | 1.502 |
| HC | T30M | 0.700 | 1.280 | 0.797 | 0.834 |
| HC | T30N | 1.065 | 1.917 | 1.093 | 1.311 |
| HC | T30P | 1.064 | 1.607 | 0.996 | 1.397 |
| HC | T30S | 0.786 | 1.636 | 0.929 | 1.233 |
| HC | T30V | 0.728 | 1.567 | 0.912 | 0.991 |
| HC | T30W | 1.003 | 1.900 | 0.927 | 1.169 |
| HC | T30Y | 0.708 | 1.688 | 0.797 | 1.561 |
| HC | N31K | 0.352 | 0.248 | 0.253 | 0.400 |
| HC | N31H | 0.317 | 0.212 | 0.201 | 0.280 |
| HC | N31D | 0.794 | 0.331 | 0.483 | 0.587 |
| HC | N31E | 0.203 | 0.184 | 0.173 | 0.517 |
| HC | N31G | 0.506 | 0.301 | 0.414 | 0.613 |

(continued)

| Table 16. Normalized Specific Activity at pH 6.0 and pH 7.4 of Variant Anti-EGFR Antibodies. | | | | | |
|---|---|---|---|---|---|
| Chain[a] | Mutation | Normalized Specific Activity (NSA) (pH 6.0) | | Normalized Specific Activity (NSA) (pH 7.4) | |
| | | Dilution 1 | Dilution 2 | Dilution 1 | Dilution 2 |
| HC | N31I | 0.933 | 0.303 | 0.333 | 0.435 |
| HC | N31T | 0.503 | 0.125 | 0.370 | 0.472 |
| HC | N31V | 0.763 | 0.281 | 0.318 | 0.197 |
| HC | N31L | 0.659 | 0.222 | 0.461 | 0.665 |
| HC | Y32H | 0.609 | 1.470 | 0.552 | 1.002 |
| HC | Y32R | 0.943 | 1.707 | 1.059 | 1.413 |
| HC | Y32C | 0.703 | 1.348 | 0.573 | 1.284 |
| HC | Y32M | 0.878 | 1.559 | 0.859 | 1.345 |
| HC | Y32N | 0.500 | 1.335 | 0.420 | 0.483 |
| HC | Y32T | 0.455 | 0.953 | 0.376 | 0.759 |
| HC | Y32V | 0.742 | 1.043 | 0.672 | 1.448 |
| HC | Y32L | 1.385 | 2.612 | 1.287 | 1.974 |
| HC | G33E | 1.207 | 2.300 | 0.931 | 0.950 |
| HC | G33M | 2.556 | 3.457 | 1.992 | 2.807 |
| HC | G33S | 0.801 | 3.041 | 0.790 | 2.418 |
| HC | G33T | 1.148 | 1.830 | 0.970 | 1.767 |
| HC | G33Y | 1.133 | 4.983 | 1.165 | 4.590 |
| HC | V34A | 2.444 | 4.254 | 1.716 | 3.125 |
| HC | V34C | 0.372 | 1.062 | 0.558 | 0.573 |
| HC | V34I | 1.073 | 2.295 | 1.141 | 1.650 |
| HC | V34M | 0.434 | 1.212 | 0.467 | 0.835 |
| HC | V34P | 0.692 | 3.135 | 0.920 | 1.994 |
| HC | V34L | 1.696 | 3.294 | 1.682 | 2.238 |
| HC | H35I | 2.905 | 96.092 | 3.285 | 16.573 |
| HC | H35Q | 4.926 | 6.014 | 3.510 | 6.052 |
| HC | W36K | 0.617 | 2.808 | 0.790 | 1.992 |
| HC | W36A | 2.841 | 4.005 | 2.076 | 3.920 |
| HC | W36I | 0.588 | 1.638 | 0.729 | 1.452 |
| HC | W36V | 0.864 | 2.274 | 1.114 | 1.961 |
| HC | W36Y | 1.484 | 2.844 | 1.570 | 2.454 |
| HC | V50K | 0.043 | 0.244 | 0.042 | 0.212 |
| HC | V50H | 1.916 | 3.631 | 1.774 | 2.742 |
| HC | V50A | 1.390 | 3.507 | 1.143 | 2.243 |
| HC | V50D | 1.070 | 1.811 | 0.702 | 1.284 |
| HC | V50E | 1.239 | 2.253 | 1.061 | 2.100 |

(continued)

| Table 16. Normalized Specific Activity at pH 6.0 and pH 7.4 of Variant Anti-EGFR Antibodies. | | | | | |
|---|---|---|---|---|---|
| Chain[a] | Mutation | Normalized Specific Activity (NSA) (pH 6.0) | | Normalized Specific Activity (NSA) (pH 7.4) | |
| | | Dilution 1 | Dilution 2 | Dilution 1 | Dilution 2 |
| HC | V50G | 0.844 | 1.656 | 0.669 | 1.261 |
| HC | V50I | 4.049 | 5.405 | 1.466 | 2.398 |
| HC | V50N | 1.053 | 1.512 | 0.471 | 0.700 |
| HC | V50Q | 2.666 | 4.891 | 1.815 | 2.384 |
| HC | V50T | 0.738 | 3.047 | 0.743 | 2.675 |
| HC | V50L | 0.440 | 0.735 | 0.191 | 0.205 |
| HC | I51K | 2.359 | 4.025 | 1.370 | 1.844 |
| HC | I51H | 3.723 | 14.555 | 1.822 | 2.338 |
| HC | I51A | 1.534 | 2.984 | 0.736 | 0.886 |
| HC | I51C | 1.765 | 2.990 | 0.932 | 1.094 |
| HC | I51E | 3.724 | 5.988 | 2.106 | 3.947 |
| HC | I51G | 5.223 | 8.038 | 1.801 | 3.716 |
| HC | I51N | 2.514 | 3.995 | 1.257 | 1.529 |
| HC | I51Q | 0.877 | 2.625 | 0.777 | 1.184 |
| HC | I51S | 1.255 | 2.857 | 0.936 | 1.196 |
| HC | I51V | 0.562 | 2.392 | 0.568 | 1.375 |
| HC | I51Y | 5.123 | 7.721 | 3.253 | 5.278 |
| HC | I51L | 0.874 | 3.046 | 0.818 | 1.280 |
| HC | W52I | 0.965 | 1.402 | 0.751 | 1.419 |
| HC | W52N | 0.592 | 1.877 | 0.774 | 1.549 |
| HC | W52Y | 0.981 | 3.959 | 1.220 | 2.463 |
| HC | S53H | 0.417 | 1.759 | 0.410 | 1.184 |
| HC | S53R | 1.583 | 2.285 | 1.370 | 1.749 |
| HC | S53A | 0.348 | 1.680 | 0.344 | 1.338 |
| HC | S53C | 0.908 | 2.714 | 0.953 | 2.487 |
| HC | S53G | 0.223 | 1.081 | 0.225 | 0.949 |
| HC | S53I | 0.384 | 1.723 | 0.382 | 1.274 |
| HC | S53M | 1.206 | 3.172 | 1.147 | 1.947 |
| HC | S53P | 0.669 | 2.501 | 0.662 | 1.757 |
| HC | S53Q | 1.381 | 2.721 | 0.940 | 0.979 |
| HC | S53L | 0.900 | 3.262 | 0.910 | 2.425 |
| HC | S53T | 0.419 | 1.674 | 0.450 | 0.671 |
| HC | S53V | 0.545 | 1.894 | 0.552 | 1.373 |
| HC | S53Y | 0.344 | 1.426 | 0.334 | 1.031 |
| HC | G54H | 0.543 | 0.241 | 0.445 | 0.850 |

(continued)

| Table 16. Normalized Specific Activity at pH 6.0 and pH 7.4 of Variant Anti-EGFR Antibodies. | | | | | |
|---|---|---|---|---|---|
| Chain[a] | Mutation | Normalized Specific Activity (NSA) (pH 6.0) | | Normalized Specific Activity (NSA) (pH 7.4) | |
| | | Dilution 1 | Dilution 2 | Dilution 1 | Dilution 2 |
| HC | G54R | 0.462 | 0.148 | 0.345 | 0.524 |
| HC | G54A | 0.304 | 0.185 | 0.268 | 0.519 |
| HC | G54C | 0.567 | 0.120 | 0.375 | 0.423 |
| HC | G54D | 0.377 | 0.163 | 0.279 | 0.387 |
| HC | G54P | 0.530 | 0.175 | 0.307 | 0.430 |
| HC | G54S | 0.376 | 0.106 | 0.195 | 0.341 |
| HC | G55H | 0.391 | 1.139 | 0.590 | 0.968 |
| HC | G55R | 0.342 | 1.464 | 0.563 | 1.211 |
| HC | G55M | 0.338 | 0.797 | 0.509 | 0.715 |
| HC | G55S | 0.335 | 0.715 | 0.480 | 0.548 |
| HC | G55Y | 0.530 | 0.664 | 0.472 | 0.679 |
| HC | N56K | 0.782 | 1.706 | 0.754 | 1.399 |
| HC | N56A | 1.330 | 2.417 | 0.785 | 0.816 |
| HC | N56P | 4.302 | 21.666 | 2.925 | 3.906 |
| HC | N56S | 1.096 | 2.358 | 0.854 | 1.178 |
| HC | N56V | 0.938 | 1.863 | 0.843 | 1.374 |
| HC | N56G | 1.586 | 2.626 | 0.854 | 1.056 |
| HC | T57H | 1.322 | 2.243 | 0.789 | 1.007 |
| HC | T57R | 0.609 | 1.872 | 0.571 | 1.152 |
| HC | T57L | 0.795 | 2.304 | 0.711 | 1.287 |
| HC | T57A | 0.913 | 3.028 | 0.822 | 1.533 |
| HC | T57C | 0.868 | 2.235 | 0.729 | 1.097 |
| HC | T57D | 2.819 | 3.335 | 1.244 | 1.571 |
| HC | T57F | 0.477 | 1.873 | 0.446 | 1.312 |
| HC | T57M | 1.025 | 2.955 | 0.931 | 0.830 |
| HC | T57N | 1.244 | 2.328 | 0.872 | 1.091 |
| HC | T57Q | 1.019 | 2.576 | 0.859 | 1.226 |
| HC | T57W | 0.744 | 1.996 | 0.636 | 0.832 |
| HC | T57Y | 0.572 | 1.829 | 0.528 | 1.233 |
| HC | D58L | 0.494 | 1.142 | 0.642 | 0.909 |
| HC | D58G | 0.733 | 1.998 | 0.620 | 1.097 |
| HC | D58M | 0.527 | 0.975 | 0.380 | 0.687 |
| HC | D58N | 0.602 | 1.969 | 0.544 | 1.330 |
| HC | D58Q | 0.879 | 1.255 | 0.450 | 0.681 |
| HC | Y59H | 0.664 | 1.120 | 0.711 | 0.837 |

(continued)

| Table 16. Normalized Specific Activity at pH 6.0 and pH 7.4 of Variant Anti-EGFR Antibodies. | | | | | |
|---|---|---|---|---|---|
| Chain[a] | Mutation | Normalized Specific Activity (NSA) (pH 6.0) | | Normalized Specific Activity (NSA) (pH 7.4) | |
| | | Dilution 1 | Dilution 2 | Dilution 1 | Dilution 2 |
| HC | Y59R | 0.317 | 0.937 | 0.491 | 0.829 |
| HC | Y59A | 0.688 | 0.847 | 0.504 | 0.670 |
| HC | Y59C | 0.581 | 0.976 | 0.673 | 0.791 |
| HC | Y59D | 2.044 | 2.694 | 1.189 | 2.433 |
| HC | Y59E | 0.618 | 1.008 | 0.525 | 0.351 |
| HC | Y59G | 0.337 | 1.012 | 0.511 | 0.905 |
| HC | Y59I | 0.334 | 1.053 | 0.422 | 0.955 |
| HC | Y59P | 3.607 | 5.871 | 1.953 | 7.045 |
| HC | Y59Q | 0.438 | 1.039 | 0.583 | 0.710 |
| HC | Y59S | 0.340 | 0.895 | 0.514 | 0.731 |
| HC | Y59T | 0.395 | 1.082 | 0.567 | 0.770 |
| HC | Y59V | 0.559 | 1.032 | 0.625 | 0.773 |
| HC | Y59W | 0.488 | 1.054 | 0.600 | 0.712 |
| HC | N60K | 0.456 | 1.285 | 0.595 | 1.149 |
| HC | N60A | 0.432 | 1.286 | 0.646 | 1.134 |
| HC | N60C | 1.097 | 1.578 | 1.054 | 1.345 |
| HC | N60D | 2.085 | 42.638 | 2.049 | 8.023 |
| HC | N60F | 0.656 | 1.286 | 0.634 | 0.929 |
| HC | N60G | 0.669 | 1.192 | 0.660 | 0.873 |
| HC | N60P | 1.643 | 2.562 | 1.066 | 2.171 |
| HC | N60Q | 0.967 | 1.396 | 0.871 | 1.282 |
| HC | N60S | 0.722 | 1.169 | 0.631 | 0.853 |
| HC | N60T | 0.607 | 1.193 | 0.715 | 0.973 |
| HC | N60Y | 0.514 | 1.166 | 0.739 | 1.038 |
| HC | T61N | 0.436 | 1.348 | 0.634 | 1.140 |
| HC | T61Q | 0.719 | 1.201 | 0.766 | 0.524 |
| HC | P62G | 0.674 | 1.312 | 0.780 | 1.123 |
| HC | F63H | 0.498 | 0.315 | 0.438 | 0.624 |
| HC | F63R | 0.877 | 0.251 | 0.362 | 0.490 |
| HC | F63L | 0.736 | 0.375 | 0.532 | 0.752 |
| HC | F63A | 0.956 | 0.299 | 0.418 | 0.540 |
| HC | F63C | 1.107 | 0.294 | 0.339 | 0.699 |
| HC | F63D | 0.865 | 0.370 | 0.547 | 0.678 |
| HC | F63G | 0.746 | 0.280 | 0.398 | 0.527 |
| HC | F63M | 0.490 | 0.243 | 0.347 | 0.367 |

(continued)

| Table 16. Normalized Specific Activity at pH 6.0 and pH 7.4 of Variant Anti-EGFR Antibodies. | | | | | |
|---|---|---|---|---|---|
| Chain[a] | Mutation | Normalized Specific Activity (NSA) (pH 6.0) | | Normalized Specific Activity (NSA) (pH 7.4) | |
| | | Dilution 1 | Dilution 2 | Dilution 1 | Dilution 2 |
| HC | F63N | 2.041 | 0.601 | 1.138 | 0.713 |
| HC | F63Q | 0.732 | 0.297 | 0.419 | 0.482 |
| HC | F63S | 0.352 | 0.123 | 0.188 | 0.101 |
| HC | F63V | 0.514 | 0.269 | 0.364 | 0.415 |
| HC | T64R | 0.182 | 0.570 | 0.297 | 0.474 |
| HC | T64L | 0.298 | 1.068 | 0.467 | 0.919 |
| HC | T64C | 0.516 | 1.008 | 0.703 | 0.933 |
| HC | T64F | 0.693 | 1.832 | 0.598 | 3.168 |
| HC | T64G | 0.172 | 0.535 | 0.284 | 0.451 |
| HC | T64N | 0.493 | 1.405 | 0.331 | 1.574 |
| HC | T64Q | 0.540 | 0.941 | 0.562 | 0.746 |
| HC | T64V | 0.299 | 0.675 | 0.374 | 0.238 |
| HC | S65H | 0.325 | 1.329 | 0.335 | 0.814 |
| HC | S65R | 0.396 | 1.420 | 0.395 | 0.663 |
| HC | S65L | 0.139 | 0.596 | 0.143 | 0.404 |
| HC | S65C | 0.585 | 2.535 | 0.557 | 1.236 |
| HC | S65E | 0.508 | 1.810 | 0.500 | 1.056 |
| HC | S65F | 0.452 | 1.653 | 0.440 | 1.031 |
| HC | S65G | 0.254 | 1.139 | 0.264 | 0.944 |
| HC | S65I | 0.558 | 1.828 | 0.539 | 1.062 |
| HC | S65M | 0.556 | 1.700 | 0.513 | 0.874 |
| HC | S65N | 0.483 | 1.706 | 0.454 | 0.825 |
| HC | S65P | 0.974 | 2.084 | 0.684 | 0.842 |
| HC | S65Q | 0.492 | 1.734 | 0.472 | 0.880 |
| HC | S65T | 0.680 | 1.921 | 0.615 | 1.012 |
| HC | S65W | 0.380 | 1.476 | 0.380 | 0.924 |
| HC | S65Y | 0.329 | 1.393 | 0.329 | 0.990 |
| HC | R66L | 0.671 | 1.783 | 0.588 | 0.671 |
| HC | R66A | 0.998 | 2.099 | 0.814 | 0.996 |
| HC | R66C | 1.915 | 3.034 | 0.953 | 1.308 |
| HC | R66E | 1.811 | 3.037 | 1.102 | 1.597 |
| HC | R66F | 1.562 | 1.318 | 0.751 | 0.814 |
| HC | R66N | 0.935 | 1.661 | 0.709 | 0.995 |
| HC | R66P | 0.974 | 1.756 | 0.631 | 1.267 |
| HC | R66Q | 0.852 | 2.010 | 0.669 | 0.999 |

(continued)

| Chain[a] | Mutation | Normalized Specific Activity (NSA) (pH 6.0) | | Normalized Specific Activity (NSA) (pH 7.4) | |
|---|---|---|---|---|---|
| | | Dilution 1 | Dilution 2 | Dilution 1 | Dilution 2 |
| HC | R66S | 0.900 | 1.594 | 0.619 | 0.658 |
| HC | R66T | 0.877 | 1.430 | 0.463 | 0.871 |
| HC | R66V | 0.936 | 2.491 | 0.893 | 0.761 |
| HC | R66G | 2.068 | 2.616 | 0.897 | 1.132 |
| HC | L67A | 0.980 | 2.026 | 0.702 | 0.889 |
| HC | L67C | 0.634 | 1.857 | 0.545 | 0.697 |
| HC | L67D | 0.033 | 0.125 | 0.026 | 0.120 |
| HC | L67E | 1.164 | 2.276 | 0.819 | 1.129 |
| HC | L67I | 0.879 | 1.748 | 0.589 | 0.706 |
| HC | L67M | 0.953 | 2.144 | 0.719 | 0.955 |
| HC | L67Q | 1.865 | 2.571 | 0.940 | 1.249 |
| HC | L67S | 0.925 | 2.316 | 0.769 | 0.996 |
| HC | L67T | 1.099 | 2.115 | 0.667 | 0.765 |
| HC | L67V | 1.443 | 2.291 | 0.723 | 0.940 |
| HC | L67Y | 0.852 | 2.598 | 0.792 | 0.633 |
| HC | S68K | 0.351 | 1.123 | 0.329 | 0.607 |
| HC | S68H | 1.426 | 5.677 | 1.487 | 3.076 |
| HC | S68R | 0.352 | 1.259 | 0.340 | 0.643 |
| HC | S68L | 0.862 | 2.245 | 0.715 | 0.945 |
| HC | S68C | 0.340 | 1.251 | 0.340 | 0.675 |
| HC | S68D | 0.661 | 1.879 | 0.600 | 0.439 |
| HC | S68E | 0.443 | 1.435 | 0.418 | 0.710 |
| HC | S68F | 0.713 | 1.782 | 0.396 | 1.344 |
| HC | S68G | 0.704 | 1.879 | 0.573 | 0.969 |
| HC | S68I | 0.608 | 1.796 | 0.558 | 0.775 |
| HC | S68N | 0.456 | 1.188 | 0.414 | 0.580 |
| HC | S68Q | 0.138 | 0.444 | 0.135 | 0.238 |
| HC | S68T | 0.288 | 1.183 | 0.295 | 0.805 |
| HC | S68V | 0.494 | 1.355 | 0.440 | 0.629 |
| HC | I69A | 2.116 | 3.280 | 1.694 | 3.009 |
| HC | I69C | 0.977 | 1.481 | 1.075 | 1.235 |
| HC | I69G | 2.582 | 15.344 | 2.793 | 10.186 |
| HC | I69Y | 1.759 | 2.635 | 1.624 | 3.153 |
| HC | N70H | 1.429 | 3.411 | 0.909 | 1.563 |
| HC | N70R | 3.154 | 3.380 | 1.291 | 1.930 |

(continued)

| Table 16. Normalized Specific Activity at pH 6.0 and pH 7.4 of Variant Anti-EGFR Antibodies. | | | | | |
|---|---|---|---|---|---|
| Chain[a] | Mutation | Normalized Specific Activity (NSA) (pH 6.0) | | Normalized Specific Activity (NSA) (pH 7.4) | |
| | | Dilution 1 | Dilution 2 | Dilution 1 | Dilution 2 |
| HC | N70L | 1.429 | 3.144 | 1.023 | 1.394 |
| HC | N70D | 3.706 | 5.415 | 1.729 | 2.720 |
| HC | N70E | 1.457 | 3.831 | 1.105 | 1.664 |
| HC | N70F | 0.867 | 2.802 | 0.810 | 1.857 |
| HC | N70G | 1.321 | 3.008 | 1.064 | 1.522 |
| HC | N70I | 1.764 | 3.564 | 1.075 | 1.597 |
| HC | N70P | 1.315 | 15.234 | 2.622 | 8.199 |
| HC | N70Q | 1.347 | 3.162 | 1.011 | 1.838 |
| HC | N70S | 1.059 | 3.405 | 0.978 | 0.825 |
| HC | N70T | 1.246 | 3.260 | 0.987 | 1.481 |
| HC | N70V | 0.071 | 3.033 | 0.851 | 1.850 |
| HC | N70Y | 1.291 | 3.180 | 0.849 | 1.649 |
| HC | K71H | 1.177 | 2.348 | 0.919 | 1.275 |
| HC | K71R | 1.746 | 2.774 | 1.135 | 1.501 |
| HC | K71L | 1.026 | 2.924 | 0.942 | 2.108 |
| HC | K71A | 2.515 | 3.244 | 1.558 | 2.316 |
| HC | K71C | 1.157 | 3.410 | 0.947 | 1.926 |
| HC | K71F | 0.767 | 1.728 | 0.731 | 1.477 |
| HC | K71G | 1.641 | 2.750 | 1.201 | 1.536 |
| HC | K71Q | 1.185 | 2.462 | 1.039 | 2.148 |
| HC | K71S | 1.258 | 2.360 | 1.046 | 1.419 |
| HC | K71T | 1.657 | 2.713 | 1.128 | 0.999 |
| HC | K71V | 1.077 | 2.833 | 0.970 | 1.617 |
| HC | K71W | 0.961 | 2.491 | 0.899 | 1.878 |
| HC | K71Y | 0.777 | 2.371 | 0.690 | 1.677 |
| HC | D72K | 0.483 | 1.078 | 0.382 | 1.374 |
| HC | D72H | 0.610 | 1.573 | 0.529 | 0.685 |
| HC | D72R | 0.299 | 0.897 | 0.287 | 0.424 |
| HC | D72L | 0.353 | 1.304 | 0.166 | 0.308 |
| HC | D72A | 0.760 | 1.625 | 0.783 | 1.435 |
| HC | D72G | 1.060 | 1.912 | 0.631 | 0.854 |
| HC | D72I | 0.528 | 1.484 | 0.453 | 0.691 |
| HC | D72M | 0.444 | 1.080 | 0.393 | 0.444 |
| HC | D72N | 0.699 | 1.986 | 0.639 | 0.992 |
| HC | D72Q | 0.547 | 2.197 | 0.488 | 0.693 |

(continued)

| Table 16. Normalized Specific Activity at pH 6.0 and pH 7.4 of Variant Anti-EGFR Antibodies. | | | | | |
|---|---|---|---|---|---|
| Chain[a] | Mutation | Normalized Specific Activity (NSA) (pH 6.0) | | Normalized Specific Activity (NSA) (pH 7.4) | |
| | | Dilution 1 | Dilution 2 | Dilution 1 | Dilution 2 |
| HC | D72S | 0.552 | 1.649 | 0.537 | 0.821 |
| HC | D72V | 0.435 | 1.389 | 0.423 | 0.709 |
| HC | D72W | 0.537 | 1.453 | 0.213 | 0.314 |
| HC | D72Y | 0.566 | 1.469 | 0.454 | 0.642 |
| HC | N73H | 1.037 | 1.850 | 0.722 | 0.716 |
| HC | N73R | 0.877 | 1.547 | 0.531 | 0.590 |
| HC | N73L | 0.661 | 1.749 | 0.590 | 0.930 |
| HC | N73A | 1.179 | 1.975 | 0.526 | 0.777 |
| HC | N73C | 1.032 | 2.006 | 0.680 | 0.810 |
| HC | N73G | 0.960 | 1.702 | 0.622 | 0.834 |
| HC | N73I | 0.551 | 1.755 | 0.506 | 0.841 |
| HC | N73M | 0.809 | 1.639 | 0.483 | 0.649 |
| HC | N73P | 1.410 | 2.152 | 0.752 | 1.070 |
| HC | N73Q | 0.898 | 1.693 | 0.609 | 0.329 |
| HC | N73S | 1.138 | 2.107 | 0.762 | 0.943 |
| HC | N73T | 1.090 | 2.026 | 0.782 | 1.090 |
| HC | N73V | 1.339 | 1.945 | 0.743 | 0.963 |
| HC | N73W | 1.193 | 1.868 | 0.673 | 0.901 |
| HC | N73Y | 0.901 | 1.764 | 0.627 | 0.672 |
| HC | S74K | 0.672 | 1.141 | 0.430 | 0.471 |
| HC | S74H | 0.612 | 1.249 | 0.396 | 0.480 |
| HC | S74R | 0.538 | 1.169 | 0.395 | 0.455 |
| HC | S74L | 0.425 | 1.435 | 0.419 | 0.689 |
| HC | S74A | 0.672 | 1.191 | 0.454 | 0.527 |
| HC | S74C | 0.718 | 1.164 | 0.456 | 0.464 |
| HC | S74D | 0.522 | 1.199 | 0.353 | 0.419 |
| HC | S74E | 0.729 | 1.554 | 0.545 | 0.546 |
| HC | S74G | 0.570 | 2.236 | 0.346 | 0.433 |
| HC | S74I | 0.648 | 1.174 | 0.473 | 0.544 |
| HC | S74M | 0.658 | 1.228 | 0.466 | 0.494 |
| HC | S74P | 0.566 | 1.369 | 0.449 | 0.552 |
| HC | S74T | 0.559 | 1.281 | 0.456 | 0.542 |
| HC | S74V | 0.386 | 1.147 | 0.391 | 0.548 |
| HC | S74Y | 0.692 | 1.191 | 0.394 | 0.414 |
| HC | K75H | 0.447 | 1.338 | 0.396 | 0.256 |

(continued)

| Table 16. Normalized Specific Activity at pH 6.0 and pH 7.4 of Variant Anti-EGFR Antibodies. | | | | | |
|---|---|---|---|---|---|
| Chain[a] | Mutation | Normalized Specific Activity (NSA) (pH 6.0) | | Normalized Specific Activity (NSA) (pH 7.4) | |
| | | Dilution 1 | Dilution 2 | Dilution 1 | Dilution 2 |
| HC | K75R | 0.688 | 1.393 | 0.451 | 0.537 |
| HC | K75L | 0.555 | 1.058 | 0.407 | 0.620 |
| HC | K75A | 0.732 | 1.360 | 0.475 | 0.550 |
| HC | K75C | 0.915 | 1.617 | 0.477 | 0.552 |
| HC | K75E | 0.791 | 1.535 | 0.527 | 0.683 |
| HC | K75F | 0.709 | 1.374 | 0.406 | 0.453 |
| HC | K75M | 0.841 | 1.338 | 0.412 | 0.555 |
| HC | K75Q | 0.611 | 1.463 | 0.448 | 0.508 |
| HC | K75T | 0.836 | 1.280 | 0.452 | 0.561 |
| HC | K75V | 1.060 | 1.712 | 0.530 | 0.599 |
| HC | K75W | 0.932 | 1.398 | 0.390 | 0.498 |
| HC | K75Y | 0.670 | 1.338 | 0.401 | 0.450 |
| HC | S76H | 1.102 | 1.634 | 0.544 | 0.615 |
| HC | S76R | 0.960 | 1.737 | 0.498 | 0.554 |
| HC | S76L | 1.414 | 1.615 | 0.543 | 0.704 |
| HC | S76A | 0.658 | 1.242 | 0.423 | 0.743 |
| HC | S76C | 1.207 | 2.072 | 0.640 | 0.831 |
| HC | S76D | 1.224 | 4.313 | 0.577 | 0.655 |
| HC | S76E | 1.121 | 2.268 | 0.704 | 0.849 |
| HC | S76F | 0.877 | 1.662 | 0.577 | 0.652 |
| HC | S76M | 0.841 | 1.815 | 0.558 | 0.665 |
| HC | S76P | 1.362 | 2.185 | 0.637 | 0.782 |
| HC | S76Q | 1.035 | 1.633 | 0.619 | 0.824 |
| HC | S76T | 1.702 | 2.724 | 0.810 | 0.918 |
| HC | S76Y | 1.264 | 2.087 | 0.639 | 0.788 |
| HC | Q77H | 0.760 | 1.558 | 0.588 | 0.785 |
| HC | Q77R | 0.480 | 1.093 | 0.369 | 0.577 |
| HC | Q77L | 1.600 | 2.089 | 0.673 | 0.839 |
| HC | Q77A | 0.869 | 1.914 | 0.656 | 0.753 |
| HC | Q77E | 0.684 | 1.794 | 0.598 | 0.362 |
| HC | Q77G | 1.626 | 2.074 | 0.646 | 0.826 |
| HC | Q077I | 1.339 | 1.978 | 0.608 | 0.745 |
| HC | Q77M | 0.987 | 1.708 | 0.487 | 0.579 |
| HC | Q77N | 1.063 | 2.097 | 0.646 | 0.845 |
| HC | Q77S | 0.792 | 1.539 | 0.556 | 0.712 |

(continued)

| Table 16. Normalized Specific Activity at pH 6.0 and pH 7.4 of Variant Anti-EGFR Antibodies. | | | | | |
|---|---|---|---|---|---|
| Chain[a] | Mutation | Normalized Specific Activity (NSA) (pH 6.0) | | Normalized Specific Activity (NSA) (pH 7.4) | |
| | | Dilution 1 | Dilution 2 | Dilution 1 | Dilution 2 |
| HC | Q77V | 0.701 | 1.773 | 0.595 | 0.692 |
| HC | Q77W | 1.272 | 1.805 | 0.600 | 0.828 |
| HC | Q77Y | 0.967 | 1.351 | 0.525 | 0.599 |
| HC | Y93H | 2.932 | 5.071 | 2.461 | 5.308 |
| HC | Y93V | 1.325 | 2.246 | 1.209 | 2.162 |
| HC | Y93W | 1.070 | 2.602 | 0.969 | 3.648 |
| HC | Y94R | 0.194 | 0.808 | 0.302 | 0.665 |
| HC | Y94L | 0.509 | 1.104 | 0.427 | 1.011 |
| HC | R97H | 0.853 | 1.644 | 0.638 | 1.390 |
| HC | R97W | 0.106 | 0.509 | 0.195 | 0.417 |
| HC | A98P | 0.284 | 1.092 | 0.265 | 0.824 |
| HC | L99N | 0.368 | 1.014 | 0.319 | 0.975 |
| HC | L99W | 0.259 | 1.101 | 0.291 | 0.550 |
| HC | T100H | 0.350 | 1.194 | 0.345 | 1.092 |
| HC | T100L | 0.545 | 1.609 | 0.463 | 1.172 |
| HC | T100A | 0.600 | 2.227 | 0.557 | 1.628 |
| HC | T100D | 0.551 | 0.846 | 0.507 | 0.802 |
| HC | T100I | 0.510 | 1.480 | 0.382 | 1.194 |
| HC | T100N | 0.535 | 1.642 | 0.500 | 1.166 |
| HC | T100P | 0.341 | 0.526 | 0.108 | 0.246 |
| HC | T100Q | 0.521 | 1.617 | 0.504 | 1.373 |
| HC | T100S | 0.441 | 1.218 | 0.408 | 0.564 |
| HC | T100V | 0.651 | 1.967 | 0.553 | 1.302 |
| HC | T100Y | 0.724 | 1.533 | 0.607 | 1.150 |
| HC | Y101H | 0.251 | 1.091 | 0.244 | 0.826 |
| HC | Y101E | 0.016 | 0.072 | 0.017 | 0.036 |
| HC | Y101F | 1.117 | 1.976 | 0.681 | 0.890 |
| HC | Y101M | 0.523 | 1.534 | 0.488 | 1.328 |
| HC | Y101W | 0.424 | 1.078 | 0.399 | 0.491 |
| HC | Y102R | 0.025 | 0.116 | 0.020 | 0.103 |
| HC | Y102C | 0.020 | 0.084 | 0.017 | 0.086 |
| HC | Y102D | 0.018 | 0.081 | 0.016 | 0.076 |
| HC | Y102I | 0.017 | 0.072 | 0.013 | 0.065 |
| HC | Y102N | 0.017 | 0.072 | 0.013 | 0.066 |
| HC | Y102W | 0.627 | 1.446 | 0.551 | 0.956 |

(continued)

| | | Normalized Specific Activity (NSA) (pH 6.0) | | Normalized Specific Activity (NSA) (pH 7.4) | |
|---|---|---|---|---|---|
| Chain[a] | Mutation | Dilution 1 | Dilution 2 | Dilution 1 | Dilution 2 |
| HC | D103R | 0.014 | 0.050 | 0.012 | 0.053 |
| HC | D103L | 0.017 | 0.100 | 0.018 | 0.035 |
| HC | D103A | 0.669 | 0.302 | 0.604 | 1.536 |
| HC | D103C | 0.015 | 0.078 | 0.015 | 0.071 |
| HC | D103I | 0.158 | 0.657 | 0.124 | 0.648 |
| HC | D103P | 0.016 | 0.084 | 0.017 | 0.072 |
| HC | D103Q | 0.544 | 1.768 | 0.522 | 1.441 |
| HC | D103Y | 0.016 | 0.085 | 0.016 | 0.075 |
| HC | Y104H | 0.244 | 0.833 | 0.205 | 0.699 |
| HC | Y104L | 0.496 | 1.289 | 0.439 | 0.931 |
| HC | Y104D | 0.159 | 0.539 | 0.099 | 0.306 |
| HC | Y104F | 0.414 | 1.495 | 0.331 | 0.910 |
| HC | Y104I | 0.283 | 0.616 | 0.237 | 0.555 |
| HC | Y104M | 0.157 | 0.648 | 0.133 | 0.503 |
| HC | Y104S | 0.119 | 0.447 | 0.068 | 0.227 |
| HC | Y104V | 0.091 | 0.376 | 0.080 | 0.269 |
| HC | E105H | 0.149 | 0.720 | 0.226 | 0.555 |
| HC | E105T | 0.185 | 0.550 | 0.194 | 0.498 |
| HC | F106L | 0.266 | 0.740 | 0.253 | 0.636 |
| HC | F106V | 0.492 | 0.889 | 0.455 | 1.165 |
| HC | F106W | 0.537 | 0.997 | 0.575 | 0.879 |
| HC | F106Y | 0.579 | 1.578 | 0.889 | 1.385 |
| HC | A107K | 0.207 | 0.617 | 0.309 | 0.549 |
| HC | A107H | 0.182 | 0.624 | 0.280 | 0.541 |
| HC | A107R | 0.354 | 1.088 | 0.468 | 0.977 |
| HC | A107L | 0.552 | 1.866 | 0.546 | 0.774 |
| HC | A107C | 0.208 | 0.552 | 0.253 | 0.483 |
| HC | A107D | 0.237 | 0.604 | 0.225 | 0.520 |
| HC | A107E | 0.512 | 1.843 | 0.592 | 1.422 |
| HC | A107G | 0.263 | 0.877 | 0.321 | 0.738 |
| HC | A107N | 0.146 | 0.419 | 0.236 | 0.360 |
| HC | A107S | 0.258 | 0.818 | 0.428 | 0.720 |
| HC | A107T | 0.460 | 1.239 | 0.528 | 1.110 |
| HC | A107Y | 0.457 | 1.340 | 0.577 | 1.086 |
| HC | Y108K | 0.716 | 1.023 | 0.531 | 0.886 |

Table 16. Normalized Specific Activity at pH 6.0 and pH 7.4 of Variant Anti-EGFR Antibodies.

(continued)

| Chain[a] | Mutation | Normalized Specific Activity (NSA) (pH 6.0) | | Normalized Specific Activity (NSA) (pH 7.4) | |
|---|---|---|---|---|---|
| | | Dilution 1 | Dilution 2 | Dilution 1 | Dilution 2 |
| HC | Y108H | 0.426 | 1.161 | 0.518 | 0.970 |
| HC | Y108R | 1.797 | 3.006 | 1.473 | 2.831 |
| HC | Y108L | 0.578 | 1.209 | 0.600 | 0.871 |
| HC | Y108C | 0.538 | 1.044 | 0.497 | 0.879 |
| HC | Y108F | 0.491 | 1.507 | 0.688 | 1.240 |
| HC | Y108I | 0.023 | 0.053 | 0.025 | 0.042 |
| HC | Y108N | 0.582 | 1.362 | 0.535 | 1.072 |
| HC | Y108S | 0.620 | 1.458 | 0.601 | 1.018 |
| HC | Y108T | 0.487 | 1.104 | 0.458 | 0.755 |
| HC | Y108V | 0.730 | 1.321 | 0.592 | 1.263 |
| HC | Y108W | 0.559 | 1.422 | 0.539 | 1.004 |
| HC | W109I | 1.412 | 2.041 | 1.112 | 1.788 |
| HC | W109M | 0.718 | 1.364 | 0.823 | 1.101 |
| HC | W109Y | 1.076 | 1.749 | 0.989 | 1.472 |
| HC | G110R | 1.165 | 5.194 | 1.833 | 4.433 |
| HC | G110A | 0.720 | 1.560 | 0.838 | 1.427 |
| HC | G110M | 3.249 | 11.020 | 2.455 | 14.823 |
| HC | G110P | 2.292 | 4.834 | 1.970 | 5.395 |
| HC | G110T | 1.168 | 1.938 | 1.149 | 1.733 |
| HC | Q111K | 0.603 | 1.796 | 0.568 | 1.108 |
| HC | Q111H | 2.478 | 3.661 | 1.487 | 1.909 |
| HC | Q111R | 1.220 | 2.302 | 0.870 | 1.091 |
| HC | Q111L | 1.349 | 2.631 | 0.999 | 1.282 |
| HC | Q111D | 1.207 | 2.244 | 0.889 | 1.094 |
| HC | Q111E | 1.067 | 2.588 | 0.869 | 1.163 |
| HC | Q111G | 1.342 | 2.672 | 1.033 | 1.386 |
| HC | Q111M | 2.871 | 3.716 | 1.426 | 0.855 |
| HC | QUIP | 2.397 | 3.826 | 1.384 | 1.795 |
| HC | Q111S | 1.171 | 2.598 | 0.974 | 1.227 |
| HC | Q111T | 0.760 | 2.478 | 0.678 | 1.274 |
| HC | Q111W | 1.646 | 2.561 | 0.994 | 1.186 |
| HC | Q111Y | 1.385 | 2.843 | 1.005 | 1.343 |
| HC | G112A | 2.121 | 2.898 | 1.723 | 2.371 |
| HC | G112N | 1.740 | 12.480 | 1.841 | 5.701 |
| HC | G112P | 1.475 | 2.338 | 1.641 | 2.080 |

Table 16. Normalized Specific Activity at pH 6.0 and pH 7.4 of Variant Anti-EGFR Antibodies.

| Table 16. Normalized Specific Activity at pH 6.0 and pH 7.4 of Variant Anti-EGFR Antibodies. | | | | | |
|---|---|---|---|---|---|
| Chain[a] | Mutation | Normalized Specific Activity (NSA) (pH 6.0) | | Normalized Specific Activity (NSA) (pH 7.4) | |
| | | Dilution 1 | Dilution 2 | Dilution 1 | Dilution 2 |
| HC | G112S | 1.962 | 2.630 | 1.832 | 2.422 |
| HC | G112T | 2.850 | 6.175 | 2.537 | 7.080 |
| HC | G112Y | 2.473 | 5.622 | 2.147 | 6.430 |
| LC | D1W | 0.388 | 1.173 | 0.590 | 0.657 |
| LC | I2C | 0.739 | 2.597 | 0.981 | 1.004 |
| LC | I2V | 0.616 | 1.321 | 0.793 | 1.102 |
| LC | I2W | 0.715 | 0.885 | 0.844 | 0.427 |
| LC | L3D | 0.755 | 1.080 | 0.632 | 0.979 |
| LC | L3F | 0.811 | 1.170 | 0.629 | 1.131 |
| LC | L3G | 0.362 | 1.254 | 0.608 | 0.408 |
| LC | L3S | 0.668 | 0.872 | 0.578 | 0.833 |
| LC | L3T | 0.914 | 1.597 | 1.219 | 1.265 |
| LC | L3V | 0.968 | 1.999 | 1.315 | 0.949 |
| LC | L3W | 1.668 | 2.322 | 1.500 | 1.892 |
| LC | L3Y | 0.690 | 1.223 | 0.911 | 1.106 |
| LC | L3R | 0.337 | 1.002 | 0.597 | 0.733 |
| LC | L4C | 0.417 | 0.578 | 0.364 | 0.611 |
| LC | L4E | 1.177 | 1.636 | 1.114 | 1.502 |
| LC | L4F | 0.386 | 0.568 | 0.324 | 0.660 |
| LC | L4I | 0.508 | 0.738 | 0.517 | 0.661 |
| LC | L4P | 3.143 | 4.380 | 2.872 | 4.720 |
| LC | L4S | 0.594 | 0.862 | 0.518 | 0.975 |
| LC | L4T | 0.725 | 0.992 | 0.610 | 0.921 |
| LC | L4V | 0.444 | 0.613 | 0.371 | 0.275 |
| LC | L4W | 1.436 | 1.865 | 1.193 | 2.067 |
| LC | L4K | 2.048 | 2.777 | 1.767 | 2.957 |
| LC | L4H | 1.617 | 2.059 | 1.191 | 2.088 |
| LC | L4R | 1.694 | 2.439 | 1.489 | 2.087 |
| LC | T5A | 0.810 | 2.769 | 1.000 | 1.025 |
| LC | T5C | 1.935 | 2.614 | 1.631 | 2.098 |
| LC | T5D | 0.392 | 0.701 | 0.528 | 0.541 |
| LC | T5E | 0.508 | 1.285 | 0.936 | 1.092 |
| LC | T5F | 0.716 | 1.325 | 1.017 | 1.137 |
| LC | T5G | 0.756 | 1.267 | 1.011 | 1.058 |
| LC | T5N | 0.410 | 0.796 | 0.630 | 0.665 |

(continued)

| Table 16. Normalized Specific Activity at pH 6.0 and pH 7.4 of Variant Anti-EGFR Antibodies. | | | | | |
|---|---|---|---|---|---|
| Chain[a] | Mutation | Normalized Specific Activity (NSA) (pH 6.0) | | Normalized Specific Activity (NSA) (pH 7.4) | |
| | | Dilution 1 | Dilution 2 | Dilution 1 | Dilution 2 |
| LC | T5P | 0.296 | 0.440 | 0.308 | 0.450 |
| LC | T5S | 0.722 | 1.303 | 0.889 | 0.999 |
| LC | T5W | 0.866 | 1.382 | 0.972 | 1.055 |
| LC | T5L | 0.400 | 1.004 | 0.758 | 0.910 |
| LC | T5K | 0.652 | 1.202 | 0.909 | 1.026 |
| LC | T5H | 0.731 | 1.518 | 0.949 | 1.008 |
| LC | T5R | 0.436 | 1.099 | 0.665 | 0.762 |
| LC | R24A | 0.260 | 0.831 | 0.507 | 0.754 |
| LC | R24C | 1.592 | 2.429 | 1.766 | 2.150 |
| LC | R24F | 0.785 | 1.386 | 1.015 | 1.159 |
| LC | R24L | 0.494 | 1.508 | 0.974 | 1.393 |
| LC | R24M | 0.401 | 1.490 | 0.794 | 0.852 |
| LC | R24S | 0.968 | 1.941 | 1.399 | 1.573 |
| LC | R24W | 1.050 | 1.704 | 1.259 | 1.476 |
| LC | R24Y | 0.379 | 0.974 | 0.717 | 0.880 |
| LC | A25C | 1.571 | 2.505 | 2.125 | 2.024 |
| LC | A25G | 1.487 | 3.471 | 2.526 | 3.021 |
| LC | A25L | 2.166 | 2.983 | 2.459 | 2.496 |
| LC | A25V | 1.278 | 5.119 | 1.608 | 2.168 |
| LC | S26A | 0.864 | 1.323 | 0.863 | 1.156 |
| LC | S26C | 2.409 | 2.992 | 1.848 | 2.805 |
| LC | S26D | 1.717 | 10.871 | 1.367 | 2.694 |
| LC | S26I | 1.494 | 2.221 | 1.563 | 2.043 |
| LC | S26M | 1.137 | 1.543 | 0.969 | 1.392 |
| LC | S26N | 1.285 | 2.593 | 1.655 | 1.947 |
| LC | S26V | 1.567 | 2.436 | 1.269 | 2.357 |
| LC | S26W | 1.334 | 1.853 | 1.024 | 1.689 |
| LC | S26L | 1.073 | 1.903 | 1.219 | 1.668 |
| LC | S26G | 1.112 | 2.334 | 1.308 | 1.734 |
| LC | S26H | 0.557 | 1.482 | 0.924 | 1.348 |
| LC | S26R | 0.922 | 1.639 | 0.841 | 2.244 |
| LC | Q27A | 0.440 | 1.468 | 0.643 | 1.199 |
| LC | Q27D | 0.347 | 1.129 | 0.533 | 0.956 |
| LC | Q27E | 0.460 | 1.279 | 0.695 | 0.977 |
| LC | Q27F | 0.724 | 1.673 | 1.017 | 1.339 |

(continued)

| Table 16. Normalized Specific Activity at pH 6.0 and pH 7.4 of Variant Anti-EGFR Antibodies. | | | | | |
|---|---|---|---|---|---|
| Chain[a] | Mutation | Normalized Specific Activity (NSA) (pH 6.0) | | Normalized Specific Activity (NSA) (pH 7.4) | |
| | | Dilution 1 | Dilution 2 | Dilution 1 | Dilution 2 |
| LC | Q27I | 0.700 | 2.265 | 0.941 | 1.973 |
| LC | Q27M | 0.463 | 1.487 | 0.612 | 1.129 |
| LC | Q27N | 0.496 | 1.556 | 0.737 | 1.404 |
| LC | Q27P | 0.890 | 3.095 | 1.342 | 2.384 |
| LC | Q27T | 0.273 | 1.106 | 0.411 | 0.606 |
| LC | S28A | 0.930 | 1.472 | 0.889 | 1.193 |
| LC | S28D | 0.395 | 1.247 | 0.542 | 1.096 |
| LC | S28N | 0.233 | 1.039 | 0.411 | 0.749 |
| LC | S28Q | 0.350 | 0.696 | 0.466 | 0.547 |
| LC | S28L | 0.901 | 1.246 | 0.822 | 1.048 |
| LC | S28K | 0.753 | 1.147 | 0.855 | 1.056 |
| LC | S28H | 0.622 | 1.213 | 0.799 | 0.900 |
| LC | I29A | 0.426 | 1.249 | 0.570 | 1.137 |
| LC | I29E | 2.165 | 4.067 | 1.903 | 3.483 |
| LC | I29F | 1.438 | 2.842 | 1.628 | 2.610 |
| LC | I29S | 0.742 | 2.224 | 0.741 | 1.306 |
| LC | I29T | 0.575 | 1.496 | 0.733 | 1.260 |
| LC | I29R | 0.172 | 10.634 | 0.669 | 1.587 |
| LC | G30A | 0.643 | 1.440 | 0.851 | 1.236 |
| LC | G30E | 0.606 | 1.900 | 0.898 | 1.678 |
| LC | G30F | 0.938 | 1.806 | 1.064 | 1.232 |
| LC | G30I | 2.886 | 6.582 | 3.948 | 5.708 |
| LC | G30M | 0.594 | 1.535 | 0.819 | 1.364 |
| LC | G30P | 2.033 | 3.978 | 2.296 | 3.399 |
| LC | G30Q | 0.691 | 1.394 | 0.895 | 1.202 |
| LC | G30S | 0.885 | 2.369 | 1.126 | 1.755 |
| LC | G30V | 2.228 | 3.868 | 2.118 | 2.908 |
| LC | G30Y | 0.770 | 1.069 | 0.630 | 0.675 |
| LC | G30L | 1.918 | 4.755 | 2.578 | 1.786 |
| LC | G30K | 0.924 | 1.590 | 0.924 | 1.059 |
| LC | G30H | 0.649 | 1.224 | 0.809 | 1.069 |
| LC | G30R | 0.540 | 1.105 | 0.713 | 0.886 |
| LC | T31A | 0.581 | 1.710 | 0.793 | 1.213 |
| LC | T31F | 1.376 | 5.199 | 1.344 | 1.642 |
| LC | T31G | 0.617 | 1.869 | 0.816 | 1.460 |

(continued)

| Chain[a] | Mutation | Normalized Specific Activity (NSA) (pH 6.0) | | Normalized Specific Activity (NSA) (pH 7.4) | |
|---|---|---|---|---|---|
| | | Dilution 1 | Dilution 2 | Dilution 1 | Dilution 2 |
| LC | T31M | 0.639 | 2.107 | 0.898 | 1.517 |
| LC | T31S | 0.646 | 1.633 | 0.870 | 1.393 |
| LC | T31V | 0.567 | 1.498 | 0.812 | 1.275 |
| LC | T31W | 0.831 | 1.665 | 0.843 | 1.153 |
| LC | T31L | 0.889 | 1.945 | 1.066 | 1.613 |
| LC | T31K | 0.370 | 1.312 | 0.554 | 1.100 |
| LC | T31H | 0.423 | 1.446 | 0.659 | 1.196 |
| LC | N32G | 0.315 | 1.257 | 0.501 | 1.072 |
| LC | I33F | 2.925 | 4.843 | 3.859 | 4.235 |
| LC | I33G | 2.950 | 21.553 | 2.553 | 4.840 |
| LC | I33M | 1.602 | 3.012 | 2.012 | 2.650 |
| LC | I33T | 1.451 | 2.643 | 1.855 | 2.419 |
| LC | I33V | 1.020 | 2.650 | 1.502 | 2.204 |
| LC | I33H | 2.665 | 4.156 | 2.098 | 3.474 |
| LC | I48M | 0.296 | 0.926 | 0.450 | 0.755 |
| LC | I48S | 0.380 | 1.161 | 0.617 | 0.925 |
| LC | I48L | 0.401 | 1.104 | 0.537 | 0.843 |
| LC | I48K | 0.406 | 1.063 | 0.564 | 0.843 |
| LC | K49A | 0.482 | 1.910 | 0.502 | 1.306 |
| LC | K49E | 1.256 | 3.021 | 1.146 | 2.287 |
| LC | K49F | 1.447 | 4.245 | 1.325 | 2.299 |
| LC | K49G | 0.658 | 2.569 | 0.679 | 1.657 |
| LC | K49N | 0.578 | 2.026 | 0.570 | 1.043 |
| LC | K49Q | 0.536 | 2.041 | 0.524 | 1.540 |
| LC | K49S | 0.362 | 1.597 | 0.376 | 1.112 |
| LC | K49T | 0.592 | 2.367 | 0.594 | 1.297 |
| LC | K49V | 1.002 | 2.973 | 0.903 | 1.586 |
| LC | K49Y | 1.231 | 3.109 | 0.970 | 1.505 |
| LC | K49L | 0.903 | 3.860 | 0.881 | 2.054 |
| LC | K49H | 0.912 | 2.578 | 0.843 | 1.128 |
| LC | K49R | 0.342 | 1.611 | 0.345 | 1.300 |
| LC | A51T | 0.980 | 2.366 | 1.388 | 1.877 |
| LC | A51L | 0.763 | 2.758 | 1.330 | 2.485 |
| LC | S52A | 0.398 | 1.531 | 0.770 | 0.850 |
| LC | S52C | 1.091 | 1.720 | 1.195 | 1.383 |

Table 16. Normalized Specific Activity at pH 6.0 and pH 7.4 of Variant Anti-EGFR Antibodies.

(continued)

| Table 16. Normalized Specific Activity at pH 6.0 and pH 7.4 of Variant Anti-EGFR Antibodies. | | | | | |
|---|---|---|---|---|---|
| Chain[a] | Mutation | Normalized Specific Activity (NSA) (pH 6.0) | | Normalized Specific Activity (NSA) (pH 7.4) | |
| | | Dilution 1 | Dilution 2 | Dilution 1 | Dilution 2 |
| LC | S52D | 0.499 | 1.721 | 0.920 | 1.535 |
| LC | S52E | 0.535 | 1.648 | 1.011 | 1.400 |
| LC | S52G | 0.519 | 0.996 | 0.672 | 0.840 |
| LC | S52I | 0.893 | 1.799 | 1.225 | 1.511 |
| LC | S52M | 1.055 | 1.884 | 1.390 | 1.453 |
| LC | S52Q | 0.770 | 1.500 | 1.142 | 1.279 |
| LC | S52V | 0.559 | 1.542 | 0.982 | 1.388 |
| LC | S52W | 0.877 | 1.678 | 1.176 | 1.399 |
| LC | S52R | 0.447 | 1.379 | 0.713 | 1.159 |
| LC | S52K | 0.689 | 1.700 | 0.974 | 1.264 |
| LC | E53G | 0.124 | 0.591 | 0.143 | 0.447 |
| LC | S54M | 0.196 | 1.032 | 0.394 | 0.943 |
| LC | I55A | 0.256 | 0.952 | 0.446 | 0.603 |
| LC | I55F | 0.445 | 1.383 | 0.762 | 1.190 |
| LC | S56G | 0.760 | 3.081 | 0.742 | 1.858 |
| LC | S56L | 0.934 | 3.319 | 0.889 | 1.682 |
| LC | S56A | 0.545 | 2.069 | 0.555 | 1.215 |
| LC | S56C | 1.565 | 3.809 | 1.294 | 0.889 |
| LC | S56D | 0.622 | 2.026 | 0.596 | 1.222 |
| LC | S56E | 0.523 | 2.033 | 0.513 | 1.279 |
| LC | S56F | 0.591 | 1.918 | 0.584 | 1.086 |
| LC | S56N | 0.555 | 1.949 | 0.573 | 1.119 |
| LC | S56P | 0.397 | 1.746 | 0.410 | 1.441 |
| LC | S56Q | 0.511 | 1.827 | 0.504 | 1.015 |
| LC | S56V | 0.563 | 2.070 | 0.564 | 1.279 |
| LC | S56W | 0.731 | 2.335 | 0.674 | 1.212 |
| LC | S56H | 0.414 | 1.379 | 0.325 | 1.528 |
| LC | S56R | 0.786 | 2.454 | 0.716 | 1.271 |
| LC | S56K | 0.476 | 1.808 | 0.470 | 1.146 |
| LC | Y86F | 2.224 | 3.759 | 1.675 | 3.937 |
| LC | Y86M | 2.183 | 3.912 | 1.679 | 4.267 |
| LC | Y86H | 1.595 | 2.459 | 1.224 | 1.087 |
| LC | Y87L | 1.922 | 2.752 | 0.963 | 1.374 |
| LC | Y87C | 1.722 | 2.953 | 1.047 | 1.408 |
| LC | Y87D | 3.099 | 4.313 | 1.364 | 2.624 |

(continued)

| Table 16. Normalized Specific Activity at pH 6.0 and pH 7.4 of Variant Anti-EGFR Antibodies. | | | | | |
|---|---|---|---|---|---|
| Chain[a] | Mutation | Normalized Specific Activity (NSA) (pH 6.0) | | Normalized Specific Activity (NSA) (pH 7.4) | |
| | | Dilution 1 | Dilution 2 | Dilution 1 | Dilution 2 |
| LC | Y87F | 0.376 | 1.332 | 0.398 | 0.817 |
| LC | Y87G | 3.312 | 4.426 | 1.249 | 2.134 |
| LC | Y87I | 0.609 | 1.941 | 0.593 | 1.044 |
| LC | Y87N | 2.778 | 9.344 | 1.195 | 1.738 |
| LC | Y87P | 2.621 | 3.657 | 1.389 | 2.794 |
| LC | Y87S | 1.480 | 2.785 | 0.972 | 1.264 |
| LC | Y87T | 1.323 | 2.744 | 1.064 | 1.629 |
| LC | Y87V | 0.736 | 2.206 | 0.684 | 1.058 |
| LC | Y87W | 0.793 | 2.161 | 0.782 | 1.338 |
| LC | Y87K | 2.610 | 3.360 | 1.262 | 1.867 |
| LC | Y87H | 0.236 | 0.470 | 0.160 | 0.429 |
| LC | Y87R | 1.388 | 2.280 | 0.834 | 1.237 |
| LC | Q89E | 0.961 | 1.749 | 1.031 | 1.387 |
| LC | N91L | 1.701 | 3.145 | 0.851 | 2.286 |
| LC | N91A | 0.275 | 1.079 | 0.251 | 0.948 |
| LC | N91C | 0.640 | 1.490 | 0.543 | 0.990 |
| LC | N91I | 0.678 | 2.153 | 0.632 | 0.685 |
| LC | N91M | 0.655 | 1.996 | 0.607 | 1.575 |
| LC | N91S | 0.363 | 1.688 | 0.345 | 1.494 |
| LC | N91T | 0.450 | 1.583 | 0.418 | 1.138 |
| LC | N91V | 0.401 | 1.446 | 0.377 | 1.272 |
| LC | N91H | 0.568 | 1.649 | 0.541 | 0.880 |
| LC | N91R | 0.029 | 0.121 | 0.026 | 0.118 |
| LC | N92C | 1.107 | 2.897 | 1.022 | 2.216 |
| LC | N92D | 0.819 | 2.986 | 0.792 | 2.241 |
| LC | N92L | 0.688 | 1.848 | 0.730 | 0.962 |
| LC | N92M | 0.576 | 1.892 | 0.593 | 0.912 |
| LC | N92S | 0.639 | 2.367 | 0.566 | 1.408 |
| LC | N92T | 0.735 | 2.923 | 0.759 | 1.960 |
| LC | N92V | 0.924 | 4.052 | 0.933 | 2.824 |
| LC | N92W | 0.563 | 2.322 | 0.532 | 2.091 |
| LC | N92Y | 1.771 | 2.649 | 1.465 | 2.052 |
| LC | N92H | 0.620 | 2.443 | 0.579 | 2.005 |
| LC | N92K | 0.635 | 0.817 | 0.687 | 0.700 |
| LC | N92R | 0.711 | 1.893 | 0.705 | 0.821 |

(continued)

| Chain[a] | Mutation | Normalized Specific Activity (NSA) (pH 6.0) | | Normalized Specific Activity (NSA) (pH 7.4) | |
|---|---|---|---|---|---|
| | | Dilution 1 | Dilution 2 | Dilution 1 | Dilution 2 |
| LC | N93T | 0.035 | 0.085 | 0.050 | 0.062 |
| LC | T96L | 0.577 | 1.631 | 0.727 | 0.917 |
| LC | T96C | 1.341 | 3.139 | 1.570 | 2.292 |
| LC | T96M | 0.726 | 2.470 | 0.963 | 1.503 |
| LC | T96V | 0.476 | 1.360 | 0.663 | 1.032 |
| LC | T97L | 0.758 | 1.256 | 0.934 | 1.050 |
| LC | T97A | 0.430 | 0.978 | 0.852 | 0.427 |
| LC | T97D | 1.045 | 1.378 | 1.007 | 1.186 |
| LC | T97G | 0.722 | 1.113 | 1.018 | 0.988 |
| LC | T97Q | 0.616 | 1.181 | 1.025 | 1.058 |
| LC | T97S | 0.636 | 0.821 | 0.952 | 0.657 |
| LC | T97V | 0.629 | 1.355 | 0.919 | 1.176 |
| LC | T97K | 0.388 | 0.782 | 0.747 | 0.721 |
| LC | T97R | 0.859 | 1.424 | 0.748 | 1.681 |
| LC | F98A | 1.103 | 1.634 | 1.008 | 0.762 |
| LC | F98M | 1.483 | 2.284 | 1.354 | 2.064 |
| LC | F98S | 1.355 | 2.066 | 1.373 | 1.903 |
| LC | F98V | 2.045 | 2.874 | 1.802 | 2.446 |
| LC | F98Y | 0.548 | 1.302 | 0.669 | 1.077 |
| LC | G99L | 1.340 | 1.901 | 0.964 | 1.484 |
| LC | G99D | 1.750 | 2.208 | 1.387 | 1.970 |
| LC | G99E | 2.145 | 3.593 | 1.738 | 3.642 |
| LC | G99F | 4.569 | 7.151 | 2.674 | 4.939 |
| LC | G99I | 3.247 | 4.768 | 2.653 | 4.512 |
| LC | G99M | 3.359 | 5.103 | 2.188 | 4.173 |
| LC | G99N | 2.872 | 3.607 | 2.348 | 3.907 |
| LC | G99S | 0.960 | 1.660 | 1.158 | 0.999 |
| LC | G99T | 1.757 | 2.243 | 1.591 | 1.690 |
| LC | G99V | 2.850 | 3.773 | 2.351 | 2.864 |
| LC | G99K | 8.663 | 14.378 | 6.534 | 6.695 |
| LC | G99H | 1.416 | 1.792 | 1.323 | 1.311 |
| LC | Q100C | 1.256 | 2.897 | 1.023 | 1.414 |
| LC | Q100D | 0.772 | 2.716 | 0.802 | 1.875 |
| LC | Q100E | 0.797 | 2.698 | 0.808 | 1.570 |
| LC | Q100F | 0.716 | 2.360 | 0.696 | 1.404 |

**Table 16. Normalized Specific Activity at pH 6.0 and pH 7.4 of Variant Anti-EGFR Antibodies.**

(continued)

| Table 16. Normalized Specific Activity at pH 6.0 and pH 7.4 of Variant Anti-EGFR Antibodies. | | | | | |
|---|---|---|---|---|---|
| Chain[a] | Mutation | Normalized Specific Activity (NSA) (pH 6.0) | | Normalized Specific Activity (NSA) (pH 7.4) | |
| | | Dilution 1 | Dilution 2 | Dilution 1 | Dilution 2 |
| LC | Q100I | 1.154 | 3.244 | 1.113 | 1.986 |
| LC | Q100M | 0.867 | 2.533 | 0.800 | 1.460 |
| LC | Q100N | 0.472 | 2.107 | 0.491 | 1.691 |
| LC | Q100P | 0.736 | 2.823 | 0.778 | 1.850 |
| LC | Q100T | 0.870 | 3.235 | 0.864 | 0.896 |
| LC | Q100V | 0.672 | 2.331 | 0.659 | 1.447 |
| LC | Q100W | 0.791 | 2.343 | 0.783 | 1.211 |
| LC | Q100Y | 0.736 | 2.780 | 0.760 | 2.001 |
| LC | Q100K | 0.726 | 2.396 | 0.694 | 1.351 |
| LC | Q100H | 0.630 | 2.086 | 0.618 | 1.323 |
| LC | Q100R | 0.761 | 2.471 | 0.759 | 1.380 |
| [a] HC = Heavy Chain, LC = Light Chain | | | | | |

## 2. Confirmation Screen

A confirmation screen was performed as described in part 1, except that 5% serum was used at both pH values. Table 17 sets forth the OD at pH 6.0 ($OD_{6.0}$) at each dilution, OD at pH 7.4 ($OD_{7.4}$) at each dilution, and the ratio of the average OD values at pH 6.0 and 7.4 ($OD_{6.0}/OD_{7.4}$) for the exemplary tested modified antibodies at Dilution 1 and Dilution 2.

| Table 17. Variant anti-EGFR antibodies | | | | | | | | | | |
|---|---|---|---|---|---|---|---|---|---|---|
| Chain[a] | Mutation | OD pH6.0 | | | | OD pH7.4.0 | | | | (OD pH 6.0) / (OD pH 7.4) | |
| | | Dilution 1 | | Dilution 2 | | Dilution 1 | | Dilution 2 | | Dilution 1 | Dilution 2 |
| HC | L029Y | 0.181 | 0.179 | 0.092 | 0.093 | 0.132 | 0.130 | 0.075 | 0.079 | 1.370 | 1.206 |
| HC | L029S | 1.116 | 1.143 | 0.303 | 0.327 | 0.545 | 0.583 | 0.074 | 0.074 | 2.004 | 4.251 |
| HC | L029K | 1.940 | 1.906 | 0.529 | 0.724 | 1.122 | 1.134 | 0.324 | 0.000 | 1.705 | 1.970 |
| HC | L029H | 0.814 | 0.790 | 0.216 | 0.203 | 0.396 | 0.355 | 0.132 | 0.124 | 2.142 | 1.635 |
| HC | L029N | 0.248 | 0.259 | 0.111 | 0.099 | 0.156 | 0.157 | 0.083 | 0.092 | 1.623 | 1.215 |
| HC | L029D | 0.543 | 0.496 | 0.156 | 0.149 | 0.285 | 0.290 | 0.104 | 0.115 | 1.808 | 1.401 |
| HC | L029V | 0.807 | 0.851 | 0.204 | 0.237 | 0.397 | 0.409 | 0.135 | 0.130 | 2.058 | 1.669 |
| HC | L029F | 0.420 | 0.445 | 0.131 | 0.137 | 0.251 | 0.244 | 0.105 | 0.108 | 1.748 | 1.255 |
| HC | L029I | 1.753 | 1.713 | 0.514 | 0.522 | 0.948 | 0.918 | 0.273 | 0.278 | 1.858 | 1.880 |
| HC | L029A | 1.822 | 1.861 | 0.582 | 0.662 | 0.938 | 0.888 | 0.275 | 0.289 | 2.019 | 2.202 |
| HC | L029M | 1.472 | 1.358 | 0.435 | 0.465 | 0.751 | 0.729 | 0.206 | 0.215 | 1.912 | 2.135 |
| HC | L029G | 0.291 | 0.289 | 0.111 | 0.111 | 0.171 | 0.165 | 0.089 | 0.093 | 1.723 | 1.226 |
| HC | T030V | 2.448 | 2.492 | 1.150 | 1.727 | 2.045 | 2.162 | 0.673 | 0.812 | 1.175 | 1.917 |
| HC | T030G | 2.528 | 2.483 | 1.323 | 1.329 | 1.748 | 1.659 | 0.557 | 0.000 | 1.471 | 2.399 |

(continued)

| Chain[a] | Mutation | OD pH6.0 | | | | OD pH7.4.0 | | | | (OD pH 6.0) / (OD pH 7.4) | |
|---|---|---|---|---|---|---|---|---|---|---|---|
| | | Dilution 1 | | Dilution 2 | | Dilution 1 | | Dilution 2 | | Dilution 1 | Dilution 2 |
| HC | T030S | 2.411 | 2.423 | 1.046 | 1.209 | 1.520 | 1.545 | 0.467 | 0.418 | 1.578 | 2.567 |
| HC | T030M | 2.189 | 2.256 | 0.713 | 0.900 | 1.289 | 1.230 | 0.338 | 0.322 | 1.766 | 2.453 |
| HC | T030R | 2.326 | 2.269 | 0.683 | 0.995 | 1.260 | 1.376 | 0.340 | 0.370 | 1.748 | 2.349 |
| HC | T030P | 2.217 | 2.216 | 0.688 | 0.911 | 1.261 | 1.159 | 0.318 | 0.375 | 1.836 | 2.298 |
| HC | T030H | 2.062 | 2.003 | 0.658 | 0.749 | 1.225 | 1.163 | 0.276 | 0.354 | 1.703 | 2.249 |
| HC | T030W | 2.042 | 2.058 | 0.683 | 0.702 | 1.208 | 1.139 | 0.316 | 0.322 | 1.748 | 2.170 |
| HC | T030D | 2.378 | 2.404 | 1.055 | 1.352 | 1.462 | 1.549 | 0.461 | 0.478 | 1.589 | 2.559 |
| HC | T030N | 2.276 | 2.237 | 0.907 | 1.027 | 1.396 | 1.326 | 0.373 | 0.410 | 1.659 | 2.470 |
| HC | Y032L | 1.263 | 1.249 | 0.307 | 0.349 | 0.570 | 0.567 | 0.182 | 0.161 | 2.209 | 1.932 |
| HC | Y032R | 1.871 | 1.881 | 0.513 | 0.554 | 0.994 | 0.980 | 0.279 | 0.259 | 1.901 | 1.987 |
| HC | Y032N | 2.023 | 2.042 | 0.611 | 2.097 | 0.992 | 1.090 | 0.365 | 0.359 | 1.957 | 1.832 |
| HC | Y032H | 2.097 | 2.099 | 0.804 | 0.841 | 1.199 | 1.229 | 0.397 | 0.396 | 1.729 | 2.074 |
| HC | Y032C | 0.426 | 0.451 | 0.137 | 0.168 | 0.378 | 0.401 | 0.140 | 0.137 | 1.125 | 1.104 |
| HC | Y032T | 1.927 | 1.880 | 0.732 | 0.963 | 1.333 | 1.316 | 0.582 | 0.643 | 1.437 | 1.378 |
| HC | Y032M | 2.356 | 2.411 | 0.557 | 0.825 | 1.622 | 1.604 | 0.462 | 0.504 | 1.478 | 1.423 |
| HC | V034L | 0.554 | 0.537 | 0.157 | 0.171 | 0.270 | 0.251 | 0.103 | 0.103 | 2.095 | 1.592 |
| HC | V034I | 1.200 | 1.061 | 0.317 | 1.455 | 0.533 | 0.503 | 0.147 | 0.163 | 2.183 | 2.076 |
| HC | V034M | 1.455 | 1.549 | 0.433 | 0.466 | 0.723 | 0.762 | 0.222 | 0.230 | 2.023 | 1.986 |
| HC | V034C | 1.317 | 1.265 | 0.359 | 0.357 | 0.667 | 0.652 | 0.197 | 0.192 | 1.956 | 1.845 |
| HC | H035N | 0.888 | 0.839 | 0.319 | 0.436 | 0.718 | 0.748 | 0.241 | 0.254 | 1.179 | 1.518 |
| HC | W036L | 1.601 | 1.562 | 0.456 | 0.495 | 0.629 | 0.599 | 0.201 | 0.207 | 2.578 | 2.332 |
| HC | W036Y | 0.608 | 0.574 | 0.171 | 0.176 | 0.242 | 0.224 | 0.101 | 0.108 | 2.533 | 1.662 |
| HC | I069T | 0.496 | 0.486 | 0.154 | 0.150 | 0.216 | 0.217 | 0.096 | 0.099 | 2.272 | 1.557 |
| HC | I069M | 0.498 | 0.509 | 0.148 | 0.181 | 0.216 | 0.239 | 0.096 | 0.100 | 2.218 | 1.673 |
| HC | I069C | 0.512 | 0.505 | 0.159 | 0.164 | 0.253 | 0.243 | 0.106 | 0.112 | 2.049 | 1.482 |
| HC | Y093H | 0.551 | 0.523 | 0.187 | 0.170 | 0.328 | 0.358 | 0.115 | 0.123 | 1.571 | 1.504 |
| HC | Y094L | 1.173 | 1.151 | 0.334 | 0.279 | 0.497 | 0.532 | 0.109 | 0.187 | 2.262 | 2.276 |
| HC | Y094R | 2.325 | 2.409 | 0.662 | 1.199 | 1.494 | 1.455 | 0.446 | 0.454 | 1.606 | 2.063 |
| HC | R097H | 1.077 | 1.150 | 0.211 | 0.360 | 0.406 | 0.425 | 0.139 | 0.143 | 2.679 | 2.019 |
| HC | L099N | 2.052 | 2.205 | 0.704 | 0.695 | 1.227 | 1.220 | 0.265 | 0.524 | 1.739 | 2.209 |
| HC | Y104S | 0.695 | 0.801 | 0.211 | 0.280 | 0.117 | 0.111 | 0.075 | 0.084 | 6.562 | 3.059 |
| HC | Y104V | 2.078 | 2.153 | 0.687 | 0.947 | 1.288 | 1.358 | 0.421 | 0.512 | 1.599 | 1.741 |
| HC | Y104D | 1.486 | 1.505 | 0.580 | 0.724 | 0.439 | 0.413 | 0.111 | 0.196 | 3.515 | 4.457 |
| HC | Y104M | 1.847 | 1.883 | 0.670 | 1.043 | 0.899 | 0.852 | 0.316 | 0.428 | 2.132 | 2.279 |
| HC | Y104F | 1.527 | 1.463 | 0.410 | 0.527 | 0.638 | 0.574 | 0.183 | 0.241 | 2.471 | 2.217 |

**Table 17. Variant anti-EGFR antibodies**

(continued)

| Chain[a] | Mutation | OD pH6.0 | | | | OD pH7.4.0 | | | | (OD pH 6.0) / (OD pH 7.4) | |
|---|---|---|---|---|---|---|---|---|---|---|---|
| | | Dilution 1 | | Dilution 2 | | Dilution 1 | | Dilution 2 | | Dilution 1 | Dilution 2 |
| HC | Y104L | 1.343 | 1.585 | 0.418 | 0.557 | 0.681 | 0.646 | 0.204 | 0.247 | 2.213 | 2.152 |
| HC | A107E | 1.556 | 1.487 | 0.505 | 0.500 | 0.665 | 0.648 | 0.203 | 0.232 | 2.318 | 2.324 |
| HC | Y108I | 1.148 | 1.257 | 0.282 | 0.411 | 0.533 | 0.492 | 0.173 | 0.179 | 2.353 | 1.964 |
| HC | Y108L | 0.880 | 0.950 | 0.287 | 0.244 | 0.481 | 0.473 | 0.133 | 0.195 | 1.918 | 1.704 |
| HC | Y108W | 1.331 | 1.377 | 0.414 | 0.426 | 0.673 | 0.667 | 0.188 | 0.286 | 2.022 | 1.844 |
| HC | Y108T | 1.494 | 1.566 | 0.292 | 0.528 | 0.608 | 0.709 | 0.192 | 0.220 | 2.333 | 1.961 |
| HC | Y108S | 1.293 | 1.358 | 0.311 | 0.404 | 0.595 | 0.565 | 0.169 | 0.174 | 2.288 | 2.079 |
| HC | Y108N | 1.409 | 1.374 | 0.360 | 0.435 | 0.623 | 0.640 | 0.182 | 0.193 | 2.205 | 2.114 |
| HC | Y108K | 0.183 | 0.541 | 0.149 | 0.164 | 0.266 | 0.261 | 0.109 | 0.105 | 1.380 | 1.471 |
| HC | W109M | 0.583 | 0.678 | 0.175 | 0.205 | 0.303 | 0.314 | 0.108 | 0.128 | 2.042 | 1.616 |
| HC | W109I | 0.440 | 0.466 | 0.143 | 0.151 | 0.241 | 0.234 | 0.099 | 0.108 | 1.907 | 1.420 |
| HC | G110A | 1.906 | 2.205 | 0.608 | 0.910 | 1.130 | 1.104 | 0.360 | 0.334 | 1.843 | 2.205 |
| HC | G110D | 1.639 | 1.450 | 0.431 | 0.445 | 0.731 | 0.713 | 0.218 | 0.191 | 2.138 | 2.156 |
| HC | G110T | 0.770 | 0.783 | 0.203 | 0.176 | 0.381 | 0.399 | 0.145 | 0.112 | 1.992 | 1.482 |
| HC | G112A | 1.162 | 1.194 | 0.303 | 0.392 | 0.623 | 0.644 | 0.180 | 0.147 | 1.860 | 2.173 |
| LC | I033M | 2.183 | 2.207 | 0.721 | 0.639 | 1.336 | 1.183 | 0.372 | 0.348 | 1.750 | 1.889 |
| LC | I033L | 2.051 | 2.115 | 0.639 | 0.797 | 1.071 | 1.047 | 0.287 | 0.315 | 1.968 | 2.379 |
| LC | I033T | 0.928 | 0.912 | 0.244 | 0.237 | 0.389 | 0.388 | 0.134 | 0.128 | 2.367 | 1.838 |
| LC | I033F | 0.780 | 0.765 | 0.193 | 0.215 | 0.346 | 0.346 | 0.124 | 0.124 | 2.235 | 1.646 |
| LC | I033A | 0.922 | 1.005 | 0.274 | 0.265 | 0.462 | 0.486 | 0.149 | 0.153 | 2.031 | 1.785 |
| LC | I033C | 1.370 | 1.356 | 0.399 | 0.417 | 0.642 | 0.708 | 0.196 | 0.233 | 2.025 | 1.912 |
| LC | I033V | 2.386 | 2.389 | 1.302 | 1.776 | 1.895 | 1.804 | 0.703 | 0.766 | 1.292 | 2.085 |
| LC | Y086H | 0.503 | 0.561 | 0.140 | 0.165 | 0.232 | 0.275 | 0.103 | 0.103 | 2.107 | 1.481 |
| LC | Y086F | 0.496 | 0.517 | 0.157 | 0.158 | 0.241 | 0.253 | 0.099 | 0.097 | 2.051 | 1.602 |
| LC | F098S | 0.704 | 0.726 | 0.165 | 0.225 | 0.288 | 0.303 | 0.111 | 0.148 | 2.422 | 1.503 |
| LC | F098Y | 2.236 | 2.283 | 0.899 | 1.225 | 1.360 | 1.352 | 0.399 | 0.000 | 1.667 | 2.291 |
| LC | F098M | 0.408 | 0.452 | 0.136 | 0.144 | 0.190 | 0.185 | 0.096 | 0.096 | 2.296 | 1.457 |
| LC | G099D | 0.734 | 0.717 | 0.200 | 0.214 | 0.330 | 0.285 | 0.111 | 0.123 | 2.373 | 1.774 |
| LC | G099S | 1.948 | 1.945 | 0.684 | 0.732 | 1.140 | 1.225 | 0.314 | 0.321 | 1.648 | 2.230 |
| LC | G099L | 0.546 | 0.501 | 0.160 | 0.188 | 0.262 | 0.256 | 0.110 | 0.101 | 2.020 | 1.660 |
| LC | G099T | 0.845 | 0.809 | 0.233 | 0.227 | 0.432 | 0.415 | 0.137 | 0.127 | 1.951 | 1.742 |
| LC | G099H | 0.448 | 0.445 | 0.139 | 0.152 | 0.253 | 0.251 | 0.098 | 0.092 | 1.771 | 1.528 |

Table 17. Variant anti-EGFR antibodies

[a]HC = Heavy Chain, LC = Light Chain

**Example 2**

**Generation and Screening of a Combinatorial Library**

A combinatorial library of anti-EGFR variant members was generated that contained antibody members having all combinations of mutations LC-I29S, V24E, S25C, F27R, R97H, Y104D and/or HC-Q11 IP in the Heavy Chain. The multiple point mutants were generated in the Cetuximab anti-EGFR reference antibody described in Example 1 by site-directed mutagenesis. The library contained variants of Cetuximab anti-EGFR antibody, whereby each member contained 1, 2, 3, 4, 5, 6 or 7 amino acid mutations compared to the reference antibody in the variable regions of the heavy chain (SEQ ID NO:8 with the variable heavy chain set forth in SEQ ID NO:3) or light chain (SEQ ID NO:9 with the variable light chain set forth in SEQ ID NO:10). The total number of variant members of the combinatorial library that were generated was 128. Each member of the library was sequenced. Glycerol stocks of members of the library were prepared and stored at -80 °C. For screening, an expression vector encoding a member of the library was separately expressed in CHO cells as IgG antibodies and supernatants collected.

The library was screened as described in Example 1, with 25% human serum added and the antibodies diluted to concentrations 4 ng/mL, 2 ng/mL and 1 ng/mL. Exemplary antibodies exhibiting higher binding activity at pH 6.0 compared to pH 7.4 are set forth in Table 18, which sets forth the OD at pH 6.0 ($OD_{6.0}$), the OD at pH 7.4 ($OD_{7.4}$), and the ratio of the average OD values at pH 6.0 and 7.4 ($OD_{6.0}/OD_{7.4}$) for the modified antibodies and Cetuximab at a concentration of 4 ng/mL. Among the antibodies with the highest OD $pH_{6.0}$/OD pH7.4 binding ratio were those containing a heavy chain HC-Y104D/ Q111P (SEQ ID NO: 1062), HC-V24E/ F27R/ R97H/ Q111P (SEQ ID NO:1093), HC-S25C/ Y104D (SEQ ID NO:1112), and HC-S25C/ Q111P (SEQ ID NO: 1113).

| Table 18 Modified anti-EGFR antibodies | | | | | |
|---|---|---|---|---|---|
| **Mutation(s)** | **OD (pH 6.0) 4 ng/mL** | | **OD (pH 7.4) 4 ng/mL** | | **(OD pH 6.0)/(OD pH 7.4) 4 ng/mL** |
| HC-V24E | 0.379 | 0.325 | 0.216 | 0.260 | 1.476 |
| HC-V24E | 2.028 | 2.100 | 1.796 | 1.621 | 1.208 |
| HC-S25C | 2.179 | 2.044 | 1.785 | 1.765 | 1.190 |
| HC-S25C | 2.021 | 2.229 | 1.822 | 1.832 | 1.163 |
| HC-F27R | 1.988 | 1.714 | 1.727 | 1.628 | 1.103 |
| HC-F27R | 1.821 | 1.808 | 1.650 | 1.549 | 1.135 |
| HC-R97H | 2.167 | 2.077 | 1.649 | 1.585 | 1.312 |
| HC-R97H | 2.021 | 1.988 | 1.345 | 1.289 | 1.523 |
| HC-Y104D | 1.655 | 1.702 | 0.609 | 0.596 | 2.786 |
| HC-Y104D | 1.421 | 1.402 | 0.476 | 0.470 | 2.986 |
| HC-S25C/HC-Y104D | 1.026 | 1.045 | 0.131 | 0.132 | 7.905 |
| HC-S25C/HC-Y104D | 1.025 | 0.882 | 0.200 | 0.145 | 5.528 |
| HC-Y104D | 1.245 | 1.254 | 0.311 | 0.322 | 3.948 |
| HC-Q111P | 2.177 | 2.101 | 1.887 | 1.732 | 1.182 |
| HC-Q111P | 2.030 | 2.032 | 1.607 | 1.587 | 1.272 |
| HC-S25C/HC-Q111P | 1.967 | 1.890 | 1.553 | 1.557 | 1.240 |
| LC-I29S | 1.888 | 1.918 | 1.500 | 1.575 | 1.238 |
| HC-Y104D/HC-Q111P | 1.140 | 1.081 | 0.166 | 0.167 | 6.670 |
| HC-S25C/LC-I29S | 1.840 | 1.793 | 1.694 | 1.616 | 1.098 |
| HC-Y104D/LC-I29S | 1.181 | 1.188 | 0.750 | 0.636 | 1.709 |
| HC-Q111P/LC-I29S | 1.785 | 1.763 | 1.287 | 1.361 | 1.340 |
| HC-Y104D/HC-Q111P/LC-I29S | 1.048 | 0.990 | 0.700 | 0.768 | 1.388 |

(continued)

| Table 18 Modified anti-EGFR antibodies | | | | | | |
|---|---|---|---|---|---|
| Mutation(s) | OD (pH 6.0) 4 ng/mL | | OD (pH 7.4) 4 ng/mL | | (OD pH 6.0)/(OD pH 7.4) 4 ng/mL |
| HC-V24E/HC-F27R/ HC-R97H/HC-Q111P | 0.374 | 0.393 | 0.201 | 0.239 | 1.743 |
| HC-V24E/HC-F27R/HC-R97H/HC-Q111P | 0.460 | 0.440 | 0.246 | 0.227 | 1.903 |
| Reference Cetuximab | 1.784 | 1.700 | 1.326 | 1.266 | 1.344 |

**Example 3**

**Effect of Addition of Human Serum on ELISA**

The effect of human serum on binding of the unmodified (Cetuximab reference antibody) and variant anti-EGFR antibodies (HC-Y104D and HC-Y104D/HC-Q111P) was determined by a quantitative ELISA.

**1. Expression and Purification**

CHO-S cells were cultured in shaker flasks using CD-CHO media supplemented with GlutaMAX (8 mM). On the day of transfection, 300 mL of CHO-S cells at an approximate density of $1.0 \times 10^6$ cells/mL were transfected using 375 $\mu$g of plasmid DNA with 375 $\mu$L of FreeStyle™ MAX Reagent (Invitrogen) following the manufacturer's protocol. The media were harvested by centrifugation (4000g x 20') at 96 hours after transfection. The expressed antibodies in the conditioned media were further purified by affinity chromatography using a protein A column (2 mL) prepared from Protein A resin (BioRad: Cat# 156-0218). IgG bound to the column was eluted in one step with 0.1 M glycine-HCl, pH 2.5. The eluted IgG was neutralized and dialyzed prior to protein determination using a BCA protein assay (Pierce Biotechnology).

To establish stable cell lines expressing the HC-Y104D mutant, 30 mL of CHO-S cells at an approximate density of $1.0 \times 10^6$ cells/mL were transfected using 37.5 $\mu$g of plasmid DNA with 37.5 $\mu$L of FreeStyle™ MAX Reagent (Invitrogen) following the manufacturer's protocol. 72-hour post transfection, the cells were cloned in CD-CHO media supplemented with GlutaMAX (8 mM) and 1 mg/mL G418 using the 1-dimensional serial dilution strategy in 15 wells of 96-well round bottom plates (Nunc). Four weeks later, clones expressing Y104D mutant were screened by western blot analysis (WB) using peroxidase conjugated anti-human IgG Fc (Jackson Immonolab) as detecting antibodies. Postive clones were expanded step-wise into 12-well then 6-well plates, followed by T-25 and T-75 flasks and evantually to shaker flasks. Two clones, 13E3 and 15D6 expressing at 5 mg/L of Y104D, were further expanded to wavebag bioreactor production to support all the in vitro, ex vivo and in vivo characterization of Y104D mutants.

To establish stable cell lines expressing the HC-Y104D/Q111P double mutant, 25x5 mL of CHO-S cells at an approximate density of $1.0 \times 10^6$ cells/mL were transfected using 25 $\mu$g of plasmid DNA by electroporation using the MaxCyte STX apparatus following the manufacturer's protocol. 72-hour post transfection, 4x5 mL of the cells were cloned in CD-CHO media supplemented with GlutaMAX (8 mM) and 1 mg/mL G418 using the 1-dimensional serial dilution strategy in 25 wells of 96-well round bottom plates (Nunc). Four weeks later, clones expressing the Y104D/Q111P mutant were screened by western blot analysis (WB) using peroxidase conjugated anti-human IgG Fc (Jackson Immonolab) as the detecting antibody. Postive clones were then expanded step-wise to shaker flasks as described above. Two clones, 9-1-5B and 9-4-6A, expressing at 2-5 mg/L of Y104D/Q111P, were further expanded to wavebag bioreactor production to support all the in vitro, ex vivo and in vivo characterization of Y104D/Q111P mutants.

**2. Effect of Addition of Human Serum on ELISA**

The ELISA assay was performed as described in Example 1 with some modifications. Three (3)-fold serial dilutions of the Cetuximab anti-EGFR-FLAG reference antibody and FLAG-tagged anti-EGFR HC-Y104D and HC-Y104D/Q111P mutant antibodies were prepared from a starting concentration of 100 ng/mL. The standard and mutant antibodies were prepared in pH 7.4 Buffer (KRB, pH 7.4, 1 mM lactic acid) containing either no serum, 5% human serum or 25 % human serum. The standard and mutant antibodies also were prepared in pH 6.0 Buffer (KRB, 16.6 mM lactic acid) containing either no serum, 5% human serum or 25 % human serum. For each condition, the final concentrations of anti-EGFR-FLAG antibody standard and HC-Y104D and HC-Y104D/Q111P mutant antibodies were 666.67 pM (100 ng/mL), 222.22 pM (33.33 ng/mL), 74.07 pM (11.11 ng/mL), 24.69 pM (3.70 ng/mL), 8.23 pM (1.23 ng/mL), 2.74 pM (0.41 ng/mL), 0.91 pM (0.137 ng/mL) and 0. One hundred microliters (100 $\mu$L) of each concentration of each antibody were added to wells of a 96-well plate. Each plate included an anti-EGFR-FLAG antibody standard, a positive control (parental antibody) and a negative control (no primary antibody). The ELISA was performed in triplicate.

The results showed that human serum increased binding of the variant anti-EGFR to sECD EGFR. Compared to binding to sECD EGFR in the presence of no human serum, the addition of 5 % human serum resulted in approximately a 3-fold increase in OD for the HC-Y104D and HC-Y104D / HC-Q111P anti-EGFR variants at pH 6.0 or pH 7.4. The addition of 25 % human serum resulted in approximately a 4- to 5-fold increase in OD for the HC-Y104D and HC-Y104D / HC-Q111P anti-EGFR variants at pH 6.0 or pH 7.4, compared to binding in the presence of no human serum. For Cetuximab, addition of 5% or 25% human serum at pH 6.0 or pH 7.4 resulted in less than a 2-fold increase in OD compared to the OD in the presence of no human serum.

### Example 4

### Effect of Lactic Acid Concentration on ELISA

The effect of lactic acid on binding of the unmodified (Cetuximab reference antibody) and variant anti-EGFR antibodies (HC-Y104D and HC-Y104D/HC-Q111P) was determined by pH-sensitive ELISA, as described in Example 3, with modification to the assay buffer.

The Cetuximab anti-EGFR-FLAG reference antibody standards, and FLAG-tagged anti-EGFR antibody variants (HC-Y104D or HC-Y104D / HC-Q111P) to be tested, were prepared in pH 6.0 Buffer (KRB, pH 6.0, without serum) containing either 16.7 mM lactic acid or no lactic acid. Standards and mutants also were prepared in pH 7.4 Buffer (KRB, without serum) containing either 16.7 mM lactic acid or no lactic acid. Each antibody was diluted to final concentrations of 666.67 pM (100 ng/mL), 222.22 pM (33.33 ng/mL), 74.07 pM (11.11 ng/mL), 24.69 pM (3.70 ng/mL), 8.23 pM (1.23 ng/mL), 2.74 pM (0.41 ng/mL), 0.91 pM (0.137 ng/mL) and 0. 100 $\mu$L of the standards and antibody variants were added to the appropriate wells. Each plate included an anti-EGFR-FLAG antibody standard, a positive control (parental antibody) and a negative control (no primary antibody). The ELISA was performed in triplicate. The results showed that the presence of increased lactic acid had no significant effect on the binding of Cetuximab or the anti-EGFR variants to sECD EGFR at pH6.0 or pH 7.4.

### Example 5

### Binding Affinity of Identified Hits

Bio-layer interferometry was performed to measure binding of variant anti-EGFR antibodies to EGFR at pH 6.5 and pH 7.4. The dissociation constant ($K_D$) of Cetuximab and variant anti-EGFR antibodies for sEGFR was measured by bio-layer interferometry in a 96-well format using an Octet QKe instrument (ForteBio, Menlo Park, CA). Data Acquisition software was used for all the operation steps including biotinylated sECD EGFR ligand loading and antibody association and dissociation steps. The ligand loading and antibody association and dissociation steps were performed with two groups at different pH values (pH 6.5 group and pH 7.4 group).

Biotinylated sECD EGFR was prepared by adding 15 $\mu$L of NHS-PEG4-Biotin solution (20 mM in ultrapure water) (PIERCE, Cat# 21329) for 1 mg of sEGFR in solution, and incubating the reaction mixture at room temperature for 30 minutes. Nonreacted NHS-PEG4-Biotin was removed by dialysis, and the protein concentration of biotinylated sEGFR was measured by BCA protein assay (PIERCE, Cat# 23225) according to manufacturer's instruction.

### 1. Binding Affinity of Anti-EGFR Variants at pH 6.5 and pH 7.4

To assess the difference in binding at different pH, for the ligand loading step, biotinylated sECD EGFR was bound to a streptavidin biolayer in PBS (pH 6.5 or 7.4). The streptavidin sensors were dipped in wells containing PBS (pH 6.5 or 7.4) for 1 minute, then the sensors were dipped in wells containing biotinylated sEGFR (50 $\mu$g/mL) in PBS (pH 6.5 or 7.4) for 2 minutes. Sensors were rinsed in wells containing PBS (pH 6.5 or 7.4). During all steps, the plate was agitated at 1000 rpm.

### Antibody Association and Dissociation Steps for affinity measurement:

To measure association rates, the immobilized sECD-EGFR sensors were dipped into wells with antibody (Cetuximab or variant anti-EGFR antibodies) at 22 nM or 66.7 nM in PBS at pH 6.5 or 7.4 for 2 min. To measure dissociation rates, antibody bound sensors were dipped in PBS wells at pH 6.5 or 7.4 for 4 min. Association and dissociation of sEGFR and antibody (Cetuximab or variant anti-EGFR antibody) were quantitated by measuring changes in the interference pattern generated from light reflected from the optical layer and the biolayer.

Association rates, dissociation rates and $K_D$ values were calculated with Software Data Analysis (v. 6.4) using global curve fitting. The $K_D$ values of Cetuximab and variant anti-EGFR antibodies at pH 6.5 and pH 7.4 are set forth in Table 19.

| Table 19. Dissociation constants ($K_D$) of Cetuximab and modified anti-EGFR antibodies | | |
|---|---|---|
| **Mutation(s)** | **$K_D$ (nM)** | |
| | **pH 6.5** | **pH 7.4** |
| Cetuximab | 0.390 | 0.299 |
| HC-Y104D | 1.96 | 2.30 |
| HC-Y104D/HC-Q111P | 1.74 | 1.98 |

**2. Effect of Buffer Composition and pH on Binding Affinity of Anti-EGFR Variants**

In a further experiment, the binding of variant anti-EGFR antibodies to sECD EGFR at pH 6.0, 6.5 and pH 7.4 in PBS, Krebs-Ringer Bicarbonate Buffer (KRB) or KRB with 25% human serum were measured using bio-layer interferometry similar to the methods described above. The results are set forth in Table 20 below.

| Table 20. Dissociation constants of Cetuximab and modified anti-EGFR antibodies at pH 6.0, pH 6.5 and pH 7.4 in PBS, KRB or KRB with 25% human serum | | | | | | |
|---|---|---|---|---|---|---|
| **Sample ID** | **Buffer** | **pH** | **$K_D$(M)** | **$k_{on}$(1/Ms)** | **$k_{off}$(1/s)** | **Full R^2** |
| Cetuximab | PBS | 6.0 | 1.55E-10 | 9.00E+05 | 1.39E-04 | 0.9991 |
| Y104D | PBS | 6.0 | 7.48E-10 | 7.91E+05 | 5.92E-04 | 0.9937 |
| Y104DQ111P | PBS | 6.0 | 7.97E-10 | 8.82E+05 | 7.02E-04 | 0.9906 |
| Cetuximab | KRB | 6.0 | 6.01E-11 | 9.61E+05 | 5.78E-05 | 0.9990 |
| Y104D | KRB | 6.0 | 8.82E-10 | 7.07E+05 | 6.23E-04 | 0.9912 |
| Y104DQ111P | KRB | 6.0 | 11.9E-10 | 8.03E+05 | 9.52E-04 | 0.9874 |
| Cetuximab | KRB + 25% Hu S | 6.0 | 8.22E-12 | 1.14E+06 | 9.37E-06 | 0.9864 |
| Y104D | KRB + 25% Hu S | 6.0 | 1.98E-10 | 2.00E+06 | 3.94E-04 | 0.9892 |
| Y104DQ111P | KRB + 25% Hu S | 6.0 | 2.95E-10 | 2.09E+06 | 6.17E-04 | 0.9855 |
| Cetuximab | PBS | 6.0 | 1.55E-10 | 9.00E+05 | 1.39E-04 | 0.9991 |
| Y104D | PBS | 6.0 | 7.48E-10 | 7.91E+05 | 5.92E-04 | 0.9937 |
| Y104DQ111P | PBS | 6.0 | 7.97E-10 | 8.82E+05 | 7.02E-04 | 0.9906 |
| Cetuximab | KRB | 6.0 | 6.01E-11 | 9.61E+05 | 5.78E-05 | 0.9990 |
| Y104D | KRB | 6.0 | 8.82E-10 | 7.07E+05 | 6.23E-04 | 0.9912 |
| Y104DQ111P | KRB | 6.0 | 11.9E-10 | 8.03E+05 | 9.52E-04 | 0.9874 |
| | | | | | | |
| Cetuximab | PBS | 6.5 | 1.70E-10 | 9.84E+05 | 1.67E-04 | 0.9988 |
| Y104D | PBS | 6.5 | 8.78E-10 | 8.01L+05 | 7.04E-04 | 0.9916 |
| Y104DQ111P | PBS | 6.5 | 8.89E-10 | 9.02E+05 | 8.02E-04 | 0.9918 |
| Cetuximab | KRB | 6.5 | 1.04E-10 | 8.96E+05 | 9.35E-05 | 0.9991 |

(continued)

| Table 20. Dissociation constants of Cetuximab and modified anti-EGFR antibodies at pH 6.0, pH 6.5 and pH 7.4 in PBS, KRB or KRB with 25% human serum | | | | | | |
|---|---|---|---|---|---|---|
| Sample ID | Buffer | pH | $K_D$(M) | $k_{on}$(1/Ms) | $k_{off}$(1/s) | Full R^2 |
| Y104D | KRB | 6.5 | 10.4E-10 | 9.48E+05 | 9.87E-04 | 0.9923 |
| Y104DQ111P | KRB | 6.5 | 10.5E-10 | 1.12E+06 | 1.18E-03 | 0.9748 |
| Cetuximab | KRB + 25% Hu S | 6.5 | 5.81E-11 | 1.33E+06 | 7.69E-05 | 0.9984 |
| Y104D | KRB + 25% Hu S | 6.5 | 2.52E-10 | 1.85E+06 | 4.66E-04 | 0.9931 |
| Y104DQ111P | KRB + 25% Hu S | 6.5 | 2.82E-10 | 1.94E+06 | 5.46E-04 | 0.9897 |
| | | | | | | |
| Cetuximab | PBS | 7.4 | 1.13E-10 | 8.49E+05 | 9.58E-05 | 0.9992 |
| Y104D | PBS | 7.4 | 1.52E-09 | 4.08E+05 | 6.20E-04 | 0.9919 |
| Y104DQ111P | PBS | 7.4 | 1.41E-09 | 5.52E+05 | 7.76E-04 | 0.9906 |
| Cetuximab | KRB | 7.4 | 1.04E-10 | 9.62E+05 | 9.98E-05 | 0.9992 |
| Y104D | KRB | 7.4 | 1.66E-09 | 9.65E+05 | 1.60E-03 | 0.9746 |
| Y104DQ111P | KRB | 7.4 | 1.42E-09 | 1.37E+06 | 1.94E-03 | 0.9607 |
| Cetuximab | KRB + 25% Hu S | 7.4 | <1.0E-12 | 1.61E+06 | <1.0E-07 | 0.9908 |
| Y104D | KRB + 25% Hu S | 7.4 | 6.61E-10 | 3.44E+06 | 2.27E-03 | 0.9354 |
| Y104DQ111P | KRB + 25% Hu S | 7.4 | 10.0E-10 | 3.81E+06 | 3.81E-03 | 0.9470 |

For each buffer condition, the Y104D and Y104D/Q111P mutants exhibited lower affinities (higher $K_D$ values) for sEGFR at pH 7.4 than at pH 6.0 or pH 6.5, and similar binding affinities at pH 6.0 and 6.5. The binding affinities of Cetuximab for sEGFR were similar across the pH values within each buffer type. Within pH 6.0 and pH 6.5 conditions, Cetuximab exhibited a slightly lower affinity for sEGFR in PBS compared to in KRB, and similar affinities in PBS and KRB at pH 7.4. The Y104D and Y104D/Q111P mutants exhibited similar binding in PBS and KRB, within all three pH conditions. The binding affinities of all three antibodies were highest in the presence of 25% human serum. In the presence of 25% serum, the Y104D and Y104D/Q111P mutants and Cetuximab exhibited similar rates of association ($k_{on}$), but the Y104D and Y104D/Q111P mutants exhibited higher rates of dissociation ($k_{off}$), resulting in a decrease in the binding affinity ($K_D$) of the mutants. The difference in the rates of dissociation ($k_{off}$) between Cetuximab and the mutants was greatest at pH 7.4.

### Example 6

### EGFR Phosphorylation

The concentration of phosphorylated EGFR from human neonatal keratinocytes and A431 cells treated with the reference Cetuximab antibody or anti-EGFR antibody variants was measured by ELISA (RnD systems reagents, #DYC3570-2).

### 1. Preparation of Samples

Approximately 10,000 cells, human neonatal keratinocytes (Invitrogen C-001-5C) or A431 cells (ATCC CRL 1555); were plated in wells of a 96 well plate (BD Falcon #35-3072). After overnight incubation at 37° C in a humidified

atmosphere of 5% $CO_2$ incubator, the cells were washed, resuspended in serum free Dulbecco's Modified Eagle Medium (DMEM) and incubated overnight under the same conditions. The cells were washed with cold Phosphate Buffered Saline (PBS; 137 mM NaCl, 2.7 mM KCl, 8.1 mM $Na_2HPO_4$, 1.5 mM $KH_2PO_4$, pH 7.2 - 7.4). Then, the plates were divided into two groups. In the first group, 10 $\mu$g/mL purified Cetuximab or HC-Y104D anti-EGFR antibody or buffer control was added in phosphate buffer adjusted to pH 7.4. In the second group, purified Cetuximab or HC-Y104D anti-EGFR antibody or buffer control was added in phosphate buffer adjusted to pH 6.5. For A431 cells, a dose-response also was performed, whereby Cetuximab or HC-Y104D anti-EGFR antibody were added to samples at a concentration of 30 $\mu$g/mL, 10 $\mu$g/mL, 3.33 $\mu$g/mL, 1.11 $\mu$g/mL, 0.37 $\mu$g/mL, 0.123 $\mu$g/mL and 0.001 $\mu$g/mL. The cells were incubated for 15-30 minutes at 37°C.

After the initial incubation with antibody, EGF (RnD Systems, catalog no. 236-E) (100 $\mu$g/mL) was also added separately to cells in the same buffer as the antibody. Control cells were also tested where no antibody was added (EGF only) or where no antibody or EGF was added (no Rx). The cells were incubated for 15-30 minutes at 37°C. After incubation with the antigen, the cells were washed with cold PBS, and cold lysis buffer (1% NP-40 Alternative, 20 mM Tris (pH 8.0), 137 mM NaCl, 10% glycerol, 2 mM EDTA, 1 mM activated sodium orthovanadate, 10 $\mu$g/mL Aprotinin, 10 $\mu$g/mL Leupeptin) was added. The lysate was collected and was assayed immediately or stored at $\leq$ -70°C.

**2. ELISA**

A 96 well Microplate (Costar #2592) was coated with rat anti-human anti-phospho EGFR capture antibody (8.0 $\mu$g/mL in PBS, 100 $\mu$L/well) (R&D Systems # 842428) overnight at room temperature. Each well was aspirated, and washed with wash buffer (0.05% TWEEN® 20 in PBS, pH 7.2 - 7.4 (R&D Systems # WA126) for a total of five washes. Plates were blocked for 1-2 hours at room temperature with 300 $\mu$L of Block Buffer (1% BSA, 0.05% $NaN_3$ in PBS, pH 7.2-7.4). The wells were aspirated and washed with wash buffer for a total of five washes. Cell lysate (Cetuximab, Y104D anti-EGFR antibody, EGF only or No Rx) was diluted in IC Diluent (1% NP-40 Alternative, 20 mM Tris (pH 8.0), 137 mM NaCl, 10% glycerol, 2 mM EDTA, 1 mM activated sodium orthovanadate) and 100 $\mu$L were added. The aspiration and wash steps were repeated, and 100 $\mu$L mouse anti-human phospho-EGF R (Y1068) antibody conjugated to HRP (20 mM Tris (pH 8.0), 137 mM NaCl, 0.05% TWEEN® 20, 0.1% BSA) was added. Plates were sealed and incubated for 2 hours at room temperature. The aspiration and wash steps were repeated. Substrate (1:1 mixture of $H_2O_2$ and Tetramethylbenzidine (R&D Systems, Catalog # DY999)) (100$\mu$L) was added to each well and the plate was incubated for 20 minutes at room temperature. Stop solution (2 N $H_2SO_4$ (R&D Systems, Catalog # DY994) (50$\mu$L) was added, and the optical density (OD) of the wells were measured immediately in a microplate reader set to 450 nm with a wavelength correction at 540 nm or 570 nm.

**3. Results**

**a) A431 cells**

The results showed that EGF antigen induced phosphorylation of EGFR (see Figure 3A). In samples from A431 cells pre-treated with antibody at pH 6.5 and 7.4, the results showed that the presence of anti-EGFR Cetuximab antibody inhibited EGF-induced phosphorylation of EGFR such that the levels of phosphorylated EGFR (EGFR-P) was comparable to control cells that were not stimulated with EGF. Pre-incubation of cells with the variant HC-Y104D variant also inhibited EGF-induced phosphorylation, although to a lesser degree than reference Cetuximab. The effect of the variant HC-Y104D on inhibiting EGF-induced phosphorylation of EGFR was greater at pH 6.5.

The inhibitory effect of the antibodies was dose-dependent (Figure 3B). The concentration of phosphorylated EGFR was plotted against the concentration of antibody (Cetuximab or HC-Y104D anti-EGFR antibody). For the reference Cetuximab antibody (WT), the inhibitory effect was observed beginning at concentrations of antibody greater than 1 $\mu$g/mL and plateaued at concentrations greater than 10 $\mu$g/mL. The inhibitory effect of the reference Cetuximab antibody was similar at pH 6.5 and 7.4. For cells pre-incubated with HC-Y104D, the inhibition of EGF-induced phosphorylation was also observed beginning at a concentration of 1 $\mu$g/mL, although the inhibition was less than for the reference WT antibody. At 30 $\mu$g/mL inhibition had not yet plateaued. The results show, however, that pre-incubation of HC-Y104D at pH 6.5 resulted in a greater inhibitory effect than was observed at pH 7.4.

**b) Neonatal Keratinocytes**

Similar results were observed in samples from Neonatal Keratinocytes (see Figure 3C). At pH 6.5 and pH 7.4, the reference Cetuximab antibody resulted in a similar inhibition of EGF-induced phosphorylation. At pH 7.4, pre-incubation of cells with the HC-Y104D antibody did not result in any inhibition of EGF-induced phosphorylation. At pH 6.5, however, EGF-induced phosphorylation of EGFR was reduced by approximately one fourth compared to samples without antibody demonstrating that the variant antibody exhibited greater activity at pH 6.5 compared to pH 7.4

**Example 7**

**Growth of Human and neonatal keratinocytes in the presence of Cetuximab or HC-Y104D anti-EGFR antibody**

The growth of Human neonatal keratinocytes (Invitrogen C-001-5C) and Human adult keratinocytes (Invitrogen C-005-5C) was measured after incubation with Cetuximab or HC-Y104D anti-EGFR antibody. Cetuximab or HC-Y104D anti-EGFR antibody was added to normal growth medium (10% FBS, DMEM (pH 7.4)) to a concentration of 10 μg/mL, 3.33 μg/mL, 1.11 μg/mL, 0.37 μg/mL, 0.123 μg/mL, 0.0411 μg/mL, 0.0137 μg/mL and 0.00457 μg/mL.

Human neonatal keratinocytes and human adult keratinocytes were added (1000 cells / well) to a 96-well plate (BD Falcon 35-3072) in the presence of the normal growth medium containing Cetuximab or HC-Y104D anti-EGFR antibody. Each condition was assayed in n=5 wells per condition. Cells were incubated for 5 days at 37° C in a humidified atmosphere of 5% $CO_2$ incubator. Cell growth was measured by CellTiter-Glo® Luminescent Cell Viability Assay (Promega Cat# G-7571) and expressed as percent surviving cells compared to control cells grown without antibody.

The results are set forth in Table 21 and Figure 4. In human adult keratinocytes (Figure 4A) and neonatal keratinocytes (Figure 4B), Cetuximab inhibited cell growth in a dose-dependent manner, and the percent surviving cells decreased as the antibody concentration increased. At the highest concentration of Cetuximab (10 μg/mL), 23% surviving cells were observed for human adult keratinocytes and neonatal keratinocytes. In the human adult keratinocytes and neonatal keratinocytes, cell growth did not decrease as the concentration of HC-Y104D anti-EGFR antibody increased. In human adult keratinocytes and neonatal keratinocytes, at antibody concentrations of 0.0411 μg/mL, 0.0137 μg/mL and 0.00457 μg/mL, the percent surviving cells in assays with HC-Y104D was comparable to the percent surviving cells with Cetuximab. At antibody concentrations of 10 μg/mL, 3.33 μg/mL, 1.11 μg/mL, 0.37 μg/mL and 0.123 μg/mL, the percent surviving cells with HC-Y104D anti-EGFR antibody was significantly higher than the percent surviving cells with Cetuximab in human adult keratinocytes and neonatal keratinocytes. This demonstrates that the reference anti-EGFR Cetuximab antibody inhibits the growth of neonatal keratinocytes, but that the anti-EGFR antibody variant HC-Y104D does not.

| Table 21. Percent surviving cells for adult keratinocytes and neonatal keratinocytes with Cetuximab and HC-Y104D anti-EGFR antibody | | | | |
|---|---|---|---|---|
| **Concentration (μg/mL)** | **Percent surviving cells (Adult Keratinocytes)** | | **Percent surviving cells (Neonatal Keratinocytes)** | |
| | **Cetuximab** | **HC-Y104D** | **Cetuximab** | **HC-Y104D** |
| 10 | 23% | 87% | 23% | 83% |
| 3.33 | 34% | 103% | 36% | 91% |
| 1.11 | 41% | 90% | 42% | 86% |
| 0.37 | 57% | 117% | 57% | 116% |
| .123 | 62% | 83% | 65% | 98% |
| .0411 | 64% | 65% | 72% | 68% |
| 0.0137 | 65% | 71% | 90% | 82% |
| 0.00457 | 68% | 76% | 104% | 95% |

**Example 8**

**Effects of Cetuximab or HC-Y104D anti-EGFR antibodies on Tumor Growth in Xenograft Models**

A431 epidermoid carcinoma cells, FaDu hypopharyngeal carcinoma cells, and engineered cell lines A431LDHA and A431CA9, derived from A431 cells, were used to generate xenograft tumor models, which were used to evaluate the antitumor activity of Cetuximab and HC-Y104D anti-EGFR antibodies.

**1. Subcutaneous A431 Tumors**

An A431 Epidermoid Carcinoma xenograph model was used to evaluate and compare the antitumor activities of Cetuximab and HC-Y104D anti-EGFR antibodies.

**a. Cetuximab Dose Response**

Male, athymic NCr-nu/nu mice were inoculated with a 0.1 mL subcutaneous (SC) injection of $3.3 \times 10^6$ A431 epidermoid carcinoma cells (ATCC CRL1555) suspended in RPMI-1640 medium into their right flanks. When tumors reached a size of ~100 mm³, animals were randomized into six study groups (n=5/group; 30 total animals) that received either vehicle (Cetuximab buffer), 1.2, 4, 12, 40 or 80 mg/kg body weight Cetuximab by intraperitoneal (IP) administration twice weekly on days 0, 5, 7, 11, and 14 of the study. Tumor growth, measured as tumor volume (mm³), was determined twice per week using digital calipers, with tumor volume calculated according to the formula ($\frac{1}{2}*L*W^2$), where L (Length) is the longest diameter of the tumor and W (Width) is the longest diameter perpendicular to the "Length" of the measurement. Specifically, tumor volume was measured on days 0, 5, 7, 11 and 14.

The tumor volume increased linearly over the course of the study in the vehicle only control animals. The tumor volume of the tumors in the mice administered Cetuximab exhibited slower growth at days 5 and 7 than the vehicle only control. After day 7, the tumor growth of the Cetuximab-treated tumors was arrested at the size measured on day 7 for the remainder of the study. The reduction in tumor growth observed was dose-dependent, ranging from a 50% reduction to almost no tumor growth in mice administered Cetuximab at 80 mg/kg body weight.

**b. Cetuximab vs. HC-Y104D**

The A431 xenograft model was generated as described above. When the tumors reached a size of -100 mm³, animals were randomized into five study groups (n=4/group): (1) Group 1 - vehicle (Cetuximab buffer), (2) Group 2 - 0.1 mg/mouse (4 mg/kg body weight) Cetuximab, (3) Group 3 - 1.0 mg/mouse (40 mg/kg body weight) Cetuximab, (4) Group 4 - 0.1 mg/mouse (4 mg/kg body weight) HC-Y104D anti-EGFR antibody, or (5) Group 5 - 1.0 mg/mouse (40 mg/kg body weight) HC-Y104D anti-EGFR antibody. Mice were intraperitoneally (IP) administered 0.1 mg or 1.0 mg of Cetuximab reference or HC-Y104D anti-EGFR antibody variant or vehicle control twice weekly on days 0, 4, 7, 11, and 14. Tumor growth was measured as described above on days 0, 4, 7, 11, 14 and 18, except that in animals treated with vehicle the last tumor measurement was on day 14 because animals were sacrificed. Tumor Growth Inhibition (TGI) for the Cetuximab or HC-Y104D anti-EGFR antibody treatment groups was calculated using the formula: %TGI = [1 - ($T_B$-$T_A$) / ($C_B$-$C_A$)] × 100; where $T_B$ is the average tumor volume (mm³) in the treatment group at 14 days after initiation of treatment, $T_A$ is the average tumor volume (mm³) in the treatment group at day 0 before treatment, $C_B$ is the average tumor volume in the control group at 14 days after initiation of treatment, and $C_A$ is the average tumor volume in the control group at day 0 before treatment (see, e.g., Teicher BA and Andrews PA: Anticancer Drug Development, Guide: Preclinical Screening, Clinical Trials and Approval, 2nd edition. Humana Press, Totowa, New Jersey, pp. 134, 2004 and T. Friess et al., (2006) Anticancer Research 26:3505-3512).

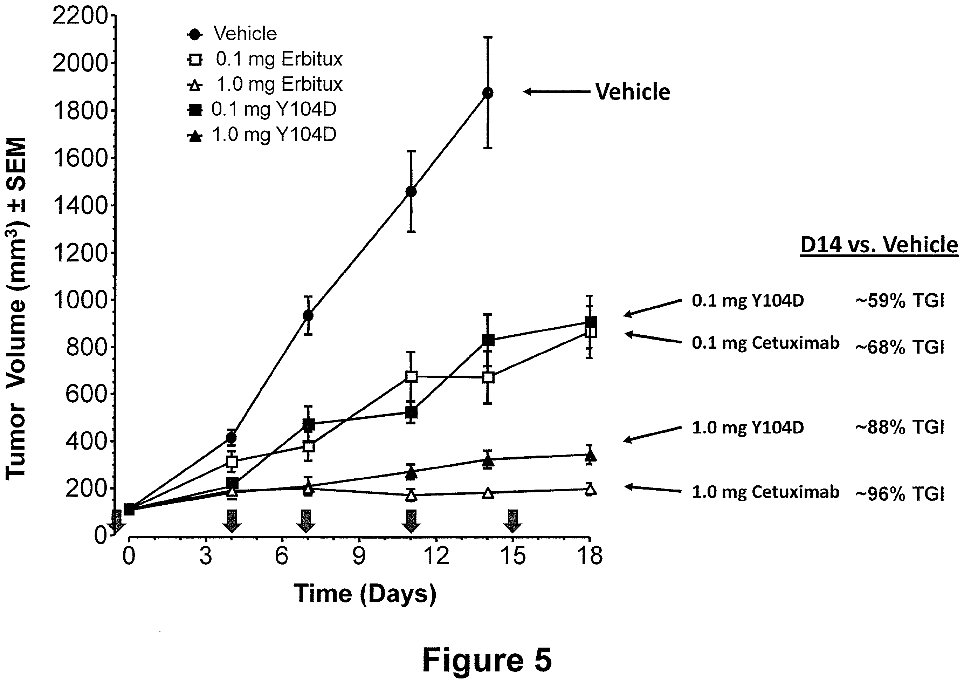

The results showed that in the absence of antibody, tumor volume steadily increased over time in the presence of vehicle only (Figure 5). Tumor volumes were comparably reduced in animals treated with reference Cetuximab or HC-Y104D anti-EGFR variant antibody compared to vehicle only control at both tested concentrations, with minimal tumor growth occurring in animals treated with 1.0 mg of antibody. On day 14, animals treated with 0.1 mg HC-Y104D anti-EGFR antibody and 0.1 mg Cetuximab had 59% and 68% tumor growth inhibition, respectively. On day 14, animals treated with 1.0 mg HC-Y104D anti-EGFR antibody and 1.0 mg Cetuximab had 88% and 96% tumor growth inhibition, respectively. Thus, the results show that *in vivo* the efficacy of the HC-Y104D anti-EGFR variant was similar to the reference Cetuximab antibody.

**2. Subcutaneous FaDu tumors**

An FaDu hypopharyngeal carcinoma xenograph model was used to evaluate and compare the antitumor activities of Cetuximab and HC-Y104D anti-EGFR antibodies.

**a. Cetuximab Dose Response**

Male, athymic NCr-nu/nu mice were inoculated with a 0.1 mL subcutaneous (SC) injection of $5.0 \times 10^6$ FaDu hypopharyngeal carcinoma cells (ATCC) suspended in RPMI-1640 medium into their right flanks. When tumors reached a size of ~100 mm³, animals were randomized into six study groups (n=7/group) that received either vehicle (Cetuximab buffer), 1.2, 4, 12, 40, or 80 mg/kg body weight Cetuximab by intraperitoneal (IP) administration twice weekly on days 0, 3, 7, 10, and 14 of the study. Tumor growth was measured as described above on days -1, 3, 7, 10 and 14.

Animals administered vehicle only exhibited steady growth over the course of the study. The tumors in animals administered 1.2 mg/kg Cetuximab grew about 50% less rapidly than the vehicle-treated controls. Administration of 4, 12, 40, or 80 mg/kg Cetuximab completely arrested the tumor growth at all time points measured. These results indicate that the FaDu xenograft model is more sensitive to Cetuximab than the A431 xenograft model.

### b. Cetuximab vs. HC-Y104D

The FaDu xenograft model was generated as described above. When the tumors reached a size of -100 mm$^3$, animals were randomized into five study groups (n=4/group): (1) Group 1 - vehicle (Cetuximab buffer), (2) Group 2 - 4 mg/kg Cetuximab, (3) Group 3 - 40 mg/kg Cetuximab, (4) Group 4 - 4 mg/kg HC-Y104D anti-EGFR antibody, or (5) Group 5 - 40 mg/kg HC-Y104D anti-EGFR antibody. The antibodies or vehicle only were administered intraperitoneally (IP) twice weekly on days 0, 3, 7, and 10. Tumor growth was measured using calipers as described above on days -1, 3, 7, 10, 14 just prior to antibody administration.

Animals administered vehicle only exhibited steady growth over the course of the study. Treatment with 4 mg/kg or 40 mg/kg of variant anti-EGFR steadily reduced the tumor growth over time with about 40% and 70%, respectively, reduction in tumor growth at day 14. Treatment with 4 mg/kg or 40 mg/kg Cetuximab completely arrested the tumor growth for the course of the study.

### Example 9

### Binding of anti-EGFR antibody Y104D to A431 Subcutaneous Tumors or Skin Grafts Ex Vivo

### 1. Ex Vivo Binding Studies to Subcutaneous Tumors

### a. EGFR expression in Subcutaneous tumors

Immunohistochemistry (IHC) was used to assess the levels of EGFR expression in A431 human tumors grown as xenografts in nude mice as described in Example 8.1. A431 subcutaneous tumors were harvested and fixed in 10% neutral buffered formalin (NBF) and embedded in paraffin. Five (5) $\mu$m sections were mounted on slides and dried. Prior to staining, the slides were deparaffinized and rehydrated. Sections were immunolabeled using an EGFR IHC kit (Dako, Carpinteria, CA). Staining was visualized with 3,3'-diaminobenzidine (DAB) according to the manufacturer's instructions. Nuclei were counterstained with hematoxylin. Micrographs were captured with a Nikon Eclipse TE2000U microscope coupled to a Insight FireWire digital camera (Diagnostic Instruments, Michigan). The intense cell membrane positivity for EGFR in the tumor cells confirmed that xenograft tumors derived from A431 cells retain high levels of EGFR expression.

### b. Binding of Anti-EGFR Antibodies to Subcutaneous Tumors

Immunofluorescence (IF) was used to assess the ability of Cetuximab and HC-Y104D to bind, and therefore label, human EGFR. Erbitux and HC-Y104D were conjugated to DyLight$^{594}$ at 10, 5, 1, 0.3, 0.1 $\mu$g/mL in PBS using the DyLight 594 Antibody Labeling Kit (Thermo Scientific; Rockford, IL), according to the manufacturer's instructions. Following sectioning, frozen sections of A431 tumors were fixed for 10 min in cold acetone and incubated for one hour with 5 $\mu$g/mL or 1 $\mu$g/mL of either DyLight$^{594}$-conjugated Cetuximab or HC-Y104D antibody. After washing in PBS, sections were counter-stained with DAPI (4',6-Diamidino-2-Phenylindole, Dihydrochloride) (Molecular Probes, Eugene). Micrographs were captured, with 20x and 40x objectives, using a Nikon Eclipse TE2000U microscope coupled to a Insight FireWire digital camera (Diagnostic Instruments, Michigan) using the same settings for each image to allow for comparison between experimental conditions.

Both Cetuximab and HC-Y104D antibodies demonstrated intense immunolabeling of the A431 solid tumors. The labeling intensity with the HC-Y104D antibody was lower compared to that of Cetuximab at each concentration (5 $\mu$g/mL and 1 $\mu$g/mL), although the intensity obtained with 5 $\mu$g/mL HC-Y104D antibody was comparable to that observed using 1 $\mu$g/mL Cetuximab.

### 2. Ex Vivo Binding Studies to Primate Skin

### a. EGFR Expression in Primate Skin

Immunohistochemistry (IHC) was used to assess the levels of EGFR expression in human and non human primate skin samples. Human skin samples were obtained from a local surgical center; cynomolgus monkey, marmoset monkey and squirrel monkey skin were received from Worldwide Primates Inc.(Miami, FL). Formaldehyde fixed samples of human, cynomolgus monkey, marmoset monkey and squirrel monkey were sectioned and processed for IHC with the EGFR IHC kit (DAKO) as described above. Nuclei were counterstained with hematoxylin. Micrographs were captured above, with 20x and 40x objectives.

As expected, the membranes of the basal keratinocytes exhibited intense staining for EGFR. The basal keratinocytes of cynomolgus and squirrel monkey skin tissues also exhibited staining, with the cynomolgus skin staining with slightly less intensity than that observed for human and squirrel monkey tissues. The marmoset monkey skin did not exhibit any detectable staining, even when using a 40x objective. These results indicate that cynomolgus monkey and squirrel monkey EGFR, but not Marmoset monkey EGFR, are sufficiently similar to human EGFR to be recognized by the anti-human EGFR monoclonal antibody.

**b. Binding of Anti-EGFR Antibodies to Frozen Skin Samples**

Immunofluorescence (IF) was used to assess the ability of Cetuximab and HC-Y104D to bind, and therefore label, human EGFR in skin samples. Cryosections of human, cynomolgus monkey, marmoset monkey and squirrel monkey (human skin received from a local surgical center; cynomolgus monkey, marmoset monkey and squirrel monkey skin received from Worldwide Primate, Florida), were directly immunolabeled at neutral pH as described above, using 1.0 $\mu$g/mL Cetuximab or HC-Y104D conjugated to Alexafluor 594 (Thermo Scientific DyLight 594 Antibody Labeling Kit; Rockford, IL). Nuclei were counterstained with DAPI. Micrographs were captured as described above, using 20x and 40x objectives.

Cetuximab demonstrated intense immunolabeling of pre-keratinocytes and basal cells in the human tissue and, to a lesser extent, in cynomolgus skin samples. HC-Y104D antibody demonstrated much lower immunolabeling intensity of the pre-keratinocytes and basal cells in the dermis of human skin and cynomolgus monkey skin compared to Cetuximab-labeled sections. Neither Cetuximab nor HC-Y104D exhibited detectable labeling in squirrel monkey skin nor marmoset monkey skin.

**Example 10**

**Selective Binding of Fluorescently-labeled Anti-EGFR antibody Y104D to Tumors versus Skin *In Vivo***

Cetuximab, Y104D anti-EGFR antibody, and a control Human IgG were labeled at room temperature for 60 minutes with DyLight755 Sulfydryl-Reactive Dye (DL755) (Thermo Scientific, Rockford, IL), a near-IR fluor. The binding of DL755 labeled IgG, Cetuximab, and HC-Y104D to xenograft tumors or human or monkey skin grafts was assessed using the IVIS Caliper fluorescent imaging system with an excitation wavelength of 745nm and an emission wavelength of 800 nm. Images were captured before administration of antibody and at 1 minute, 2 minutes, 10 minutes, 60 minutes, 120 minutes, 240 minutes, 360 minutes, 1 day, and daily after administration of the antibodies. In the human skin graft models, images were also captured at 10 days post administration of the antibodies.

**1. Cetuximab and HC-Y104D Binding to Subcutaneous A431 Tumors**

A431 xenograft tumors were produced by injecting A431 cells into the right flanks of nude mice as described in Example 8 above. 21 days post-implantation, the mice were administered 10 $\mu$g/mouse (0.5 mg/kg) Human IgG$^{DL755}$, HC-Y104D$^{DL755}$, or Ceuximab$^{DL755}$ (n=4/group). The DL755 label was detected, in 4 animals/group, using the IVIS Caliper fluorescent imaging system with an excitation wavelength of 745nm and an emission wavelength of 800nm. Images were captured before administration of antibody and at 4 hr after administration of the antibodies, and then daily for 7 days.

The binding of the negative control antibody, human IgG$^{DL755}$, to the tumor mass was minimal at 4 hr post-administration, but increased slightly (about 3 fold) over the 7 days of the study. The binding demonstrated by Cetuximab$^{DL755}$ was greater than that for Y104D $^{DL755}$ for the first 4 days of the study, reaching a peak intensity of about 13 times greater than the baseline signal (the IgG$^{DL755}$ signal at 4 hr post administration) on day 4. After day 4, the Cetuximab signal decreased slightly to approximately 11-11.5 times the baseline signal. The signal for tumor-bound Y104D $^{DL755}$ increased steadily over the course of the study to about 11-11.5 fold greater than baseline on days 6 and 7. The signals from both anti-EGFR antibodies (Cetuximab $^{DL755}$ and Y104D $^{DL755}$) covered the entire surface of the tumors and were much greater than the signal detected for tumor-bound IgG $^{DL755}$ at every time point.

Immunohistochemical staining of human IgG$^{DL755}$, HC-104D$^{DL755}$, and Ceuximab$^{DL755}$-injected mice, by $F_c$ detection, was used to assess the localization of antibody binding in more detail. As described above, nude mice were injected with A431 cells in the right flanks to generate A431 tumors. On day 21 post-A431 cell implantation, the mice were administered a single i.v. dose of IgG$^{DL755}$, Y104D$^{DL755}$, or Cetuximab$^{DL755}$ at 1 mg/mouse (n=2/group). 48 hr after the dose of antibody, the tumors were visualized using the IVIS Caliper fluorescent imaging system as described above. Following tumor imaging, the mice were perfused, the tumors were harvested, and cryosections of the tumor were incubated with HRP-conjugated goat anti-human IgG secondary antibody for detection of the $F_c$ regions of the injected antibodies using standard methods as described above. DAB was used as the HRP substrate to enable visualization. The stained tissues were examined using a Nikon Eclipse TE2000U microscope coupled to a Insight FireWire digital camera (Diagnostic Instruments, Michigan) equipped with a 20x objective.

The tumors from animals injected with IgG$^{DL755}$ exhibited diffuse, non-specific IgG staining. In contrast, the tumors injected with Y104D$^{DL755}$ or Cetuximab$^{DL755}$ exhibited distinct, membrane-specific binding. These results are consistent with Y104D$^{DL755}$ and Cetuximab$^{DL755}$ antibodies binding the EGFR target on the surface of cell membranes in the A431 tumors.

**2. Cetuximab and HC-Y104D Binding to Subcutaneous PC-3 Tumors**

Xenograft tumors derived from PC-3 cells were generated by injecting $2x10^6$ PC-3 cells (Caliper Life Sciences), in 100 $\mu$l serum-free Opti-MEM®, into the right peritibial muscle of male nude mice. 35 days after implantation, the PC-3 tumor bearing mice were administered 10 $\mu$g/mouse (0.5 mg/kg) human IgG$^{DL755}$, HC-Y104D$^{DL755}$, or Ceuximab$^{DL755}$ (n=2/group), and the DL755 label was detected using the IVIS Caliper fluorescent imaging system as described above. Images were captured before administration of antibody and then daily for 5 days. A parallel immunohistochemical study also was performed on the PC-3 xenograft tumors, 48 hr. following 1mg/mouse i.v. administrations of human IgG (control), HC-Y104D, or Cetuximab by $F_c$ detection as described above.

Mice administered human IgG$^{DL755}$ exhibited some low DL755 signal that localized to the tumor site. Mice administered HC-Y104D$^{DL755}$ or Cetuximab$^{DL755}$ exhibited more tumor-localized DL755 signal than that observed in animals administered human IgG$^{DL755}$, but the signals were reduced compared to the corresponding signals observed in Example 9.2.a. The $F_c$-detection immunohistochemical study revealed non-specific staining for all three antibodies. No membrane-specific staining was observed, indicating human IgG, HC-Y104D, and Cetuximab may generally accumulate within EGFR poor PC3 tumors, rather than specifically binding to the tumor surface.

As a model to assess skin toxicity, binding of a fluorescently-labeled Cetuximab and HC-Y104D anti-EGFR antibody to human and monkey skin grafts implanted in mice was assessed *in vivo.* Cetuximab, Y104D anti-EGFR antibody, and a control Human IgG were labeled at room temperature for 60 minutes with DyLight755 Sulfydryl-Reactive Dye (DL755) (Thermo Scientific, Rockford, IL), a near-IR fluor.

**3. Binding of Cetuximab and HC-Y104D anti-EGFR Antibodies to Human Skin Grafts**

Human split thickness skin graft (STSG) (human skin received from a local surgical center) and human foreskin grafts (purchased from NDRI (1628 JFK Blvd, 8 Penn Center, Philadelphia, PA) were surgically transplanted on the left dorsal flank in Ncr nu/nu mice. EGFR expression was confirmed in the human skin grafts on days 70 and 32 post implantation, respectively, by anti-EGFR IHC kit (Dako). On day 32 and day 36 post-implantation, the labeled antibodies were administered by i.v. to mice with human skin grafts at a dose of 300 $\mu$g/mouse.

In the human STSG mouse models, a circulating systemic signal was observed in all mice at one hour post administration, consistent with circulating labeled antibody. This circulating signal lasted for approximately 7 or 8 days. In mice administered DL755 labeled Cetuximab, on day 1 post-administration, signal with greater intensity than the systemic signal was detected at the site of the skin graft. This signal was visible in the images taken on each of days 1-10 post-administration. In mice administered HC-Y104D modified anti-EGFR antibody or control human IgG antibody, at all time points tested, very minimal signal above the systemic signal was observed at the skin graft locations. At each time point measured, signal at the site of the tumor graft was significantly greater in mice administered the Cetuximab antibody than in mice administered the HC-Y104D modified anti-EGFR antibody.

These results were confirmed in a follow-up study, wherein on day 21 post implantation, mice receiving human foreskin grafts were analyzed for antibody binding, using the same method (n=3/group), before and 4 hr after intravenous administration of 10 $\mu$g/mouse (0.5 mg/kg) human IgG$^{DL755}$, Y104D$^{DL755}$, or Cetuximab$^{DL755}$ and then daily thereafter for a total of 6 days. At day 1 post-administration, signal with greater intensity than the systemic signal was detected at the site of the skin graft in mice administered Y104D$^{DL755}$ or Cetuximab$^{DL755}$. The skin graft binding, evidenced by signal intensity, in Cetuximab$^{DL755}$ -administered mice increased until day 3 and then remained at the same level for the remaining 3 days of the study. The skin graft binding in mice administered Y104D$^{DL755}$, remained about the level observed at day 1 for the remaining days of the study. Minimal binding of human IgG$^{DL755}$ to the skin graft was observed over the course of the study. These results indicate Cetuximab$^{DL755}$ exhibits greater binding to epitopes in human skin than Y104D$^{DL755}$. The binding results of IgG$^{DL755}$, Y104D$^{DL755}$, or Cetuximab$^{DL755}$ to human foreskin grafts were verified by immunohistochemistry. On day 28 post-implantation, mice receiving human foreskin grafts were administered a single i.v. dose of IgG, Y104D, or Cetuximab at 1 mg/mouse. 48 hr after the dose of antibody, the mice were perfused and cryosections were incubated with HRP-conjugated goat anti-human IgG secondary antibody. DAB was used as the HRP substrate. The stained tissues were examined using a Nikon Eclipse TE2000U microscope coupled to a Insight FireWire digital camera (Diagnostic Instruments, Michigan) equipped with a 40x objective. Consistent with the in vivo studies, tissues from mice administered Cetuximab exhibited the most binding to the human skin graft; tissues from mice administered Y104D exhibited much reduced staining compared to the Cetuximab-treated sections; and staining was undetectable in tissues of mice administered human IgG.

### 4. Binding of Cetuximab and HC-Y104D anti-EGFR Antibodies to Monkey Skin Grafts

Monkey STSG (cynomolgus monkey skin received from BioTox) were surgically transplanted on the left dorsal flank in 7 Ncr nu/nu mice. EGFR expression was confirmed in the monkey skin grafts on days 70 and 32 post implantation, respectively, by anti-EGFR IHC kit (Dako). On day 32 and day 36 post-implantation, the labeled antibodies were administered by i.v. to mice with monkey skin graft models at a dose of and 30 μg/mouse.

In mice containing monkey STSG skin grafts, a circulating systemic signal was observed in all mice at one hour post administration, consistent with circulating labeled antibody. This circulating signal lasted for approximately 5-7 days. In mice administered DL755 labeled Cetuximab, signal above the systemic signal was detected in the skin graft on each of days 1-9 post-administration. In mice administered HC-Y104D modified anti-EGFR antibody, signal above the systemic signal was detected on each of days 1-9 post-administration, but with significantly less intensity on all days measured than the signal observed in mice that were administered Cetuximab. In mice administered control human IgG antibody, only a faint signal was observed at the skin graft location on each of days 1-9.

### 5. A431 Tumor vs. skin binding

The quantitated fluorescent signal intensities were used to determine the ratio of antibody tumor: skin binding for Cetuximab and HC-Y104D antibodies by dividing the DL755 signal intensity of the tumor binding, determined in Example 10.1, by the corresponding DL755 signal intensity of the human skin graft binding from the same antibody determined in Example 10.2 (n=2/group). The ratios were then normalized to the tumor: skin binding ratio calculated for the control IgG-administered animals.

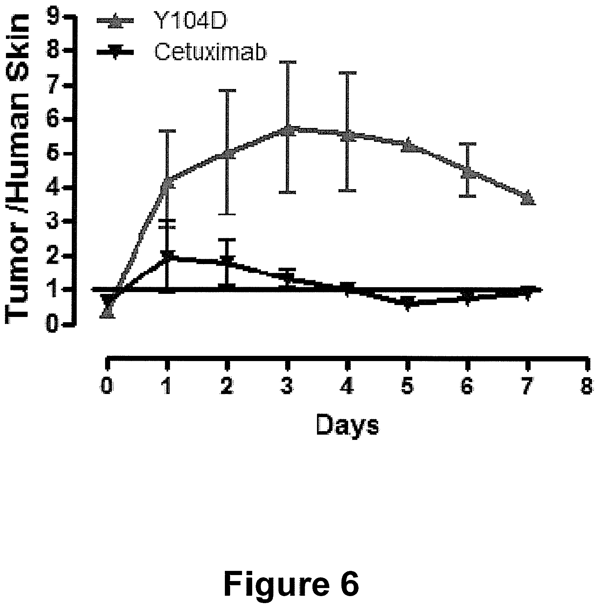

The results are set forth in Figure 6. The Cetuximab tumor binding was approximately equal to the skin binding at all time points, yielding a tumor: skin binding ratio of approximately 1 at each time point. In contrast, HC-Y104D tumor binding was much greater than Y104D skin binding. The tumor:skin binding ratio was approximately 4 to 5.5 at each time point. These results demonstrate that HC-Y104D preferentially and selectively binds the tumor cells compared to the skin graft.

### Example 11

### Effects of Cetuximab on Skin Toxicity in a Skin Graft Model

Donor skin from the palpebral fissure of a patient was harvested and split thickness skin grafts were transplanted to 4 Ncr nu/nu mice. Starting on day 15 post skin transplantation, two of the mice were each intravenously administered 2 mg Cetuximab (100 mg/kg, HED 60 mg/kg) twice weekly for 4 weeks. On day 35 post-Cetuximab, 7 days after the final dose of Cetuximab, the condition of the skin grafts were visually assessed. Samples of donor skin and grafted skin, containing both the human donor skin graft and the host mouse skin, were collected and analyzed by immunohistochemistry using the anti-EGFR IHC kit (Dako).

On day 35 post commencement of Cetuximab treatment, the skin grafts of the mice that did not receive Cetuximab, were integrated into the skin. In contrast, the skin grafts of mice receiving Cetuximab, on day 35 post commencement of treatment, had shrunk to less than half the size of the original skin graft and the interface between the skin graft and the host skin was red and irritated, indicating Cetuximab stimulated a response against the human tissue.

IHC analysis of the donor skin revealed strong HuEGFR staining. IHC analysis of the graft site, containing a region of both human graft and mouse skin, revealed strong HuEGFR staining in the human graft pre-keratinocytes and basal cells and no staining in the mouse pre-keratinocytes or basal cells of the adjacent mouse skin.

### Example 12

### Anti-EGFR Antibody and Chemotherapy Combinatorial Treatment

The efficacies of Cetuximab and HC-Y104D anti-EGFR antibodies in combination with the chemotherapeutic reagent, cisplatin, were evaluated for the inhibition of A431 xenograft tumor growth.

**1. Cetuximab and Cisplatin**

Subcutaneous A431 xenograft tumors were established in male nude mice as described in Example 8 above. When the tumors were approximately 100-200 mm$^3$ in size, animals were randomized into nine study groups (n=5/group), as set forth in Table 22, and administered Cetuximab by intraperitoneal administration twice per week and/or cisplatin twice per week by intravenous administration. Specifically, the test article(s) were administered on days 0, 4, 7, 11, and 14.

| Table 22. Cetuximab and/or Cisplatin Dose and Resulting Tumor Growth Inhibition | | | | | | |
|---|---|---|---|---|---|---|
| Group | Test Article(s) | Cetuximab Dose | | Cisplatin Dose | | % Tumor Growth Inhibition |
| | | mg/mouse | mg/kg | mg/mouse | mg/kg | |
| 1 | Vehicle | - | - | - | - | - |
| 2 | Cetuximab | 0.1 | 4 | - | - | 41.8 |
| 3 | Cetuximab | 0.3 | 12 | - | - | 65.9* |
| 4 | Cisplatin | - | - | 0.04 | 1.5 | 16.7 |
| 5 | Cisplatin | - | - | 0.125 | 5 | 34.2* |
| 6 | Cetuximab + Cisplatin | 0.1 | 4 | 0.04 | 1.5 | 53.2* |
| 7 | Cetuximab + Cisplatin | 0.1 | 4 | 0.125 | 5 | 50.2* |
| 8 | Cetuximab + Cisplatin | 0.3 | 12 | 0.04 | 1.5 | 74.1* |
| 9 | Cetuximab + Cisplatin | 0.3 | 12 | 0.125 | 5 | 85.2* |
| * = p < 0.05 vs. vehicle only | | | | | | |

Tumor growth, measured as tumor volume (mm$^3$), was determined on days - 1, 4, 7, 11 and 14 using digital calipers and calculation as described in Example 8. Tumor growth inhibition (TGI) for the treatment groups was calculated using the formula: $\%TGI = [1 - (T_B-T_A) / (C_B-C_A)] \times 100$; where $T_B$ is the average tumor volume (mm$^3$) in the treatment group at day 14, $T_A$ is the average tumor volume (mm$^3$) in the treatment group the day before the first treatment (day -1), $C_B$ is the average tumor volume in the vehicle only control group at day 14, and $C_A$ is the average tumor volume in the vehicle only control group the day before the first treatment (day -1) (see, *e.g.*, Teicher BA and Andrews PA: Anticancer Drug Development, Guide: Preclinical Screening, Clinical Trials and Approval, 2nd edition. Humana Press, Totowa, New Jersey, pp. 134, 2004 and T. Friess et al., (2006) Anticancer Research 26:3505-3512). The results are set forth in Table 22 above.

While 1.5 mg/kg cisplatin did not significantly inhibit tumor growth on its own, it did contribute to additional tumor growth inhibition when in combination with Cetuximab at 4 mg/kg (41.8% TGI for Cetuximab alone vs. 53.2% TGI for the combination) and at 12 mg/kg (65.9% TGI for Cetuximab alone vs. 74.1% TGI for the combination). Treatment with 5 mg/kg cisplatin alone resulted in 34.2% TGI and further contributed to TGI when in combination with Cetuximab at 4 mg/kg (41.8% TGI for Cetuximab alone vs. 50.2% TGI for the combination) and at 12 mg/kg (65.9% TGI for Cetuximab alone vs. 85.2% TGI for the combination). The maximum tumor growth inhibition was observed with 12 mg/kg Cetuximab + 5 mg/kg cisplatin.

**2. Cetuximab vs. HC-Y104D and Cisplatin**

Subcutaneous A431 xenograft tumors were established in male nude mice as described above. When the tumors were approximately 100 mm$^3$ in size, animals were randomized into eight study groups (n=5/group), as set forth in Table 23, and administered Cetuximab or HC-Y104D, by IP administration, and/or cisplatin, by IV administration, twice per week. Specifically, the test article(s) were administered on days 0, 4, 7 and 11. Tumor volume (mm$^3$) was determined on days -1, 4, 7, 11 and 14 as described previously.

| Table 23. Cetuximab, HC-Y104D and/or Cisplatin Dose | | | | | | | |
|---|---|---|---|---|---|---|---|
| Group | Test Article(s) | Cetuximab Dose | | HC-Y104D Dose | | Cisplatin Dose | |
| | | mg/mouse | mg/kg | mg/mouse | mg/kg | mg/mouse | mg/kg |
| 1 | Vehicle | - | - | - | - | - | - |

(continued)

| Table 23. Cetuximab, HC-Y104D and/or Cisplatin Dose | | | | | | | |
|---|---|---|---|---|---|---|---|
| Group | Test Article(s) | Cetuximab Dose | | HC-Y104D Dose | | Cisplatin Dose | |
| | | mg/mouse | mg/kg | mg/mouse | mg/kg | mg/mouse | mg/kg |
| 2 | Cetuximab | 0.3 | 12 | - | - | - | - |
| 3 | Cisplatin | - | - | - | - | 0.125 | 5 |
| 4 | Y104D | - | - | 0.3 | 12 | - | - |
| 5 | Y104D | - | - | 1.0 | 40 | - | - |
| 6 | Cetuximab + Cisplatin | 0.3 | 12 | - | - | 0.125 | 5 |
| 7 | Y104D + Cisplatin | - | - | 0.3 | 12 | 0.125 | 5 |
| 8 | Y104D + Cisplatin | - | - | 1.0 | 40 | 0.125 | 5 |

The average tumor volume of vehicle treated animals grew progressively over the course of the study until it reached approximately 2200 mm$^3$ on day 14. Administration of 5 mg/kg cisplatin reduced the tumor growth by about 45% by day 14. The tumors of mice receiving doses of 12 mg/kg Cetuximab exhibited about a 80% reduction in tumor growth. In this experiment no additive effect was observed for the combined treatment of 12 mg/kg Cetuximab and 5 mg/kg cisplatin. The tumors of mice receiving 12 mg/kg HC-Y104D were reduced in size by about 55% compared to the vehicle control group. The additional treatment of 5 mg/kg cisplatin did not reduce the tumor growth any further than was observed for 12 mg/kg HC-Y104D alone. Increasing the dose of HC-Y104D to 40 mg/kg yielded tumor growth inhibition that was similar to that observed for 12 mg/kg Cetuximab. There was no additional tumor inhibition observed when 40 mg/kg HC-Y104D was administered in combination with 5 mg/kg cisplatin.

**Example 13**

**Effect of Anti-EGFR Antibody-Drug Conjugates (ADCs) on Tumor Cell and Keratinocyte Cell Growth Inhibition**

Anti-EGFR antibody-drug conjugates (ADCs) were generated by fusing the immunotoxin Saporin to Cetuximab, HC-Y104D and HC-Y104D/Q111P anti-EGFR antibodies by either mixing biotinylated antibodies and streptavidin-Saporin (Advanced Targeting Systems Bio, Cat# IT-27) or using a cleavable protein crosslinker (service provided by Advanced Targeting Systems Bio) to permit drug release inside the target cells.

For biotin-streptavidin based ADC formation, antibodies at a concentration of 1-2 mg/ml in 0.1 M phosphate buffer, pH 7.2 were oxidized, converting adjacent hydroxyl groups of the antibody sugar chains into aldehyde groups, using sodium periodate (NaIO$_4$) at a final concentration of 5 mg/ml, 4 °C for 30 min. The oxidized antibodies were dialyzed against 0.1 M phosphate buffer, pH 7.2. The dialyzed antibodies were then mixed with 50 mM hydrazide-biotin prepared in DMSO at volume ratio 9 to 1, resulting in 5 mM hydrazide-biotin in the reaction, and incubated at room temperature for 2 hours to form hydrazone bonds between the aldehyde groups of the antibodies and hydrazide groups. The biotinylated antibodies were dialyzed against 1XPBS, and mixed with streptavidin-saporin in equal molar ratio to form the antibody-saporin complex. The ADCs were then tested for their abilities to inhibit cell growth of human tumor cell lines, A431 and MDA-MB-468, and a human keratinocyte cell line, HEK-N.

**1. Saporin ADC Inhibition of A431 Cell Growth**

A431 cells were cultured in RPMI 1640 medium supplemented with 10% fetal bovine serum (FBS; Mediatech). The day before ADC treatment, A431 cells were seeded at 1,000 cells/well in 200 μL volume in clear bottom white 96-well plates. The cells were left untreated or were treated with the Saporin conjugated Cetuximab (Wt-Sap), Saporin conjugated Y104D (Y104D-Sap), Saporin conjugated Y104D/Q111P (YDQP-Sap), or Saporin-conjugated human IgG at increasing concentrations starting from 1 μg/mL. The cells were subjected to ADC treatment for 5 days. Live cells were measured on day 5 using the Cell Titer-glo Luminescent kit (Promega) according to the manufacturer's instructions. The percentages of surviving cells were calculated relative to untreated cells and EC50 values were computed using GraphPad Prism. The results are set forth below in Table 24. The results show that WT-Sap showed similar cell growth inhibition (CGI) activity as Y104D-Sap and TDQP-Sap on A431 Cancer cells.

**2. Saporin ADC Inhibition of Neonatal Keratinocyte (HEK-N) Cells**

Neonatal Keratinocyte (HEK-N) cells were cultured in growth factor supplemented Epilife medium (Gibco). The day prior to Saporin ADC treatment, HEK-N cells were seeded at 1,000 cells/ well in 200 μL volume in clear bottom white 96-well plates. The cells were left untreated or were treated with the Saporin conjugated Cetuximab (Wt-Sap), Saporin conjugated Y104D (Y104D-Sap), Saporin conjugated Y104DQ111P (YDQP-Sap), or Saporin-conjugated human IgG at increasing concentrations starting from a concentration of 1 μg/mL. The cells were subjected to ADC treatment for 5 days. Live cells were measured on day 5 using the Cell Titer-glo Luminescent kit (Promega) according to the manufacturer's instructions. The percentages of surviving cells were calculated relative to untreated cells and EC50 values were computed using GraphPad Prism. The results are set forth in Table 24. The results show that WT-Sap showed much greater (CGI) activity than Y104D-Sap and TDQP-Sap on keratinocytes.

| Table 24: EC50 for Cell Growth Inhibition (CGI) ng/mL | | | |
|---|---|---|---|
| | **WT-Sap** | **Y104D-Sap** | **YDQP-Sap** |
| **A431** | 0.7 | 1 | 2.4 |
| **Keratinocyte** | 0.2 | 15.8 | 22.5 |

**Example 14**

**Generation and Screening of a Second Combinatorial Library**

A second generation library of combinatorial anti-EGFR antibody mutants was generated to provide additional mutant anti-EGFR antibody candidates. The candidates were tested for selective binding under reduced pH conditions.

**1. Second Library Construction**

The second combinatorial library was generated by generating full-length anti-EGFR antibody mutants HC-S053G/Y104D and HC-S053G/Y104D/Q111P by site directed mutagenesis of HC-Y104D and HC-YI04D/Q111P using methods described in Example 2. The newly generated mutants and previously generated HC-Y104D and HC-Y104D/Q111P were then used as parental clones to which the mutations S025V, F027G, T030F and D072L were individually added to generate a library of 20 constructs as outlined in Table 25. All constructs were sequence verified.

**2. Screening of Second Combinatorial Library**

The constructs of the second combinatorial library and Cetuximab were transfected into CHO cells, using standard transfection procedures as described in Example 1 above, and the expression of the antibodies was determined by measuring the concentration in the supernatant as previously described (Example 1). The Results are set forth in Table 25. The results show that many clones of the mini-CPS library exhibit low expression.

**Table 25. Constructs of Second Combinatorial Library**

| Clone # | | SEQ ID NO | Transfection 1 (ng/mL) | Transfection 2 (ng/mL) |
|---|---|---|---|---|
| 2-1 | HC-Y104D | | 320.7 | 246.2 |
| 2-2 | HC-Y104D/Q111P | | 196.8 | 94.5 |
| 2-3 | HC-S053G/Y104D | | 341.8 | 223.7 |
| 2-4 | HC-S053G/ Y104D/Q111P | | 298.5 | 157.5 |
| | | | | |
| 2-5 | HC-S025V/Y104D | | 36.2 | 12.1 |
| 2-6 | HC-S025V/Y104D/Q111P | | 40.1 | 15.5 |
| 2-7 | HC-S025V/S053G/Y104D | | 87.5 | 36.0 |
| 2-8 | HC-S025V/S053G/ Y104D/Q111P | | 70.3 | 38.8 |

(continued)

| Clone # | | SEQ ID NO | Transfection 1 (ng/mL) | Transfection 2 (ng/mL) |
|---------|---|-----------|------------------------|------------------------|
| | | | | |
| 2-9 | HC- F027G/Y104D | | 0.7 | 3.5 |
| 2-10 | HC- F027G/Y104D/Q111P | | 0.5 | 2.4 |
| 2-11 | HC- F027G/S053G/Y104D | | 15.1 | 14.8 |
| 2-12 | HC- F027G/S053G/Y104D/Q111P | | 13.3 | 0.5 |
| | | | | |
| 2-13 | HC- T030F/Y104D | | 68.3 | 56.5 |
| 2-14 | HC- T030F/Y104D/Q111P | | 49.6 | 32.3 |
| 2-15 | HC- T030F/S053G/Y104D | | 67.3 | 69.2 |
| 2-16 | HC- T030F/S053G/ Y104D/Q111P | | 100.2 | 74.9 |
| | | | | |
| 2-17 | HC- D072L/Y104D | | 31.0 | 28.1 |
| 2-18 | HC- D072L/Y104D/Q111P | | 10.3 | 0.5 |
| 2-19 | HC-S053G/ D072L/Y104D | | 61.6 | 46.5 |
| 2-20 | HC-S053G/ D072L/Y104D/Q111P | | 55.9 | 18.9 |

The supernatants were then adjusted to concentrations of 4 ng/mL, 2 ng/mL and 1 ng/mL for testing EGFR binding at pH 6.0 and pH 7.4 using pH sensitive ELISA as described in Example 1. The transfection and pH sensitive ELISA were conducted twice for each construct at each concentration. The screening results are set forth in Table 26. The concentration of clones 2-9 and 2-10 were extremely low and were tested undiluted and at dilutions of 1:2 and 1:4.
Cetuximab bound EGFR similarly at pH 6.0 and pH 7.4. All mutant clones exhibited lower binding at pH 6.0 compared to clones HC-Y104D and HC-Y104D/Q111P, but some clones demonstrated binding at pH 7.4 that was reduced to background levels, resulting in higher pH 6.0/pH 7.4 ratios.

**Table 26.**

| clone | mutation | OD, pH 6.0 | | | | | | OD, pH 7.4 | | | | | | pH 6.0/pH 7.4 OD ratio | | |
|---|---|---|---|---|---|---|---|---|---|---|---|---|---|---|---|---|
| | | 4ng/mL | | 2 ng/mL | | 1 ng/mL | | 4ng/mL | | 2 ng/mL | | 1 ng/mL | | 4ng/mL | 1 ng/mL | 1 ng/mL |
| Cetuximab | | 2.40 | 2.39 | 1.99 | 1.92 | 1.41 | 1.33 | 2.15 | 2.15 | 1.70 | 1.74 | 1.15 | 1.09 | 1.11 | 1.14 | 1.22 |
| | | | | | | | | | | | | | | | | |
| 2-1 | HC-Y104D | 2.29 | 2.27 | 1.84 | 1.80 | 1.24 | 1.27 | 1.33 | 1.18 | 0.67 | 0.75 | 0.45 | 0.42 | 1.82 | 2.55 | 2.88 |
| 2-2 | HC-Y104D/Q111P | 2.27 | 2.23 | 1.71 | 1.70 | 1.14 | 1.21 | 0.81 | 0.76 | 0.43 | 0.39 | 0.16 | 0.20 | 2.87 | 4.18 | 6.55 |
| 2-3 | HC-S053G/Y104D | 1.36 | 1.51 | 0.74 | 0.86 | 0.46 | 0.47 | 0.12 | 0.14 | 0.08 | 0.08 | 0.07 | 0.07 | 11.11 | 10.17 | 6.58 |
| 2-4 | HC-S053G/Y104D/Q111P | 0.89 | 0.85 | 0.56 | 0.43 | 0.21 | 0.29 | 0.08 | 0.10 | 0.07 | 0.08 | 0.07 | 0.07 | 9.78 | 6.65 | 3.45 |
| | | | | | | | | | | | | | | | | |
| 2-1 | HC-Y104D | 2.27 | 2.26 | 1.62 | 1.74 | 1.09 | 1.09 | 0.71 | 0.81 | 0.45 | 0.45 | 0.24 | 0.25 | 2.99 | 3.73 | 4.42 |
| 2-2 | HC-Y104D/Q111P | 2.05 | 2.09 | 1.50 | 1.51 | 1.01 | 0.94 | 0.34 | 0.30 | 0.16 | 0.16 | 0.11 | 0.09 | 6.43 | 9.34 | 9.93 |
| 2-5 | HC-S025V/Y104D | 1.61 | 1.59 | 1.06 | 1.02 | 0.56 | 0.59 | 0.21 | 0.23 | 0.10 | 0.10 | 0.07 | 0.08 | 7.23 | 10.49 | 7.49 |
| 2-6 | HC-S025V/Y104D/Q111P | 1.51 | 1.54 | 0.92 | 0.94 | 0.54 | 0.54 | 0.16 | 0.13 | 0.08 | 0.09 | 0.07 | 0.07 | 10.32 | 10.70 | 7.65 |
| 2-7 | HC-S025V/S053G/Y104D | 1.37 | 1.37 | 0.38 | 0.48 | 0.14 | 0.17 | 0.07 | 0.07 | 0.07 | 0.07 | 0.06 | 0.07 | 18.76 | 6.27 | 2.32 |
| 2-8 | HC-S025V/S053G/Y104D/Q111P | 0.15 | 0.17 | 0.10 | 0.11 | 0.09 | 0.08 | 0.06 | 0.07 | 0.06 | 0.06 | 0.05 | 0.05 | 2.57 | 1.89 | 1.57 |
| 2-11 | HC-F027G/S053G/Y104D | 0.08 | 0.08 | 0.08 | 0.08 | 0.07 | 0.07 | 0.06 | 0.07 | 0.06 | 0.06 | 0.06 | 0.06 | - | - | - |
| 2-12 | HC- F027G/S053G/Y104D/Q111P | 0.08 | 0.09 | 0.08 | 0.08 | 0.07 | 0.07 | 0.06 | 0.06 | 0.06 | 0.06 | 0.06 | 0.06 | - | - | - |
| 2-13 | HC- T030F/Y104D | 1.73 | 1.70 | 1.02 | 1.10 | 0.62 | 0.63 | 0.15 | 0.15 | 0.09 | 0.09 | 0.07 | 0.07 | 11.30 | 11.82 | 8.45 |
| 2-14 | HC-T030F/Y104D/Q111P | 1.56 | 1.47 | 0.98 | 0.90 | 0.49 | 0.51 | 0.10 | 0.10 | 0.07 | 0.07 | 0.06 | 0.07 | 15.61 | 12.84 | 7.34 |
| 2-15 | HC-T030F/S053G/Y104D | 0.39 | 0.36 | 0.19 | 0.18 | 0.14 | 0.12 | 0.09 | 0.07 | 0.06 | 0.06 | 0.06 | 0.06 | 4.71 | 2.94 | 2.17 |

(continued)

| clone | mutation | OD, pH 6.0 | | | | | | OD, pH 7.4 | | | | | | pH 6.0/pH 7.4 OD ratio | | |
|---|---|---|---|---|---|---|---|---|---|---|---|---|---|---|---|---|
| | | 4ng/mL | | 2 ng/mL | | 1 ng/mL | | 4ng/mL | | 2 ng/mL | | 1 ng/mL | | 4ng/mL | 1 ng/mL | 1 ng/mL |
| 2-16 | HC- T030F/S053G/ Y104D/Q111P | 0.21 | 0.18 | 0.13 | 0.12 | 0.10 | 0.09 | 0.07 | 0.07 | 0.06 | 0.06 | 0.06 | 0.06 | 2.94 | 2.01 | 1.62 |
| 2-17 | HC- D072L/Y104D | 1.82 | 1.86 | 1.19 | 1.20 | 0.72 | 0.73 | 0.40 | 0.33 | 0.17 | 0.17 | 0.11 | 0.13 | 5.03 | 7.07 | 6.21 |
| 2-18 | HC- D072L/Y104D/Q111P | 1.66 | 1.65 | 1.31 | 1.29 | 0.78 | 0.83 | 0.33 | 0.28 | 0.15 | 0.15 | 0.11 | 0.11 | 5.43 | 8.69 | 7.16 |
| 2-19 | HC-S053G/ D072L/Y104D | 0.32 | 0.44 | 0.20 | 0.18 | 0.12 | 0.12 | 0.07 | 0.08 | 0.07 | 0.07 | 0.07 | 0.07 | 5.11 | 2.72 | 1.77 |
| 2-20 | HC-S053G/ D072L/Y104D/Q111P | 0.14 | 0.14 | 0.10 | 0.10 | 0.08 | 0.08 | 0.07 | 0.07 | 0.06 | 0.06 | 0.06 | 0.06 | 2.11 | 1.72 | 1.42 |
| | | | | | | | | | | | | | | | | |
| | | no | dilution | 1:2 | dilution | 1:4 | dilution | no | dilution | 1:2 | dilution | 1:4 | dilution | | | |
| 2-9 | HC- F027G/Y104D | 0.10 | 0.09 | 0.09 | 0.08 | 0.07 | 0.08 | 0.07 | 0.08 | 0.06 | 0.06 | 0.06 | 0.06 | - | - | - |
| 2-10 | HC- F027G/Y104D/Q111P | 0.10 | 0.11 | 0.09 | 0.09 | 0.08 | 0.08 | 0.08 | 0.08 | 0.07 | 0.07 | 0.06 | 0.06 | - | - | - |

**Example 15**

**Humanization and Screening of Y104D/Q111P and T030F/Y104D/Q111P Mutants**

Double stranded DNA fragments encoding the full-length light chain and heavy chain CDR sequences of HC-Y104D/Q111P (clone 2-2; also called DP) and HC-T030F/Y104D/Q111P (clone 2-14; also called FDP) were used to generate a library of humanized clones that were then screened for pH-dependent EGFR binding and protein expression levels.

**1. Screening of Humanized Library**

CHO-S cells were plated in 96-well plates and transfected with the humanized clones. Each plate also contained a positive control (HC-Y104D/Q111P or HC-T030F/Y104D/Q111P) and a negative control (vector only). The supernatants were collected 48 hours post transfection. The IgG concentration was determined as described in Example 1. The supernatants were adjusted to 2 ng/mL and were tested for pH-dependent binding of EGFR binding at pH 6.0 and pH 7.4 using the pH sensitive ELISA described in Example 1. The binding activities at pH 6.0 and pH 7.4 were compared to the binding activity of the positive controls (HC-Y104D/Q111P or HC-T030F/Y104D/Q111P) on the same plate.

Primary hits were selected, excluding clones with low expression levels, and subjected to a secondary construction and confirmation screening. For screening, transfected supernatant was adjusted to concentrations of 4 ng/mL, 2 ng/mL and 1 ng/mL and were tested for pH-dependent binding of EGFR binding at pH 6.0 and pH 7.4 using the pH sensitive ELISA described in Example 1. The results are set forth in Table 27. The sequences of the identified hits were determined. In the initial screen, some of the hits identified contained a mixture of two sequences, and hence are designated with a "/" (*e.g.* FDP-h9/FDP-h13). The individual sequences within the mixtures were isolated for subsequent confirmation screenings. All hits contain the parental mutations Y104D and Q111P and/or T30F. Sequence analysis showed that there are 11 unique heavy chains and 16 unique light chains, with each of the hits having a unique combination of humanized light and heavy chain. SEQ ID NO of the variable heavy and light chain of the full-length antibodies are set forth in the Table.

The screening of identified Hits was repeated using transfected supernatant adjusted to concentrations of 30 ng/mL, 10 ng/mL, 3.3 ng/mL, 1.1 ng/mL, which were tested for pH-dependent binding of EGFR at pH 6.0, pH 6.5 and pH 7.4 using the procedures substantially as described using the pH sensitive ELISA described in Example 1, except that the pH 6.5 condition was added. The results for selected mutants are set forth in Tables 28 and 29. The SEQ ID NO of the variable heavy and light chain of the full-length antibodies are set forth in Table 28.

In summary, the results show that most selected hits exhibited similar or better ratios of the binding activities at pH 6.0 versus binding activities at pH 7.4 compared to the parental positive controls (HC-Y104D/Q111P or HC-T030F/Y104D/Q111P). In some cases, binding activity was reduced at pH 6.0 compared to the parental positive control, although generally binding activity of selected hits at pH 6.0 was substantially the same or increased compared to the parental positive control. For some hits, binding activity at pH 7.4 also was reduced compared to the parental positive control.

**Table 27 : 1st Confirmation Screening of Selected Anti-EGFR Humanized Hits**

| clone | SEQ ID NO | | OD, pH 6.0 | | | OD, pH 7.4 | | | pH 6.0/pH 7.4 OD ratio | | | pH 6.0/pH 7.4 OD ratio Mutant / pH 6.0/pH7.4 OD ratio Parental | | |
|---|---|---|---|---|---|---|---|---|---|---|---|---|---|---|
| | HC | LC | 4 ng/mL | 2 ng/mL | 1 ng/mL | 4 ng/mL | 2 ng/mL | 1 ng/mL | 4 ng/mL | 1 ng/mL | 1 ng/mL | 4 ng/mL | 2 ng/mL | 1 ng/mL |
| **Parental HC-Y104D/Q111P (2-2; DP)** | 1062 | 10 | 1.69 | 1.14 | 0.60 | 0.27 | 0.16 | 0.05 | 6.54 | 7.32 | 11.73 | 1 | 1 | 1 |
| DP-h1 | 1134 | 1138 | 1.76 | 1.39 | 1.05 | 0.17 | 0.08 | 0.05 | 10.56 | 16.78 | 20.79 | 1.61 | 2.29 | 1.77 |
| DP-h2 | 1134 | 1139 | 0.94 | 0.54 | 0.29 | 0.01 | 0.00 | 0.00 | 68.55 | n/a | n/a | 10.48 | n/a | n/a |
| DP-h3 | 1135 | 1138 | 0.63 | 0.22 | 0.11 | 0.02 | 0.01 | 0.00 | 26.44 | 17.80 | n/a | 4.04 | 2.43 | n/a |
| DP-h4 | 1134 | 1140 | 1.56 | 1.07 | 0.79 | 0.08 | 0.04 | 0.03 | 18.97 | 24.47 | 31.08 | 2.90 | 3.34 | 2.65 |
| DP-h5 | 1134 | 1141 | 2.03 | 1.64 | 1.05 | 1.28 | 0.67 | 0.33 | 1.58 | 2.45 | 3.16 | 0.24 | 0.33 | 0.27 |
| DP-h6 | 1134 | 1142 | 1.92 | 1.31 | 0.85 | 0.20 | 0.09 | 0.04 | 9.37 | 15.28 | 19.86 | 1.43 | 2.09 | 1.69 |
| DP-h7 | 1135 | 1142 | 1.91 | 1.22 | 0.86 | 0.04 | 0.03 | 0.02 | 44.94 | 43.76 | 50.71 | 6.87 | 5.98 | 4.32 |
| DP-h8 | 1134 | 1143 | 1.94 | 1.45 | 0.96 | 0.17 | 0.20 | 0.06 | 11.30 | 7.21 | 17.35 | 1.73 | 0.99 | 1.48 |
| DP-h9 | 1136 | 1142 | 0.92 | 0.36 | 0.17 | 0.03 | 0.02 | 0.02 | 35.63 | 19.53 | 9.82 | 5.45 | 2.67 | 0.84 |
| DP-10 | 1137 | 1144 | 1.34 | 0.84 | 0.59 | 0.13 | 0.21 | 0.08 | 10.41 | 4.08 | 7.05 | 1.59 | 0.56 | 0.60 |
| DP-h12 | 1136 | 1144 | 1.59 | 0.93 | 0.53 | 0.20 | 0.10 | 0.08 | 7.76 | 9.56 | 6.22 | 1.19 | 1.31 | 0.53 |
| DP-h13 | 1137 | 1145 | 1.62 | 1.39 | 0.79 | 0.15 | 0.13 | 0.03 | 10.65 | 10.87 | 24.80 | 1.63 | 1.49 | 2.11 |
| DP-h14 | 1136 | 1145 | 1.19 | 0.79 | 0.41 | 0.18 | 0.13 | 0.04 | 6.64 | 6.01 | 11.31 | 1.02 | 0.82 | 0.96 |
| | | | | | | | | | | | | | | |
| **Parental HC-T030F/Y104D/Q111P (2-14; FDP)** | 1125 | 10 | 1.27 | 0.98 | 0.46 | 0.06 | 0.05 | 0.03 | 21.04 | 25.93 | 17.19 | 1 | 1 | 1 |
| FDP-h1 | 1146 | 1153 | 1.74 | 1.43 | 0.76 | 0.23 | 0.12 | 0.02 | 7.52 | 11.88 | 47.70 | 0.36 | 0.46 | 2.77 |
| FDP-h2 | 1147 | 1153 | 1.28 | 0.82 | 0.52 | 0.07 | 0.03 | 0.01 | 17.05 | 30.30 | 51.21 | 0.81 | 1.17 | 2.98 |
| FDP-h3 | 1148 | 1154 | 1.79 | 1.47 | 1.01 | 0.03 | 0.00 | 0.00 | 51.37 | n/a | n/a | 2.44 | n/a | n/a |
| FDP-h4 | 1149 | 1154 | 1.83 | 1.37 | 0.82 | 0.09 | 0.04 | 0.00 | 21.33 | 31.20 | n/a | 1.01 | 1.20 | n/a |

**Table 27 : 1st Confirmation Screening of Selected Anti-EGFR Humanized Hits**

| clone | SEQ ID NO | | OD, pH 6.0 | | | OD, pH 7.4 | | | pH 6.0/pH 7.4 OD ratio | | | pH 6.0/pH 7.4 OD ratio Mutant / pH 6.0/pH7.4 OD ratio Parental | | |
|---|---|---|---|---|---|---|---|---|---|---|---|---|---|---|
| | HC | LC | 4 ng/mL | 2 ng/mL | 1 ng/mL | 4 ng/mL | 2 ng/mL | 1 ng/mL | 4 ng/mL | 1 ng/mL | 1 ng/mL | 4 ng/mL | 2 ng/mL | 1 ng/mL |
| FDP-h5 | 1150 | 1155 | 1.77 | 1.15 | 0.65 | 0.15 | 0.16 | 0.04 | 11.69 | 7.32 | 16.95 | 0.56 | 0.28 | 0.99 |
| FDP-h6 | 1151 | 1156 | 1.18 | 1.01 | 0.66 | 0.15 | 0.07 | 0.00 | 7.73 | 14.86 | n/a | 0.37 | 0.57 | n/a |
| FDP-h7 | 1146 | 1156 | 1.86 | 1.72 | 1.09 | 0.31 | 0.25 | 0.11 | 5.99 | 6.75 | 9.82 | 0.28 | 0.26 | 0.57 |
| FDP-h8 | 1149 | 1156 | 1.63 | 1.30 | 0.58 | 0.11 | 0.05 | 0.04 | 15.38 | 24.86 | 14.58 | 0.73 | 0.96 | 0.85 |
| FDP-h9/FDP-h13* | 1150 | 1157/ 1186 | 1.19 | 0.75 | 0.63 | 0.03 | 0.00 | 0.00 | 36.19 | n/a | n/a | 0.53 | n/a | n/a |
| FDP-h10/FDP-h14* | 1152 | 1157/ 1186 | 1.65 | 1.02 | 0.73 | 0.06 | 0.02 | 0.00 | 27.00 | 52.86 | n/a | 1.28 | 2.04 | n/a |
| clone | SEQ ID NO | | OD, pH 6.0 | | | OD, pH 7.4 | | | pH 6.0/pH 7.4 OD ratio | | | pH 6.0/pH 7.4 OD ratio Mutant / pH 6.0/pH7.4 OD ratio Parental | | |
| | HC | LC | 4 ng/mL | 2 ng/mL | 1 ng/mL | 4 ng/mL | 2 ng/mL | 1 ng/mL | 4 ng/mL | 1 ng/mL | 1 ng/mL | 4 ng/mL | 2 ng/mL | 1 ng/mL |
| FDP-h11/FDP-h15* | 1148 | 1157/ 1186 | 1.85 | 1.36 | 0.83 | 0.06 | 0.03 | 0.01 | 30.61 | 42.13 | 59.49 | 1.45 | 1.63 | 3.46 |
| FDP-h12/FDP-h16* | 1149 | 1157/ 1186 | 1.79 | 1.25 | 0.83 | 0.06 | 0.03 | 0.02 | 28.79 | 46.07 | 45.12 | 1.37 | 1.78 | 2.62 |
| FDP-h17 | 1150 | 1158 | 1.61 | 1.22 | 0.77 | 0.16 | 0.09 | 0.02 | 9.83 | 14.32 | 31.46 | 0.47 | 0.55 | 1.83 |
| | | | | | | | | | | | | | | |
| Parental HC-T030F/Y104D/Q111P (2-14; FDP) | 1125 | 10 | 1.16 | 0.85 | 0.53 | 0.04 | 0.02 | 0.00 | 31.72 | 37.08 | n/a | 1 | 1 | 1 |
| FDP-h18 | 1152 | 1159 | 1.62 | 1.31 | 0.88 | 0.04 | 0.03 | 0.02 | 45.45 | 51.20 | 57.00 | 1.43 | 1.38 | n/a |
| FDP-h19 | 1146 | 1159 | 1.19 | 0.81 | 0.44 | 0.02 | 0.02 | 0.01 | 51.30 | 46.07 | 33.55 | 1.62 | 1.24 | n/a |

| clone | SEQ ID NO | | OD, pH 6.0 | | | OD, pH 7.4 | | | pH 6.0/pH 7.4 OD ratio | | | pH 6.0/pH 7.4 OD ratio Mutant / pH 6.0/pH7.4 OD ratio Parental | | |
|---|---|---|---|---|---|---|---|---|---|---|---|---|---|---|
| | HC | LC | 4 ng/mL | 2 ng/mL | 1 ng/mL | 4 ng/mL | 2 ng/mL | 1 ng/mL | 4 ng/mL | 1 ng/mL | 1 ng/mL | 4 ng/mL | 2 ng/mL | 1 ng/mL |
| FDP-h20/FDP-h21 * | 1146 | 1157/ 1186 | 1.43 | 1.08 | 0.73 | 0.01 | 0.00 | 0.00 | 151.47 | n/a | n/a | 4.78 | n/a | n/a |
| *contains a mixture of two antibodies | | | | | | | | | | | | | | |

| Table 28 : Further Confirmation Screening of Selected Anti-EGFR Humanized Hits: Optical Density (OD) Values | | | | | | | | | | | | | | |
|---|---|---|---|---|---|---|---|---|---|---|---|---|---|---|
| clone | SEQ ID NO | | OD, pH 6.0 | | | | OD, pH 6.5 | | | | OD, pH 7.4 | | | |
| | HC | LC | 30 ng/mL | 10 ng/mL | 3.3 ng/mL | 1.1 ng/mL | 30 ng/mL | 10 ng/mL | 3.3 ng/mL | 1.1 ng/mL | 30 ng/mL | 10 ng/mL | 3.3 ng/mL | 1.1 ng/mL |
| Parental HC-Y104D/Q111P (2-2; DP) | 1062 | 10 | 1.84 | 1.69 | 1.19 | 0.48 | 0.81 | 0.65 | 0.39 | 0.18 | 0.21 | 0.16 | 0.10 | 0.07 |
| DP-h1 | 1134 | 1138 | 1.82 | 1.42 | 1.19 | 0.74 | 0.96 | 0.67 | 0.35 | 0.18 | 0.35 | 0.22 | 0.11 | 0.06 |
| DP-h2 | 1134 | 1139 | 1.68 | 1.60 | 1.23 | 0.59 | 0.60 | 0.42 | 0.33 | 0.14 | 0.15 | 0.09 | 0.07 | 0.06 |
| DP-h3 | 1135 | 1138 | 1.84 | 1.56 | 1.15 | 0.67 | 0.75 | 0.57 | 0.32 | 0.21 | 0.19 | 0.14 | 0.09 | 0.07 |
| DP-h4 | 1134 | 1140 | 1.66 | 1.55 | 1.10 | 0.60 | 0.49 | 0.51 | 0.21 | 0.08 | 0.12 | 0.07 | 0.05 | 0.05 |
| | | | | | | | | | | | | | | |
| Parental HC-Y104D/Q111P (2-2; DP) | 1062 | 10 | 1.86 | 1.67 | 1.19 | 0.58 | 1.31 | 1.05 | 0.66 | 0.29 | 0.56 | 0.32 | 0.14 | 0.08 |
| DP-h5 | 1134 | 1141 | 1.91 | 1.73 | 1.35 | 0.71 | 1.21 | 0.92 | 0.53 | 0.26 | 0.42 | 0.25 | 0.11 | 0.08 |
| DP-h6 | 1134 | 1142 | 1.85 | 1.75 | 1.34 | 0.74 | 1.46 | 1.37 | 0.94 | 0.49 | 0.96 | 0.76 | 0.43 | 0.21 |
| DP-h7 | 1135 | 1142 | 1.93 | 1.60 | 1.06 | 0.49 | 1.06 | 0.80 | 0.43 | 0.19 | 0.29 | 0.19 | 0.11 | 0.07 |
| DP-h8 | 1134 | 1143 | 1.58 | 1.38 | 0.78 | 0.33 | 0.62 | 0.40 | 0.18 | 0.09 | 0.11 | 0.07 | 0.05 | 0.05 |
| clone | HC | LC | 30 ng/mL | 10 ng/mL | | 3.3 ng/mL 1.1 ng/mL | 30 ng/mL | 10 ng/mL | 3.3 ng/mL | 1.1 ng/mL | 30 ng/mL | 10 ng/mL | 3.3 ng/mL | 1.1 ng/mL |
| | | | | | | | | | | | | | | |
| Parental HC-Y104D/Q111P (2-2; DP) | 1062 | 10 | 1.85 | 1.78 | 1.18 | 0.58 | 1.25 | 1.10 | 0.63 | 0.28 | 0.54 | 0.32 | 0.15 | 0.07 |
| DP-h9 | 1136 | 1142 | 1.18 | 0.75 | 0.34 | 0.16 | 0.31 | 0.17 | 0.10 | 0.07 | 0.10 | 0.08 | 0.07 | 0.07 |
| DP-h10 | 1137 | 1144 | 1.52 | 1.19 | 0.63 | 0.26 | 0.96 | 0.72 | 0.36 | 0.14 | 0.49 | 0.29 | 0.12 | 0.07 |
| DP-h12 | 1136 | 1144 | 1.74 | 1.40 | 0.93 | 0.40 | 1.06 | 0.80 | 0.43 | 0.17 | 0.37 | 0.22 | 0.13 | 0.08 |

| clone | HC | LC | 30 ng/mL | 10 ng/mL | | 3.3 ng/mL 1.1 ng/mL | 30 ng/mL | 10 ng/mL | 3.3 ng/mL | 1.1 ng/mL | 30 ng/mL | 10 ng/mL | 3.3 ng/mL | 1.1 ng/mL |
|---|---|---|---|---|---|---|---|---|---|---|---|---|---|---|
| DP-h13 | 1137 | 1145 | 1.20 | 0.92 | 0.50 | 0.21 | 0.77 | 0.51 | 0.26 | 0.12 | 0.39 | 0.22 | 0.11 | 0.06 |
| | | | | | | | | | | | | | | |
| **Parental HC-Y104D/Q111P (2-2; DP)** | 1062 | 10 | 1.72 | 1.58 | 1.11 | 0.54 | 0.95 | 0.91 | 0.53 | 0.22 | 0.42 | 0.27 | 0.12 | 0.06 |
| DP-h14 | 1136 | 1145 | 1.44 | 1.20 | 0.74 | 0.36 | 0.77 | 0.53 | 0.28 | 0.13 | 0.24 | 0.13 | 0.09 | 0.08 |
| | | | | | | | | | | | | | | |
| **Parental HC-T030F/Y104D/Q111P (2-14; FDP)** | 1125 | 10 | 1.86 | 1.72 | 1.07 | 0.51 | 1.09 | 1.05 | 0.52 | 0.25 | 0.52 | 0.29 | 0.14 | 0.08 |
| FDP-h1 | 1146 | 1153 | 2.09 | 1.78 | 1.23 | 0.66 | 1.23 | 0.91 | 0.42 | 0.18 | 0.44 | 0.22 | 0.11 | 0.08 |
| FDP-h2 | 1147 | 1153 | 1.64 | 1.40 | 0.90 | 0.41 | 0.82 | 0.59 | 0.31 | 0.12 | 0.17 | 0.10 | 0.09 | 0.06 |
| FDP-h3 | 1148 | 1154 | 2.14 | 1.87 | 1.13 | 0.61 | 1.26 | 0.92 | 0.40 | 0.18 | 0.30 | 0.15 | 0.10 | 0.07 |
| FDP-h4 | 1149 | 1154 | 1.67 | 1.50 | 1.01 | 0.50 | 0.93 | 0.72 | 0.42 | 0.18 | 0.27 | 0.15 | 0.07 | 0.06 |
| | | | | | | | | | | | | | | |
| **Parental HC-T030F/Y104D/Q111P (2-14; FDP)** | 1125 | 10 | 1.84 | 1.81 | 1.29 | 0.57 | 1.28 | 1.19 | 0.76 | 0.36 | 0.60 | 0.32 | 0.19 | 0.11 |
| FDP-h5 | 1150 | 1155 | 2.16 | 2.00 | 1.42 | 0.65 | 1.40 | 1.03 | 0.76 | 0.39 | 0.70 | 0.56 | 0.29 | 0.16 |
| FDP-h6 | 1151 | 1156 | 2.02 | 1.63 | 1.03 | 0.51 | 1.46 | 0.96 | 0.53 | 0.22 | 0.56 | 0.30 | 0.16 | 0.08 |
| FDP-h7 | 1146 | 1156 | 2.25 | 2.05 | 1.67 | 1.00 | 1.47 | 1.60 | 1.23 | 0.68 | 1.11 | 0.86 | 0.52 | 0.27 |
| FDP-h8 | 1149 | 1156 | 1.83 | 1.42 | 0.97 | 0.47 | 1.34 | 1.00 | 0.63 | 0.30 | 0.60 | 0.31 | 0.18 | 0.09 |

(continued)

| clone | HC | LC | 30 ng/mL | 10 ng/mL | 3.3 ng/mL | 1.1 ng/mL | 30 ng/mL | 10 ng/mL | 3.3 ng/mL | 1.1 ng/mL | 30 ng/mL | 10 ng/mL | 3.3 ng/mL | 1.1 ng/mL |
|---|---|---|---|---|---|---|---|---|---|---|---|---|---|---|
| Parental HC-T030F/Y104D/Q111P (2-14; FDP) | 1125 | 10 | 1.83 | 1.61 | 1.01 | 0.47 | 1.33 | 1.14 | 0.71 | 0.33 | 0.46 | 0.35 | 0.18 | 0.10 |
| FDP-h9 | 1150 | 1157 | 2.16 | 1.68 | 1.02 | 0.43 | 1.03 | 0.61 | 0.23 | 0.11 | 0.21 | 0.14 | 0.09 | 0.08 |
| FDP-h10 | 1152 | 1157 | 2.00 | 1.79 | 1.20 | 0.58 | 1.08 | 0.83 | 0.44 | 0.22 | 0.46 | 0.17 | 0.10 | 0.08 |
| FDP-h11 | 1148 | 1157 | 2.15 | 1.78 | 1.09 | 0.55 | 1.46 | 0.88 | 0.40 | 0.19 | 0.34 | 0.22 | 0.13 | 0.09 |
| FDP-h12 | 1149 | 1157 | 1.60 | 1.47 | 1.01 | 0.53 | 0.71 | 0.58 | 0.29 | 0.13 | 0.34 | 0.12 | 0.08 | 0.07 |
| Parental HC-T030F/Y104D/Q111P (2-14; FDP) | 1125 | 10 | 1.90 | 1.60 | 1.03 | 0.49 | 1.31 | 0.93 | 0.53 | 0.26 | 0.40 | 0.24 | 0.13 | 0.09 |
| FDP-h13 | 1150 | 1186 | 2.21 | 1.81 | 1.22 | 0.62 | 1.40 | 0.79 | 0.42 | 0.19 | 0.31 | 0.12 | 0.08 | 0.07 |
| FDP-h14 | 1152 | 1186 | 1.99 | 1.86 | 1.36 | 0.67 | 1.20 | 0.93 | 0.55 | 0.23 | 0.35 | 0.23 | 0.11 | 0.07 |
| FDP-h15 | 1148 | 1186 | 2.24 | 1.94 | 1.46 | 0.75 | 1.63 | 1.16 | 0.71 | 0.33 | 0.57 | 0.21 | 0.13 | 0.08 |
| FDP-h16 | 1149 | 1186 | 1.78 | 1.65 | 1.31 | 0.75 | 1.01 | 0.80 | 0.47 | 0.21 | 0.23 | 0.16 | 0.08 | 0.06 |
| Parental HC-T030F/Y104D/Q111P (2-14; FDP) | 1125 | 10 | 1.69 | 1.31 | 0.92 | 0.57 | 1.17 | 0.76 | 0.57 | 0.32 | 0.26 | 0.31 | 0.16 | 0.11 |
| FDP-h17 | 1150 | 1158 | 2.25 | 1.84 | 1.19 | 0.53 | 1.16 | 0.75 | 0.47 | 0.28 | 0.51 | 0.25 | 0.18 | 0.14 |
| Parental HC-T030F/Y104D/Q111P (2-14; FDP) | 1125 | 10 | 1.130 | 1.060 | 0.812 | 0.410 | 0.589 | 0.469 | 0.320 | 0.179 | 0.173 | 0.127 | 0.091 | 0.072 |
| FDP-h18 | 1152 | 1159 | 1.299 | 1.157 | 0.875 | 0.469 | 0.669 | 0.510 | 0.304 | 0.153 | 0.129 | 0.109 | 0.083 | 0.069 |
| FDP-h19 | 1146 | 1159 | 1.017 | 0.868 | 0.598 | 0.323 | 0.397 | 0.268 | 0.136 | 0.084 | 0.083 | 0.065 | 0.053 | 0.050 |

(continued)

| clone | HC | LC | 30 ng/mL | 10 ng/mL | 3.3 ng/mL | 1.1 ng/mL | 30 ng/mL | 10 ng/mL | 3.3 ng/mL | 1.1 ng/mL | 30 ng/mL | 10 ng/mL | 3.3 ng/mL | 1.1 ng/mL |
|---|---|---|---|---|---|---|---|---|---|---|---|---|---|---|
| Parental HC-T030F/Y104D/Q111P (2-14; FDP) | 1125 | 10 | 1.368 | 1.233 | 0.850 | 0.430 | 0.789 | 0.720 | 0.467 | 0.236 | 0.300 | 0.197 | 0.124 | 0.087 |
| FDP-h20 | 1146 | 1157 | 1.571 | 1.490 | 1.174 | 0.606 | 0.591 | 0.467 | 0.311 | 0.160 | 0.196 | 0.143 | 0.085 | 0.067 |
| FDP-h21 | 1146 | 1186 | 1.495 | 1.288 | 0.922 | 0.531 | 0.763 | 0.588 | 0.343 | 0.186 | 0.219 | 0.131 | 0.085 | 0.058 |

**Table 29 : Further Confirmation Screening of Selected Anti-EGFR Humanized Hits: pH Ratios**

| clone | pH 6.0/pH 7.4 OD ratio | | | | pH 6.0/pH 7.4 OD ratio Mutant / pH 6.0/pH7.4 OD ratio Parental | | | | pH 6.5/pH 7.4 OD ratio | | | | pH 6.5/pH 7.4 OD ratio Mutant / pH 6.5/pH7.4 OD ratio Parental | | | |
|---|---|---|---|---|---|---|---|---|---|---|---|---|---|---|---|---|
| | 30 ng/mL | 10 ng/mL | 3.3 ng/mL | 1.1 ng/mL | 30 ng/mL | 10 ng/mL | 3.3 ng/mL | 1.1 ng/mL | 30 ng/mL | 10 ng/mL | 3.3 ng/mL | 1.1 ng/mL | 30 ng/mL | 10 ng/mL | 3.3 ng/mL | 1.1 ng/mL |
| Parental HC-Y104D/Q111P (2-2; DP) | 5.33 | 7.72 | 10.52 | 7.57 | 1 | 1 | 1 | 1 | 2.33 | 2.99 | 3.46 | 2.91 | 1 | 1 | 1 | 1 |
| DP-h1 | 8.64 | 8.76 | 11.98 | 10.69 | 1.62 | 1.13 | 1.14 | 1.41 | 4.56 | 4.13 | 3.47 | 2.54 | 1.96 | 1.38 | 1.00 | 0.87 |
| DP-h2 | 11.48 | 18.48 | 18.81 | 10.65 | 2.12 | 2.40 | 1.79 | 1.41 | 4.11 | 4.89 | 5.12 | 2.51 | 1.77 | 1.64 | 1.48 | 0.86 |
| DP-h3 | 9.86 | 11.50 | 12.23 | 9.37 | 1.85 | 1.49 | 1.16 | 1.24 | 3.99 | 4.19 | 3.41 | 2.87 | 1.71 | 1.40 | 0.98 | 0.99 |
| DP-h4 | 13.39 | 22.97 | 20.19 | 11.93 | 2.51 | 2.97 | 1.92 | 1.58 | 3.94 | 7.52 | 3.95 | 1.51 | 1.69 | 2.51 | 1.14 | 0.52 |
| | | | | | | | | | | | | | | | | |
| Parental HC-Y104D/Q111P (2-2; DP) | 3.30 | 5.18 | 8.21 | 7.21 | 1 | 1 | 1 | 1 | 2.33 | 3.26 | 4.55 | 3.61 | 1 | 1 | 1 | 1 |
| DP-h5 | 4.51 | 7.00 | 12.25 | 8.70 | 1.36 | 1.35 | 1.49 | 1.21 | 2.85 | 3.71 | 4.83 | 3.15 | 1.22 | 1.14 | 1.06 | 0.87 |
| DP-h6 | 1.93 | 2.31 | 3.12 | 3.54 | 0.58 | 0.44 | 0.38 | 0.49 | 1.52 | 1.80 | 2.19 | 2.34 | 0.65 | 0.55 | 0.48 | 0.65 |
| DP-h7 | 6.68 | 8.60 | 9.30 | 6.64 | 2.02 | 1.66 | 1.13 | 0.92 | 3.68 | 4.30 | 3.81 | 2.64 | 1.58 | 1.32 | 0.84 | 0.73 |
| DP-h8 | 14.88 | 21.13 | 15.12 | 6.81 | 4.51 | 4.08 | 1.84 | 0.95 | 5.84 | 6.04 | 3.49 | 1.86 | 2.51 | 1.85 | 0.77 | 0.52 |
| | | | | | | | | | | | | | | | | |
| Parental HC-Y104D/Q111P (2-2; DP) | 3.40 | 5.51 | 7.73 | 7.94 | 1 | 1 | 1 | 1 | 2.30 | 3.40 | 4.09 | 3.74 | 1 | 1 | 1 | 1 |
| DP-h9 | 12.30 | 9.88 | 4.68 | 2.39 | 3.61 | 1.79 | 0.61 | 0.30 | 3.27 | 2.30 | 1.37 | 1.07 | 1.42 | 0.68 | 0.33 | 0.29 |
| DP-h10 | 3.10 | 4.13 | 5.19 | 3.92 | 0.91 | 0.75 | 0.67 | 0.49 | 1.95 | 2.50 | 2.94 | 2.20 | 0.85 | 0.74 | 0.72 | 0.59 |
| DP-h12 | 4.74 | 6.43 | 7.47 | 4.96 | 1.39 | 1.17 | 0.97 | 0.63 | 2.87 | 3.70 | 3.47 | 2.10 | 1.25 | 1.09 | 0.85 | 0.56 |
| DP-h13 | 3.10 | 4.10 | 4.34 | 3.55 | 0.91 | 0.74 | 0.56 | 0.45 | 1.99 | 2.28 | 2.31 | 2.08 | 0.87 | 0.67 | 0.57 | 0.56 |

**Table 29 : Further Confirmation Screening of Selected Anti-EGFR Humanized Hits: pH Ratios**

| clone | pH 6.0/pH 7.4 OD ratio | | | | pH 6.0/pH 7.4 OD ratio Mutant / pH 6.0/pH7.4 OD ratio Parental | | | | pH 6.5/pH 7.4 OD ratio | | | | pH 6.5/pH 7.4 OD ratio Mutant / pH 6.5/pH7.4 OD ratio Parental | | | |
|---|---|---|---|---|---|---|---|---|---|---|---|---|---|---|---|---|
| | 30 ng/mL | 10 ng/mL | 3.3 ng/mL | 1.1 ng/mL | 30 ng/mL | 10 ng/mL | 3.3 ng/mL | 1.1 ng/mL | 30 ng/mL | 10 ng/mL | 3.3 ng/mL | 1.1 ng/mL | 30 ng/mL | 10 ng/mL | 3.3 ng/mL | 1.1 ng/mL |
| Parental HC-Y104D/Q111P (2-2; DP) | 4.06 | 5.94 | 9.40 | 8.58 | 1 | 1 | 1 | 1 | 2.24 | 3.44 | 4.46 | 3.53 | 1 | 1 | 1 | 1 |
| DP-h14 | 5.95 | 8.94 | 8.42 | 4.71 | 1.47 | 1.51 | 0.90 | 0.55 | 3.19 | 3.94 | 3.15 | 1.69 | 1.42 | 1.15 | 0.71 | 0.48 |
| Parental HC-T030F/YI04D/Q111P (2-14; FDP) | 3.55 | 5.98 | 7.86 | 6.24 | 1 | 1 | 1 | 1 | 2.08 | 3.65 | 3.87 | 3.00 | 1 | 1 | 1 | 1 |
| FDP-h1 | 4.79 | 8.07 | 11.59 | 8.49 | 1.35 | 1.35 | 1.48 | 1.36 | 2.82 | 4.11 | 3.93 | 2.31 | 1.35 | 1.13 | 1.02 | 0.77 |
| FDP-h2 | 9.45 | 14.16 | 9.55 | 6.61 | 2.66 | 2.37 | 1.21 | 1.06 | 4.73 | 5.99 | 3.25 | 1.98 | 2.27 | 1.64 | 0.84 | 0.66 |
| FDP-h3 | 7.18 | 12.11 | 11.33 | 8.26 | 2.02 | 2.02 | 1.44 | 1.32 | 4.25 | 5.97 | 4.04 | 2.50 | 2.04 | 1.63 | 1.04 | 0.83 |
| FDP-h4 | 6.11 | 10.30 | 13.62 | 8.38 | 1.72 | 1.72 | 1.73 | 1.34 | 3.40 | 4.96 | 5.64 | 2.95 | 1.63 | 1.36 | 1.46 | 0.98 |
| Parental HC-T030F/YI04D/Q111P (2-14; FDP) | 3.10 | 5.56 | 6.95 | 5.33 | 1 | 1 | 1 | 1 | 2.15 | 3.67 | 4.10 | 3.37 | 1 | 1 | 1 | 1 |
| FDP-h5 | 3.09 | 3.60 | 4.93 | 4.08 | 1.00 | 0.65 | 0.71 | 0.77 | 2.01 | 1.85 | 2.65 | 2.44 | 0.93 | 0.50 | 0.65 | 0.72 |
| FDP-h6 | 3.59 | 5.52 | 6.32 | 6.49 | 1.16 | 0.99 | 0.91 | 1.22 | 2.60 | 3.26 | 3.23 | 2.79 | 1.201 | 0.89 | 0.79 | 0.83 |
| FDP-h7 | 2.03 | 2.38 | 3.23 | 3.75 | 0.66 | 0.43 | 0.47 | 0.70 | 1.32 | 1.86 | 2.39 | 2.55 | 0.62 | 0.51 | 0.58 | 0.76 |
| FDP-h8 | 3.06 | 4.53 | 5.24 | 5.32 | 0.99 | 0.81 | 0.75 | 1.00 | 2.25 | 3.19 | 3.39 | 3.36 | 1.05 | 0.87 | 0.83 | 1.00 |

**Table 29 : Further Confirmation Screening of Selected Anti-EGFR Humanized Hits: pH Ratios**

| clone | pH 6.0/pH 7.4 OD ratio | | | | pH 6.0/pH 7.4 OD ratio Mutant / pH 6.0/pH7.4 OD ratio Parental | | | | pH 6.5/pH 7.4 OD ratio | | | | pH 6.5/pH 7.4 OD ratio Mutant / pH 6.5/pH7.4 OD ratio Parental | | | |
|---|---|---|---|---|---|---|---|---|---|---|---|---|---|---|---|---|
| | 30 ng/mL | 10 ng/mL | 3.3 ng/mL | 1.1 ng/mL | 30 ng/mL | 10 ng/mL | 3.3 ng/mL | 1.1 ng/mL | 30 ng/mL | 10 ng/mL | 3.3 ng/mL | 1.1 ng/mL | 30 ng/mL | 10 ng/mL | 3.3 ng/mL | 1.1 ng/mL |
| Parental HC-T030F/YI04D/Q111P (2-14; FDP) | 3.97 | 4.54 | 5.67 | 4.95 | 1 | 1 | 1 | 1 | 2.90 | 3.23 | 3.97 | 3.43 | 1 | 1 | 1 | 1 |
| FDP-h9 | 10.06 | 12.05 | 11.30 | 5.49 | 2.53 | 2.66 | 1.99 | 1.11 | 4.82 | 4.34 | 2.50 | 1.41 | 1.66 | 1.35 | 0.63 | 0.41 |
| FDP-h10 | 4.38 | 10.27 | 11.72 | 7.50 | 1.10 | 2.26 | 2.06 | 1.51 | 2.37 | 4.76 | 4.35 | 2.86 | 0.82 | 1.47 | 1.10 | 0.83 |
| FDP-h 11 | 6.28 | 8.18 | 8.20 | 6.26 | 1.58 | 1.80 | 1.44 | 1.26 | 4.26 | 4.06 | 3.02 | 2.17 | 1.47 | 1.26 | 0.76 | 0.63 |
| FDP-h12 | 4.71 | 12.15 | 12.67 | 7.71 | 1.19 | 2.68 | 2.23 | 1.56 | 2.09 | 4.77 | 3.59 | 1.87 | 0.72 | 1.48 | 0.91 | 0.55 |
| | | | | | | | | | | | | | | | | |
| Parental HC-T030F/YI04D/Q111P (2-14; FDP) | 4.77 | 6.58 | 7.92 | 5.71 | 1 | 1 | 1 | 1 | 3.30 | 3.81 | 4.09 | 3.09 | 1 | 1 | 1 | 1 |
| FDP-h13 | 7.12 | 15.35 | 14.91 | 8.28 | 1.49 | 2.33 | 1.88 | 1.45 | 4.52 | 6.74 | 5.13 | 2.53 | 1.37 | 1.77 | 1.25 | 0.82 |
| FDP-h14 | 5.62 | 8.14 | 12.66 | 9.25 | 1.18 | 1.24 | 1.60 | 1.62 | 3.38 | 4.08 | 5.07 | 3.16 | 1.02 | 1.07 | 1.24 | 1.02 |
| FDP-h15 | 3.93 | 9.23 | 11.45 | 9.86 | 0.82 | 1.40 | 1.45 | 1.73 | 2.85 | 5.51 | 5.57 | 4.39 | 0.86 | 1.44 | 1.36 | 1.42 |
| FDP-h16 | 7.63 | 10.18 | 15.52 | 11.90 | 1.60 | 1.55 | 1.96 | 2.08 | 4.32 | 4.91 | 5.63 | 3.35 | 1.31 | 1.29 | 1.38 | 1.09 |
| Parental HC-T030F/YI04D/Q111P (2-14; FDP) | 6.44 | 4.29 | 5.88 | 5.00 | 1 | 1 | 1 | 1 | 4.49 | 2.47 | 3.69 | 2.83 | 1 | 1 | 1 | 1 |
| FDP-h17 | 4.43 | 7.23 | 6.46 | 3.91 | 0.69 | 1.69 | 1.10 | 0.78 | 2.28 | 2.96 | 2.52 | 2.07 | 0.51 | 1.20 | 0.69 | 0.73 |
| | | | | | | | | | | | | | | | | |
| Parental HC-T030F/YI04D/Q111P (2-14; FDP) | 6.55 | 8.37 | 8.97 | 5.73 | 1 | 1 | 1 | 1 | 3.41 | 3.70 | 3.53 | 2.50 | 1 | 1 | 1 | 1 |

(continued)

| | pH 6.0/pH 7.4 OD ratio | | | | pH 6.0/pH 7.4 OD ratio Mutant / pH 6.0/pH7.4 OD ratio Parental | | | | pH 6.5/pH 7.4 OD ratio | | | | pH 6.5/pH 7.4 OD ratio Mutant / pH 6.5/pH7.4 OD ratio Parental | | | |
|---|---|---|---|---|---|---|---|---|---|---|---|---|---|---|---|---|
| clone | 30 ng/mL | 10 ng/mL | 3.3 ng/mL | 1.1 ng/mL | 30 ng/mL | 10 ng/mL | 3.3 ng/mL | 1.1 ng/mL | 30 ng/mL | 10 ng/mL | 3.3 ng/mL | 1.1 ng/mL | 30 ng/mL | 10 ng/mL | 3.3 ng/mL | 1.1 ng/mL |
| FDP-h18 | 10.11 | 10.64 | 10.54 | 6.79 | 1.54 | 1.27 | 1.17 | 1.18 | 5.21 | 4.69 | 3.67 | 2.22 | 1.53 | 1.27 | 1.04 | 0.89 |
| FDP-h19 | 12.24 | 13.41 | 11.35 | 6.40 | 1.87 | 1.60 | 1.26 | 1.12 | 4.77 | 4.15 | 2.58 | 1.66 | 1.40 | 1.12 | 0.73 | 0.66 |
| | | | | | | | | | | | | | | | | |
| Parental HC-T030F/YI04D/Q111P (2-14; FDP) | 4.57 | 6.25 | 6.86 | 4.94 | 1 | 1 | 1 | 1 | 2.63 | 3.65 | 3.77 | 2.71 | 1 | 1 | 1 | 1 |
| FDP-h20 | 8.03 | 10.41 | 13.84 | 9.11 | 1.76 | 1.66 | 2.02 | 1.84 | 3.02 | 3.26 | 3.67 | 2.40 | 1.15 | 0.89 | 0.97 | 0.89 |
| FDP-h21 | 6.84 | 9.87 | 10.89 | 9.23 | 1.50 | 1.58 | 1.59 | 1.87 | 3.49 | 4.51 | 4.05 | 3.24 | 1.32 | 1.23 | 1.07 | 1.19 |

Table 29 : Further Confirmation Screening of Selected Anti-EGFR Humanized Hits: pH Ratios

**2. Expression of Selected Humanized Antibodies in CHO-S cells**

The expression of the humanized antibody hits above also were screened for levels of expression. CHO-S cells were plated in 96-well plates and transfected with the selected humanized clones set forth in Tables 28 and 29 above using the methods described in Example 1. The IgG concentration was determined as described in Example 1. The results are set forth in Table 30. The results show that the yields of the humanized clones are substantially increased compared to the parental clones.

| Table 30: Expression of Selected Hits | | | |
|---|---|---|---|
| Clone | Quantitation 1 (ng/mL) | Quantitation 2 (ng/mL) | Quantitation 3 (ng/mL) |
| cetuximab | 257.29 | 251.67 | 254.48 |
| Y104D/Q111P; FP | 253.43 | 228.45 | 240.94 |
| T30F/Y104D/Q111P; DFP | 82.71 | 79.91 | 81.31 |
| DP-h1 | 2631.42 | 2482.36 | 2556.89 |
| DP-h2 | 2335.73 | 2251.82 | 2293.77 |
| DP-h3 | 2069.61 | 1997.00 | 2033.30 |
| DP-h4 | 2496.69 | 2552.73 | 2524.71 |
| DP-h5 | 1924.45 | 1889.72 | 1907.09 |
| DP-h6 | 1721.98 | 1573.85 | 1647.92 |
| DP-h7 | 931.96 | 791.67 | 861.82 |
| DP-h8 | 1497.07 | 1198.49 | 1347.78 |
| DP-h9 | 1672.25 | 1763.52 | 1717.88 |
| DP-h10 | 2325.02 | 2412.02 | 2368.52 |
| DP-h12 | 2304.56 | 2288.86 | 2296.71 |
| DP-h13 | 2796.34 | 2702.32 | 2749.33 |
| DP-h14 | 2443.26 | 2182.60 | 2312.93 |
| FDP-h1 | 3621.47 | 3431.47 | 3526.47 |
| FDP-h2 | 2914.16 | 2778.90 | 2846.53 |
| FDP-h3 | 1163.38 | 1131.05 | 1147.21 |
| FDP-h4 | 1055.94 | 1048.72 | 1052.33 |
| FDP-h5 | 2671.64 | 2523.70 | 2597.67 |
| FDP-h6 | 2650.07 | 2482.81 | 2566.44 |
| FDP-h7 | 1983.05 | 1825.43 | 1904.24 |
| FDP-h8 | 2373.23 | 2181.37 | 2277.30 |
| FDP-h9 | 3235.35 | 3211.05 | 3223.20 |
| FDP-h10 | 2656.16 | 2618.89 | 2637.52 |
| FDP-h11 | 2109.93 | 2095.10 | 2102.51 |
| FDP-h12 | 1792.14 | 1715.23 | 1753.69 |
| FDP-h13 | 2745.20 | 2686.71 | 2715.96 |
| FDP-h14 | 2253.08 | 2133.30 | 2193.19 |
| FDP-h15 | 1859.46 | 1676.10 | 1767.78 |
| FDP-h16 | 1825.07 | 1572.16 | 1698.61 |

(continued)

| Table 30: Expression of Selected Hits | | | |
|---|---|---|---|
| Clone | Quantitation 1 (ng/mL) | Quantitation 2 (ng/mL) | Quantitation 3 (ng/mL) |
| FDP-h17 | 1979.96 | 2033.30 | 2006.63 |

Since modifications will be apparent to those of skill in this art, it is intended that this invention be limited only by the scope of the appended claims.

**Claims**

1. A modified anti-EGFR antibody, or antigen-binding fragment thereof, comprising replacement with aspartic acid at a position corresponding to position 104 (Y104D) in the variable heavy chain of the unmodified antibody, wherein:

   a) the modified anti-EGFR antibody or antigen-binding fragment thereof comprises a variable heavy (VH) chain and a variable light (VL) chain, whereby the VH alone or both the VH and VL is modified;
   corresponding amino acid positions are identified by alignment of the VH chain of the antibody with the VH chain set forth in SEQ ID NO:3;
   the modified anti-EGFR antibody, or antigen-binding fragment thereof, specifically binds to epidermal growth factor receptor (EGFR) or a soluble fragment thereof, and contains up to 1, 2, 3, 4 or 5 amino acid replacements, including Y104D, in the unmodified antibody or antigen-binding fragment;
   if the modified anti-EGFR antibody or antigen-binding fragment thereof, contains amino acid replacement(s) in addition to Y104D, the additional replacement(s) are selected from among:

   one or more amino acid replacement(s) in the variable heavy chain (VH) corresponding to Q111P, S53G, S25V, T30F, D72L and S25C, with reference to amino acid positions set forth in SEQ ID NO:3, wherein corresponding amino acid positions are identified by alignment of the VH chain of the antibody with the VH chain set forth in SEQ ID NO:3; and wherein
   the unmodified anti-EGFR antibody is selected from among cetuximab, a variant thereof or an antigen-binding fragment thereof, comprising

   i) a variable heavy chain set forth in SEQ ID NO:3 and a variable light chain set forth in SEQ ID NO:4 or 10;
   ii) a heavy chain set forth in SEQ ID NO:1 and a light chain set forth in SEQ ID NO:2;
   iii) an antibody comprising a heavy chain set forth in SEQ ID NO:8 and a light chain set forth in SEQ ID NO:9; and
   iv) a Fab fragment comprising a heavy chain set forth in SEQ ID NO:5 and a light chain set forth in SEQ ID NO:2; or

   b) the modified anti-EGFR antibody or antigen-binding fragment thereof comprises the amino acid replacements of the modified anti-EGFR antibody or antigen-binding fragment thereof of (a) and is a humanized variant of the modified anti-EGFR antibody or antigen-binding fragment thereof of (a); and wherein
   the modified anti-EGFR antibody, or antigen-binding fragment thereof, exhibits a ratio of binding activity to human epidermal growth factor receptor (EGFR) or a soluble fragment thereof under conditions in a tumor environment compared to under conditions in a non-tumor environment of at least 2.0, whereby the anti-EGFR antibody or fragment thereof is conditionally active under conditions in the tumor microenvironment, wherein;

   conditions in a tumor environment comprise one or both of pH between 6.0 to 6.5 or lactate concentration between 10 mM to 20 mM, and protein concentration of 10 mg/mL to 50 mg/mL; and
   conditions in a non-tumor environment comprise one or both of pH between 7.0 to 7.8 or lactate concentration between 0.5 mM to 5 mM, and protein concentration of 10 mg/mL to 50 mg/mL.

2. The modified anti-EGFR antibody, or antigen-binding fragment thereof, of claim 1, wherein the ratio of binding activity is at least 3.0.

3. The modified anti-EGFR antibody, or antigen-binding fragment thereof, of claim 1, wherein the anti-EGFR antibody,

or antigen-binding fragment thereof, exhibits the ratio of activity under conditions that exist in a tumor microenvironment that comprise a pH of between 6.0 to 6.5 compared to under conditions that exist in a non-tumor microenvironment that comprise a pH of 7.4.

4. The modified anti-EGFR antibody, or antigen-binding fragment thereof, of any of claims 1-3, wherein the anti-EGFR antibody, or antigen-binding fragment thereof, exhibits the ratio of activity under conditions of a tumor microenvironment that comprise pH of 6.0 to 6.5 and lactate concentration of 10 mM to 20 mM compared to under conditions of a non-tumor microenvironment comprise pH of 7.0 to 7.4, inclusive, and lactate concentration of 0.5 mM to 5 mM.

5. The modified anti-EGFR antibody, or antigen-binding fragment thereof, of any of claims 1-4, wherein the protein concentration under conditions in a tumor microenvironment and under conditions in a non-tumor microenvironment is substantially the same or is the same.

6. The modified anti-EGFR antibody, or antigen-binding fragment thereof, of any of claims 1-5, selected from among:

i) an antibody, comprising:

a) a variable heavy (VH) chain comprising the sequence of amino acids set forth in SEQ ID NO:495, 1062, 1112, 1114-1117, 1124-1126 or 1128-1130; and
b) a variable light (VL) chain comprising the sequence of amino acids set forth in SEQ ID NO:4 or 10,

ii) antigen-binding fragments of the antibody of i); and
iii) humanized variants of i) or ii).

7. The modified anti-EGFR antibody, or antigen-binding fragment thereof, of any of claims 1-6, selected from among an antibody comprising:

a) the variable heavy chain set forth in SEQ ID NO:495 and the variable light chain set forth in SEQ ID NO:4 or 10;
b) the variable heavy chain set forth in SEQ ID NO:1062 and the variable light chain set forth in SEQ ID NO:4 or 10;
c) the variable heavy chain set forth in SEQ ID NO:1112 and the variable light chain set forth in SEQ ID NO:4 or 10;
d) the variable heavy chain set forth in SEQ ID NO:1114 and the variable light chain set forth in SEQ ID NO:4 or 10;
e) the variable heavy chain set forth in SEQ ID NO:1115 and the variable light chain set forth in SEQ ID NO:4 or 10;
f) the variable heavy chain set forth in SEQ ID NO:1116 and the variable light chain set forth in SEQ ID NO:4 or 10;
g) the variable heavy chain set forth in SEQ ID NO:1117 and the variable light chain set forth in SEQ ID NO:4 or 10;
h) the variable heavy chain set forth in SEQ ID NO:1124 and the variable light chain set forth in SEQ ID NO:4 or;
i) the variable heavy chain set forth in SEQ ID NO:1125 and the variable light chain set forth in SEQ ID NO:4 or 10;
j) the variable heavy chain set forth in SEQ ID NO:1126 and the variable light chain set forth in SEQ ID NO:4 or 10;
k) the variable heavy chain set forth in SEQ ID NO:1128 and the variable light chain set forth in SEQ ID NO:4 or 10;
l) the variable heavy chain set forth in SEQ ID NO:1129 and the variable light chain set forth in SEQ ID NO:4 or 10;
m) the variable heavy chain set forth in SEQ ID NO:1130 and the variable light chain set forth in SEQ ID NO:4 or 10; and
n) the variable heavy chain set forth in SEQ ID NO:1118 and the variable light chain set forth in SEQ ID NO:4 or 10.

8. The modified anti-EGFR antibody, or antigen-binding fragment thereof, of any of claims 1-7, wherein the unmodified cetuximab antibody, antigen-binding fragment thereof or variant thereof comprises:

a heavy chain having a sequence of amino acids set forth in SEQ ID NO:1 and a light chain having a sequence of amino acids set forth SEQ ID NO:2; or
a heavy chain having a having a sequence of amino acids set forth in SEQ ID NO:8 and a light chain having a sequence of amino acids set forth SEQ ID NO:9.

9. The modified anti-EGFR antibody, or antigen-binding fragment thereof, of any of claims 1-8, wherein:

the unmodified anti-EGFR antibody is an antigen-binding fragment; and
the antigen-binding fragment is selected from among a Fab, Fab', F(ab')2, single-chain Fv (scFv), Fv, dsFv, diabody, Fd and Fd' fragments.

10. The modified anti-EGFR antibody, or antigen binding fragment thereof, of any of claims 1-9 that is a full-length IgG antibody or is an antigen-binding fragment selected from among a Fab, Fab', F(ab')2, single-chain Fv (scFv), Fv, dsFv, diabody, Fd and Fd' fragments.

11. A conjugate, comprising a modified anti-EGFR antibody, or antigen-binding fragment thereof, of any of claims 1 -10 linked directly or indirectly to a targeted agent.

12. The conjugate of claim 11, wherein the targeted agent is a therapeutic moiety.

13. The conjugate of claim 12, wherein the therapeutic moiety is a cytotoxic moiety, a radioisotope, a chemotherapeutic agent, a lytic peptide or a cytokine.

14. The conjugate of claim 13, wherein the therapeutic moiety is selected from among taxol; cytochalasin B; gramicidin D; ethidium bromide; emetine; mitomycin; etoposide; tenoposide; vincristine; vinblastine; colchicin; doxorubicin; daunorubicin; dihydroxy anthracin dione;a maytansinoid; an auristatin; dolastatin 10 or 15; irinotecan; mitoxantrone; mithramycin; actinomycin D; 1-dehydrotestosterone; a glucocorticoid; procaine; tetracaine; lidocaine; propranolol; puromycin; calicheamicin or an analog or derivative thereof; an antimetabolite; an alkylating agent; cisplatin or carboplatin, duocarmycin SA, rachelmycin (CC-1065); an antibiotic; pyrrolo[2,l-c][l, 4]-benzodiazepines (PDB); a toxin; ribonuclease (RNase); DNase I, Staphylococcal enterotoxin A; and pokeweed antiviral protein.

15. The conjugate of claim 13 or claim 14, wherein the therapeutic moiety is a maytansinoid selected from among ansamitocin or mertansine (DM1); or is
an auristatin that is monomethyl auristatin E (MMAE) or F (MMAF); or is
an antimetabolite selected from among methotrexate, 6 mercaptopurine, 6 thioguanine, cytarabine, fludarabin, 5 fluorouracil, decarbazine, hydroxyurea, asparaginase, gemcitabine, and cladribine; or is
an alkylating agent selected from among mechlorethamine, thioepa, chlorambucil, melphalan, carmustine (BSNU), lomustine (CCNU), cyclophosphamide, busulfan, dibromomannitol, streptozotocin, dacarbazine (DTIC), procarbazine and mitomycin C; or is
cisplatin or carboplatin; or is
an antibiotic selected from among dactinomycin, bleomycin, daunorubicin, doxorubicin, idarubicin, mithramycin, mitomycin, mitoxantrone, plicamycin and anthramycin (AMC); or is
a toxin selected from among a diphtheria toxin and active fragments thereof and hybrid molecules, a ricin toxin, cholera toxin, a Shiga-like toxin, LT toxin, C3 toxin, Shiga toxin, pertussis toxin, tetanus toxin, soybean Bowman-Birk protease inhibitor, Pseudomonas exotoxin, alorin, saporin, modeccin, gelanin, abrin A chain, modeccin A chain, alpha-sarcin, Aleurites fordii proteins, dianthin proteins, Phytolacca americana proteins, momordica charantia inhibitor, curcin, crotin,, gelonin, mitogellin, restrictocin, phenomycin, and enomycin toxins.

16. A nucleic acid molecule comprising a sequence of nucleotides encoding the modified anti-EGFR antibody, or antigen-binding fragment thereof, of any of claims 1-15.

17. A vector, comprising the nucleic acid molecule of claim 16.

18. A cell, comprising a vector of claim 17.

19. A method of making a modified anti-EGFR antibody, or antigen-binding fragment thereof, comprising expressing the heavy chain or antibody encoded from a vector of claim 17 in a suitable host cell and, optionally, recovering the antibody.

20. A pharmaceutical composition comprising:

a modified anti-EGFR antibody or antigen-binding fragment of any of claims 1-10 or the conjugate of any of claims 11-15; and
a pharmaceutically acceptable carrier or excipient.

21. A pharmaceutical composition of claim 20, the modified anti-EGFR antibody or antigen-binding fragment of any of claims 1 -10, or the conjugate of any of claims 11-15 for use for treating a tumor, cancer or metastasis.

## A. Cetuximab Heavy Chain (SEQ ID NO:1)

```
        10         20         30         40         50         60
QVQLKQSGPG LVQPSQSLSI TCTVSGFSLT NYGVHWVRQS PGKGLEWLGV IWSGGNTDYN
        70         80         90        100        110        120
TPFTSRLSIN KDNSKSQVFF KMNSLQSNDT AIYYCARALT YYDYEFAYWG QGTLVTVSAA
       130        140        150        160        170        180
STKGPSVFPL APSSKSTSGG TAALGCLVKD YFPEPVTVSW NSGALTSGVH TFPAVLQSSG
       190        200        210        220        230        240
LYSLSSVVTV PSSSLGTQTY ICNVNHKPSN TKVDKRVEPK SPKSCDKTHT CPPCPAPELL
       250        260        270        280        290        300
GGPSVFLFPP KPKDTLMISR TPEVTCVVVD VSHEDPEVKF NWYVDGVEVH NAKTKPREEQ
       310        320        330        340        350        360
YNSTYRVVSV LTVLHQDWLN GKEYKCKVSN KALPAPIEKT ISKAKGQPRE PQVYTLPPSR
       370        380        390        400        410        420
DELTKNQVSL TCLVKGFYPS DIAVEWESNG QPENNYKTTP PVLDSDGSFF LYSKLTVDKS
       430        440        450
RWQQGNVFSC SVMHEALHNH YTQKSLSLSP GK
```

## B. Cetuximab Light Chain (SEQ ID NO:2)

```
        10         20         30         40         50         60
DILLTQSPVI LSVSPGERVS FSCRASQSIG TNIHWYQQRT NGSPRLLIKY ASESISGIPS
        70         80         90        100        110        120
RFSGSGSGTD FTLSINSVES EDIADYYCQQ NNNWPTTFGA GTKLELKRTV AAPSVFIFPP
       130        140        150        160        170        180
SDEQLKSGTA SVVCLLNNFY PREAKVQWKV DNALQSGNSQ ESVTEQDSKD STYSLSSTLT
       190        200        210
LSKADYEKHK VYACEVTHQG LSSPVTKSFN RGA
```

**FIGURE 1**

Heavy Chain

SEQIDNO:3    QVQLKQSGPGLVQPSQSLSITCTVSGFSLTNYGVHWVRQSPGKGLEWLGVIWSGGNTDYN 60
SEQIDNO:28   EVQLVESGGGLVQPGGSLRLSCAASGFSLTNYGVHWVRQAPGKGLEWLGVIWSGGNTDYN 60
             :*** :** ***** . ** ::*:.*******************:***************

SEQIDNO:3    TPFTSRLSINKDNSKSQVFFKMNSLQSNDTAIYYCARALTYYDYEFAYWGQGTLVTVSA 119
SEQIDNO:28   TPFTSRLTINKDNSKNTVYLQMNSLRAEDTAVYYCARALTYYDYEFAYWGQGTLVTVSS 119
             *******:*******. *::*****::***:*************************:


Light Chain

SEQIDNO:4    DILLTQSPVILSVSPGERVSFSCRASQSIGTNIHWYQQRTNGSPRLLIKYASESISGIPS 60
SEQIDNO:29   DILLTQSPGTLSLSPGERATLSCRASQSIGTNIHWYQQKPGQAPRLLIKYASESISGIPD 60
             ********  **:*****.::*****************:.. :**************** .

SEQIDNO:4    RFSGSGSGTDFTLSINSVESEDIADYYCQQNNNWPTTFGAGTKLELK 107
SEQIDNO:29   RFSGSGSGTDFTLTISRLEPEDFAVYYCQQNNNWPTTFGQGTKLEIK 107
             *************:*. :*.**:* ************** *****:*


FIGURE 2A

Heavy Chain

```
SEQIDNO:3    QVQLKQSGPGLVQPSQSLSITCTVSGFSLTNYGVHWVRQSPGKGLEWLGVIWSGGNTDYN  60
SEQIDNO:3    QVQLKQSGPGLVQPSQSLSITCTVSGFSLTNYGVHWVRQSPGKGLEWLGVIWSGGNTDYN  60
             ************************************************************

SEQIDNO:3    TPFTSRLSINKDNSKSQVFFKMNSLQSNDTAIYYCARALTYYDYEFAYWGQGTLVTVSA  119
SEQIDNO:3    TPFTSRLSINKDNSKSQVFFKMNSLQSNDTAIYYCARALTYYDYEFAYWGQGTLVTVSA  119
             ***********************************************************
```

Light Chain

```
SEQIDNO:4    DILLTQSPVILSVSPGERVSFSCRASQSIGTNIHWYQQRTNGSPRLLIKYASESISGIPS  60
SEQIDNO:10   DILLTQSPVILSVSPGERVSFSCRASQSIGTNIHWYQQRTNGSPRLLIKYASESISGIPS  60
             ************************************************************

SEQIDNO:4    RFSGSGSGTDFTLSINSVESEDIADYYCQQNNNWPTTFGAGTKLELK  107
SEQIDNO:10   RFSGSGSGTDFTLSINSVESEDIADYYCQQNNNWPTTFGQGTKLELK  107
             **************************************** *******
```

**FIGURE 2B**

**Figure 3**

**A)**

**Adult Keratinocytes**

**B)**

**Neonatal Keratinocytes**

# Figure 4

Figure 5

Figure 6

Europäisches
Patentamt

European
Patent Office

Office européen
des brevets

# EUROPEAN SEARCH REPORT

Application Number

EP 17 19 4775

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 03/105757 A2 (GENENCOR INT [US]; SCHELLENBERGER VOLKER [US]; TRESSLER ROBERT J [US];) 24 December 2003 (2003-12-24) * figure 4 * * page 17, lines 1-3 * * page 17, lines 31-32 * * page 28, line 31 - page 29, line 8 * * page 33, lines 26-32 * * page 34, lines 15-17 * * page 57, line 6 - page 63, line 11; example 1 * * page 75, line 5 - page 80; example 4 * | 1-21 | INV. C07K16/28 C07K16/30 G01N33/53 G01N33/574 |
| A | US 2005/260711 A1 (DATTA DEEPSHIKHA [US] ET AL) 24 November 2005 (2005-11-24) * abstract * * paragraph [0046] * * paragraph [0365] * * paragraph [0027] * * paragraph [0373] * * examples I-IV * * paragraph [0389] * | 1-21 | |
| A | WO 2005/058236 A2 (GENENCOR INT [US]; FOX JUDITH A [US]; HARDING FIONA A [US]; SCHELLENBE) 30 June 2005 (2005-06-30) * page 43, line 6 - page 48; example 2 * | 1-21 | TECHNICAL FIELDS SEARCHED (IPC) C07K G01N |
| A,P | WO 2012/033953 A1 (HALOZYME INC [US]; KODANDAPANI LALITHA [US]; BOOKBINDER LOUIS HOWARD []) 15 March 2012 (2012-03-15) * the whole document * * page 125; example 9 * * page 124; example 8 * | 1-21 | |

-/--

The present search report has been drawn up for all claims

| Place of search | Date of completion of the search | Examiner |
|---|---|---|
| Munich | 30 November 2017 | Irion, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt
European
Patent Office
Office européen
des brevets

**EUROPEAN SEARCH REPORT**

Application Number

EP 17 19 4775

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SV KOZIN ET AL: "Cytotoxicity of weak electrolytes after the adaptation of cells to low pH: role of the transmembrane pH gradient", BRITISH JOURNAL OF CANCER, vol. 77, no. 10, 1 May 1998 (1998-05-01), pages 1580-1585, XP055019075, ISSN: 0007-0920, DOI: 10.1038/bjc.1998.260 * the whole document * | 1-21 | |
| A | O. TREDAN ET AL: "Drug Resistance and the Solid Tumor Microenvironment", JNCI JOURNAL OF THE NATIONAL CANCER INSTITUTE, vol. 99, no. 19, 25 September 2007 (2007-09-25), pages 1441-1454, XP055019083, ISSN: 0027-8874, DOI: 10.1093/jnci/djm135 * the whole document * | 1-21 | |
| A | J. MARTIN BROWN ET AL: "Exploiting tumour hypoxia in cancer treatment", NATURE REVIEWS CANCER, vol. 4, no. 6, 1 June 2004 (2004-06-01), pages 437-447, XP055019077, ISSN: 1474-175X, DOI: 10.1038/nrc1367 * the whole document * | 1-21 | TECHNICAL FIELDS SEARCHED (IPC) |

The present search report has been drawn up for all claims

| Place of search | Date of completion of the search | Examiner |
|---|---|---|
| Munich | 30 November 2017 | Irion, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

EP 3 296 320 A1

## ANNEX TO THE EUROPEAN SEARCH REPORT
## ON EUROPEAN PATENT APPLICATION NO.

EP 17 19 4775

30-11-2017

| Patent document cited in search report | | Publication date | Patent family member(s) | | Publication date |
|---|---|---|---|---|---|
| WO 03105757 | A2 | 24-12-2003 | AU | 2003256266 A1 | 31-12-2003 |
| | | | CA | 2488836 A1 | 24-12-2003 |
| | | | EP | 1511861 A2 | 09-03-2005 |
| | | | US | 2006141456 A1 | 29-06-2006 |
| | | | WO | 03105757 A2 | 24-12-2003 |
| US 2005260711 | A1 | 24-11-2005 | US | 2005260711 A1 | 24-11-2005 |
| | | | US | 2009035836 A1 | 05-02-2009 |
| WO 2005058236 | A2 | 30-06-2005 | EP | 1691763 A2 | 23-08-2006 |
| | | | JP | 4982183 B2 | 25-07-2012 |
| | | | JP | 2007516709 A | 28-06-2007 |
| | | | US | 2011160440 A1 | 30-06-2011 |
| | | | WO | 2005058236 A2 | 30-06-2005 |
| WO 2012033953 | A1 | 15-03-2012 | CA | 2810668 A1 | 15-03-2012 |
| | | | CN | 103201293 A | 10-07-2013 |
| | | | EP | 2614086 A1 | 17-07-2013 |
| | | | JP | 6121904 B2 | 26-04-2017 |
| | | | JP | 2013541940 A | 21-11-2013 |
| | | | JP | 2016166875 A | 15-09-2016 |
| | | | KR | 20130096731 A | 30-08-2013 |
| | | | US | 2012108455 A1 | 03-05-2012 |
| | | | WO | 2012033953 A1 | 15-03-2012 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61685089 A
- US 13815553 B
- US 13200666 B
- US 1150891 W
- US 61402979 A
- WO 2011059762 PCT
- WO 2005056606 A2
- WO 2006009694 A
- WO 2010080463 A
- WO 2012020059 A
- WO 2008152537 A
- WO 9640210 A
- US 7060808 B
- US 7723484 B
- US 7930107 A
- WO 2011059762 A
- US 7930107 B
- US 7781405 B
- US 7229619 B
- WO 2010054007 A
- US 3896111 A
- US 4151042 A
- US 4137230 A
- US 4248870 A
- US 4256746 A
- US 4260608 A
- US 4265814 A
- US 4294757 A
- US 4307016 A
- US 4308268 A
- US 4308269 A
- US 4309428 A
- US 4313946 A
- US 4315929 A
- US 4317821 A
- US 4322348 A
- US 4331598 A
- US 4361650 A
- US 4364866 A
- US 4424219 A
- US 4450254 A
- US 4362663 A
- US 4371533 A
- US 5500362 A
- US 5821337 A
- WO 2005090407 A
- US 7657380 B
- US 2011014822 A
- US 2005142133 A

- US 2011117110 A
- WO 2012003995 A
- US 4946778 A
- US 5840300 A
- US 5667988 A
- US 5658727 A
- US 5258498 A
- WO 9606641 A
- US 4751180 A
- US 4935233 A
- US 5723323 A
- US 5976862 A
- US 5824514 A
- US 5817483 A
- US 5814476 A
- US 5763192 A
- US 5766886 A
- US 5714352 A
- US 6204023 B
- US 6180370 B
- US 5693762 A
- US 5530101 A
- US 5585089 A
- US 5225539 A
- US 4816567 A
- US 9816280 W
- US 9618978 B
- US 9109630 B
- US 9105939 B
- US 9401234 B
- GB 8901344 A
- GB 9101134 A
- GB 9201755 A
- WO 9014443 A
- WO 9014424 A
- WO 9014430 A
- EP 229246 A
- US 4753894 A
- US 5629197 A
- US 4958009 A
- US 4956453 A
- EP 1391213 A
- US 8142784 B
- US 5208020 A
- US 20110217321 A
- US 5663149 A
- WO 2002088172 A
- US 20110020343 A
- US 5622 A

- US 958 A
- US 5082927 A
- US 5257970 A
- US 5252720 A
- US 5238940 A
- US 5192788 A
- US 5171749 A
- US 5149708 A
- US 5202317 A
- US 5217966 A
- US 5053423 A
- US 5109016 A
- US 5087636 A
- US 5028594 A
- US 5093349 A
- US 4968715 A
- US 4920143 A
- WO 9302192 A
- WO 9303709 A
- US 07745900 B
- WO 9301286 A
- US 07723454 B
- US 5218088 A
- US 5175269 A
- US 5109124 A
- US 5168053 A, Altman
- US 5190931 A, Inouye
- US 5135917 A, Burch
- US 5087617 A, Smith
- US 5176996 A, Hogan
- US 5272262 A
- US 5144019 A
- US 5180818 A
- US 5116742 A
- US 5093246 A, Cech
- WO 8805077 A
- US 5237016 A
- US 20050276812 A
- US 6214345 B
- WO 02088172 A

- US 2003130189 A
- US 2003096743 A
- WO 03026577 A
- WO 03043583 A
- WO 04032828 A
- US 5223409 A
- US 5571698 A
- US 5403484 A
- WO 04043276 A
- US 7989606 B
- WO 9807408 A
- US 8071093 B
- US 20110111059 A
- US 200666 A
- WO 9316185 A
- US 5571894 A
- US 5587458 A
- US 5641870 A
- US 20080248028 A
- US 5679377 A
- US 5916597 A
- US 5912015 A
- US 5989463 A
- US 5128326 A
- WO 9915154 A
- WO 9920253 A
- US 5323907 A
- US 5052558 A
- US 5033252 A
- US 5804396 A
- WO 9909016 A
- WO 9843960 A
- WO 9738983 A
- WO 9906378 A
- WO 9906396 A
- WO 9630347 A
- WO 9633978 A
- WO 9633979 A
- WO 9633980 A

**Non-patent literature cited in the description**

- Computational Molecular Biology. Oxford University Press, 1988
- Biocomputing: Informatics and Genome Projects. Academic Press, 1993
- Computer Analysis of Sequence Data, Part I. Humana Press, 1994
- Sequence Analysis in Molecular Biology. Academic Press, 1987
- Sequence Analysis Primer. M Stockton Press, 1991
- **CARRILLO et al.** *SIAM J Applied Math,* 1988, vol. 48, 1073
- **SCATCHARD et al.** Ann N.Y. Acad. Sci. 1949, vol. 51, 660

- **RICH ; MYSZKA.** *Curr. Opin. Biotechnol,* 2000, vol. 11, 54
- **ENGLEBIENNE.** *Analyst.,* 1998, vol. 123, 1599
- Fundamental Immunology. Raven Press, 1989, 332-336
- **MALMQVIST.** *Biochem. Soc. Trans.,* 2000, vol. 27, 335
- Methods in Molecular Biology. Recombinant Antibodies for Cancer Therapy Methods and Protocols. 2003, vol. 207, 3-25
- **ARNDT et al.** *J Mol Biol.,* 2001, vol. 7 (312), 221-228

- **KABAT, E.A. et al.** Sequences of Proteins of Immunological Interest. U.S. Department of Health and Human Services, 1991
- **CHOTHIA, C. et al.** *J. Mol. Biol.,* 1987, vol. 196, 901-917
- **EDELMAN et al.** *Proc Natl. Acad. Sci. USA,* 1969, vol. 63, 78-85
- **LEFRANC.** *Nucleic Acids Res.,* 2003, vol. 31, 307-310
- Bioinformatics Tools for Antibody Engineering. **MARTIN et al.** Handbook of Therapeutic Antibodies. Wiley-VCH, 2007, 104-107
- **LEFRANC et al.** *Briefings in Bioinformatics,* 2008, vol. 9, 263-275
- **FIELD et al.** *Mol. Cell. Biol.,* 1988, vol. 8, 2159-2165
- **EVAN et al.** *Molecular and Cellular Biology,* 1985, vol. 5, 3610-3616
- **PABORSKY et al.** *Protein Engineering,* 1990, vol. 3, 547-553
- *J. Biol. Chem.,* 1968, vol. 243, 3557-59
- **WATSON et al.** Molecular Biology of the Gene. The Benjamin/Cummings Pub. co, 1987, 224
- **NEEDLEMAN et al.** *J. Mol. Biol.,* 1970, vol. 48, 443
- *Adv. Appl. Math.,* 1981, vol. 2, 482
- **GRIBSKOV et al.** *Nucl. Acids Res.,* 1986, vol. 14, 6745
- Atlas of Protein Sequence and Structure. National Biomedical Research Foundation, 1979, 353-358
- *Adv. Appl. Math.,* 1991, vol. 12, 337-357
- **KAWAI et al.** *Chem. Pharm. Bull.,* 1984, 3441-3451
- **RAVETCH ; KINET.** *Annu. Rev. Immunol,* 1991, vol. 9, 457-92
- **CLYNES et al.** *PNAS (USA),* 1998, vol. 95, 652-656
- **WILMAN.** *Biochemical Society Transactions,* 1986, vol. 14, 375-382
- **STELLA et al.** Prodrugs: A Chemical Approach to Targeted Drug Delivery. Humana Press, 1985, 247-267
- *Biochem.,* 1972, vol. 11 (9), 1726-1732
- **ENG C.** *Nat. Rev. Clin. Oncol.,* 2009, vol. 6, 207-218
- **YARDEN ; SLIWKOWSKI.** *Nat Rev Mol Cell Biol,* 2001, vol. 2, 127-137
- **HERBST ; HONG.** *Seminars in Oncology,* 2002, vol. 29 (5), 18-30
- **HERBST et al.** *Expert Opin. Biol. Ther.,* 2001, vol. 1 (4), 719-732
- **PETIT.** *Am J Pathol,* 1997, vol. 151, 1523-1530
- **LACOUTURE ; MELOSKY.** *Skin Therapy Lett.,* 2007, vol. 12, 1-5
- **NANNEY et al.** *J. Invest. Dermatol,* 1990, vol. 94 (6), 742-748
- **LACOUTURE, M.E.** *Nat Rev Cancer,* 2006, vol. 6, 803-812
- **LE ; PEREZ-SOLER.** *Target Oncol,* 2009, vol. 4, 107-119
- **ENG.** *Nat Rev Clin Oncol,* 2009, vol. 6, 207-218
- **MONTI et al.** *Int J Biol Markers,* 2007, vol. 22, S53-S61
- **SAIF ; KIM.** *Expert Opin Drug Saf,* 2007, vol. 6, 175-182
- **CHUNG et al.** *N Engl J Med,* 2008, vol. 358, 1109-1117
- **HOAG et al.** *J Experimental & Clinical Cancer Research,* 2009, vol. 28, 113
- **FAKIH ; VINCENT.** *Curr. Oncol.,* 2010, vol. 17 (S1), S18-S30
- **GILL et al.** *J Biol Chem,* 1984, vol. 259, 7755-7760
- **SATO et al.** *Mol Biol Med,* 1983, vol. 1, 511-529
- **MASUI et al.** *Cancer Res,* 1984, vol. 44, 1002-1007
- **KAWAMOTO et al.** *Proc Natl Acad Sci USA,* 1983, vol. 80, 1337-1341
- **MARTIN et al.** *Proc Natl Acad Sci USA,* 1989, vol. 86, 9268-9272
- **MARTIN et al.** *Methods Enzymol,* 1991, vol. 203, 121-153
- **PEDERSON et al.** *Immunomethods,* 1992, vol. 1, 126
- **PEDERSEN et al.** *Immunomethods,* 1992, vol. 1, 126
- **LI et al.** *Cancer Cell,* 2005, vol. 7, 301-311
- **LIPPOW et al.** *Nat Biotechnol.,* 2007, vol. 25 (10), 1171-1176
- **BLICK et al.** *Drugs,* 2007, vol. 67 (17), 2585-2607
- **PERROTTE et al.** *Clin. Cancer Res.,* 1999, vol. 5, 257-264
- **PETIT et al.** *Am. J. Pathol.,* 1997, vol. 151, 1523-1530
- **PREWETT et al.** *Clin. Cancer Res.,* 1998, vol. 4, 2957-2966
- **FOGH ANDERSEN et al.** *Clin. Chem.,* 1995, vol. 41, 1522-1525
- **BHUJWALLA et al.** *NMR Biomed.,* 2002, vol. 15, 114-119
- **HELMLINGER et al.** *Nature Med.,* 1997, vol. 3, 177
- **GERWECK ; SEETHARAMAN.** *Cancer Res.,* 1996, vol. 56 (6), 1194-1198
- **WALENTA et al.** *American Journal of Pathology,* 1997, vol. 150 (2), 409-415
- **SCHWICKERT et al.** *Cancer Research,* 1995, vol. 55, 4757-4759

- **WALENTA et al.** *Cancer Research,* 2000, vol. 60, 916-921
- **GUO et al.** *J Nucl Med,* 2004, vol. 45, 1334-1339
- **MATHUPALA et al.** *J Bioenerg Biomembr,* 2007, vol. 39, 73-77
- **HOLROYDE et al.** *Cancer Research,* 1979, vol. 39, 4900-4904
- **SCHURR.** *Neuroscience,* 2007, vol. 147, 613-619
- **QUENNETA et al.** *Radiotherapy and Oncology,* 2006, vol. 81, 130-135
- **AUKLAND ; REED.** *Physiological Reviews,* 1993, vol. 73, 1-78
- **RICH et al.** *Analytical Biochemistry,* 2009, vol. 386, 194-216
- Recombinant Antibodies for Cancer Therapy Methods and Protocols. *Methods in Molecular Biology,* 2003, vol. 207, 3-25
- **WHITLOW ; FILPULA.** *Methods,* 1991, vol. 2, 97-105
- **BIRD et al.** *Science,* 1988, vol. 242, 423-426
- **PACK et al.** *Bio/Technology,* 1993, vol. 11, 1271-77
- **MARASCO et al.** *Proc. Natl. Acad. Sci. USA,* 1993, vol. 90, 7889-7893
- **HUSTON et al.** *Proc Natl Acad Sci USA,* 1988, vol. 85, 5879-5883
- **WHITLOW et al.** *Protein Engineering,* 1993, vol. 6, 989-995
- **NEWTON et al.** *Biochemistry,* 1996, vol. 35, 545-553
- **KABAT et al.** Sequences of Proteins of Immunological Interest. U.S. Dept. Health, 1983
- **JONES et al.** *Nature,* 1986, vol. 321, 522
- **RIECHMANN et al.** *Nature,* 1988, vol. 332, 323
- **VERHOEYEN et al.** *Science,* 1988, vol. 239, 1534
- **SIMS et al.** *J. Immunol.,* 1993, vol. 151, 2296
- **CHOTHIA ; LESK.** *J. Mol. Biol.,* 1987, vol. 196, 901
- **CARTER et al.** *Proc. Natl. Acad. Sci. U.S.A.,* 1992, vol. 89, 4285
- **PRESTA et al.** *J. Immunol.,* 1993, vol. 151, 2623
- **CUNNINGHAM ; WELLS.** *Science,* 1989, vol. 244, 1081-1085
- **SMITH et al.** *J. Mol. Biol.,* 1992, vol. 224, 899-904
- **DE VOS et al.** *Science,* 1992, vol. 255, 306-312
- **MARTIN et al.** Bioinformatics Tools for Antibody Engineering in Handbook of Therapeutic Antibodies. Wiley-VCH, 2007, 104-107
- **NAGDELAINE-BEUZELIN et al.** *Critical Reviews in Oncology/Hematology,* 2007, vol. 64, 210-225
- **SEETHARAM et al.** *J. Biol. Chem.,* 1991, vol. 266, 17376-17381
- **BUCHNER et al.** *Anal. Biochem.,* 1992, vol. 205, 263-270
- **TAKAHASHI et al.** *Cancer,* 1988, vol. 61, 881-888
- **ROWLAND et al.** *Cancer Immunol Immunother,* 1986, vol. 21 (3), 183-187
- **MANDLER et al.** *J. Nat. Cancer Inst.,* 2000, vol. 92 (19), 1573-1581
- **MANDLER et al.** *Bioorg. Med. Chem. Lett.,* 2000, vol. 10, 1025-1028
- **MANDLER et al.** *Bioconjug. Chem.,* 2002, vol. 13, 786-791
- **CHARI.** *Acc Chem Res,* 2008, vol. 41, 98-107
- **LIU et al.** *Proc. Natl. Acad. Sci. USA,* 1996, vol. 93, 8618-8623
- **DAMLE.** *Expert Opin Biol Ther,* 2004, vol. 4, 1445-1452
- **LODE et al.** *Cancer Res.,* 1998, vol. 58, 2928
- **HINMAN et al.** *Cancer Res.,* vol. 53, 3336-3342
- **DORONIN et al.** *Nature Biotechnology,* 2003, vol. 21 (7), 778-784
- **REMILLARD et al.** *Science,* 1975, vol. 189, 1002-1005
- **ISSEL et al.** *Can. Treatment. Rev.,* 1978, vol. 5, 199-207
- **YU et al.** *PNAS,* 2002, vol. 99, 7968-7973
- **WOYKE et al.** *Antimicrob. Agents and Chemother,* 2001, vol. 45 (12), 3580-3584
- **PETTIT et al.** *Antimicrob. Agents Chemother,* 1998, vol. 42, 2961-2965
- **SENTER.** *Curr Opin Chem Biol,* 2009, vol. 13, 235-244
- **DORONINA et al.** *Bioconjug Chem.,* 2006, vol. 17, 114-124
- **DORONINA et al.** *Nat Biotechnol.,* 2003, vol. 21, 778-784
- **SENTER et al.** *Proceedings of the American Association for Cancer Research,* 2004, vol. 45
- **ISLAM et al.** *J. Med. Chem.,* 1991, vol. 34, 2954-61
- **SKIBO et al.** *J. Med. Chem.,* 1994, vol. 37, 78-92
- **BEHROOZI et al.** *Biochemistry,* 1996, vol. 35, 1568-74
- **HELISSEY et al.** *Anticancer Drug Res.,* 1996, vol. 11, 527-51
- **UNNO et al.** *Chem. Pharm. Bull.,* 1997, vol. 45, 125-33
- **UNNO et al.** *Bioorg. Med. Chem.,* 1997, vol. 5, 903-19
- **UNNO et al.** *Bioorg. Med. Chem.,* 1997, vol. 5, 883-901
- **XU et al.** *Biochemistry,* 1998, vol. 37, 1890-7
- **LEE et al.** *Biochem. Mol. Biol. Int.,* 1996, vol. 40, 151-7

- **ROUTIER et al.** *Bioconjug. Chem.,* 1997, vol. 8, 789-92
- **STIRPE et al.** *Bio/Technology,* 1992, vol. 10, 405-12
- **PASTAN et al.** *Annu. Rev. Biochem.,* vol. 61, 331-54
- **BRINKMANN ; PASTAN.** *Biochim. et Biophys. Acta,* 1994, vol. 1198, 27-45
- **SANDVIG ; VAN DEURS.** *Physiol. Rev.,* 1996, vol. 76, 949-66
- **ARMSTRONG.** *J. Infect. Dis.,* 1995, vol. 171, 1042-5
- **KIM et al.** *Microbiol. Immunol.,* 1997, vol. 41, 805-8
- **SKINNER et al.** *Microb. Pathog.,* 1998, vol. 24, 117-22
- **MESRI et al.** *J. Biol. Chem.,* 1993, vol. 268, 4853-62
- **OGAWA et al.** *Science,* 1999, vol. 283, 2097-100
- **SMARDA et al.** *Folia Microbiol (Praha),* 1998, vol. 43, 563-82
- **WOOL et al.** *Trends Biochem. Sci.,* 1992, vol. 17, 266-69
- **SULLENGER et al.** *Science,* 1994, vol. 262, 1566-1569
- **RIORDAN et al.** *Science,* 1989, vol. 245, 1066-1073
- **AGRAWAL et al.** *Tetrahedron Lett.,* 1987, vol. 28, 3539-3542
- **MILLER et al.** *J. Am. Chem. Soc.,* 1971, vol. 93, 6657-6665
- **STEC et al.** *Tetrahedron Lett.,* 1985, vol. 26, 2191-2194
- **MOODY et al.** *Nucl. Acids Res.,* 1989, vol. 17, 4769-4782
- **LETSINGER et al.** *Tetrahedron,* 1984, vol. 40, 137-143
- **ECKSTEIN.** *Annu. Rev. Biochem.,* 1985, vol. 54, 367-402
- **ECKSTEIN.** *Trends Biochem. Sci.,* 1989, vol. 14, 97-100
- **STEIN.** Oligodeoxynucleotides. Antisense Inhibitors of Gene Expression. Macmillan Press, 1989, 97-117
- **JAGER et al.** *Biochemistry,* 1988, vol. 27, 7237-7246
- **CLUSEL et al.** *Nucl. Acids Res.,* 1993, vol. 21, 3405-3411
- **DANG et al.** *J. Biol. Chem.,* 1989, vol. 264, 18019-18023
- **DANG et al.** *Mol. Cell. Biol.,* 1988, vol. 8, 4048-4054
- **GOODCHILD.** Perspectives in Bioconjugate Chemistry. American Chemical Society, 1993, 77-99
- **SPERLING et al.** *Nucleic Acids Res.,* 1978, vol. 5, 2755-2773
- **FISER et al.** *FEBS Lett.,* 1975, vol. 52, 281-283
- **BÄUMERT et al.** *Eur. J. Biochem.,* 1978, vol. 89, 353-359
- **OSTE et al.** *Mol. Gen. Genet.,* 1979, vol. 168, 81-86
- **VANIN et al.** *FEBS Lett.,* 1981, vol. 124, 89-92
- **RINKE et al.** *J.Mol.Biol.,* 1980, vol. 137, 301-304
- **MILLON et al.** *Eur. J. Biochem.,* 1980, vol. 110, 485-492
- **CHENG et al.** *Nucleic Acids Res.,* 1983, vol. 11, 659-669
- **CHU et al.** *Nucleic Acids Res.,* 1983, vol. 11, 6513-6529
- **CHU et al.** *Nucl. Acids Res.,* 1988, vol. 16, 3671-3691
- **MANIATIS et al.** Molecular Cloning: A Laboratory Manual. Cold Spring Harbor Laboratory, 1982, 122
- **HERMANSON, G. T.** Bioconjugate Techniques. Academic Press, 1996, 234-242
- **E. SCHRODER ; K. LUBKE.** The Peptides. Academic Press, 1965, vol. 1, 76-136
- Automation of solid-phase peptide synthesis. **GEISER et al.** Macromolecular Sequencing and Synthesis. Alan R. Liss, Inc, 1988, 199-218
- **FIELDS, G. ; NOBLE, R.** Solid phase peptide synthesis utilizing 9-fluorenylmethoxycarbonyl amino acids. *Int. J. Peptide Protein Res.,* 1990, vol. 35, 161-214
- **DOSIO et al.** *Toxins,* 2010, vol. 3, 848-883
- **SANDERSON et al.** *Clin Cancer Res,* 2005, vol. 11, 843-852
- **DUCRY ; STUMP.** *Bioconjug Chem.,* 2010, vol. 21, 5-13
- **TOKI et al.** *J. Org. Chem.,* 2002, vol. 67, 1866-1872
- **AXWORTHY et al.** *Proc. Natl. Acad. Sci. USA,* 2000, vol. 97 (4), 1802-1807
- **LEWIS et al.** *Bioconj. Chem.,* 1998, vol. 9, 72-86
- **SUN et al.** *Bioorganic & Medicinal Chemistry Letters,* 2002, vol. 12, 2213-2215
- **SUN et al.** *Bioorganic & Medicinal Chemistry,* 2003, vol. 11, 1761-1768
- **KING et al.** *Tetrahedron Letters,* 2002, vol. 43, 1987-1990
- **KOHLER et al.** *Nature,* 1975, vol. 256, 495
- **GODING.** Monoclonal Antibodies: Principles and Practice. Academic Press, 1986, 59-103
- **MUNSON et al.** *Anal. Biochem.,* 1980, vol. 107, 220
- **LILIOM et al.** *J. Immunol Methods,* 1991, vol. 143 (1), 119-25
- **HOUGHTEN.** *Bio/Techniques,* 1992, vol. 13, 412-421

- **LAM.** *Nature,* 1991, vol. 354, 82-84
- **FODOR.** *Nature,* 1993, vol. 364, 555-556
- **CULL et al.** *Proc. Natl. Acad. Sci. USA,* 1992, vol. 89, 1865-1869
- **SCOTT ; SMITH.** *Science,* 1990, vol. 249, 386-390
- **DEVLIN.** *Science,* 1990, vol. 249, 404-406
- **CWIRLA et al.** *Proc. Natl. Acad. Sci. USA,* 1990, vol. 87, 6378-6382
- **FELICI.** *J. Mol. Biol.,* 1991, vol. 222, 301-310
- **PILIARIK et al.** *Methods Mol Biol.,* 2009, vol. 503, 65-88
- **BIACORE.** Biacore AB, Upsala. Sweden and GE Healthcare Life Sciences, 2000
- **MALMQVIST et al.** *Curr Opin Immunol.,* 1993, vol. 5 (2), 282-6
- **GARCIA-OJEDA et al.** *Infect Immun.,* 2004, vol. 72 (6), 3451-60
- **SAXENA et al.** *J. Clin. Oncol.,* 2011, vol. 29, e13030
- **JECKLIN et al.** *J. Mol. Recognit.,* 2009, vol. 22 (4), 319-29
- **NGUYEN et al.** *Methods,* 2007, vol. 42 (2), 150-61
- **TANIOUS et al.** *Methods Cell Biol.,* 2008, vol. 84, 53-77
- **ALVARENGA et al.** *Anal. Biochem,* 2012, vol. 421 (1), 138-151
- Current Protocols in Molecular Biology. John Wiley & Sons, Inc, 1994, vol. 1
- **MONROE et al.** *Amer. Clin. Prod. Rev.,* 1986, vol. 5, 34-41
- **MYERS et al.** *Proc. Natl. Acad. Sci. USA,* 1975
- **FEAU et al.** *J. Biomol. Screen.,* 2009, vol. 14 (1), 43-48
- **PEROZZO et al.** *J. Recept Signal. Transduct Res.,* 2004, vol. 24 (1-2), 1-52
- **HOLDGATE.** *Biotechniques,* 2001, vol. 31 (1), 164-166, 168, , 170
- **CELEJ et al.** *Anal. Biochem.,* 2006, vol. 350 (2), 277-284
- **PEROZZO et al.** *J. Recept. Signal. Transduct. Res.,* 2004, vol. 24 (1-2), 1-52
- **WU et al.** *J. Pharm. Biomed. Anal.,* 2007, vol. 44 (3), 796-801
- **GLEYSTEEN et al.** *Head & Neck,* 2008, vol. 30 (6), 782-789
- **ROSENTHAL et al.** *Mol. Cancer Ther.,* 2007, vol. 6, 1230-1238
- **ZHANG et al.** *Spectrochim Acta A Biomol. Spectrosc.,* 2009, vol. 72 (3), 621-626
- **OLIVE et al.** *Vitro Cell Dev Biol.,* 1993, vol. 29A (3), 239-248
- **WU et al.** *J. Clin. Invest.,* 1995, vol. 95 (4), 1897-1905
- **ALBANELL et al.** *J. Clin. Oncol.,* 2002, vol. 20 (1), 110-124
- **LIMAT ; HUNZIKER.** *Methods Mol Med.,* 1996, vol. 2, 21-31
- **ABDEL-NASER et al.** *Egypt. Dermatol. Online J.,* 2005, vol. 1 (2), 1
- **KAUFMAN R.J.** *Methods in Enzymology,* 1990, vol. 185, 537-566
- **KAUFMAN et al.** *Methods in Enzymology,* 1990, vol. 185, 537-566
- **HAHN ; SCANLAN.** *Top. Curr. Chem.,* 2010, vol. 296, 1-13
- **CRYAN et al.** *Eur J Pharm Sci.,* 2004, vol. 21 (5), 625-33
- **GAO ; HUANG.** *Gene Ther.,* 1995, vol. 2 (10), 710-722
- **THIERY et al.** *Gene Ther.,* 1997
- **FELGNER et al.** *Annals N.Y. Acad. Sci.,* 1995
- **EASTMAN et al.** *Hum. Gene Ther.,* 1997
- **CHU et al.** *Nucl. Acid. Res.,* 1987, vol. 15 (3), 1311-1326
- **KUMON et al.** *Ultrasound Med Biol.,* 2009, vol. 35 (3), 494-506
- **O'BRIEN ; LUMMIS.** *Methods,* 2004, vol. 33 (2), 121-125
- **FLOTTE ; CARTER.** *Gene Ther.,* 1995, vol. 2 (6), 357-362
- **SESHACHARYULU et al.** *Expert. Opin. Ther. Targets.,* 2012, vol. 16 (1), 15-31
- **MAGHNI et al.** *J. Immunol. Method.,* 1999, vol. 223 (2), 185-194
- *Genetic Eng. Biotechnol. News.,* vol. 26 (6
- **KLEIN et al.** *Nature Medicine,* 1997, vol. 3, 402-408
- **SANTON et al.** *Cancer Res.,* 1986, vol. 46, 4701-05
- **OZAWA et al.** *Int. J. Cancer,* 1987, vol. 40, 706-10
- **REUSCH et al.** *Clin. Cancer Res.,* 2006, vol. 12 (1), 183-190
- **YANG et al.** *Int. J. Nanomedicine,* 2011, vol. 6, 1739-1745
- **RATHINAVELU et al.** *Cancer Biochem. Biophys.,* 1999, vol. 17, 133-146
- **WANG et al.** *J. Invest. Dermatol.,* 2006, vol. 126, 1372-1377
- **MICHAEL J. DERELANKO.** TOXICOLOGIST'S POCKET HANDBOOK. CRC Press, 2000, 16
- **HUANG et al.** *PNAS,* 2009, vol. 106, 3426-3430
- Preclinical Safety Evaluation Of Biotechnology-Derived Pharmaceuticals. *International Conference on Harmonisation Of Technical Requirements For Registration of Pharmaceuticals For Human Use,* July 1997
- **ENG.** *Nat. Rev.,* 2009, vol. 6, 207-218
- **SCHRAG et al.** *J. Natl. Cancer Inst.,* vol. 97 (16), 1221-1224

- **NANNEY et al.** *J. Invest. Dermatol.,* 1996, vol. 106 (6), 1169-1174
- **LUTTERBUESE et al.** *Proc. Natl. Acad. Sci.,* 2010, vol. 107 (28), 12605-12610
- **KLUTCHKO et al.** *J. Med. Chem.,* 1998, vol. 41, 3276-3292
- **TILLER et al.** *J Immunol. Methods,* 2008, vol. 329, 112-24
- **COLOMA et al.** *J Immunol. Methods,* 1992, vol. 152, 89-104
- **MORIMOTO et al.** *Journal of Biochemical and Biophysical Methods,* 1992, vol. 24, 107-117
- **BRENNANCE et al.** *Science,* 1985, vol. 229, 81
- **CARTER et al.** *Bio/Technology,* 1992, vol. 10, 163-167
- **BERNOIST ; CHAMBON.** *Nature,* 1981, vol. 290, 304-310
- **YAMAMOTO et al.** *Cell,* 1980, vol. 22, 787-797
- **WAGNER et al.** *Proc. Natl. Acad. Sci. USA,* 1981, vol. 78, 1441-1445
- **BRINSTER et al.** *Nature,* 1982, vol. 296, 39-42
- **JAY et al.** *Proc. Natl. Acad. Sci. USA,* 1981, vol. 78, 5543
- **DEBOER et al.** *Proc. Natl. Acad. Sci. USA,* 1983, vol. 80, 21-25
- Useful Proteins from Recombinant Bacteria. *Scientific American,* 1980, vol. 242, 79-94
- **HERRERA-ESTRELLA et al.** *Nature,* 1984, vol. 303, 209-213
- **GARDNER et al.** *Nucleic Acids Res.,* 1981, vol. 9, 2871
- **HERRERA-ESTRELLA et al.** *Nature,* 1984, vol. 310, 115-120
- **SWIFT et al.** *Cell,* 1984, vol. 38, 639-646
- **ORNITZ et al.** *Cold Spring Harbor Symp. Quant. Biol.,* 1986, vol. 50, 399-409
- **MACDONALD.** *Hepatology,* 1987, vol. 7, 425-515
- **HANAHAN et al.** *Nature,* 1985, vol. 315, 115-122
- **GROSSCHEDL et al.** *Cell,* 1984, vol. 38, 647-658
- **ADAMS et al.** *Nature,* 1985, vol. 318, 533-538
- **ALEXANDER et al.** *Mol. Cell Biol.,* 1987, vol. 7, 1436-1444
- **LEDER et al.** *Cell,* 1986, vol. 45, 485-495
- **PINKERT et al.** *Genes and Devel.,* 1987, vol. 1, 268-276
- **KRUMLAUF et al.** *Mol. Cell. Biol.,* 1985, vol. 5, 1639-1648
- **HAMMER et al.** *Science,* 1987, vol. 235, 53-58
- **KELSEY et al.** *Genes and Devel.,* 1987, vol. 1, 161-171
- **MAGRAM et al.** *Nature,* 1985, vol. 315, 338-340
- **KOLLIAS et al.** *Cell,* 1986, vol. 46, 89-94
- **READHEAD et al.** *Cell,* 1987, vol. 48, 703-712
- **SHANI.** *Nature,* 1985, vol. 314, 283-286
- **MASON et al.** *Science,* 1986, vol. 234, 1372-1378
- **TAN et al.** *Protein Eng.,* 2002, vol. 15, 337
- **COLLEY et al.** *J. Biol. Chem.,* 1989, vol. 264, 17619-17622
- Methods in Enzymology. 1990, vol. 182
- **MORRISON.** *J. Bact.,* 1977, vol. 132, 349-351
- **CLARK-CURTISS ; CURTISS.** *Methods in Enzymology,* 1983, vol. 101, 347-362
- **PHAM et al.** *Biotechnol. Bioeng.,* 2003, vol. 84, 332-42
- **MAYFIELD et al.** *PNAS,* 2003, vol. 100, 438-442
- **LIU et al.** *MAbs,* 2010, vol. 2 (5), 480-499
- **BJORCK.** *J. Immunol.,* 1988, vol. 140 (4), 1194-1197
- **KASTERN et al.** *J. Biol. Chem.,* 1992, vol. 267 (18), 12820-12825
- **ELIASSON et al.** *J. Biol. Chem.,* 1988, vol. 263, 4323-4327
- **ANSEL.** Introduction to Pharmaceutical Dosage Forms. 1985, 126
- Goodman and Gilman's: The Pharmacological Bases of Therapeutics. Pergamon Press, 1990
- Remington's Pharmaceutical Sciences. Mack Publishing Co, 1990
- Pharmaceutical Dosage Forms: Parenteral Medications. Dekker, 1993
- Pharmaceutical Dosage Forms: Tablets. Dekker, 1990
- Pharmaceutical Dosage Forms: Disperse Systems. Dekker, 1990
- Remington: The Science and Practice of Pharmacy (Formerly Remington's Pharmaceutical Sciences). Mack Publishing Company, 1995
- **ALFONSO R. GENNARO.** Remington: The Science and Practice of Pharmacy. Lippincott Williams & Wilkins, 2000
- Remington's Pharmaceutical Sciences. Mack Publishing Co,
- Medical Applications of Controlled Release. CRC Pres, 1974
- Controlled Drug Bioavailability, Drug Product Design and Performance. Wiley, 1984
- **LANGER ; PEPPAS.** *J. Macromol. Sci.,* 1983, vol. 23, 61
- **LEVY et al.** *Science,* 1985, vol. 228, 190
- **DURING et al.** *Ann. Neurol.,* 1989, vol. 25, 351
- **HOWARD et al.** *J. Neurosurg.,* 1989, vol. 71, 105
- **REEVES et al.** *Otolaryngol Head Neck Surg.,* 2011, vol. 144 (5), 676-84

- **ADAMS et al.** *Expert Rev Anticancer Ther.,* 2008, vol. 8 (8), 1237-45
- **BELDA-INIESTA et al.** *Cancer Biol Ther.,* 2006, vol. 5 (8), 912-4
- **LIU et al.** *Cancer Chemother Pharmacol.,* 2010, vol. 65 (5), 849-61
- **BRONTE et al.** *Front Biosci.,* 2011, vol. 3, 879-887
- **HARDING ; BURTNESS.** *Drugs Today,* 2005, vol. 41 (2), 107-127
- **SZABO et al.** *Oral Oncol.,* 2011, vol. 47 (6), 487-496
- **STEFAN WALENTA ; AHMAD SALAMEH ; HEIDI LYNG ; JAN F. EVENSEN ; MARGARETHE MITZE ; EINAR K. ROFSTAD ; WOLFGANG MU-ELLER-KLIESER.** *American Journal of Pathology,* 1997, vol. 150 (2), 409-415
- **GEORG SCHWICKERT ; STEFAN WALENTA ; KOLBEIN SUIULFOR ; EINAR K. ROFSTAD ; WOLFGANG MUELLER-KLIESER.** *Cancer Research,* 1995, vol. 55, 4757-4759
- **STEFAN WALENTA ; MICHAEL WETTERLING ; MICHAEL LEHRKE ; GEORG SCHWICKERT ; KOLBEIN SUNDFØR ; EINAR K. ROFSTAD ; WOLFGANG MUELLER-KLIESER.** *Cancer Research,* 2000, vol. 60, 916-921
- **JIANFEI GUO ; KOTARO HIGASHI ; HAJIME YOKOTA ; YOSINOBU NAGAO ; YOSHIMICHI UEDA ; YUKO KODAMA ; MANABU OGUCHI ; SU-ZUKA TAKI ; HISAO TONAMI ; ITARU YAMAMO-TO.** *J Nucl Med,* 2004, vol. 45, 1334-1339
- **SAROJ P. MATHUPALA ; CHAIM B. COLEN ; PRAHLAD PARAJULI ; ANDREW E. SLOAN.** *J Bioenerg Biomembr,* 2007, vol. 39, 73-77
- **CHRISTOPHER P. HOLROYDE ; RITA S. AXELROD ; CHARLES L. SKUTCHES ; AGNES C. HAFF ; PAVLE PAUL ; GEORGE A. REICHARD.** *Cancer Research,* 1979, vol. 39, 4900-4904
- **A SCHURR ; R.S. PAYNE.** *Neuroscience,* 2007, vol. 147, 613-619
- **VERENA QUENNET ; ALA YAROMINAB ; DANIEL ZIPSB ; ANDREA ROSNERB ; STEFAN WALENTAA ; MICHAEL BAUMANNB ; WOLF-GANG MUELLER-KLIESERA.** *Radiotherapy and Oncology,* 2006, vol. 81, 130-135
- **FISKE et al.** *Sci Trasl. Med.,* 2009, vol. 1 (8), 8ra18
- **MYERS et al.** *Mol. Cell. Proteomics,* 2012, vol. 11 (10), 1-15
- **THARIAT et al.** *Clin. Cancer Res.,* 2012, vol. 18, 1313
- **ERVIN-HAYNES et al.** *J. Clin. Oncol. ASCO Annual Meeting Proceedings Part I.,* 20 June 2006, vol. 24 (18S
- **GOLDSTEIN ; ARMIN.** *Cancer,* 2001, vol. 92 (5), 1331-1346
- **BIBEAU et al.** *Virchows Arch.,* 2006, vol. 449 (3), 281-287
- **DALL'OZZO.** *Cancer Res.,* 2004, vol. 64, 4664-4669
- **CARTON.** *Blood,* 2002, vol. 99, 754-758
- **WENG.** *J. Clin. Oncol.,* 2003, vol. 21, 3940-3947
- **SCHRAG et al.** *J. Natl. Cancer Inst.,* 2005, vol. 97 (16), 1221-1224
- **JONKER et al.** *N. Engl. J. Med.,* 2007, vol. 357, 2040-2048
- **NANNEY et al.** *J. Invest. Dermatol,* 1996, vol. 94 (6), 742-748
- CTCAE. U.S. Department of Health and Human Services, 14 June 2010, vol. 4.03
- **SCHRAG J.** *Natl. Cancer. Inst.,* vol. 97 (16), 1221-1224
- **DIELEMAN et al.** *J. Acquir Immune Defic Syndr.,* 2001, vol. 28 (1), 9-13
- **DANTZLER et al.** *Pflugers Arch.,* 2000, vol. 440 (1), 140-148
- **VAN CUTSEM et al.** *J. Clin. Oncol,* 20 May 2008, vol. 26
- **PFEIFFER et al.** *Acta. Oncol.,* 2007, vol. 46 (5), 697-701
- **BEIGIER-BOMPADRE.** *Scand. J. Immunol.,* 2003, vol. 57, 221-8
- **TE ; ANDREWS PA.** Anticancer Drug Development, Guide: Preclinical Screening, Clinical Trials and Approval. Humana Press, 2004, 134
- **T. FRIESS et al.** *Anticancer Research,* 2006, vol. 26, 3505-3512
- **TEICHER BA ; ANDREWS PA.** Anticancer Drug Development, Guide: Preclinical Screening, Clinical Trials and Approval. Humana Press, 2004, 134